# EUROPEAN PATENT APPLICATION

(11) **EP 1 767 656 A2**
(43) Date of publication of application: **28.03.2007**
(21) Application number: 06076080.8
(22) Date of filing: 30.10.2002
(51) Int. Cl.: C12Q 1/68

(54) **Methods for assessing and treating leukemia**

(30) Priority: 30.10.2001 US 340938 P; 30.10.2001 US 338997; 30.10.2001 US 340081; 30.10.2001 US 341012
(62) Divisional of application: 02791197.3
(71) Applicant: ORTHO-CLINICAL DIAGNOSTICS, INC., Raritan, NJ 08869-0606 (US)
(72) Inventor: Raponi, Mitch, San Diego, CA 92121 (US)
(74) Representative: Mercer, Christopher Paul

(57) **Abstract**

Methods for treating leukemia patients include analyzing gene expression profiles of a patient to determine whether the patient is likely to respond to treatment with farnesyl transferase inhibitor (FTI) and, optionally, other therapeutics. The methods are also useful for monitoring patient therapy and for selecting a course of therapy. Genes modulated in response to FTI treatment are provided and are used for formulating the profiles.

## Description

### BACKGROUND

This application claims the benefit of the following US Provisional applications: 60/340,938; 60/338.997; 60/340,081; and 60/341,012. This invention relates to diagnostics, prognostics, and treatments for leukemia based on the gene expression profiles of leukemia cells.

Some molecules, such as Ras, that are implicated in cancers must be farnesylated by the farnesyl transferase enzyme in order to interact with the inner leaflet of the plasma membrane of the cell and become involved in various signaling pathways. Ras is not the only protein implicated in cancer that has a CAAX box that is prenylated. Farnesyl transferase inhibitors (FTIs) are therapeutic agents that inhibit the covalent attachment of the carbon farnesyl moieties to the C-terminal CAAX motif of various proteins. They have utility in the treatment of cancers and proliferative disorders such as leukemia. Acute myelogenous leukemia (AML) is among the diseases that can most beneficially be addressed with FTIs.

As is true in the case of many treatment regimens, some patients respond to treatment with FTIs and others do not. Prescribing the treatment to a patient who is unlikely to respond to it is not desirable. Thus, it would be useful to know how a patient could be expected to respond to such treatment before a drug is administered so that non-responders would not be unnecessarily treated and so that those with the best chance of benefiting from the drug are properly treated and monitored. Further, ofthose who respond to treatment, there may be varying degrees of response. Treatment with therapeutics other than FTIs or treatment with therapeutics in addition to FTIs may be beneficial for those patients who would not respond to FTIs or in whom response to FTIs alone is less than desired.

### SUMMARY OF THE INVENTION

The invention is a method of treating a patient with leukemia with an FTI. In one such method, the patient's gene expression profile is analyzed to determine whether the patient is likely to respond to the FTI and treating a patient with the FTI if they are likely to respond.

In another aspect of the invention, a patient with leukemia is monitored for treatment with an FTI in which the patient's gene expression profile is analyzed to determine whether the patient is responding to the FTI and treating a patient with the FTI if they are likely to respond in a desirable fashion.

In yet another aspect of the invention, a patient is treated if the gene expression profile shows up regulation of one or more particular genes indicative of FTI responders.

In yet another aspect of the invention, gene expression profiles indicative of FTI responders are those which show at least a 1.5, 1.7, or 2 fold difference relative to FTI non-responders.

In yet another aspect of the invention, a patient is treated if the gene expression profile shows down regulation of one or more particular genes indicative of FTI responders

In yet another aspect of the invention, a patient is treated if the gene expression profile shows modulation of a gene selected from the group of genes identified in Tables 1-3 infra.

In yet another aspect of the invention, the FTI is a quinilone or quinoline derivative.

In yet another aspect of the invention, the FTI is (B)-6-[amino(4-chlorophenyl)(1-methyl-1 H-imidazol-5-yl)methyl]-4-(3-chlorophenyl)-1-methyl-2(1H)-quinolinone).

Articles used in practicing the methods are also an aspect of the invention. Such articles include gene expression profiles or representations of them that are fixed in computer readable media. Other articles according to the invention include nucleic acid arrays used to determine the gene expression profiles of the invention.

In another aspect of the invention, a method of treating a patient with leukemia comprises administering an FTI and a therapeutic composition that modulates the MAPK/ERK signaling pathways, TGFβ, WNT or apoptotic pathways.

In another aspect of the invention, the patient is treated with an FTI and a therapeutic composition selected from the group consisting of tyrosine kinase inhibitors, MEK kinase inhibitors, PI3K kinase inhibitors, MAP kinase inhibitors, apoptosis modulators and combinations thereof.

In yet another aspect of this invention, the gene expression profile of a patient with leukemia is analyzed to determine whether the patient is likely to respond to an FTI or if the patient would likely benefit from the combination of an FTI and another drug. The patient is then treated with such combination or, if the patient is unlikely to respond to an FTI, the patient is treated with drug selected from the group consisting of tyrosine kinase inhibitors, MEK kinase inhibitors, PI3K kinase inhibitors, MAP kinase inhibitors, apoptosis modulators and combinations thereof.

### BRIEF DESCRIPTION OF THE DRAWINGS

Fig. 1 is an example of a graphical display of gene expression patterns used to analyze the gene expression profiles of this invention.
Fig. 2 is a schematic diagram of the MAPK/ERK pathway.
Fig. 3 is a schematic diagram of the TGFβ and Wnt pathway.
Fig. 4 is a schematic diagram of the apoptotic pathway.

### DETAILED DESCRIPTION

The therapeutic agents referred to in this specification are FTIs. They take on a multitude of forms but share the essential inhibitory function of interfering with or lessening the farnesylation of proteins implicated in cancer and proliferative diseases. Preferably, the FTIs are those indicated for the treatment of leukemias such as AML. A patient who responds to an FTI is one in whom a reduction of more than 50% of blast cells is seen in bone marrow following treatment with the FTI.

Numerous FTIs are within the scope of the invention and include those described in U.S. Patents: 5,976,851 to Brown et al; 5,972,984 to Anthony et al.; 5,972,966 to deSolms; 5,968,965 to Dinsmore et al.; 5,968,952 to Venet et al.; 6,187,786 to Venet et al.; 6,169,096 to Venet et al.; 6,037,350 to Venet et. al.; 6,177,432 to Angibaud et al.; 5,965,578 to Graham et al.; 5,965,539 to Sebti et al.; 5,958,939 to Afonso et al.; 5,939,557 to Anthony et al.; 5,936,097 to Commercon et al.; 5,891,889 to Anthony et al.; 5,889,053 to Baudin et al.; 5,880,140 to Anthony; 5,872,135 to deSolms; 5,869,682 to deSolms; 5,861,529 to Baudoin; 5,859,015 to Graham et al.; 5,856,439 to Clerc; 5,856,326 to Anthony et al.; 5,852,010 to Graham et al.; 5,843,941 to Marsters et al.; 5,807,852 to Doll; 5,780,492 to Dinsmore et al.; 5,773,455 to Dong et al.; 5,767,274 to Kim et al.; 5,756,528 to Anthony et al.; 5,750,567 to Baudoin et al.; 5,721,236 to Bishop et al,; 5,700,806 to Doll et al.; 5,661,161 to Anthony et al.; 5,602,098 to Sebti et al.; 5,585,359 to Breslin et al.; 5,578,629 to Ciccarone et al.; 5,534,537 to Ciccarone et al.; 5,532,359 to Marsters et al.; 5,523,430 to Patel et al.; 5,504,212 to de Solms et al.; 5,491,164 to desolms et al.; 5,420,245 to Brown et al.; and 5,238,922 to Graham et al. each of which is incorporated herein by reference. Non-peptidal, so-called "small molecule" therapeutics are preferred. More preferred FTIs are quinolines or quinoline derivatives such as:
7-(3-chlorophenyl)-9-[(4-chlorophenyl)-1H-imidazol-1-ylmethyl]-2,3-dihydro- 1H,5H-benzo [i j]quino lizin-5-one,
7-(3-chlorophenyl)-9-[(4-chlorophenyl)-1H-imidazol-1-ylmethyl]-1,2-dihydro-4H-pyrrolo[3,2, I-ij]quinoline-4-one,
8-[amino(4-chlorophenyl)(1-methyl-1H-imidazol-5-yl),methyl]-6-(3-chlorophen yl)-1,2-dihydro-4H pynolo[3,2,1-ij]quinolin-4-one, and
8-[amino(4-chlorophenyl)(1-methyl-1H-imidazol-5-yl)methyl]-6-(3-chloropheny 1)-2,3-dihydro-1H,5H-benzo[ij]quinolizin-5-one.
The most preferred FTI is (B)-6-[amino(4-chlorophenyl)(1-methyl-1H-imidazol-5-yl)methyl]-4-(3-chlorophenyl)-1-methyl-2(1H)-quinolinone).

In the aspect of the invention comprising treating leukemia with FTIs and other therapeutic agents, The therapeutic agents referred to in this specification are those that have an effect on the biological pathway explicated through the gene expression analysis of leukemic cells subjected to treatment with quinilone-based FTIs.

The mere presence of nucleic acid sequences having the potential to express proteins or peptides ("genes") within the genome is not determinative of whether a protein or peptide is expressed in a given cell. Whether or not a given gene capable of expressing proteins or peptides does so and to what extent such expression occurs, if at all, is determined by a variety of complex factors. Irrespective of difficulties in understanding and assessing these factors, assaying gene expression can provide useful information about the cellular response to a given stimulus such as the introduction of a drug or other therapeutic agent. Relative indications of the degree to which genes are active or inactive can be found in gene expression profiles. The gene expression profiles of this invention are used to identify and treat patients who will likely benefit from a given therapy or exclude patients from a given therapy where the patient likely would experience little or no beneficial response to the drug or therapy.

Preferred methods for establishing gene expression profiles (including those used to arrive at the explication of the relevant biological pathways) include determining the amount ofRNA that is produced by a gene that can code for a protein or peptide. This is accomplished by reverse transcription PCR (RT-PCR), competitive RT-PCR, real time RT-PCR, differential display RT-PCR, Northern Blot analysis and other related tests. While it is possible to conduct these techniques using individual PCR reactions, it is best to amplify copy DNA (cDNA) or copy RNA (cRNA) produced from mRNA and analyze it via microarray. A number of different array configurations and methods for their production are known to those of skill in the art and are described in U.S. Patents such as: 5,445,934; 5,532,128; 5,556,752; 5,242,974; 5,384,261; 5,405,783; 5,412,087; 5,424,186; 5,429,807; 5,436,327; 5,472,672; 5,527,681; 5,529,756; 5,545,531; 5,554,501; 5,561,071; 5,571,639; 5,593,839; 5,599,695; 5,624,711; 5,658,734; and 5,700,637; the disclosures of which are herein incorporated by reference.

Microarray technology allows for the measurement of the steady-state mRNA level of thousands of genes simultaneously thereby presenting a powerful tool for identifying the effect of FTIs on cell biology and the likely effect of treatment based on analysis of such effects. Two microarray technologies are currently in wide use. The first are cDNA arrays and the second are oligonucleotide arrays. Although differences exist in the construction of these chips, essentially all downstream data analysis and output are the same. The product of these analyses are typically measurements of the intensity of the signal received from a labeled probe used to detect a cDNA sequence from the sample that hybridizes to a nucleic acid sequence at a known location on the microarray. Typically, the intensity of the signal is proportional to the quantity of cDNA, and thus mRNA, expressed in the sample cells. A large number of such techniques are available and useful. Preferred methods for determining gene expression can be found in US Patents 6,271,002 to Linsley, et al.; 6,218,122 to Friend, et al.; 6,218,114 to Peck, et al.; and 6,004,755 to Wang, et al., the disclosure of each of which is incorporated herein by reference.

Analysis of the expression levels is conducted by comparing such intensities. This is best done by generating a ratio matrix of the expression intensities of genes in a test sample versus those in a control sample. For instance, the gene expression intensities from a tissue that has been treated with a drug can be compared with the expression intensities generated from the same tissue that has not been treated with the drug. A ratio of these expression intensities indicates the fold-change in gene expression between the test and control samples.

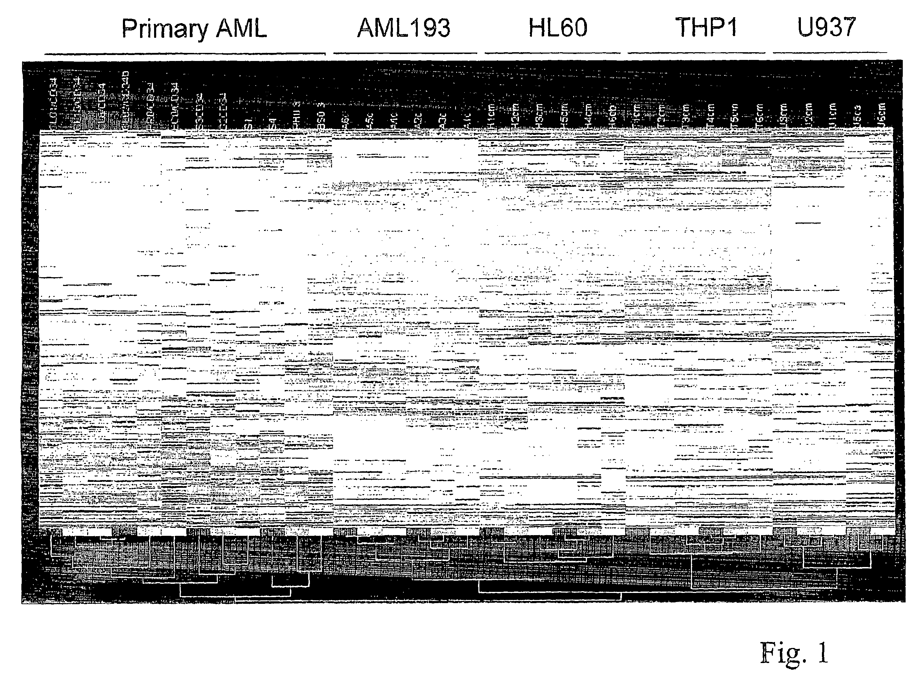

Gene expression profiles can also be displayed in a number of ways. The most common method is to arrange a ratio matrix into a graphical dendogram where columns indicate test samples and rows indicate genes. The data is arranged so genes that have similar expression profiles are proximal to each other (e.g., Fig. 1). The expression ratio for each gene is visualized as a color. For example, a ratio less than one (indicating down-regulation) may appear in the blue portion of the spectrum while a ratio greater than one (indicating up-regulation) may appear as a color in the red portion of the specrtum. Commercially available computer software programs are available to display such data including "OMNIVIZ PRO" software from Batelle and "TREE VIEW" software from Stanford

The genes that are differentially expressed are either up regulated or down regulated in diseased cells following treatment with an FTI. Up regulation and down regulation are relative terms meaning that a detectable difference (beyond the contribution of noise in the system used to measure it) is found in the amount of expression of the genes relative to some baseline. In this case, the baseline is the measured gene expression of the untreated diseased cell. The genes of interest in the treated diseased cells are then either up regulated or down regulated relative to the baseline level using the same measurement method. Preferably, levels of up and down regulation are distinguished based on fold changes of the intensity measurements of hybridized microarray probes. A 1.5 fold difference is preferred for making such distinctions. That is, before a gene is said to be differentially expressed in treated versus untreated diseased cells, the treated cell is found to yield at least 1.5 times more, or 1.5 times less intensity than the untreated cells. A 1.7 fold difference is more preferred and a 2 or more fold difference in gene expression measurement is most preferred. Table 3 lists genes that were commonly modulated across all cell lines and in responder samples.

A portfolio of genes is a set of genes grouped so that information obtained about them provides the basis for making a clinically relevant judgment such as a diagnosis, prognosis, or treatment choice. In this case, the judgments supported by the portfolios involve the treatment ofleukemias with FTIs. Portfolios of gene expression profiles can be comprised of combinations of genes shown in Tables 1-3.

One method of the invention involves comparing gene expression profiles for various genes to determine whether a person is likely to respond to the use of a therapeutic agent. Having established the gene expression profiles that distinguish responder from nonresponder, the gene expression profiles of each are fixed in a medium such as a computer readable medium as described below. A patient sample is obtained that contains diseased cells (such as hematopoietic blast cells in the case of AML) is then obtained. Sample RNA is then obtained and amplified from the diseased patient cell and a gene expression profile is obtained, preferably via micro-array, for genes in the appropriate portfolios. The expression profiles of the samples are then compared to those previously determined as responder and non-responder. If the sample expression patterns are consistent with an FTI responder expression pattern then treatment with an FTI could be indicated (in the absence of countervailing medical considerations). If the sample expression patterns are consistent with an FTI non-responder expression pattern then treatment with an FTI would not be indicated. Preferably, consistency of expression patterns is determined based on intensity measurements of micro-array reading as described above.

In similar fashion, gene expression profile analysis can be conducted to monitor treatment response. In one aspect of this method, gene expression analysis as described above is conducted on a patient treated with an FTI at various periods throughout the course of treatment. If the gene expression patterns are consistent with a responder then the patient's therapy is continued. If it is not, then the patient's therapy is altered as with additional therapeutics such as tyrosine kinase inhibitor, changes to the dosage, or elimination of FTI treatment. Such analysis permits intervention and therapy adjustment prior to detectable clinical indicia or in the face of otherwise ambiguous clinical indicia.

It is possible to attain ambiguous results in which some gene expression profiles are recorded that are in some respects indicative of a responder and in other respects indicative of a non-responder. For example, the profiles may show that three genes are up-regulated consistent with a responder but that another gene is not up-regulated as would ordinarily be the case for a responder. In such a case, statistical algorithms can be applied to determine the probability that the patient will respond or not respond to the drug. Statistical algorithms suitable for this purpose are well known and are available.

Articles of this invention are representations of the gene expression profiles useful for treating, diagnosing, prognosticating, staging, and otherwise assessing diseases that are reduced to a medium that can be automatically read such as computer readable media (magnetic, optical, and the like). The articles can also include instructions for assessing the gene expression profiles in such media. For example, the articles may comprise a CD ROM having computer instructions for comparing gene expression profiles of the portfolios of genes described above. The articles may also have gene expression profiles digitally recorded therein so that they may be compared with gene expression data from patient samples. Alternatively, the profiles can be recorded in different representational format. A graphical recordation is one such format. Fig. 1 shows an example of the graphical display of such a recordation. Clustering algorithms such as those incorporated in "OMNIVIZ" and "TREE VIEW" computer programs mentioned above can best assist in the visualization of such data.

Additional articles according to the invention are nucleic acid arrays (e.g. cDNA or oligonucleotide arrays), as described above, configured to discern the gene expression profiles ofthe invention.

Using clustering analysis (including the algorithms mentioned above) one can compare the expression levels of patient samples to establish regulatory relationships among genes with a certain statistical confidence. A dynamic map was constructed based upon such expression data. Such a genetic network map is useful for drug discovery. For example, once basic genes of interest were identified, a list of potential up-stream regulatory genes was found using such a genetic network map. The genes so identified or their expression products were then analyzed for their use as drug targets. In some embodiments, the regulatory function ofthe particular genes identified was used to identify therapeutics for use in treating leukemia.

The regulation of transcription, RNA processing and RNA editing are all accomplished by proteins which are coded by their own genes. In addition, DNA sequences can exert long range control over the expression of other genes by positional effects. Therefore, the expression of genes is often regulated by the expression of other genes. Those regulatory genes are called upstream genes, relative to the regulated or downstream genes. In a simple regulatory pathway:
A++>B-->C++>D
where: A, B, C, D are genes
++ up-regulates
- down-regulates
Gene A is an up-stream gene of gene B and B is an up-stream gene of C. One of skill in the art would appreciate that the network is frequently looped and inter-connected. In some instances, the expression of a gene is regulated by its own product as either a positive or negative feedback.

Cluster analysis methods were used to group genes whose expression level is correlated. Methods for cluster analysis are described in detail in Harfigan (1975) Clustering Algorithms, NY, John Wile and Sons, Inc, and Everritt, (1980) Cluster Analysis 2nd. Ed. London Heineman Educational books, Ltd., incorporated herein for all purposed by reference. Path analysis was used to decompose relations among variables and for testing causal models for the genetic networks. Multiple primary targets of a drug in leukemic cells were identified as were drugs/drug classes useful in treating such cells. According to the current invention, drugs are any compounds of any degree of complexity that perturb a biological system.

The biological effect of a drug may be a consequence of drug-mediated changes in the rate of transcription or degradation of one or more species ofRNA, the rate or extent of translation or post-translational processing of one or more polypeptides, the rate or extent of the degradation of one or more proteins, the inhibition or stimulation of the action or activity of one or more proteins, and so forth. In addition to the FTIs that are preferred, the preferred drugs of this invention are those that modulate the MAPK/ERK signaling pathways, TGFβ, WNT or apoptotic pathways. These include, without limitation, tyrosine kinase inhibitors, MEK kinase inhibitors, P13K kinase inhibitors, MAP kinase inhibitors, apoptosis modulators and combinations thereof. Exemplary drugs that are most preferred among these are the "GLEEVEC" tyrosine kinase inhibitor ofNovartis, U-0126 MAP kinase inhibitor, PD-098059 MAP kinase inhibitor, SB-203580 MAP kinase inhibitor, and antisense, ribozyme, and DNAzyme Bcl-XL anti-apoptotics. Examples of other useful drugs include, without limitation, the calanolides of US Patent 6,306,897; the substituted bicyclics of US Patent 6,284,764; the indolines of US Patent 6,133,305; and the antisense oligonucleotides of US Patent 6,271,210.

As noted, the drugs of the instant invention can be therapeutics directed to gene therapy or antisense therapy. Oligonucleotides with sequences complementary to a mRNA sequence can be introduced into cells to block the translation of the mRNA, thus blocking the function of the gene encoding the mRNA. The use of oligonucleotides to block gene expression is described, for example, in, Strachan and Read, Human Molecular Genetics, 1996, incorporated herein by reference.

These antisense molecules may be DNA, stable derivatives ofDNA such as phosphorothioates or methylphosphonates, RNA, stable derivatives ofRNA such as 2'-*O-*alkylRNA, or other antisense oligonucleotide mimetics. Antisense molecules may be introduced into cells by microinjection, liposome encapsulation or by expression from vectors harboring the antisense sequence.

In the case of gene therapy, the gene of interest can be ligated into viral vectors that mediate transfer of the therapeutic DNA by infection of recipient host cells. Suitable viral vectors include retrovirus, adenovirus, adeno-associated virus, herpes virus, vaccinia virus, polio virus and the like. Alternatively, therapeutic DNA can be transferred into cells for gene therapy by non-viral techniques including receptor-mediated targeted DNA transfer using ligand-DNA conjugates or adenovirus-ligand-DNA conjugates, lipofection membrane fusion or direct microinjection. These procedures and variations thereof are suitable for *ex vivo* as well as *in vivo* gene therapy. Protocols for molecular methodology of gene therapy suitable for use with the gene is described in Gene Therapy Protocols, edited by Paul D. Robbins, Human press, Totawa NJ, 1996.

Pharmaceutically useful compositions comprising the drugs of this invention may be formulated according to known methods such as by the admixture of a pharmaceutically acceptable carrier. Examples of such carriers and methods of formulation may be found in Remington's Pharmaceutical Sciences. To form a pharmaceutically acceptable composition suitable for effective administration, such compositions will contain an effective amount of the drug. The effective amount of the drug may vary according to a variety of factors such as the individual's condition, weight, sex and age. Other factors include the mode of administration. The pharmaceutical compositions may be provided to the individual by a variety of routes such as subcutaneous, topical, oral and intramuscular.

The drugs of this invention include chemical derivatives ofthe base molecules of the drug. That is, they may contain additional chemical moieties that are not normally a part ofthe base molecule. Such moieties may improve the solubility, half-life, absorption, etc. of the base molecule. Alternatively the moieties may attenuate undesirable side effects of the base molecule or decrease the toxicity of the base molecule. Examples of such moieties are described in a variety of texts, such as Remington's Pharmaceutical Sciences.

Compounds identified according to the methods disclosed herein may be used alone at appropriate dosages defined by routine testing in order to obtain optimal inhibition or activity while minimizing any potential toxicity. In addition, co-administration or sequential administration of other agents may be desirable.

The drugs of this invention can be administered in a wide variety of therapeutic dosage forms in conventional vehicles for administration. For example, the drugs can be administered in such oral dosage forms as tablets, capsules (each including timed release and sustained release formulations), pills, powders, granules, elixirs, tinctures, solutions, suspensions, syrups and emulsions, or by injection. Likewise, they may also be administered in intravenous (both bolus and infusion), intraperitoneal, subcutaneous, topical with or without occlusion, or intramuscular form, all using forms well known to those of ordinary skill in the pharmaceutical arts. An effective but non-toxic amount of the compound desired can be employed as a modulating agent.

The daily dosage of the products may be varied over a wide range from 0.01 to 1,000 mg per patient, per day. For oral administration, the compositions are preferably provided in the form of scored or unscored tablets containing 0.01, 0.05, 0.1, 0.5, 1.0, 2.5, 5.0, 10.0, 15.0, 25.0, and 50.0 milligrams of the active ingredient for the symptomatic adjustment of the dosage to the patient to be treated. An effective amount of the drug is ordinarily supplied at a dosage level of from about 0.0001 mg/kg to about 100 mg/kg of body weight per day. The range is more particularly from about 0.001 mg/kg to 10 mg/kg of body weight per day. The dosages are adjusted when combined to achieve desired effects. On the other hand, dosages of these various agents may be independently optimized and combined to achieve a synergistic result wherein the pathology is reduced more than it would be if either agent were used alone.

Advantageously, compounds or modulators used in the present invention may be administered in a single daily dose, or the total daily dosage may be administered in divided doses of two, three or four times daily. Furthermore, compounds or modulators for the present invention can be administered in intranasal form via topical use of suitable intranasal vehicles, or via transdermal routes, using those forms of transdermal skin patches well known to those of ordinary skill in that art. To be administered in the form of a transdermal delivery system, the dosage administration will, of course, be continuous rather than intermittent throughout the dosage regimen.

For combination treatment with more than one active agent, where the active agents are in separate dosage formulations, the active agents can be administered concurrently, or they each can be administered at separately staggered times.

The dosage regimen utilizing the compounds or modulators in the present invention is selected in accordance with a variety of factors including type, species, age, weight, sex and medical condition ofthe patient; the severity of the condition to be treated; the route of administration; the renal and hepatic function ofthe patient; and the particular drug employed. A physician or veterinarian of ordinary skill can readily determine and prescribe the effective amount ofthe drug required to prevent, counter or arrest the progress of the condition. Optimal precision in achieving concentrations of drug within the range that yields efficacy without toxicity requires a regimen based on the kinetics of the drug's availability to target sites. This involves a consideration of the distribution, equilibrium, and elimination of a drug.

The drugs of this invention can form the active ingredient, and are typically administered in admixture with suitable pharmaceutical diluents, excipients or carriers (collectively referred to herein as "carrier" materials) suitably selected with respect to the intended form of administration, that is, oral tablets, capsules, elixirs, syrups and the like, and consistent with conventional pharmaceutical practices.

For instance, for oral administration in the form of a tablet or capsule, the active drug component can be combined with an oral, non-toxic pharmaceutically acceptable inert carrier such as ethanol, glycerol, water and the like. Moreover, when desired or necessary, suitable binders, lubricants, disintegrating agents and coloring agents can also be incorporated into the mixture. Suitable binders include, without limitation, starch, gelatin, natural sugars such as glucose or beta-lactose, corn sweeteners, natural and synthetic gums such as acacia, tragacanth or sodium alginate, carboxymethylcellulose, polyethylene glycol, waxes and the like. Lubricants used in these dosage forms include, without limitation, sodium oleate, sodium stearate, magnesium stearate, sodium benzoate, sodium acetate, sodium chloride and the like. Disintegrators include, without limitation, starch, methyl cellulose, agar, bentonite, xanthan gum and the like.

For liquid forms the active drug component can be combined in suitably flavored suspending or dispersing agents such as the synthetic and natural gums, for example, tragacanth, acacia, methyl-cellulose and the like. Other dispersing agents that may be employed include glycerin and the like. For parenteral administration, sterile suspensions and solutions are desired. Isotonic preparations, which generally contain suitable preservatives, are employed when intravenous administration is desired.

The drugs in the present invention can also be administered in the form of liposome delivery systems, such as small unilamellar vesicles, large unilamellar vesicles and multilamellar vesicles. Liposomes can be formed from a variety of phospholipids, such as cholesterol, stearylamine or phosphatidylcholines.

Drugs in the present invention may also be delivered by the use of monoclonal antibodies as individual carriers to which the compound molecules are coupled. The drugs in the present invention may also be coupled with soluble polymers as targetable drug carriers. Such polymers can include polyvinyl-pyrrolidone, pyran copolymer, polyhydroxypropylmethacryl-amidephenol, polyhydroxy-ethylaspartamidephenol, or polyethyl-eneoxidepolylysine substituted with palmitoyl residues. Furthermore, the drugs in the present invention may be coupled to a class of biodegradable polymers useful in achieving controlled release of a drug, for example, polylactic acid, polyepsilon caprolactone, polyhydroxy butyric acid, polyorthoesters, polyacetals, polydihydro-pyrans, polycyanoacrylates and cross-linked or amphipathic block copolymers ofhydrogels.

For oral administration, the drugs may be administered in capsule, tablet, or bolus form or alternatively they can be mixed with feed. The capsules, tablets, and boluses are comprised of the active ingredient in combination with an appropriate carrier vehicle such as starch, talc, magnesium stearate, or di-calcium phosphate. These unit dosage forms are prepared by intimately mixing the active ingredient with suitable finely-powdered inert ingredients including diluents, fillers, disintegrating agents, and/or binders such that a uniform mixture is obtained. An inert ingredient is one that will not react with the drugs and which is non-toxic to the animal being treated. Suitable inert ingredients include starch, lactose, talc, magnesium stearate, vegetable gums and oils, and the like. These formulations may contain a widely variable amount of the active and inactive ingredients depending on numerous factors such as the size and type of the animal species to be treated and the type and severity of the infection. The active ingredient may also be administered by simply mixing the compound with the feedstuff or by applying the compound to the surface of the foodstuff.

The compounds or modulators may alternatively be administered parenterally via injection of a formulation consisting of the active ingredient dissolved in an inert liquid carrier. Injection may be either intramuscular, intraruminal, intratracheal, or subcutaneous. The injectable formulation consists of the active ingredient mixed with an appropriate inert liquid carrier. Acceptable liquid carriers include the vegetable oils such as peanut oil, cotton seed oil, sesame oil and the like as well as organic solvents such as solketal, glycerol formal and the like. As an alternative, aqueous parenteral formulations may also be used. The vegetable oils are the preferred liquid carriers. The formulations are prepared by dissolving or suspending the active ingredient in the liquid carrier such that the final formulation contains from 0.005 to 10% by weight of the active ingredient.

The invention is further illustrated by the following nonlimiting examples.

### Example 1: Cell culture

The AML-like cell lines HL-60 (promyelocytic) and U-937 (promonocytic) were obtained from the ATCC. AML-193 (monocytic) and THP-1 (monocytic) cells were obtained from the RW Johnson Pharmaceutical Research Center, San Diego. Cells were grown in Roswell Park Memorial Institute medium (RPMI) with 20% Fetal Bovine Serum (FBS). AML-193 was also supplemented with granulocyte-macrophage colony-stimulating factor (GM-CSF) (10 ng/ml), insulin (0.005 mg/ml), and transferrin (0.005 mg/ml).

### Example 2: Toxic Dose Assay

The cells of Example 1 were inoculated into 6-well plates at an initial concentration of I x 10⁵ cells/ml. (B)-6-[amino(4-chlorophenyl)(1-methyl-1H-imidazol-5-yl)methyl]-4-(3-chlorophenyl)-1-methyl-2(1H)-quinolinone) was added at concentrations ranging form 0.5 to 500 nM in 3 µl of DMSO directly to the culture medium. Control cells from Example 1 were grown in medium alone or in medium supplemented with vehicle (0.1% DMSO). Cell numbers were counted at days four and seven in a hemocytometer and cell viability was determined by trypan blue exclusion assay. The IC₅₀ was defined as the dose at which the number of viable cells in the treated sample was 50% of that in the control at day seven. Calculations were made based on duplicate runs of the experiment. The IC₅₀ of the four cell lines was calculated after seven days of treatment with the FTI. AML-193 had an IC₅₀ of 134 nM, HL-60 had an IC₅₀ of 24 nM, TBP-1 had an IC₅₀ of 19 nM, and U-937 had an IC₅₀ of 44 nM. This indicated that the four AML-like cell lines were sensitive to FTI treatement.

### Example 3: Time Course Assay

Duplicate cultures of the cells of Example 1 were inoculated into 6-well plates at an initial concentration of 1 x 10⁵ cells/ml. (B)-6-[amino(4-chlorophenyl)(1-methyl-1H-imidazol-5-yl)methyl]-4-(3-chlorophenyl)-1-methyl-2(1H)-quinolinone) was supplemented at a concentration of 100 nM in 3 µl of DMSO directly to the culture medium. The concentration of 100 nM was chosen for the subsequent time course experiments to normalize the treatment protocol based, in part, on the results of Example 2. Duplicate control cultures were grown in medium containing 0.1% DMSO. Duplicate cultures were harvested daily for a total of six days. Cells were counted, assayed for viability, and total RNA isolated according to the manufacturer's protocol (Qiagen RNeasy). The analysis showed that cells from different cell lines were effected at different times. RNA was treated with DNase1 (Qiagen DNase1 kit) to remove any residual genomic DNA. Linear amplification of RNA was conducted according to the procedure described in US Patent 5,545,522 to Van Gelder et. al. Aliquots of 5 µg of aRNA were then prepared for hybridization to cDNA arrays.

### Example 4: Bone Marrow Processing

Bone marrow aspirates were obtained from two patients diagnosed with AML who had been treated with FTI. These AML patients were administered 600 mg (B)-6-[amino(4-chlorophenyl)(1-methyl-1H-imidazol-5-yl)methyl]-4-(3-chlorophenyl)-1-methyl-2(1H)-quinolinone) twice daily over a 21 day period. Bone marrow aspirates were taken at baseline and once a week for the three weeks oftreatment. One of these patients did not respond (RH) while the other responded (BS) to the FTI. Response was determined as a reduction of more than 50% of blast cells in bone marrow aspirates. The aspirates were diluted to 15 ml with PBS and Ficoll-density centrifuged. White blood cells were washed twice with PBS, resuspended in FBS with 10% DMSO and immediately frozen at -80°C. Cells were cryogenically preserved to maintain cell viability. Samples were thawed at 37°C and 10X volume ofRPMI with 20% FBS was added drop-wise over a period of 5 min. Cells were centrifuged at 1600 rpm for 10 min and resuspended in 10 ml PBS with 2 mM EDTA and 0.5% BSA. Samples were then passed through a 70 µM filter to remove any cell clumps. Cell viability was determined by Trypan Blue assay. If sample viability was less than 50% a Miltenyi Dead Cell Removal Kit was employed to enrich for the live cell fraction. 2x10⁵ viable cells were then double labeled with CD33-FITC and CD34-PE antibodies (Pharminigen) and FACS analysis was performed. Post (B)-6-[amino(4-chlorophenyl)(1-methyl-1H-imidazol-5-yl)methyl]-4-(3-chlorophenyl)-1-methyl-2(1 H)-quinolinone)-treated bone marrow samples were enriched for leukemic cells by magnetic bead cell separation using either CD33 or CD34 antibodies (Miltenyi). The extracted cells had RNA extracted as described in Example 3.

### Example 5: Probe Preparation

RNA samples obtained in Examples 3 and 4 were prepared for hybridization to cDNA microarrays according to the following procedure. One to two rounds of linear amplification was performed on total RNA depending on the amount of starting material. Initially, 1-10 µg total RNA was reverse transcribed using the Superscript cDNA transcription kit (Gibco BRL). Ten µl total RNA was first mixed with 1 µl of 0.5 mg/ml T7-oligodT primer, incubated at 70°C for 10 min, and then chilled on ice. Next, 8 µl of 5X first-strand reaction buffer, 0.1M DTT, 10 mM dNTPs, and 1 µl Rnase Block were added, and the solution incubated at 42°C for 5 min. One µl Superscript II was then added and the reaction was incubated at 42°C for 2 hr. The reaction was heat deactivted at 70°C for 10 min and 1 µl was removed for PCR. Next, 92 µl Rnase-free water, 30 µl 5X second-strand reaction buffer, 3 µl 10mM dNTP, 4 µl DNA polymerase 1, 1 µl *E*. *coli* Rnase H, 1 µl *E*. *coli* DNA ligase were added and the mixture incubated at 16°C for 2 hr. cDNA was linear amplified using the Ampliscribe T7-transcription kit (Epicenter). If required, a second round of RNA amplification was performed by the random hexamer approach. Fluorescently labeled cDNA probes were synthesized by priming aRNA with random hexamers and including Cy3-dCTP in the nucleotide mix. Reactions were purified using a QIAquick PCR purification kit (Qiagen), the volumes of probe normalized using relative fluoresence (Cytofluor), and resuspended in 50 µl of Version 2 hybridization buffer (Amersham Pharmacia Biotech, Pistcataway, NJ) with 50% formamide and human Cot1 DNA (Life Technologies).

### Example 6: Array Hybridization and Analysis

The arrays contained 7452 cDNAs from the IMAGE consortium (Integrated Molecular Analysis of Genome and their Expression: Research Genetics, Huntsville, AL) and Incyte libraries. Micro-arrays were generated as follows and probes hybridized as described in Example 5. cDNAs were printed on amino silane-coated slides (Coming) with a Generation III Micro-array Spotter (Molecular Dynamics). The cDNAs were PCR amplified, purified (Qiagen PCR purification kit), and mixed 1:1 with 10 M NaSCN printing buffer. Prior to hybridization micro-arrays were incubated in isopropanol at room temperature for 10 min. The probes were incubated at 95°C for 2 min, at room temperature for 5 min, and then applied to three replicate slides. Cover slips were sealed onto the slides with DPX (Fluka) and incubated at 42°C overnight. Slides were then washed at 55°C for 5min in 1X SSC/0.2% SDS and 0.1X SSC/0.2% SDS, dipped in 0.1X SSC and dried before being scanned by a GenIII Array Scanner (Molecular Dynamics). The fluorescence intensity for each spot was analyzed with AUTOGENE software (Biodiscovery, Los Angeles).

The intensity level of each micro-array was normalized so that the 75^{th} percentile of the expression levels was equal across micro-arrays. Clones displaying a coefficient of variance (CV) greater than 50% of the mean were excluded from the analysis. Since background intensity was a maximum of 32 units for all experiments a threshold of 32 was assigned to all clones exhibiting an expression level lower than this. A ratio matrix was then generated based on pair-wise analysis of treated and control samples and Hierarchical clustering was performed using an euclidean metric and average linkage (Omniviz Pro™).

Each sample was hybridized to three identical arrays and the mean signal intensity was compared by scatter-plot analysis. High correlation coefficients were also observed when control samples were compared to treated samples from the same day. This indicated there were no gross changes in gene expression due to treatment with (B)-6-[amino(4-chlorophenyl)(1-methyl-1H-imidazol-5-yl)methyl]-4-(3-chlorophenyl)-1-methyl-2(1H-quinolinone). In addition, the variation between control samples from different days was examined. Cells were mock-treated and RNA was isolated after 1, 2, 3, 4, 5, and 6 days. Following labeling and hybridization the mean intensity of duplicate samples and the coefficient of variance (CV) of each clone (3 spots per clone) were calculated. Data points which displayed a CV of more than 50% were discarded from further analysis.

### Example 7: Differential Gene Expression in Treated Cell Line Samples

Hierarchical clustering was performed on the time-course data sets using the OmniViz Pro™ software (Battelle). Initially, fold-changes of 1.5, 1.7, and 2.0 were used as filters for the treated versus control intensity ratios for each day of the time-course. The gene expression profiles of genes modulated beyond these thresholds were analyzed to examine those genes that were commonly modulated between the three data sets and identify gene clusters that shared similar expression profiles. Results are shown in Tables 1-3 below.

Genes analyzed according to this invention are identified in the tables below by reference to Gene ID Numbers (internally generated) and accession numbers in the Genbank database where such genes have been entered in the Genbank database. The attached sequence listing, incorporated herein by reference, shows sequences corresponding to the Gene ID Number and are named with those Gene ID Numbers. In some cases, the listed sequences are to full length nucleic acid sequences that code for the production of a protein or peptide. One skilled in the art will recognize that identification of full-length sequences is not necessary from an analytical point of view. That is, portions of the sequences or ESTs can be selected according to well-known principles for which probes can be designed to assess gene expression for the corresponding gene. Further, it should be noted that some of the sequences in the listing contain the letter "N" in place of a nucleotide designation. One skilled in the art will recognize that the "N" indicates placement of any nucleotide in that portion of the sequence.

### Example 8: Identification of Gene Networks

Genes that were regulated in two or more cell lines by at least 1.5-fold in drug treated cell lines (Table 1) were identified as described above. The list of these genes was employed to identify major gene pathways that were being modulated by the most preferred FTI, (B)-6-[amino(4-chlorophenyl)(1-methyl-1H-imidazol-5-yl)methyl]-4-(3-chlorophenyl)-1-methyl-2(1H)-quinolinone). If clones did not perfectly match a known gene annotations from the best BLAST result of the clone sequence were used. Since the level of regulation of these genes varied over the course of treatment of the cell lines, the gene expression profiles from the primary AML tissue that responded to this FTI were determined.

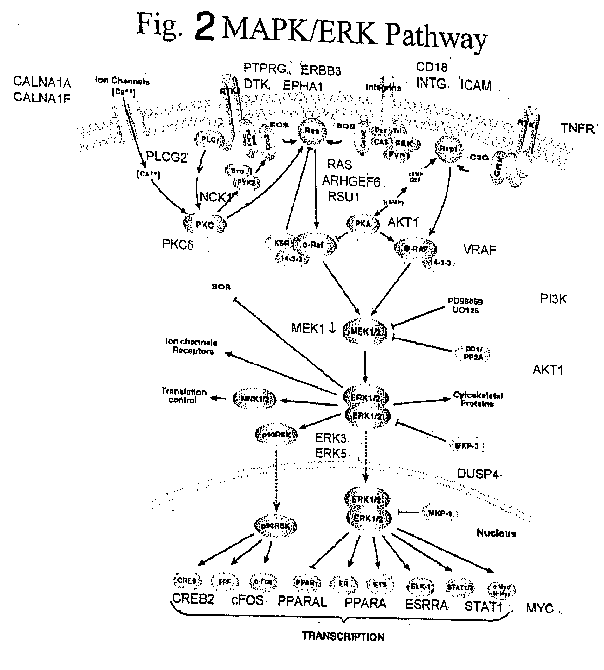

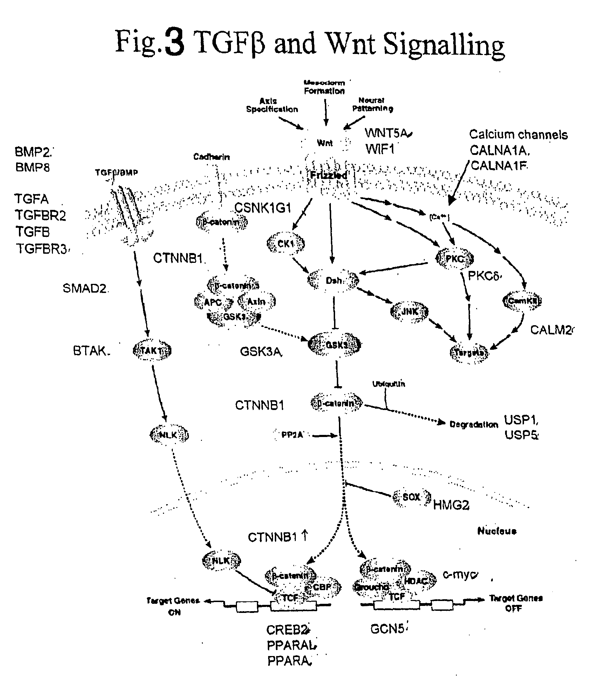

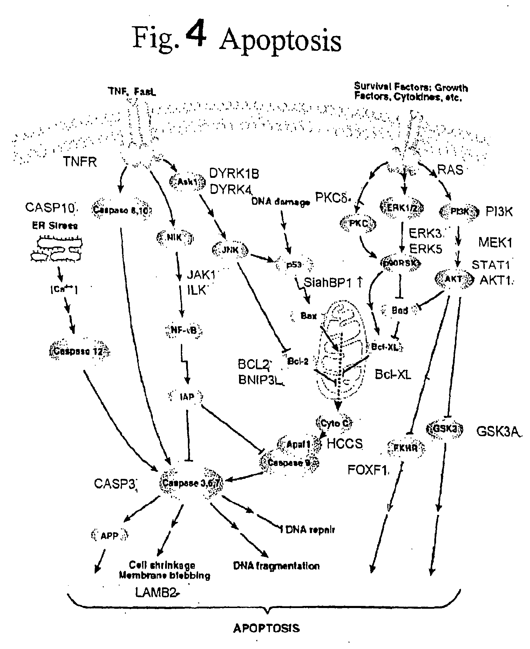

It was found that many genes in the MAPK/ERK (Fig. 2) signaling pathways were being down-regulated and that genes in the TGFβ (Fig. 3) and WNT (Fig. 3) signaling pathways were generally up-regulated, while apoptotic pathways were also activated (Fig.4). This allowed the identification of other gene targets sensitive to treatment with known or novel drug compounds. For example, beneficial treatment can result from FTIs used in conjunction with tyrosine kinase, MEK kinase, PI3K and/or MAP kinase inhibitors to obtain a more potent effect. In addition, given the finding that apoptotic pathways are activated in FTI treated cells, drugs that modulate apoptosis could be expected to have beneficial effect when employed in conjunction with an FTI. Examples of these types of compounds include tyrosine kinase inhibitors (eg Gleevec, Novartis), MAP kinase inhibitors (eg U-0126, PD-098059, SB-203580), and inhibitors of anti-apoptotic genes such as Bel-XL (eg antisense, ribozymes, DNAzymes).

**Table 1: Genes (by Genbank Accession Number) modulated at least 1.5 fold in 2 or more of the cell lines over the 6 day time course.**

| Gene ID | Accession No. |
|---|---|
| 3434105F7 | AB026898 |
| 3881595H1 | AC000134 |
| Al939481 | AC005155 |
| AA961061 | AC005670 |
| 3918104H1 | AC006023 |
| A1025519 | AC008427 |
| 5543360F8 | AC009220 |
| AA744682 | AC009289 |
| AA932129 | AC009756 |
| AI148008 | AC011473 |
| Y00052 | AC013722 |
| R52476 | AC021078 |
| AA864819 | AC022087 |
| AI815593 | AC022150 |
| AI141943 | AC026448 |
| AI300541 | AC073585 |
| 2515486H1 | AF161372 |
| 1731618H1 | AJ003147 |
| BE222911 | AJ400879 |
| R13802 | AK022901 |
| A1656222 | AL021155 |
| Al553823 | AL022313 |
| AA010251 | AL034397 |
| AA237071 | AL035420 |
| 2445101F6 | AL049824 |
| AI638342 | AL122004 |
| AA779424 | AL136980 |
| H81171 | AL137073 |
| R77754 | AL137790 |
| A1209040 | AL139082 |
| 6421806H1 | AL139396 |
| H61066 | AL161787 |
| R59209 | AL355136 |
| AA486141 | AL355352 |
| A1339252 | AP001630 |
| AW023438 | BC009732 |
| 914979H1 | BC013834 |
| AA455969 | D00015 |
| T64335 | D00017 |
| X02308 | D00596 |
| X67098 | D00596 |
| X01023 | D10493 |
| D10493 | D10493 |
| Y00396 | D10493 |
| X69292 | D10667 |
| AA736561 | D11094 |
| S68252 | D11139 |
| M25315 | D12592 |
| Al186110 | D13118 |
| H84153 | D13639 |
| AA598561 | D14043 |
| D14695 | D14695 |
| 2206642T6 | D16889 |
| A1878943 | D17004 |
| U38846 | D17080 |
| J03801 | D21235 |
| AI762926 | D23660 |
| D25215 | D25215 |
| U41078 | D26512 |
| AA485961 | D30648 |
| AA456408 | D38441 |
| A1311090 | D38616 |
| 1869911H1 | D42084 |
| D43950 | D43950 |
| AW006368 | D44467 |
| U29092 | D45050 |
| D45887 | D45887 |
| W68193 | D49489 |
| 3633286H1 | D63874 |
| N76967 | D66904 |
| AA985407 | D82326 |
| A1962797 | D83260 |
| M59829 | D85730 |
| 3107995H1 | D86322 |
| AA279906 | D86550 |
| Al016874 | D86586 |
| 556963H1 | D86955 |
| AI810687 | D86997 |
| AF045581 | D87462 |
| U41070 | D89078 |
| D90209 | D90209 |
| AA812265 | D90767 |
| 2135769H1 | J02763 |
| 1876511H1 | J03072 |
| J04088 | J04088 |
| A1590075 | J04973 |
| H30357 | J05451 |
| K00558 | K03460 |
| L10413 | L00634 |
| L01087 | L01087 |
| AI027898 | L02426 |
| L06139 | L06139 |
| U28936 | L07914 |
| M86400 | L07955 |
| AA428915 | L08634 |
| AA464627 | L09604 |
| M94859 | L10284 |
| R78541 | L10717 |
| L15189 | L11066 |
| L11284 | L11284 |
| T87908 | L12387 |
| T80827 | L13802 |
| L08044 | L15203 |
| A1278029 | L16862 |
| M60278 | L17032 |
| M60278 | L17032 |
| M65128 | L18980 |
| W49672 | L20861 |
| L22005 | L22005 |
| Al380522 | L23822 |
| AA988469 | L25591 |
| L26336 | L26336 |
| Al074664 | L32866 |
| M63175 | L35233 |
| 2470939H1 | L35848 |
| AA190648 | L36055 |
| A1243166 | L38716 |
| L39833 | L39833 |
| M97347 | L41415 |
| 2005142H1 | L42324 |
| M11723 | L43615 |
| U04045 | L47574 |
| AA284067 | L76938 |
| M13142 | M13142 |
| W68291 | M15395 |
| 5560880H1 | M19483 |
| 3171275H1 | M20199 |
| M22489 | M22489 |
| AA070627 | M22810 |
| H39560 | M24736 |
| M25897 | M25897 |
| M27492 | M27492 |
| AA570304 | M29366 |
| 205581R6 | M29696 |
| M84739 | M32294 |
| M84739 | M32294 |
| M35857 | M32315 |
| 3801801 H1 | M33680 |
| M63904 | M63904 |
| A1091579 | M63971 |
| M69215 | M69215 |
| M74782 | M74782 |
| M80254 | M80254 |
| M80647 | M80647 |
| M84526 | M84526 |
| 6805226H1 | M84526 |
| S93414 | M86553 |
| M91556 | M91556 |
| M95678 | M95678 |
| 2017923F6 | M96326 |
| H73054 | NM_000551 |
| AA488324 | NM_001211 |
| N73242 | NM_001274 |
| AF013611 | NM_001335 |
| AW163686 | NM_001524 |
| AI921879 | NM_002287 |
| AI652785 | NM_002333 |
| AI423526 | NM_003332 |
| 3028719F6 | NM_003600 |
| AB010882 | NM_003601 |
| AF030424 | NM_003642 |
| AW194791 | NM_003668 |
| AF075599 | NM_003969 |
| AF031141 | NM_004223 |
| 2676931T6 | NM_004412 |
| AF053304 | NM_004725 |
| AF119815 | NM_004885 |
| AI816398 | NM_004888 |
| 2185556H1 | NM_004917 |
| 3357511H1 | NM_004917 |
| AA047585 | NM_005109 |
| AA448972 | NM_005592 |
| AW629084 | NM_005817 |
| AJ001015 | NM_005854 |
| AJ001016 | NM_005856 |
| U85055 | NM_006480 |
| 3618886F6 | NM_006536 |
| AA906714 | NM_006573 |
| AF060153 | NM_007037 |
| 1467864F6 | NM_012089 |
| AI097079 | NM_012100 |
| AF204944 | NM_012105 |
| W44673 | NM_012428 |
| Al522316 | NM_013386 |
| Al700673 | NM_013439 |
| AA057781 | NM_014172 |
| Al299795 | NM_014251 |
| 1931159F6 | NM_014397 |
| AW630208 | NM_014413 |
| 2821685T6 | NM_014967 |
| 1539060H1 | NM_015343 |
| AI762738 | NM_015449 |
| AA401397 | NM_015596 |
| AW243944 | NM_015596 |
| A1436551 | NM_016141 |
| AI651159 | NM_016440 |
| AA527334 | NM_016625 |
| g922698 | NM_017555 |
| 1693028H1 | NM_017636 |
| 002783H1 | NM_017860 |
| AI368583 | NM_017874 |
| AW170305 | NM_017903 |
| H62827 | NM_018321 |
| M78706 | NM_019020 |
| BE048230 | NM_020216 |
| A1214466 | NM_020334 |
| AA452802 | NM_021196 |
| AI808824 | NM_022082 |
| W77977 | NM_022336 |
| AA535015 | NM_022570 |
| AA861140 | NM_022829 |
| AW078834 | NM_023080 |
| 5122087H1 | NM_024056 |
| AF017182 | NM_024101 |
| AA449040 | NM_024116 |
| 2792728F6 | NM_024902 |
| BE218593 | NM_025230 |
| AA669885 | NM_030763 |
| AI339565 | NM_030908 |
| AF038564 | NM_031483 |
| A1126706 | NM_032038 |
| 1961084H1 | NM_032188 |
| 4609810F6 | NM_032554 |
| AA745592 | NM_032844 |
| AW612141 | NM_033050 |
| Al740538 | NM_033280 |
| U58522 | S51016 |
| M57703 | S63697 |
| AA873257 | S73591 |
| AA521213 | S77359 |
| N77754 | S79873 |
| AA984230 | S80071 |
| R79935 | S81439 |
| T71391 | T71391 |
| AA598776 | U05340 |
| U11053 | U11053 |
| T55353 | U12597 |
| AA456616 | U14970 |
| U18300 | U18300 |
| AF017306 | U20657 |
| AA459663 | U25182 |
| U27699 | U27699 |
| A1884916 | U29171 |
| U29171 | U29171 |
| 1671033F6 | U33429 |
| AA017042 | U40989 |
| AI126520 | U48405 |
| U48807 | U48807 |
| U49395 | U49395 |
| AA186542 | U50078 |
| U51586 | U51586 |
| AW150605 | U54558 |
| AA455800 | U55206 |
| AF029777 | U57316 |
| 119355 | U58913 |
| 319095H1 | U58913 |
| AF027964 | U59911 |
| U60519 | U60519 |
| AI371158 | U65378 |
| U69883 | U69883 |
| H30148 | U73641 |
| R80718 | U75283 |
| U77180 | U77180 |
| U78180 | U78180 |
| U83115 | U83115 |
| AA938905 | U86218 |
| A1401546 | U88844 |
| 2526581H1 | U90904 |
| AA773114 | U95740 |
| AA176596 | U96781 |
| X13274 | V00543 |
| 116618 | V00595 |
| R91899 | X00226 |
| AW519155 | X00318 |
| X87344 | X00369 |
| X02910 | X01394 |
| M15840 | X02532 |
| AA401046 | X02592 |
| X02812 | X02812 |
| X87344 | X03066 |
| X03084 | X03084 |
| M10901 | X03225 |
| X03225 | X03225 |
| R81823 | X03742 |
| X04011 | X04011 |
| K02400 | X04076 |
| X07036 | X04408 |
| X07036 | X04408 |
| Y00816 | X05309 |
| N20475 | X05344 |
| M11233 | X05344 |
| X02544 | X05784 |
| X52192 | X06292 |
| R33755 | X06547 |
| X06989 | X06989 |
| X07979 | X07979 |
| X14723 | X08004 |
| M20566 | X12830 |
| M20566 | X12830 |
| X13197 | X13197 |
| M21304 | X13709 |
| X00351 | X13839 |
| X60236 | X14008 |
| H57180 | X14034 |
| M33011 | X14758 |
| X14768 | X14768 |
| M31625 | X14768 |
| M31626 | X14768 |
| M30816 | X14768 |
| X52882 | X14983 |
| AA598758 | X15187 |
| X15606 | X15606 |
| K03515 | X16539 |
| AA868186 | X17093 |
| X51416 | X51416 |
| M23699 | X51439 |
| AA455222 | X51675 |
| X51757 | X51757 |
| X52195 | X52195 |
| U06434 | X53682 |
| J03198 | X54048 |
| M32304 | X54533 |
| AA487812 | X56134 |
| X56134 | X56134 |
| M16985 | X56257 |
| N31660 | X56257 |
| H27379 | X57198 |
| X57522 | X57522 |
| X57830 | X57830 |
| M57765 | X58377 |
| X58528 | X58528 |
| M81182 | X58528 |
| S60489 | X60111 |
| AI739095 | X61157 |
| R76314 | X61587 |
| M83665 | X62534 |
| X65921 | X65921 |
| M37722 | X66945 |
| AA453816 | X69516 |
| AA187162 | X69654 |
| X69819 | X69711 |
| X70070 | X70070 |
| X70697 | X70697 |
| S40706 | X71427 |
| T53775 | X71874 |
| X71877 | X71877 |
| X73458 | X73458 |
| X74801 | X74801 |
| AA454585 | X75755 |
| R43734 | X76939 |
| A1189206 | X77303 |
| 2496221H1 | X77303 |
| H17504 | X80692 |
| R26434 | X80910 |
| X83688 | X83688 |
| U24231 | X84709 |
| 3576337H1 | X85030 |
| X87212 | X87212 |
| T56477 | X87212 |
| AA464034 | X89401 |
| X89576 | X89576 |
| AA187458 | X92396 |
| M15887 | X94565 |
| X94991 | X94991 |
| X96427 | X96427 |
| X97058 | X97058 |
| AA425120 | X98262 |
| 3283686H1 | XM_005825 |
| AW027188 | XM_005958 |
| 1525902F6 | XM_006646 |
| R48796 | XM_008099 |
| AK000599 | XM_027140 |
| AA044653 | XM_031608 |
| AW665954 | XM_035574 |
| H86407 | XM_037453 |
| R00285 | XM_038150 |
| X57447 | XM_039395 |
| 778372H1 | XM_040459 |
| R82530 | XM_041024 |
| A1580830 | XM_042041 |
| 3097063H1 | XM_044784 |
| 3038910H1 | XM_046691 |
| H53340 | XM_048213 |
| 1654210F6 | XM_048530 |
| L42856 | XM_054964 |
| 2707270F6 | XM_056259 |
| M23468 | Y00062 |
| Y00649 | Y00649 |
| Y00757 | Y00757 |
| M17017 | Y00787 |
| M28130 | Y00787 |
| L02932 | Y07619 |
| Y10256 | Y10256 |
| AA454813 | Y12395 |
| AA149850 | Y12670 |
| 704183H1 | Y13710 |
| 059476H1 | Y13829 |
| Y13834 | Y13834 |
| L11016 | Y14768 |
| 3141315H1 | Y17803 |
| AI341167 | Y18391 |
| AI707852 | Z12962 |
| U51278 | Z23115 |
| A1686653 | Z26876 |
| AA043102 | Z35102 |
| AA136533 | Z35481 |
| U49083 | Z49148 |
| R70234 | Z56852 |
| 257274R6 | Z58168 |
| U62027 | Z73157 |
| 391237F1 | Z73157 |
| 510997F1 | Z73157 |
| Al808621 | Z82214 |
| AA460801 | Z98749 |
| 2673259F6 | Z98752 |
| R22977 | Z98946 |
| L03380 | Z99995 |
| AA425422 | |
| AA460392 | |
| AA508510 | |
| AA552028 | |
| AA576785 | |
| AA663307 | |
| AI015248 | |
| A1024468 | |
| AI086865 | |
| A1190605 | |
| A1203269 | |
| A1264420 | |
| A1333013 | |
| AI435052 | |
| AI671268 | |
| A1796718 | |
| AI990816 | |
| AW027164 | |
| AW167520 | |
| H24679 | |
| H66015 | |
| H91370 | |
| W07570 | |
| 1274737F6 | |
| 195337H1 | |
| 2398102H1 | |
| 2531082H1 | |
| 264639H1 | |
| 2794246F6 | |
| 3290073H1 | |
| 335737H1 | |
| 4539942F8 | |
| 6300669H1 | |
| 938765H1 | |

**Table 2: Genes (by GenBank Accession Number) modulated at least 1.7 fold in primary AML Sample.**

| Gene ID | Accession No. |
|---|---|
| T94331 | AB026898 |
| 3881595H1 | AC000134 |
| 1329021F6 | AC002073 |
| 2858615H1 | AC002325 |
| Al791539 | AC002428 |
| Al821217 | AC004258 |
| AA774798 | AC004671 |
| H29666 | AC004845 |
| T95173 | AC005071 |
| AA814523 | AC005160 |
| 5905620T9 | AC005212 |
| 5986963H1 | AC005280 |
| 5825251H1 | AC005306 |
| N36113 | AC005670 |
| 1700438H1 | AC005682 |
| R48756 | AC005757 |
| 5538589F6 | AC005839 |
| 3918104H1 | AC006023 |
| AA443719 | AC007240 |
| Al867297 | AC007883 |
| R63067 | AC008073 |
| AW022174 | AC008382 |
| 5537789F6 | AC008525 |
| H00249 | AC008733 |
| 1436240H1 | AC008860 |
| 2668191F6 | AC008949 |
| 5543360F8 | AC009220 |
| AA737674 | AC009892 |
| 5104579H1 | AC009892 |
| 3335217F6 | AC010311 |
| BE326380 | AC010521 |
| 3746214H1 | AC011088 |
| 1671315F6 | AC011500 |
| H60969 | AC012351 |
| AA926944 | AC012377 |
| 3100089H1 | AC012454 |
| Y00052 | AC013722 |
| R52476 | AC021078 |
| N45149 | AC021106 |
| AI742120 | AC022137 |
| 4177228F6 | AC022224 |
| 2676312H1 | AC022415 |
| H73476 | AC022740 |
| AA652121 | AC046170 |
| Al308320 | AC046170 |
| 1956982H1 | AC046170 |
| 1428534F6 | AC051619 |
| 2914934H1 | AC055707 |
| AA621370 | AC064807 |
| 5514511R6 | AC073333 |
| AI698737 | AC074331 |
| 3406131H1 | AC079118 |
| N20072 | AC096579 |
| 5911413H1 | AC096667 |
| A1458182 | AF042782 |
| 2291436H1 | AF074333 |
| W32067 | AF136745 |
| 6755801J1 | AF157623 |
| 2397317F6 | AF235100 |
| R53190 | AF384819 |
| 1731618H1 | AJ003147 |
| 2959801H1 | AJ003147 |
| 3123232H1 | AJ003147 |
| 2760110H1 | AJ006345 |
| X64073 | AJ239325 |
| 3986782F7 | AJ249275 |
| Al366098 | AJ276674 |
| Al695385 | AJ289236 |
| BE222911 | AJ400879 |
| Al400473 | AK017738 |
| Al299633 | AK021499 |
| R13802 | AK022901 |
| 1489075H1 | AK025775 |
| AI656222 | AL021155 |
| W96144 | AL021155 |
| 2459540H1 | AL031282 |
| 3461693F6 | AL031588 |
| 4333034H1 | AL031726 |
| 3332309H1 | AL031728 |
| R61661 | AL032821 |
| U71321 | AL033519 |
| AA935151 | AL034374 |
| AA010251 | AL034397 |
| U43431 | AL035367 |
| AA237071 | AL035420 |
| AA609779 | AL049610 |
| AA167461 | AL049612 |
| 4228729H2 | AL049742 |
| 6712339H1 | AL049766 |
| A1051176 | AL049872 |
| 1747028H1 | AL078600 |
| 5164454H1 | AL109840 |
| 7007735H1 | AL117382 |
| AA526337 | AL121601 |
| AI638342 | AL122004 |
| 4835576H1 | AL122035 |
| W01596 | AL133243 |
| U64205 | AL133367 |
| 4820983H1 | AL135786 |
| 5594552H1 | AL136381 |
| H12102 | AL136979 |
| H81171 | AL137073 |
| AA151374 | AL137790 |
| AA578089 | AL138787 |
| AI209040 | AL139082 |
| 6421806H1 | AL139396 |
| H60498 | AL157776 |
| 3721604H1 | AL160271 |
| H61066 | AL161787 |
| 2798009H1 | AL162252 |
| 2225447F6 | AL162430 |
| A1885557 | AL162729 |
| 2918417F6 | AL163279 |
| AA489975 | AL355151 |
| U77456 | AL355794 |
| 5375277T9 | AL356266 |
| AI051860 | AL356489 |
| 4019605F6 | AL356489 |
| U29607 | AL356801 |
| AA861429 | AL359512 |
| AA767859 | AL359915 |
| 1362587H1 | AL391122 |
| R09122 | AL391194 |
| R93094 | AP000173 |
| AA954331 | AP000432 |
| R10535 | AP000555 |
| 5327443H1 | AP000936 |
| 1569726H1 | AP001347 |
| R92422 | AP001672 |
| 3422674H1 | AP002800 |
| AI310451 | AP002812 |
| 3568042H1 | AP003900 |
| AA455969 | D00015 |
| AF030575 | D00015 |
| T64335 | D00017 |
| D12614 | D00102 |
| X67098 | D00596 |
| R27585 | D00759 |
| AA465593 | D00762 |
| M80436 | D10202 |
| M80436 | D10202 |
| M80436 | D10202 |
| M80436 | D10202 |
| AA464600 | D10493 |
| AI147046 | D10653 |
| S68252 | D11139 |
| M25315 | D12592 |
| A1186110 | D13118 |
| S57708 | D13515 |
| D13626 | D13626 |
| AA682625 | D13641 |
| AA598561 | D14043 |
| D14695 | D14695 |
| D14825 | D14825 |
| 855326R1 | D16234 |
| L20046 | D16305 |
| V00496 | D17206 |
| AA629808 | D17554 |
| M57285 | D21214 |
| J03801 | D21235 |
| Al700360 | D21878 |
| D25216 | D25216 |
| U41078 | D26512 |
| AF245447 | D28468 |
| AF245447 | D28468 |
| AA070997 | D29012 |
| 2134847H1 | D30756 |
| AI147295 | D30756 |
| AA455067 | D31839 |
| AI311090 | D38616 |
| AW629690 | D42084 |
| 1869911H1 | D42084 |
| 5122374H1 | D43701 |
| D43950 | D43950 |
| D45887 | D45887 |
| W68193 | D49489 |
| X72498 | D50326 |
| L11667 | D63861 |
| D63874 | D63874 |
| 3633286H1 | D63874 |
| X61598 | D83174 |
| AA279906 | D86550 |
| AA729988 | D86550 |
| D86956 | D86956 |
| L36719 | D87116 |
| D89078 | D89078 |
| U41070 | D89078 |
| AI821897 | D89675 |
| D90209 | D90209 |
| 2135769H1 | J02763 |
| J03040 | J03040 |
| 1876511H1 | J03072 |
| J03258 | J03258 |
| J03571 | J03571 |
| J04111 | J04111 |
| A1125073 | J04132 |
| 1634342H1 | J04794 |
| H30357 | J05451 |
| K02054 | K02054 |
| X02415 | K02569 |
| K03000 | K03000 |
| H58873 | K03195 |
| Al791949 | K03474 |
| L10413 | L00634 |
| H22919 | L03558 |
| L04288 | L04288 |
| AA405769 | L05144 |
| H62473 | L07594 |
| L08177 | L08177 |
| L08177 | L08177 |
| AA234897 | L08895 |
| AA464627 | L09604 |
| M94859 | L10284 |
| R78541 | L10717 |
| L15189 | L11066 |
| M15400 | L11910 |
| L12168 | L12168 |
| L12350 | L12350 |
| L12350 | L12350 |
| T87908 | L12387 |
| L09600 | L13974 |
| M14221 | L16510 |
| M60278 | L17032 |
| M60278 | L17032 |
| M60278 | L17032 |
| M60278 | L17032 |
| 2745317H1 | L17411 |
| M65128 | L18980 |
| W49672 | L20861 |
| A1380522 | L23822 |
| AA988469 | L25591 |
| NM_001168 | L26245 |
| R20939 | L31848 |
| 2470939H1 | L35848 |
| AA442810 | L36034 |
| L36148 | L36148 |
| M11723 | L43615 |
| M14745 | M14745 |
| W68291 | M15395 |
| M16038 | M16038 |
| 339598H1 | M16038 |
| M17783 | M17783 |
| 3171275H1 | M20199 |
| 5189380H1 | M21121 |
| 4130807F7 | M22440 |
| M22612 | M22612 |
| AA070627 | M22810 |
| 1445982H1 | M23254 |
| M28638 | M24906 |
| R45525 | M28215 |
| A1051962 | M28983 |
| 736837R6 | M29696 |
| M29870 | M29870 |
| AI264247 | M30309 |
| 1512407F6 | M30310 |
| M30471 | M30471 |
| M30704 | M30703 |
| AW467649 | M31158 |
| M84739 | M32294 |
| M84739 | M32294 |
| U52165 | M32315 |
| M35857 | M32315 |
| 5077322H1 | M32315 |
| N72918 | M34175 |
| M63193 | M58602 |
| M59465 | M59465 |
| 2294719H1 | M60858 |
| 2992331H1 | M63005 |
| AA069596 | M63582 |
| M63904 | M63904 |
| AI091579 | M63971 |
| M74782 | M74782 |
| AA410680 | M77016 |
| M80647 | M80647 |
| M84526 | M84526 |
| S93414 | M86553 |
| AI310138 | M91463 |
| M95678 | M95678 |
| 2017923F6 | M96326 |
| R60624 | NM_000702 |
| AA488324 | NM_001211 |
| AA488341 | NM_001336 |
| AF006823 | NM_002246 |
| 1322305T6 | NM_002250 |
| A1921879 | NM_002287 |
| AW129770 | NM_002349 |
| AJ004977 | NM_002873 |
| Al423526 | NM_003332 |
| 4516963H1 | NM_003576 |
| 3028719F6 | NM_003600 |
| AB010882 | NM_003601 |
| AF030424 | NM_003642 |
| AF029899 | NM_003814 |
| AF055993 | NM_003864 |
| AI220935 | NM_004142 |
| AW665782 | NM_004142 |
| AI191941 | NM_004226 |
| 1392516T6 | NM_004621 |
| AA449579 | NM_004769 |
| 1810447H1 | NM_004917 |
| AA047585 | NM_005109 |
| 4181072F6 | NM_005468 |
| AA448972 | NM_005592 |
| AA742351 | NM_005739 |
| 3406436F6 | NM_005845 |
| AJ001015 | NM_005854 |
| 3118530H1 | NM_005880 |
| AA906714 | NM_006573 |
| Al016020 | NM_006672 |
| AW770551 | NM_006770 |
| AW009940 | NM_006871 |
| 864164H1 | NM_007194 |
| 1467864F6 | NM_012089 |
| AF204944 | NM_012105 |
| W23427 | NM_012115 |
| 3363678H2 | NM_012226 |
| AI652076 | NM_012243 |
| 346874T6 | NM_013308 |
| AI522316 | NM_013386 |
| AI338030 . | NM_013439 |
| AI700673 | NM_013439 |
| 4540025H1 | NM_014322 |
| W00842 | NM_014331 |
| AW511388 | NM_014358 |
| AW630208 | NM_014413 |
| H63640 | NM_014834 |
| AI743175 | NM_014959 |
| 2821685T6 | NM_014967 |
| W38474 | NM_015542 |
| AW243944 | N M_015596 |
| W07181 | NM_015701 |
| 2997457H1 | NM_015938 |
| AA631149 | NM_016205 |
| AA527334 | NM_016625 |
| 5543749F6 | NM_017414 |
| AW170305 | NM_017903 |
| AA160974 | NM_018155 |
| AA625433 | NM_018404 |
| AA074666 | NM_018834 |
| 767295H1 | NM_018983 |
| M78706 | NM_019020 |
| AF245447 | NM_020126 |
| AF245447 | NM_020126 |
| 4294821H1 | NM_020344 |
| 2490994H1 | NM_021624 |
| 3556218H1 | NM_021634 |
| 2435705R6 | NM_022048 |
| 3092423H1 | NM_022054 |
| W77977 | NM_022336 |
| AA429219 | NM_023930 |
| 1001514R6 | NM_024022 |
| A1031531 | NM_024083 |
| AA449040 | NM_024116 |
| 2803571H1 | NM 024586 |
| 1390130H1 | NM_024671 |
| 3241088H1 | NM_024850 |
| H96170 | NM_030779 |
| 1540906H1 | NM_030779 |
| Al824146 | NM_030811 |
| W90438 | NM_032127 |
| AA430653 | NM_032177 |
| 3495438F6 | NM_032294 |
| AW612141 | NM_033050 |
| AA417237 | NM_033225 |
| AI740538 | NM_033280 |
| M57703 | 563697 |
| 780099H1 | S63912 |
| AA714835 | S67156 |
| AA777347 | S76736 |
| AA521213 | S77359 |
| U39231 | S79852 |
| N77754 | S79873 |
| AA984230 | S80071 |
| U00672 | U00672 |
| U02478 | U02478 |
| AA019459 | U02680 |
| 3401107H1 | U03019 |
| A1580044 | U04816 |
| 3041874H1 | U07563 |
| 2457652H1 | U12465 |
| U39318 | U13175 |
| U13668 | U13666 |
| U13695 | U13695 |
| AA056652 | U14176 |
| AA456616 | U14970 |
| U18242 | U18242 |
| U18300 | U18300 |
| AA465444 | U18422 |
| U20537 | U20536 |
| U25128 | U25128 |
| U35237 | U26174 |
| AI884916 | U29171 |
| AA481076 | U31278 |
| NM_002411 | U33147 |
| 1671033F6 | U33429 |
| AA664389 | U35048 |
| 6313632H1 | U43030 |
| R09288 | U43522 |
| AA488645 | U47007 |
| U47077 | U47077 |
| 5801413H1 | U48449 |
| 2405358R6 | U48729 |
| AA186542 | U50078 |
| U51586 | U51586 |
| 1355140F1 | U51586 |
| AA455800 | U55206 |
| U56390 | U56390 |
| U83410 | U58088 |
| AA121261 | U58675 |
| AF027964 | U59911 |
| U60519 | U60519 |
| 2836805T6 | U62293 |
| U62433 | U62433 |
| 3188135H1 | U66673 |
| 3188135H1 | U66673 |
| 3188135H1 | U66673 |
| 3188135H1 | U66673 |
| 1360938T6 | U66679 |
| 809631T6 | U66684 |
| AA454652 | U67058 |
| Al214335 | U68755 |
| U69883 | U69883 |
| R98589 | U81375 |
| 5695322H1 | U82671 |
| AA745989 | U82979 |
| AA188256 | U83661 |
| 2526581H1 | U90904 |
| AA434064 | U95000 |
| AA773114 | U95740 |
| AA514978 | U96776 |
| Y07503 | V00510 |
| X96754 | V00557 |
| N67917 | V01512 |
| V01514 | V01514 |
| X87344 | X00369 |
| N53169 | X00567 |
| X02910 | X01394 |
| X01451 | X01451 |
| X01451 | X01451 |
| X01451 | X01451 |
| X01451 | X01451 |
| AA401046 | X02592 |
| 5537736F6 | X02592 |
| X87344 | X03066 |
| M10901 | X03225 |
| M54894 | X04403 |
| M54894 | X04403 |
| M54894 | X04403 |
| M54894 | X04403 |
| X07036 | X04408 |
| X07036 | X04408 |
| N75719 | X04744 |
| M19507 | X04876 |
| Y00816 | X05309 |
| M11233 | X05344 |
| AA479102 | X05972 |
| N24824 | X06182 |
| R33755 | X06547 |
| N41062 | X06820 |
| M86511 | X06882 |
| X07549 | X07549 |
| 1686702H1 | X07730 |
| X07979 | X07979 |
| X14723 | X08004 |
| J03561 | X12510 |
| J03561 | X12510 |
| J03561 | X12510 |
| J03561 | X12510 |
| M20566 | X12830 |
| M20566 | X12830 |
| M20566 | X12830 |
| M20566 | X12830 |
| U76549 | X12882 |
| M21304 | X13709 |
| X00351 | X13839 |
| X14830 | X14830 |
| X52882 | X14983 |
| AA598758 | X15187 |
| H27564 | X15729 |
| W15277 | X15940 |
| AA393214 | X15949 |
| M23502 | X16166 |
| K03515 | X16539 |
| M28880 | X16609 |
| 2403512H1 | X16674 |
| AA868186 | X17093 |
| J03236 | X51345 |
| X51416 | X51416 |
| AA411440 | X51521 |
| AA058828 | X51602 |
| AA455222 | X51675 |
| X51804 | X51804 |
| T72877 | X52015 |
| X52195 | X52195 |
| X52947 | X52947 |
| U06434 | X53682 |
| 3081284F6 | X53702 |
| M36821 | X53799 |
| AA490256 | X54048 |
| J03198 | X54048 |
| M60761 | X54228 |
| M11025 | X55283 |
| M33294 | X55313 |
| M33294 | X55313 |
| M31627 | X55543 |
| X55544 | X55544 |
| AA487812 | X56134 |
| X56134 | X56134 |
| X56777 | X56777 |
| H27379 | X57198 |
| M83652 | X57748 |
| X58528 | X58528 |
| M81182 | X58528 |
| S60489 | X60111 |
| X60592 | X60592 |
| R76314 | X61587 |
| M83665 | X62534 |
| R11490 | X62947 |
| AI436567 | X63422 |
| X63465 | X63465 |
| AA083577 | X63527 |
| X63547 | X63546 |
| 2159360H1 | X63692 |
| X64074 | X63926 |
| X63926 | X63926 |
| X64083 | X63926 |
| 2535659H1 | X69168 |
| AA187162 | X69654 |
| X69819 | X69711 |
| AI310990 | X71491 |
| T53775 | X71874 |
| 3285272H1 | X73568 |
| U11087 | X75299 |
| X75299 | X75299 |
| AA454585 | X75755 |
| X75821 | X75821 |
| X75918 | X75918 |
| X76029 | X76029 |
| R43734 | X76939 |
| A1189206 | X77303 |
| H17504 | X80692 |
| R26434 | X80910 |
| AI521155 | X81892 |
| AA088861 | X83228 |
| U10440 | X84849 |
| 407169H1 | X84909 |
| 3576337H1 | X85030 |
| T55802 | X85117 |
| 4407508H1 | X85337 |
| AA025432 | X85373 |
| T56477 | X87212 |
| AA464034 | X89401 |
| X89576 | X89576 |
| X89576 | X89576 |
| R83270 | X89750 |
| 917064H1 | X91249 |
| X91809 | X91809 |
| X92106 | X92106 |
| AA187458 | X92396 |
| AJ000519 | X92962 |
| X94991 | X94991 |
| X96427 | X96427 |
| R85213 | X98022 |
| X98296 | X98296 |
| X99585 | X99585 |
| R48796 | XM_008099 |
| R50354 | XM_009915 |
| W15172 | XM_016514 |
| AK000599 | XM_027140 |
| 7157414H1 | XM_031246 |
| AA044653 | XM_031608 |
| L16953 | XM_032556 |
| 1266202T6 | XM_033674 |
| AA805691 | XM_033788 |
| AA861582 | XM_036492 |
| H86407 | XM_037453 |
| 778372H1 | XM_040459 |
| AA016239 | XM_041087 |
| A1580830 | XM_042041 |
| Al732875 | XM_042637 |
| AA463411 | XM_045320 |
| AA648280 | XM_046411 |
| 3038910H1 | XM_046691 |
| H63831 | XM_047328 |
| 1654210F6 | XM_048530 |
| AA460131 | XM_049228 |
| 5539620F6 | XM_049755 |
| AA682896 | XM_050250 |
| L42856 | XM_054964 |
| 1483347H1 | XM_056259 |
| AI307255 | XM_058135 |
| H74265 | Y00062 |
| Y00064 | Y00064 |
| M17017 | Y00787 |
| M28130 | Y00787 |
| L02932 | Y07619 |
| AA504415 | Y09781 |
| A1809036 | Y12336 |
| AA516206 | Y12851 |
| 000527H1 | Y13829 |
| 059476H1 | Y13829 |
| Y13834 | Y13834 |
| L11016 | Y14768 |
| 3141315H1 | Y17803 |
| 551234R6 | Y17803 |
| AA426103 | Y18000 |
| H97778 | Z13009 |
| AA402431 | Z15005 |
| L07555 | Z22576 |
| U51278 | Z23115 |
| M58525 | Z26491 |
| AW772610 | Z26652 |
| Z29090 | Z29090 |
| H19371 | Z32684 |
| AA136533 | Z35481 |
| Z48810 | Z48810 |
| U49083 | Z49148 |
| R70234 | Z56852 |
| 4902714H1 | Z69918 |
| 150224T6 | Z80147 |
| M29871 | Z82188 |
| AI808621 | Z82214 |
| AA699919 | Z83821 |
| 5538394H1 | Z83843 |
| 5020377F9 | Z97832 |
| AA460801 | Z98749 |
| A1625585 | Z98750 |
| 2673259F6 | Z98752 |
| R22977 | Z98946 |
| AA007595 | |
| AA188574 | |
| AA280754 | |
| AA283874 | |
| AA460392 | |
| AA508510 | |
| AA515469 | |
| AA526772 | |
| AA576785 | |
| AA634241 | |
| AA663307 | |
| AA663482 | |
| AA713864 | |
| AA714520 | |
| AA828809 | |
| AA868502 | |
| AI061445 | |
| AI086865 | |
| AI264420 | |
| AI378131 | |
| AI440504 | |
| AI567491 | |
| AI693066 | |
| A1709066 | |
| A1766478 | |
| A1821337 | |
| A1949694 | |
| AW439329 | |
| AW630054 | |
| H24679 | |
| H29257 | |
| H51856 | |
| H66015 | |
| H72339 | |
| N57580 | |
| N54592 | |
| W07570 | |
| T75463 | |
| R88730 | |
| R91509 | |
| T56441 | |
| T77711 | |
| W92423 | |
| 1274737F6 | |
| 1338107F6 | |
| 1508571F6 | |
| 1548205H1 | |
| 1594182F6 | |
| 1594701F6 | |
| 1879290H1 | |
| 1902928H1 | |
| 194370H1 | |
| 195337H1 | |
| 198381H1 | |
| 2021568H1 | |
| 205203T6 | |
| 2194064H1 | |
| 224922R6 | |
| 2398102H1 | |
| 2531082H1 | |
| 2630745F6 | |
| 264639H1 | |
| 2704982H1 | |
| 2716787H1 | |
| 2798810F6 | |
| 2832401H1 | |
| 2894096F6 | |
| 2919406F6 | |
| 2937644F6 | |
| 2950021H1 | |
| 3010621F6 | |
| 3123948H1 | |
| 3253054R6 | |
| 3290073H1 | |
| 3330472H1 | |
| 335737H1 | |
| 3674358H1 | |
| 3749346F6 | |
| 3820429H1 | |
| 3978404F6 | |
| 4031124H1 | |
| 4056384H1 | |
| 4097060H1 | |
| 4288779H1 | |
| 4301823H1 | |
| 4558488F6 | |
| 4570377H1 | |
| 5058893F9 | |
| 5541621H1 | |
| 5546249F6 | |
| 5546336H1 | |
| 5771839H1 | |
| 5804485H1 | |
| 5849807H1 | |
| 6530555H1 | |
| 656258H1 | |
| 6591535H1 | |
| 859993H1 | |
| 930273R6 | |
| 938765H1 | |

**Table 3: Genes (By Genbank Accession Number) modulated at least 1.5 fold in all cell lines and at least 1.7 fold in patient responder sample.**

| Gene ID | Accession No. |
|---|---|
| 5543360F8 | AC009220 |
| AA237071 | AL035420 |
| AA455969 | D00015 |
| M25315 | D12592 |
| U41078 | D26512 |
| L10413 | L00634 |
| AA464627 | L09604 |
| 2470939H1 | L35848 |
| M84526 | M84526 |
| AI921879 | NM_002287 |
| AF204944 | NM_012105 |
| W77977 | NM_022336 |
| AA449040 | NM_024116 |
| AA521213 | S77359 |
| AA984230 | S80071 |
| AA456616 | U14970 |
| A1884916 | U29171 |
| U60519 | U60519 |
| X00351 | X13839 |
| AA868186 | X17093 |
| H27379 | X57198 |
| AA454585 | X75755 |
| X89576 | X89576 |
| AI580830 | XM_042041 |
| U49083 | Z49148 |
| 2398102H1 | |
| 2531082H1 | |

Organization Applicant
--------------------
      Street :
      City : New Brunswick
      State : New Jersey
      Country : USA
      PostalCode :
      PhoneNumber : (732) 524-6202
      FaxNumber : (732) 524-5008
      EmailAddress : tvolyn@corus.jnj.com
<110> OrganizationName : Johnson & Johnson

Application Project
-------------------
<120> Title : Methods of Assessing and Treating Leukemia
<130> AppFileReference : CDS 293
<140> CurrentAppNumber :
<141> CurrentFilingDate : ____-__-__

Sequence
--------
<213> OrganismName : HUMAN
<400> PreSequenceString :
ctgtttaatt atctgtaata atccttaaaa atcagcacac aaatccattc caggtagctt      60
ttgcaaccca actgctgtgg tggcaattgt ccagatgcca caacccccta agagtgggtc     120
atcctggggg agcaaggcct gagaaaggaa aggaagggaa aggcccccta gtgcctgccc     180
cacagccctg aggaaggcac atatttagcc tggcctgaga caggacagga aggggccaca     240
gggctgaagt gcagggacaa aggggtcagg gtcccaagtg tccggggccc cagctaccca     300
ccccatgtct gtttttggtt ttgtcattaa aaaaaataaa gtgacaatac tggtggagac     360
cagttgttgc actgtc                                                     376
<212> Type : DNA
<211> Length : 376
      SequenceName : AA454813
      SequenceDescription :

Sequence
--------
<213> OrganismName : HUMAN
<400> PreSequenceString :
gattggtaacctaatttgtggcctcctgacttttaaggaaaacgtgtgcagccattacacacagcctaaagctgtcaagagatt
gactcggcattgccttcattccttaaaattaaaaacctacaaaagttggtgtaaatttgtatatgttatttaccttcagatcta
aatggtaatctgaacccaaatttgtataaagacttttcaggtgaaaagacttgatttttgaaaggattgtttatcaaacacaa
ttctaatctcttctcttatgtatttttgtgcactaggcgcagttgtgtagcagttgagtaatgctggttagctgttaaggtggc
gtgttgcagtgcagagtgcttggctgtttcctgttttctcccgattgctcctgtgtaaagatgccttgtcgtgcagaaacaaat
ggctgtccagtttattaaaatgcctgacaactgcacttccagtcacccgggccttgcatataaataacggagcatacagt
<212> Type : DNA
<211> Length : 500
      SequenceName : AA454585
      SequenceDescription :

Sequence
--------
<213> OrganismName : HUMAN
<400> PreSequenceString :
taagttgtaa aaaaaatttt gttatttata aatgaattac acaataaaat aattattttt      60
aaaagtcaca aatacaatcg ttgtataaag tcattttggc atcaagtatc tcttaaatat     120
gttgcaaact attttccaaa gagatgtggt ccaaaccctc tgaaggcttt ataattttgt      180
attagataac aagtgaacaa aactgacaat aaatactcca acaattattt tttaaaatac     240
taagtggcaa atgctattct aagtggcaaa acaatc                               276
<212> Type : DNA
<211> Length : 276
      SequenceName : AA454652
      SequenceDescription :

Sequence
--------
<213> OrganismName : HUMAN
<400> PreSequenceString :
tatcaacatt tatatgcttt attgaaagtt gacaagtgca acagttaaat acagtgacac 60
cttacaattg tgtagagaac atgcacagaa acatatgcat ataactacta tacaggtgat 120
atgcagaaac ccctactggg aaatccattt cattagttag aactgagcat ttttcaaagt 180
attcaaccag ctcaattgaa agacttcagt gaacaaggat ttacttcagc gtattcagca 240
gctagatttc agattacaca aagtgagtaa ctgtgccaaa ttcttaaaat ttctttaggt 300
gtggttttgt tcatgtagca gtttttatgt agatctatat ataaaagtcc acacctcctc 360
agacagccaa tgaaacaact aaatttcaat ctgtacaacc taaatagtag ttacagtcct 420
ctattgtaca aaatagttac acta 444
<212> Type : DNA
<211> Length : 444
SequenceName : AA664389
SequenceDescription :

Sequence
--------
<213> OrganismName : HUMAN
<400> PreSequenceString :
aggctacata ctgccaacaa ccatgtgtct ctctagtcca cctcctgccc ctgagatccg 60
gactcacatc cgattgcctc tttaatctct ccgtttgaat gcccaacagg catctcaaaa 120
acacttcccc catggcctcc cagtttcccc catggtaaga aacagcccac cggctactct 180
gttcgagtca cactctcaga cagatttctc ctttctgtcc atcatctctt ccccacctct 240
gtctctaaag tatatcacag cttcgtgccc cagcacctag cacagcacat ggcaagcttc 300
ccgtaaatgt gtgctgagtc acctctggag gtcacctgct aatgagggag gtctctcctg 360
gcacaggtca cttaggaaag g 381
<212> Type : DNA
<211> Length : 381
SequenceName : AA515469
SequenceDescription :

Sequence
--------
<213> OrganismName : HUMAN
<400> PreSequenceString :
tttttttttttcatcttaacaacctttaatagttatctaaatgcagagtttgtttatgaaatgaaccaaagcagtttgtcattt
cttactataaaatattgaaaatcaagtgcgaaactcagccactattggctaaagaaactaataaaaaacgttaatgacctaga
aaagcaaaagcccattttaaactacttaaagcctctgcactagtccaatgagtcaaaggcaagggagaagtaaacctctaaaac
tgaagacgaccctctaaaggagaaaactatagaagttaaagtatgcatgcttattatactatgggaaaccaggaaggaagggag
agaaaaagaaaaagcacagtctattcatccagggcaatcagtaaaaatctacagtaacctgatcaaccacaaaatccttaggtg
ttgtgaaattacattggtcacttctgtaatcctaaaacttaaaatgattggtctctttaaaataaatggtttcaaaataatagg
atggtcccattaaa
<212> Type : DNA
<211> Length : 518
SequenceName : AA598561
SequenceDescription :

Sequence
--------
<213> OrganismName : HUMAN
<400> PreSequenceString :
tttatttggg atctttttta tttttataca catgacaaga ttttacatca agaatagtca 60
gttaaatagt acaaatttac attcatgagg aatgttaaag aaaattcaac taaaaaaccc 120
actttttcct gtgacccata atcccacatt ttacagtgca ggggagaagg ggattagggg 180
gcatccaaaa caagtctctc ccaaaaagaa atgatgtaaa tttcacattc cctctccaca 240
caggatccaa atggtgagag tataatttac aattcatctt tttcagctgt agattccttt 300
gctgtttctt cttcttcatc tgttcccaca tccatttctg catcttcgtc ttgctctgtg 360
tcttctgttg tgtcttctgc 380
<212> Type : DNA
<211> Length : 380

SequenceName : AA598758
SequenceDescription :

Sequence
--------
<213> OrganismName : HUMAN
<400> PreSequenceString :
aaaaaaaaaacatgaagggagacatgactttattagaaaaataaaaaacaactgaggtgatgggttggtcttcagcggatcctt
ggtggatgaggctgcttttggctgcactggccttctcccgctcccgccgccgcgcagggtccaactcaaaacagcgccatagcc
tcagggtctcatctgctgctgcggatgccactgtggccccatctgggctcatggtcagactcaggacccgggatgtgtgacctt
tgagttcagccaccttggccatggttgggtacttccaaataactagctgattctgtgcaaagccatggcctgagatgagctcct
tgtaagggggagaccagaggatggagcacacctgggaatgggcatccacggcactcagacaggccccagagcaaaaattccaga
tgcgaatgtgtcgatcactggtgcaccctcctgtggcaaggacatttga
<212> Type : DNA
<211> Length : 469
SequenceName : AA598776
SequenceDescription :

Sequence
--------
<213> OrganismName : HUMAN
<400> PreSequenceString :
caacgagaaggtggagctgcaggagctgaatgaccgcttcgccaactacatcgacaaggtgcgcttcctggagcagcagaataa
gatcctgctggccgagctcgagcagctcaagggccaaggcaagtcgcgcctgggggacctctacgaggaggagatgcgggactg
cgccggcagtggaccagctaaccaacgacaaagcccgcgtcgaggtggagcgcgacaacctggccgaggacatcatgcgcctcc
gggagaaattgcaggaggagatgcttcagagagaggaagccgaaaacaccctgcaatctttcagacaggatgttgacaatgcg
<212> Type : DNA
<211> Length : 335
SequenceName : AA487812
SequenceDescription :

Sequence
--------
<213> OrganismName : HUMAN
<400> PreSequenceString :
aagtcacctataaatatacttacagaaccaccaagtcaaaatgtggagaaagagatgaattatccccaaagagaattaaagtgg
aggatgggtttccagatttccaggattctgtgcaaacactcttccagcaggctagagctaagagtgaagaacttgcagctctta
gttcacagcagcctgaaaaggtgatggcaaagcagatggagttcctttgcaaccaagctggctatgagagactgcagcatgccg
agaggaggttgtctgcagggctttacaggcagagctcagaagagcacagtcctaacggcttgacttccgataactcagatggac
aaggagaaagacctttgaatctccgaatgcctaatttacagaacagacaaccccatcattttgtggtggatggggagctgagca
gactttaccccagtgaggcaaagtcccactcatcagagagccttggattttaaaagactaccctcattcagcttttaccttag
aaaagaaagtcatc
<212> Type : DNA
<211> Length : 518
SequenceName : AA488645
SequenceDescription :

Sequence
--------
<213> OrganismName : HUMAN
<400> PreSequenceString :
ttttatttttttttttttttcttactcaccatttaatactgccgccaactataacagattaaaaatgtacacatgacaaag
tggaaaaaagtcccaaaatgcaacagtttcagcaaaagaacatactggctaaggattactacagttaacatcggtacagtaaaa
acgatggcaaacagggatttggcaccacatttacaaagtaaaagcatgcactgttaatacactttagatgtttctcaacagaaa
aggccatgaagatggaaaacaagaggcaccatgtacaaaactccctataactgggacaaacaagcaagaatcaaagtggtcaat
ttagtaaatatgtagcagcaaagtcactggttctgtttggaatttagcaatttgcatttctgattggcagctgccctgggtgtg
tctgtatccaagaagctgactttatccaactacatcagca
<212> Type : DNA
<211> Length : 460
SequenceName : AA490256
SequenceDescription :

Sequence

--------
<213> OrganismName : HUMAN
<400> PreSequenceString :
ttttttttttttgcatatattaattcagtttaatcatatcaattcttaaacaaaaacttcctccaagagacaatagttcacag
taactgcaaagcttactcacaattttaaaaatatcgctcatgcttagcattcaaagaagatattatagaataactgtaaaaac
ttttaccaggaacatcagcttctgacttgaaaaattacaaatactgaagcattttggtatcagacacagtagttttgacattgt
ctgttcatttgatgagtttctggaagtttggaaaattgtacctctataattggataatgcgcttattctaaactaaacattttc
aaatgtagaaaacaaactagcaagctacacatacaaagaaaagccctctaagatttgcttttcacatgttgaatgccagcatgt
gcaccgtgaatcctggaagactagaattgatacatacgcttggcttgaagtctataccccaggctcacgatgg
<212> Type : DNA
<211> Length : 493
      SequenceName : AA479102
      SequenceDescription :

Sequence
--------
<213> OrganismName : HUMAN
<400> PreSequenceString :
ttttttaaaacaaacttaactttatttcctcactttcacttaaacttgatttataaaacacatgaaaaaacatttttaaga
gttctgtatcacagaacattaaacagtacaaatatccattgcttcataggttcaagttacataaattaaagtcaaataattgga
aactgattcaatagggaaaactatacatgaaatgaaggtcaaaaggagctatacagcaatatttcattgtttatagattatgag
ttactttcaggaccttaacaaagattctgaatatttagacttcctttgttgtattttatacttaaatatctccctacctatact
gagtcaaactacttgaccaaaacatctgatttaggaaagcatctagctttatagcacaagtttttccatctacagttactatct
tcaaagga
<212> Type : DNA
<211> Length : 428
      SequenceName : AA481076
      SequenceDescription :

Sequence
--------
<213> OrganismName : HUMAN
<400> PreSequenceString :
gcactgtgtgttcagtgaaaactagagaaacacaataagcaaatcacatgaaggctgagatgattgagatgaaagttaacatag
tgtgaatgaaagtctggccaacagagcagtagatgatataacaaagaaagcatgctttctgaggagatagtggggataatccct
gaagagttggcagtgtgagtgcactcatttatcttctgtgaggtgggattttgtgttcctcacacccatgtaattattcttag
aattgagattgctacacggagcagtccttgacgatcgtgtttctgacgtttctcttccttgcctggactgcctttgacaaggca
gaaatgaacatgaaaagtccccaggagaggggagctgtgtagtgagggatactgagggcagtcctgtggctgaggggcacagat
tgaactgctgaactagttggaggtctagatgaggtgctttacgcatcagctgc
<212> Type : DNA
<211> Length : 473
      SequenceName : AA465444
      SequenceDescription :

Sequence
--------
<213> OrganismName : HUMAN
<400> PreSequenceString :
cgatgactcaatcggcactgggtatgacctgtcagcctctacattctctcctgacggaagagtttttcaagttgaatatgctat
gaaggctgtggaaaatagtagtacagctattggaatcagatgcaaagatggtgttgtctttggggtagaaaaattagtcctttc
taaactttatgaagaaggttccaacaaaagacttttaatgttgatcggcatgttggaatggcagtagcaggtttgttggcaga
tgctcgttctttagcagacatagcaagagaagaagcttccaacttcagatctaactttggctacaacattccactaaaacatct
tgcagacagagtggccatgtatgtgcatgcatatacactctacagtgctgttagaccttttgggctgcagtttca
<212> Type : DNA
<211> Length : 411
      SequenceName : AA465593
      SequenceDescription :

Sequence
--------
<213> OrganismName : HUMAN
<400> PreSequenceString :
gccggagagtctacaatgttacccagcatgctgttggcattgttgtaaacaaacaagttaagggcaagattcttgccaagagaa

ttaatgtgcgtattgagcacattaagcactctaagagccgagatagcttcctgaaacgtgtgaaggaaaatg[?]tcagaaaaaga
aagaagccaaagagaaaggtacctgggttcaactaaagcgccacgctgctccacccagagaagcacactttgtgagaaccaatg
ggaaggagcctgagctgctggaacctattccctat
<212> Type : DNA
<211> Length : 287
      SequenceName : AA464034
      SequenceDescription :

Sequence
--------
<213> OrganismName : HUMAN
<400> PreSequenceString :
tttcaatgatatattgccagttattttatttttctaaaaacaatagaaaaaaatcaactttaaaaagcaaaatgtacttaaa
taaaaaaaaattagggtttatagtacctataatactaggtactatatactaggattgaaattctgtgtaactgctataaacgttt
tattaaagttatttacatttaatggcaatatttacagagaaacattgtgtaaatcttaaaatttttaaaaacaattcttaaat
acaaatctgttaaagaaaaaaaaaagatggtaagcataaaaaagttcttttatgcccaaagtccaatttgaggcagtttacatt
atggctaaatctttcagtctcaagactcagccaaggtttgtgaggttggcattgattctggcatttgaaacaagttcataggtg
attgctcggggacatttcggttagaaggaatcggtttcccgacttttgcctta
<212> Type : DNA
<211> Length : 473
      SequenceName : AA464600
      SequenceDescription :

Sequence
--------
<213> OrganismName : HUMAN
<400> PreSequenceString :
tcatcaactggccctggagtgatttcttccgaaccctcatagcggcaatcctctacctgatcacctccattgttgtccttgttg
agagaggaaccactccaaatcgtcgcagggtactggctaatcgtagtgccttggtagagctatgtacttccgtcggagcagaa
tagagccatgccgagatggccgtgtaggacttcctcatctcctgaactgaatactctcatgaatatctccaccacaaacattca
gcagcaatccacctctttgagtaaagtgcttattgggaactttgcttccagctgcaatcaccctcctggtgtggcacatatgtg
tgtgctag
<212> Type : DNA
<211> Length : 344
      SequenceName : AA464627
      SequenceDescription :

Sequence
--------
<213> OrganismName : HUMAN
<400> PreSequenceString :
ggtaatgaaaccgtgaactttattgagaactttcaagcatcataacttgaagaagtatttctcaattcagtttatcgaaatact
tcagctttccagctggatctgggattattgtttcactaccaggtttccagccagcagggcagacttctccgtgtttgtcagtgt
actggaatgcttgaaccaaacgtagtgtctcatccactgatctacccacaggaagatcattcagagtaatttgtcttaggattc
ctttgtcatcaataatgaagagacctctaagagtgtggcctga
<212> Type : DNA
<211> Length : 295
      SequenceName : AA459663
      SequenceDescription :

Sequence
--------
<213> OrganismName : HUMAN
<400> PreSequenceString :
tggtgctcatcttcgctcgtttaatttcagtcagatattaaacatttacaatttttttatataacaaaacaagaacatgcaaag
ttacaaatatagaaaatataaagtacatgcatatttcaaagacctgttaatggtgtccactttggattcttacatgaaacgatt
cagtgcacattgtaagcctaaggaccacgcaaaagggtttccacatattaagtattcagtaccttacaaaagttaatgcattag
acacttcagatgttaactgctctaaacaaaactcctaagtctgtcctatgcaatatatatttatatacatatatatttttaca
tagaatactcacaaagtgcaagccaataataacattgcagaaaaaaaaaa
<212> Type : DNA
<211> Length : 387
      SequenceName : AA455969
      SequenceDescription :

Sequence
--------
<213> OrganismName : HUMAN
<400> PreSequenceString :
ttcccagcca tgctttgcaa gatgggcttt gcggtacata ctagtgaact atcgtgaatc 60
cacgggcttt ggccaggaca gcatcctcac cctcccaggc aatgtgggac accaggatga 120
gaaggatgtc cagtttgcag tggaacaagg agcaccagga ggaacacatt gatgcaagcc 180
atgtggacct tatgggtgga tcccatggtg gcatcaatac cagccacatg attggtcagt 240
aaccagagac ctncagggcc tgagtggcac gagaacccgt gataaacata gccaccatgt 300
agggcaccac tgacatccct gactggtaag aggtggaggc tgg 343
<212> Type : DNA
<211> Length : 343
SequenceName : AA456408
SequenceDescription :

Feature
-------
Sequence: AA456408:
<221> FeatureKey : Unsure
<222> LocationFrom : 253
<222> LocationTo : 253
Other Information : Any base may occupy this position.
CDSJoin : No

Feature
-------
Sequence: AA456408:
<221> FeatureKey : misc_feature
<222> LocationFrom : 253
<222> LocationTo : 253
Other Information : Any base can occupy this position.
CDSJoin : No

Sequence
--------
<213> OrganismName : HUMAN
<400> PreSequenceString :
ttttttttttttttggctgggactgccccaaaggcagacgaggtttattgggcagcagctgggaaaatcagcggttggacttgg
ccacacgctccagctcgtccttcttcttaatggcataggagttcgaggagcccttggcagcattgatgagctcatctgccaggc
actcagcaatggtcttaatgttccggaaggcagcctcacgagcgcctgtgcacagcagccagatggcctggttcacacggcgca
ggggggacacatccacagcctgtcgtctcacagtcccggcgcgcccaatgcgtgtggagtcctcccggggaccactgttgatga
tggcgttcaccaggacctgcagagggttctcgcctgtgagcaggtgtatgatctcgaaggcatgcttgacgatgcgcacagtca
tgagcttcttgccgttgttgcggccgtgcatcatcatggagttagtgaggcgctccacaatgggacactgaactttgggaaa
<212> Type : DNA
<211> Length : 502
SequenceName : AA456616
SequenceDescription :

Sequence
--------
<213> OrganismName : HUMAN
<400> PreSequenceString :
gcaatgagataacgttttatttaattctcaccatttatatacaaacacaagtgaataaaacacatcgcaaaatggtaaaattt
catatttagtatttataggtgcatagtttcatgctcacatatttttgagtattatatatattaacaatttcacaatacgtcat
tattcttagacagtatcattaaaagacacctaaaaatcttataatatatgatagcaaatcactaacaacttctgaacaacagca
acaaaaaaatagtgaggatttagaaataagtggtagtcacttaggtgtttttaatttgttttaacatcgtagattgaagccaca
aaatccacagcacacaaagaccctgctaccatgtattcacttcagtgaaagggaagcaccgaaatgctgagtgggggcaggtac
agatacatcaatcactgctgatggaagacttcgagatacac
<212> Type : DNA
<211> Length : 461
SequenceName : AA455067
SequenceDescription :

Sequence
--------
<213> OrganismName : HUMAN
<400> PreSequenceString :
aattcggaagaggaaggtgaagaagggcgtccaaaggatgaccgccacctccgtggctgtggctaccttcccggctgcccgggc
tccaatggtttccacaacaacgacaccttccacttcctgaaatgctgcaacaccaccaaatgcaacgagggcccaatcctggag
cttgaaaatctgccgcagaatggccgccagtgttacagctgcaaggggaacagcacccatggatgctcctctgaagagactttc
ctcattgactgccgaggccccatgaatcaatgtctggtagccaccggactcacgaaccgaaaaaccaaagctatatggtaagag
gctgtgcaaccgcctcaatgtgccaacatgcccacctgggtgacgccttcagcatgaaccacattgatgtctcctgctgtacta
aaagtggctgtaaccacccagacctggatgtccagtaccgcagtggggctgctcctcagcctggccctgccatctcagcctcac
catcaccctgctaatgactgccagactgtggggag
<212> Type : DNA
<211> Length : 539
      SequenceName : AA455222
      SequenceDescription :

Sequence
--------
<213> OrganismName : HUMAN
<400> PreSequenceString :
aatattttataacttgtccatacagagttttgatgatggagactattttcctgtgtggggcacatgccttggatttgaagagct
ttcactgctgattagtggagagtgcttattaactgccacagatactgttgacgtggcaatgccgctgaacttcactggaggtca
attgcacagcagaatgttccagaattttcctactgagttgttgctgtcattagcagtagaacctctgactgccaatttccataa
gtggagcctctccgtgaagaatttacaatgaatgaaaagttaaagaagtttttcaatgtcttaactacaaatacagatggcaa
gattgagtttatttcaacaatggaaggatataagtatccagtatatggtgtccagtggcatccagagaaagcaccttatgagtg
gaagaatttggatggcatttcccatgcagctaatgctgtgaaaaccgcatttatttagcagagttttgtta
<212> Type : DNA
<211> Length : 493
      SequenceName : AA455800
      SequenceDescription :

Sequence
--------
<213> OrganismName : HUMAN
<400> PreSequenceString :
agtgactccagaggccttcacaaagggcagctggagtggggaagtaggactcaaaggtgcggcagagagccccagctgggcact
tgttaactcctgaggtccagtcccatcctctgtgccagttgctcttgcacgtgtgggaggtgtgacaatcctcccaccagcgct
gacagtcctctttgcataagggcacatccaggaagcgttctttgcgccagtcctgattcacctgctggatccagggccccaggt
tgggtgagcactcatagagacaggtgtcctggatgaagtggcgcttgcaggcgggctccatcttgccgcagtggtcccagttaa
agttgtacaggcgggaggtgtccttgtgcagctcctggctggtgctggctgtgcagcaggcattcttcttccagggactgcatt
ggtcatgcagcttgtcctcaggacctgg
<212> Type : DNA
<211> Length : 448
      SequenceName : AA453816
      SequenceDescription :

Sequence
--------
<213> OrganismName : HUMAN
<400> PreSequenceString :
gtgatctgcatgtggcaggctgcgcagtggagcggccagtgggcaggatgacgagccagacccctctgccccagtccccccgg
cccaggcggccaacgatgtctactgttgtggagctgaacgtcggggtgagttccacaccaccaccctgggtaccctgaggaag
tttccgggctcaaagctggcagagatgttctctagcttagccaaggcctccacggacgcggagggccgcttcttcatcgaccgc
cccagcacctatttcagacccatcctggactacctgcgcactgggcaagtgccacacagcacatccctgaagtgtaccgtgagg
ctcagttctacgaaatcaagcctttggtcaagctgctggaggacatgccacagatctttggtgagcaggtgtctcggaagcagt
t
<212> Type : DNA
<211> Length : 421
      SequenceName : AA429219
      SequenceDescription :

Sequence

--------
<213> OrganismName : HUMAN
<400> PreSequenceString :
cactcccgacggatctttgcttctcacgccagctggatgtgtggaatctggtgaaaatgtaatgaataccacttatgttttctc
caggaagaatcttaaaggcccatcgctcatcttccatgtcctggaaaagccactcttgctgttcgctgctgtccggtctactt
tgaactgaggccaatggtggaaacaggtgtggagctgatgagtctgccctaccgcctggtgtttgctgtggcctcggaggattc
cgtgcttctgtatgacacccagcagtccttcccttttggttacgtgtctaatatacattaccaaaccctcagtgaaatttcatg
gtccagcgatggtgccttcctggccatttcttccacggacggttactgcccatttgtgacatttgggaaaaatgaacttggaat
tcctttgaaaaagaagccacttttgaaaatgggaactcctgttaaagcaaggaaaaccacgggtctgagacatcggggtgtttt
tccaggaccccaaccgctaaaggggacc
<212> Type : DNA
<211> Length : 532
      SequenceName : AA425120
      SequenceDescription :

Sequence
--------
<213> OrganismName : HUMAN
<400> PreSequenceString :
ggcggctgcc aggatcccgg gcccggatcg cacgaagccc gcgcggccgt ctcctccgcg      60
gccacccctg cgcctcccgc gagctccact tcccatctgc gctaggccac cccagctggg     120
gaagtnntng tggctgtcga gaatgggggt ctgtgggtac ctgttcctgc cctggaagtg     180
cctcgtggtc gtgtctctca ggctgctgtt ccttgtaccc acaggagtgc ccgtgcgcag     240
gangatgcca ccttccccaa ag                                              262
<212> Type : DNA
<211> Length : 262
      SequenceName : AA425422
      SequenceDescription :

Feature
-------
Sequence: AA425422:
<221> FeatureKey : misc_feature
<222> LocationFrom : 126
<222> LocationTo :
      Other Information : Any base.
      CDSJoin : No

Feature
-------
Sequence: AA425422:
<221> FeatureKey : misc_feature
<222> LocationFrom : 126
<222> LocationTo : 243
      Other Information : N=any base
      CDSJoin : No

Sequence
--------
<213> OrganismName : HUMAN
<400> PreSequenceString :
cttttctataaaacttcatttgtaatttttttccaagaagtagtaaggggaaatattgcaattatgtaaaggtaaggaacaa
tggtgtataaaaataatttaagcacaatattaataacaaagcaactttttttcaaggaaaactagtaggtcatcttgcccaaga
tttttccaaaggtattaaatatattttcaataacagtacatacaaatacacatacacaaatataccacacagacacatgtgcac
atacatgcacacacacacacacacacacacacacacacacacatatatatatacagctcaagtattttttggtgtgcattcta
gctaatccacatagctaaagtgaaaaaacactgaaaagatcaatgcctgagtaaccttaaatttgaacaaactatgcattggat
gtgaattttggaggcatgctaagatttc
<212> Type : DNA
<211> Length : 449
      SequenceName : AA405769
      SequenceDescription :

Sequence

--------
<213> OrganismName : HUMAN
<400> PreSequenceString :
aacagatttaatattatattgcagttttcagcgatgcagaatgagctgcaattgtgttttaaggagaagccaaatggggatggt
tgtccctgcaacatggtg[?]cactcctgggccatgtgcagcctcagtggacactcttccatagcgctgaggccctgcccgcctcc
agttaccctgtactgcccatgccttacagtcagtgcgcaggcttcaccttttcatcaccagcctctctgctcagtgctctggag
ttcttgaccttgtcctttatcatgagatttgctgaaatcactaatgaaaataactcccaaaagcaacaaacaaaaatattagtt
taactggcactgtggtatattaaaggcacaagggcattgtggcttaacacttttgctggatcccaagagacgcacatgatgtt
aaaaagagatctggcagcagtactaatactacatttcagtgtaatcatcttggggtggtttgg
<212> Type : DNA
<211> Length : 483
      SequenceName : AA410680
      SequenceDescription :

Sequence
--------
<213> OrganismName : HUMAN
<400> PreSequenceString :
ttttttttttttttttttttttgcctttgcaaagcttttatttcatgtctgcggcatggaatccacctgcacatggcatctta
gctgtgaaggagaaagcagtgcacgagaaggaatgagtgggcggaaccaacggcctccacaagctgccttccagcagcctgcca
agcgcatggcagagagagactgcaaacaaacacaagcaaacagagtctcttcacagctggagtctgaaagctcatagtggcatg
tgtgaatctgacaaaattaaaagtgtgcatagtccattacatgcataaaacactaataataatcctgtttacacgtgactgcag
caggcaggtccagctccaccactggcctcctgccacatcacatcaagtgccatggtttagagggtttttcatatgtaattcttt
tattctgtaaaaggtaacaaaatatacagaacaaaactttcccttttttaaaactaatgttacaaatctgtattatcacttgtat
ataaatagtatatagctgatcattaataaggtgtataagtacaatgtattctaaaactgttaagc
<212> Type : DNA
<211> Length : 569
      SequenceName : AA411440
      SequenceDescription :

Sequence
--------
<213> OrganismName : HUMAN
<400> PreSequenceString :
atccgccagacatttgccaagttgtagaggtgaccactgagagcgacgagagccggtcagcatgagcgagctctaccctctgca
gatctcccccgtgtcttcctatgcagaaagcgaaacgactgatagtgtgcccagcgatgaagagagtgccgaggggcggccaca
ctggcggaagaggaatattgaaggcaaacagtacctcagcaacatggggactcgaggctcctacctgctgcccggcatggcgtc
cttcgtcacttccaacaaaccggacctccaggtcaccatcaaagaggagagcaatccggtgccttacaacagctcctggccccc
ttttcaagacctcccccttttcttcctccatgaccccagcatccagcagcagtcggccagaccgtgagacccgggccagcgtcat
caagaaaacatcggatatcacccaggcccgcgtcaagagctgttaa
<212> Type : DNA
<211> Length : 466
      SequenceName : AA393214
      SequenceDescription :

Sequence
--------
<213> OrganismName : HUMAN
<400> PreSequenceString :
tgaacatataaggctagaaaaagaaatttctaagttaaagcagcaaaatgaacagctaataaaacaaaagaatgaattgttaag
caataatcagcatctttccaatgaggtcaaaactctggaaggaaagaacccttaaaagagaggctcacaaacaagtaacttgtg
agaattctccaaagtctcctaaagtgactggaacagcttctaaaaagaaacaaattacacccgtctcaatgcaaggaacggaat
ttacaagatcctgtgccaaaggaatcaccaaaagcattgtttttttgatagccgatcaaagtctttaccatcactcatccagtt
cgctatttttgatacatcaagtttaggcctttgtcagaggtgaaaatgcaggacagagagtgtggatctcagccaggtcttggac
cgctctcaggaaggatgtgctgagtg
<212> Type : DNA
<211> Length : 446
      SequenceName : AA402431
      SequenceDescription :

Sequence
--------
<213> OrganismName : HUMAN

<400> PreSequenceString :
acctctctgt ggtggattta catggccaag ctgtgtgaag aaataggaaa gaagaagtta 60
ccattaacca aggatatgag agaacaagga gttaaaagca atccatgtga ctcaagcctt 120
tcacatactg acagatggta tctgccagtc tcttcaaccc tcttctcact ttttaaaatc 180
ttgttccatg cctccaggtt tatctttgtc ttatctacca gtttattcct gtgaacttca 240
gattgaacca ttcattgcag cagtagcctt aaaaaggctt ttgtttattt ctttggtttg 300
ttaactagtg tcatctattt agagaaacat ttttgttttt aattgctcaa agctgtccgc 360
cgctagtctt atgagccaat ctactaaaac tntggagaaa ctttgtatgt gcacacaaaa 420
gtattcaaga gacagtattg ctaacatctc atcttaatgg cctttggtaa tgagaagttt 480
taggtgcntc caaaccaata taaatgggat aatggtggta atttggggn 529
<212> Type : DNA
<211> Length : 529
SequenceName : AA187458
SequenceDescription :

Feature
-------
Sequence: AA187458:
<221> FeatureKey : misc_feature
<222> LocationFrom : 392
<222> LocationTo : 529
Other Information : N=any base
CDSJoin : No

Sequence
--------
<213> OrganismName : HUMAN
<400> PreSequenceString :
actctgcttacattttataagtttaaggtcagctgtcaaaggataacctgtggggttagaacatatcacattgcaacaccta
aattgtttttaatacattagcaatctattgggtcaactgacatccattgtatatactagtttctttcatgctatttttatttg
tttttgcattttatcaaatgcagggcccctttctgatctcaccatttcaccatgcatcttggaattcagtaagtgcatatcc
taacttgcccatattctaaatcatctggttggttttcagcctagaatttgatacgctttttagaaatatgcccagaatagaaaa
gctatgttggggcacatgtcctgcaaatatggccctagaaacaagtgatatggaatttacttggtgaataagttataaattccc
act
<212> Type : DNA
<211> Length : 423
SequenceName : AA234897
SequenceDescription :

Sequence
--------
<213> OrganismName : HUMAN
<400> PreSequenceString :
acagaaacaa gaaggcagct gctgcttcct gctcagcact ggggactatg cctgcgccga 60
tggtagtgtc cggaaagaga cctgccaaga cgccctctcg tccaactatg cccaggctga 120
agtcctcacc ctctcttaaa gcggcaccaa cgtgagagag acaggcagac agacagaaag 180
ccagagntta ggggaaactc tggaacccag acaagaatct tttcgctggg aaagactcag 240
atatccttgt ttgcacagga ctggtggaaa atctcccatg cgaccctcgg ggcccagagc 300
catctgggtc tgatgttctg ttccatttgt acatcgaaga gatatatatg cacatattag 360
tatctatatt cattacatat tatactcttg tgtgtagtgc aaa 403
<212> Type : DNA
<211> Length : 403
SequenceName : AA151374
SequenceDescription :

Feature
-------
Sequence: AA151374:
<221> FeatureKey : misc_feature
<222> LocationFrom : 187
<222> LocationTo : 187
Other Information : N=any base
CDSJoin : No

Sequence
--------
<213> OrganismName : HUMAN
<400> PreSequenceString :
ttttcaattg tcattgtttt atttttttcca aaataacata aaaatacttt ccagttgaat 60
tacataaaaa atttaatcca taaaaccatt atacagtcat gcattggata acaatgggaa 120
tgtgttctga tgaatgcatc cttgggcgat tttgtcactg tgtgaacatc ataagagtgt 180
acttacacaa acctagatgg tatagcctat tgttcctagg ctataagctt gtaaagtgtg 240
ttactgtact gagtactata gg[?]aactgta acacagtggt acntgtgtat ctaaacaaat 300
ctaaaacatt agaaaaggtn ccaaaaaata tgggtataat ggnttaaaaa aatgggtaca 360
cct 363
<212> Type : DNA
<211> Length : 363
SequenceName : AA074666
SequenceDescription :

Feature
-------
Sequence: AA074666:
<221> FeatureKey : misc_feature
<222> LocationFrom : 283
<222> LocationTo : 343
Other Information : n=any base
CDSJoin : No

Sequence
--------
<213> OrganismName : HUMAN
<400> PreSequenceString :
tttttttgga aatccaaagg tctcatttat taatggcatt ttatgagtgt catagcattt 60
atttaaacat agagttgtca ttggtaatag aaagttaaac agaaacccaa ctcatgaatc 120
tgaaaatgat ccattaataa tatatagtat tgtccgaagc ctcccagctg ctgggaacat 180
tgggaaggca tgatatttgt cttcaataaa ccattttctt tgtcaggttc accagcttat 240
tttcctttcc cctataaaca tattcccctt gcttttgccc ttgatttaaa taaacatnaa 300
aaagataaca tttagntcca ttcacaagaa actggtaata tgnaaatatt ttgaatggaa 360
acatatctct ttggggagtg gaaatgcggg tngaagtagg acattttagg taaagagtaa 420
agaggtaatn aagggtaatc nctcagggga agtggtaata ntt 463
<212> Type : DNA
<211> Length : 463
SequenceName : AA083577
SequenceDescription :

Feature
-------
Sequence: AA083577:
<221> FeatureKey : misc_feature
<222> LocationFrom : 298
<222> LocationTo : 463
Other Information : n=any base
CDSJoin : No

Sequence
--------
<213> OrganismName : HUMAN
<400> PreSequenceString :
ntgncaacac gtttaattct gtggccaggc tagcctgtct ccaaggcctg gtggacagca 60
cgtcaccaga ggctgccgna ggagcaggca gggccagccc tganagagga gtgcaggcaa 120
aggcgggggc tctgaagtgg ctgctggagg canagngccc cgggctggga gtgctcagta 180
gccgtcgtta gcccaggtga cctcgtagtc ggggtacttg gctttgattt tctcagttga 240
aatggcgtgc tgggcaagga ccataggcca tggaatagcc gtacacgtga atcttnttgt 300
cntgactctc ngtcgggaga tt 322
<212> Type : DNA

<211> Length : 322
      SequenceName : AA057781
      SequenceDescription :

Feature
-------
Sequence: AA057781:
<221> FeatureKey : misc_feature
<222> LocationFrom : 1
<222> LocationTo : 104
      Other Information : n=any base
      CDSJoin : No

Feature
-------
Sequence: AA057781:
<221> FeatureKey : misc_feature
<222> LocationFrom : 1
<222> LocationTo : 311
      Other Information : n=any base
      CDSJoin : No

Sequence
--------
<213> OrganismName : HUMAN
<400> PreSequenceString :
gtgtttttgg aagtttatta tatgaagatg gtatacaaaa tacattcatc atgactagaa      60
atataggacc aaaccatgtc tgtcttatat ctgtagcata tattcttggt ttgtataaaa     120
gtaactttaa aattccagtt tccttaaata gttatgcaca aaacacacat acacccacac     180
acacacacac acacacacac acacatacag ttacaccact gtcggccaaa gatgcactcc     240
tcctttaatc aatttaaatg aggctagcga gtatctgttt gatgtttgca ttcttgtggg     300
ctaggaaaca aggcacgggt ccctaaaatt aacatctcgg tgtcacttct tggactgaca     360
agacacagac ttgcacatgg tttcagcccc attccaccca gactgttcca cgtacattat     420
ctcagaaact ctgaaaggaa gtgctcgttc tttgttagtg ccaaccattt ttgtcataaa     480
tggcaaatga ttgggatatt atcagttaat tcatgtttca atttcagtgc tattttaatg     540
gacaagcact tgtaactagc ccattattac aagtctccat ttttttccac attaanctcc     600
ngagggacca tctttggccg atggagg                                         627
<212> Type : DNA
<211> Length : 627
      SequenceName : AA058828
      SequenceDescription :

Feature
-------
Sequence: AA058828:
<221> FeatureKey : misc_feature
<222> LocationFrom : 1
<222> LocationTo : 627
      Other Information : n=any base
      CDSJoin : No

Sequence
--------
<213> OrganismName : HUMAN
<400> PreSequenceString :
ttgattatct ggatgcaggt tttattatca ttaggatgac tgtcatcgac aagggatagg      60
ggcttagagg gttatataca aaccacaatt cctaggcgat gtttcaatat tcccacacac     120
atttatccca cagccagaca cagcacaaca ccacaggntt tgcccctaca tatgcttctc     180
caagcagtaa cacagctgtg ggtcagggct ctgcacccac aaagggatgc ccagaaagga     240
gaagatcctg ggtcctgggg gtgaagttta cacatggaga atcctgccag gttggggagg     300
ggatggaagc tggattttnc acacaacgct tcagtcccac cnccaccctt cacttggctt     360
gccctcccca gggggctccc tggtccccac ttggggggag tctttgncaa agacacagaa     420
gggaccatnt ttagccangg ctaanggaag gagcaggc                             458

<212> Type : DNA
<211> Length : 458
      SequenceName : AA069596
      SequenceDescription :

Feature
-------
Sequence: AA069596:
<221> FeatureKey : misc_feature
<222> LocationFrom : 1
<222> LocationTo : 458
      Other Information : n=any base
      CDSJoin : No

Sequence
--------
<213> OrganismName : HUMAN
<400> PreSequenceString :
ttttntttttttttttttctctgtgacaaactttattaaattttgcatcagtacagggcatctcttattgcatactagaat
cccaggattcaggcgggtggtaaagtggcaacgacgaatttgggtatctggtctcccaaaagtacttgccgctctacccctgac
tctgcaatggctgccaggcggatcactcctccactggagccatcccgctccatggccaaagcgaganatntggcacgtgaattg
cagacactcttccttggtcatgccttcccggtaggtagcatcaacatagccatagatgtaggagctcccggagcctccaatggc
aaaggactgccttaccatcatacccccataggcactggagtacacctngccctccttncttggagggtcccagcctgcgaatg
atgattccngccatcaggtcttcccggtatcggtaaca
<212> Type : DNA
<211> Length : 458
      SequenceName : AA070997
      SequenceDescription :

Sequence
--------
<213> OrganismName : HUMAN
<400> PreSequenceString :
attcggcacgatgggaagatggcggaggtgggggagataatcgagngctgccgctacccgtgctgcggcggaaccaggacaacg
aagatgagtggcccctgncgaggatcctgagcgtgaaggacatcagtggccggaagcttttctacgtc
<212> Type : DNA
<211> Length : 152
      SequenceName : AA017042
      SequenceDescription :

Sequence
--------
<213> OrganismName : HUMAN
<400> PreSequenceString :
ccagagccagaaatggaaagtacagacttctgaaaatatctattggaaaatgagcaacttgtgattggatcatatagtcagcct
tcagattcctgggataaggattatgattcctttgttttacccctgttggaggacaaacaaccatgctatatattattcaggtta
gattctcagaatgcccagggatatgaatggatattcattgcatggtctccagatcattctcatgttccgtcaaaaaatgttgta
tgcagcaacaagagcaactctgaagaaggaatttggaggtggccacattaaag
<212> Type : DNA
<211> Length : 305
      SequenceName : AA019459
      SequenceDescription :

Sequence
--------
<213> OrganismName : HUMAN
<400> PreSequenceString :
caatcagtaaatatttattatttctgtaactaaagtactgggctgtggtaggggagacaacaatcaaagacatagccttgtct
tccagaagctcacaatctagttgggaattcaagaataccccttagaaaagttcattgacaatttacgaggtgctattagacagt
aggagagaaaggcatacataccagggcccagggaagaagagctttgtgaatgaagaaggaaaaagtgtaagt
<212> Type : DNA
<211> Length : 240
      SequenceName : AA010251

SequenceDescription :

Sequence
--------
<213> OrganismName : HUMAN
<400> PreSequenceString :
atctgccacgatgttgctgctcagcctgaccctaagcctggttctcctcggctcctcctggggctgcggcattcctgccatcaa
accggcactgagcttcagccagaggattgtcaacggggagaatgcagtgttgggctcctggccctggcaggtgtccctgcagga
cagcagcggcttccacttctgcggtggttctctcatcagccagtcctgggtggtcactgctgcccactgcaatgtcagccctgg
ccgccatttgttgtcctgggcgagtatgaccgatcatcaaacgcagagcccttgcaggttctgtccgtctctcgggccattac
acaccctagctggaactctaccaccatgaacaatgacgtgacgctgctgaagctcgcctcgccagcccagtacacaacacgcat
ctcgccagtttgcctggcatcctcaaacgaggctctgactgaaggcctcacgtgtgtcaccaccggctggggtcgcctcagtgg
cgtgggcaatgtgacaccagcacatctgcagcaggtggctttgcccctggtcactgtgaatcagtgccggcagtactgggctc
aagtatcactgactccatgatctgtgcaggtggcgcaggtgcctcctcgtgccagggtgactccggaggccctcttgtctgcca
gaagggaaacacatgggtgcttattggtattgtctcctggggcaccaaaaactgcaatgtgcgcgcacctgctgtgtatactcg
agttagcaagttcagcacctggatcaaccaggtcatagcctacaactgagctcaccacaggccctccccagctcaacccattaa
agacccaggccctgtcccatcatgcattcatgtctgtcttcctggctcaggagaaagaagaggctgttgagggtccgactccct
acttggacttctggcacagaaggggctgagtgactccttgagtagcagtggctcttcctagagtagccatgccgaggccggggc
ccccacccctcctccagggcaaccccttggtcctacagcaagaagccagaactgttggaatgaatggcagccctccctggagag
gcagcctgtttactgaatacagaggatacgtttacaaactgaatacgcataataaataactgcacattctccatccaaaaaaa
aaaaaaa
<212> Type : DNA
<211> Length : 1184
SequenceName : X71877
SequenceDescription :

Sequence
--------
<213> OrganismName : HUMAN
<400> PreSequenceString :
cagggcagaccatgcgcccgccaagtccgctgcccgcccgctggctatgcgtgctggcaggcgccctcgcctgggcccttgggc
cggcgggcggccaggcggccaggctgcaggaggagtgtgactatgtgcagatgatcgaggtgcagcacaagcagtgcctggagg
aggcccagctggagaatgagacaataggctgcagcaagatgtgggacaacctcacctgctggccagccacccctcggggccagg
tagttgtcttggcctgtcccctcatcttcaagctcttctcctccattcaaggccgcaatgtaagccgcagctgcaccgacgaag
gctggacgcacctggagcctggcccgtaccccattgcctgtggtttggatgacaaggcagcgagtttggatgagcagcagacca
tgttctacggttctgtgaagaccggctacaccattggctacggcctgtccctcgccacccttctggtcgccacagctatcctga
gcctgttcaggaagctccactgcacgcggaactacatccacatgcacctcttcatatccttcatcctgagggctgccgctgtct
tcatcaaagacttggccctcttcgacagcggggagtcggaccagtgctccgagggctcggtgggctgtaaggcagccatggtct
tttccaatattgtgtcatggctaacttcttctggctgctggtggagggcctctacctgtacaccctgcttgccgtctccttct
tctctgagcggaagtacttctgggggtacatactcatcggctgggggtacccagcacattcaccatggtgtggaccatcgcca
ggatccattttgaggattatggtctgctcaggtgctgggacaccatcaactcctcactgtggtggatcataaagggccccatcc
tcacctccatcttggtaaacttcatcctgtttatttgcatcatccgaatcctgcttcagaaactgcggccccccagatatcagga
agagtgacagcagtccatactcaaggctagccaggtccacactcctgctgatcccccctgtttggagtacactacatcatgttcg
cccttctttccggacaattttaagcctgaagtgaagatggtctttgagctcgtcgtggggtctttccagggttttgtggtggcta
tcctctactgcttcctcaatggtgaggtgcaggcggagctgaggcggaagtggcggcgctggcacctgcagggcgtcctgggct
ggaaccccaaataccggcacccgtcgggaggcagcaacggcgccacgtgcagcacgcaggtttccatgctgacccgcgtcagcc
caggtgcccgccgctcctccagcttccaagccgaagtctccctggtctgaccaccaggatcccagcccaagcggcccctcccgc
cccttcccactcgcagcagacgccggggacagaggcctgcccgggcgcgccagccccggccctgggctcggaggctgcccccgg
ccccctggtctctggtccggacactcctagagaacgcagccctagagcctgcctggagcgtttctagcaagtgagagagatggg
agctcctctcctggaggatgcaggtggaactcagtcattagactcctcctccaaaggcccccctacgccaatcaagggcaaaaag
tctacatactttcatcctgactctgccccctgctggctcttctgcccaattggaggaaagcaaccggtggatcctcaaacaaca
ctggtgtgacctgagggcagaaaggttctgcccgggaaggtcaccagcaccaacaccacggtagtgcctgaaatttcaccattg
ctgtcaagttcctttgggttaagcattaccactcaggcatttgactgaagatgcagctcactaccctattctctctttacgctt
agttatcagcttttaaagtgggttattctggagtttttgtttggagagcacacctatcttagtggttccccaccgaagtggac
tggccccctgggtcagtctggtgggaggacggtgcaacccaaggactgagggactctgaagcctctgggaaatgagaaggcagcc
accagcgaatgctaggtctcggactaagcctacctgctctccaagtctcagtggcttcatctgtcaagtgggactctgtcacac
cagccattcttatctctctgtgctgtggaagcaacaggaatcaagagactgccctccttgtccacccacctatgtgccaactgt
tgtaactaggctcagagatgtgcacccatgggctctgacagaaagcagatcctcaccctgctacacatacaggatttgaactca
gatctgtctgataggaatgtgaaagcacggactcttactgctaactttgtgtatcgtaaccagccagatcctcttggttattt
gtttaccacttgtattattaatgccattatccctgaattccccttgccaccccaccctccctggagtgtggctgaggaggcctc
catctcatgtatcatctggataggagcctgctggtcacagcctcctctgtctgcccttcaccccagtggccactcagcttccta
cccacacctctgccagaagatcccctcaggactgcaacaggcttgtgcaacaataaatgttggcttggaaaaaaaaaaaa
<212> Type : DNA

<211> Length : 2684
SequenceName : X75299
SequenceDescription :

Sequence
--------
<213> OrganismName : HUMAN
<400> PreSequenceString :
atggcaggacaagcgtttagaaagtttcttccactctttgaccgagtattggttgaaaggagtgctgctgaaactgtaaccaaa
ggaggcattatgcttccagaaaaatctcaaggaaaagtattgcaagcaacagtagtcgctgttggatcgggttctaaaggaaag
ggtggagagattcaaccagttagcgtgaaagttggagataaagttcttctcccagaatatggaggcaccaaagtagttctagat
gacaaggattatttcctatttagagatggtgacattcttggaaagtacgtagactga
<212> Type : DNA
<211> Length : 309
SequenceName : X75821
SequenceDescription :

Sequence
--------
<213> OrganismName : HUMAN
<400> PreSequenceString :
aattcttcacctcttttctcagctccctgcagcatgggtgctgggccctccttgctgctcgccgccctcctgctgcttctctcc
ggcgacggcgccgtgcgctgcgacacacctgccaactgcacctatcttgacctgctgggcacctgggtcttccaggtgggctcc
agcggttcccagcgcgatgtcaactgctcggttatgggaccacaagaaaaaaagtagtggtgtaccttcagaagctggataca
gcatatgatgaccttggcaattctggccatttcaccatcatttacaaccaaggctttgagattgtgttgaatgactacaagtgg
tttgcctttttaagtataaagaagagggcagcaaggtgaccacttactgcaacgagacaatgactgggtgggtgcatgatgtg
ttgggccggaactgggcttgtttcaccggaaagaaggtgggaactgcctctgagaatgtgtatgtcaacacagcacaccttaag
aattctcaggaaaagtattctaataggctctacaagtatgatcacaactttgtgaaagctatcaatgccattcagaagtcttgg
actgcaactacatacatggaatatgagactcttaccctgggagatatgattaggagaagtggtggccacagtcgaaaaatccca
aggcccaaacctgcaccactgactgctgaaatacagcaaaagattttgcatttgccaacatcttgggactggagaaatgttcat
ggtatcaattttgtcagtcctgttcgaaaccaagcatcctgtggcagctgctactcatttgcttctatgggtatgctagaagcg
agaatccgtatactaaccaacaattctcagaccccaatcctaagccctcaggaggttgtgtcttgtagccagtatgctcaaggc
tgtgaaggcggcttcccataccttattgcaggaaagtacgcccaagattttgggctggtggaagaagcttgcttcccctacaca
ggcactgattctccatgcaaaatgaaggaagactgctttcgttattactcctctgagtaccactatgtaggaggtttctatgga
ggctgcaatgaagccctgatgaagcttgagttggtccatcatgggcccatggcagttgcttttgaagtatatgatgacttcctc
cactacaaaaaggggatctaccaccacactggtctaagagaccctttcaacccctttgagctgactaatcatgctgttctgctt
gtgggctatggcactgactcagcctctgggatggattactggattgttaaaaacagctggggcaccggctggggtgagaatggc
tacttccggatccgcagaggaactgatgagtgtgcaattgagagcatagcagtggcagccacaccaattcctaaattgtagggt
atgccttccagtatttcataatgatctgcatcagttgtaaaggggaattggtatattcacagactgtagactttcagcagcaat
ctcagaagcttacaaatagatttccatgaagatatttgtcttcagaattaaaactgcccttaattttaatatacctttcaatcg
gccactggccatttttttctaagtattcaattaagtgggaattttctggaagatggtcagctatgaagtaatagagtttgctta
atcatttgtaattcaaacatgctatatttttaaaatcaatgtgaaaacatagacttattttaaattgtaccaatcacaagaa
aataatggcaataattatcaaacttttaaaatagatgctcatattttaaaataaagttttaaaataactgc
<212> Type : DNA
<211> Length : 1838
SequenceName : X87212
SequenceDescription :

Sequence
--------
<213> OrganismName : HUMAN
<400> PreSequenceString :
aagagacaagaggtgccttgtgggcagatagggggctgggagggggcctgcccggaagcagtggtggcccgtggcaggcttctc
actgggtaggaccgggccctctgttgcacccctcaccctgctctctgccctcaggagtggctaagcaggttcggttacctgcc
cccggctgaccccacaacagggcagctgcagacgcaagaggagctgtctaaggccatcacagccatgcagcagtttggtggcct
ggaggccaccggcatcctggacgaggccaccctggccctgatgaaaaccccacgctgctccctgccagacctccctgtcctgac
ccaggctcgcaggagacgccaggctccagccccaccaagtggaacaagaggaacctgtcgtggagggtccggacgttcccacg
ggactcaccactggggcacgacacggtgcgtgcactcatgtactacgccctcaaggtctggagcgacattgcgcccctgaactt
ccacgaggtggcgggcagcaccgccgacatccagatcgacttctccaaggccgaccataacgacggctaccccttcgacggccc
cggcggcaccgtggcccacgccttcttccccggccaccaccacaccgccggggacacccactttgacgatgacgaggcctggac
cttccgctcctcggatgcccacgggatggacctgtttgcagtggctgtccacgagtttggccacgccattggttaagccatgt
ggccgctgcacactccatcatgcggccgtactaccagggcccggtgggtgacccgctgcgctacgggctcccctacgaggacaa
ggtgcgcgtctggcagctgtacggtgtgcgggagtctgtgtctcccacggcgcagcccgaggagcctcccctgctgccggagcc

cccagacaaccggtccagcgccccgcccaggaaggacgtgccccacagatgcagcactcactttgacgcggtggcccagatccg
gggtgaagctttcttcttcaaaggcaagtacttctggcggctgacgcgggaccggcacctggtgtccctgcagccggcacagat
gcaccgcttctggcggggcctgccgctgcacctggacagcgtggacgccgtgtacgagcgcaccagcgaccacaagatcgtctt
ctttaaaggagacaggtactgggtgttcaaggacaataacgtagaggaaggatacccgcgccccgtctccgacttcagcctccc
gcctggcggcatcgacgctgccttctcctgggcccacaatgacaggacttatttctttaaggaccagctgtactggcgctacga
tgaccacacgaggcacatggaccccggctaccccgcccagagcccccctgtggagggtgtccccagcacgctggacgacgccat
gcgctggtccgacggtgcctcctacttcttccgtggccaggagtactggaaagtgctggatggcgagctggaggtggcacccgg
gtacccacagtccacggcccgggactggctggtgtgtggagactcacaggccgatggatctgtggctgcgggcgtggacgcggc
agaggggccccgcgcccctccaggacaacatgaccagagccgctcggaggacggttacgaggtctgctcatgcacctctggggc
atcctctcccccgggggccccaggcccactggtggctgccaccatgctgctgctgctgccgccactgtcaccaggcgccctgtg
gacagcggcccaggccctgacgctatgacacacagcgcgagcccatgagaggacagaggcggtgggacagcctggccacagagg
gcaaggactgtgccggagtccctggggggaggtgctggcgcgggatgaggacgggccaccctggcaccggaaggccagcagaggg
cacggcccgccagggctgggcaggctcaggtggcaaggacggagctgtcccctagtgagggactgtgttgactgacgagccgag
gggtggccgctccagaagggtgcccagtcaggccgcaccgccgccagcctcctccggccctggagggagcatctcgggctgggg
gcccacccctctctgtgccggcgccaccaaccccacccacactgctgcctggtgctcccgccggcccacagggcctccgtcccc
aggtccccagtggggcagccctccccacagacgagcccccacatggtgccgcggcacgtccccctgtgacgcgttccagacc
aacatgacctctccctgctttgtagcggcccggaattc
<212> Type : DNA
<211> Length : 2306
      SequenceName : X89576
      SequenceDescription :

Sequence
--------
<213> OrganismName : HUMAN
<400> PreSequenceString :
cagcgagccgcagcgcaatcccggcgctcgcccaaggaccctggaagctaccgttaccccgccggcagcgtgggcgccatgagc
agctcgggactgaattcggagaaggtagctgctctgatacagaaactgaattccgacccccagttcgtacttgcccagaatgtc
gggaccacccacgacctgctggacatctgtctgaagcggccacggtgcagcgcgcgcagcatgtgttccagcacgccgtgccc
caggagggcaagccaatcaccaaccagaagagctcagggcgatgctggatcttttcttgtctgaatgttatgaggcttccattc
atgaaaaagttaaatattgaagaatttgagtttagccaatcttacctgtttttttgggacaaggttgaacgctgttatttcttc
ttgagtgcttttgtggacacagcccagagaaaggagcctgaggatgggaggctggtgcagtttttgcttatgaaccctgcaaat
gatggtggccaatgggatatgcttgttaatattgttgaaaaatatggtgttatccctaagaaatgcttccctgaatcttataca
acagaggcaaccagaaggatgaatgatattctgaatcacaagatgagagaattctgtatacgactgcggaacctggtacacagt
ggagcaaccaaaggagaaatctcggccacacaggacgtcatgatggaggagatattccgagtggtgtgcatctgtttgggtaat
ccaccagagacattcacctgggaatatcgagacaaagataaaaattatcagaaaattggccccataacacccttggagttttac
agggaacatgtcaagccactcttcaatatggaagataagatttgtttagtgaatgaccctaggccccagcacaagtacaacaaa
ctttacacagtggaatacttaagcaatatggttggtgggagaaaaactctatacaacaaccagcccattgacttcctgaaaaag
atggttgctgcctccatcaaagatggagaggctgtgtggtttggctgtgatgttggaaaacacttcaatagcaagctgggcctc
agtgacatgaatctctatgaccatgagttagtgtttggtgtctccttgaagaacatgaataaagcggagaggctgacttttggt
gagtcacttatgacccacgccatgaccttcactgctgtctcagagaaggatgatcaggatggtgctttcacaaaatggagagtg
gagaattcatggggtgaagaccatggccacaaaggttacctgtgcatgacagatgagtggttctctgagtatgtctacgaagtg
gtggtggacaggaagcatgtccctgaagaggtgctagctgtgttagagcaggaacccattatcctgccagcatgggaccccatg
ggagctttggctgagtgatactgccctccagctctttcctccttccatggaacctgacgtagctgcaaaggacagatccaggga
ctgaagccaaagttatgcaagggactgtgtgttgccacaggacacagtcagatttccagtctccaccaggaacctcttcagaaa
gtgtgctttatgctgaaacagaatactgttaaaggaaaaaaagagggggaagatcaggtcatactatctactctcctcatct
ctaacagctcaggatctcttagcattttaattagatgtaattgtttgtctttaactgtcaaaaagtttggttctgtgtctgtgt
tttaataagacgagaggacgagcgattgaggtgtatggagagaaaacagacctaatgctccttgttcctagagtagagtggagg
gagggtggcctaagagttgagctctcggaactgcatgctgctggacagtatcactgtctttcctagatggcagtcactgaattc
<212> Type : DNA
<211> Length : 1932
      SequenceName : X92106
      SequenceDescription :

Sequence
--------
<213> OrganismName : HUMAN
<400> PreSequenceString :
cggcacgagggtgctgcttgtgtgctcgtttggtgcggacctggtacctcttyttgtgaagcggcagctgaggagactccggcg
ctcgccatggccgacgaaaagcccaaggaaggagtcaagactgagaacaacgatcatattaatttgaaggtggcggggcaggat
ggttctgtggtgcagtttaagattaagaggcatacaccacttagtaaactaatgaaagcctattgtgaacgacagggattgtca
atgaggcagatcagattccgatttgacgggcaaccaatcaatgaaacagacacacctgcacagttggaaatggaggatgaagat
acaattgatgtgttccaacagcagacgggaggtgtctactgaaaagggaacctgcttctttactccagaactctgttctttaaa

gaccaagattacattctcaattagaaaactgcaatttggttccaccacatcctgactactaccgtatagtttctctattcttt
catttccccttccccattcctttattgtacataaagtaactggtatatgtgcacaagcatattgcatttttttttttttaac
taaacagccaatggtatgttttgattgacatccaagtggagacggggatggggaaaaatactgattctgtggaaaataccccc
tttctcccattagtggncatgctccattcagcccttaaacctttataatcccaggtaaggtaatttngcccncaccggttttac
ccaaaaaaaaaaaactt
<212> Type : DNA
<211> Length : 774
      SequenceName : X99585
      SequenceDescription :

Sequence
--------
<213> OrganismName : HUMAN
<400> PreSequenceString :
gtgtactattagccatggtcaaccccaccgtgttcttcgacattgccgtcgacggcgagcccttgggccgcgtctcctttgagc
tgtttgcagacaaggtcccaaagacagcagaaaattttcgtgctctgagcactggagagaaaggatttggttataagggttcct
gctttcacagaattattccagggtttatgtgtcagggtggtgacttcacacgccataatggcactggtggcaagtccatctatg
gggagaaatttgaagatgagaacttcatcctaaagcatacgggtcctggcatcttgtccatggcaaatgctggacccaacacaa
atggttcccagtttttcatctgcactgccaagactgagtggttggatggcaagcatgtggtgtttggcaaagtgaaagaaggca
tgaatattgtggaggccatggagcgctttgggtccaggaatggcaagaccagcaagaagatcaccattgctgactgtggacaac
tcgaataagtttgacttgtgttttatcttaaccaccagatcattccttctgtagctcaggagagcacccctccaccccatttgc
tcgcagtatcctagaatctttgtgctctcgctgcagttccctttgggttccatgttttccttgttccctcccatgcctagctgg
attgcagagttaagtttatgattatgaaataaaaactaaataacaattgtc
<212> Type : DNA
<211> Length : 723
      SequenceName : Y00052
      SequenceDescription :

Sequence
--------
<213> OrganismName : HUMAN
<400> PreSequenceString :
gctctttccaacgctgtaaaaaaggacagaggctgttccctatggcagaaggcaaccacagaaaaaagccacttaaggtgttgg
aatccctgggcaaagatttcctcactggtgttttggataacttggtggaacaaaatgtactgaactggaaggaagaggaaaaaa
agaaatattacgatgctaaaactgaagacaaagttcgggtcatggcagactctatgcaagagaagcaacgtatggcaggacaaa
tgcttcttcaaacctttttaacatagaccaaatatcccccaataaaaagctcatccgaatatggaggctggaccacctgagt
caggagaatctacagatgccctcaagctttgtcctcatgaagaattcctgagactatgtaaagaaagagctgaagagatctatc
caataaaggagagaaacaaccgcacacgcctggctctcatcatatgcaatacagagtttgaccatctgcctccgaggaatggag
ctgactttgacatcacagggatgaaggagctacttgagggtctggactatagtgtagatgtagaagagaatctgacagccaggg
atatggagtcagcgctgagggcatttgctaccagaccagagcacaagtcctctgacagcacattcttggtactcatgtctcatg
gcatcctggagggaatctgcggaactgtgcatgatgagaaaaaaccagatgtgctgctttatgacaccatcttccagatattca
acaaccgcaactgcctcagtctgaaggacaaacccaaggtcatcattgtccaggcctgcagaggtgcaaaccgtggggaactgt
gggtcagagactctccagcatccttggaagtggcctcttcacagtcatctgagaacctggaggaagatgctgtttacaagaccc
acgtggagaaggacttcattgctttctgctcttcaacgccacacaacgtgtcctggagagacagcacaatgggctctatcttca
tcacacaactcatcacatgcttccagaaatattcttggtgctgccacctagaggaagtatttcggaaggtacagcaatcatttg
aaactccaagggccaaagctcaaatgcccaccatagaacgactgtccatgacaagatatttctacctctttcctggcaattgaa
aatggaagccacaagcagcccagccctccttaatcaacttcaaggagcaccttcattagtacagcttgcatatttaacattttg
tatttcaataaaagtgaagacaaaaaaaaaa
<212> Type : DNA
<211> Length : 1291
      SequenceName : Z48810
      SequenceDescription :

Sequence
--------
<213> OrganismName : HUMAN
<400> PreSequenceString :
ccgccgccgcgcatggctgcctcggaacgagtaagaccgaggaccagcgcaacgaggagaaggcgcagcgtgaggccaacaaa
aagatcgagaagcagctgcagaaggacaagcaggtctaccgggccacgcaccgcctgctgctgctgggtgctggagaatctggt
aaaagcaccattgtgaagcagatgaggatcctgcatgttaatgggtttaatggagagggcggcgaagaggacccgcaggctgca
aggagcaacagcgatggtgagaaggcaaccaaagtgcaggacatcaaaaacaacctgaaagaggcgattgaaaccattgtggcc
gccatgagcaacctggtgccccccgtggagctggccaaccccgagaaccagttcagagtggactacatcctgagtgtgatgaac
gtgcctgactttgacttccctcccgaattctatgagcatgccaaggctctgtgggaggatgaaggagtgcgtgcctgctacgaa

cgctccaacgagtaccagctgattgactgtgcccagtacttcctggacaagatcgacgtgatcaagcaggctgactatgtgccg
agcgatcaggacctgcttcgctgccgtgtcctgacttctggaatctttgagaccaagttccaggtggacaaagtcaacttccac
atgtttgacgtgggtggccagcgcgatgaacgccgcaagtggatccagtgcttcaacgatgtgactgccatcatcttcgtggtg
gccagcagcagctacaacatggtcatccgggaggacaaccagaccaaccgcctgcaggaggctctgaacctcttcaagagcatc
tggaacaacagatggctgcgcaccatctctgtgatcctgttcctcaacaagcaagatctgctcgctgagaaagtccttgctggg
aaatcgaagattgaggactactttccagaatttgctcgctacactactcctgaggatgctactcccgagcccggagaggaccca
cgcgtgacccgggccaagtacttcattcgagatgagtttctgaggatcagcactgccagtggagatgggcgtcactactgctac
cctcatttcacctgcgctgtggacactgagaacatccgccgtgtgttcaacgactgccgtgacatcattcagcgcatgcacctt
cgtcagtacgagctgctctaagaaggaacccccaaatttaattaaagccttaagcacaattaattaaaagtgaaacgtaattgt
acaagcagttaatcacccaccatagggcatgattaacaaagcaacctttcccttcccccgagtgattttgcgaaaccccctttt
cccttcagcttgcttagatgttccaaatttagaagcttaaggcggcctacagaaaaggaaaaaggccacaaaagttccctct
cactttcagtaaaaataaataaaacagcagcagcaaacaaataaaatgaaataaaagaaacaaatgaaataaatattgtgttgt
gcagcattaaaaaaatcaaaataaaaattaaatgtgagc
<212> Type : DNA
<211> Length : 1552
      SequenceName : X07036
      SequenceDescription :

Sequence
--------
<213> OrganismName : HUMAN
<400> PreSequenceString :
ttgctgaaataaaacacaagtatctctggtcagagcctcagaattgctcagccaccaaaagtaactaccttcgaggtactggtc
cctacccaccttccgtggactggcggaaaaaggaaattttgtctcacctgtgaaaaatcagggtgcctgcggcagttgctgga
ctttctccaccactggggccctggagtctgcaatcgccatcgcaaccggaaagatgctgtccttggcggaacagcagctggtgg
actgcgcccaggacttcaataattacggctgccaaggggggtctccccagccaggctttcgagtatatcctgtacaacaagggga
tcatgggtgaagacacctacccctaccagggcaaggatggttattgcaagttccaacctggaaaggccatcggctttgtcaagg
atgtagccaacatcacaatctatgacgaggaagcgatggtggaggctgtggccctctacaaccctgtgagctttgcctttgagg
tgactcaggacttcatgatgtatagaacgggcatctactccagtacttcctgccataaaactccagataaagtaaaccatgcag
tactggctgttgggtatggagaaaaaaatgggatcccttactggatcgtgaaaaactcttggggtccccagtggggaatgaacg
ggtacttcctcatcgagcgcggaaagaacatgtgtggcctggctgcctgcgcctcctaccccatccctctggtgtgagccgtgg
cagccgcagcgcagactggcggagaaggagaggaacgggcagcctgggcctgggtggaaatcctgccctggaggaagttgtggg
gagatccactgggacccccaacattctgccctcacctctgtgcccagcctggaaacctacagacaaggaggagttccaccatga
gctcacccgtgtctatgacgcaaagatcaccagccatgtgccttagtgtccttcttaacagactcaaaccacatggaccacgaa
tattctttctgtccagaagggctactttccacatatagagctccagggactgtcttttctgtattcgctgttcaataaacattg
agtgagcacctcca
<212> Type : DNA
<211> Length : 1106
      SequenceName : X07549
      SequenceDescription :

Sequence
--------
<213> OrganismName : HUMAN
<400> PreSequenceString :
ctaacccagaaacatccaattctcaaactgaagctcgcactctcgcctccagcatgaaagtctctgccgcccttctgtgcctgc
tgctcatagcagccaccttcattccccaagggctcgctcagccagatgcaatcaatgccccagtcacctgctgttataacttca
ccaataggaagatctcagtgcagaggctcgcgagctatagaagaatcaccagcagcaagtgtcccaaagaagctgtgatcttca
agaccattgtggccaaggagatctgtgctgaccccaagcagaagtgggttcaggattccatggaccacctggacaagcaaaccc
aaactccgaagacttgaacactcactccacaacccaagaatctgcagctaacttattttcccctagctttccccagacaccctg
ttttattttattataatgaattttgtttgttgatgtgaaacattatgccttaagtaatgttaattcttatttaagttattgatg
ttttaagtttatctttcatggtactagtgtttttagatacagagacttggggaaattgcttttcctcttgaaccacagttcta
cccctgggatgttttgagggtctttgcaagaatcattaatacaaagaatttttttaacattccaatgcattgctaaaatatta
ttgtggaaatgaatattttgtaactattacaccaaataaatatatttttgtac
<212> Type : DNA
<211> Length : 725
      SequenceName : X14768
      SequenceDescription :

Sequence
--------
<213> OrganismName : HUMAN
<400> PreSequenceString :

acgtgtatcgctgccgtcaagatggaggggcctttgtccgtgttcggtgaccgcagcactggggaaacgatccgctcccaaaac
gttatggctgcagcttcgattgccaatattgtaaaagttctcttggtccagttggcttggataaaatgttggtggatgatatt
ggtgatgtaaccattactaacgatggtgcaaccatcctgaagttactggaggtagaacatcctgcagctaaagttctttgtgag
ctggctgatctgcaagacaaagaagttggagatggaactacttcagtggttattattgcagcagaactcctaaaaaatgcagat
gaattagtcaaacagaaaattcatcccacatcagttattagtggctatcgacttgcttgcaaggaagcagtgcgttatatcaat
gaaaacctaattgttaacacagatgaactgggaagagattgcctgattaatgctgctaagacatccatgtcttccaaaatcatt
gggataaatggtgatttctttgctaacatggtagtagatgctgtacttgctattaaatacacagacataagaggccagccacgc
tatccagtcaactctgttaatattttgaaagcccatgggagaagtcaaatggagagtatgctcatcagtggctatgcactcaac
tgtgtggtgggatcccagggcatgcccaagagaatcgtaaatgcaaaaattgcttgccttgacttcagcctgcaaaaaacaaaa
atgaagcttggtgtacaggtggtcattacagaccctgaaaaactggaccaaattagacagagagaatcagatatcaccaaggag
agaattcagaagatcctggcaactggtgccaatgttattctaaccactggtggaattgatgatatgtgtctgaagtattttgtg
gaggctggtgctatggcagttagaagagttttaaaaaggaccttaaacgcattgccaaagcttctggagcaactattctgtca
accctggccaatttggaaggtgaagaaacttttgaagctgcaatgttgggacaggcagaagaagtggtacaggagagaatttgt
gatgatgagctgatcttaatcaaaaatactaaggctcgtacgtctgcatcgattatcttacgtggggcaaatgatttcatgtgt
gatgagatggagcgctctttacatgatgcactttgtgtagtgaagagagttttggagtcaaaatctgtggttcccggtggggt
gctgtagaagcagccctttccatataccttgaaaactatgcaaccagcatggggtctcgggaacagcttgcgattgcagagttt
gcaagatcacttcttgttattcccaatacactagcagttaatgctgcccaggactccacagatctggttgcaaaattaagagct
tttcataatgaggcccaggttaacccagaacgtaaaaatctaaaatggattggtcttgatttgagcaatggtaaacctcgagac
aacaaacaagcaggggtgtttgaaccaaccatagttaaagttaagagtttgaaatttgcaacagaagctgcaatcaccattctt
cgaattgatgatcttattaaattacatccagaaatccttcggattaaacatggaagttatgaagatgctgttcactctggagcc
cttaatgattgatctgatgttccttttatttataacaatgttaaatgcaatgtcttgtaccttgagttgagtattacacattaa
agtaaagtacaagctgtaaacttgggttttgtgatgtaggaaatggtttccatctgtactttggtcctctgatttcagatatt
gcaacctagtactttattagtttaaaaagaaattgaggttgttcaaagtttaagcaattcattctctctgaacacacattgcta
ttcccatcccacccccaatgcacagggctgcaacaccacgacttctgcccattctctccagtgtgtgtaacagggtcacaagaa
ttc
<212> Type : DNA
<211> Length : 2019
      SequenceName : X52882
      SequenceDescription :

Sequence
--------
<213> OrganismName : HUMAN
<400> PreSequenceString :
gaattcgggtgagccagctccgggagaacagcatgtacaccagcctcagtgttacagagtgtgggtacatcaaggtgaatggtg
agcagaaactataacctgttagtccttctacacctcatctgctacaagttctggcttagacatggatattctttgtgaagaaaa
tacttctttgagctcaactacgaactccctaatgcaattaaatgatgacaccaggctctacagtaatgactttaactctggaga
agctaacacttctgatgcatttaactggacagtcgactctgaaaatcgaaccaacctttcctgtgaagggtgcctctcaccgtc
gtgtctctccttacttcatctccaggaaaaaaactggtctgctttactgacagccgtagtgattattctaactattgctggaaa
catactcgtcatcatggcagtgtccctagagaaaaagctgcagaatgccaccaactatttcctgatgtcacttgccatagctga
tatgctgctgggtttccttgtcatgcccgtgtccatgttaaccatcctgtatgggtaccggtggcctctgccgagcaagctttg
tgcagtctggatttacctggacgtgctcttctccacggcctccatcatgcacctctgcgccatctcgctggaccgctacgtcgc
catccagaatcccatccaccacagccgcttcaactccagaactaaggcatttctgaaaatcattgctgtttggaccatatcagt
aggtatatccatgccaataccagtctttgggctacaggacgattcgaaggtctttaaggaggggagttgcttactcgccgatga
taactttgtcctgatcggctcttttgtgtcattttcattcccttaaccatcatggtgatcacctactttctaactatcaagtc
actccagaaagaagctactttgtgtgtaagtgatcttggcacacgggccaaattagcttctttcagcttcctccctcagagttc
tttgtcttcagaaaagctcttccagcggtcgatccatagggagccagggtcctacacaggcaggaggactatgcagtccatcag
caatgagcaaaaggcatgcaaggtgctgggcatcgtcttcttcctgtttgtggtgatgtggtgccctttcttcatcacaaacat
catggccgtcatctgcaaagagtcctgcaatgaggatgtcattggggccctgctcaatgtgtttgtttggatcggttatctctc
ttcagcagtcaacccactagtctacacactgttcaacaagacctataggtcagccttttcacggtatattcagtgtcagtacaa
ggaaaacaaaaaaccattgcagttaatttagtgaacacaataccggctttggcctacaagtctagccaacttcaaatgggaca
aaaaaagaattcaaagcaagatgccaagacaacagataatgactgctcaatggttgctctaggaaagcagcattctgaagaggc
ttctaaagacaatagcgacggagtgaatgaaaaggtgagctgtgtgtgataggctagttgccgtggcaactgtggaaggcacac
tgagcaagttttcacctatctggaaaaaaaaaatatgagattggaaaaaattagacaagtctagtggaaccaacgatcatatct
gtatgcctcatttattctgtcaatgaaaagcggggttcaatgctacaaaatgtgtgcttggaaaatgttctgacagcatttca
gctgtgagctttctgatacttatttataacattgtaaatgatatgtctttaaaatgattcacttttattgtataattatgaagc
cctaagtaaatctaaattaacttctattttcaagtggaaaccttgctgctatgctgttcattgatgacatgggattgagttggt
tacctattgccgtaaataaaaatagctataaatagtgaaaattttattgaatataatggcctcttaaaaattatctttaaaact
tactatggtatatatttgaaaggagaaaaaaaaaaagccactaaggtcagtgttataaaatctgtattgctaagataattaaa
tgaaatacttgacaacattttcatagataccatttgaaatattcacaaggttgctggcatttgctgcatttcaagttaattc
tcagaagtgaaaaagacttcaaatgttattcaataactattgctgctttctcttctacttcttgtgctttactctgaatttcca
gtgtggtcttgtttaatatttgttcctctaggtaaactagcaaaaggatgatttaacattaccaaatgcctttctagcaattgc
ttctctaaaacagcactatcgaggtatttggtaacttgctgtgaaatgactgcatcatgcatgcactcttttgagcagtaaatg

tacattgatgtaactgtgtcaggattgaggatgaactcaggtttccggctactgacagtggtagagtcctaggacatctctgta
aaaagcaggtgactttcctatgacactcatcaggtaaactgatgctttcagatccatcggtttatactatttattaaaaccatt
ctgcttggttccacaatcatctattgagtgtacatttatgtgtgaagcaaatttctagatatgagaaatataaaaataattaaa
acaaaatccttgccttcaaacgaaatggctcggccaggcacggaggctcgtgcatgtaatcctagcactttgggaggctgagat
gggaggatcacttgaggccaagagtttgagaccaacctgggtaacaaagtgagacctccctgtctctacaaaaaaaatcaaaa
attatctgatccttgtggcacacaactgtggtcccagctacaggggaggctgagacgcaaggatcacttgagcccagaagctca
aggctgcagtgagccaagttcacaccactgccatttcctcctgggcaacagagtgagaccctatcaccccgaattc
<212> Type : DNA
<211> Length : 3016
      SequenceName : X57830
      SequenceDescription :

Sequence
--------
<213> OrganismName : HUMAN
<400> PreSequenceString :
tcaagctcgccccgcgcagcccgagccgggctgggcgctgtcctcgggggcctggggaaccgcgcggtttggagatcggaggca
cctggaacccgtggcaagcgccgagccgggagacagcccgaggaaccacgggttctggagctaggagccggaagctgggagtcc
ggaggagagcggagcccggagcccggagcccggggcggcgcgtctgggtctggcgcttcccgactggacggcgcgcccgctggt
cttcgccacgcgccctcccctgggctcgcgttcatcggtccccgcctgagacgcgcccactcctgcccggacttccagccccgg
aggcgccggacagagccgcggactccagcgcccaccatgcgcctcaacagctccgcgccgggaaccccgggcacgccggccgcc
gaccccttccagcgggcgcaggccggactggaggaggcgctgctggccccgggcttcggcaacgcttcgggcaacgcgtcggag
cgcgtcctggcggcacccagcagcgagctggacgtgaacaccgacatctactccaaagtgctggtgaccgccgtgtacctggcg
ctcttcgtggtgggcacggtgggcaacacggtgacggcgttcacgctggcgcggaagaagtcgctgcagagcctgcagagcacg
gtgcattaccacctgggcagcctggcgctgtccgacctgctcaccctgctgctggccatgcccgtggagctgtacaacttcatc
tgggtgcaccacccctgggccttcggcgacgccggctgccgcggctactacttcctgcgcgacgcctgcacctacgccacggcc
ctcaacgtggccagcctgagtgtggagcgctacctggccatctgccaccccttcaaggccaagaccctcatgtcccgaagccgc
accaagaagttcatcagcgccatctggctcgcctcggccctgctgacggtgcctatgctgttcaccatgggcgagcagaaccgc
agcgccgacggccagcacgccggcggcctggtgtgcacccccaccatccacactgccaccgtcaaggtcgtcatacaggtcaac
accttcatgtccttcatattccccatggtggtcatctcggtcctgaacaccatcatcgccaacaagctgaccgtcatggtacgc
caggcggccgagcagggccaagtgtgcacggtcgggggcgagcacagcacattcagcatggccatcgagcctggcagggtccag
gccctgcggcacggcgtgcgcgtcctacgtgcagtggtcatcgcctttgtggtctgctggctgccctaccacgtgcggcgcctc
atgttctgctacatctcggatgagcagtggactccgttcctctatgacttctaccactacttctacatggtgaccaacgcactc
ttctacgtcagctccaccatcaaccccatcctgtacaacctcgtctctgccaacttccgccacatcttcctggccacactggcc
tgcctctgcccggtgtggcggcgcaggaggaagaggccagccttctcgaggaaggccgacagcgtgtccagcaaccacaccctc
tccagcaatgccacccgcgagacgctgtactaggctgtgcgccccggaacgtgtccaggaggagcctggccatgggtccttgcc
cccgacagacagagcagccccccacccgggagccttgatgggggtcaggcagaggccagcctgcactggagtctgaggcctggga
cccccccctcccacccccctaacccatgtttctcattagtgtctcccgggcctgtccccaactcctccccacccctcccccatct
cctctttgaaagccagaacaagagagcgctcctctcccagataggaaaagggcctctaacaaggagaaattagtgtgcggcaaa
aggcagtttctttgttctcagactaatggatggttccagagaaggaaatgaaatgtgctgggtgggccgggcctccggcggc
ccggctgctgttcccatgtccacatctctgaggcctgcacccctctgtctagctcggggagtccagccccagtcccgcaggct
ccgtggctttgggcctcacgtgcagaccctgccatgcagacccatgccccctcccccaggcagctccaagaaagctccctgac
tcgccccttcaggcctggcaagctgggggcccatcgccgtggggagtccctcccaccaccctcgccgcaggcagctgcagcccc
cagaggggaccacaagcccaaaaaggacaaaaatgggctggcctggaatggcccagaccccagcctcccctcctccctcccatc
ctcacccaggccaaggcccaggggctctgccaggacaccacatgggaggggggctcaggcctcagcctcaagatcttcagctgtg
gcctctcgggctcggcagaagggacgccggatcaggggcctggtctccagcacctgcccgagtggccgtggccaggatggggtg
cgcattccgtgtgctttgcttgtagctgtgcaggctgaggtctggagccaggcccagagctggcttcagggtggggccttgaga
aggggaatgtgggacaggggcgatggtgcctggtctctgagtaagatgccaggtcccaggaactcaggcttcaggtgagaagga
gcggtgtgtccaggcaccgctggccggcagccctgggctgaggcacagactcatttgtcaccttctggcggcggcagccctggc
cccggcctccaagcagttgaaaaagctggcgcctccttggtctctaggatccaggctccacagagcacatgactagccaggccc
ctggcttaagaaggtcgcctaagcctaagagaagacagtcccaggagaagctggccgggaccagccaggagctgggagccacag
gaagcaaaagtcagccttttcttcaagggatttccctgtctcagagcagcctttgccccagggaaatgggctctgggctggctg
cctgcaccggccatgtcgacccaggacccggacacctggtcttgggctgtgttcagccactttgccttct[?]tggactcagtttc
cccgtctgagaaatgagagtcgaatgctacagtatctgcagtcgcttggatctggctgttgagttgacgggttccttgaacccc
acaaaatccctctccaaccacaggacccttcggctcaccaagaacggggcccaggggagtcaggcctattcgctgcacttcctg
ccaaactttgccccccacaagcctggtcatcagccaggcagccctcccagtgcccaagggccaccaaccccagggaaacaggcc
agcacagaggggccttcctcccccacagagctcccatgacatagtctgctctgggcggaagagctttgctgccagccagggatg
tccagaggtcggtgcagcccctatccctgctcaggagtggctcagagtctagcaaatgctaaggcccctcaggctgggctctg
aacgaggacctggactcagagccagacagggcagcctcagacccttctctggggctcctggaccttgggccataatttctgagc
ctcggtttccccatctaaggaacagatgtggtcgttccgccctctcagctggatgagactgtcctggaggatccaccccggaac
agacagaacggtgtctctcaggatggtgctctgagagagggcagagtggatgccccactgccctagaccctcggtagacgtggg
gtctctggggcggggtctgtggctgtgactgaagtcggctttcccgttgatgtcttgatgctcctatctgtgcacttaccgtag
gtagggacacgtgtccatgcaccacagacacacccacgacacctgatctcgtatcactagcttgcggccaggtcatgatgtggc

cccggaagctggccctgcgtgccatgagtgcgtcggtcatggagtccggagcccctgagccggcccctggtgacggcacagccc
tcacagctcaaacgcccacccccactcccaccatctgcaggtggtgaaaacaaaccccgtgtatctctcaataaaggtggccga
agggcctcgatgtgg
<212> Type : DNA
<211> Length : 4131
      SequenceName : X70070
      SequenceDescription :

Sequence
--------
<213> OrganismName : HUMAN
<400> PreSequenceString :
tgaagtctcttcccaagcaaatgggagcttctttggaccttggagcacacagaggattctactttctttaaactttgttttca
ggcaatttccctgagaaccgtttacttccagaagattggtggagcttgatctgaaggctggccatgaaatctcaaggtcaacat
tggtattccagttcagataaaaactgtaaagtgagctttcgtgagaagcttctgattattgattcaaacctggggtccaagat
gtggagaacctcaagtttctctgcataggattggtccccaacaagaagctggagaagtccagctcagcctcagatgtttttgaa
catctcttggcagaggatctgctgagtgaggaagaccctttcttcctggcagaactcctctatatcatacggcagaagaagctg
ctgcagcacctcaactgtaccaaagaggaagtggagcgactgctgcccacccgacaaagggtttctctgtttagaaacctgctc
tacgaactgtcagaaggcattgactcagagaacttaaaggacatgatcttccttctgaaagactcgcttcccaaaactgaaatg
acctccctaagtttcctggcatttctagagaaacaaggtaaaatagatgaagataatctgacatgcctggaggacctctgcaaa
acagttgtacctaaacttttgagaaacatagagaaatacaaaagagagaaagctatccagatagtgacacctcctgtagacaag
gaagccgagtcgtatcaaggagaggaagaactagtttcccaaacagatgttaagacattcttggaagccttaccgagggcagct
gtgtacaggatgaatcggaaccacagaggcctctgtgtcattgtcaacaaccacagctttacctccctgaaggacagacaagga
acccataaagatgctgagatcctgagtcatgtgttccagtggcttgggttcacagtgcatatacacaataatgtgacgaaagtg
gaaatggagatggtcctgcagaagcagaagtgcaatccagcccatgccgacggggactgcttcgtgttctgtattctgacccat
gggagatttggagctgtctactcttcggatgaggccctcattcccattcgggagatcatgtctcacttcacagccctgcagtgc
cctagactggctgaaaaacctaaactctttttcatccaggcctgccaaggtgaagagatacagccttccgtatccatcgaagca
gatgctctgaaccctgagcaggcacccacttccctgcaggacagtattcctgccgaggctgacttcctacttggtctggccact
gtcccaggctatgtatcctttcggcatgtggaggaaggcagctggtatattcagtctctgtgtaatcatctgaagaaattggtc
ccaagacatgaagacatcttatccatcctcactgctgtcaacgatgatgtgagtcgaagagtggacaaacagggaacaaagaaa
cagatgccccagcctgctttcacactaaggaaaaaactagtattccctgtgcccctggatgcactttcaatatagcagagagtt
tttgttggttcttagacctcaaacgaatcattgggtataacctccagcctcctgcccagcacaggaatcggtggtctccacctg
tcattctagaaacaggaaacaccgtgttttctgacacagtcaattctgatttctttttcttttgcaagtctaaatgttagaaa
actttctttttttggagatagtctcattctgtcacccagactggagtgcagggggcaatcacggctcactgtagtctcgacct
cccaggctcaagctgtcctcccacctcagcctcccaagtagctgagactacaggtgtgtgtccatgcacagctaacttttttatt
tttttgtggagatggggtttcactatgttgcctaagctggtctcaaactcctgggctcaagcgatcctcccacctcagcttct
caaagttctgggactacaggcatgaaatactgtgcctggcctggggaccaggtgcatttaaggttccttggtgttcaaaaacc
acgttcttagcctagattgagcttagattgcctctctagacaactaccccttagttataattctgtgtcccctctgcatgccct
taaacattggacagtgaggtcacagtccacccaccctctctctgatctcccccttcctaagacttctcttttgcacatctagtg
aggtgaaaatttggtctatgccaggcccatttcctgcttttgtgtaaggaaggtgctcacataggaagtttttatttggttaga
gacaggtttccctgtaggaagatgatggctcatttacactcagctgctctgcaagcagaaactttacaacctgatgtcatattc
cattttggactgggtgcggtgactcatgcctgtaatcccagtactctgggaagccaaggcaggcagatcacttgaggtcaggag
ttcgagaccagcctggccaatacggcaaaacctcatcattactaaaaacacaaaaattagccaggtgtggcggcgagcacctgt
aatcccagctactcgggaggctgagacaggagaatctcttgaatccaggaggcagaggctgtggtgagccaagatgacacaact
gcactccagcttgggcaacagggcgagaccttgtttaaaaaaaaaattcaatattggggttggaacatttcagttgccattgac
agaacacccaattcaaattgactgaagcaaagaagggaatttattgcctctttcacattgaaacccaggagtggataacactgg
cttcaggcaaagcttgaatcaggactcaatctacaggccagcacctttctcttggccggatgtcctcagggctggcagatgcag
tagactgcagtggacagtccccaccttgttactgctactacactttgctcctctggcccaaggcatgaggagagaggctgtgtc
agaaactgaagctgttctcaggatcactgggctcttcttggcagaggggatgtctggcttgcctgaagggagtggctctgtaag
gacgccttgatgctttcttcattaagatttgagcatttttacgtacttgagcttttttttttttttttcaatttctagagg
aactttttctctgttaattcctggaactgtattttgaatccttaaaggtgagccctcatagggagatccaaagtcctgtggtta
acgccttcatttatagatgaggcagctgaggcctggggatgtgaacaacctgctcacagtcctcatttactggatttgacttca
gccaggtgaactggaatgccttggggcgtggaagggcattaggagtgtttcatttgatatgtgaatgctcataaaaaaatgtca
aggaatgaagaacaacaactctcagtggtgcctgcatttataattatttatgtgaaagtcaaattcatgtacagtaaatttgtt
ataagaat
<212> Type : DNA
<211> Length : 3536
      SequenceName : U60519
      SequenceDescription :

Sequence
--------
<213> OrganismName : HUMAN

<400> PreSequenceString :
cacgaggagaacagaagaagagaaagctcagcaaattttcttgccatacttcatgacttcactgtggctaagtgtggggaccag
acaggactcgtggagacatccaggtgctgaagccttcagctactgtctcagtttttgaagtttagcaatggcgtctttctctg
ctgagaccaattcaactgacctactctcacagccatggaatgagccccagtaattctctccatggtcattctcagccttactt
ttttactgggattgccaggcaatgggctggtgctgtgggtggctggcctgaagatgcagcggacagtgaacacaatttggttcc
tccacctcaccttggcggacctcctctgctgcctctccttgcccttctcgctggctcacttggctctccagggacagtggccct
acggcaggttcctatgcaagctcatccccctccatcattgtcctcaacatgtttgccagtgtcttcctgcttactgccattagcc
tggatcgctgtcttgtggtattcaagccaatctggtgtcagaatcatcgcaatgtagggatggcctgctctatctgtggatgta
tctgggtggtggcttgtgtgatgtgcattcctgtgttcgtgtaccgggaaatcttcactacagacaaccataatagatgtggct
acaaatttggtctctccagctcattagattatccagacttttatggagatccactagaaaacaggtctcttgaaaacattgttc
agccgcctggagaaatgaatgataggttagatccttcctctttccaaacaaatgatcatccttggacagtccccactgtcttcc
aacctcaaacatttcaaagaccttctgcagattcactccctaggggttctgctaggttaacaagtcaaaatctgtattctaatg
tatttaaacctgctgatgtggtctcacctaaaatccccagtgggtttcctattgaagatcacgaaaccagcccactggataact
ctgatgcttttctctctactcatttaaagctgttccctagcgcttctagcaattccttctacgagtctgagctaccacaaggtt
tccaggattattacaatttaggccaattcacagatgacgatcaagtgccaacacccctcgtggcaataacgatcactaggctag
tggtgggtttcctgctgccctctgttatcatgatagcctgttacagcttcattgtcttccgaatgcaaaggggccgcttcgcca
agtctcagagcaaaacctttcgagtggccgtggtggtggtggctgtctttcttgtctgctggactccataccacattttggag
tcctgtcattgcttactgacccagaaactcccttggggaaaactctgatgtcctgggatcatgtatgcattgctctagcatctg
ccaatagttgctttaatcccttcctttatgccctcttggggaaagatttaggaagaaagcaaggcagtccattcagggaattc
tggaggcagccttcagtgaggagctcacacgttccacccactgtccctcaaacaatgtcatttcagaaagaaatagtacaactg
tgtgaaaatgtggagcagccaacaagcaggggctcttaggcaatcacatagtgaaagtttataagaggatgaagtgatatggtg
agcagcggacttcaaaaactgtcaaagaatcaatccagcggttctcaaacggtacacagactattgacatcagcatcacctaga
aacttgttagaaatgcaaattctcaagccgcatcccagacttgctgaatcggaatctctggggggttgggacccagcaagggcac
ttaacaaaccccgtttctgattaatgctaaatgtaagaatcattgtaaacattagttctatttctatcccaaactaagctatg
tgaaataagagaagctactttgtttttaaatgatgttgaatatttgtcgatatttccatcattaaattttccttagcattgtc
taagtcaaaaaaaaaaaaaaaaaa
<212> Type : DNA
<211> Length : 2040
      SequenceName : U62027
      SequenceDescription :

Sequence
--------
<213> OrganismName : HUMAN
<400> PreSequenceString :
gagccagcgagccagcgcgcgcgggcgggcggacagatcggagccgagcggggccgggcggggcgctccctgcagggctctgcg
cggcgtgccgcggcggccgcgggctccggccccgggccatgagcccctccgcgactcggcgctgagcccgccaccggtccagcg
ccccaggacccgccgccggctgccggcttgccgaagcccccctcaggatcccctcaacaaggatggaactgaaggccgaggagga
ggaggtgggtggcgtccagccggtgagcatccaggccttcgccagcagctccacactgcacggcctggcccacatcttctccta
cgagcggctgtctctgaagcgggcactgtgggccctgtgcttcctgggctcgctggctgtgctgctgtgtgtgtgcacggagcg
tgtgcagtactacttccactaccaccatgtcaccaagctcgacgaggtggctgcctctcagcttaccttccctgctgtcacgct
gtgcaacctcaacgagttccgctttagccaagtctccaagaatgacctgtatcatgctggggagctgctggccctgctcaacaa
caggtatgagataccagacacacagatggcagatgaaaagcagctggagatactgcaggacaaagccaacttccgcagcttcaa
acccaaacccttcaacatgcgtgagttctacgaccgagctgggcacgacattcgagacatgctgctctcctgccacttccgggg
ggaggtctgcagcgctgaagacttcaaggtggtcttcacacgctatggaaagtgctacacgttcaactcgggccgagatgggcg
gccgcggctgaagaccatgaaggatgggacggcaatgggctggaaatcatgctggacatccagcaggacgagtacctgcctgt
gtgggggggagactgacgagacgtccttcgaagcaggcatcaaagtgcagatccatagtcaggatgaacctcctttcatcgacca
gctgggctttggcgtggccccaggcttccagacctttgtggcctgccaggagcagcggctcatctacctgcccccaccctgggg
cacctgcaaagctgttaccatggactcggatttggatttcttcgactcctacagcatcactgcctgccgcatcgactgtgagac
gcgctacctggtggagaactgcaactgccgcatggtgcacatgccaggggatgccccatactgtactccagagcagtacaagga
gtgtgcagatcctgctctggacttcctggtggagaaggaccaggagtactgcgtgtgtgaaatgccttgcaacctgacccgcta
tggcaaagagctgtccatggtcaagatccccagcaaagcctcagccaagtacctggccaagaagttcaacaaatctgagcaata
catagggggagaacatcctggtgctggacatttctttgaagtcctcaactatgagaccattgaacagaagaaggcctatgagat
tgcagggctcctgggtgagctgctgatgacacctgtccccttctcatgccatgggcatggcgtggctccctatcatccaaaagc
agggtgctcacttctgtcccatgagggtcctccaccccagaggcccttccccaaaccctgttgtcttggtgacatcgggggcca
gatggggctgttcatcggggccagcatcctcacggtgctggagctctttgactacgcctacgaggtcattaagcacaagctgtg
ccgacgaggaaaatgccagaaggaggccaaaaggagcagtgcggacaagggcgtggccctcagcctggacgacgtcaaaagaca
caacccgtgcgagagccttcggggccaccctgccgggatgacatacgctgccaacatcctacctcaccatccggcccgaggcac
gttcgaggactttacctgctgagccccgcaggccgctgaaccaaaggcctagatggggaggactaggagagcgrgggggccccc
agctgcctcctcacatctgccctgggractccccacactccggggcagatctttcctcttgtctgtggtaaggaaggagtcttg
accatagagtcctctctctgcctctatcccattcytttacatttaacaaaactaatctaaaaagaactaaaaagggagaacg
gggcaagggacctcaggctgcccctctctcctccatgctgcctcccctagctcccagcctgaattctgtctatctagctgtctg
ccatctgagtgtccatctacattctgctgccaccagtcaccaaaggcccttcccagtgaggggtggaagggatctctggggtct

ggaatttggccccaaaccagagaatgtaccttaagggggagggctagtgtgggggagggaggcttccccagccttaagagaccc
tctcagcccagtgactgtccccaaacccaagtctcctggcaggaactaaaacctcagccccactctctcacaccatgtggaatc
tcgtgggggtcggggatccccttaagaagtggtaatggggacaagatgcggccctggtgctgtaggctacatcctgatacctat
aagttcacccccaccccacagctgctggagagaaatcccaagaggcagcccttcctcaccatcccattaaagaccKggctggtt
agcgtccagctcagggagaagggcgctagtgcctaacctcactggtccctctcccggaggcccttgtagagggccacgtccata
aatttcttatggaactctcccacatcctcttccccaacttcatttgcttctctcaacaacctcatctgcatttctatttcta
tatgatacagactctatattgctatatctctgtatatactttcccagccctgtctgtctccaccccatcccctcttgtctctga
gaaccattctcccaccccaagttccaccttctatgtttctactccctccctggtctctgaatgccttygcctgtataaagagtt
ggactctctcccctggtgtctgtactgtgtacacacatccctctgagaagcacaaggagacgacacgcgcattgtaacctttgc
actgtctcagtggcgacaaaggaagctgtgaatcacaagctctgcctctttctggcctcaccctctcccccaacccgggcaccc
tcggccctccctgcagccttaacattctcttcccctgctcctcctatcccattgccctctgcccagctgacagtggcatcccca
gggaaggggttgctgtagagatagccccacccaggggatggaggtctaccctggacactaagccaagtgtgtcagagacagaa
gggagctggggattggcgactcctgaagttggggcagtgggatgctgacaggcagaagctgaggtcctcagtcagtggcctttc
ctccttctgggtgcccagcccccttttcctcacctgatacccaagcccaccactttttatttctggtgaggtgggtttgggagga
aagagaggcctagaggaggagttgaaagctctgctgttgtctcaccctatcttaatgagagacaagtgaggtggagggcctgcc
ccccctccctccaccagacactccttccaggcctgagccccaacccctcttcaggccttccttccctagctgtgtcttggtctt
caatcccagaacaggacctgtgagcagctgcattggcctggagctggagagtaaggctgtaggatctttggaatctcttggttc
ctaagagtttcctcagagatcatacctccccagagggaagcaggaatgaggccaaaaagtgtgcattggataggggaacagcag
gcagggctctgggtgacgcatgcctctggtctaataaactgggtttcaaccaaaaaaa
<212> Type : DNA
<211> Length : 3923
      SequenceName : U78180
      SequenceDescription :

Sequence
--------
<213> OrganismName : HUMAN
<400> PreSequenceString :
gcgagctgacagccgccgccgccgcctccgcccaccttcctcgccggggcttcgtctttcactccttcgggctgcctcccc
ctccccttgtcccctgccccttgccctgcttctgcagaagatttcaacactacacttgcacaatgtctttgaaaccaagagtag
tagattttgatgaaacatggaacaaacttttgacgacaataaaagccgtggtcatgttggaatacgtcgaaagagcaacatgga
atgaccgtttctcagatatctatgctttatgtgtggcctatcctgaaccccttggagaaagactttatacagaaactaagattt
ttttggaaaatcatgttcggcatttgcataagagagttttggagtcagaagaacaagtacttgttatgtatcataggtactggg
aagaatacagcaagggtgcagactatatggactgcttatataggtatctcagcacccagtttattaaaaagaataaattaacag
aagcggaccttcagtatggctatggtggtgtagatatgaatgaaccacttatggaaataggagagctagcattggatatgtgga
ggaaattgatggttgaaccacttcaggccatccttatccgaatgctgctccgagaaatcaaaaatgatcgtggtggagaagacc
caaaccagaaagtaatccatggggttattaactcctttgttcatgttgaacagtataagaaaaaattccccttaaagttttatc
aggaaattttttgagtctccctttctgactgaaacaggagagtattacaaacaagaagcttcaaatttattacaagaatcaaact
gctcacagtatatggaaaaggtttaggtagattaaaagatgaagaaattcgatgtcgaaaatacctacatccaagttcatata
ctaaggtgattcatgaatgtcaacaacgaatggtagcagaccacttacagtttttacatgcagaatgtcataatataattcgac
aagagaaaaaaaatgacatggcaaatatgtacgtcttactccgtgctgtgtccactggtttacctcatatgattcaggagctgc
aaaaccacatccatgatgagggccttcgagcaaccagcaaccttactcaggaaaacatgccaacactatttgtggagtcagttt
tggaagtgcatggtaaatttgttcagcttatcaacactgttttgaatggtgatcagcattttatgagtgcgttggataaggccc
ttacgtcagttgtaaattacagagaacctaagtctgtttgcaaagcacctgaactgcttgctaagtactgtgacaacttactga
agaagtcagcgaaagggatgacagagaatgaagtggaagacaggcttacgagcttcatcacagtgttcaaatacattgatgaca
aggacgtctttcaaaagttctacgcaagaatgctggcaaaacgtttaattcatgggttatccatgtctatggactctgaagaag
ccatgatcaacaaattaaagcaagcctgtggttatgagtttaccagcaagctacatcggatgtatacagatatgagtgtcagcg
ctgatctcaacaataagttcaacaattttatcaaaaaccaagacacagtaatagatttgggaattagttttcaaatatatgttc
tacaggctggtgcgtggcctcttactcaggctccttcatctacgtttgcaattccccaggaattagaaaaaagtgtacagatgt
ttgaattattttatagccaacatttcagtggaaggaaacttacatggttacattatctgtgtacaggtgaagttaaaatgaact
atttgggcaaaccatatgtagccatggttacaacataccaaatggcagttcttcttgcctttaacaacagtgaaactgtcagtt
ataaagagcttcaggacagcactcagatgaatgaaaaggaactgacaaaaacaatcaaatcattacttgatgtgaaaatgatta
accatgattcagaaaaggaagatattgatgcagaatcttcgttttcattaaatatgaactttagcagtaaaagaacaaaattta
aaattactacatcaatgcagaaagacacaccacaagaaatggagcagactagaagtgcagttgatgaggaccggaaaatgtatc
tccaagctgctatagttcgtatcatgaaagcacgaaaagtgcttcggcacaatgcccttattcaagaggtgattagccagtcaa
gagctaggtttaatcccagtatcagcatgattaagaagtgtattgaagttctgatagacaaacaatacatagaacgcagccagg
cgtcggcagatgaatacagctacgtcgcgtgatgtcgctctcctccagcgtggtgtgagaagatcattgccatcaccatttggt
gtgttcctgtgggaaaaagcaggactgtgcctccataatttggtcatttggcagcccctgtttttctgctgtttacaacatcacc
agtgccacgtcatgagcgtcaaagaaaatgcctagagatatttcaagctcatgacattatgacatttcttaaaactttattaaa
agaatgagtgaagtattgctgaaaagtggaaaatcggttgggtaccatgctttttctccccttcacgtttgcagttgatgtgtc
tttttttttttttttaatgtatcttaaaggacataaaatttaaaaacttaaatattgtaatatgacagataacctaataattgt
atctacattaaaatgacaaacatgatactgctgcttgtcaataaaaaaaaaaaaaaaa
<212> Type : DNA

<211> Length : 2832
      SequenceName : U83410
      SequenceDescription :

Sequence
--------
<213> OrganismName : HUMAN
<400> PreSequenceString :
atattgtgcttccaccactgccaataacaaaataactagcaaccatgaagtgggtggaatcaatttttttaattttcctactaa
atttactgaatccagaacactgcatagaaatgaatatggaatagcttccatattggattcttaccaatgtactgcagagataa
gtttagctgacctggctaccatatttttgcccagtttgttcaagaagccacttacaaggaagtaagcaaaatggtgaaagatg
cattgactgcaattgagaaacccactggagatgaacagtcttcagggtgtttagaaaaccagctacctgcctttctggaagaac
tttgccatgagaaagaaattttggagaagtacggacattcagactgctgcagccaaagtgaagagggaagacataactgttttc
ttgcacacaaaaagcccactccagcatcgatcccacttttccaagttccagaacctgtcacaagctgtgaagcatatgaagaag
acagggagacattcatgaacaaattcatttatgagatagcaagaaggcatcccttcctgtatgcacctacaattcttctttggg
ctgctcgctatgacaaaataattccatcttgctgcaaagctgaaaatgcagttgaatgcttccaaacaaaggcagcaacagtta
caaaagaattaagagaaagcagcttgttaaatcaacatgcatgtgcagtaatgaaaaattttgggacccgaactttccaagcca
taactgttactaaactgagtcagaagtttaccaaagttaattttactgaaatccagaaactagtcctggatgtggcccatgtac
atgagcactgttgcagaggagatgtgctggattgtctgcaggatggggaaaaaatcatgtcctacatatgttctcaacaagaca
ctctgtcaaacaaaataacagaatgctgcaaactgaccacgctggaacgtggtcaatgtataattcatgcagaaaatgatgaaa
aacctgaaggtctatctccaaatctaaacaggtttttaggagatagagattttaaccaatttcttcaggggaaaaaaatatct
tcttggcaagtttgttcatgaatattcaagaagacatcctcagcttgctgtctcagtaattctaagagttgctaaaggatacc
aggagttattggagaagtgtttccagactgaaaaccctcttgaatgccaagataaaggagaagaagaattacagaaatacatcc
aggagagccaagcattggcaaagcgaagctgcggcctcttccagaaactaggagaatattacttacaaaatgcgtttctcgttg
cttacacaaagaaagcccccccagctgacctcgtcggagctgatggccatcaccagaaaaatggcagccacagcagccacttgtt
gccaactcagtgaggacaaactattggcctgtggcgagggagcggctgacattattatcggacacttatgtatcagacatgaaa
tgactccagtaaaccctggtgttggccagtgctgcacttcttcatatgccaacaggaggccatgcttcagcagcttggtggtgg
atgaaacatatgtccctcctgcattctctgatgacaagttcattttccataaggatctgtgccaagctcagggtgtagcgctgc
aaacgatgaagcaagagtttctcattaaccttgtgaagcaaaagccacaaataacagaggaacaacttgaggctgtcattgcag
atttctcaggcctgttggagaaatgctgccaaggccaggaacaggaagtctgctttgctgaagagggacaaaaactgatttcaa
aaactcgtgctgctttgggagtttaaattacttcaggggaagagaagacaaaacgagtctttcattcggtgtgaacttttctct
ttaatttaactgatttaacacttttgtgaattaatgaaatgataaagacttttatgtgagatttccttatcacagaaataaa
atatctccaaatg
<212> Type : DNA
<211> Length : 2029
      SequenceName : V01514
      SequenceDescription :

Sequence
--------
<213> OrganismName : HUMAN
<400> PreSequenceString :
gggctgcagtgagccaaaagcatgccattgcactccagcttgggcaacagagtgagaccctgtctcaaaaaaaagaaaaaataa
tactatgtctggtccataacctgaaatattttatcttcacgttccttatcattcactgaactttattttcttttaaaattt
tttcctttctttttaaatttgcttctacagatttcttcattctccatttagcaaggtcatggaagatttggaggaaacattatt
tgaagaatttgaaaactattcctatgacctagactattactctctggagtctgatttggaggagaaagtccagctgggagttgt
tcactgggtctccctggtgttatattgtttggcttttgttctgggaattccaggaaatgccatcgtcatttggttcacggggct
caagtggaagaagacagtcaccactctgtggttcctcaatctagccattgcggatttcatttttcttctctttctgcccctgta
catctcctatgtggccatgaatttccactggccctttggcatctggctgtgcaaagccaattccttcactgcccagttgaacat
gtttgccagtgtttttttcctgacagtgatcagcctggaccactatatccacttgatccatcctgtcttatctcatcggcatcg
aaccctcaagaactctctgattgtcattatattcatctggcttttggcttctctaattggcggtcctgccctgtacttccggga
cactgtggagttcaataatcatactctttgctataacaattttcagaagcatgatcctgacctcactttgatcaggcaccatgt
tctgacttgggtgaaatttatcattggctatctcttccctttgctaacaatgagtatttgctacttgtgtctcatcttcaaggt
gaagaagcgaacagtcctgatctccagtaggcatttctggacaattctggttgtggttgtggcctttgtggtttgctggactcc
ttatcacctgtttagcatttgggagctcaccattcaccacaatagctattcccaccatgtgatgcaggctggaatcccccctctc
cactggtttggcattcctcaatagttgcttgaaccccatcctttatgtcctaattagtaagaagttccaagctcgcttccggtc
ctcagttgctgagatactcaagtacacactgtgggaagtcagctgttctggcacagtgagtgaacagctcaggaactcagaaac
caagaatctgtgtctcctggaaacagctcaataagttattacttttccacaaatcagtatatggctttttatgtgggtcctctg
actgatgctttcagattaaaattgtttccaagatagagagccgactccactttcatagttattgtttctggtcacatatatggc
atcacatttt
<212> Type : DNA
<211> Length : 1438
      SequenceName : U13666

SequenceDescription :

Sequence
--------
<213> OrganismName : HUMAN
<400> PreSequenceString :
cgccactgccacccctcccagactgtggacgggaggatggagtcgatggccgtcgctaccgacggcggggagaggccgggggtc
ccagcgggctcaggtctgtcggcttcccagcgtcgggcggagctgcgtcggagaaagctgctcatgaactcggaacagcgcatc
aaccggatcatgggctttcacaggcccgggagcggcgcggaagaagaaagtcaaacaaaatcaaagcagcaggacagtgataaa
ctgaactccctcagcgttccttccgtttcaaagcgagtagtgctgggtgattcagtcagtacaggaacaactgaccagcagggt
ggtgtggccgaggtaaaggggacccaactgggagacaaattggactcgttcattaaaccacctgagtgcagtagtgatgtcaac
cttgagctccggcagcggaacagaggggacctgacagcggactcggtccagaggggttcccgccatggcctagagcagtacctt
tccagattcgaagaagcaatgaagctaaggaaacagctgattagtgaaaaacccagtcaagaggatggaaatacaacagaagaa
tttgactcttttcgaatatttagattggtgggatgtgctcttcttgctcttggagtcagagcttttgtttgcaaatacttgtcc
atatttgctccatttcttactttacaacttgcgtacatgggattatacaaatatttcccaagagtgaaaagaagataaagaca
acagtactaacagctgcacttctattgtcgggaattcctgccgaagtgataaatcgatcaatggatacctatagcaaaatgggc
gaagtcttcacagatctctgtgtctacttttcacttttatcttttgtcatgaactgcttgattattggggctctgaagtacca
tgaagcctgtagaactgagaaggagaagcttacgaaaaaaatcctcttctatattgcagtgtctctaaaggaggcaaattggtt
tacaccttcatgtaattctttactttaggggttgtaaagctactttattagatatagaatggcagattctctgatttaaaagg
gctgagtttgtattattactgatatgaagaatagagtaccaatgtcattaattgattttcttgttaatcagaattcctattct
gtacctttcctctaacttctcagatttgtaattcttcttttcgggagctgagctagtgcttttaggagaacagataaatgtggt
ctcagccagccctagagactgcttcttgtgtttgtgtcattctgtcctgagaaatgaagtcatctgaaaaataaaaatgcagaa
acccaa
<212> Type : DNA
<211> Length : 1350
SequenceName : U18242
SequenceDescription :

Sequence
--------
<213> OrganismName : HUMAN
<400> PreSequenceString :
atgagctcggcctcggggctccgcagggggcacccggcagtgtcaactgttagccacgcagatgccgattgctttgtgtgtgtc
ttcctgagccatggcgaaggcaatcacatttatgcatatgatgctaaaatcgaaattcagacattaactggcttgttcaaagga
gacaagtgtcacagcctggttggaaaacccaagatatttatcatccaggcatgtcggggaaaccagcacgatgtgccagtcatt
cctttggatgtagtagataatcagacagagaagttggacaccaacataactgaggtggatgcagcctccgtttacacgctgcct
gctggagctgacttcctcatgtgttactctgttgcagaaggatattattctcaccgggaaactgtgaacggctcatggtacatt
caagatttgtgtgagatgttgggaaaatatggctcctccttagagttcacagaactcctcacactggtgaacaggaaagtttct
cagcgccgagtggacttttgcaaagacccaagtgcaattggaaagaagcaggttccctgttttgcctcaatgctaactaaaaag
ctgcatttctttccaaaatctaattaattaatagaggctatctaatttcacactctgtattgaaaatggctttctcagccaggc
gtggttactcacacctgtaatcccagcactttgggagtccaaggtgggcggatcacctgaggtcgggagttcgagaccagcctg
accaacatggcagaagccccgcctctactaaaaatgcaaaaaaaaatttagctaggcatggcggcgcatgcctgcaatcccagc
tacttggaaggctgaggcaggagaatcacttgaacccaggaggtggaggctgcggtgagccgagcattgcgccattgcactcca
gcctgggcaacgagtgaaactccgtctcaaaaaaaaagaaaatgtctttctcttcctttatataaatatcgttagggtgaagc
attatggtctaatgattcaaatgttttaaagtttaatgcctagcagagaactgccttaaaaaaaaaaagttcatgttggccatg
gtgaaagggtttgatatggagaaacaaaatcctcaggaaattagataaatagaaatttataagcatttgtattatttttttaata
aactgcagggttacaccaaaatctagctgatttaacttgtattttgtcactttttataaaagtttattgtttgatgtttttaa
aggtttttgaaatccaggaattaaatcatcccttaataaaatattcgaaattc
<212> Type : DNA
<211> Length : 1313
SequenceName : U20537
SequenceDescription :

Sequence
--------
<213> OrganismName : HUMAN
<400> PreSequenceString :
ggccggtggcccgggcccgaccaccccagctgcgcgtcgttactggccacaagtttgctctgggccagccaagttggcaacttg
gaagcttctcccgggctctggaggagggtccctgcttcttcctacagccgttccgggcatggccgggctgggggcgtcgctcca
cgtctggggttggctaatgctcggcagctgcctcctggccagagcccagctggattctgatggcaccattactatagaggagca
gattgtccttgtgctgaaagcgaaagtacaatgtgaactcaacatcacagctcaactccaggagggagaaggtaattgtttccc
tgaatgggatggactcatttgttggcccagaggaacagtggggaaaatatcggctgttccatgccctccttatatttatgactt
caaccataaaggagttgctttccgacactgtaaccccaatggaacatgggatttatgcacagcttaaataaaacatgggccaa

ttattcagactgccttcgctttctgcagccagatatcagcataggaaagcaagaattctttgaacgcctctatgtaatgtatac
cgttggctactccatctcttttggttccttggctgtggctattctcatcattggttacttcagacgattgcattgcactaggaa
ctatatccacatgcacttatttgtgtctttcatgctgagagctacaagcatctttgtcaaagacagagtagtccatgctcacat
aggagtaaaggagctggagtccctaataatgcaggatgacccacaaaattccattgaggcaacttctgtggacaaatcacaata
tatcgggtgcaagattgctgttgtgatgtttatttacttcctggctacaaattattattggatcctggtggaaggtctctacct
gcataatctcatctttgtggctttcttttcggacaccaaatacctgtggggcttcatcttgataggctggggtttccagcagc
atttgttgcagcatgggctgtggcacgagcaactctggctgatgcgaggtgctgggaacttagtgctggagacatcaagtggat
ttatcaagcaccgatcttagcagctattggctgaattttattctgtttctgaatacggttagagttctagctaccaaaatctg
ggagaccaatgcagttgggcatgacacaaggaagcaatacaggaaactggccaaatcgacactggtcctggtcctagtctttgg
agtgcattacatcgtgttcgtatgcctgcctcactccttcactgggctcgggtgggagatccgcatgcactgtgagctcttctt
caactcctttcagggtttctttgtgtctatcatctactgctactgcaatggagaggttcaggcagaggtgaagaagatgtggag
tcggtggaatctctccgtggactggaaaaggacaccgccatgtggcagccgcagatgcggctcagtgctcaccaccgtgacgca
cagcaccagcagccagtcacaggtggcggccagcacacgcatggtgcttatctctggcaaagctgccaagatcgccagcagaca
gcctgacagccacatcactttacctggctatgtctggagtaactcagagcaggactgcctgccacactctttccacgaggagac
caaggaagatagtgggaggcagggagatgatattctaatggagaagccttccaggcctatggaatctaacccagacactgaagg
atgccaaggagaaactgaggatgttctctgaatggacatttgtggctgactttcatgggctggtccaatggctggttgtgtgag
agggcttggctgatactcctatgcttgagttcaaaggctgaaaattcagttaaggtgttacttaataatagtttttaggctcca
tgaattggctcctgtaaatactaacgacatgaaaatgcaagtgtcaatggagtagtttattaccttctattggcatcaagtttt
cctctaaattaatgtatggtatttgctctgtgattgttcatttttttctgctactttgggtagaaaaaagattcaattgcttg
gctgtagctttctctcatatatatcaccctaaatataatgaagatcttttagtgtgtatcatttcctttttagaaactagtatt
ctcttatttcttactttaatgtacttctatcactgcatttatttgcctgtgcataggagcaattaggatctaaaaaaatatat
gggaagataaaagatctaagaacaagtacttgctggaaaattagttggctggacattgataaaataatgcatttataacaatta
catgtgtttttgggaacaaggaaaatttctcaaaaaagaatatttcacacatcccttcttttgaatggcctctttgtgaccagc
cagacctcaggtcttcactctttcttctttgtaaaccatgtcatgtggaaagatttcctcagttagtgagcttgtgtctgcaaa
ttgatttgtttgtaatgtattttgatagcaaatcatgctgcatctatatctttttcttgtttgagctgttactacattgtaca
tggcatgtgggatcaattaaaaatttgttttaaaaat
<212> Type : DNA
<211> Length : 2641
      SequenceName : U25128
      SequenceDescription :

Sequence
--------
<213> OrganismName : HUMAN
<400> PreSequenceString :
atggatgatcgagaggatctggtgtaccaggcgaagctggccgagcaggctgagcgatacgacgaaatggtggagtcaatgaag
aaagtagcagggatggatgtggagctgacagttgaagaaagaaacctcctatctgttgcatataagaatgtgattggagctaga
agagcctcctggagaataatcagcagcattgaacagaaagaagaaaacaagggaggagaagacaagctaaaaatgattcgggaa
tatcggcaaatggttgagactgagctaaagttaatctgttgtgacattctggatgtactggacaaacacctcattccagcagct
aacactggcgagtccaaggttttctattataaaatgaaaggggactaccacaggtatctggcagaatttgccacaggaaacgac
aggaaggaggctgcggagaacagcctagtggcttataaagctgctagtgatattgcaatgacagaacttccaccaacgcatcct
attcgcttaggtcttgctctcaatttttccgtattctactacgaaattcttaattcccctgaccgtgcctgcaggttggcaaaa
gcagcttttgatgatgcaattgcagaactggatacgctgagtgaagaaagctataaggactctacacttatcatgcagttgtta
cgtgataatctgacactatggacttcagacatgcagggtgacggtgaagagcagaataaagaagcgctgcaggacgtggaagac
gaaaatcagtgagacataagccaacaagagaaacca
<212> Type : DNA
<211> Length : 792
      SequenceName : U28936
      SequenceDescription :

Sequence
--------
<213> OrganismName : HUMAN
<400> PreSequenceString :
atgactaagttttcttccttttctctgtttttcctaatagttggggcttatatgactcatgtgtgtttcaatatggaaattatt
ggagggaaagaagtgtcacctcattccaggccatttatggcctccatccagtatggcggacatcacgtttgtggaggtgttctg
attgatccacagtgggtgctgacagcagcccactgccaatatcggtttaccaaaggccagtctcccactgtggttttaggcgca
cactctctctcaaagaatgaggcctccaaacaaacactggagatcaaaaaatttataccattctcaagagttacatcagatcct
caatcaaatgatatcatgctggttaagcttcaaacagccgcaaaactcaataaacatgtcaagatgctccacataagatccaaa
acctctcttagatctggaaccaaatgcaaggttactggctggggagccaccgatccagattcattaagaccttctgacaccctg
cgagaagtcactgttactgtcctaagtcgaaaactttgcaacagccaaagttactacaacggcgacccttttatcaccaaagac
atggtctgtgcaggagatgccaaaggccagaaggattcctgtaagggtgactcaggggggcccttgatctgtaaaggtgtcttc
cacgctatagtctctggaggtcatgaatgtggtgttgccacaaagcctggaatctacaccctgttaaccaagaaataccagact

tggatcaaaagcaaccttgtcccgcctcatacaaattaagttacaaataatttattggatgcacttgcttctttttcctaat
atgctcgcaggttagagttgggtgtaagtaaagcagagcacatatggggtccattttgcacttgtaagtcattttattaagga
atcaagttctttttcacttgtatcactgatgtatttctaccatgctggttttattctaaataaaatt
<212> Type : DNA
<211> Length : 991
      SequenceName : U35237
      SequenceDescription :

Sequence
--------
<213> OrganismName : HUMAN
<400> PreSequenceString :
atgcccgagaatgtggcaccccggagcggggcgactgccggggctgccggcggccgcgggaaaggcgcctatcaggaccgcgac
aagccagcccagatccgcttcagcaacatttccgccgccaaagcggttgctgatgctattagaacaagccttggaccaaaagga
atggataaaatgattcaagatggaaaaggtgatgtaaccattacaaatgatggtgctaccattctgaaacaaatgcaagtatta
catccagcagccagaatgctggtggagctgtctaaggctcaagatatagaagcaggagatggcaccacatcagtagtcatcatt
gctggctccctcttagattcttgtaccaagcttcttcagaaagggattcatccaaccatcatttctgagtcattccagaaggcc
ctggaaaagggcattgaaatcttgactgacatgtctcgacctgtggaactgagtgacagagaaactttgttaaatagtgcaacc
acttcactgaactcaaaggtggtttctcagtattcaagtctgctttctccaatgagtgtaaatgcagtgatgaaagtgattgac
ccagccacagccaccagtgtagatcttagagatattaaaatagttaagaagcttggtgggacaattgatgactgtgagttggtg
gaagggctggttctcacccaaaaagtgtcaaattctggcataaccagagttgaaaaggccaagattggcttattcagttttgc
ttatctgctcccaaaacagacatggataatcaaatagtggtttctgactatgcccagatggaccgagtgctgcgagaagagaga
gcctatattttaaatttagtgaagcaaattaaaaaaacaggatgtaatgtccttctcatacagaaatctattctaagagatgct
cttagtgatcttgcattacactttctgaataaaatgaagatcatggtgattaaggatattgaaagagaagacattgaattcatt
tgtaagacaattggaaccaagccagttgctcatattgaccaatttactgctgacatgctgggttctgctgagttagctgaggag
gtcaatttaaatggttctggcaaactgctcaagattacaggctgtgccagccctggaaaaacagttacaattgttgttcgtggt
tctaacaaactggtgattgaagaagctgagcgctccattcatgatgccctatgtgttattcgttgtttagtgaagaagagggct
cttattgcaggaggtggtgctccagaaatagagttggccctagcattaactgaatattcacgaacactgagtggtatggaatcc
tactgcgttcgtgcttttgcagatgctatggaggtcattccatctacactagctgaaaatgccggcctgaatcccatttctaca
gtaacagaactaagaaaccggcatgcccagggagaaaaaactgcaggcattaatgtccgaaagggtggtatttccaacatttg
gaggaactggttgtccagcctctgttggtatcagtcagtgctctgactcttgcaactgaaactgttcggagcattctgaaaata
gatgatgtggtaaacactcgataatctggataactgactagcaccattatgatcaccagtattgtggctggaatggaagaagat
caccttggtgttccttgtttggaagattatttcctctgaatttctggcttggtcttccagttggcatttgcctgaagttgtat
tgaaacaatttaatgaaaatattaaatatttggtttcaaaaggcagatttatcttctcccaacattctgttatttctgatactt
ttgaaaaactaataaaaactaataaaagaagcgta
<212> Type : DNA
<211> Length : 1883
      SequenceName : U38846
      SequenceDescription :

Sequence
--------
<213> OrganismName : HUMAN
<400> PreSequenceString :
ggcagcggtggcaggggctgcaggagcaagtgaccaggagcaggactggggacaggcctgatcgcccctgcacgaaccagaccc
ttcgccgccctcacgatgactacctctccgatcctgcagctgctgctgcggctctcactgtgcggctgctgctccagagggcg
gagacaggctctaaggggcagacggcgggggagctgtaccagcgctgggaacggtaccgcagggagtgccaggagaccttggca
gccgcggaaccgccttcaggcctcgcctgtaacgggtccttcgatatgtacgtctgctgggactatgctgcacccaatgccact
gcccgtgcgtcctgcccctggtacctgccctggcaccaccatgtggctgcaggtttcgtcctccgccagtgtggcagtgatggc
caatggggactttggagagaccatacacaatgtgagaacccagagaagaatgaggcctttctggaccaaaggctcatcttggag
cggttgcaggtcatgtacactgtcggctactccctgtctctcgccacactgctgctagccctgctcatcttgagtttgttcagg
cggctacattgcactagaaactatatccacatcaacctgttcacgtctttcatgctgcgagctgcggccattctcagccgagac
cgtctgctacctcgacctggccccctaccttggggaccaggcccttgcgctgtggaaccaggccctcgctgcctgccgcacggcc
cagatcgtgacccagtactgcgtgggtgccaactacacgtggctgctggtggagggcgtctacctgcacagtctcctggtgctc
gtgggaggctccgaggagggccacttccgctactacctgctcctcggctgggggcccccgcgcttttcgtcattccctgggtg
atcgtcaggtacctgtacgagaacacgcagtgctgggagcgcaacgaagtcaaggccatttggtggattatacggacccccatc
ctcatgaccatcttgattaatttcctcatttttatccgcattcttggcattctcctgtccaagctgaggacacggcaaatgcgc
tgccgggattaccggctgaggctggctcgctccacgctgacgctggtgcccctgctgggtgtccacgaggtggtgtttgctccc
gtgacagaggaacaggcccggggcgccctgcgcttcgccaagctcggctttgagatcttcctcagctccttccagggcttcctg
gtcagcgtcctctactgcttcatcaacaaggaggtgcagtcggagatccgccgtggctggcaccactgccgcctgcgccgcagc
ctgggcgaggagcaacgccagctcccggagcgcgccttccgggccctgccctccggctccggcccgggcgaggtccccaccagc
cgcggcttgtcctcggggaccctcccagggcctgggaatgaggccagccgggagttggaaagttactgctaggggcgggatcc
ccgtgtctgttcagttagcatggatttattgagtgccaactgcgtgccaggcccagtacggaggacgctggggaaatggtgaag

gaaacagaaaaaggtccctgcccttctggagatgacaactgagtggggaaaacagaccgtgaacacaaaacatcaagttccac
acacgctatggaatggttatgaagggaagcgagaagggggcctagggtggtctgggaggcgtctccaaggaggtgacacttaag
ccatccccgaaagaggtgaaagagatcactttggggagagctggagaacaggattctaggcggaagcgatagcataggcaaagg
cccttgggcaggaaggcgctcagccttggctggagtagaattaagtcagagccaacaggttggggagagacagagaagtgggca
ggggcacccaagttgggatttcatttcaggtgcattggagattcttaggagtgtctcttggggtaatattttatttttaaaa
aatgaggat
<212> Type : DNA
<211> Length : 2025
      SequenceName : U39231
      SequenceDescription :

Sequence
--------
<213> OrganismName : HUMAN
<400> PreSequenceString :
ggcggtgctctacgtcttcaccgctggagatctgctgccccgggcaggtccccgtttcctcacgcggctcttcgaaggctctgg
ggaggcccgagggggcggccgctctagggaagggaccatggagctccgaactacccctcagctgaaagtggtggggcagggccg
cggcaatggagacccgggggtgggatggagaaggacggtccggaatgggacctttgacagcagaccctacaacctgctgccct
tccctgtccctttccacccccccacccaccctccagaggtcctcccgacggccatgaacactacatcttctgcagcacccccctc
actaggtgtagagttcatctctctgctggctatcatcctgctgtcagtggcgctggctgtggggcttcccggcaacagctttgt
ggtgtggagtatcctgaaaaggatgcagaagcgctctgtcactgccctgatggtgctgaacctggccctggccgacctggccgt
attgctcactgctcccttttttccttcacttcctggcccaaggcacctggagttttggactggctggttgccgcctgtgtcacta
tgtctgcggagtcagcatgtacgccagcgtcctgcttatcacggccatgagtctagaccgctcactggcggtggcccgcccctt
tgtgtcccagaagctacgcaccaaggcgatggcccggcgggtgctggcaggcatctgggtgttgtcctttctgctggccacacc
cgtcctcgcgtaccgcacagtagtgccctggaaaacgaacatgagcctgtgcttcccgcggtaccccagcgaagggcaccgggc
cttccatctaatcttcgaggctgtcacgggcttcctgctgcccttcctggctgtggtggccagctactcggacatagggcgtcg
gctacaggcccggcgcttccgccgcagccgccgcaccggccgcctggtggtgctcatcatcctgaccttcgccgccttctggct
gccctaccacgtggtgaacctggctgaggcgggccgcgcgctggccggccaggccgccgggttagggctcgtggggaagcggct
gagcctggcccgcaacgtgctcatcgtactcgccttcctgagcagcagcgtgaaccccgtgctgtacgcgtgcgccggcggcgg
cctcgtgcgctcggcgggcgtgggcttcgtcgccaagctgctggagggcacgggctccgaggcgtccagcacgcgccgcggggg
cagcctgggccagaccgctaggagcggccccgccgctctggagcccggcccttccgagagcctcactgcctccagccctctcaa
gttaaacgaactgaactaggcctggtggaaggaggcgcactttcctcctggcagaatgctagctctgagccagttcagtacctg
gaggaggagcaggggcgtggagggcgtggagggcgtggagcgtgggaggcgggagtggagtggaagaagagggagagatggag
caaagtgagggccgagtgagagcgtgctccagcctggctcccacaggcagctttaaccattaaaactgaagtctgaaatttggt
caaaaaaaaaaaa
<212> Type : DNA
<211> Length : 1610
      SequenceName : U41070
      SequenceDescription :

Sequence
--------
<213> OrganismName : HUMAN
<400> PreSequenceString :
gcccactcccaccgccagctggaaccctggggactacgacgtccctcaaaccttgcttctaggagataaaaagaacatccagtc
atggataaaaatgagctggttcagaaggccaaactggccgagcaggctgagcgatatgatgacatggcagcctgcatgaagtct
gtaactgagcaaggagctgaattatccaatgaggagaggaatcttctctcagttgcttataaaaatgttgtaggagcccgtagg
tcatcttggagggtcgtctcaagtattgaacaaaagacggaaggtgctgagaaaaaacagcagatggctcgagaatacagagag
aaaattgagacggagctaagagatatctgcaatgatgtactgtctcttttggaaaagttcttgatccccaatgcttcacaagca
gagagcaaagtcttctatttgaaaatgaaaggagattactaccgttacttggctgaggttgccgctggtgatgacaagaaaggg
attgtcgatcagtcacaacaagcataccaagaagcttttgaaatcagcaaaaaggaaatgcaaccaacacatcctatcagactg
ggtctggcccttaacttctctgtgttctattatgagattctgaactccccagagaaagcctgctctcttgcaaagacagctttt
gatgaagccattgctgaacttgatacattaagtgaagagtcatacaaagacagcacgctaataatgcaattactgagagacaac
ttgacattgtggacatcggatacccaaggagacgaagctgaagcaggagaaggaggggaaaattaaccggccttccaacttttg
tctgcctcattctaaaatttacacagtagaccatttgtcatccatgctgtcccacaaatagtttttgtttacgatttatgaca
ggtttatgttacttctatttgaatttctatatttcccatgtggttttatgtttaatattaggggagtagagccagttaacatt
tagggagttatctgttttcatcttgaggtggccaatatggggatgtggaattttatacaagttataagtgtttggcatagtac
ttttggtacattgtggcttcaaaagggccagtgtaaaactgcttccatgtctaagcaaagaaaactgcctacatactggtttgt
cctggcggggaataaaagggatcattggttccagtcacaggtgtagtaattgtgggtactttaaggtttggagcacttacaagg
ctgtggtagaatcataccccatggataccacatattaaaccatgtatatctgtggaatactcaatgtgtacacctttgactaca
gctgcagaagtgttcctttagacaaagttgtgacccatttactctggataagggcagaaacggttcacattccattatttgta
aagttacctgctgttagctttcattattttgctacactcattttatttgtatttaaatgtttaggcaacctaagaacaaatg
taaaagtaaagatgcaggaaaaatgaattgcttggtattcattacttcatgtatatcaagcacagcagtaaaacaaaaaccat

gtatttaacttttttttaggattttgctttgtgatttttttttttttttttgatacttgcctaacatgcatgtgctgtaaa
aatagttaacagggaaataacttgagatgatggctagctttgtttaatgtcttatgaaattttcatgaacaatccaagcataat
tgttaagaacacgtgtattaaattcatgtaagtggaataaaagttttatgaatggacttttcaactactttctctacagctttt
catgtaaattagtcttggttctgaaacttctctaaaggaaattgtacattctttgaaatttattccttattccctcttggcagc
taatgggctcttaccaagtttaaacacaaaatttatcataacaaaaatactactaatataactactgtttccatgtcccatgat
cccctctcttcctccccaccctgaaaaaaatgagttcctatttttctgggagagggggggattgattagaaaaaaatgtagtg
tgttccatttaaaatttggcatatggcattttctaacttaggaagccacaatgttcttggcccatcatgacattgggtagcat
taactgtaagttttgtgcttccaaatcacttttggtttttaagaatttcttgatactcttatagcctgccttcaattttgatc
ctttattctttctatttgtcaggtgcacaagattaccttcctgtttagccttctgtcttgtcaccaaccattcttacttggtg
gccatgtacttggaaaaaggccgcatgatctttctggctccactcagtgtctaaggcaccctgcttcctttgcttgcatcccac
agactatttccctcatcctatttactgcagcaaatctctccttagttgatgagactgtgtttatctccctttaaaccctacct
atcctgaatggtctgtcattgtctgcctttaaaatccttcctctttcttcctcctctattctctaaataatgatggggctaagt
tatacccaaagctcactttacaaatatttcctcagtactttgcagaaaacaccaaacaaaaatgccatttaaaaaggtgta
ttttttctttagaatgtaagctcctcaagagcagggacaatgttttctgtatgttctattgtgcctagtacactgtaaatgct
caataaatattgatgatgggaggcagtgagtcttgatgataagggtgagaaactgaaatccc
<212> Type : DNA
<211> Length : 2834
      SequenceName : M86400
      SequenceDescription :

Sequence
--------
<213> OrganismName : HUMAN
<400> PreSequenceString :
gcggccgcgcaagtcctccaccatgtgaatgccaacatggccaggtcattagagctgagggaaaactagtgcccaaagatatga
aaagagtgtggatcttctggagaagtgctgttgttcaacaggtacaaaattggaaatgttggcttcaccagaacctaagggcct
tgttcccttcactaaagagtcttttgaacttataaaacagcatattgctaaaacacataatgaagaccatgaagaagaagactt
aaagccaaatcctgatttggaagttggcaaaaagcttccatttatttatggaaacctttctcaaggaatggtgtcagagccctt
ggaagatgtggacccatattactacaagaaaaaaaatactttcatagtattaaataaaaaatagaacaatcttcagattcaatgc
ggcttccatcttgtgtacattgtctcctttcaattgtattagaagaacaactatcaaggttttggtacatcccttttttccaact
gtttattctaattagtgtcctgattgattgcgtattcatgtccctgactaatttgccaaaatggagaccagtattagagaatac
tttgcttggaatttacacatttgaaatacttgtaaaactctttgcaagaggtgtctgggcaggatcattttccttcctcggtga
tccatggaactggctcgatttcagcgtaactgtgtttgaggttattataagatactcacctctggacttcattccaacgcttca
aactgcaagaactttgagaattttaaaaattattcctttaaatcaaggtctgaaatcccttgtaggggtcctgatccactgctt
gaagcagcttattggtgtcattatcctaactctgtttttctgagcatatttctctaattgggatggggctcttcatggcaa
cttgaaacacaaatgtttcgatggcccaagagaatgaaaatgaaaccctgcacaacagaactggaaacccatattatattcg
agaaacagaaaacttttattatttggaaggagaaagatatgctctcctttgtggcaacaggacagatgctggtcagtgtcctga
aggatatgtgtgtgtaaaagctggcataaatcctgatcaaggcttcacaaattttgacagttttggctgggccttatttgccct
atttcggttaatggctcaggattaccctgaagtactttatcacctgatactttatgcttctgggaaggtctacatgatattttt
tgtggtggtaagtttttgttttcctttatatggcaagtttgttcttaggcatacttgccatggcctatgaagaagaaaagca
gagagttggtgaaatatctaagaagattgaaccaaaatttcaacagactggaaaagaacttcaagaaggaaatgaaacagatga
ggccaagaccatacaaatagaaatgaagaaaaggtcaccaatttccacagacacatcattggatgtgttggaagatgctactct
cagacataaggaagaacttgaaaaatccaagaagatatgcccattatactggtataagtttgctaaaactttcttgatctggaa
ttgttctccctgttggttaaaattgaaagagtttgtccataggattataatggcaccatttactgatcttttccttatcatatg
cataattttaaacgtatgttttctgaccttggagcattatccaatgagtaaacaaactaacactcttctcaacattggaaacct
ggttttcattggaattttcacagcagaaatgattttaaaaataattgcaatgcatccatatgggtatttccaagtaggttggaa
cattttgatagcatgatagtgttccatggtttaatagaactttgtctagcaaatgttgcaggaatggctcttcttcgattatt
caggatgttaagaattttcaagttgggaaagtattggccaacattccagattttgatgtggtctcttagtaactcatgggtggc
cctgaaagacttggtcctgttgttgttcacattcatcttcttttctgctgcattcggcatgaagctgtttggtaagaattatga
agaatttgtctgccacatagacaaagactgtcaactcccacgctggcacatgcatgacttttttccactccttcctgaatgtgtt
ccgaattctctgtggagagtgggtagagaccttgtgggactgtatggaggttgcaggccaatcctggtgtattcctttttacct
gatggtcattttaattggaaatttactggtactttacctgtttctggcattggtgagctcatttagttcatgcaaggatgtaac
agctgaagagaataatgaagcaaaaaatctccagcttgcagtggcaagaattaaaaaggaataaactatgtgcttcttaaaat
actatgcaaaacacaaaatgtcccaaaggacacaatggaccatgtaaatgaggtatatgttaaagaagatatttctgaccatac
cctttctgaattgagcaacacccaagatttctcaaagataaggaaaaaagcagtggcacagagaaaaacgctactgaaaatga
gagccaatcacttatccccagtcctagtgtctcagaaactgtaccaattgcttcaggagaatctgatatagaaaatctggataa
taaggagattcagagtaagtctggtgatggaggcagcaaagagaaaataaagcaatctagctcatctgaatgcagtactgttga
tattgctatctctgaagaagaagaaatgttctatggaggtgaaagatcaaagcatctgaaaaatggttgcagacgcggatcttc
acttggtcaaatcagtggagcatccaagaaaggaaaaatctggcagaacatcaggaaaacctgctgcaagattgtagagaacaa
ttggtttaagtgttttattgggcttgttactctgctcagcactggcactctggcttttgaagatatatatattgatcagagaaa
gacaattaaaatttattagaatatgctgacatgatctttacttatatcttcattctggaaatgcttctaaaatggatggcata
tggttttaaggcctatttctctaatggctggtacaggctggacttcgtggttgttattgtgttttgtcttagcttaataggcaa
aactcgggaagaactaaaacctcttatttccatgaaattccttcggcccctcagagttctatctcaatttgaaagaatgaaggt

ggttgtgagagctttgatcaaaacaaccttacccactttgaatgtgtttcttgtctgcctgatgatctggctgattttagtat
catgggagtagacttatttgctggcagattctatgaatgcattgacccaacaagtggagaaaggtttccttcatctgaagtcat
gaataagagtcggtgtgaaagccttctgtttaacgaatccatgctatgggaaaatgcaaaaatgaactttgataatgttggaaa
tggtttcctttctctgcttcaagtagcaacatttaatggatggatcactattatgaattcagcaattgattctgttgctgttaa
tatacagcctcattttgaagtcaacatctacatgtattgttactttatcaactttattatatttggagtatttctccctctgag
tatgctgattactgttattattgataatttcaacaagcataaaataaagctgggaggctcaaatatctttataacggttaaaca
gagaaaacagtaccgcaggctgaagaagctaatgtatgaggattctcaaagaccagtacctcgcccattaaacaagctccaagg
attcatctttgatgtggtaacaagccaagcttttaatgtcattgttatggttcttatatgtttccaagcaatagccatgatgat
agacactgatgttcagagtctacaaatgtccattgctctctactggattaactcaattttgttatgctatatactatggaatg
tatactgaagctcatcgctttccgttgttttatttcaccattgcgtggaacattttgattttatggtggttatttctccat
cacaggactatgtctgcctatgacagtaggatcctaccttgtgcctccttcacttgtgcaactgatacttctctcacggatcat
tcacatgctgcgtcttggaaaaggaccaaaggtgtttcataatctgatgcttcctttgatgctgtccctcccagcattattgaa
catcattcttctcatcttcctggtcatgttcatctatgccgtatttggaatgtataattttgcctatgttaaaaaagaagctgg
aattaatgatgtgtctaattttgaaacctttggcaacagtatgctctgtctttttcaagttgcaatatttgctggttgggatgg
gatgcttgatgcaattttcaacagtaaatggtctgactgtgatcctgataaaattaaccctgggactcaagttagaggagattg
tgggaacccctctgttggatttttattttgtcagttatatcctcatatcatggctgatcattgtaaatatgtacattgttgt
tgtcatggagtttttaaatattgcttctaagaagaaaaacaagaccttgagtgaagatgatttaggaaattctttcaggtatg
gaaaaggtttgatcctgataggacccagtacatagactctagcaagctttcagattttgcagctgctcttgatcctcctcttt
catggcaaaaccaaacaagggccagctcattgctttggacctccccatggctgttggggacagaattcattgcctcgatatctt
acttgcttttacaaagagagttatgggtcaagatgtgaggatggagaaagttgtttcagaaatagaatcagggttttgttagc
caacccttttaagatcacatgtgagccaattacgactactttgaaacgaaaacaagaggcagtttcagcaaccatcattcaacg
tgcttataaaaattaccgcttgaggcgaaatgacaaaaatacatcagatattcatatgatagatggtgacagagatgttcatgc
tactaaagaaggtgcctattttgacaaagctaaggaaaagtcacctattcaaagccagatctaataccacttaccacctctttt
catatttcttcacatatctgaaaaatgttgaaagcctaagccaggaataaaagaaaagtagagataataatcagttctttacaa
ccgatggtaattaagcttgtattcacaagacttcatgccaaattcacttttagcattatatctaacaaatcaagagaatcctt
aatattgctgcag
<212> Type : DNA
<211> Length : 5389
SequenceName : M91556
SequenceDescription :

Sequence
--------
<213> OrganismName : HUMAN
<400> PreSequenceString :
ctaaagatgaaacggctggtttgtgtgctcttggtgtgctcctctgcagtggcacagttgcataaagatcctaccctggatcac
cactggcatctctggaagaaaacctatggcaaacaatacaaggaaaagaatgaagaagcagtacgacgtctcatctgggaaaag
aatctaaagtttgtgatgcttcacaacctggagcattcaatgggaatgcactcatacgatctgggcatgaaccacctgggagac
atgaccagtgaagaagtgatgtctttgacgagttccctgagagttcccagccagtggcagagaaatatcacatataagtcaaac
cctaatcggatattgcctgattctgtggactggagagagaaagggtgtgttactgaagtgaaatatcaaggttcttgtggtgct
tgctgggctttcagtgctgtgggggccctggaagcacagctgaagctgaaaacaggaaagctggtgactctcagtgcccagaac
ctggtggattgctcaactgaaaaatatggaaacaaaggctgcaatggtggcttcatgacaacggctttccagtacatcattgat
aacaagggcatcgactcagacgcttcctatccctacaaagccatggatcagaaatgtcaatatgactcaaaatatcgtgctgcc
acatgttcaaagtacactgaacttccttatgggagagaagatgtcctgaaagaagctgtggccaataaaggcccagtgtctgtt
ggtgtagatgcgcgtcatccttctttcttcctctacagaagtggtgtctactatgaaccatcctgtactcagaatgtgaatcat
ggtgtacttgtggttggctatggtgatcttaatgggaaagaatactggcttgtgaaaaacagctggggccacaactttggtgaa
gaaggatatattcggatggcaagaaataaaggaaatcattgtgggattgctagctttccctcttacccagaaatctagaggatc
tctccttttataacaaatcaagaaatatgaagcactttctcttaacttaattttcctgctgtatccagaagaaataattgtg
tcatgattaatgtgtatttactgtactaatagaaaatatagtttgaggccgggcactgtctggctcacgcctgtaatcccagta
cttgggaggccaaggaggcatatcaacttgaggccaggagttaaagagcagcctggctaactgtgaaaccctctctact
<212> Type : DNA
<211> Length : 1255
SequenceName : S93414
SequenceDescription :

Sequence
--------
<213> OrganismName : HUMAN
<400> PreSequenceString :
ctgcgcctctcttccccagacctgcgcgctactgcggctcgggcggtcgctcgcctggctctgctccatttgactgtctgtgtg
cagtcgcagaacttcgaagagggttttgcgctccatccgtggcgtttcgcttttgttcggtttgttgtttatttcattttttt
ttttccggagagaggcgaggcggtggtccacacccgcccgaggaggaagatgtcaaacgtgagagtgtctaacggagcccgag
cctggagcggatggacgccagacaagcggatcaccccaagccttccgcctgcagaaatctcttcggcccggtcaatcatgaaga

actaacccgggacttggagaagcactgccgggatatggaagaagcgagtcagcgcaagtggaatttcgactttcagaatcataa
gcccctggagggcagatacgaatggcaggaggtggagaggggcagcttgcccgagttctactacaggcccccgcgccccccaa
gagcgcctgcaaggtgctggcgcaggagagccaggatgtcagcgggagccgccaggcggtgcctttaattgggtctcaggcaaa
ctctgaggaccggcatttggtggaccaaatgcctgactcgtcagacaatcaggctgggttagcggagcagtgtccagggatgag
gaagcgacctgctgcagaagattcttcttcgcaaaacaaaagggccaacagaacagaagaaaatgtttcagacggttccccgaa
cgctggcactgtggagcagacgcccaagaagcccggccttcgacgccagacgtaaacagctccgaattaagaatatttccttgt
ttattagatacatcactgctttatgaagcaaggaagatatacatgaaaa
<212> Type : DNA
<211> Length : 889
      SequenceName : U10440
      SequenceDescription :

Sequence
--------
<213> OrganismName : HUMAN
<400> PreSequenceString :
tgcagcactcaccatggaatccccgattcagatcttccgcggggagcctggccctacctgcgccccgagcgcctgcctgccccc
caacagcagcgcctggtttcccggctgggccgagcccgacagcaacggcagcgccggctcggaggacgcgcagctggagcccgc
gcacatctccccggccatcccggtcatcatcacggcggtctactccgtagtgttcgtcgtgggcttggtgggcaactcgctggt
catgttcgtgatcatccgatacacaaagatgaagacagcaaccaacatttacatatttaacctggctttggcagatgctttagt
tactacaaccatgccctttcagagtacggtctacttgatgaattcctggccttttggggatgtgctgtgcaagatagtaatttc
cattgattactacaacatgttcaccagcatcttcaccttgaccatgatgagcgtggaccgctacattgccgtgtgccaccccgt
gaaggctttggacttccgcacacccttgaaggcaaagatcatcaatatctgcatctggctgctgtcgtcatctgttggcatctc
tgcaatagtccttggaggcaccaaagtcagggaagacgtcgatgtcattgagtgctccttgcagttcccagatgatgactactc
ctggtgggacctcttcatgaagatctgcgtcttcatctttgccttcgtgatccctgtcctcatcatcatcgtctgctacaccct
gatgatcctgcgtctcaagagcgtccggctcctttctggctcccgagagaaagatcgcaacctgcgtaggatcaccagactggt
cctggtggtggtggcggttttcgtcgtctgctggactcccattcacatattcatcctggtggaggctctggggagcacctccca
cagcacagctgctctctccagctattacttctgcatcgccttaggctataccaacagtagcctgaatcccattctctacgcctt
tcttgatgaaaacttcaagcggtgtttccgggacttctgctttccactgaagatgaggatggagcggcagagcactagcagagt
ccgaaatacagttcaggatcctgcttacctgagggacatcgatgggatgaataaaccagtatgactagtcgtggagatgtcttc
gtacag
<212> Type : DNA
<211> Length : 1182
      SequenceName : U11053
      SequenceDescription :

Sequence
--------
<213> OrganismName : HUMAN
<400> PreSequenceString :
atggggcgcccactgcacctcgtcctgctcagtgcctccctggctggcctcctgctgctcggggaaagtctgttcatccgcagg
gagcaggccaacaacatcctggcgagggtcacgagggccaattcctttcttgaagagatgaagaaaggacacctcgaaagagag
tgcatggaagagacctgctcatacgaagaggcccgcgaggtctttgaggacagcgacaagacgaatgaattctggaataaatac
aaagatggcgaccagtgtgagaccagtccttgccagaaccagggcaaatgtaaagacggcctcggggaatacacctgcacctgt
ttagaaggattcgaaggcaaaaactgtgaattattcacacggaagctctgcagcctggacaacggggactgtgaccagttctgc
cacgaggaacagaactctgtggtgtgctcctgcgcccgcgggtacaccctggctgacaacggcaaggcctgcattcccacaggg
ccctacccctgtgggaaacagaccctggaacgcaggaagaggtcagtggcccaggccaccagcagcagcggggaggcccctgac
agcatcacatggaagccatatgatgcagccgacctggaccccaccgagaaccccttcgacctgcttgacttcaaccagacgcag
cctgagaggggcgacaacaacctcaccaggatcgtgggaggccaggaatgcaaggacggggagtgtccctggcaggccctgctc
atcaatgaggaaaacgagggtttctgtggtggaactattctgagcgagttctacatcctaacggcagcccactgtctctaccaa
gccaagagattcaaggtgagggtaggggaccggaacacggagcaggaggagggcggtgaggcggtgcacgaggtggaggtggtc
atcaagcacaaccggttcacaaaggagacctatgacttcgacatcgccgtgctccggctcaagacccccatcaccttccgcatg
aacgtggcgcctgcctgcctccccgagcgtgactggccgagtccacgctgatgacgcagaagacggggattgtgagcggcttc
gggcgcacccacgagaagggccggcagtccaccaggctcaagatgctggaggtgccctacgtggaccgcaacagctgcaagctg
tccagcagcttcatcatcacccagaacatgttctgtgccggctacgacaccaagcaggaggatgcctgccagggggacagcggg
ggcccgcacgtcacccgcttcaaggacacctacttcgtgacaggcatcgtcagctggggagagggctgtgcccgtaaggggaag
tacgggatctacaccaaggtcaccgccttcctcaagtggatcgacaggtccatgaaaaccaggggcttgcccaaggccaagagc
catgccccggaggtcataacgtcctctccattaaagtgagatcccactcaaaaaaaaaaaaaaaaaaaaaaaaaaaa
<212> Type : DNA
<211> Length : 1507
      SequenceName : M57285
      SequenceDescription :

Sequence
--------
<213> OrganismName : HUMAN
<400> PreSequenceString :
ggatcctatgagcctgggaggtcaggactgcagtgagccatgattacaccactgcagtgcagcctgcgtgacaaaacgagaccc
tgtctctaaaaaatgagaaaaaaaaatggttgttaccaggcgataaagggaggggaaaacgggagttacttaatgagtatacag
tttcagttttgcgagatgaacagaattctggaaattggttgaacaccgctgtgattgaactcactaccaaactctacacttaaa
aatggttaagatggtacaatttgtatgtattttaccacaataaaaaataaaaaaaaaggctgggcgagatgttcactcctgtaat
cccagtacttggggaggctggggctgaaggatcgtttgagccctgaaggagtttgagaccagcctgagcaacataaggagaccc
catctgtacacaaaattaaaacattagccaggcagagagctggtcacggtggctcacgtatgtaatcccagcactttgggaggc
cgaggcgggcgggcggatcacctgaggtcaggagtttgagaccagcctggccaacatagtgaaaccgtgaaaccccatctctac
taaaaatacaaaaattagctgggcgtggtggtgccctcataatcccagccactcgggaggctgagacaggagaatcgcttgaac
tcaggaggtggaggttgcagtgagcctagatcacaccactgcagtccaaagcaagactccgtctcaaaaaaaaaaaaaattagc
ccggctgttgtctccagttattctggaggctaaggcaggaagattgctggagcctaggagatcaaagctgcagtgagctatgac
tgcgcctctgcactccaacctgggtgacagaggaagaccctgtctcaaaaaaataaataacattgaaaaggaactctcccaaaa
gtatcttattctttctccataggcctcagagaaccatggctactgccaagggaatcgccataggaatcgacctgggcaccacct
actcctgtgtgggggtgttccagcacggcaaggtggagatcatcgccaacgaccagggcaaccgcaccacccccagctacgtgg
ccttcacagacaccgagcggctcattggggatgcggccaagaaccaggtagcaatgaatccccagaacactgtttttgatgcta
aacgtctgatcggcaggaaatttaatgatcctgttgtacaagcagatatgaaactttggccttttcaagtgattaatgaaggag
gcaagcccaaagtccttgtgtcctacaaaggggagaataaagctttctaccctgaggaaatctcttcgatggtattgactaagt
tgaaggagactgctgaggcctttttgggccaccctgtcaccaatgcagtgattaccgtgccagcctatttcaatgactctcaac
gtcaggctactaaggatgcaggtgtgattgctggacttaatgtgctaagaatcatcaatgagcccacggctgctgccattgcct
atggtttagataaaggaggtcaaggagaacgacatgtcctgatttttgatctgggtggaggcacatttgatgtgtcaattctga
ccatagatgatgggatttttgaggtaaaggccactgctggggacactcacctgggtggggaggactttgacaacaggcttgtga
gccacttcgtggaggagttcaagaggaaacacaaaaaggacatcagccagaacaagcgagccgtgaggcggctgcgcaccgcct
gcgagagggccaagaggaccctgtcgtccagcacccaggccaacctagaaattgattcactttatgaaggcattgacttctata
catccatcaccagagctcgatttgaagagttgtgtgcagacctgtttaggggtaccctggagcctgtagaaaaagcgcttcggg
atgccaagatggataaggctaaaatccatgacattgttttagtaggggggctccacccgcatccccaaggtgcagcggctgcttc
aggactacttcaatggacgtgatctcaacaagagcatcaaccctgatgaggccgtagcatatggggctgcggtacaagcagcca
tcctgatgggggacaagtctgagaaggtacaggacctgctgctgctggacgtggctcccctgtccctgggtctggagacggttg
gggcgtgatgactgccctgataaagcgcaactccaccatcccacccaagcagacacagattttcaccacctactctgacaacc
aacccggggtgctgatccaggtgtatgagggcgagagggccatgacaaaggacaacaacctgctggggcggtttgatctgactg
gaatccctccagcacccaggggagttcctcagatcgaggtgacgtttgacattgatgccaatggtattctcaatgtcacagcca
cggacaagagcaccggcaaggtgaacaagatcaccatcaccaatgacaagggccgcctgagcaaggaggagattgagcggatgg
ttctggatgctgagaaatataaagctgaagatgaggtccagagggagaaaattgctgcaaagaatgccttagaatcctatgctt
ttaacatgaagagtgttgtgagtgatgaaggtttgaagggcaagattagtgagtctgataaaaataaaatattggataaatgca
acgagctcctttcgtggctggaggtcaatcaactggcagagaaagatgagtttgatcataagagaaaggaattggagcagatgt
gtaaccctatcatcacaaaactctaccaaggaggatgcactgggcctgcctgcggaacagggtatgtgcctggaaggcctgcca
caggccccacaattgaagaagtagattaattcttttagaactgaagcatcctaggatgcctctacatgtatttcattcccctc
atgttgaaacatcattattattcttgaccagacctgaatctaagttaccatcccttggaaattctggagaaggagtctcatgca
ccacctatcacactccctcacatcctgtttctgactttggaatggactcaggaaaactaggcccctctttaaccgtgtgatgta
tttgaatgtctgttatttccagccaccctaacattcttcttcctgtgtggatgcttatttgtcaatcagtaaattgttcgtaa
agaaaattacttctggtatttaggctgtgaatgtaccttgaaggggagagttcatggagagagcatgtgttctctgattgtgag
gtcactgtgaatgattaaattggtaagggtaaagtatttgaattttcatgaact
<212> Type : DNA
<211> Length : 3330
      SequenceName : M59829
      SequenceDescription :

Sequence
--------
<213> OrganismName : HUMAN
<400> PreSequenceString :
tgttcccagcactcaagccttgccaccgccgagccgggcttcctgggtgtttcaggcaaggaagtctaggtccctgggggtga
cccccaaggaaaaggcagcctccctgcgcacccggttgcccggagccctctccagggccggctggctggggttgccctggcc
agcaggggcccgggggcgatgccacccggtgccgactgaggccaccgcaccatggcccgctcgctgacctggcgctgctgcccc
tggtgcctgacggaggatgagaaggccgccgcccgggtggaccaggagatcaacaggatcctcttggagcagaagaagcaggac
cgcggggagctgaagctgctgcttttgggcccaggcgagagcgggaagagcaccttcatcaagcagatgcggatcatccacggc
gccggctactcggaggaggagcgcaagggcttccggcccctggtctaccagaacatcttcgtgtccatgcgggccatgatcgag
gccatggagcggctgcagattccattcagcaggcccgagagcaagcaccacgctagcctggtcatgagccaggaccccctataaa
gtgaccacgtttgagaagcgctacgctgcggccatgcagtggctgtggagggatgccggcatccgggcctgctatgagcgtcgg
cgggaattccacctgctcgattcagccgtgtactacctgtcccacctggagcgcatcaccgaggagggctacgtccccacagct
caggacgtgctccgcagccgcatgcccaccactggcatcaacgagtactgcttctccgtgcagaaaaccaacctgcggatcgtg

gacgtcgggggccagaagtcagagcgtaagaaatggatccattgtttcgagaacgtgatcgccctcatctacctggcctcactg
agtgaatacgaccagtgcctggaggagaacaaccaggagaaccgcatgaaggagagcctcgcattgtttgggactatcctggaa
ctaccctggttcaaaagcacatccgtcatcctctttctcaacaaaaccgacatcctggaggagaaaatccccacctcccacctg
gctacctatttccccagtttccagggccctaagcaggatgctgaggcagccaagaggttcatcctggacatgtacacgaggatg
tacaccgggtgcgtggacggccccgagggcagcaagaagggcgcacgatcccgacgccttttcagccactacacatgtgccaca
gacacacagaacatccgcaaggtcttcaaggacgtgcgggactcggtgctcgcccgctacctggacgagatcaacctgctgtga
cccaggccccacctggggcaggcggcaccggcgggcgggtgggaggtgggagtggctgcagggaccctagtgtcctggtctatc
tctccagcctcggcccacacgcaagggagtcgggggacggcccgctgctggccgctctcttctctgcctctcaccaggacagcc
gccccccagggtactcctgcccttgcttgactcagtttccctcctttgaaagggaaggagcaaaacggccatttgggatgccag
ggtggatgaaaaggtgaagaatcaggggattgagacttgggtgggtgggcatctctcaggagccccatctccgggcgtgtcac
ctcctgggcagggttctgggaccctctgtgggtgacgcacaccctgggatggggctagtagagccttcaggcgccttcgggcgt
ggactctggcgcactctagtggacaggagaaggaacgccttccaggaacctgtggactaggggtgcagggacttccctttgcaa
ggggtaacagaccgctggaaaacactgtcactttcagagctcggtggctcacagcgtgtcctgccccggtttgcggacgagaga
aatcgcggcccacaagcatcccccatcccttgcaggctgggggctgggcatgctgcatcttaaccttttgtatttattccctca
ccttctgcagggctccgtgcgggctgaaattaaagatttcttag
<212> Type : DNA
<211> Length : 2060
SequenceName : M63904
SequenceDescription :

Sequence
--------
<213> OrganismName : HUMAN
<400> PreSequenceString :
ggccggggttgactccgggggcgcggcgaggagagacatgaggctgagctggttccgggtcctgacagtactgtccatctgcct
gagcgccgtggccagcacggggaccgagggcaaaaggaagctgcagatcggggtcaagaagcgggtggaccactgtcccatcaa
atcgcgcaaaggggatgtcctgcacatgcactacacggggaagctggaagatgggacagagtttgacagcagcctgccccagaa
ccagccctttgtcttctcccttggcacaggccaggtcatcaagggctgggaccaggggctgctggggatgtatgagggggaaaa
gcgcaagctggtgatcccatccgagctagggtatggagagcggggagctcccccaaagattccaggcggtgcaaccctggtgtt
cgaggtggagctgctcaaaatagagcgacgaactgagctgtaaccagactgggaggggcaggggagaggccccccatcaggacca
gactgttccaaaaaaaaaaaacaaaaacaaacaaaaaaacactt
<212> Type : DNA
<211> Length : 550
SequenceName : M65128
SequenceDescription :

Sequence
--------
<213> OrganismName : HUMAN
<400> PreSequenceString :
tggtttgtggtttttatgaagagatgtgaaaaaggaagtgtggaatgatgggatgagaagttgtatggggaagatgaatagaag
aattaggtggttgaataaaattaaaaggtgtgtggttggatgaatgaatgagtgggatgatagatggacctaagtggttagtgg
atggacaggaggatggatggatgtgagagccccagaaggacataaggaaagatgggtggatagatggatggcggatggaagga
tatttaggaggatgaatgagcatgtgtgtggagagaggtgcccattcacactggcttgaacacatgggttagctgagccaaatg
ccagccctatgacaggccatcagtagctttccctgagctgttctgccaagaagctaaaattcattcaagccatgtggacttgtt
attgaggggaaaaagaatgagctctccctctttccacttggaagattcaccaactccccacccctcactccccactgtgggcac
ggaggcactgcgccacccagggcaagacctcgccctctctccagctcctctcccaggatatccaacatccctgtgaaaccagag
atcttgctccagccggattcagagaaatttagcgggaaaggagaggccaaaggctgaacccaatggtgcaaggttttacggttc
ggtcatcctctgtcctgacgccgcggggccagcgggagaagaaagccagtgcgtctctgggcgcaggggccagtggggctcgga
ggcacaggcaccccgcgacactccaggttccccgacccacgtccctggcagccccgattatttacagcctcagcagagcacggg
gcgggggcagaggggcccgcccgggagggctgctacttcttaaaacctctgcgggctgcttagtcacagcccccccttgcttggg
tgtgtccttcgctcgctccctccctccgtcttaggtcactgttttcaacctcgaataaaaactgcagccaacttccgaggcagc
ctcattgcccagcggaccccagcctctgccaggttcggtccgccatcctcgtcccgtcctccgccggccccctgccccgcgccca
gggatcctccagctcctttcgcccgcgccctccgttcgctccggacaccatggacaagttttggtggcacgcagcctggggact
ctgcctcgtgccgctgagcctggcgcagatcggtgagtgcccgccgcaggctgggcagcaagatgggtgcggggtgctcagcgc
ggac
<212> Type : DNA
<211> Length : 1264
SequenceName : M69215
SequenceDescription :

Sequence
--------

<213> OrganismName : HUMAN
<400> PreSequenceString :
gaattccggagttccgggcgcgcgcgacgtcagtttgagttctgtgttctccccgcccgtgtcccgcccgacccgcgcccgcga
tgctggcgctgcgctgcggctcccgctggctcggcctgctctccgtcccgcgctccgtgccgctgcgcctccccgcggcccgcg
cctgcagcaaggctccggcgacccgtcctcttcctcctcctccgggaacccgctcgtgtacctggacgtggacgccaacggga
agccgctcggccgcgtggtgctggagctgaaggcagatgtcgtcccaaagacagctgagaacttcagagccctgtgcactggtg
agaagggcttcggctacaaaggctccaccttccacagggtgatcccttccttcatgtgccaggcgggcgacttcaccaaccaca
atggcacaggcgggaagtccatctacggaagccgctttcctgacgagaactttacactgaagcacgtggggccaggtgtcctgt
ccatggctaatgctggtcctaacaccaacggctcccagttcttcatctgcaccataaagacagactggttggatggcaagcatg
ttgtgttcggtcacgtcaaagagggcatggacgtcgtgaagaaaatagaatctttcggctctaagagtgggaggacatccaaga
agattgtcatcacagactgtggccagttgagctaatctgtggccagggtgctggcatggtggcagctgcaaatgtccatgcacc
caggtggccgcgttgggctgtcagccaaggtgcctgaaacgatacgtgtgcccactccactgtcacagtgtgcctgaggaaggc
tgctagggatgttagacggaattcc
<212> Type : DNA
<211> Length : 865
      SequenceName : M80254
      SequenceDescription :

Sequence
--------
<213> OrganismName : HUMAN
<400> PreSequenceString :
ctggtggcctttaatacctggctgttgctgaaaggtctttagaaacggcgctaacagcaggtttgtggaatgccggatcgctca
acggcctgacgtgggcaaaaacctcgccttccgcacccatcattatattgatgctcattgccgccgccttactggtacgccgga
tgcgcttgctggaaatgggacacacggtcactgcagctgaagccgctgcccctgctacaggcaccaccaggaccagctgatcat
tccagcccacagcaatggagccacatgactcctcccacatggactctgagttccgatacactctcttcccgattgtttacagca
tcatctttgtgctcggggtcattgctaatggctacgtgctgtgggtctttgcccgcctgtacccttgcaagaaattcaatgaga
taaagatcttcatggtgaacctcaccatggcggacatgctcttcttgatcaccctgccactttggattgtctactaccaaaacc
agggcaactggatactccccaaattcctgtgcaacgtggctggctgccttttcttcatcaacacctactgctctgtggccttcc
tgggcgtcatcacttataaccgcttccaggcagtaactcggcccatcaagactgctcaggccaacacccgcaagcgtggcatct
ctttgtccttggtcatctgggtggccattgtgggagctgcatcctacttcctcatcctggactccaccaacacagtgcccgaca
gtgctggctcaggcaacgtcactcgctgctttgagcattacgagaagggcagcgtgccagtcctcatcatccacatcttcatcg
tgttcagcttcttcctggtcttcctcatcatcctcttctgcaacctggtcatcatccgtaccttgctcatgcagccggtgcagc
agcagcgcaacgctgaagtcaagcgccgggcgctgtggatggtgtgcacggtcttggcggtgttcatcatctgcttcgtgcccc
accacgtggtgcagctgccctggacccttgctgagctgggcttccaggacagcaaattccaccaggccattaatgatgcacatc
aggtcaccctctgcctccttagcaccaactgtgtcttagaccctgttatctactgtttcctcaccaagaagttccgcaagcacc
tcaccgaaaagttctacagcatgcgcagtagccggaaatgctcccgggccaccacggatacggtcactgaagtggttgtgccat
tcaaccagatccctggcaattccctcaaaaattagtccctgcttccaggcctgaagtcttctcctccatgaacatcatggactg
agctgggggaagaagggatatctactgtggtctgggcaccacctctgtgggcactggtgggccattagatttggaggctacctc
acctgggcagggatgatggcagagccaggctgttggaaaatccagaactcaaatgagccccttcatccgcctgtggggcatact
acagtaactgtgacttgatgactttatctgagtccttat
<212> Type : DNA
<211> Length : 1551
      SequenceName : M80436
      SequenceDescription :

Sequence
--------
<213> OrganismName : HUMAN
<400> PreSequenceString :
gcagttctggtcctcctaggagcggccgcctgcgcggcgcggccccgtggtcggatgctgggcggcagagaggccgaggcgcac
gcgcggccctacatggcgtcggtgcagctgaacggcgcgcacctgtgcgcaggcgtcctggtggcggagcggtgggtgctgagc
gcggcgcactgcctggaggacgcggccgacgggaaggtgcaggttctcctgggcgcgcactccctgtcgcagccggagccctcc
aagcgcctgtacgacgtgctccgcgcagtgccccacccggacagccagcccgacaccatcgaccacgacctcctgctgctacag
ctgtcggagaaggccacactgggccctgctgtgcgccccctgccctggcagcgcgtggaccgcgacgtggcaccgggaactctc
tgcgacgtggccggctggggcatagtcaaccacgcgggccgccgcccggacagcctgcagcacgtgctcttgccagtgctggac
cgcgccacctgcaaccggcgcacgcaccacgacggcgccatcaccgagcgcttgatgtgcgcggagagcaatcgccgggacagc
tgcaagggtgactccgggggcccgctggtgtgcgggggcgtgctcgagggcgtggtcacctcgggctcgcgcgtttgcggcaac
cgcaagaagcccgggatctacacccgcgtggcgagctatgcggcctggatcgacagcgtcctggcctagggtgccggggcctga
aggtcagggtcacccaagcaacaaagtcccgagcaatgaagtcatccactcctgcatctggttggtctttattgagcacctact
atatgcagaaggggaggccgaggtgggaggatcattggatctcaggagttggagatcagcatgggccacgtagcgcgactccat
ctctacaaataaataaaaattagctgggcaattggcgggcatggaggtgggtgcttgtagttccagctactcaggaggctgagg
tgggaggatgacttgaacgcaggaggctgaggctgcagtgagttgtgattgcaccactgccct

<212> Type : DNA
<211> Length : 1071
      SequenceName : M84526
      SequenceDescription :

Sequence
--------
<213> OrganismName : HUMAN
<400> PreSequenceString :
acaggacacagctgggttctgaagcttctgagttctgcagcctcaccctctgagaaacctcttttccaccaataccatgaagc
tctgcgtgactgtcctgtctctcctcatgctagtagctgccttctgctctccagcgctctcagcaccaatgggctcagaccctc
ccaccgcctgctgcttttcttacacccgagaagcttcctcgaactttgtggtagattactatgagaccagcagcctctgctccc
agccagctgtggtattccaaaccaaaagaagcaagcaagtctgtgctgatcccagtgaatcctgggtccaggagtacgtgtatg
acctggaactgaactgagctgctcagagacaggaagtcttcagggaaggtcacctgagcccggatgcttctccatgagacacat
ctcctccatactcaggactcctctccgcagttcctgtcccttctcttaatttaatcttttttatgtgccgtgttattgtattag
gtgtcatttccattatttatattagtttagccaaaggataagtgtcccctatggatggtccactgtcactgtttctctgctgtt
gcaaatacatggataacacatttgattcgtgtgtgttttcataataaactttaaaataaaatgc
<212> Type : DNA
<211> Length : 653
      SequenceName : M23502
      SequenceDescription :

Sequence
--------
<213> OrganismName : HUMAN
<400> PreSequenceString :
gaattcccgggcagcagacagtggtcagtcctttcttggctctgctgacactcgagcccacattccgtcacctgctcagaatca
tgcaggtctccactgctgcccttgctgtcctcctctgcaccatggctctctgcaaccagttctctgcatcacttgctgctgaca
cgccgaccgcctgctgcttcagctacacctcccggcagattccacagaatttcatagctgactactttgagacgagcagccagt
gctccaagcccggtgtcatcttcctaaccaagcgaagccggcaggtctgtgctgaccccagtgaggagtgggtccagaaatatg
tcagcgacctggagctgagtgcctgaggggtccagaagcttcgaggcccagcgacctcggtgggcccagtggggaggagcagga
gcctgagccttgggaacatgcgtgtgacctccacagctacctcttctatggactggttgttgccaaacagccacactgtgggac
tcttcttaacttaaattttaatttatttatactatttagtttttgtaatttattttcgatttcacagtgtgtttgtgattgttt
gctctgagagttcccctgtcccctcccccttccctcacaccgcgtctggtgacaaccgagtggctgtcatcagcctgtgtaggc
agtcatggcaccaaagccaccagactgacaaatgtgtatcggatgcttttgttcagggctgtgatcggcctggggaaataataa
agatgctcttttaaaaggt
<212> Type : DNA
<211> Length : 775
      SequenceName : M25315
      SequenceDescription :

Sequence
--------
<213> OrganismName : HUMAN
<400> PreSequenceString :
ccgcagcatgagctccgcagccgggttctgcgcctcacgccccgggctgctgttcctggggttgctgctcctgccacttgtggt
cgccttcgccagcgctgaagctgaagaagatggggacctgcagtgcctgtgtgtgaagaccacctcccaggtccgtcccaggca
catcaccagcctggaggtgatcaaggccggaccccactgccccactgcccaactgatagccacgctgaagaatggaaggaaaat
ttgcttggacctgcaagccccgctgtacaagaaaataattaagaaacttttggagagttagctactagctgcctacgtgtgtgc
atttgctatatagcatacttcttttttccagtttcaatctaactgtgaaagaaacttctgatatttgtgttatccttatgattt
taaataaacaaaataaatc
<212> Type : DNA
<211> Length : 439
      SequenceName : M25897
      SequenceDescription :

Sequence
--------
<213> OrganismName : HUMAN
<400> PreSequenceString :
gaattcagtaacccaggcattattttatcctcaagtcttaggttggttggagaaagataacaaaaagaaacatgattgtgcaga
aacagacaaacctttttggaaagcatttgaaaatggcattccccctccacagtgtgttcacagtgtgggcaaattcactgctct
gtcgtactttctgaaaatgaagaactgttacaccaaggtgaattatttataaattatgtacttgcccagaagcgaacagacttt

tactatcataagaacccttccttggtgtgctctttatctacagaatccaagacctttcaagaaaggtcttggattcttttcttc
aggacactaggacataaagccaccttttatgatttgttgaaatttctcactccatccctttttgctgatgatcatgggtcctca
gaggtcagacttggtgtccttggataaagagcatgaagcaacagtggctgaaccagagttggaacccagatgctctttccacta
agcatacaactttccattagataacacctccctcccaccccaaccaagcagctccagtgcaccactttctggagcataaacata
ccttaactttacaacttgagtggccttgaatactgttcctatctggaatgtgctgttctctttcatcttcctctattgaagccc
tcctattcctcaatgccttgctccaactgcctttggaagattctgctcttatgcctccactggaattaatgtcttagtaccact
tgtctattctgctatatagtcagtccttacattgctttcttcttctgatagaccaaactctttaaggacaagtacctagtctta
tctatttctagatcccccacattactcagaaagttactccataaatgtttgtggaactgatttctatgtgaagacatgtgcccc
ttcactctgttaactagcattagaaaaacaatcttttgaaaagttgtagtatgcccctaagagcagtaacagttcctagaaac
tctctaaaatgcttagaaaaagatttattttaaattacctccccaataaaatgattggctggcttatcttcaccatcatgatag
catctgtaattaactgaaaaaaataattatgccattaaaagaaaatcatccatgatcttgttctaacacctgccactctagta
ctatatctgtcacatggtctatgataaagttatctagaaataaaaaagcatacaattgataattcaccaattgtggagcttca
gtattttaaatgtatattaaaattaaattatttaaagatcaaagaaaactttcgtcatactccgtatttgataaggaacaaat
aggaagtgtgatgactcaggtttgccctgaggggatgggccatcagttgcaaatcgtggaatttcctctgacataatgaaaaga
tgagggtgcataagttctctagtagggtgatgatataaaagccaccggagcactccataaggcacaaactttcagagacagca
gagcacacaagcttctaggacaagagccaggaagaaaccaccggaaggaaccattctcactgtgtgtaaacatgacttccaagc
tggccgtggctctcttggcagccttcctgatttctgcagctctgtgtgaaggtaagcacatctttctgacctacagcgttttcc
tatgtctaaatgtgatccttagatagcaaagctattcttgatgctttggtaacaaacatcctttttattcagaaacagaatata
atcttagcagtcaattaatgttaaattgaagatttagaaaaaactatatataacacttaggaaatataaaggtttgatcaatat
agatattctgcttttataatttataccaggtagcatgcatatatttaacgtaaataagtaatttatagtatgtcctattgagaa
ccacggttacctatattatgtattaatattgagttgagcaaggtaactcagacaattccactccttgtagtatttcattgacaa
gcctcagatttgtcattaattcctgtctggtttaaagataccctgattatagaccaggcatgtataacttatttatatatttct
gttaattctttctgaaggcaatttctatgctggagagtcttagcttgcctactataaataacactgtggtatcacagaggatta
tgcaatattgaccagataaaaataccatgaagatgttgatattgtacaaaaagaactctaactcttatataggaagttgttcaa
tgttgtcagttatgactgtttttaaaacaaagaactaactgaggtcaagggctaggagatattcaggaatgagttcactagaa
acatgatgccttccatagtctccaaataatcatattggaattagaaggaagtagctggcagagctgtgcctgttgataaaatca
atccttaatcacttttttccccaacaggtgcagttttgccaaggagtgctaaagaacttagatgtcagtgcataaagacatact
ccaaacctttccaccccaaatttatcaaagaactgagagtgattgagagtggaccacactgcgccaacacagaaattatgtaag
tactttaaaaagattagatattttgttttagcaaacttaaaattaaggaaggtggaaatatttaggaaagttccaggtgttag
gattacagtagtaaatgaaacaaaacaaaataaaaatatttgtctacatgacatttaaatatggtagcttccacaactactata
aatgttattttggacttagactttatgcctgacttaaggaatcatgatttgaatgcaaaaactaaatattaatctgaaccattt
ctttcttatttcagtgtaaagctttctgatggaagagagctctgtctggaccccaaggaaaactgggtgcagagggttgtggag
aagtttttgaagaggtaagttatatatttttaatttaaatttttcatttatcctgagacatataatccaaagtcagcctataa
atttctttctgttgctaaaaatcgtcattaggtatctgccttttggttaaaaaaaaggaatagcatcaatagtgagtttgtt
gtacttatgaccagaaagaccatacatagtttgcccaggaaattctgggtttaagcttgtgtcctatactcttagtaaagttct
ttgtcactcccagtagtgtcctatttagatgataatttctttgatctccctatttatagttgagaatatagagcatttctaac
acatgaatgtcaaagactatattgacttttcaagaaccctactttccttcttattaaacatagctcatctttatatttttaatt
ttattttagggctgagaattcataaaaaaattcattctctgtggtatccaagaatcagtgaagatgccagtgaaacttcaagca
aatctacttcaacacttcatgtattgtgtgggtctgttgtagggttgccagatgcaatacaagattcctggttaaatttgaatt
tcagtaaacaatgaatagttttcattgtaccatgaaatatccagaacatacttatatgtaaagtattatttatttgaatctac
aaaaaacaacaaataatttttaaatataaggatttcctagatattgcacgggagaatatacaaatagcaaaattggccaagg
gccaagagaatatccgaactttaatttcaggaattgaatgggtttgctagaatgtgatatttgaagcatcacataaaaatgatg
ggacaataaattttgccataaagtcaaatttagctggaaatcctggatttttttctgttaaatctggcaaccctagtctgctag
ccaggatccacaagtccttgttccactgtgccttggtttctcctttatttctaagtggaaaaagtattagccaccatcttacct
cacagtgatgttgtgaggacatgtggaagcactttaagtttttcatcataacataaattattttcaagtgtaacttattaacc
tatttattatttatgtatttatttaagcatcaaatatttgtgcaagaatttggaaaaatagaagatgaatcattgattgaatag
ttataaagatgttatagtaaatttattttatttagatattaaatgatgttttattagataaatttcaatcagggtttttagat
taaacaaacaaacaattgggtacccagttaaattttcatttcagatatacaacaaataatttttagtataagtacattattgt
ttatctgaaattttaattgaactaacaatcctagtttgatactcccagtcttgtcattgccagctgtgttggtagtgctgtgtt
gaattacggaataatgagttagaactattaaaacagccaaaactccacagtcaatattagtaatttcttgctggttgaaacttg
tttattatgtacaaatagattcttataatattatttaaatgactgcattttaaatacaaggctttatatttttaactttagtg
tttttatgtgctctccaaattttttttactgtttctgattgtatggaaatataaaagtaaatatgaaacatttaaaatataatt
tgttgtcaaagtaatcaagtgtttgtcttttttttagtttagcttattgggattctctttgtttatatttaaaattatacttt
gatttagaaaacataaatgcttccccttagcattttgttatggaaaattacaaactttttattttagaaaacagaactcctttc
cagaaataggttacaaacagtagtgtcctccacagaatgttggaaatgttttcaactccccactgtatactatcttgctaataa
gtctgtcttcagatttcgattaaccggtttgtatgtctgtgcactttagcatagctggacattaaagaggaaagagagtacata
ttataagttgcttatcagtaactgaggagtaaaactgataaatgtgaggcaaagaagtttaaaatatggttaaagcctaagcat
atttgcaaacaaatcaaacaatactctgagaagtaaaaacataattatttaattaacaaatttcagtggataaattttataaca
aattagacacagttgaaaataaaattagaaaactagaaaatagaacaaaagaaacttctggaattca
<212> Type : DNA
<211> Length : 5191
      SequenceName : M28130
      SequenceDescription :

Sequence
--------
<213> OrganismName : HUMAN
<400> PreSequenceString :
gtcgacaccacccaaaatagtgccgagcctcttgggggggagggctgggagtgggggccctgagtgagagcaacgagggtgt
gaccagcgccgcccggacccctagtcccctcccccgcacactcttcagctgtcgcaggggcctgagaggacagctgagggtcc
tggctgggaacgagctggggagggggagctggtggtgcctggggcatgaagaggcctcgctgagaccctcacaaacggtttgca
cgtttccacacctcatttctcctcttcggtggcaggcactgtgcacccaattcctaaagcactcctggatttaatgttctgag
agccacatagaacgaaagatgcaagaaatctgtttgctctttttcaggggtggggtctttctgcccagatgtgggatcctct
cctaaacccaggtcaacccagggcacgaggcagatggctggtgctgacatgttgaccatcactgctctcttccaaggactcaca
aagagttaatgtccctgggctcagcctaggaagattccagtccctgcccaggcccaagatagttgctggcctgattcccctgg
cattcaggactggaaaggaggaggaggggcacactacgccggctcccatcctcccccaccccgcgtgcctgcttgggattcct
gactctgtaccagcttcagagaacaggggtgggggtgggtgccattgggtgtggacagaaagctagtgaaacaagaccatgaca
agtcactggccggctcagacgtgtttgtgtctctctttcttagctcagtgagtactgggtatgtgtcacattgccaaatcccg
gatcacaagtctccatgaactgctggtgagctaggataataaaacccctgacatcaccattccagaagcttcacaagactgcat
atataaggggctggctgtagctgcagctgaaggagctgaccagccagctgacccctcacactcacctagccaccatggacatcg
ccatccaccaccccctggatccgccgccccttctttcctttccactccccagccgcctctttgaccagttcttcggagagcacc
tgttggagtctgatcttttcccgacgtctacttccctgagtcccttctaccttcggccaccctccttcctgcgggcacccagct
ggtttgacactggactctcagaggtgagtctccccacagctaggacgggagagtccttactggaacctcctggaaacttctcca
tccatttcctttcctaccctgcctaaaccattttaggcacatgtgtgtccaaatgtgaagaaaaatgaggaggttgctagtgc
cttcctcccccatcacctgtttctatttgatagtcctctgtatcccatttattacatttttcatgcactgtcaagtttatcct
ccgtcccctaacttctctacaggataccccttttctggtttggttcatgacaatctgcagggaaagagctgccttcaaactcctt
tgcttatctcttccaacaccttggactcttgaccgatttaccatctcaggtttcagagccaggagagagccctgcctcatcct
gagctgttcatccccatgggtatttctgcctttctattccctcttctatgatttctgggtttctcagggctacgacagggcg
ctggcctgggtccaatcaagccctacgaggaaacaatatagggacgcccatttgtcctaagagggtggaagaacagggtgaaca
aataaggttgacagagctgtcacagataacactctggtttaaaaatattcaagtgtgagtaaacaggagctgagtgggcaaggg
ctttggaaggacaagcaggaccagcagaacattccagattgggtgggtggaaaactggcaaagagacctgagccagaagaagag
gcctttgtctcacagacaaaccacaaagccaggcattggagtcagagaggcagcagatgccaggcttgcacccatccttgcgac
tggtcccctgggtgatctgtcttcttctctgtccctgtaaataaagtttgggtctgatcaccatgagccttaggtatcactgtg
gtggctccctgaagcagacagctatgtttatttaaaaaggagatttttaagcagagaagagaaggatgaattacccggacaga
aagcagctctgcagaataagacagcacctgtgtaatcagtattttgccctctttctcccatcccattcccttaccttgctatt
tctagatgcgcctggagaaggacaggttctctgtcaacctggatgtgaagcacttctccccagaggaactcaaagttaaggtgt
tgggagatgtgattgaggtgcatggaaaacatgaagagcgccaggtatgtagcttgtttttttgttttctgctcattcattcag
tgatactgtaatagtccaggtagtgctatcagctttggaggctggctacattccagtcccaagccataacagtcgggatcaggg
gttacaaatcaatgtctagaagactaagttaggatagacatattgctgttgttactattatggccagagatgtggcctttgatt
tgatcgccttagatgggatgatgggatgctgatgccccatttaagccagtggttctgaatctgggccacattagaatcaccagg
ggaactttcaaaaacctaatgctcgggcatcctccagaccaattagcatatgtgctgccgaagcgagcactactccagaccaat
taaatcagcatttttaagggtgggacccaggcatcagcaattttaaggtaattctaatctacagtcaaggttgagaaccactg
attaggtatagggctgtcagacacctagttgctttgcataattacattaactacaggtaccctaaaagcacttgagttgtgact
tctcttttagctgtgcaagaatccgtgtctcttctttagcccatcttaatgctgaactacttggtttgtctaaatttcagagct
gtgctcagtctttaatcccctacagcccatgtggtaatcagttaacgagagcctgtttggctacatgcttgagagtcagcaggc
atacgggttaaggtcatctactctttgggggagttctgacaaatggaacagcttgttatgactttataagagggctttaaaatt
gcttctcaccatttaacgatagctcagaacctgtgcgtcaaccagtacagtttgtcctcagtaatgtcctcaggctgtttcaat
tttgcttatatgatttaggtttgggtcatagtctccttggatggagtcatttttttttttttttaatttcagcagcagtcctat
tgttctggaaccttctgggacattcctgaagagtcaggacaatttcagggcttcctcagggactcagattctaaatgagattcc
aaattctgtaggcccagccaacattgatctaaacctttgggaaataccccctaaacatatctatgcctcagggtttgaaaaacaa
tgaagtgttggactgtttcagacttctcagattctcactggtaggagtgactacctaggcaatttcatcttagctgcaaccctg
aaacgaagctctatttatttttcctatgttgtcatggcatttggtctcacctaaggggaaatcaggatgcctgagttctgggca
ggtgataatagttcctgttcttatctctctgcctctttcctcattcttttgggttaggatgaacatggtttcatctccagggag
ttccacaggaaataccggatcccagctgatgtagaccctctcaccattacttcatccctgtcatctgatggggtcctcactgtg
aatggaccaaggaaacaggtctctggccctgagcgcaccattcccatcacccgtgaagagaagcctgctgtcaccgcagccccc
aagaaatagatgccctttcttgaattgcatttttaaaacaagaaagtttccccaccagtgaatgaaagtcttgtgactagtgc
tgaagcttattaatgctaagggcaggcccaaattatcaagctaataaaatatcattcagcaacagataactgtcttgtgtttga
atattccacacacttttaaataaatatacagataccacagatctatttatgattgcattatgatttagagggctccaaggattt
tagagt
<212> Type : DNA
<211> Length : 4206
SequenceName : M28638
SequenceDescription :

Sequence
--------

<213> OrganismName : HUMAN
<400> PreSequenceString :
agaggctgcggtgagtccgccagccccagctgctcctcctcaagcccccaaggcccttcggcggcccctgctgaaagaccggc
atgccctattctgtggcttccgcgaagccgatgctgctaccagctttctgagagcagcaagatcaggtaacttggacaaagct
ttggatcacctgcggaatggggtagatattaacacctgtaaccagaatgggttgaatggcttgcatctggcttctaaggaaggc
catgtgaaaatggtggttgaacttctgcacaaagaaatcattctagaaacgacaaccaagaaggggaacacggccctgcacatc
gctgctctagccgggcaggatgaggtggtccgggagcttgtcaactatggagccaacgtcaatgcccagtcacagaaaggtttt
acacccctgtacatggcagcacaagagaaccacttggaagtggttaagtttttactggaaaatggagctaaccagaatgtagcc
acagaagacggcttcacgcctctggcggtagccctgcagcagggccatgagaacgtcgtcgcgcacctcatcaactacggcacc
aaggggaaggtgcgcctcccggccctgcacatcgcggcccgcaacgacgacacgcgcacggctgcggtgctgctgcagaacgac
cccaacccggacgtgctttccaagacgggattcacgcccctgcacattgcggctcactacgagaacctcaacgtggcccagttg
ctcctcaacagaggcagcagcgtcaatttcacaccacagaacggcatcacgccactgcacatcgcctcccgcaggggcaacgtg
atcatggtgcggctgctgctggatcggggagcccagatagaaaccaagaccaaggacgaattgacacctctccactgtgcagct
cgaaatgggcacgtgcgaatctcagagatcctgctggaccacggggcaccaatccaagccaaaaccaagaacggcctgtcccca
attcacatggcggctcagggagaccacctcgactgtgtccggctcctgttgcaatacgacgcagagatagacgacatcaccctg
gaccacctgaccccactccacgtggctgcccactgtggacaccacagggtggctaaggtccttctggataaaggggccaaaccc
aactccagagccctgaatggctttaccccttacacatcgcctgcaaaaagaaccacgtccgtgtcatggagctgctgctgaag
acgggagcctcgatcgacgcggtcaccgagtctggcctgacacctctccacgtggcctccttcatggggcaccttcccatcgtg
aagaacctcctgcagcgggggggcgtcgcccaacgtctccaacgtgaaagtggagaccccgctacacatggcagccagagccggg
cacacggaagtggccaaatatttactccagaacaaagccaaagtcaatgccaaggccaaggatgaccagaccccacttcactgt
gcagctcgcatcggccacacaaacatggtgaagctcctgctggaaaataacgccaaccccaacctggccaccaccgccgggcac
accccccctgcacattgcagcccgtgagggccatgtggaaacagtcctggcccttctggaaaaggaagcatcccaggcctgcatg
accaagaaaggatttacccctctgcacgtggcggccaagtacgggaaggtgcgggtggcagagctgctgctggagcgggacgca
cacccgaatgctgccggaaaaaatggcctgacccccctgcacgtggccgtccatcacaacaacctggacatcgtcaagctgctg
cttccccggggcggctccccgcacagccctgcctggaatggctacacccctttgcacatcgctgccaagcagaaccaggtggag
gtggcccgtagtctgctgcagtatgggggctcagcaaacgccgagtcggtgcaaggtgtgacgccccttcacctggccgcccag
gagggccacgcagagatggtggctctgctgctctcgaaacaagccaatggcaacctggggaacaagagcggactcactcccctc
catctggtagcacaagaaggccacgttccagtggcagatgtgctgatcaaacacggcgtcatggtggacgccaccacccggatg
ggctacactcccctccatgtggccagtcactatggaaacatcaagctggtgaagtttctgctgcagcaccaggcagatgtcaat
gccaagaccaagctaggatacagcccccctgcaccaggcagcccagcagggacacacagacatcgtgactctgcttctgaaaaac
ggtgcttccccaaacgaggtcagctcggatggaaccacacctctggccatagccaagcgcttgggctacatttctgtcaccgac
gtgctcaaggtcgtcacggatgaaaccagtttcgtgttagtcagtgataagcatcgaatgagtttccctgagacagttgatgag
atcctggatgtctcggaagatgaaggggaagaactcatcagcttcaaggctgagaggcgggattccagggatgttgatgaagag
aaggagctgctggattttgtgccgaagctagaccaagtggtggaatctccagccatccccaggattccctgtgccatgcctgag
acagtggtgatcaggtcagaagagcaggagcaggcatctaaagagtatgatgaggactccctcatccccagcagcccggccacc
gagacctcagacaacatcagcccggtggccagcccggtgcatacagggtttctggtgagcttcatggttgacgcccggggtggt
tccatgagaggaagtcgccacaacggcctgcgagtggtgatcccgccacggacgtgcgcagcgcccacccgcatcacctgccgc
ctggtcaagccccagaagctcagcacgccgcccccactggccgaggaggagggcctggccagcaggatcatagcactggggccc
acgggcgcacagttcctgagccctgtaatcgtggagatcccgcactttgcctcccatggccgtggagaccgcgagctcgtggtt
ctgaggagcgaaaacggctccgtgtggaaggagcacaggagccgctatggagagagctacctggatcagatcctcaacgggatg
gacgaagagctggggagcctggaggagctagagaagaagagggtgtgccgaatcatcaccaccgacttcccgctgtacttcgtg
atcatgtcacggctctgccaggactacgacatcatcggtcccgaagggggctccctgaagagcaagctggtgcccctggtacag
gcaacgttcccggagaatgccgtcaccaagagagtgaagctggctctgcaggcccagcctgtcccggatgagcttgtcactaag
ctcctgggcaaccaggccacattcagccccattgtcaccgtggagccccggcgccggaagttccaccgccccattggcttcgg
atcccactacctccttcctggaccgacaacccgagggacagtggggagggagacaccaccagcctgcgcctgctttgcagcgtc
attggaggaacagaccaagcccagtgggaagacataacaggaaccaccaaacttgtatatgccaacgagtgcgccaacttcacc
accaatgtctctgccaggttttggctgtcggactgtcctcggactgctgaggctgtgaactttgccaccctgctgtacaaagag
ctcactgcagtgccctacatggccaaattcgtcatctttgccaagatgaatgacccccgagaggggcgcctgcgctgctactgc
atgacagatgataaagtggacaagaccctggagcagcatgagaacttcgtggaggtggcccggagcagggacatagaggtgttg
gaaggaatgtccctgtttgcagaactctctgggaacctggtgcctgtgaagaaagctgcccagcagcggagcttccacttccag
tcatttcgggagaaccgtctggccatgcctgtaaaggtgagggacagcagtcgagagccgggagggtccctgtcgtttctgcgc
aaggcgatgaagtacgaggacacccagcacattctctgccacctgaacatcaccatgccccccctgcgctaagggaagtggagcc
gaagataggagaaggaccccgacgcccctggccctgcgatacagcattctcagtgagtccacaccaggttctctcagtgggaca
gagcaggcagagatgaagatggctgttatctcagagcacctcggtctcagctgggcagagttggcccgggagctgcagttcagt
gtggaagacatcaacaggatccgagtggaaaatcccaactccctgttggagcagagtgtggccttgctgaacctctgggtcatc
cgtgaaggccaaaacgcaaacatggagaatctgtacacagccctgcagagcattgaccgtggcgagatcgtgaacatgctggag
ggttccggccgacagagccgcaacttgaagccagacaggcggcacaccgaccgcgactactcgctgtcaccctcccagatgaat
ggttactcctcactgcaggacgagctgctgtcccctgcctccctgggctgtgcactttcctctccgctacgtgcagaccagtac
tggaatgaggtggccatcctagacgccatccccttggcggccacggagcatgacaccatgctggagatgtctgacatgcaggtg
tggtctgcgggcctcacgccttctctggtcactgctgaggactcctctctggagtgtagcaaggctgaggactctgatgccaca
ggtcacgagtggaagttggagggggcactctcagaggaaccgcggggccccgagttgggctctctggaacttgtggaggacgac
acagtggattcagatgccacaaatggccttatcgatttgcttgaacaggaggaaggtcagaggtcagaagagaagctgccaggt
tctaagaggcaggatgacgcgacaggtgcagggcaggactcagagaatgaagtgtctcttgtttcaggccatcagaggggcaa

gcccgaatcacacattcccccaccgtgagtcaggtgacggagaggagtcaggacagactgcaggactgggatgcagacggctcg
attgtctcatacctgcaagatgctgcacaaggttcctggcaagaggaggtcacgcaaggtccacactcattccagggaacaagt
accatgactgaagggctagagcccggtggatctcaggagtacgagaaggtcctggtgtctgtaagtgagcacacgtggacagaa
cagcccgaggctgagagctcccaggccgacagggaccggaggcagcaaggccaagaagagcaggtgcaggaggccaagaacacc
ttcacccaagtggtgcaggggaatgagtttcagaatattccaggggagcaggtgacagaggagcaattcacggatgagcagggc
aacattgtcaccaagaagatcattcgcaaggtggttcgacagatagacttgtccagcgccgatgccgcccaggagcacgaggag
gtggagctgagagggagtggcctacagccggacctgatagagggcaggaaggggggcgcagatagtgaagcgggccagcctgaaa
aggggaaacagtgaccccgagccgctctccttggagtagcctctcgggaggatcacacctcgacacccaaccccctgaacccca
cacactctgccatgcacacaggaggagagctggacctgagggccaccgcagcggtgcacacattcctctgggctgacggcatga
cctctgtaagggactcctgctagtcccctcttggcatgaatgactgactgtagacgcatgacctccaggcttcaatcctgcctc
ttgcaatgacagctgatctgtcggaaccaggacacaaaagcagcaagaagcggggagagagagggatagaaaacaagcgcagga
gagcctgcgaacgcaaaagtgaatgagggctttttgtggctggggatgggttttggttttggggtttttttttaaattgtttt
gacttcgtacaggtactttttcccggcctcatctgtcagaaatccatgtgggcttcctg
<212> Type : DNA
<211> Length : 6192
      SequenceName : M28880
      SequenceDescription :

Sequence
--------
<213> OrganismName : HUMAN
<400> PreSequenceString :
gaattcagttcaatgtttacacaatcctacagttctgctaggcttctatgatgctactattctgcatttgaatgagcaaatgga
tttaatgcattgtcagggagccggccaaagcttgagagctccttcctggctgggaggccccttggaatgtggcctgaaggtaag
ctggcagcgagcctgacatgctttcatctagtttcctcgcttccttccttttctgcagttttcgcttcacagaaagcagaatcc
ttaaaaataaccctcttagttcacatctgtggtcagtctgggcttaatggcaccccatcctccccatttgctcatttggtctca
gcagtgaatggaaaaagtgtctcgtcctgacccccctgcttccctttcctacttcctggaaatccacaggatgctgcatttgctc
agcagatttaacagcccacttatcactcatggaagatccctcctcctgcttgactccgccctctctccctctgcccgctttcaa
taagaggcagagacagcagccagaggaaccgagaggctgagactaacccagaaacatccaattctcaaactgaagctcgcactc
tcgcctccagcatgaaagtctctgccgcccttctgtgcctgctgctcatagcagccaccttcattccccaagggctcgctcagc
caggtaggc
<212> Type : DNA
<211> Length : 681
      SequenceName : M30816
      SequenceDescription :

Sequence
--------
<213> OrganismName : HUMAN
<400> PreSequenceString :
tccagatgcaatcaatgccccagtcacctgctgctataacttcaccaataggaagatctcagtgcagaggctcgcgagctatag
aagaatcaccagcagcaagtgtcccaaagaagctgtgatgtgag
<212> Type : DNA
<211> Length : 128
      SequenceName : M31625
      SequenceDescription :

Sequence
--------
<213> OrganismName : HUMAN
<400> PreSequenceString :
cacagcttcaagaccattgtggccaaggagatctgtgctgaccccaagcagaagtgggttcaggattccatggaccacctggac
aagcaaacccaaactccgaagacttgaacactcactccacaacccaagaatctgcagctaacttatttcccctagctttcccc
agacaccttgttttattttattataatgaatttgtttgttgatgtgaaacattatgccttaagtaatgttaattcttatttaag
ttattgatgttttaagtatctttcatggtactagtgtttttagatacagagacttggggaaattgcttttcctcttttgaacc
acagttctacccctgggatgttttgagggtctttgcaagaatcattaatacaaagaatttttttaacattccaatgcattgct
aaaatattattgtggaaatgaatattttgtaactattacaccaaataaatatatttttgtac
<212> Type : DNA
<211> Length : 482
      SequenceName : M31626
      SequenceDescription :

Sequence

--------
<213> OrganismName : HUMAN
<400> PreSequenceString :
ccggccgcggagctcttgcgaccccgccaggacccgaacagagcccgggggcggcgggccggagccggggacgcgggcacacgc
ccgctcgcacaagccacggcggactctcccgaggcggaacctccacgccgagcgagggtcagtttgaaaaggaggatcgagctc
actgtggagtatccatggagatgtggagccttgtcaccaacctctaactgcagaactgggatgtggagctggaagtgcctcctc
ttctgggctgtgctggtcacagcaacactctgcaccgctaggccgtccccgaccttgcctgaacaagatgctctcccctcctcg
gaggatgatgatgatgatgatgactcctcttcagaggagaaagaaacagataacaccaaaccaaaccccgtagctccatattgg
acatccccagaaaagatggaaaagaaattgcatgcagtgccggctgccaagacagtgaagttcaaatgcccttccagtgggacc
ccaaaccccacactgcgctggttgaaaaatggcaaagaattcaaacctgaccacagaattggaggctacaaggtccgttatgcc
acctggagcatcataatggactctgtggtgccctctgacaagggcaactacacctgcattgtggagaatgagtacggcagcatc
aaccacacataccagctggatgtcgtggagcggtcccctcaccggcccatcctgcaagcagggttgcccgccaacaaaacagtg
gccctgggtagcaacgtggagttcatgtgtaaggtgtacagtgacccgcagccgcacatccagtggctaaagcacatcgaggtg
aatgggagcaagattggcccagacaacctgccttatgtccagatcttgaagactgctggagttaataccaccgacaaagagatg
gaggtgcttcacttaagaaatgtctcctttgaggacgcaggggagtatacgtgcttggcgggtaactctatcggactctcccat
cactctgcatggttgaccgttctggaagccctggaagagaggccggcagtgatgacctcgcccctgtacctggagatcatcatc
tattgcacaggggccttcctcatctcctgcatggtggggtcggtcatcgtctacaagatgaagagtggtaccaagaagagtgac
ttccacagccagatggctgtgcacaagctggccaagagcatccctctgcgcagacaggtaacagtgtctgctgactccagtgca
tccatgaactctggggttcttctggttcggccatcacggctctcctccagtgggactcccatgctagcaggggtctctgagtat
gagcttcccgaagaccttcgctgggagctgcctcgggacagactggtcttaggcaaacccctgggagagggctgctttgggcag
gtggtgttggcagaggctatcggcctggacaaggacaaacccaaccgtgtgaccaaagtggctgtgaagatgttgaagtcggac
gcaacagagaaagacttgtcagacctgatctcagaaatggagatgatgaagatgatcgggaagcataagaatatcatcaacctg
ctgggggcctgcacgcaggatggtcccttgtatgtcatcgtggagtatgcctccaagggcaacctgcgggagtacctgcaggcc
cggaggcccccagggctggaatactgctacaaccccagccacaacccagaggagcagctctcctccaaggacctggtgtcctgc
gcctaccaggtggcccgaggcatggagtatctggcctccaagaagtgcatacaccgagacctggcagccaggaatgtcctggtg
acagaggacaatgtgatgaagatagcagactttggcctcgcacgggacattcaccacatcgactactataaaaagacaaccaac
ggccgactgcctgtgaagtggatggcacccgaggcattatttgaccggatctacacccaccagagtgatgtgtggtctttcggg
gtgctcctgtgggagatcttcactctgggcggctccccataccccggtgtgcctgtggaggaacttttcaagctgctgaaggag
ggtcaccgcatggacaagcccagtaactgcaccaacgagctgtacatgatgatgcgggactgctggcatgcagtgccctcacag
agacccaccttcaagcagctggtggaagacctggaccgcatcgtggccttgacctccaaccaggagtacctggacctgtccatg
cccctggaccagtactcccccagctttcccgacacccggagctctacgtgctcctcaggggaggattccgtcttctctcatgag
ccgctgcccgaggagccctgcctgccccgacacccagcccagcttgccaatggcggactcaaacgccgctgactgccacccaca
cgccctccccagactccaccgtcagctgtaaccctcacccacagcccctgctgggcccaccacctgtccgtccctgtcccctttt
cctgctggcaggagccggctgcctaccaggggccttcctgtgtggcctgccttcaccccactcagctcacctctccctccacct
cctctccacctgctggtgagaggtgcaaagaggcagatctttgctgccagccacttcatcccctccagatgttggaccaacacc
cctccctgccaccaggcatctgccggatgggcagagtggagcaatgaacaggcatgcaagtgagagcttcctgagctttctcct
gtcggtttggtctgttttgccttcacccataagcccctcgcactctggtggcaggtgcttgtcctcagggctacagcagtaggg
aggtcagtgcttcgtgcctcgattgaaggtgacctctgccccagataggtggtgccagtggcttattaattccgatactagttt
gctttgctgaccaaatgcctggtaccagaggatggtgaggcgaaggccaggttgggggcagtgttgtgccctggcccagccaaa
ctggggggctctgtgggggctctgtatatagctatgaagaaaacacaaagtgtataaatctgagtatatatttacatgtcttttt
aaaagggtcgttaccagagatttacccatcgggtaagatgctcctggtggctgggaggcatcagttgctatatattaaaaacaa
aaaagaaaaaaaaggaaaatgtttttaaaaaggtcatatatttttttgctacttttgctgttttattttttttaaattatgttcta
aacctattttcagtttaggtccctcaataaaaattgctgctgcttaaaaacc
<212> Type : DNA
<211> Length : 3328
      SequenceName : M37722
      SequenceDescription :

Sequence
--------
<213> OrganismName : HUMAN
<400> PreSequenceString :
tttttagaaaaaaaaatatatttccctcctgctccttctgcgttcacaagctaagttgtttatctcggctgcggcgggaactg
cggacggtggcgggcgagcggctcctctgccagagttgatattcactgatggactccaaagaatcattaactcctggtagagaa
gaaaaccccagcagtgtgcttgctcaggagaggggagatgtgatggacttctataaaaccctaagaggaggagctactgtgaag
gtttctgcgtcttcaccctcactggctgtcgcttctcaatcagactccaagcagcgaagacttttggttgattttccaaaaggc
tcagtaagcaatgcgcagcagccagatctgtccaaagcagtttcactctcaatgggactgtatatgggagagacagaaacaaaa
gtgatgggaaatgacctgggattcccacagcagggccaaatcagcctttcctcgggggaaacagacttaaagcttttggaagaa
agcattgcaaacctcaataggtcgaccagtgttccagagaaccccaagagttcagcatccactgctgtgtctgctgcccccaca
gagaaggagtttccaaaaactcactctgatgtatcttcagaacagcaacatttgaagggccagactggcaccaacggtggcaat
gtgaaattgtataccacagaccaaagcacctttgacattttgcaggatttggagttttcttctgggtccccaggtaaagagacg
aatgagagtccttggagatcagacctgttgatagatgaaaactgtttgctttctcctctggcgggagaagacgattcattcctt
ttggaaggaaactcgaatgaggactgcaagcctctcattttaccggacactaaacccaaaattaaggataatggagatctggtt

ttgtcaagccccagtaatgtaacactgccccaagtgaaaacagaaaaagaagatttcatcgaactctgcacccctggggtaatt
aagcaagagaaactggcacagtttactgtcaggcaagctttcctggagcaaatataattggtaataaaatgtctgccatttct
gttcatggtgtgagtacctctggaggacagatgtaccactatgacatgaatacagcatccctttctcaacagcaggatcagaag
cctatttttaatgtcattccaccaattcccgttggttccgaaaattggaataggtgccaaggatctggagatgacaacttgact
tctctggggactctgaacttccctggtcgaacagtttttctaatggctattcaagccccagcatgagaccagatgtaagctct
cctccatccagctcctcaacagcaacaacaggaccacctcccaaactctgcctggtgtgctctgatgaagcttcaggatgtcat
tatggagtcttaacttgtggaagctgtaaagttttcttcaaaagagcagtggaaggacagcacaattacctatgtgctggaagg
aatgattgcatcatcgataaaattcgaagaaaaaactgcccagcatgccgctatcgaaaatgtcttcaggctggaatgaacctg
gaagctcgaaaaacaaagaaaaaaataaaaggaattcagcaggccactacaggagtctcacaagaaacctctgaaaatcctggt
aacaaaacaatagttcctgcaacgttaccacaactcacccctaccctggtgtcactgttggaggttattgaacctgaagtgtta
tatgcaggatatgatagctctgttccagactcaacttggaggatcatgactacgctcaacatgttaggagggcggcaagtgatt
gcagcagtgaaatgggcaaaggcaataccaggtttcaggaacttacacctggatgaccaaatgaccctactgcagtactcctgg
atgtttcttatggcatttgctctggggtggagatcatatagacaatcaagtgcaaacctgctgtgttttgctcctgatctgatt
attaatgagcagagaatgactctaccctgcatgtacgaccaatgtaaacacatgctgtatgtttcctctgagttacacaggctt
caggtatcttatgaagagtatctctgtatgaaaaccttactgcttctctcttcagttcctaaggacggtctgaagagccaagag
ctatttgatgaaattagaatgacctacatcaaagagctaggaaaagccattgtcaagagggaaggaaactccagccagaactgg
cagcggttttatcaactgacaaaactcttggattctatgcatgaagtggttgaaaatctccttaactattgcttccaaacattt
ttggataagaccatgagtattgaattccccgagatgttagctgaaatcatcaccaatcagataccaaaatattcaaatggaaat
atcaaaaaacttctgtttcatcaaaagtgactgccttaataagaatggttgccttaaagaaagtcgaattaatagcttttattg
tataaactatcagtttgtcctgtagaggttttgttgttttatttttttattgttttcatctgttgttttgttttaaatacgcact
acatgtggtttatagagggccaagacttggcaacagaagcagttgagtcgtcatcacttttcagtgatgggagagtagatggtg
aaatttattagttaatatatcccagaaattagaaaccttaatatgtggacgtaatctccacagtcaaagaaggatggcacctaa
accaccagtgcccaaagtctgtgtgatgaactttctcttcatactttttttcacagttggctggatgaaattttctagactttc
tgttggtgtatccccccctgtatagttaggatagcattttgatttatgcatggaaacctgaaaaaaagtttacaagtgtata
tcagaaaagggaagttgtgccttttatagctattactgtctggttttaacaatttcctttatatttagtgaactacgcttgctc
atttttcttacataatttttattcaagttattgtacagctgtttaagatgggcagctagttcgtagctttcccaaataaact
ctaaacattaatcaatcatctgtgtgaaaatgggttggtgcttctaacctgatggcacttagctatcagaagaccacaaaaatt
gactcaaatctccagtattcttgtcaaaaaaaaaaaaaaaaaagctcatatttgtatatatctgcttcagtggagaattatat
aggttgtgcaaattaacagtcctaactggtatagagcacctagtccagtgacctgctgggtaaactgtggatgatggttgcaaa
agactaatttaaaaaataactaccaagaggccctgtctgtacctaacgccctattttgcaatggctatatggcaagaaagctg
gtaaactatttgtctttcaggaccttttgaagtagtttgtataacttcttaaaagttgtgattccagataaccagctgtaacac
agctgagagacttttaatcagacaaagtaattcctctcactaaactttacccaaaaactaaatctctaatatggcaaaaatggc
tagacacccattttcacattcccatctgtcaccaattggttaatctttcctgatggtacaggaaagctcagctactgattttg
tgatttagaactgtatgtcagacatccatgtttgtaaaactacacatccctaatgtgtgccatagagtttaacacaagtcctgt
gaatttcttcactgttgaaaattatttaaacaaaatagaagctgtagtagccctttctgtgtgcaccttaccaactttctgta
aactcaaaacttaacatatttactaagccacaagaaatttgatttctattcaaggtggccaaattatttgtgtaatagaaaact
gaaaatctaatattaaaaatatggaacttctaatatatttttatatttagttatagtttcagatatatatcatattggtattca
ctaatctgggaagggaagggctactgcagctttacatgcaatttattaaaatgattgtaaaatagcttgtatagtgtaaaataa
gaatgattttagatgagattgtttatcatgacatgttatatatttttgtaggggtcaaagaaatgctgatggataacctat
atgatttatagtttgtacatgcattcatacaggcagcgatggtctcagaaaccaaacagtttgctctaggggaagagggagatg
gagactggtcctgtgtgcagtgaaggttgctgaggctctgacccagtgagattacagaggaagttatcctctgcctcccattct
gaccacccttctcattccaacagtgagtctgtcagcgcaggtttagtttactcaatctccccttgcactaaagtatgtaaagta
tgtaaacaggagacaggaaggtggtgcttacatccttaaaggcaccatctaatagcgggttactttcacatacagccctccccc
agcagttgaatgacaacagaagcttcagaagtttggcaatagtttgcatagaggtaccagcaatatgtaaatagtgcagaatct
cataggttgccaataatacactaattcctttctatcctacaacaagagtttatttccaaataaaatgaggacatgtttttgttt
tctttgaatgctttttgaatgttatttgttattttcagtattttggagaaattatttaataaaaaaacaatcatttgcttttg
<212> Type : DNA
<211> Length : 4788
      SequenceName : M10901
      SequenceDescription :

Sequence
--------
<213> OrganismName : HUMAN
<400> PreSequenceString :
ggctataagcgcacggcctcggcgaccctctccgacccggccgccgccgccatgcagccctccagccttctgccgctcgccctc
tgcctgctggctgcacccgcctccgcgctcgtcaggatcccgctgcacaagttcacgtccatccgccggaccatgtcggaggtt
ggggctctgtggaggacctgattgccaaaggccccgtctcaaagtactcccaggcggtgccagccgtgaccgaggggcccatt
cccgaggtgctcaagaactacatggacgcccagtactacggggagattggcatcgggacgcccccccagtgcttcacagtcgtc
ttcgacacgggctcctccaacctgtgggtcccctccatccactgcaaactgctggacatcgcttgctggatccaccacaagtac
aacagcgacaagtccagcacctacgtgaagaatggtacctcgtttgacatccactatggctcgggcagcctctccgggtacctg
agccaggacactgtgtcggtgccctgccagtcagcgtcgtcagcctctgccctgggcggtgtcaaagtggagaggcaggtcttt
ggggaggccaccaagcagccaggcatcaccttcatcgcagccaagttcgatggcatcctgggcatggcctaccccgcatctcc

gtcaacaacgtgctgcccgtcttcgacaacctgatgcagcagaagctggtggaccagaacatcttctccttctacctgagcagg
gacccagatgcgcagcctgggggtgagctgatgctgggtggcacagactccaagtattacaagggttctctgtcctacctgaat
gtcacccgcaaggcctactggcaggtccacctggaccaggtggaggtggccagcgggctgaccctgtgcaaggagggctgtgag
gccattgtggacacaggcacttccctcatggtgggcccggtggatgaggtgcgcgagctgcagaaggccatcggggccgtgccg
ctgattcagggcgagtacatgatcccctgtgagaaggtgtccaccctgcccgcgatcacactgaagctgggaggcaaaggctac
aagctgtccccagaggactacacgctcaaggtgtcgcaggccgggaagaccctctgcctgagcggcttcatgggcatggacatc
ccgccacccagcgggccactctggatcctgggcgacgtcttcatcggccgctactacactgtgtttgaccgtgacaacaacagg
gtgggcttcgccgaggctgcccgcctctagttcccaaggcgtccgcgcgccagcacagaaacagaggagagtcccagagcagga
ggcccctggcccagcggcccctcccacacacacccacacactcgcccgcccactgtcctgggcgccctggaagccggcggccca
agcccgacttgctgttttgttctgtggttttcccctccctgggttcagaaatgctgcctgcctgtctgtctctccatctgtttg
gtgggggtagagctgatccagagcacagatctgtttcgtgcattggaagaccccacccaagcttggcagccgagctcgtgtatc
ctggggctcccttcatctccagggagtcccctccccggccctaccagcgcccgctgggctgagcccctaccccacaccaggccg
tcctcccgggccctcccttggaaacctgccctgcctgagggcccctctgcccagcttgggcccagctgggctctgccaccctac
ctgttcagtgtcccgggcccgttgaggatgaggccgctagaggcctgaggatgagctggaaggagtgagaggggacaaaaccca
ccttgttggagcctgcagggtggtgctgggactgagccagtcccaggggcatgtattggcctggaggtggggttgggattgggg
gctggtgccagccttcctctgcagctgacctctgttgtcctcccttgggcggctgagagccccagctgacatggaaatacagt
tgttggcctccggcctcccctc
<212> Type : DNA
<211> Length : 2038
      SequenceName : M11233
      SequenceDescription :

Sequence
--------
<213> OrganismName : HUMAN
<400> PreSequenceString :
ttggagtcaacactttcgattccaccttgggaagccccaaggagcataagtacaaagctgaagagcacacagtcgttctcact
gtcaccggggagccctgccacttccccttccagtaccaccggcagctgtaccacaaatgtacccacaagggccggccaggccct
cagccctggtgtgctaccaccccaactttgatcaggaccagcgatgggatactgtttggagcccaagaaagtgaaagaccac
tgcagcaaacacagcccctgccagaaaggagggacctgtgtgaacatgccaagcggcccccactgtctctgtccacaacacctc
actggaaaccactgccagaaagagaagtgctttgagcctcagcttctccggtttttccacaagaatgagatatggtatagaact
gagcaagcagctgtggccagatgccagtgcaagggtcctgatgcccactgccagcggctggccagccaggcctgccgcaccaac
ccgtgcctccatggggtcgctgcctagaggtggagggccaccgcctgtgccactgcccggtgggctacaccggacccttctgc
gacgtggacaccaaggcaagctgctatgatggccgcggctcagctaccgcggcctggccaggaccacgctctcgggtgcgccc
tgtcagccgtggcctcggaggccacctaccggaacgtgactgccgagcaagcgcggaactggggactgggcggccacgccttc
tgccggaacccggacaacgacatccgcccgtggtgcttcgtgctgaaccgcgaccggctgagctgggagtactgcgacctggca
cagtgccagaccccaacccaggcggcgcctccgaccccggtgtcccctaggcttcatgtcccactcatgcccgcgcagccggca
ccgccgaagcctcagcccacgacccggaccccgtctcagtcccagaccccgggagccttgccggcgaagcgggagcagccgcct
tccctgaccaggaacggcccactgagctgcgggcagcggctccgcaagagtctgtcttcgatgacccgcgtcgttggcgggctg
gtggcgctacgcggggcgcacccctacatcgccgcgctgtactgggccacagtttctgcgccggcagcctcatcgcccccctgc
tgggtgctgacggccgctcactgcctgcaggaccggcccgcacccgaggatctgacggtggtgctcggccaggaacgccgtaac
cacagctgtgagccgtgccagacgttggccgtgcgctcctaccgcttgcacgaggccttctcgcccgtcagctaccagcacgac
ctggctctgttgcgccttcaggaggatgcggacggcagctgcgcgctcctgtcgccttacgttcagccggtgtgcctgccaagc
ggcgccgcgcgaccctccgagaccacgctctgccaggtggccggctggggccaccagttcgagggggcggaggaatatgccagc
ttcctgcaggaggcgcaggtaccgttcctctccctggagcgctgctcagccccggacgtgcacggatcctccatcctccccggc
atgctctgcgcagggttcctcgagggcggcaccgatgcgtgccagggtgattccggaggcccgctggtgtgtgaggaccaagct
gcagagcgccggctcaccctgcaaggcatcatcagctggggatcgggctgtggtgaccgcaacaagccaggcgtctacaccgat
gtggcctactacctggcctggatccgggagcacaccgtttcctgattgctcagggactcatctttccctccttggtgattccgc
agtgagagagtggctggggcatggaaggcaagattgtgtcccattcccccagtgcggccagctccgcgccaggatggcgcagga
actcaataaagtgctttgaaaatgctg
<212> Type : DNA
<211> Length : 1959
      SequenceName : M11723
      SequenceDescription :

Sequence
--------
<213> OrganismName : HUMAN
<400> PreSequenceString :
ttattaagaattgcagcaagtaagccaacaaggtcttttcaggatgatttcttatatcaagtggtacatttcattttatttac
ttcagtttctggtgaatgtgtgactcagttgttgaaggacacctgctttgaaggaggggacattactacggtcttcacaccaag
cgccaagtactgccaggtagtctgcacttaccacccaagatgtttactcttcactttcacggcggaatcaccatctgaggatcc
cacccgatggtttacttgtgtcctgaaagacagtgttacagaaacactgccaagagtgaataggacagcagcgatttctgggta

ttctttcaagcaatgctcacaccaaataagcgcttgcaacaaagacatttatgtggacctagacatgaagggcataaactataa
cagctcagttgccaagagtgctcaagaatgccaagaaagatgcacggatgacgtccactgccacttttcacgtacgccacaag
gcagtttcccagcctggagcatcgtaacatttgtctactgaagcacacccaaacagggacaccaaccagaataacgaagctcga
taaagtggtgtctggattttcactgaaatcctgtgcactttctaatctggcttgtattagggacattttccctaatacggtgtt
tgcagacagcaacatcgacagtgtcatggctcccgatgcttttgtctgtggccgaatctgcactcatcatcccggttgcttgtt
ttttaccttcttttcccaggaatggcccaaagaatctcaaagaaatctttgtctccttaaaacatctgagagtggattgcccag
tacacgcattaaaaagagcaaagctctttctggtttcagtctacaaagctgcaggcacagcatcccagtgttctgccattcttc
atttaccatgacactgatttcttgggagaagaactggatattgttgctgcaaaaagtcacgaggcctgccagaaactgtgcac
caatgccgtccgctgccagtttttacctataccccagcccaagcatcctgcaacgaagggaagggcaagtgttacttaaagct
ttcttcaaacggatctccaactaaaatacttcacgggagaggaggcatctctggatacacattaaggttgtgtaaaatggataa
tgagtgtaccaccaaaatcaagcccaggatcgttggaggaactgcgtctgttcgtggtgagtggccgtggcaggtgaccctgca
cacaacctcacccactcagagacacctgtgtggaggctccatcattggaaaccagtggatattaacagccgctcactgtttcta
tggggtagagtcacctaagatttgcgtgtctacagtggcattttaaatcaatctgaaataaaagaggacacatctttctttgg
ggttcaagaaataataatccatgatcagtataaaatggcagaaagcgggtatgatattgccttgttgaaactggaaaccacagt
gaattacacagattctcaacgacccatatgcctgccttccaaaggagatagaaatgtaatatacactgattgctgggtgactgg
atggggtacagaaaactaagagacaaaatacaaaatactctccagaaagccaagataccccttagtgaccaacgaagagtgcca
gaagagatacagaggacataaaataacccataagatgatctgtgccggctacagggaaggagggaaggacgcttgcaagggaga
ttcgggaggccctctgtcctgcaaacacaatgaggtctggcatctggtaggcatcacgagctggggcgaaggctgtgctcaaag
ggagcggccaggtgtttacaccaacgtggtcgagtacgtggactggattctggagaaaactcaagcagtgtgaatggttccca
ggggccattggagtccctgaaggacccaggatttgctgggagagggtgttgagttcactgtgccagcatgcttcctccacagta
acacgctgaaggggcttggtgtttgtaagaaaatgctagaagaaaacaaactgtcacaagttgttatgtcc
<212> Type : DNA
<211> Length : 2087
      SequenceName : M13142
      SequenceDescription :

Sequence
--------
<213> OrganismName : HUMAN
<400> PreSequenceString :
aattccgcggcaaccgctccggcaacgccaaccgctccgctgcgcgcaggctgggctgcaggctctcggctgcagcgctgggct
ggtgtgcagtggtgcgaccacggctcacggcagcctcagccacccagatgtaagcgatctggttcccacctcagccttccgagt
agtggatctaggatctggcttccaacatgtggcagctctgggcctccctctgctgcctgctggtgttggccaatgcccggagca
ggccctctttccatcccgtgtcggatgagctggtcaactatgtcaacaaacggaataccacgtggcaggccgggcacaacttct
acaacgtggacatgagctacttgaagaggctatgtggtaccttcctgggtgggcccaagccacccccagagagttatgtttaccg
aggacctgaagctgcctgcaagcttcgatgcacgggaacaatggccacagtgtcccaccatcaaagagatcagagaccagggct
cctgtggctcctgctgggccttcggggctgtggaagccatctctgaccgcatctgcatccacaccaatgcgcacgtcagcgtgg
aggtgtcggcggaggacctgctcacctgctgtggcagcatgtgtggggacggctgtaatggtggctatcctgctgaagcttgga
acttctggacaagaaaaggcctggtttctggtggcctctatgaatcccatgtagggtgcagaccgtactccatccctccctgtg
agcaccacgtcaacggctcccggcccccatgcacgggggagggagatacccccaagtgtagcaagatctgtgagcctggctaca
gcccgacctacaaacaggacaagcactacggatacaattcctacagcgtctccaatagcgagaaggacatcatggccgagatct
acaaaaacggccccgtggagggagctttctctgtgtattcggacttcctgctctacaagtcaggagtgtaccaacacgtcaccg
gagagatgatgggtggccatgccatccgcatcctgggctggggagtggagaatggcacaccctactggctggttgccaactcct
ggaacactgactggggtgacaatggcttctttaaaatactcagaggacaggatcactgcggaatcgaatcagaagtggtggctg
gaattccacgcaccgatcagtactgggaaaagatctaatctgccgtgggcctgtcgtgccagtcctgggggcgagatcggggta
gaaagtcattttattctttaagttcacgtaagatacaagtttcaggcagggtctgaaggactggattggccaaagtcctccaag
gagaccaagtcctggctacatcccagcctgtggttacagtgcagacaggccatgtgagccaccgctgccagcacagagcgtcct
tccccctgtagactagtgccgtgggagtacctgctgcccagctgctgtggcccccctccgtgatccatccatctccagggagcaa
gacagagacgcaggatggaaagcggagttcctaacaggatgaaagttcccccatcagttcccccagtacctccaagcaagtagc
tttccacatttgtcacagaaatcagaggagagatggtgttgggagccctttggagaacgccagtctccaggtcccccctgcatct
atcgagtttgcaatgtcacaacctctctgatcttgtgctcagcatgattctttaatagaagttttatttttcgtgcactctgct
aatcatgtgggtgagccagtggaacagcgggagcctgtgctggtttgcagattgcctcctaatgacgcggctcaaaaggaaacc
aagtggtcaggagttgtttctgacccactgatctctactaccacaaggaaaatagtttaggagaaaccagcttttactgttttt
gaaaaattacagcttcaccctgtcaagttaacaaggaatgcctgtgccaataaaaggtttctccaacttg
<212> Type : DNA
<211> Length : 2002
      SequenceName : M14221
      SequenceDescription :

Sequence
--------
<213> OrganismName : HUMAN
<400> PreSequenceString :

ggatataggcgcccccacacccgggcccggctaagcgccgccgccgctcctcgcctccttgctgcacgatggcctcgctccggg
tggagcgcgccggcggcccgcgtctccctaggacccgagtcgggcggccggcagccgtccgcctcctccttctgctgggcgctg
tcctgaatccccacgaggccctggctcagcctcttcccaccacaggcacaccagggtcagaagggggacggtgaagaactatg
agacagctgtccaattttgctggaatcattataaggatcaaatggatcctatcgaaaaggattggtgcgactggccatgatta
gcaggccttatagcaccctgcgagattgcctggagcactttgcagagttgtttgacctgggcttccccaatcccttggcagaga
ggatcatctttgagactcaccagatccactttgccaactgctccctggtgcagcccaccttctctgacccccagaggatgtac
tcctggccatgatcatagcccccatctgcctcatccccttcctcatcactcttgtagtatggaggagtaaagacagtgaggccc
aggcctagggggcacgagcttctcaacaaccatgttactccacttccccacccccaccaggcctccctcctcccctcctactcc
cttttctcactctcatccccaccacagatccctggattgctgggaatggaagccagggttgggcatggcacaagttctgtaatc
ttcaaaataaaacttttttttga
<212> Type : DNA
<211> Length : 780
      SequenceName : AJ001015
      SequenceDescription :

Sequence
--------
<213> OrganismName : HUMAN
<400> PreSequenceString :
gagcgtgacccagctgcggccggccagccatggagactggagcgctgcggcgcccgcaacttctcccgttgctgctgctgctct
gcggtgggtgtcccagagcaggcggctgcaacgagacaggcatgttggagaggctgcccctgtgtgggaaggctttcgcagaca
tgatgggcaaggtggacgtctggaagtggtgcaacctgtccgagttcatcgtgtactatgagagtttcaccaactgcaccgaga
tggaggccaatgtcgtgggctgctactggcccaacccctggcccagggcttcatcaccggcatccacaggcagttcttctcca
actgcaccgtggacagggtccacttggaggacccccagacgaggttctcatcccgctgatcgttatacccgtcgttctgactg
tcgccatggctggcctggtggtgtggcgcagcaaacgcaccgacacgctgctgtgagggtcccggtgagatggagtggtcaca
cctggcaagctggaagaaagttccctggggatgggagatcgggtgggtgctgccaatctccagctactgtggccacaccccacc
tggtcatgggcagacccctcccttcctgggctgacctgctccctcgaggccagcctgctccctggctgaggctcaggctatccg
cccaagctctttgctcattctagggccagtggaggaaaatgtgataaggccagagcttgtgtgctgggcaagaaatcacctgct
gcatcctgtgctccgcaggctgggccggaagcctctgcctgcaggtttctatgctgtttcttagcacagaatccagcctagcct
tagccgcagtctaggccctgcttggactaggactccttgcttgaccccatctctggttcctgccctggctcctgcaccagcccc
agctcctgcctacatccaggcagaaatataggcaggggctcttggaagacgttccgtgctgtgacctccgagccctcctggtgg
gaagacagctggaaaggctgggaggagaagggaggggctgggggttcccaggagccatgcgtggcctgcagagtccattccatc
atgatgctgtgcccgctatgggctgtgtccatgaccagaggctggagtgggggtgtgttatagcccctcaccgggacttgctgt
gcggatgggcctgggcctccttcctacaggggctcctctgtgggtgaggggccctctggaatggcatcccatgagcttgtggc
ctctatctgctaccatctgtgttttatctgagtaaagttaccttacttctgg
<212> Type : DNA
<211> Length : 1312
      SequenceName : AJ001016
      SequenceDescription :

Sequence
--------
<213> OrganismName : HUMAN
<400> PreSequenceString :
attccggttgttgcaccatggcgtccatggggaccctcgccttcgatgaatatgggcgccctttcctcatcatcaaggatcagg
accgcaagtcccgtcttatggacttgaggccctcaagtctcatataatggcagcaaaggctgtagcaaatacaatgagaacat
cacttggaccaaatgggcttgataagatgatggtggataaggatggagatgtgactgtaactaatgatggggccaccatcttaa
gcatgatggatgttgatcatcagattgccaagctgatggtggaactgtccaagtctcaggatgatgaaattggagatggaacca
caggagtggttgtcctggctggtgccttgttagaagaagcggagcaattgctagaccgaggcattcacccaatcagaatagccg
atggctatgagcaggctgctcgtgttgctattgaacacctggacaagatcagcgatagcgtccttgttgacataaaggacaccg
aacccctgattcagacagcaaaaaccacgctgggctccaaagtggtcaacagttgtcaccgacagatggctgagattgctgtga
atgccgtcctcactgtagcagatatggagcggagagacgttgactttgagcttatcaaagtagaaggcaaagtgggcggcaggc
tggaggacactaaactgattaagggcgtgattgtggacaaggatttcagtcacccacagatgccaaaaaaagtggaagatgcga
agattgcaattctcacatgtccatttgaaccacccaaaccaaaaacaaagcataagctggatgtgacctctgtcgaagattata
aagcccttcagaaatacgaaaaggagaaatttgaagagatgattcaacaaattaaagagactggtgctaacctagcaatttgtc
agtggggctttgatgatgaagcaaatcacttacttcttcagaacaacttgcctgcggttcgctgggtaggaggacctgaaattg
agctgattgccatcgcaacaggagggcggatcgtccccaggttctcagagctcacagccgagaagctgggctttgctggtcttg
tacaggagatctcatttgggacaactaaggataaaatgctggtcatcgagcagtgtaagaactccagagctgtaaccatttta
ttagaggaggaaataagatgatcattgaggaggcgaaacgatcccttcacgatgctttgtgtgtcatccggaacctcatccgcg
ataatcgtgtggtgtatggaggaggggctgctgagatatcctgtgccctggcagttagccaagaggcggataagtgccccacct
tagaacagtatgccatgagagcgtttgccgacgcactggaggtcatccccatggccctctctgaaaacagtggcatgaatccca
tccagactatgaccgaagtccgagccagacaggtgaaggagatgaaccctgctcttggcatcgactgtttgcacaaggggacaa
atgatatgaagcaacagcatgtcatagaaaccttgattggcaaaaagcaacagatatctcttgcaacacaaatggttagaatga

ttttgaagattgatgacattcgtaagcctggagaatctgaagaatgaagacattgagaaaactatgtagcaagatccacttctg
tgattaagtaaatggatgtctcgtgatgcatctacagttatttattgttacatccttttccagacactgtagatgctataataa
aaatagctgtttggtaaccatagtttcacttgttcaaagctgtgtaatcgtgggggtaccatctcaactgcttttgtattcatt
gtattaaaagaatctgtttaaacaacctttatcttctcttcgggtttaagaaacgtttattgtaacagtaattaaatgctgcct
taattg
<212> Type : DNA
<211> Length : 1938
      SequenceName : D43950
      SequenceDescription :

Sequence
--------
<213> OrganismName : HUMAN
<400> PreSequenceString :
gccattctctcacatcccgtgcggtcaggaagcccttcctgaactctgacttcagttcttgctgcggtttctgcccattttttt
catatcctctgacagctgcgaggtcatctctgctctggcttttctccaagcagaacaagtgggggctctggaaaggttaaggga
cctcagtggccaccattatactttgcatctttcctgagaagtgagagttgaaagggaagcaggaaggcccatggtcagattgaa
ggaaggactttttagtttcttttttttttttttgaaatggagtctcgctctgtcattcaggctggagtgcagtggtgcgatctc
agctcactgcagcctccacttcctgggttcacatgattctcctgcctcagcctcccaagtagctgagactacaggcacatgcca
ctacacccagctaacttttgtattttagtagagacggggtttcaccatgttggccaggctggtctcaaactgctaacatcaag
tgatctgctcccctcagcctcccaaagtgctgggattaccggtatgaaccaccacaacctgccaggaattttagtttttagct
tttgcaggagacttcaaggaaaggagacattcctctgtccaggaaacgggtaaggggaccatttctgcattgctggtttcccct
cttggcagggtgggcatgaggcatcactgttcctgctccctcactcctgctcctcatgctcagcctgccagctcggcctcaact
ttgtgtgtctaaagtggaactgaatagtagctgtgagaagataggaaagaggtagtgccaatctccttgcccagatcataaatc
cagactcagcagggtaaccacatgggcaagcacaaggtaggtgcttggggaaaggggaagtaattggcattctgtgtgatacca
aggagaccatttggattttggcttctaccaaagagaatggagaattggttgacctaaatggaaccagtccctttaagtaagggg
aggaaaggggggtgctggaagatggccctcttcccaccacctagatcatagcttgaactgaagccaaggacagagtgctgccccc
ttcggcatttactgatgtgccctctttaaatcatgatgttatctaacccaaacccagacccaggacctagtcacagctccaacc
tacacttcctattaatcttaaaacaaagcgaaacaaacacaaaaagatatcagcattgtagcctccaatctgagcccatttccc
ttctctggctaccatacctccttctcctatatgataccattcactactttgttcaattatccagtctagacctgcatcttgagg
ccacacccagccttctcactccccacacccctctttcctctctcactgctccttcctggtctcttctcatctggccccacctct
aaggagtcctcctgccttctgggttgccctggaaaacagactatccccccctcctagtgaagggagtgggtaggggtttcagccc
cacccctcaggaagatgcgtcttccctgtcctctgctctgtggtacttcctctctggctgatttagcaaacagcacctagacctg
gggccaggcctttggcagtgggacagatccagggataggctacaccaccctgccctgaccctgggattggcatcagcttccaac
cagttcctgccaaagcttgtaagtcctcccgacggccatgaacactacatcttctgcagcacccccctcactaggtgtagagtt
catctctctgctggctatcatcctgctgtcagtggcgctggctgtggggcttcccggcaacagctttgtggtgtggagtatcct
gaaaaggatgcagaagcgctctgtcactgccctgatggtgctgaacctggccctggccgacctggccgtattgctcactgctcc
ctttttccttcacttcctggcccaaggcacctggagttttggactggctggttgccgcctgtgtcactatgtctgcggagtcag
catgtacgccagcgtcctgcttatcacggccatgagtctagaccgctcactggcggtggcccgcccctttgtgtcccagaagct
acgcaccaaggcgatggcccggcgggtgctggcaggcatctgggtgttgtcctttctgctggccacacccgtcctcgcgtaccg
cacagtagtgccctggaaaacgaacatgagcctgtgcttcccgcggtaccccagcgaagggcaccgggccttccatctaatctt
cgaggctgtcacgggcttcctgctgcccttcctggctgtggtggccagctactcggacatagggcgtcggctacaggcccggcg
cttccgccgcagccgccgcaccggccgcctggtggtgctcatcatcctgaccttcgccgccttctggctgccctaccacgtggt
gaacctggctgaggcgggccgcgcgctggccggccaggccgccgggttagggctcgtggggaagcggctgagcctggcccgcaa
cgtgctcatcgcactcgccttcctgagcagcagcgtgaaccccgtgctgtacgcgtgcgccggcggcggcctgctgcgctcggc
gggcgtgggcttcgtcgccaagctgctggagggcacgggttccgaggcgtccagcacgcgccgcgggggcagcctgggccagac
cgctaggagcggccccgccgctctggagcccggcccttccgagagcctcactgcctccagccctctcaagttaaacgaactgaa
ctaggcctggtggaaggaggcgcactttcctcctggcagaatgctagctctgagccagttcagtacctggaggaggagcagggg
cgtggagggcgtggagggcgtgggagcgtgggaggcgggagtggagtggaagaagagggagagatggagcaaagtgagggccga
gtgagagcgtgctccagcctggctcccacaggcagctttaaccattaaaactgaagtctgaa
<212> Type : DNA
<211> Length : 3002
      SequenceName : D89078
      SequenceDescription :

Sequence
--------
<213> OrganismName : HUMAN
<400> PreSequenceString :
ggcccaggctgaagctcagggccctgtctgctctgtggactcaacagtttgtggcaagacaagctcagaactgagaagctgtca
ccacagttctggaggctgggaagttcaagatcaaagtgccagcagattcagtgtcatgtgaggacgtgcttcctgcttcataga
taagagtagcttggagctcggcggcacaaccagcaccatctggtcgcgatggtggacacggaaagcccactctgccccctctcc
ccactcgaggccggcgatctagagagcccgttatctgaagagttcctgcaagaaatgggaaacatccaagagatttcgcaatcc

atcggcgaggatagttctggaagctttggctttacggaataccagtatttaggaagctgtcctggctcagatggctcggtcatc
acggacacgctttcaccagcttcgagcccctcctcggtgacttatcctgtggtccccggcagcgtggacgagtctcccagtgga
gcattgaacatcgaatgtagaatctgcggggacaaggcctcaggctatcattacggagtccacgcgtgtgaaggctgcaagggc
ttctttcggcgaacgattcgactcaagctggtgtatgacaagtgcgaccgcagctgcaagatccagaaaaagaacagaaacaaa
tgccagtattgtcgatttcacaagtgcctttctgtcgggatgtcacacaacgcgattcgttttggacgaatgccaagatctgag
aaagcaaaactgaaagcagaaattcttacctgtgaacatgacatagaagattctgaaactgcagatctcaaatctctggccaag
agaatctacgaggcctacttgaagaacttcaacatgaacaaggtcaaagcccgggtcatcctctcaggaaaggccagtaacaat
ccacctttttgtcatacatgatatggagacactgtgtatggctgagaagacgctggtggccaagctggtggccaatggcatccag
aacaaggaggtggaggtccgcatctttcactgctgccagtgcacgtcagtggagaccgtcacggagctcacggaattcgccaag
gccatcccagcgttcgcaaacttggacctgaacgatcaagtgacattgctaaaatacggagtttatgaggccatattcgccatg
ctgtcttctgtgatgaacaaagacgggatgctggtagcgtatggaaatgggtttataactcgtgaattcctaaaaagcctaagg
aaaccgttctgtgatatcatggaacccaagtttgattttgccatgaagttcaatgcactggaactggatgacagtgatatctcc
ctttttgtggctgctatcatttgctgtggagatcgtcctggccttctaaacgtaggacacattgaaaaaatgcaggagggtatt
gtacatgtgctcagactccacctgcagagcaaccacccggacgatatctttctcttcccaaaacttcttcaaaaaatggcagac
ctccggcagctggtgacggagcatgcgcagctggtgcagatcatcaagaagacggagtcggatgctgcgctgcacccgctactg
caggagatctacagggacatgtactgagttccttcagatcagccacacctttttccaggagttctgaagctgacagcactacaaa
ggagacgggggagcagcacgatttgcacaaatatccaccactttaaccttagagcttggacagtctgagctgtaggtaaccgg
catattattccatatctttgttttaaccagtacttctaagagcatagaactcaaatgctgggggaggtggctaatctcaggact
gggaag
<212> Type : DNA
<211> Length : 1854
      SequenceName : L02932
      SequenceDescription :

Sequence
--------
<213> OrganismName : HUMAN
<400> PreSequenceString :
ggaattccctgatatacacctggaccaccaccaatggatatacaaatggcaaacaattttactccgccctctgcaactcctcag
ggaaatgactgtgacctctatgcacatcacagcacggccaggatagtaatgcctctgcattacagcctcgtcttcatcattggg
ctcgtgggaaacttactagccttggtcgtcattgttcaaaacaggaaaaaaatcaactctaccaccctctattcaacaaatttg
gtgatttctgatatactttttaccacggctttgcctacacgaatagcctactatgcaatgggctttgactggagaatcggagat
gccttgtgtaggataactgcgctagtgttttacatcaacacatatgcaggtgtgaactttatgacctgcctgagtattgaccgc
ttcattgctgtggtgcaccctctacgctacaacaagataaaaaggattgaacatgcaaaaggcgtgtgcatatttgtctggatt
ctagtatttgctcagacactcccactcctcatcaaccctatgtcaaagcaggaggctgaaaggattacatgcatggagtatcca
aactttgaagaaactaaatctcttccctggattctgcttggggcatgtttcataggatatgtacttccacttataatcattctc
atctgctattctcagatctgctgcaaactcttcagaactgccaaacaaaacccactcactgagaaatctggtgtaaacaaaaag
gctctcaacacaattattcttattattgttgtgtttgttctctgtttcacaccttaccatgttgcaattattcaacatatgatt
aagaagcttcgtttctctaatttcctggaatgtagccaaagacattcgttccagatttctctgcactttacagtatgcctgatg
aacttcaattgctgcatggaccctttttatctacttctttgcatgtaaagggtataagagaaaggttatgaggatgctgaaacgg
caagtcagtgtatcgatttctagtgctgtgaagtcagcccctgaagaaaattcacgtgaaatgacagaaacgcagatgatgata
cattccaagtcttcaaatggaaagtgaaatggattgtatttggtttatagtgacgtaaactgtatgacaaactttgcaggact
tcccttataaagcaaaataattgttcagcttccaattagtattcttttatatttctttcattggcgctttcccatctccaact
cggaagtaagcccaagagaacaacataaagcaaacaacataaagcacaataaaaatgcaaataaatattttcatttttatttgt
aaacgaatacaccaaaaggaggcgctcttaataactcccaatgtaaaaagttttgttttaataaaaaattaattattattcttg
ccaacaaatggctagaaaggactgaatagattatatattgccagatgttaatactgtaacatacttttaaataacatatttct
taaatccaaatttctctcaatgttagatttaattccctcaataacaccaatgttttgttttgtttcgttctgggtcataaaact
ttgttaaggaactcttttggaataaagagcaggatgctgcggaattc
<212> Type : DNA
<211> Length : 1643
      SequenceName : L08177
      SequenceDescription :

Sequence
--------
<213> OrganismName : HUMAN
<400> PreSequenceString :
cgtcgctgccggtctcccggagaganctctggagtttgggcggcccgggcggccactaggtactctgatattccgtactaaaca
cgtctgcaagtcaagatgtcgcacccgtcccccaagccaagccctccaaccccagtaaccctcgagtcttctttgacgtggac
atcggaggggagcgagttggtcgaattgtcttagaattgtttgcagatatcgtacccaaaactgcggaaaattttcgtgcactg
tgtacaggagaaaaaggcattggacacacgactgggaaacctctccatttcaaaggatgcccttttcatcgaattattaagaaa
tttatgattcagggtggagacttctcaaatcagaatgggacaggtggagaaagtatttatggtgaaaaatttgaagatgaaaat
ttccattacaagcatgatcgggagggtttactgagcatggcaaatgcaggccgcaacacaaacggttctcagtttttatcaca

acagttccaactcctcatttggatgggaaacatgtggtgtttggccaagtaattaaaggaataggagtggcaaggatattggaa
aatgtggaagtgaaaggtgaaaaacctgctaaattgtgcgttattgcagaatgtggagaattgaaggaaggagatgacgggga
atattcccaaaagatggctctggcgacagtcatccagatttccctgaggatgcggatatagatttaaaagatgtagataaaatt
ttattaataacagaagacttaaaaaacattggaaatactttttcaaatcccagaactgggagatggctattaaaaaatatgca
gaagttttaagatacgtggacagttcaaaggctgttattgagacagcagatagagccaagctgcaacctatagctttaagctgt
gtactgaatattggtgcttgtaaactgaagatgtcaaattggcagggagcaattgacagttgtttagaggctcttgaactagac
ccatcaaataccaaagcattgtaccgcagagctcaaggatggcaaggattaaaagaatatgatcaagcattggctgatcttaag
aaagctcaggggatagcaccagaagataaagctatccaggcagaattgctgaaagtcaaacaaaagataaaggcacagaaagat
aaagagaaggcagtatatgcaaaaatgtttgcttagaaaggattcagttttgcttattgtgtgttgattgtataaatgcaataa
gaaaatgtaaaggtttttgtctatgaatatgatccctaatgtgtttcttttgacaccttagttccttactgtttacagtttagg
agtactgataggggttcatgcttaataaacatgtcacaatacagtaagtaaagtggttttgtttgtttctttgagatggagtct
tgctctgtcacccaggctggagtgcggtggcgcaatctcggctcactgcatcctctgcctcccgggttcaagcaattctcctgc
ctcagcttcccaagtagctgggattacaggcacgtgccaccacgcccagactaattttgtattttagtagagatggggtttc
accatattggtcacgtcacgttggtcttgaactcctgaccttgtgatccaccccgccttggcctcccaaagtgctgggattaca
ggtgtgagccaccgtgcccggccaagtaaaatgtttttaaaatggttatgtgcattattcataaaaaataatggtgtccagtc
tttttaaacttgtaaagacacatcttattgaataaagagatgagagcg
<212> Type : DNA
<211> Length : 1812
      SequenceName : L11667
      SequenceDescription :

Sequence
--------
<213> OrganismName : HUMAN
<400> PreSequenceString :
cagcgcgatgtgggttcccggcttcggatccgcgcgacttccccagcgccggcgctccgggttggagtcatcctcggtgcggcc
gctgtggctgctgctgctgttcctgctggcagccgtgcggccagtgcgcgcctgggagagcggagacctggagttgttcgactt
ggtggaagaggtgcagttgaacttctacgagttcctcggggtgcagcaggatgcttcatctgcagacatcagaaaagcatatcg
taagctttcactaaccttacatccagacaagaataaagatgaaaatgcagaaactcaatttagacaattggtggccatttatga
agttttaaaggatgatgaaagaaggcagaggtatgatgatgttctgatcaatggacttccagattggcgacagcctgtattcta
ctacaggcgagtgagaaaaatgagcaatgctgagctggctttgctcttgttcattattctcacagtgggtcactatgctgtggt
ttggtccatctacctggaaaagcaactggatgaactgcttggtagaaaaaagagagaaaggaagaagaagacaggaagcaagag
tgtggacgcagcaaaacttggtgcttctgaaaagaacgaaagattgcttataaaaccacaatggcatgatttgcttccatgcaa
actggggatttggttttgccttacactaaaagcattgcctcatctaatccaggatgctgggcagttttatgcaaaatataagga
gacaaaattgaaggaaaaagaagacgcactggctagaattgaaattgaaacccttcaaaaacagaagaaagttaaagttaaaaa
accaaaaccagaatttcctgtgtatatgcctttagaaaatacgtatattcagtcttatgatcatggaacttctattgaagaaat
tgaggagcaaatggatgactggctggaaaacaggaaaagaacacagaaaagacaggcacctgaatggacagaagaggacctcag
tcaactgacaagaagtatggttaagttcccaggagggactccaggtcgatgggacaagattgcccatgaattgggtcgatctgt
gacagatgtgacaaccaaagccaaggaactgaaggactcagtcaccagctcccccggaatgaccaggctctctgaacttaagtc
aaatggtcagaattccaggccaatcaagatagccacagccttgccagatgacatcatcacgcagcgggaggactcagcaggggc
catggaggatgaggagcacgaggctgctgagggtgagcaggagtcagcgaccacagaagcccgacctcggaggcggaagtcagc
cagggcggccgaggctgtaaccagagtggagccagaggagaagctgagaggaaagaggcaaaaagactttgatatatcagaaca
aaacgattccagtgacgaagagaaacaacgaaaagaaagaactcgtgctgcagaggaggcgtggactcagagtcagcagaaact
tctggaattggcattacagcaataccctaaaggagcttctgaccgctgggacaaaatagccaaatgtgtcccgtctaagagtaa
ggaagactgcattgctagatacaagctgctggttgaactggtccaaaagaaaaagcaggctaaaagctgaatcttctggaaggt
gacgatcaatgaccatcattttccaaacaagtactcgagaaatctcatgcagaaacttatatttgtacctcagtatttctaaa
catcatgtgccttagtaaaaaaaaaaaa
<212> Type : DNA
<211> Length : 1792
      SequenceName : L16953
      SequenceDescription :

Sequence
--------
<213> OrganismName : HUMAN
<400> PreSequenceString :
cctccacctcccgggttcaagcgattctcctgcctcagcctcccgagtagctgagactacaggcacgcgccaccacgcccagct
aattttgtatctttagtagagacgggctttcaccatgttggccaggatggtctcgatgtcttaacgtcgtgatccggccgcct
cggcctcccaagtgctgggattacaggcgttagccactgcgcccggccccagccaggcagttttaatcgagcgctcacaaccac
tgagacgcagcgaagcacccaccataatatcccaggaggccgaccgccggttcagactttttcttttctttaatccccgtccaa
gggatccgccctcacccccccaccccagccaccccaattccctattccctccccttggacggcgccggggaaaacaagctgctcg
agctttatttcttcggtgcaaccaactcagaatgaattcctccgcccctgcgtgctcagtgagtcggcaccctagcagtgaact
gcatttaaaacctcaggaattgagcgaactctcccagtggctctcctcaccgggatccccttccacgcctcctccccgtgccgc

gcctcagtccgcactgctcattggccgcgtgcctgccaatccgatgcacgtcggctagggcaaagaccgcgaaaaagcgcgtac
acctggctctgggagcgcgcgcctaacgccagccagcagcaggaggcgcgcgaggcaccacggcctggcggccgagagtcaggg
aggaacctcatttacataacggccgcccctctgtctcctggcgggggccggagtcccgcccctcgtccaacttgaaatctgttg
ggtcacgggccagtcactccgacctaggcaagcctgtggtggagctggaagagtttgtgagggcggtcccgggagcggattggg
tctgggagttcccagaggcggctataagaaccgggaactggcgcggggagctgagttgctggtagtgcccgtggtgcttggtt
cgaggtggccgttagttgactccgcggagttcatctccctggtttcccgtcctaacgtcgctcgcctttcagtcaggatgtct
gcccgtggcccggctatcggcatcgacctgggcaccacctattcgtgcgtcggggtcttccaacatggcaaggtggagatcatc
gccaacgaccagggcaatcgcaccaccccagctacgtggccttcacggacaccgagcgcctcatcggcgacgccgccaagaac
caggtggccatgaaccccaccaacaccatcttcgacgccaagaggctgattggacggaaattcgaggatgccacagtgcagtcg
gatatgaaacactggccgttccgggtggtgagcgagggaggcaagcccaaagtgcaagtagagtacaagggggagaccaagacc
ttcttcccagaggagatatcctccatggtcctcacgaagatgaaggagatcgcggaagcctacctgggggcaaggtgcacagc
gcggtcataacggtcccggcctatttcaacgactcgcagcgccaggccaccaaggacgcaggcaccatcacggggctcaatgtg
ctgcgcatcatcaacgagcccacggcggcggccatcgcctacggcctggacaagaagggctgcgcgggcggcgagaagaacgtg
ctcatctttgacctgggcggtggcactttcgacgtgtccatcctgaccatcgaggatggcatcttcgaggtgaagtccacggcc
ggcgatacccacctgggcggtgaggacttcgacaaccgcatggtgagccacctggcggaggagttcaagcgcaagcacaagaag
gacattgggcccaacaagcgcgccgtgaggcggctgcgcaccgcttgcgagcgcgccaagcgcaccctgagctcgtccacgcag
gcgagcatcgagatcgactcgctctacgagggcgtggacttctatacgtccatcacgcgcgcccgcttcgaggagctcaatgcc
gacctctttcgcgggaccctggagccggtggagaaggcgctgcgcgacgccaagctggacaagggccagatccaggagatcgtg
ctggtgggcggctccactcgtatccccaagatccagaagctgctgcaggatttcttcaacggcaaggagctgaacaagagcatc
aaccccgacgaggcggtggcctatggcgccgcggtgcaggcggccatcctcatcggcgacaaatcagagaatgtgcaggacctg
ctgctactcgacgtgaccccgttgtcgctgggcatcgagacagctggcggtgtcatgacccccactcatcaagaggaacaccacg
atccccaccaagcagacgcagaccttcaccacctactcggacaaccagagcagcgtactggtgcaggtatacgagggcgaacgg
gccatgaccaaggacaataacctgctgggcaagttcgacctgaccgggattcccccctgcgcctcgcggggtcccccaaatcgag
gttaccttcgacattgacgccaatggcatccttaacgttaccgccgccgacaagagcaccggtaaggaaaacaaaatcaccatc
accaatgacaaaggtcgtctgagcaaggacgacattgaccggatggtgcaggaggcggagcggtacaaatcggaagatgaggcg
aatcgcgaccgagtcgcggccaaaaacgccctggagtcctatacctacaacatcaagcagacggtggaagacgagaaactgagg
ggcaagattagcgagcaggacaaaaacaagatcctcgacaagtgtcaggaggtgatcaactggctcgaccgaaaccagatggca
gagaaagatgagtatgaacacaagcagaaagagctcgaaagagtttgcaaccccatcatcagcaaactttaccaaggtggtcct
ggcggcggcagcggcggcggcggttcaggagcctccgggggacccaccatcgaagaagtggactaagcttgcactcaagtcagc
gtaaacctctttgcctttctctctctctctttttttttttgtttgtttctttgaaatgtccttgtgccaagtacgagatctattg
ttggaagtctttggtatatgcaaatgaaaggagaggtgcaacaacttagtttaattataaaagttccaaagtttgtttttaaa
aacattattcgaggtttctctttaatgcattttgcgtgtttgctgacttgagcattttgattagttcgtgcatggagatttgt
ttgagatgagaaaccttaagtttgcacacctgttctgtagaagcttggaaacagtaaaatatataggagcttaaattgtttatt
tttatgtactactttaaaactaaactgaacattgcagtaatgttaaggacaggtatacttttgcaaacaaatgcataaatgca
aatgtaaagtaaa
<212> Type : DNA
<211> Length : 3457
      SequenceName : L26336
      SequenceDescription :

Sequence
--------
<213> OrganismName : HUMAN
<400> PreSequenceString :
ataattccatccctcctccaacttttccctctcaagctctgcccttcccagcccagcccagcctacccaacctcatctcttccc
tgtagaccacatcccaccatgttcccctgagcctccaaggaaggggctcaggggccccatggcctcccgctccctgtggcccca
cagcccccgtgggccaggggaagcgccccagaagccgaagtgcccaccatgggcaaccacacgtgggagggctgccacgtggac
tcgcgcgtggaccacctctttccgccatccctctacatctttgtcatcggcgtggggctgcccaccaactgcctggctctgtgg
gcggcctaccgccaggtgcaacagcgcaacgagctgggcgtctacctgatgaacctcagcatcgccgacctgctgtacatctgc
acgctgccgctgtgggtggactacttcctgcaccacgacaactggatccacggcccgggtcctgcaagctctttgggttcatc
ttctacaccaatatctacatcagcatcgccttcctgtgctgcatctcggtggaccgctacctggctgtggcccacccactccgc
ttcgcccgcctgcgccgcgtcaagaccgccgtggccgtgagctccgtggtctgggccacggagctgggcgccaactcggcgccc
ctgttccatgacgagctcttccgagaccgctacaaccacaccttctgctttgagaagttccccatggaaggctgggtggcctgg
atgaacctctatcgggtgttcgtgggcttcctcttcccgtgggcgctcatgctgctgtcgtaccggggcatcctgcgggccgtg
cggggcagcgtgtccaccgagcgccaggagaaggccaagatcaagcggctggccctcagcctcatcgccatcgtgctggtctgc
tttgcgccctatcacgtgctcttgctgtcccgcagcgccatctacctgggccgcccctgggactgcggcttcgaggagcgcgtc
ttttctgcataccacagctcactggctttcaccagcctcaactgtgtggcggaccccatcctctactgcctggtcaacgagggc
gcccgcagcgatgtggccaaggccctgcacaacctgctccgctttctggccagcgacaagccccaggagatggccaatgcctcg
ctcaccctggagacccccactcacctccaagaggaacagcacagccaaagccatgactggcagctgggcggccactccgcctccc
aggggaccaggtgcagctgaagatgctgccgccagcacaatgaaccccgagtggcacagaatccccagtttcccctctcatc
ccacagtcccttctctcctgg
<212> Type : DNA
<211> Length : 1365

SequenceName : L36148
SequenceDescription :

Sequence
--------
<213> OrganismName : HUMAN
<400> PreSequenceString :
tttttcatgctcattgctgtttattgaaacaaaagaatcagaagaagatcagaatgaagacaataataaaaagcagaagcagaa
gtacaagaagaataaagaaagaaagggaaagaattgtaggaaggaaaaacttgtagaagtagagggtggagagtgcgaagaggt
ggagtatgatgggcagtccgatcttttccatctgggctttcagacaatgggatatgtcatggaaggcttctttaaacaccagaa
gaaattcaggataaagctcaaaaagagcaggcaatcgataggggttgaaaatccactcagtaggccacggaaggacttcaagaa
ggttgatcgttctgtcgctggatgttgtaggtgtcctacgtgaaggcaatcgacatctggatggctgtgtgtcggctctttgtg
ttcgctgccttgctggagtatgcc
<212> Type : DNA
<211> Length : 444
SequenceName : AA609779
SequenceDescription :

Sequence
--------
<213> OrganismName : HUMAN
<400> PreSequenceString :
ggtgcgcatgcgcatcacctgggcggcacccgcggtactgcgcctgcgcggtctcgccgcttcccaggtccgctccgcagggcg
agcgcgcgccagtcccactcgtgcgccgctcttcatgtccccgcggtcgaagacggtcacatacgcccccaagaaaacgtcgcc
gaggatccacacaggtactggaggcgaacgatgtccaaggcccggaagccggacaagcagaggcggacgtcaccctgagcaaac
tggatgacgtaatcctgggccgtgagattaaaccagacccccccaatgaggagtgagactgcggggagctttgggatttctgag
caccggatgatgtactccccagccagcaaggggggattcccccaatgg
<212> Type : DNA
<211> Length : 383
SequenceName : AA648280
SequenceDescription :

Sequence
--------
<213> OrganismName : HUMAN
<400> PreSequenceString :
gcggttctggccatgaagcccacctcaggcccagaggaggcccggcggcagcctcggacatccgcgtgttcgccagcaactgct
cgatgcacgggctgggcacgtcttcgggccaggcagcctgagcctgcgccgggggatgtgggcagcggcgtggtcctgtcagtg
gccaccttcctctaccaggtggctgagagggtgcgctactacagggagttccaccaccagactgccctggatgagcgagaaagc
caccggtcagtcttcccggctgtcaccctgtgcaacatcaacccactgcgccgctcgcgcctaacgcccaacgacctgcactgg
gctgggt
<212> Type : DNA
<211> Length : 343
SequenceName : AA449579
SequenceDescription :

Sequence
--------
<213> OrganismName : HUMAN
<400> PreSequenceString :
gggcaagtccagtgcagacgtgcgggctctgacctactgcgacntgcanaagatccagcgggcagatctgctggaggtgctgga
catgtacccggcctttgcggagagcttctggagtaagctggaggtcaccttcaacctgcgggacncacgggggtctccactcat
ccccccgacagctcctggcagccaagaccaccaaggtttctttctcagtgacaacagtcagatgcagcccctnccctggagcat
ctcagatgcatctggcctctgctggagncttactgnagggaaatgcccccaaggca
<212> Type : DNA
<211> Length : 308
SequenceName : H96170
SequenceDescription :

Sequence
--------
<213> OrganismName : HUMAN
<400> PreSequenceString :

atgaaggtctccgtggctgccctctcctgcctacatgcctttttactgcccttggtcccagggccgggtcacaaaagatgcaga
gacagagttcatgaatgtcaaagcttcattggaaagatccagtacttctgggacagattccatgctactagtgctgactgctgc
atctcctacaccccacgaagcatcccgtgttcactcctggagagttactttgaaacgaacagcgagtgctccaagccgggtgtc
atcttcctcaccaagaaggggcgacgtttctgtgccaaccccagtgataagcaagttcaggtttgcatgagaatgctgaagctg
gacacacggatcaagaccaggaagaattga
<212> Type : DNA
<211> Length : 366
      SequenceName : I19355
      SequenceDescription :

Sequence
--------
<213> OrganismName : HUMAN
<400> PreSequenceString :
ccgagccgcagtttccgtgttgtgagtgagtccgggcccgtgtcccctctcccgccgccgccatgggctgcacgttgagcgccg
aagacaaggcggcagtggagcgaagcaagatgatcgaccgcaacttacggaggacggggaaaaagcggccaaagaagtgaagc
tgctgctactcggtgctggagaatctggtaaaagcaccattgtgaaacagatgaaaatcattcatgaggatggctattcagagg
atgaatgtaaacaatataaagtagttgtctacagcaatactatacagtccatcattgcaatcataagagccatgggacggctaa
agattgactttggggaagctgccagggcagatgatgcccggcaattatttgttttagctggcagtgctgaagaaggagtcatga
ctccagaactagcaggagtgattaaacggttatggcgagatggtggggtacaagcttgcttcagcagatccagggaatatcagc
tcaatgattctgcttcatattatctaaatgatctggatagaatatcccagtctaactacattccaactcagcaagatgttcttc
ggacgagagtgaagaccacaggcattgtagaaacacatttcaccttcaaagacctatacttcaagatgtttgatgtaggtggcc
aaagatcagaacgaaaaaagtggattcactgttttgagggagtgacagcaattatcttctgtgtggccctcagtgattatgacc
ttgttctggctgaggacgaggagatgaaccgaatgcatgaaagcatgaaactgtttgacagcatttgtaataacaaatggttta
cagaaacttcaatcattctcttccttaacaagaaagacctttttgaggaaaaaataaagaggagtccgttaactatctgttatc
cagaatacacaggttccaatacatatgaagaggcagctgcctatattcaatgccagtttgaagatctgaacagaagaaaagata
ccaaggagatctatactcacttcacctgtgccacagacacgaagaatgtgcagtttgtttttgatgctgttacagatgtcatca
ttaaaaacaacttaaaggaatgtggactttattgagaagcatggatgttagtgaaagttactacagtgtggagtgttgagacca
gacacctttttgctgtctcatggggcagctacaagcatgaacgggaccagggaatggcagcagcatgcagaatcttagcactctt
tagcacaatattttgtattagggaacttttaattgacatgagatgctaaagtcagacattggaattggaagaactataaagtgt
gattcgatcgtcaagacatcacttggatttcttaatcttaaatgcttatggaagatgtgaagttgaggtgctgcattctagaac
ttcaatatgtagcttactctttttttcccccttcttaaaccaccagtggttcattttaaggtttttcatcaagagaagaat
aactttactaaatttatttctttatttgcaaaagaatctttattaaacaaacaatcttaactatgcacatgatgtgaccaga
tcatcttgaaaatattcctctttagtaggaactctttgttttaactcttggtatggtcagaatataatacttccataattact
tataattctttccggttactggggctataaatacaacttttaaatgaaatttatgttattatcctgctcaaagtaccattatg
gtttccatatggtaattacattggaaagttctgcaataagattctagttctctcttttctttaagcttatggttgaaggttaac
cttgtttgtgcagattaccaaattactgctgatgtagtatgataaagtcagcttcttggatacagacacacccagggcagctgc
caatcagaaatgcaaattgctaaattccaaacagaaccagtgactttgctgctacatatttactaaattgaccactttgattct
tgcttgtttgtctcagttatagggagtttgtacatggtgcctcttgttttccatcttcatggccttttctgttgagaaacatc
taaagtgtattaacagtgcatgcttttactttgtaaatgtggtgccaaatccctgtttgccatcgtttttactgtaccgatgtt
aactgtagtaatccttagccagtatgttcttttgctgaaactgttgcatttgggactttttccactttgtcatgtgtacatt
tttaatctgttatagttggcggcagtattaaaatggtgagtaaag
<212> Type : DNA
<211> Length : 2313
      SequenceName : J03198
      SequenceDescription :

Sequence
--------
<213> OrganismName : HUMAN
<400> PreSequenceString :
ggaacagcttgtccacccgccggccggaccagaagcctttgggtctgaagtgtctgtgagacctcacagaagagcacccctggg
ctccacttacctgccccctgctccttcagggatggaggcaatggcggccagcacttccctgcctgaccctggagactttgaccg
gaacgtgccccggatctgtggggtgtgtggagaccgagccactggctttcacttcaatgctatgacctgtgaaggctgcaaagg
cttcttcaggcgaagcatgaagcggaaggcactattcacctgccccttcaacggggactgccgcatcaccaaggacaaccgacg
ccactgccaggcctgccggctcaaacgctgtgtggacatcggcatgatgaaggagttcattctgacagatgaggaagtgcagag
gaagcgggagatgatcctgaagcggaaggaggaggaggccttgaaggacagtctgcggcccaagctgtctgaggagcagcagcg
catcattgccatactgctggacgcccaccataagacctacgaccccacctactccgacttctgccagttccggcctccagttcg
tgtgaatgatggtggagggagccatccttccaggcccaactccagacacactcccagcttctctggggactcctcctcctcctg
ctcagatcactgtatcacctcttcagacatgatggactcgtccagcttctccaatctggatctgagtgaagaagattcagatga
cccttctgtgaccctagagctgtcccagctctccatgctgccccacctggctgacctggtcagttacagcatccaaaaggtcat
tggctttgctaagatgataccaggattcagagacctcacctctgaggaccagatcgtactgctgaagtcaagtgccattgaggt
catcatgttgcgctccaatgagtccttcaccatggacgacatgtcctggacctgtggcaaccaagactacaagtaccgcgtcag

tgacgtgaccaaagccggacacagcctggagctgattgagcccctcatcaagttccaggtgggactgaagaagctgaacttgca
tgaggaggagcatgtcctgctcatggccatctgcatcgtctccccagatcgtcctggggtgcaggacgccgcgctgattgaggc
catccaggaccgcctgtccaacacactgcagacgtacatccgctgccgccacccgcccccgggcagccacctgctctatgccaa
gatgatccagaagctagccgacctgcgcagcctcaatgaggagcactccaagcagtaccgctgcctctccttccagcctgagtg
cagcatgaagctaacgccccttgtgctcgaagtgtttggcaatgagatctcctgactaggacagcctgtgcggtgcctgggtgg
ggctgctcctccagggccacgtgccaggcccggggctggcggctactcagcagccctcctcacccgtctggggttcagcccctc
ctctgccacctcccctatccacccagcccattctctctcctgtccaacctaacccctttcctgcgggcttttccccggtccctt
gagacctcagccatgaggagttgctgtttgtttgacaaagaaacccaagtgggggcagagggcagaggctggaggcaggccttg
cccagagatgcctccaccgctgcctaagtggctgctgactgatgttgagggaacagacaggagaaatgcatccattcctcaggg
acagagacacctgcacctcccccactgcaggccccgcttgtccagcgcctagtggggtctccctctcctgccttactcacgat
aaataatcggcccacagctcccaccccacccccttcagtgcccaccaacatcccattgccctggttatattctcacgggcagta
gctgtggtgaggtgggttttcttcccatcactggagcaccaggcacgaacccacctgctgagagacccaaggaggaaaaacaga
caaaaacagcctcacagaagaatatgacagctgtccctgtcaccaagctcacagttcctcgccctgggtctaaggggttggttg
aggtggaagccctccttccacggatccatgtagcaggactgaattgtccccagtttgcagaaaagcacctgccgacctcgtcct
cccccctgccagtgccttacctcctgcccaggagagccagccctccctgtcctcctcggatcaccgagagtagccgagagcctgc
tcccccacccccctccccaggggagagggtctggagaagcagtgagccgcatcttctccatctggcagggtgggatggaggagaa
gaattttcagaccccagcggctgagtcatgatctccctgccgcctcaatgtggttgcaaggccgctgttcaccacagggctaag
agctaggctgccgcaccccagagtgtgggaagggagagcggggcagtctcgggtggctagtcagagagagtgtttggggggttcc
gtgatgtagggtaaggtgccttcttattctcactccaccacccaaaagtcaaaaggtgcctgtgaggcaggggcggagtgatac
aacttcaagtgcatgctctctgcaggtcgagcccagcccagctggtgggaagcgtctgtccgtttactccaaggtgggtctttg
tgagagtgagctgtaggtgtgcgggaccggtacagaaaggcgttcttcgaggtggatcacagaggcttcttcagatcaatgctt
gagtttggaatcggccgcattccctgagtcaccaggaatgttaaagtcagtgggaacgtgactgccccaactcctggaagctgt
gtccttgcacctgcatccgtagttccctgaaaacccagagaggaatcagacttcacactgcaagagccttggtgtccacctggc
cccatgtctctcagaattcttcaggtggaaaaacatctgaaagccacgttccttactgcagaatagcatatatatcgcttaatc
ttaaatttattagatatgagttgttttcagactcagactccatttgtattatagtctaatatacagggtagcaggtaccactga
tttggagatatttatgggggagaacttacattgtgaaacttctgtacattaattattattgctgttgttatttacaagggtc
tagggagagacccttgtttgattttagctgcagaactgtattggtccagcttgctcttcagtgggagaaaaacacttgtaagtt
gctaaacgagtcaatcccctcattcaggaaaactgacagaggagggcgtgactcacccaagccatatataactagctagaagtg
ggccaggacaggccgggcgcggtggctcacgcctgtaatcccagcagtttgggaggtcgaggtaggtggatcacctgaggtcgg
gagttcgagaccaacctgaccaacatggagaaaccctgtctctattaaaaatacaaaaaaaaaaaaaaaaaaaaatagccgggc
atggtggcgcaagcctgtaatcccagctactcaggaggctgaggcagaagaattgaacccaggaggtggaggttgcagtgagct
gagatcgtgccgttactctccaacctggacaacaagagcgaaactccgtcttagaagtggaccaggacaggaccagattttgga
gtcatggtccggtgtccttttcactacaccatgtttgagctcagacccccactctcattccccaggtggctgacccagtccctg
gggaagccctggatttcagaaagagccaagtctggatctgggaccctttccttccttccctggcttgtaactccaccaagccc
atcagaaggagaaggaaggagactcacctctgcctcaatgtgaatcagaccctaccccaccacgatgtgccctggctgctgggc
tctccacctcaggccttggataatgctgttgcctcatctataacatgcatttgtctttgtaatgtcaccaccttcccagctctc
cctctggccctgcttcttcggggaactcctgaaatatcagttactcagccctgggccccaccacctaggccactcctccaaagg
aagtctaggagctgggaggaaaagaaaagaggggaaaatgagttttatggggctgaacggggagaaaaggtcatcatcgattc
tactttagaatgagagtgtgaaatagacatttgtaaatgtaaaacttttaaggtatatcattataactgaaggagaaggtgccc
caaaatgcaagattttccacaagattcccagagacaggaaaatcctctggctggctaactggaagcatgtaggagaatccaagc
gaggtcaacagagaaggcaggaatgtgtggcagatttagtgaaagctagagatatggcagcgaaaggatgtaaacagtgcctgc
tgaatgatttccaaagagaaaaaaagtttgccagaagtttgtcaagtcaaccaatgtagaaagctttgcttatggtaataaaa
tggctcatacttatatagcacttactttgtttgcaagtactgctgtaaataaatgctttatgcaaacc
<212> Type : DNA
<211> Length : 4604
SequenceName : J03258
SequenceDescription :

Sequence
--------
<213> OrganismName : HUMAN
<400> PreSequenceString :
ctcgccagctcttccgctcctctcacagccgccagacccgcctgctgagccccatggcccgcgctgctctctccgccgcccca
gcaatccccggctcctgcgagtggcactgctgctcctgctcctggtagccgctggccggcgcgcagcaggagcgtccgtggcca
ctgaactgcgctgccagtgcttgcagaccctgcagggaattcaccccaagaacatccaaagtgtgaacgtgaagtcccccggac
cccactgcgcccaaaccgaagtcatagccacactcaagaatgggcggaaagcttgcctcaatcctgcatcccccatagttaaga
aaatcatcgaaaagatgctgaacagtgacaaatccaactgaccagaagggaggaggaagctcactggtggctgttcctgaagga
ggccctgcccttataggaacagaagaggaaagagagacacagctgcagaggccacctggattgtgcctaatgtgtttgagcatc
gcttaggagaagtcttctatttatttatttattcattagttttgaagattctatgttaatattttaggtgtaaaataattaagg
gtatgattaactctacctgcacactgtcctattatattcattctttttgaaatgtcaaccccaagttagttcaatctggattca
tatttaatttgaaggtagaatgttttcaaatgttctccagtcattatgttaatatttctgaggagcctgcaacatgccagccac
tgtgatagaggctggcggatccaagcaaatggccaatgagatcattgtgaaggcaggggaatgtatgtgcacatctgttttgta
actgtttagatgaatgtcagttgttatttattgaaatgatttcacagtgtgtggtcaacatttctcatgttgaaactttaagaa

ctaaaatgttctaaatatcccttggacattttatgtctttcttgtaaggcatactgccttgtttaatggtagttttacagtgtt
tctggcttagaacaaaggggcttaattattgatgttttcgga
<212> Type : DNA
<211> Length : 1050
      SequenceName : J03561
      SequenceDescription :

Sequence
--------
<213> OrganismName : HUMAN
<400> PreSequenceString :
gaattccgccagccccgccagtccccgcgcagtccccgcgcagtcccagcgccaccgggcagcagcggcgccgtgctcgctcca
gggcgcaaccatgtcgccatttcttcggattggcttgtccaactttgactgcgggtcctgccagtcttgtcagggcgaggctgt
taacccttactgtgctgtgctcgtcaaagagtatgtcgaatcagagaacgggcagatgtatatccagaaaaagcctaccatgta
cccaccctgggacagcacttttgatgcccatatcaacaagggaagagtcatgcagatcattgtgaaaggcaaaaacgtggacct
catctctgaaaccaccgtggagctctactcgctggctgagaggtgcaggaagaacaacgggaagacagaaatatggttagagct
gaaacctcaaggccgaatgctaatgaatgcaagatactttctggaaatgagtgacacaaaggacatgaatgaatttgagacgga
aggcttctttgctttgcatcagcgccggggtgccatcaagcaggcaaaggtccaccacgtcaagtgccacgagttcactgccac
cttcttcccacagcccacatttgctctgtctgccacgagtttgtctggggcctgaacaaacagggctaccagtgccgacaatg
caatgcagcaattcacaagaagtgtattgataaagttatagcaaagtgcacaggatcagctatcaatagccgagaaaccatgtt
ccacaaggagagattcaaaattgacatgccacacagatttaaagtctacaattacaagagcccgaccttctgtgaacactgtgg
gaccctgctgtggggactggcacggcaaggactcaagtgtgatgcatgtggcatgaatgtgcatcatagatgccagacaaaggt
ggccaacctttgtggcataaaccagaagctaatggctgaagcgctggccatgattgagagcactcaacaggctcgctgcttaag
agatactgaacagatcttcagagaaggtccggttgaaattggtctcccatgctccatcaaaaatgaagcaaggctgccatgttt
accgacaccgggaaaaagagagcctcagggcatttcctgggagtctccgttggatgaggtggataaaatgtgccatcttccaga
acctgaactgaacaaagaaagaccatctctgcagattaaactaaaaattgaggattttatcttgcacaaaatgttggggaaagg
aagttttggcaaggtcttcctggcagaattcaagaaaaccaatcaatttttcgcaataaaggccttaaagaaagatgtggtctt
gatggacgatgatgttgagtgcacgatggtagagaagagagttctttccttggcctgggagcatccgtttctgacgcacatgtt
ttgtacatttcagaccaaggaaaacctctttttgtgatggagtacctcaacggaggggacttaatgtaccacatccaaagctg
ccacaagttcgacctttccagagcgacgttttatgctgctgaaatcattcttggtctgcagttccttcattccaaaggaatagt
ctacagggacctgaagctagataacatcctgttagacaaagatggacatatcaagatcgcggattttggaatgtgcaaggagaa
catgttaggagatgccaagacgaataccttctgtgggacacctgactacatcgccccagagatcttgctgggtcagaaatacaa
ccactctgtggactggtggtccttcggggttctcctttatgaaatgctgattggtcagtcgcctttccacgggcaggatgagga
ggagctcttccactccatccgcatggacaatcccttttacccacggtggctggagaaggaagcaaaggaccttctggtgaagct
cttcgtgcgagaacctgagaagaggctgggcgtgaggggagacatccgccagcaccctttgtttcgggagatcaactgggagga
acttgaacggaaggagattgacccaccgttccggccgaaagtgaaatcaccatttgactgcagcaatttcgacaaagaattctt
aaacgagaagccccggctgtcatttgccgacagagcactgatcaacagcatggaccagaatatgttcaggaacttttccttcat
gaacccccggatggagcggctgatatcctgaatcttgcccctccagagacaggaaagaatttgccttgtccctgggaactggtt
caagagacactgcttgggttcctttttcaacttggaaaaagaaagaaacactcaacaataaagactgagacccgttcgcccca
tgtgacttttatctgtagcagaaaccaagtctacttcactaatgacgatgccgtgtgtctcgtctcctgacatgtctcacagac
gctcctgaagttaggtcattactaaccatagttatttacttgaaagatgggtctccgcacttggaaaggtttcaagacttgata
ctgcaataaattatggctcttcacctggcgccaactgctgatcaacgaaatgcttgttgaatcaggggcaaacggagtacaga
cgtctcaagactgaaacggccccattgcctggtctagtagcggatctcactcagccgcagacaagtaatcactaacccgtttta
ttctattcctatctgtggatgggtaaatgctgggggccagccctggataggtttttatgggaattc
<212> Type : DNA
<211> Length : 2754
      SequenceName : L01087
      SequenceDescription :

Sequence
--------
<213> OrganismName : HUMAN
<400> PreSequenceString :
gcggaagagcagaacgtttgggagtgtgcagctcctgggcccggctcaggcccgtcgcggaggcgcggcgcaggggagcgctcg
ggtgggagtctcgctcctccaccagtttattgcgacgtagcatccaggcctaggcctccccgtccatccccgccggactcgggc
ctctggcagcagcgggtgacgcagacggaacatcatgtcgtccgcggccgagcctccgccacccccgcctcccgagagcgcgcc
ttccaagcccgcagcctcgatcgccagcggcgggagcaacagcagcaacaaaggcggccccgaaggcgtcgcggcgcaggcggt
tgcgtctgcggccagcgctggtcccgcagacgccgagatggaggaaatatttgatgatgcgtcacctggaaagcaaaaggaaat
ccaagaaccagatcctacctatgaagaaaaaatgcaaactgaccgggcaaatagattcgagtatttattaaagcagacagaact
tttgcacatttcattcaacctgctgctcagaagactccaacttcacctttgaagatgaaaccagggcgcccacgaataaaaaa
agatgagaagcagaacttactatccgttggcgattaccgacaccgtagaacagagcaagaggaggatgaagagctattaacaga
aagctccaaagcaaccaatgtttgcactcgatttgaagactctccatcgtatgtaaaatggggtaaactgagagattatcaggt
ccgaggattaaactggctcatttctttgtatgagaatggcatcaatggtatccttgcagatgaaatgggcctaggaaagactct

tcaaacaatttctcttcttgggtacatgaaacattatagaaacattcctgggcctcatatggttttggttcctaagtctacatt
acacaactggatgagtgaattcaagagatgggtaccaacacttagatctgtttgtttgataggagataaagaacaaagagctgc
ttttgtcagagacgttttattaccgggagaatgggatgtatgtgtaacatcttatgaaatgcttattaaagagaagtctgtgtt
caaaaaatttaattggagatacttagtaatagatgaagctcacaggatcaaaaatgaaaaatctaagttgtcagaaatagtgag
ggaattcaagactacaaatagactattattaactggaacacctcttcagaacaacttgcatgagctgtggtcacttcttaactt
tctgttgccagatgtgtttaattcagcagatgactttgattcctggtttgatacaaacaactgccttggggatcaaaaactagt
tgagaggcttcatatggttttgcgtccattcctccttcgtcgaattaaggctgatgttgaaaagagtttgcctccaaagaagga
agtaaaaatctatgtgggcctcagcaaaatgcaaagggaatggtatactcggatattaatgaaggatatagatatactcaactc
agcaggcaagatggacaaaatgaggttattgaacatcctaatgcagttgagaaaatgttgtaatcatccatatctctttgatgg
agcagaacctggtccaccttatacaacagatatgcatctagtaaccaacagtggcaaaatggtggttttagacaagctgctccc
taagttaaaagaacaaggttcacgagtactaatcttcagtcaaatgacaagggtattggacattttggaagattattgcatgtg
gagaaattatgagtactgcaggttggatggtcagacaccccatgatgagagacaagactccatcaatgcatacaatgaaccaaa
cagcacaaagtttgttttcatgttaagcacgcgtgctggtggtcttggcatcaatcttgcgactgctgatgtagtaatttgta
tgattctgattggaatccccaagtagatcttcaggctatggaccgagcacatagaattgggcagactaagacagtcagagtgtt
ccgctttataactgataacactgtagaagaaagaatagtagaacgtgctgagatgaaactcagactggattcaatagtcattca
acaagggaggcttgtggatcagaatctgaacaaaattgggaaagatgaaatgcttcaaatgattagacatggagcaacacatgt
gtttgcttcaaaggaaagtgagatcactgatgaagatatcgatggtattttggaaagaggtgcaaagaagactgcagagatgaa
tgaaaagctctccaagatggcgaaagttcacttagaaactttacaatggatacagagtcaagtgtttataacttcgaaggaga
agactatagagaaaaacaaaagattgcattcacagagtggattgaaccacctaaacgagaaagaaaagccaactatgccgttga
tgcatatttcagggaagctcttcgtgttagtgaacctaaagcacccaaggctcctcgacctccaaaacaacccaatgttcagga
tttccagttctttcctccacgtttatttgaattactggaaaaagaaattctgttttacagaaaaactattgggtacaaggtacc
tcgaaatcctgagctgcctaacgcagcacaggcacaaaaagaagaacagcttaaaattgatgaagctgaatcccttaatgatga
agagttagaggaaaaagagaagcttctaacacagggatttaccaattggaataagagagattttaaccagtttatcaaagctaa
tgagaagtggggtcgtgatgatattgaaaatatagcaagagaagtagaaggcaaaactccagaagaagtcattgaatattcagc
tgtgttttgggaaaggtgcaacgagctccaggacatagagaagattatggctcagattgaaaggggagaggcgagaattcaaag
aagaataagcatcaagaaagcacttgacacaaagattggacggtacaaagcaccttttcatcagctgagaatatcatatggtac
taacaaaggaaaaaactatactgaagaagaagatcgttttctgatttgtatgcttcacaaacttggatttgacaaagaaaatgt
ttatgatgaattgcgacagtgtattcgcaactctcctcagttcagatttgactggtttcttaagtccagaactgcaatggagct
ccagaggagatgtaataccttaattactttgattgaaagagaaaacatggaactagaagaaaaggagaaggcagagaaaaagaa
acgaggaccaaagccttcaacacagaaacgtaaaatggatggcgcacctgatggtcgaggaagaaaaaagaagctgaaactatg
aatatgtttttgtttcataatcactaactttaaaccagtagttctttaatttacgggtcttcataagatgtactgtacaatgct
caattgttatgtcatttaaagacatcaggttcatctgtttactgagctagaaacatagtatgtagtttcactttttaaatgca
acagctgtgctgaaatttttttatcattaacacttgaagtaataaaataggcttcatttattaaaaaaaaaaaaaaa
<212> Type : DNA
<211> Length : 3606
      SequenceName : AB010882
      SequenceDescription :

Sequence
--------
<213> OrganismName : HUMAN
<400> PreSequenceString :
atggcggaaggtggaggctgccgtgagcgaccggatgcggagactcagaagtccgagcttggacccttaatgaggaccacactc
caacgcggggcgcagtggtatcttattgacagccggtggttcaagcagtggaagaagtatgtgggctttgacagctgggacatg
tacaatgtgggtgaacataacctatttcctggcccaatagacaactctgggctattttcagatcctgagagtcagaccttgaaa
gaacacttaattgatgaattggactatgtattggtccctaccgaggcgtggaataaactactaaactggtacggctgtgtagaa
ggccagcaacccatcgtcagaaaagttgtggagcatggcctgtttgtcaagcactgcaaagtcgaggtgtatttgctggaactg
aagctctgtgagaacagtgaccccaccaatgtgctgagttgccatttcagcaaggcagacaccattgcaaccatcgagaaagag
atgcggaagctattcaacatccctgcggagcgtgaaacacggctctggaacaaatacatgagcaacacctacgagcagttgagc
aagctagacaacactgtccaggatgctgggctataccagggtcaggtgctagtaattgagcctcaaaatgaagatggcacatgg
cccaggcagaccttgcagtcaaatggatctggcttttctgcttcgtataattgtcaggagccaccatcctctcatatacaacct
gggctctgtggacttggaaacctgggaaacacctgcttcatgaactccgctttgcagtgtttgagcaacactgcaccactgact
gactactttctcaaagatgagtatgaagccgaaatcaacagagacaaccctctggggatgaaaggggaaattgcagaagcctat
gctgaactcattaagcagatgtggtctggaagggacgcccatgtggcacctcgcatgttcaaaactcaagtaggacgttttgct
cctcaatttctggctaccagcaacaagattctcaggagctgctggcctttcttctagatggattgcatgaagatctgaaccgg
gtaaagaaaaagccctacttggagctgaaggatgccaatgggcggccagatgcggtggtggcaaaggaagcctgggagaatcac
aggttgaggaatgattctgtgattgtggatactttccatggcctcttcaaatctactttggtttgcccagaatgtgctaaggtt
tctgtgacctttgacccatttgctatctaacgctgccactgcccttgaagaaagatcgagttatggaggttttcctggttcct
gctgaccctcactgcagacctactcagtaccgtgtgactgtgccgctgatggggctgtgtccgacctgtgcgaggctctctcc
aggctgtctggcattgctgcagaaaatatggtggtcgcagatgtgtataatcaccgattccacaaaattttccaaatggatgaa
ggtttaaaccacatcatgcctcgggatgacattttcgtgtacgaggtctgcagcacttccgtggatggctcggaatgtgtcacg
cttccagtctacttcagggagaggaagtccaggccatcaagcacttcctccgcatcagcgctatatgggcagccactattgctt
tctgtccccaagcacaagttaacccttgagtctttgtaccaggctgtttgtgatcgtatcagccgctatgtgaaacagccttta

cctgatgagtttggcagctcacccttggagccaggggcctgcaatggctccaggaacagctgtgaaggagaagatgaggaagaa
atggagcatcaggaagaaggcaaagagcagctttcagaaacagaaggcagtggggaagatgagccaggaaatgaccccagtgag
accacccaaaagaagatcaaggccagccctgcccaaaaaggctttttaccttcagtcttgtgaactcctatggaacagctgac
ataaattcacttgcagctgatggaaaactacttaaactcaactctcgatctacactggccatggattgggacagtgaaactcgg
agactttactatgatgagcaagaatctgaggcctacgagaagcatgtgagcatgttgcagcctcagaagaagaagaagaccaca
gtggccctgagagactgcatcgagctcttcaccaccatggagacccttggggagcatgacccctggtactgtcccaactgtaag
aagcatcaacaggccacaaaaaagtttgacctatggtccttgcccaagatcctggtggtccacctcaaacgtttctcctacaac
agatactggagggataagctcgacacagtcgtagaattcccaatcagagggctgaacatgtccgagtttgtctgtaacctgtca
gcaaggccttatgtgtacgacctcattgccgtgtccaatcattatggagccatggggggttggccactacactgcatatgcgaag
aacaaactgaatggtaaatggtattactttgatgatagcaacgtgtccctggcctctgaggatcagatagtgactaaagcagct
tatgtgctattttaccaacgtcgagatgatgaattttataagacaccttcacttagcagttctggttcctctgatggagggaca
cgaccaagcagctctcagcagggctttggggatgatgaggcttgcagcatggacaccaactaa
<212> Type : DNA
<211> Length : 2751
SequenceName : AF017306
SequenceDescription :

Sequence
--------
<213> OrganismName : HUMAN
<400> PreSequenceString :
ccgcggcggcggagaagcagctcgccagccagcagcccgccagccgccgggaggttcgatacaagaggctgttttcctagcgtg
gcttgctgcctttggtaagaacatgtcgtccatcttgccattcacgccgccagttgtgaagagactgctgggatggaagaagtc
agctggtgggtctggaggagcaggcggaggagagcagaatgggcaggaagaaaagtggtgtgagaaagcagtgaaaagtctggt
gaagaagctaaagaaaacaggacgattagatgagcttgagaaagccatcaccactcaaaactgtaatactaaatgtgttaccat
accaagcacttgctctgaaatttggggactgagtacaccaaatacgatagatcagtgggatacaacaggcctttacagcttctc
tgaacaaaccaggtctcttgatggtcgtctccaggtatcccatcgaaaaggattgccacatgttatatattgccgattatggcg
ctggcctgatcttcacagtcatcatgaactcaaggcaattgaaaactgcgaatatgcttttaatcttaaaaaggatgaagtatg
tgtaaacccttaccactatcagagagttgagacaccagttttgcctccagtattagtgccccgacacaccgagatcctaacaga
acttccgcctctggatgactatactcactccattccagaaaacactaacttcccagcaggaattgagccacagagtaattatat
tccagaaacgccacctcctggatatatcagtgaagatggagaaacaagtgaccaacagttgaatcaaagtatggacacaggctc
tccagcagaactatctcctactactctttcccctgttaatcatagcttggatttacagccagttacttactcagaacctgcatt
ttggtgttcaatagcatattatgaattaaatcagagggttggagaaaccttccatgcatcacagccctcactcactgtagatgg
ctttacagacccatcaaattcagagaggttctgcttaggtttactctccaatgttaaccgaaatgccacggtagaaatgacaag
aaggcatataggaagaggagtgcgcttatactacataggtggggaagtttttgctgagtgcctaagtgatagtgcaatctttgt
gcagagccccaattgtaatcagagatatggctggcaccctgcaacagtgtgtaaaattccaccaggctgtaatctgaagatctt
caacaaccaggaatttgctgctcttctggctcagtctgttaatcagggttttgaagccgtctatcagctaactagaatgtgcac
cataagaatgagttttgtgaaagggtggggagcagaataccgaaggcagacggtaacaagtactccttgctggattgaacttca
tctgaatggacctctacagtggttggacaaagtattaactcagatgggatccccttcagtgcgttgctcaagcatgtcataaag
cttcaccaatcaagtcccatgaaaagacttaatgtaacaactcttctgtcatagcattgtgtgtggtccctatggactgtttac
tatccaaaagttcaagagagaaaacagcacttgaggtctcatcaattaaagcaccttgtggaatctgtttcctatatttgaata
ttagatgggaaaattagtgtcataaagatc
<212> Type : DNA
<211> Length : 1710
SequenceName : AF027964
SequenceDescription :

Sequence
--------
<213> OrganismName : HUMAN
<400> PreSequenceString :
cgtccttcctcagccgcgggtgatcgtagctcggaaatggcgggatttggtgctatggagaaattttggtagaatataagagt
gcagtggagaagaaactggcagagtacaaatgtaacaccaacacagcaattgaactaaaattagttcgttttcctgaagatctt
gaaaatgacattagaactttctttcctgagtatacccatcaactctttggggatgatgaaactgcttttggttacaagggtcta
aagatcctgttatactatattgctggtagcctgtcaacaatgttccgtgttgaatatgcatctaaagttgatgagaactttgac
tgtgtagaggcagatgatgttgagggcaaaattagacaaatcattccacctggattttgcacaaacacgaatgatttcctttct
ttactggaaaaggaagttgatttcaagccattcggaaccttacttcatacctactcagttctcagtccaacaggaggagaaaac
tttacctttcagatatataaggctgacatgacatgtagaggctttcgagaatatcatgaaaggcttcagacctttttgatgtgg
tttattgaaactgctagctttattgacgtggatgatgaaagatggcactactttctagtatttgagaagtataataaggatgga
gctacgctctttgcgaccgtaggctacatgacagtctataattactatgtgtacccagacaaaacccggccacgtgtaagtcag
atgctgatttgactccatttcaaggtcaaggccatggtgctcaacttcttgaaacagttcatagatactacactgaatttcct
acagttcttgatattacagcggaagatccatccaaaagctatgtgaaattacgagactttgtgcttgtgaagctttgtcaagat
ttgccctgtttttcccgggaaaaattaatgcaaggattcaatgaagatatggcgatagaggcacaacagaagttcaaaataaat

aagcaacacgctagaagggtttatgaaattcttcgactactggtaactgacatgagtgatgccgaacaatacagaagctacaga
ctggatattaaaagaagactaattagcccatataagaaaaagcagagagatcttgctaagatgagaaaatgtctcagaccagaa
gaactgacaaaccagatgaaccaaatagaaataagcatgcaacatgaacagctggaagagagtttcaggaactagtggaagat
taccggcgtgttattgaacgacttgctcaagagtaaagattatactgctctgtacaggaagcttgcaaattttctgtacaatgt
gctgtgaaaaatctgatgactttaatttaaaatcttgtgacattttgcttatactaaaagttatctatctttagttgaatatt
ttcttttggagagattgtatattttaaaatactgtttagagtttatgagcatatattgcatttaaagaaagataaagcttctga
aatactactgcaattgcttcccttcttaaacagtataataaatgcttagttgtgat
<212> Type : DNA
<211> Length : 1568
      SequenceName : AF030424
      SequenceDescription :

Sequence
--------
<213> OrganismName : HUMAN
<400> PreSequenceString :
atggcgagcatgcgagtggtgaaggagctggaggatcttcagaagaagcctcccccatacctgcggaacctgtccagcgatgat
gccaatgtcctggtgtggcacgctctcctcctacccgaccaacctccctaccacctgaaagccttcaacctgcgcatcagcttc
ccgccggagtatccgttcaagcctcccatgatcaaattcacaaccaagatctaccaccccaacgtggacgagaacggacagatt
tgcctgcccatcatcagcagtgagaactggaagccttgcaccaagacttgccaagtcctggaggccctcaatgtgctggtgaat
agaccgaatatcagggagcccctgcggatggacctcgctgacctgctgacacagaatccggagctgttcagaaagaatgccgaa
gagttcaccctccgattcggagtggaccggccctcctaa
<212> Type : DNA
<211> Length : 459
      SequenceName : AF031141
      SequenceDescription :

Sequence
--------
<213> OrganismName : HUMAN
<400> PreSequenceString :
ggtgacaaagagccaacagagacaataggagacttgtcaatttgtcttgatggctacagttagagtctgaagttgttaccaat
ggtgaaactacatgttcagaaagtgcttctcagaatgatgatggctccagatccaaggatgaaacaagagtgagcacaaatgga
tcagatgaccctgaagatgcaggagctggtgaaaataggagagtcagtgggaataattctccatcactctcaaatggtggtttt
aaaccttctagacctccaagaccttcacgaccaccaccacccaccccacgtagaccagcatctgtcaatggttcaccatctgcc
acttctgaaagtgatgggtctagtacaggctctctgccgccgacaaatacaaatacaaatacatctgaaggagcaacatctgga
ttaataattcctcttactatatctggaggctcaggccctaggccattaaatcctgtaactcaagctcccttgccacctggttgg
gagcagagagtggaccagcacgggcgagtttactatgtagatcatgttgagaaaagaacaacatgggatagaccagaacctcta
cctcctggctgggaacggcgggttgacaacatgggacgtatttattatgttgaccatttcacaagaacaacaacgtggcagagg
ccaacactggaatccgtccggaactatgaacaatggcagctacagcgtagtcagcttcaaggagcaatgcagcagtttaaccag
agattcatttatgggaatcaagatttatttgctacatcacaaagtaaagaatttgatcctcttggtccattgccacctggatgg
gagaagagaacagacagcaatggcagagtatatttcgtcaaccacaacacacgaattacacaatgggaagaccccagaagtcaa
ggtcaattaaatgaaaagcccttacctgaaggttgggaaatgagattcacagtggatggaattccatatttgtggaccacaat
agaagaactaccacctatatagatccccgcacaggaaaatctgccctagacaatggacctcagatagcctatgttcgggacttc
aaagcaaaggttcagtatttccggttctggtgtcagcaactggccatgccacagcacataaagattacagtgacaagaaaaaca
ttgtttgaggattcctttcaacagataatgagcttcagtccccaagatctgcgaagacgtttgtgggtgatttttccaggagaa
gaaggtttagattatggaggtgtagcaagagaatggttctttcttttgtcacatgaagtgttgaacccaatgtattgcctgttt
gaatatgcagggaaggataactactgcttgcagataaaccccgcttcttacatcaatccagatcacctgaaatattttcgtttt
attggcagatttattgccatggctctgttccatgggaaattcatagacacgggtttttctttaccattctataagcgtatcttg
aacaaaccagttggactcaaggatttagaatctattgatccagaattttacaattctctcatctgggttaaggaaaacaatatt
gaggaatgtgatttggaaatgtacttctccgttgacaaagaaattctaggtgaaattaagagtcatgatctgaaacctaatggt
ggcaatattcttgtaacagaagaaaataaagaggaatacatcagaatggtagctgagtggaggttgtctcgaggtgttgaagaa
cagacacaagctttctttgaaggctttaatgaaattcttccccagcaatatttgcaatactttgatgcaaaggaattagaggtc
cttttatgtggaatgcaagagattgatttgaatgactggcaaagacatgccatctaccgtcattatgcaaggaccagcaaacaa
atcatgtggttttggcagtttgttaaagaaattgataatgagaagagaatgagacttctgcagtttgttactggaacctgccga
ttgccagtaggaggatttgctgatctcatggggagcaatggaccacagaaattctgcattgaaaaagttgggaaagaaaattgg
ctacccagaagtcatacctgttttaatcgcctggacctgccaccatacaagagctatgagcaactgaaggaaaagctgttgttt
gccatagaagaaacagaaggatttggacaagagtaacttctgagaacttgcaccatgaatgggcaagaacttatttgcaatgtt
tgtccttctctgcctgttgcacatcttgtaaaattggacaatggctctttagagagttatctgagtgtaagtaaattaatgttc
tcatttaaaaaaaaaaaaaaaaaa
<212> Type : DNA
<211> Length : 2377
      SequenceName : AF038564

SequenceDescription :

Sequence
--------
<213> OrganismName : HUMAN
<400> PreSequenceString :
ggcacgaggcatggcgctgagggccgccccgcgggaagatgaataagggctggctggagctggagagcgacccaggcctcttc
accctgctcgtggaagatttcggtgtcaagggggtgcaagtggaggagatctacgaccttcagagcaaatgtcagggccctgta
tatggatttatcttcctgttcaaatggatcgaagagcgccggtcccggcgaaaggtctctaccttggtggatgatacgtccgtg
attgatgatgatattgtgaataacatgttctttgcccaccagctgatacccaactcttgtgcaactcatgccttgctgagcgtg
ctcctgaactgcagcagcgtggacctgggacccaccctgagtcgcatgaaggacttcaccaagggtttcagccctgagagcaaa
ggatatgcgattggcaatgccccggagttggccaaggcccataatagccatgccaggcccgagccacgccacctccctgagaag
cagaatggccttagtgcagtgcggaccatggaggcgttccactttgtcagctatgtgcctatcacaggccggctctttgagctg
gatgggctgaaggtctaccccattgaccatgggccctgggggaggacgaggagtggacagacaaggcccggcgggtcatcatg
gagcgtatcggcctcgccactgcaggggagccctaccacgacatccgcttcaacctgatggcagtggtgcccgaccgcaggatc
aagtatgaggccaggctgcatgtgctgaaggtgaaccgtcagacagtactagaggctctgcagcagctgataagagtaacacag
ccagagctgattcagacccacaagtctcaagagtcacagctgcctgaggagtccaagtcagccagcaacaagtccccgctggtg
ctggaagcaaacagggcccctgcagcctctgagggcaaccacacagatggtgcagaggaggcggctggttcatgcgcacaagcc
ccatcccacagccctcccaacaaacccaagctagtggtgaagcctccaggcagcagcctcaatggggttcaccccaaccccact
cccattgtccagcggctgccggcctttctagacaatcacaattatgccaagtccccatgcaggaggaagaagacctggcggca
ggtgtgggccgcagccgagttccagtccgcccaccccagcagtactcagatgatgaggatgactatgaggatgacgaggaggat
gacgtgcagaacaccaactctgcccttaggtataaggggaaggaacagggaagccaggggcattgagcggttctgctgatggg
caactgtcagtgctgcagcccaacaccatcaacgtcttggctgagaagctcaaagagtcccagaaggacctctcaattcctctg
tccatcaagactagcagcggggctgggagtccggctgtggcagtgcccacacactcgcagccctcacccacccccagcaatgag
agtacagacacggcctctgagatcggcagtgctttcaactcgccactgcgctcgcctatccgctcagccaacccgacgcggccc
tccagccctgtcacctcccacatctccaaggtgctttttggagaggatgacagcctgctgcgtgttgactgcatacgctacaac
cgtgctgtccgtgatctgggtcctgtcatcagcacaggcctgctgcacctggctgaggatggggtgctgagtcccctggcgctg
acagagggtgggaagggttcctcgccctccatcagaccaatccaaggcagccaggggtccagcagcccagtggagaaggaggtc
gtggaagccacggacagcagagagaagacggggatggtgaggcctggcgagcccttgagtggggagaaatactcacccaaggag
ctgctggcactgctgaagtgtgtggaggctgagattgcaaactatgaggcgtgcctcaaggaggaggtagagaagaggaagaag
ttcaagattgatgaccagagaaggacccacaactacgatgagttcatctgcacctttatctccatgctggctcaggaaggcatg
ctggccaacctagtggagcagaacatctccgtgcggcggcgccaaggggtcagcatcggccggctccacaagcagcggaagcct
gaccggcggaaacgctctcgcccctacaaggccaagcgccagtgaggactgctggccctgactctgcagcccactcttgccgtg
tggccctcaccagggtccttccctgccccacttccccttttcccagtattactgaatagtcccagctggagagtccaggccctg
ggaatggaggaaccaggccacattccttccatcgtgccctgaggcctgacacggcagatcagccccatagtgctcaggaggca
gcatctggagttggggcacagcgaggtactgcagcttcctccacagccggctgtggagcagcaggacctggcccttctgcctgg
gcagcagaatatatatttacctatcagagacatctatttttctgggctccaacccaacatgccaccatgttgacataagttcc
tacctgactatgctttctctcctaggagctgtcctggtgggcccaggtccttgtatcatnccacggtcccaactacagggtcct
agctgggggcctgggtgggccctgggctctgggccctgctgctctagccccagccaccagcctgtccctgttgtaaggaagcca
ggtcttctctcttcattcctcttaggagagtgccaaactcagggacccagcactgggctgggttgggagtagggtgtcccagtg
gggttggggtgagcaggctgctgggatcccatggcctgagcagagcatgtgggaactgttcagtggcctgtgaactgtcttcct
tgttctagccaggctgttcaagactgctctccatagcaaggttctagggctcttcgccttcagtgttgtggccctagctatggg
cctaaattggctctaggtctctgtccctggcgcttgaggctcagaagagcctctgtccagcccctcagtattaccatgtctcc
ctctcaggggtagcagagacagggttgcttataggaagctggcaccactcagctcttcctgctactccagtttcctcagcctct
gcaaggcactcagggtgggggacagcaggatcaagacaacccgttggagcccctgtgttccagaggacctgatgccaaggggta
atgggcccagcagtgcctctggagcccaggccccaacacagccccatggcctctnccagatggctttgnaaaaggtgatccaan
caggcccctttatctgtacatagtgactgagtggggggtgctggcaagtgtggcanctncctctgggctgagcacagcttgacc
cctctagcccctgtaaanactggatcaatgaatgaataaaactctcctaagnatctcctgagaaaaaaaaaaaaaaaagg
<212> Type : DNA
<211> Length : 3525
      SequenceName : AF045581
      SequenceDescription :

Sequence
--------
<213> OrganismName : HUMAN
<400> PreSequenceString :
acgcgtccgggaaacgttgcttctgaggggagcccaagatgaccggttctaacgagttcaagctgaaccagccacccgaggatg
gcatctcctccgtgaagttcagccccaacacctcccagttcctgcttgtctcctcctgggacacgtccgtgcgtctctacgatg
tgccggccaactccatgcggctcaagtaccagcacaccggcgccgtcctggactgcgccttctacgatccaacgcatgcctgga
gtggaggactagatcatcaattgaaaatgcatgatttgaacactgatcaagaaaatcttgttgggacccatgatgcccctatca
gatgtgttgaatactgtccagaagtgaatgtgatggtcactggaagttgggatcagacagttaaactgtgggatcccagaactc
cttgtaatgctgggaccttctctcagcctgaaaaggtatataccctctcagtgtctggagaccggctgattgtgggaacagcag

gccgcagagtgttggtgtgggacttacggaacatgggttacgtgcagcagcgcagggagtccagcctgaaataccagactcgct
gcatacgagcgtttccaaacaagcagggttatgtattaagctctattgaaggccgagtggcagttgagtatttggacccaagcc
ctgaggtacagaagaagaagtatgccttcaaatgtcacagactaaaagaaaataatattgagcagatttacccagtcaatgcca
tttcttttcacaatatccacaatacatttgccacaggtggttctgatggctttgtaaatatttgggatccatttaacaaaaagc
gactgtgccaattccatcggtaccccacgagcatcgcatcacttgccttcagtaatgatgggactacgcttgcaatagcgtcat
catatatgtatgaaatggatgacacagaacatcctgaagatggtatcttcattcgccaagtgacagatgcagaaacaaaaccca
agtcaccatgtacttgacaagatttcatttacttaagtgccatgttgatgataataaaacaattcgtactccccaatggtggat
ttattactattaaagaaaccagggaaaatattaattttaatattataacaacctgaaaataatggaaaagaggttttgaattt
tttttttaaataaacaccttcttaagtgcatgagatggtttgatggtttgctgcattaaaggtatttgggcaaacaaaattgg
agggcaagtgactgcagttttgagaatcagttttgaccttgatgatttttgtttccactgtggaaataaatgtttgtaaataa
gtgtaataaaaatccctttgcattctttctggaccttaaatggtagaggaaaaggctcgtgagccatttgtttcttttgctggt
tatagttgctaattctaaagctgcttcagactgcttcatgaggaggttaatctacaattaaacaatatttcctcttggccgtcc
attattttctgaagcagatggttcatcatttcctgggctgttaaacaaagcgaggttaaggttagactcttgggaatcagctag
ttttcaatcttattagggtgcagaaggaaaactaataagaaaacctcctaatatcattttgtgactgtaaacaattatttatta
gcaaacaattgatcccagaagggcaaattgtttgagtcagtaatgagctgagaaaagacagagcatatctgtgtatttggaaaa
ataattgtaacgtaattgcagtgcatttagacaggcatctatttggacctgtttctatctctaaatgaatttttggaaacatta
atgaggtttacatatttctctgacatttatatagttcttatgtccatttcagttgaccagccgctggtgattaaagttaaaaag
aaaaaaattatagtgagaatgagattcatttcaatgtaatgcactaaagcagaacacgaacttagcttggcctattctaggtag
ttccaaatagtatttttgttgtcaactttaaaatttatattaatttgcaaatgtatgtctctgagtaggacttggacctttcc
tgagatttattttatccgtgatgtatttttttaattcttttgatacagagaagggtcttttttttttttaagtatttcagt
gaaaacttggtgtaagtctgaacccatcttttgaaatgtattttcttcattgcaggtccacctaatcatcctgtgaaagtggtt
tctctatggaaagctttgtttgcttcctacaaatacatgcttattccttaagggatgtgttagagttactgtggatttctctgt
tttctgtcttacaagaaacttgtctatgtaccttaatactttgtttaggatgaggagtctttgtgtccctgtacagtagtctga
cgtatttccccttctgtcccctagtaagcccagttgctgtatctgaacagtttgagctcttttgtaatatactctaaacctgt
tatttctgtgctaataaacgagatgcagaaccc
<212> Type : DNA
<211> Length : 2553
      SequenceName : AF053304
      SequenceDescription :

Sequence
--------
<213> OrganismName : HUMAN
<400> PreSequenceString :
cccccatgtgacagtgacggggtccccgctccaggagacgctcgagtctgcgtcccggccctcagcactgtccactgtttcggt
gccagcagagaccagcaggcccgggacagttggtgtttggccgtgccgctgtctaacttggtgtgcagagtgaattgccgctgc
cggagcggagagaggcggagcggccaggagagaggggatttctgtcagcgccggcctcgggagctcggagacatgaacggcttc
acgcctgacgagatgagccgcggcggggatgcggccgccgcagtggccgcagtggtcgctgccgcggccgccgccgcctcggcg
gggaacgggaccggcgcgggcaccggggctgaggtgccgggcgcgggggcggtctcagcggctgggcccccgggggcggccggg
ccgggccccgggcaactgtgctgcctgcgggaggatggtgagcggtgcggccgggcggcaggcaacgccagcttcagcaagagg
atccagaagagcatctcccagaagaaggtgaagatcgagctggataagagcgcaaggcatctttacatatgtgattatcataaa
aacttaattcagagtgttcgaaacagaagaaagagaaaagggagtgatgatgatggaggtgattcacctgttcaagatattgat
accccagaggttgatttataccaattacaagtaaatacacttaggagatacaaaagacacttcaagctaccaaccagaccagga
cttaataaagcacaacttgttgagatagttggttgccactttaggtctattccagtgaatgaaaaagacaccttaacatatttc
atctactcagtgaagaatgacaagaacaaatcagatctcaaggttgatagtggtgttcactaggagacgtggaattgagactaa
taacttggatgttaacactgtttactgtttttcacatgtagaaatgttctttgtgtatttttctacagaggatttctctga
ttttattttctttgtttctgactctaataattagttggaaactcatataaaatgagctttcctaaattaaatctattttaaata
aaggttattactattaaaaaaaaaaaaaaaaa
<212> Type : DNA
<211> Length : 1125
      SequenceName : AF055993
      SequenceDescription :

Sequence
--------
<213> OrganismName : HUMAN
<400> PreSequenceString :
aggatgatcaagctgttctcgctgaagcagcagaagaaggaggaggagtcggcgggcggcaccaagggcagcagcaagaaggcg
tcggcggcgcagctgcggatccagaaggacataaacgagctgaacctgcccaagacgtgtgatatcagcttctcagatccagac
gacctcctcaacttcaagctggtcatctgtcctgatgagggcttctacaagagtgggaagtttgtgttcagttttaaggtgggc
cagggttacccgcatgatcccccaaggtgaagtgtgagacaatggtctatcaccccaacattgacctcgagggcaacgtctgc
ctcaacatcctcagagaggactggaagccagtccttacgataaactccataatttatggcctgcagtatctcttcttggagccc
aaccccgaggacccactgaacaaggaggccgcagaggtcctgcagaacaaccggcggctgtttgagcagaacgtgcagcgctcc

atgcggggtggctacatcggctccacctactttgagcgctgcctgaaatagggttggcgcatacccacccgccgccacggccac
aagccctggcatcccctgcaaatatttattggggccatgggtaggggtttgggggcggccggtgggggaatcccctgccttg
gccttgcctccccttcctgccacgtgcccctagttattttttttaacaccaggctaactaaaggggaatgttactgc
<212> Type : DNA
<211> Length : 751
      SequenceName : AF075599
      SequenceDescription :

Sequence
--------
<213> OrganismName : HUMAN
<400> PreSequenceString :
agcaccaaatccaagatggcggccagcaggaggctgatgaaggagcttgaagaaatccgcaaatgtgggatgaaaaacttccgt
aacatccaggttgatgaagctaatttattgacttggcaagggcttattgttcctgacaaccctccatatgataagggagccttc
agaatcgaaatcaactttccagcagagtacccattcaaaccaccgaagatcacatttaaaacaaagatctatcacccaaacatc
gacgaaaaggggcaggtctgtctgccagtaattagtgccgaaaactggaagccagcaaccaaaaccgaccaagtaatccagtcc
ctcatagcactggtgaatgaccccagcctgagcacccgcttcgggctgacctagctgaagaatactctaaggaccgtaaaaa
ttctgtaagaatgctgaagagtttacaaagaaatatgggaaaagcgacctgtggactaaaatctgccacgattggttccagca
agtgtgagcagagaccccgtgcagtgcattcagacaccccgcaaagcaggactctgtggaaattgacacgtgccaccgcctggc
gttcgcttgtggcagttactaactttctacagtttcttaatcaaaagtggtctaggtaacctgtaaagaaaggattaaaaatt
taagatgttctagttctgctctctttgttttaaaaatcactgcttcaatctacttcaaaagaatggtgtttcttttcttgtcca
atttatccaaaatcttcaagttacatttaacccataaggtttaaaaaaaaggaaaaaaaacggttgtggttccctttcttccc
taccccttgccactcccactttctggcaccgagtttattttcacttacttacttccccagaccccgggctcgcctccacaaagg
agaagagactgccctggcggtcctggtggcttttcttagcatgtgtggcactgttgcccagtgtgggagttggtttaaattctc
ctgactccagtttataacatcctttaaaaaatttaaaaacaaacagccacacccctcctccagtccttctcctcagttcttgt
gtgaaactccagctgatgttaccacagtaacatcagttaattggcaagccctgatgtcagtgtgtgtaactgacctctggcct
ggcctgcacagagaagccctataatcacaggtctgtggtggccccgaaatgggggcctgctagtcaggaggatgctgtgcaca
ctgtgtgtgatgaatctcgccagaaaggctcctgaggtcccaggttggcacttctccctgcagccattgtagaagatctgctgg
tccttgcaggcaaagctacagccagaatgtccgtttgaaactcctagctcatctgtcaccgagcttcatccgaatgtgccacgg
agcttgctctccacttcctccgtgcagtggccctgccacagccctccctcggcacactttgaccctttgtaggattggaattag
caggactcggctatttaaagcaccagtctggggtcgcctgggcccctgctgacccctcctccagagcagccagcccagcccgg
gaacaagacggacttcctctcccttcggactcacagcctttgcagagtcaagctccacttgaagctcactcagtaatatccttt
caatgtgttttatattgttttgactgcctttttttgtagaaataaaaattgaccttagaatttatcgtcagataaacttgtaaa
gatttgaatattaatgtcttttcaaggcaaatgggattgtccccgcactagtagagaatccatgtcgctctgacaccccaagga
agccgacgatccaaatgccgtgtgtcaccaaccccgcttctgccactggcggcttcccttcttggctcttgggggggactagat
cctgtggagaagatgacttaaactttgcttttttgttttaattttaattctataacttgagatctttccggggcctacaggcgtg
taagacagcttggtctggtctgtgcagaagtggggagtgatgggcaggttcggcagcctaacattgttcaggcgcatggcccct
gcggtgtgtacacgaactcggcttcttttgtcctaggtacgccaagggcaggtttctggagactcccttgtgcccgggatggca
agggcaccgggctggcgtttccacatctgtcttcattagcagaaaagtgatgatggatttatttcactcacactccagtttgt
aataaaatgccaaattctgtcagctatccaaacaagccaccatttgttcttgttgcttctctggatccagaaatgttgccattc
ttggaaactgtcccattgcttcgtatttctgccaacgtagctctgcctgcctgtcaacccctcactgcactctgctcatcacgg
gaggatacctgtgtgccggcagcccctcagggactctcagccctggcactggcaccccagggttggccccgtcagcagaggctt
ggctttcgagccagtgggtgtctctcctttgggcctgggcggcttgctcctgccagccatgccttcagggtaggctctgagcaa
gctggcgaacagccctggctgctccaaaaccaaaaagctgggtcctctggaggaggggcgagctgtggagcagccacccactgc
tgccccaagctcactcaggaattcacacccgcctggtttcttgaagtgtgctgggtccttccctctgctccctactccccacca
cggcagagaataggctttctaagatgctgcgatcccgttctgctgcccgtaataaaaatgctctcagacactg
<212> Type : DNA
<211> Length : 2845
      SequenceName : AJ000519
      SequenceDescription :

Sequence
--------
<213> OrganismName : HUMAN
<400> PreSequenceString :
atggtattttactttttgggaaatacttggaaatgaagacctgcaactgtaatttgaaataaggaaaactttaattttcagta
taaaaattgctcaaatagaattgcctgattttaatgacaaaagtatatgggagtccacatttatgtaagaaatgaaactataaa
atgtataaataatttgcaaatcagaattgctgtcgaaagttttactataatgaaagatattttcatactctcaaaaatatagag
gaaaggggccaagattatagtaccagtcacaatcttttgatgaggacgaaatgaatcaggtaacagactgggttgacccatcat
ttgatgatttctagagtgtagtggcgtctctactattactgccacatcattaggtgtgaataactcaagtcatagaagaaaaa
atgggccttctacattagaaagcagcagatttccagcgagaaaaagaggaaatctatcttccttagaacagatttatggtttag
aaaattcaaaagaatatctgtctgaaaatgaaccatgggtggataaatataaaccagaaactcagcatgaacttgctgtgcata
aaaagaaaattgaagaagtcgaaacctggttaaaagctcaagttttagaaaggcaaccaaaacagggtggatctattttattaa

taacaggtcctcctggatgtggaaagacaacgaccttaaaaatactatcaaaggagcatggtattcaagtacaagagtggatta
atccagttttaccagacttccaaaagatgatttcaaggggatgtttaatactgaatcaagcttccatatgtttccctatcagt
ctcagatagcagttttcaaagagtttctactaagagcgacaaagtataacaagttacaaatgcttggagatgatctgagaactg
ataagaagataattctggttgaagatttacctaaccagttttatcggattctcatactttacatgaagttctaaggaagtatg
tgaggattggtcgatgtcctcttatatttataatctcggacagtctcagtggagataataatcaaaggttattgtttcccaaag
aaattcaggaagagtgttctatctcaaatattagtttcaaccctgtggcaccaacaattatgatgaaatttcttaatcgaatag
tgactatagaagctaacaagaatggaggaaaaattactgtccctgacaaaacttctctagagttgctctgtcagggatgttctg
gtgatatcagaagtgcaataaacagcctccagtttcttcttcaaaaggagaaaacaacttacggccaaggaaaaaaggaatgt
ctttaaaatcagatgctgtgctgtcaaaatcaaaacgaagaaaaaaacctgatagggttttgaaaatcaagaggtccaagcta
ttggtggcaaagatgtttctctgtttctcttcagagctttggggaaaattctatattgtaaaagagcatctttaacagaattag
actcacctcggttgccctctcatttatcagaatatgaacggatacattacttgttgaacctgaggaggtagtagaaatgtcac
acatgcctggagacttatttaatttatatcttcaccaaaactacatagatttcttcatggaaattgatgatattgtgagagcca
gtgaatttctgagttttgcagatatcctcagtggtgactggaatacacgctctttactcagggaatatagcacatctatagcta
cgagaggtgtgatgcattccaacaaagcccgaggatatgctcattgccaaggaggaggatcaagttttcgacccttgcacaaac
ctcagtggtttctaataaataaaaagtatcgggaaaattgcctggcagcaaaagcacttttttcctgacttctgcctaccagctt
tatgcctccaaactcagctattgccataccttgctctactaaccattccaatgagaaatcaagctcagatttcttttatccaag
atattggaaggctccctctgaagcgacactttggaagattgaaaatggaagccctgactgacagggaacatggaatgatagacc
ctgacagcggagatgaagcccagcttaatggaggacattctgcagaggaatctctgggtgaacccactcaagccactgtgccgg
aaacctggtctcttcctttgagtcagaatagtgccagtgaactgcctgctagccagccccagcccttttcagcccaaggagaca
tggaagaaaacataataatagaagactacgagagtgatgggacatagaagccagcctgctaatcagattgctacttcacagctt
catttttgtttcattcagtggtacttcagcagagttaatatgcttttctgatgaattacacaacagtttgttaattcttcattc
ttgtagtatttcatcacaagaaacctactcttctgtcatcttgaagtaaatagaagatcaagccttcaaatctcttaattttt
cggtatttattaaatctgtgagtggtttaaggagcggtcagtgtgtataaagtgtgtttgaacattatgccaaatatcaagatg
tgaaggactaattcaggatgcaaaaacgttattggggggttgtaaatatcaactattcaacagtttaggatgcaattacgagtg
taaactgtgtgccttatttacactttattgtctcccgcttctcagatagttttgatgtgttgtacagtggaatatcttagatac
tttttggaaagtatttacataagttatatcacaattaaaatgtt
<212> Type : DNA
<211> Length : 2816
      SequenceName : AJ004977
      SequenceDescription :

Sequence
--------
<213> OrganismName : HUMAN
<400> PreSequenceString :
gccccatctccttggctgcccgtgcttcgtgctttggactaccgcccagcagtgtcctgccctctgcctgggcctcggtccct
cctgcacctgctgcctggatccccggcctgcctgggcctgggccttggttctccccatgacaccacctgaacgtctcttcctcc
caagggtgtgtggcaccaccctacacctcctccttctggggctgctgctggttctgctgcctggggcccaggggctccctggtg
ttggcctcacaccttcagctgcccagactgcccgtcagcaccccaagatgcatcttgcccacagcaccctcaaacctgctgctc
acctcattggagaccccagcaagcagaactcactgctctggagagcaaacacggaccgtgccttcctccaggatggtttctcct
tgagcaacaattctctcctggtccccaccagtggcatctacttcgtctactcccaggtggtcttctctggaaagcctactctc
ccaaggccacctcctccccactctacctggcccatgaggtccagctcttctcctcccagtaccccttccatgtgcctctcctca
gctcccagaagatggtgtatccagggctgcaggaaccctggctgcactcgatgtaccacggggctgcgttccagctcacccagg
gagaccagctatccacccacacagatggcatcccccacctagtcctcagccctagtactgtcttctttggagccttcgctctgt
agaacttggaaaaatccagaaagaaaaaataattgatttcaagaccttctccccattctgcctccattctgaccatttcagggg
tcgtcaccacctctcctttggccattccaacagctcaagtcttccctgatcaagtcaccggagctttcaaagaaggaattctag
gcatcccaggggaccacacctccctgaaccatccctgatgtctgtctggctgaggatttcaagcctgcctaggaattcccagcc
caaagctgttggtctgtcccaccagctaggtggggcctagatccacacacagaggaagagcaggcacatggaggagcttggggg
atgactagaggcagggaggggactatttatgaaggcaaaaaaattaaattatttatttatggaggatggagagaggggaataat
agaagaacatccaaggagaaacagagacaggcccaagagatgaagagtgagagggcatgcgcacaaggctgaccaagagagaaa
gaagtaggcatgagggatcacagggccccagaaggcagggaaaggctctgaaagccagctgccgaccagagccccacacggagg
catctgcaccctcgatgaagcccaataaacctcttttctctg
<212> Type : DNA
<211> Length : 1386
      SequenceName : D12614
      SequenceDescription :

Sequence
--------
<213> OrganismName : HUMAN
<400> PreSequenceString :
cgtgaacggtcgttgcagagattgcgggcggctgagacgccgcctgcctggcacctaggagcgcagcggagccccgacaccgcc
gccgccgccatggagtccgagaccgaacccgagcccgtcacgctcctggtgaagagccccaaccagcgccaccgcgacttggag

ctgagtggcgaccgcggctggagtgtgggccacctcaaggcccacctgagccgcgtctaccccgagcgtccgcgtccagaggac
cagaggttaatttattctgggaagctgttgttggatcaccaatgtctcagggacttgcttccaaagcaggaaaaacggcatgtt
ttgcatctggtgtgcaatgtgaagagtccttcaaaaatgccagaaatcaacgccaaggtggctgaatccacagaggagcctgct
ggttctaatcggggacagtatcctgaggattcctcaagtgatggtttaaggcaaagggaagttcttcggaacctttcttcccct
ggatgggaaaacatctcaaggcctgaagctgcccagcaggcattccaaggcctgggtcctggtttctccggttacacaccctat
gggtggcttcagctttcctggttccagcagatatatgcacgacagtactacatgcaatatttagcagccactgctgcatcaggg
gcttttgttccaccaccaagtgcacaagagatacctgtggtctctgcacctgctccagcccctattcacaaccagtttccagct
gaaaaccagcctgccaatcagaatgctgctcctcaagtggttgttaatcctggagccaatcaaaatttgcggatgaatgcacaa
ggtggccctattgtggaagaagatgatgaaataaatcgagattggttggattggacctattcagcagctacattttctgttttt
ctcagtatcctctacttctactcctccctgagcagattcctcatggtcatgggggccaccgttgttatgtacctgcatcacgtt
gggtggtttccatttagaccgaggccggttcagaacttcccaaatgatggtcctcctcctgacgttgtaaatcaggaccccaac
aataacttacaggaaggcactgatcctgaaactgaagaccccaaccacctccctccagacagggatgtactagatggcgagcag
accagcccctcctttatgagcacagcatggcttgtcttcaagactttctttgcctctcttcttccagaaggcccccccagccatc
gcaaactgatggtgtttgtgctgtagctgttggaggctttgacaggaatggactggatcacctgactccagctagattgcctct
cctggacatggcaatgatgagtttttaaaaaacagtgtggatgatgatatgcttttgtgagcaagcaaaagcagaaacgtgaag
ccgtgatacaaattggtgaacaaaaaatgcccaaggcttctcatgtgtttattctgaagagctttaatatatactctatgtagt
ttaataagcactgtacgtagaaggccttaggtgttgcatgtctatgcttgaggaacttttccaaatgtgtgtgtctgcatgtgt
gtttgtacatagaagtcatagatgcagaagtggttctgctggtaagatttgattcctgttggaatgtttaaattacactaagtg
tactactttatataatcaatgaaattgctagacatgttttagcaggacttttctaggaaagacttatgtataattgcttttaa
aatgcagtgctttactttaaactaaggggaactttgcggaggtgaaaacctttgctgggttttctgttcaataaagttttacta
tgaatgaccctg
<212> Type : DNA
<211> Length : 1860
      SequenceName : D14695
      SequenceDescription :

Sequence
--------
<213> OrganismName : HUMAN
<400> PreSequenceString :
cgaggtcagctacgtaaatccctttgcggcgggataaataaagaaaacaggaaaggaggaaagcattgattacaaatatcttaa
caatgagcaaatgtgcaaggaaaaaatatattaagacaaatccaagacaaatgaccatggaaacagttgaatcccagcatgatg
gaagtataacagcttctttgacagagagcaagtctgctcatgtgcagactcagactggccaaaattcaatccctgctttagctc
aggtagcagcaattgcagagacagatgaatctgcagaatcagaaggtgtaattgactctcataaacgtagagaaatcctttcac
gaagaccctcttataggaaaatactgaatgaactgtcctctgatgtgcctggtgttccaaagattgaagaagagagatcagagg
aagaaggaacaccacctagtattgctaccatggcagtaccaactagcatatatcagactagcacggggcaatacaatgaggaaa
ctgaacttgccccaagtcacatggctgctgccactggtgacatgccaacttaccagatccgagctcctactgctgctttgccac
agggagtggtgatggctgcatcgcccggaagtttgcgcagtccccagcagctggcagaagaagcaacacgcaaacgagagctga
ggctaatgaaaaacagggaagctgcccgggagtgtcgcaggaagaagaaagaatatgtcaaatgtcttgaaaatcgtgtggctg
tgcttgaaaaccaaaacaagactctcattgaggaactcaaggccctcaaagatctttattgccataaagtagagtaactgtctt
tgacttggaccttgtttactctaactaaggcaggagatgcagcagtcctacttattgccatgtggacttgtgggaaggacacgt
gtgacctaagaatccagtttggattagtgtttgaaattgaattgggaatgttgttccaggatgtggaatgcagccgtgatcaca
cttaccgagcttactttgatctgtttgtcaatagcatgcaaaaaagctttgtttgccctttgcttctgctttttttcagggaag
ctgccaaagaatgtcgacgtcgaaagaaagaatatgtaaaatgtctggagagccgagttgcagtgctggaagtccagaacaaga
agctgatagaggaacttgaaaccttgaaagacatttgttctcccaaaacagattactagaaattatttaactatgaactgaagg
cagcatgtatagttgcttttgaaggaatacaatatatagctggcaagaatggtggcttcttttctttgtatcattcatcttctt
ctttaatcattcatcttcttctttaatcacttaacattcctaaaaatgcttcactgtacgtagttaagtcgtagctataacttc
aaatttttaaaagggacaaactgtaaaaaatgtgtgtattcttaaaatgcaatatttgtaaggcttgttccaatgccacatac
ttgcagctcccattctatgtgtcatcaatagtgtcctatgcaataaattatttgcaggtctttaaatcatttaggaaggatga
caaaataagt
<212> Type : DNA
<211> Length : 1606
      SequenceName : D14825
      SequenceDescription :

Sequence
--------
<213> OrganismName : HUMAN
<400> PreSequenceString :
cgaaaacggagaaaccccgggtccggcgagaggggctgtgacagtcggagtcccaagctgcggttcggctgctgccgagaactg
caaggtgtggaatatttctggcttctagtccaatgccaagtgtgtgacctgtggctacatgattccctgaaagataagaacaat
gttatgttggggatattggtctctgggccaacctggtatcagcaccaacctgcagggaattgtggctgagccccaggtgtgtgg
gttcatatctgacagaagtgtcaaggaagtggcctgtgggggaaaccactctgtgttcctgctggaagatggggaagtttacac

atgtggtttgaacaccaaggggcaactgggccatgagagggaaggaaacaagccagaacaaattggagctctggcagatcagca
tatcattcatgtggcatgtggcgagtcccacagtctggccctcagtgaccgaggccagctgtttcttggggtgcagggagtga
tggtcagctaggactcatgactactgaggattctgtggcagtgcccaggttaatacaaaagctgaaccagcaaacaatattaca
agtttcctgtggcaactggcattgcttggctcttgcggctgatggccagttcttcacctggggaaagaacagccatggcagct
tggcttagggaaggagttcccctcccaagccagcccacagagggtgaggtccctggaggggatcccactggctcaggtggctgc
cggaggggctcacagctttgccctgtctctctcaggagctgtttttggctggggatgaataatgccgggcagctagggctcag
tgatgaaaaagatcgagaatctccatgccatgtaaaactcttacgcacgcaaaaagttgtctatattagttgtggagaagaaca
cacagcagttctcacaaagagtggaggtgtgtttacctttggcgctggttcctgtgggcaacttggacacgactccatgaatga
tgaggttaaccctagaagagttctagagctgatgggtagtgaagtaactcaaattgcttgtggcagacaacataccctagcctt
cgtgccttcttctggactcatctatgcatttggttgtggagcaagaggtcaattaggaactggcacacttgtaatgttaagtg
cccatctcctgtcaagggttactgggctgcccacagtggccagctttcagcccgagctgatcgctttaaatatcatatcgttaa
gcagatcttctctggaggagaccagacttttgtactttgctccaaatacgagaattattctcctgctgttgacttcaggactat
gaaccaagcacattataccagtttaataaatgatgaaaccatagcagtttggagacaaaaactctcagaacacaacaatgcaaa
tacaatcaatggtgttgttcagatattatcttctgcagcctgttggaatggaagttttcttgaaaaaaaaattgatgaacattt
taaaacgagtcccaaaatccctggattgacctgaactcaactagggtgttatttgagaagttaatgaactctcagcactccat
gattctagaacagattttgaacagttttgaaagttgtctgattccccagttgtcaagctcaccaccagatgttgaagccatgag
aatctatttaatactacctgagtttcccctactccaggattccaagtattatataacattgactattcccttggctatggccat
tcttcggctggatacaaaccccagcaaagtactagataactggtggtctcaggtatgcccgaaatatttcatgaagctggtaaa
cctctataaaggtgcagtcctttatctactgaggggaagaaagacattcttaattcccgtactgtttaacaattatatcacagc
agctctcaaactcttggagaagttatataaggtaaatcttaaagtgaagcatgtggaatatgatacattttacattcctgagat
ttccaatctcgtggacattcaggaagactacctcatgtggttcttgcatcaagcagggatgaaggctagaccatcaataataca
ggatactgtaacactttgttcctaccctttcatctttgatgcccaagccaagaccaaaatgttacagacagatgctgaactaca
gatgcaggtggcagtcaatggagccaacctgcagaatgtcttcatgcttctcaccctggagcctctgctggccagaagcccctt
cctggtccttcacgttcgcaggaacaaccttgttggagatgccctaagagagctgagcattcattctgatattgatttgaaaaa
gcctctcaaagtaatctttgatggtgaagaagcagtggatgccggtggtgttacaaaggaatttttcttttgctgttaaaaga
acttttgaatcccatctatggaatgtttacctactatcaagattcaaatctcttgtggttttcagacacgtgttttgtagagca
caactggtttcacttgattggtataacctgtggactagctatctacaactccactgtggtcgatctccacttcccattggctct
ctacaagaagttactcaatgtaaagcctggcttggaagacttaaaggagttgtcacccactgaaggaaggagtctccaagagct
tttagattaccccggggaggatgtggaggagactttctgcctcaacttcacgatctgccgagaaagctatggagtgattgaaca
gaagaagctgatacctgggggagataatgtaactgtgtgcaaggataacaggcaggaatttgtggatgcttatgtgaattatgt
cttccaaatctcagttcatgaatggtacacagccttctctagtggcttcctaaaggtgtgtggtggcaaagtacttgagctctt
ccagccttcagaactgagggctatgatggtggggaacagcaactacaactgggaagaactggaagagactgccatctacaaggg
agattactcggccacacatcccactgtaaaactattttgggaaacatttcatgagtttccattggaaaagaagaagaagtttct
cttgttcctgacaggcagcgatcggattcccatctacggcatggccagtctgcagattgtcatccagtccacagccagcggga
ggagtacttgccggtggcccacacttgctacaaccttcttgacctccccaagtacagcagcaaagagattctgagtgcccggct
gacccaggcccttgacaactatgaagggtttagtttggcctgaggcttctcagcttgtccagtatttcccttcgttcctcagtg
tccacattgaggcctatacagaaaatcatggggagtgatttctatttttttattgtctaagtgggttgggacttttaaatactg
agcctggttgatgtgtttctgggattgtatagcagtaaacaacctttttgaaaaattagaggttggggatggggtgaaaaattg
gcccttgtatgggaggtgttttgttttgtttaaaccaaactacccagtattccttgcacttgtgaatgtgttgcactctgc
tggatgaaatggcagtggattttaaactttaatttcccaaatgtctctctcagccctgatgttttctcacagtgcttccttgt
ccttctcttaacttctcattcctctataagaatgatttagactgacctgtcctttttatctgcgcatgcgagaacatcacctt
cctctgtacacttggaaatgcctctggcttgttgcagccctcctttaacccaaaggaggaaaggactgcttcagaaactcccaa
ttccaaaaagctgagtctgggtccattattttggcagaactcctaagaatttatgggagcctatataaacatatcttgcttta
aaaagttcttgagggaatagcaactttcccatggctgtgcctatttcctagacctttaaaagatgtgcagagcagcttagcat
tcgttgcagctgagcctaatttttcttgctcatccttgtccctttgacaataaggttaattgatagacccaccacctcttgca
ctctcgcttttggagcaagttgcattaactatttgagtctctatattgtccaagaaaagtagaaataataaatttactttccc
tttttctatcaccttatgtcctctaccatttctccttcctcccttcccttatttctccttttcgtaccctgtgtcctccctg
atttcctttcgtttcttctttatttatcccattctctgttacttgactcagtgctcccttcctctcctctccttctagtgga
tgcatgcagcctttttttcaattttatttaaattgcaaaattttactcagatttttttcctcttccctaattgctaagatt
taaggacgttctttattatgaaactttatcacattcgaaatgtttgtttacagtgggatttagggggggattgtgtttaaatca
aatatatgtattttaaaaataatgacatgctcaaccttcctcatcatggagtaagaaaattctacatgattaaagaatccatgt
aagtctaatttaaattcctagtaactagagaaaagacttatttatataaaatgaagtatttatgaactgtgataaagcatcaa
atcttgatgaaggattgtagattttgcttttcttttgtttttaaaacttattccaattgctaaattggtagtttttcagtc
tttataaatacaggattaaaaatatatatacagttatatgaaatgtttattttctatgtgtgtgcatatagttcaatattatgc
aataaatttggtgttttaactt
<212> Type : DNA
<211> Length : 4894
SequenceName : D25215
SequenceDescription :

Sequence
--------
<213> OrganismName : HUMAN

<400> PreSequenceString :
cggggctggaggcggtggctgcggttgcgggaccggcactatgctgggccttcctaccacttgtgtgtggcttggtagtggcct
agggtctctccctcctgctgaagtccctctcctgcaggtggccgtctgcccggcccagcaccatgcacacgcttgtgttcttga
gcacacggcaggtgctgcagtgccagccagctgcctgccaggccctgcccctgctgccacgcgaactcttcccccctgctgttca
aggtggccttcatggacaagaagacagtggtactgcgcgagttggtacacacgtggcccttcccgctgctcagtttccagcagc
tgctacaggagtgtgcccactgcagccgtgccctcctgcaggagcggcctagcactgagagcatgcaggctgttatcctggggc
tgactgcccggctccacacctcagagcctggggccagcacacagcccctctgcaggaagcatgcgctgcgggtgctggacatga
cgggcctcttggatgatggtgtggaacaggatcctggcaccatgagcatgtgggactgtactgctgccgtagctcgcacatgca
ttgcccagcagcagggtggggccgcagagcctgggccagccccccatccccgtggaggtgcgcgtggacctgcgggtgaaccggg
cctcctatgcgttcctgcgggaggcactccgaagcagcgtgggcagcccgctgcggctctgctgccgggacctgcgagctgagg
acctgcccatgcgcaacactgtggccctgctgcagcttctggatgcaggctgcctgcgccgcgtggacctgcgcttcaacaatc
tgggcctgcgcggcctgtctgtgatcatcccacacgtggcccgcttccagcacctggccagcctgcggctccactatgtgcatg
ggattcaaggcagccctccgtggatggcgaggacaacttccgctacttccttgcccagatgggccgcttcacctgtctgcgtg
agctcagcatgggctcctctctcctttcagggaggctggaccagctgctcagcaccctgcagagcccccctggagagcctggagt
tggccttctgtgctctgctgcctgaggacctacgcttcctggcacggagcccacatgctgcccacctcaagaagttggacctga
gtggtaacgacctgtctggcagccagctggcacccttccagggtctgttgcaggcatcagcagccacactgttgcatctggagc
tgactgagtgtcagctcgcagacacccagctgttggccacactacccatcctgactcagtgcgccagtctccggtaccttggcc
tctatggcaacccactgtccatggcgggcctcaaggagctgctgcgggactcagtggcacaggctgagctgcgtactgtggtgc
acccccttccctgtggactgctatgagggcttgccctggccgccgcctgcctctgtcctgctggaggcctccatcaatgaggaga
agtttgcccgcgtagaagctgagttgcaccagctgcttctagcctcaggccgtgcccatgtgctctggaccacggacatctacg
ggcgactggctgcggactacttcagcctatgatgaagtagctctgggtgagacacaggccgccctgcagtctctttaggtaggc
agggcctttgctgggacccctggtggaggccttcacaaaagcactggttactggtttcctgctgggtctaccttgcttctgggc
acacctcaagcctcccctgctttctgcagtgccccacgcggttttccctgcacttgctccataattggctgatcatctgtgggc
cccggggctggatgtcaggcctccattgccctgctcagtttggctgcatttggctgccgtctggggtcctggtcctttgtgcaa
atgctttggattccagttgtgagctgagagagatgatggcctctctgggcctttcctctccctttactgagagctcagtgctt
ctggggttgaagttggacagaggcctgcttcagggaagctgggagtccccaggcactcacgcctcttacgtgttccctaccctg
ccacccagccaccccactgggctgggctcgggctggaggggggtcatcaaggtacacatgtgcctggaagttgaatttgtggctg
tttttttttctgtccacgttggtcacccttatccttatctctgctgtcacccccaacatggccaccgggcaacaactgccatcc
agcctgtcgccccgcccttcgcggggcagccccgtcggcactgccggccagtccttgctcttcccacctttcggaggcccaaga
tcctactgtggccggccagggccagcgaggacccccccatgcagagctggaggttggggtgatgtcttttcggaagagcttc
aagggaggtgttggggcctccccggccaccttccattgctaccccaggattcccgagtgcaacgttcccggctcgcgccccaca
cacggctcagcgcacactgcgcggcttccacctttactgacggagcatgcgcgaggccgcaccggccaatctccggcgcccacg
tcatccgcgcgcccgcggccctagcagtggatctcgtaggcgaccggcgggggcacgcggagtcccggccccgcccccctgttcc
gggccgcagtcagcgggcgcctccgccggaccctcggcgaagagcggcttggagcggttgatgacgaacatctcgtggccgcgc
tcgtcgcggagctcctctagctgtgcgaacgtacaggggccgtccaagtagtcgttgacgaacagcgctccctcccccggaggc
ccccgcgcctttttttcgcctgcggcgccggcgacagatcatggcgaccaggagcagcgccgtgagcgccagcagcgcgatggcc
gccgcaatggccgtctgtgtggccacgcccagggcgcggaaggccatgctgcccgcctcggccggggctcgctgccggcgggg
cgggcggccggaggcggcggttgcgcgggctgctgcggctgctgccgggacgcgttgaccaggagccggaagggcacgcgggca
gcgccgccggcgttggaggcctcgcactcgtacttaccggcgtgcgccagcgtgatgttgctgaggaagagcatgccgctgccc
gtgtcggatgccgagtgtccgcccaggcccagcaacccgccttctagctgggcctgggctcgcggccggccctcgcgaggctgg
ggcacctttctccaggtcaccaatggctgcgggtagccggaggcttggcaggcaacccgcaggtcctcacccaggttggctgtg
agctccagcggctgcacgtggacagagggcggaatgcagatgaggctgctgtgggatacgtccaggagactctggagcgccagg
cgcggggggctctgcacacatgatcttcctgtccctggaggtgagcagccgctggccgccctccttgatccaggcccccagccag
tgcagggcgcagtcacagcgccatgggttctctgtgggagagcagcgttaggcaggtggcttgagggtgctgctaaaacagcct
gtgcagttggggttttgcaggccaggacagaggcctctttcccacctcccacagcgttttcacacggagtccaaggccctgcca
ccccttccttgaccccaagctccttggggcggcaggcccttcaccctcgccccccctcccctttagctctgtgatgcctgcctgt
tacagatcacttctccgtcggtctctgagaaagcacctgctccttaagtcttcctgcaacaagtgccactgttttaggaacct
gggcgtccacatagacatctcaccagcactgaaacctcacaagtcctctcagctttgcctttggatgccctctcttgggaatgt
ccccagtcctggtcagctgtctctctcctttgcaattttgtctgcctcccctcagcctaaaagtgtgcagaaccctcaattctg
ttaagtcaccctgtggagttcctgtcttctgtttccccaggcagggtgcctgagctgtatcccccagcacacccactcccgca
gccctccagtgtggctgcaggcggtggtgcagccttccagactgctgcccagttgcctgatgtcagagcccctccacacatgag
cctgctccctactgccaacaccgtggcccagacagagacgctttccgaggaagaggtacctgtgaggcgcaggacttgcagact
ggccaggggctgcagggcctctcggctgatggtgcccagctggttcctgctgaggtccagcagtgctagggaggacagccccgc
tagagcctggtcctccagcagctcaatgctgttttcttgcaggtgaagctcctgcagtcgctgaagtaaggacagcagatcgtg
aggaaaaagggcgccgaggttgggggcatgtctctcttcttaccaagctagactgggttgccttttctaactattccagccta
cagggcgaggggccataatggagtatcccgcccctttagaccccaggcgctcaccggcaggtgcaagaaggtgaaatccagcag
ccgcgccagctggttgcccgccaggtagagcacgcgcagctgggccaggcctacgaaggcgccgctgcgcaagccgcgcagccg
gttgctagtgagcgccagctccagcaggcgcggctgcgcgcggaaggcgccggcctccagggcgcgcaggctgttgttgtgcag
gtagagccggcgcagagcggcgagtggcgccagggctcccggctctaggcgggcgatgttgttgtcctgcaggaacagtgtctg
caggccggggaaagaggaggcgcttaccccgctgcgggggtcttcctctccctgggggcaccccgtcctcccgcagctccacac
ggtgcccacctgcgtccctggcgggattcccagcgggacgacgcgcaaccgcagggcgccacactccaccgtggcgctgtagca
gcggcaggctgctgggcagccggcggcgcggagcggcagtagtagcagcagcagcggcagcagttcggggccctcagggccat
ctccccgaggcccggttcctcaccggcccttccgcggttcagccgcagacgcgtgccctcctgaaacacaggttggcaggccagt

ctcggcagtcgagagccagccaatagatggaatggaggcctgcacctgcgtctaacttttgacactataaataggttcaagaaa
ctaataaaacgttctggttttctcctttgac
<212> Type : DNA
<211> Length : 5323
      SequenceName : D25216
      SequenceDescription :

Sequence
--------
<213> OrganismName : HUMAN
<400> PreSequenceString :
ttaattcggcggccgcggatccgagccggacgggcactgggcgactctgtgcttcgctgaggaaaaataactaaacatgggcaa
aggagatcctaagaagccgagacggaaaatgtcatcatatgcatttttgtgcaaacttgtcgggaggagcataagaagaagca
cccagatgcttcagtcaacttctcagagttttctaagaagtgctcagagaggtggaagaccatgtctgctaaagagaaaggaaa
atttgaagatatggcaaaagcggacaaggcccgttatgaaagagaaatgaaaacctatatccctcccaaaggggagacaaaaaa
gaagttcaaggatcccaatgcacccaagaggcctccttcggccttcttcctcttctgctctgagtatcgcccaaaaatcaaagg
agaacatcctggcctgtccattggtgatgttgcgaagaaactgggagagatgtggaataacactgctgcagatgacaagcagcc
ttatgaaaagaaggctgaaaagctgaaggaaaaatacgaaaaggatattgctgcatatcgagctaaaggaaagcctgatgcagc
aaaaaagggagttgtcaaggctgaaaaaagcaagaaaaagaaggaagaggaggaaggtgaggaagatgaagaggatgaggagga
ggaggaagatgaagaagatgaagatgaagaagaagatgatgatgatgaataagttggttctagcgcagtttttttttcttgtc
tataaagcatttaaccccctgtacacaactcactcctttaaagaaaaaaattgaaatgtaaggctgtgtaagatttgttttt
aaactgtacagtgtctttttttgtatagttaacacactaccgaatgtgtctttagatagccctgtcctggtggtatcttcaata
gccactaaccctgcctggtacagtatgggggttgtaaattggcatggaaatttaaagcaggttcttgtttgggcacagcacaaa
ttagttatatatggggatggtagtttttcatcttcagttgtctctgatgcagctatacgaaataattgttgttctgttaactg
aataccactctgtaattgcaaaaaaaaaaaaaagttgcagctgttttgttgacattctgaatgcttctaagtaaatacaattt
ttttattaaaaaaaaa
<212> Type : DNA
<211> Length : 1194
      SequenceName : D63874
      SequenceDescription :

Sequence
--------
<213> OrganismName : HUMAN
<400> PreSequenceString :
gttttctactttgcccgcccacagatgtagtttctctgcgcgtgtgcgttttccctcctcccccgccctcagggtccacggcc
accatggcgtattaggggcagcagtgcctgcggcagcattggcctttgcagcggcggcagcagcaccaggctctgcagcggcaa
cccccagcggcttaagccatggcgtgagtaccggggcgggtcgtccagctgtgctcctggggccggcgcgggttttggattggt
ggggtgcggcctggggccagggcggtgccgccaagggggaagcgatttaacgagcgcccgggacgcgtggtctttgcttgggtg
tccccgagacgctcgcgtgcctgggatcgggaaagcgtagtcgggtgcccggactgcttccccaggagccctacagccctcgga
ccccgagccccgcaaggtcccaggggtcttggctgttgccccacgaaacgtgcaggaaccaagatggcggcggcagggcggcgg
cgcgggcgtgagtcaagggcgggcggtgggcggggcgcggccgctggccgtatttggacgtggggacggagcgctttcctcttg
gcggccggtggaagaatcccctggtctccgtgagcgtccattttgtggaacctgagttgcaagcagggaggggcaaatacaact
gccctgttcccgattctctagatggccgatctagagaagtcccgcctcataagtggaaggatgaaattctcagaacagctaacc
tctaatgggagttggcttctgattctcattcaggcttctcacggcattcagcagcagcgttgctgtaaccgacaaagacacctt
cgaattaagcacattcctcgattccagcaaagcaccgcaacatgaccgaaatgagcttcctgagcagcgaggtgttggtgggg
acttgatgtccccccttcgacccgtcgggtttgggggctgaagaaagcctaggtctcttagatgattacctggaggtggccaagc
acttcaaacctcatgggttctccagcgacaaggctaaggcgggctcctccgaatggctggctgtggatgggttggtcagtccct
ccaacaacagcaaggaggatgccttctccgggacagattggatgttggagaaaatggatttgaaggagttcgacttggatgccc
tgttgggtatagatgacctggaaaccatgccagatgaccttctgaccacgttggatgacacttgtgatctctttgccccctag
tccaggagactaataagcagccccccagacggtgaacccaattggccatctcccagaaagtttaacaaaacccgaccaggttg
ccccttcaccttcttacaacctcttcccctttccccaggggtcctgtcctccactccagatcattcctttagtttagagctgg
gcagtgaagtggatatcactgaaggagataggaagccagactacactgcttacgttgccatgatccctcagtgcataaaggagg
aagacaccccttcagataatgatagtggcatctgtatgagcccagagtcctatctggggtctcctcagcacagcccctctacca
ggggctctccaaataggagcctcccatctccaggtgttctctgtgggtctgcccgtcccaaaccttacgatcctcctggagaga
agatggtagcagcaaaagtaaagggtgagaaactggataagaagctgaaaaaaatggagcaaaacaagacagcagccactaggt
accgccagaagaagagggcggagcaggaggctcttactggtgagtgcaaagagctggaaaagaagaacgaggctctaaaagaga
gggcggattccctggccaaggagatccagtacctgaaagatttgatagaagaggtccgcaaggcaagggggaagaaaagggtcc
cctagttgaggatagtcaggagcgtcaatgtgcttgtacatagagtgctgtagctgtgtgttccaataaattattttgtaggg
<212> Type : DNA
<211> Length : 2015
      SequenceName : D90209
      SequenceDescription :

Sequence
--------
<213> OrganismName : HUMAN
<400> PreSequenceString :
ttttttttttttttttttttacgtttaacctaatttattcatgcttgccttgggatggggaatagatcattcagtaaaaacat
acagtaaaaacaaaatgtcttatcatgtacaactttcaaactacaataatgatgtaccttaattacttccatgcacacaagtct
aacatttgtttttttaaaaaataaacacaattaagacttctaggagcattttataataaagtaattcctaattaattttncttt
gtagatagatcaagcacctccaaaatacaaattcctatacacagtgaggcacgttacttaaaatgaacacttaagtaaattaag
gtacgtgggacaggccttaggggtaagctggacattataggtggcggctagggtagggcaacaaaccntaggtggcccantggg
gaaaagtttttttggcaaattaattttaaaaactag
<212> Type : DNA
<211> Length : 457
      SequenceName : H17504
      SequenceDescription :

Sequence
--------
<213> OrganismName : HUMAN
<400> PreSequenceString :
ttttttttttnttttttttaaggattttaaaaatatatctattttgaaaatattctgaaagtaattaagatcacctattgggaa
ggaaagaaatcaacacaatgaaatttaggaagagaaatgcaaaagcagctgcagaatcctgccccttccaccccaaggggcaca
gccggnaggatgaagcttattttaggatcacaagtgcacgctctggtaggacggaggatgactttgtcagtcttctgggctgaa
gggccttgtccaaagtcaggatcagaaatagggtcagctcatcatgcttgggctttgttgggtcttggtagttaggataagggt
tcaagggctttntttttccatgaggggagagatttgggacacttcgcagggtttacg
<212> Type : DNA
<211> Length : 393
      SequenceName : H22919
      SequenceDescription :

Sequence
--------
<213> OrganismName : HUMAN
<400> PreSequenceString :
gtgttgagttggaagaattggcaaaatatctggaccattaagaaaacggatttgtaactagctttaaactaggccaagcaact
agttttcctgcaaatcaaatttttaaagcaacttgggttagactttgtttttgacctaacatcccttccttaaatgccttctgt
agtttcagatcagtagggagaccatataataattttatggtacctgtttcaaaacatatttttttctgtttttataagtaagttg
atattaattaaactcttggcaatatttctnctttcttaaaggaaaatataccttaactttttttcttt
<212> Type : DNA
<211> Length : 320
      SequenceName : H27379
      SequenceDescription :

Sequence
--------
<213> OrganismName : HUMAN
<400> PreSequenceString :
atntatnttggcagggcttgatctggaaagaagtttggaaaccctggggagaaattagttaaaaagaagtggaatcttgatga
gctgcctaaatttgagaagaattttttatcaagagcaccctgatttggctaggcgcacagcacaagaggtggaaacatacagaag
aagcaaggaaattacagttagaggtcacaactgcccgaagccagttctaaatttttatgaagccaatttccctgcaaatgtcat
gggatgttattgcaagacagaattttcactgaaccccactgctatttcaaggctcaggggggtgggccagtttgctcttagtggg
gtttgggtatggggttgggagttggccacagacttggatcnttgggggaaacattgtccttatttttgctttccccgcccattt
ttccccccatncattcnttcaggcctttccncgggggagag
<212> Type : DNA
<211> Length : 461
      SequenceName : H27564
      SequenceDescription :

Sequence
--------
<213> OrganismName : HUMAN
<400> PreSequenceString :
gcctactatgccagatgcctttatggctgaaaccgcaacacccatcaccacttcaatagatcaaagtccagcaggcaaggacgg

ccttcaactgaaaagactcagtgttccctttcctactctcaggatcaagaaagtgttggctaatgaagggaaaggatattttct
tccaagcaaaggtgaagagaccaagactctgaaatctcagaattccttttctaactctcccttgctcgctgtaaaatcttggca
cagaaacacaatatttgtggctttctttctttgcccttcacagtgtttcgacagctgattacacagttcctgtcataaggaa
tgaataattaattatccagagtttagaggaaaaaaatgactaaaaatattattaacttaaaaaatggacaggtgttggatgccc
acaggcaaatgcatggggggttgtttaatgggtgcaaatccctacttgaatgctctt
<212> Type : DNA
<211> Length : 478
      SequenceName : H39560
      SequenceDescription :

Sequence
--------
<213> OrganismName : HUMAN
<400> PreSequenceString :
ntnnnttnnaagtctaagtgtttaattattattcacatatttcacagaaaaaaaggaatgtagcaaaggggtcaagattgtagc
aaaaaacaaaaaatcctggatttacgggtcactctatttgtacttgggagcagggctgtcccgacatcaggcgcanagctgca
cttctccgatgcccctttgcagatgcagccctgggcacacttggcacagcccacaggnagncaggagcagcagctcttcttgca
ggaggtgcatttgcctctttgcacttgcaggagctggcgcaggtgcaggagacaccagcggcacaggagcagttggggggtccat
ttgcaacccgagggcgagactaggagttcccaaggcgagaaggggaaggagggcagtggggttgcacgtnggaagggnttaggg
gtnccagcacgtggggttngtaccccngggcctngggttcagacaggg
<212> Type : DNA
<211> Length : 468
      SequenceName : H53340
      SequenceDescription :

Sequence
--------
<213> OrganismName : HUMAN
<400> PreSequenceString :
ctctcaatgggcgcacggctacgttctgcagcctgagagcatgaggacagagaaatatgacccgatgccacccgagtcccaga
ggaagatcctgatgacgctgacagtcaaggttctcggtgctcgccatctccccaaacttggacgaagtattgcctgtncctttg
tagaagtggagntctgtggagccgagtatgacaacaacaagttcaagacgacggttgtgaatgataatggcctcagncctatct
gggctccaacacaggagaaggtgacatttganatttatgacccaaacctgggnatttttgcgcttngtggtttattgaaggaa
ggtattgtttcagcgnttccccaatttttttggntcatggccactttacccctttaaaggcagtcaaaatcagggnttcaggg
tncct
<212> Type : DNA
<211> Length : 425
      SequenceName : H57180
      SequenceDescription :

Sequence
--------
<213> OrganismName : HUMAN
<400> PreSequenceString :
actataacttagtgtctgtatttaatattgacaaccaaaaatatatatanttttnttgcatctatacacaacagggcaggagtc
tccatgtnttcttgagcagtgagtttgcaggctcccacaggccctcttctcatggtaatagtgtggccctagtgcaaaggagac
tagaacccggcagcccagactggcccttcccctctcctccctgcactccagtgcttcccaactggtctcaggtaaagaaagntt
antttgagtggttgggtaggaagagatgggaaggggcaaatcctaatgggagcctgacccctagagtggggagttccagggcca
gcagaacgggtgggccatagccctncctggggntagaagctttgtagttcatagttcgattagtntgtccntagggcatnaggt
nccagccctacagattagct
<212> Type : DNA
<211> Length : 440
      SequenceName : H58873
      SequenceDescription :

Sequence
--------
<213> OrganismName : HUMAN
<400> PreSequenceString :
gcttatttagctgcaaagtggcatcatattatnccatttaatgaaattcacctcaagccctttttgacatattaaatatatgg
gatatatttaaggcaagagaagtaaggcaatccaaatgagtgccctttcccaatctcagcactgtcttggtggaattggtgaca
ctattcagataaccaactggagaccgacaggatttgccatgcatttgcatcttgctagagtttggttttatgaaagggcctat
t

<212> Type : DNA
<211> Length : 253
      SequenceName : H62473
      SequenceDescription :

Sequence
--------
<213> OrganismName : HUMAN
<400> PreSequenceString :
ccccaggctggagtgcagtggcatactcatggctcactgacgcctccaattcctaaactccaatgaccttctcacctcagcctg
caagtagctaggattacagatgtgtgccgccatgctaattttnaaaaatttttgtatagatgggtnctcactatgagcccagg
ctcatcttgaagtnctgggctcaagcaatcctctnaccttggcttcccaaagtactgggattacaggcgggacacncatgccag
ccgtcactctcatttttgagcacaggaattgtgcccaaaggctaggataaggaattaaagatgcatgccaagaaatacccacaa
acattaattataaaaggggagactatacnaatgttttgtggaaaag
<212> Type : DNA
<211> Length : 382
      SequenceName : H73054
      SequenceDescription :

Sequence
--------
<213> OrganismName : HUMAN
<400> PreSequenceString :
aaatagaaataacagctaacaggaggtttggagtttcctcatttatgtcttttcctatgaaccttgatttaaagctggacttgc
aggaccattgacagaatgttctggcccctcagtgccactcgtgggttcagaaccttcagcctgttcctttgcttcagggagctt
ttctggggcaccaagtggattaacacagttaggcatcctgctttactttgtccacttcattatcaaattcaattttatcttctt
gatgggttgtttttctttacttgtccattctgaggcagggtagggtgctgggcaatgacgtcatatagggaattgatattgctc
ggaatgtgggaaaccatggcccgggnctaggctttgcgtaggaggctttttaccacttgaaaaatatccant
<212> Type : DNA
<211> Length : 408
      SequenceName : H74265
      SequenceDescription :

Sequence
--------
<213> OrganismName : HUMAN
<400> PreSequenceString :
ctcgnccaccacccaatcctcgcctctcttctgctccaccttctctctgccctcacctctcccccgaaaacccccctatttag
ccaaaggaaggaggtcaggggaacgctctcccctccccttccaaaaaacaaaaacagaaaaacccttttccaggccggggaaag
caggagggagaggggccgccgggctggccatggagctgctgtgccacgaggtggacccggtccgcaggccttgcgggtaccgca
acctgctccgagacgaccgcgtcctgcagaacctgcttcaccatcgaggagcgnttaccttnccgcagtgnttcctaattncaa
gttgcgtgnagaaggacatccaaacctnacat
<212> Type : DNA
<211> Length : 368
      SequenceName : H84153
      SequenceDescription :

Sequence
--------
<213> OrganismName : HUMAN
<400> PreSequenceString :
cgtttaacaaaattgtttaataaaatttataaaaatgcatctttgagaatacttttctcagcttgaattgttttcctttttccac
ccccaaagaaaatacacaattatcagcacccacacatgtatacactcaaaactacagtgacattctctacacagaactatattc
gatatagcttgaactgccgaaaaatcaagacaattccaaaaagtgattgcagggttgatttttttctccaaaacactttgagaa
acacgtaaagctatttcaacaaaagtcttttctttgattgtcaaaagttgaaattcacatttaaataaaaagagatccaaatca
agatcctcactnacccccctacccctcaactgaacccccttttagggccacatttcttcttgctcctaagaaaaaaatttggaa
ttttgaatattctcggttttct
<212> Type : DNA
<211> Length : 442
      SequenceName : H97778
      SequenceDescription :

Sequence

--------
<213> OrganismName : HUMAN
<400> PreSequenceString :
tgcagctgcctggctgcctggccctggctgccctgtgtagccttgtgcacagccagcatgtgttcctggctcctcagcaagcac
ggtcgctgctccagcgggtccggcgagccaacaccttcttggaggaggtgcgcaagggcaacctagagcgagagtgcgtggagg
agacgtgcagctacgaggaggccttcgaggctctggagtcctccacggctacggatgtgttctgggccaagtacacagcttgtg
agacagcgaggacgcctcgagataagcttgctgcatgtctggaaggtaactgtgctgagggtctgggtacgaactaccgagggc
atgtgaacatcacccggtcaggcattgagtgccagctatggaggagtcgctacccacataagcctgaaatcaactccactaccc
atcctggggccgacctacaggagaatttctgccgcaaccccgacagcagcaacacgggaccctggtgctacactacagacccca
ccgtgaggaggcaggaatgcagcatccctgtctgtggccaggatcaagtcactgtagcgatgactccacgctccgaaggctcca
gtgtgaatctgtcacctccattggagcagtgtgtccctgatcgggggcagcagtaccaggggcgcctggcggtgaccacacatg
ggctcccctgcctggcctgggccagcgcacaggccaaggccctgagcaagcaccaggacttcaactcagctgtgcagctggtgg
agaacttctgccgcaacccagacggggatgaggagggcgtgtggtgctatgtggccgggaagcctggcgactttgggtactgcg
acctcaactattgtgaggaggccgtggaggaggagacaggagatgggctggatgaggactcagacagggccatcgaagggcgta
ccgccacaagtgagtaccagactttcttcaatccgaggacctttggctcgggagaggcagactgtgggctgcgacctctgttcg
agaagaagtcgctggaggacaaaaccgaaagagagctcctggaatcctacatcgacgggcgcattgtggagggctcggatgcag
agatcggcatgtcaccttggcaggtgatgcttttccggaagagtccccaggagctgctgtgtgggccagcctcatcagtgacc
gctgggtcctcaccgccgcccactgcctcctgtacccgccctgggacaagaacttcaccgagaatgaccttctggtgcgcattg
gcaagcactcccgcaccaggtacgagcgaaacattgaaaagatatccatgttggaaaagatctacatccaccccaggtacaact
ggcgggagaacctggaccgggacattgccctgatgaagctgaagaagcctgttgccttcagtgactacattcaccctgtgtgtc
tgcccgacagggagacggcagccagcttgctccaggctggatacaaggggcgggtgacaggctggggcaacctgaaggagacgt
ggacagccaacgttggtaaggggcagcccagtgtcctgcaggtggtgaacctgcccattgtggagcggccggtctgcaaggact
ccacccggatccgcatcactgacaacatgttctgtgctggttacaagcctgatgaagggaaacgaggggatgcctgtgaaggtg
acagtggggggaccctttgtcatgaagagcccctttaacaaccgctggtatcaaatgggcatcgtctcatggggtgaaggctgtg
accgggatgggaaatatggcttctacacacatgtgttccgcctgaagaagtggatacagaaggtcattgatcagtttggagagt
agggggccactcatattctgggctcctggaaccaatcccgtgaaagaattattttgtgtttctaaaactatggttcccaataa
aagtgactctcagcg
<212> Type : DNA
<211> Length : 1947
      SequenceName : I16618
      SequenceDescription :

Sequence
--------
<213> OrganismName : HUMAN
<400> PreSequenceString :
ggaccacccagtaccgatcccttcacgaccgtcaccatggaagtgtcaccattgcagcctgtaaatgaaaatatgcaagtcaac
aaaataaagaaaaatgaagatgctaagaaaagactgtctgttgaaagaatctatcaaaagaaaacacaattggaacatattttg
ctccgcccagacacctacattggttctgtggaattagtgacccagcaaatgtgggtttacgatgaagatgttggcattaactat
agggaagtcacttttgttcctggtttgtacaaaatctttgatgagattctagttaatgctgcggacaacaaacaaagggaccca
aaaatgtcttgtattagagtcacaattgatccggaaaacaatttaattagtatatggaataatggaaaaggtattcctgttgtt
gaacacaaagttgaaaagatgtatgtcccagctctcatatttggacagctcctaacttctagtaactatgatgatgatgaaaag
aaagtgacaggtggtcgaaatggctatggagccaaattgtgtaacatattcagtaccaaatttactgtggaaacagccagtaga
gaatacaagaaaatgttcaaacagacatggatggataatatgggaagagctggtgagatggaactcaagcccttcaatggagaa
gattatacatgtatcacctttcagcctgatttgtctaagtttaaaatgcaaagcctggacaaagatattgttgcactaatggtc
agaagagcatatgatattgctggatccaccaaagatgtcaaagtctttcttaatggaaataaactgccagtaaaaggatttcgt
agttatgtggacatgtatttgaaggacaagttggatgaaactggtaactccttgaaagtaatacatgaacaagtaaaccacagg
tgggaagtgtgtttaactatgagtgaaaaaggctttcagcaaattagctttgtcaacagcattgctacatccaagggtggcaga
catgttgattatgtagctgatcagattgtgactaaacttgttgatgttgtgaagaagaagaacaagggtggtgttgcagtaaaa
gcacatcaggtgaaaaatcacatgtggattttgtaaatgccttaattgaaaacccaacctttgactctcagacaaaagaaaac
atgactttacaacccaagagctttggatcaacatgccaattgagtgaaaaatttatcaaagctgccattggctgtggtattgta
gaaagcatactaaactgggtgaagtttaaggcccaagtccagttaaacaagaagtgttcagctgtaaaacataatagaatcaag
ggaattcccaaactcgatgatgccaatgatgcagggggccgaaactccactgagtgtacgcttatcctgactgagggagattca
gccaaaactttggctgtttcaggccttggtgtggttgggagagacaaatatggggtttccctcttagaggaaaaatactcaat
gttcgagaagcttctcataagcagatcatggaaaatgctgagattaacaatatcatcaagattgtgggtcttcagtacaagaaa
aactatgaagatgaagattcattgaagacgcttcgttatgggaagataatgattatgacagatcaggaccaagatggttcccac
atcaaaggcttgctgattaattttatccatcacaactggccctctcttctgcgacatcgttttctggaggaatttatcactccc
attgtaaaggtatctaaaaacaagcaagaaatggcattttacagccttcctgaatttgaagagtggaagagttctactccaaat
cataaaaaatggaaagtcaaatattacaaaggtttgggcaccagcacatcaaaggaagctaaagaatactttgcagatatgaaa
agacatcgtatccagttcaaatattctggtcctgaagatgatgctgctatcagcctggcctttagcaaaaaacagatagatgat
cgaaaggaatggttaactaatttcatggaggatagaagacaacgaaagttacttgggcttcctgaggattacttgtatggacaa
actaccacatatctgacatataatgacttcatcaacaaggaacttatcttgttctcaaattctgataacgagagatctatccct
tctatggtggatggtttgaaaccaggtcagagaaaggttttgtttacttgcttcaaacggaatgacaagcgagaagtaaaggtt

gcccaattagctggatcagtggctgaaatgtcttcttatcatcatggtgagatgtcactaatgatgaccattatcaatttggct
cagaattttgtgggtagcaataatctaaacctcttgcagcccattggtcagtttggtaccaggctacatggtggcaaggattct
gctagtccacgatacatctttacaatgctcagctctttggctcgattgttatttccaccaaaagatgatcacacgttgaagttt
ttatatgatgacaaccagcgtgttgagcctgaatggtacattcctattattcccatggtgctgataaatggtgctgaaggaatc
ggtactgggtggtcctgcaaaatccccaactttgatgtgcgtgaaattgtaaataacatcaggcgtttgatggatggagaagaa
cctttgccaatgcttccaagttacaagaacttcaagggtactattgaagaactggctccaaatcaatatgtgattagtggtgaa
gtagctattcttaattctacaaccattgaaatctcagagcttcccgtcagaacatggacccagacatacaaagaacaagttcta
gaacccatgttgaatggcaccgagaagacacctcctctcataacagactatagggaataccatacagataccactgtgaaattt
gttgtgaagatgactgaagaaaaactggcagaggcagagagagttggactacacaaagtcttcaaactccaaactagtctcaca
tgcaactctatggtgctttttgaccacgtaggctgtttaaagaaatatgacacggtgttggatattctaagagactttttgaa
ctcagacttaaatattatggattaagaaaagaatggctcctaggaatgcttggtgctgaatctgctaaactgaataatcaggct
cgctttatcttagagaaaatagatggcaaaataatcattgaaaataagcctaagaaagaattaattaaagttctgattcagagg
ggatatgattcggatcctgtgaaggcctggaaagaagcccagcaaaaggttccagatgaagaagaaaatgaagagagtgacaac
gaaaaggaaactgaaaagagtgactccgtaacagattctggaccaaccttcaactatcttcttgatatgcccctttggtattta
accaaggaaaagaaagatgaactctgcaggctaagaaatgaaaaagaacaagagctggacacattaaaaagaaagagtccatca
gatttgtggaaagaagacttggctacatttattgaagaattggaggctgttgaagccaaggaaaaacaagatgaacaagtcgga
cttcctgggaaagggggaaggccaaggggaaaaaaacacaaatggctgaagttttgccttctccgcgtggtcaaagagtcatt
ccacgaataaccatagaaatgaaagcagaggcagaaaagaaaaataaaaagaaaattaagaatgaaaatactgaaggaagccct
caagaagatggtgtggaactagaaggcctaaaacaaagattagaaaagaaacagaaaagagaaccaggtacaaagacaaagaaa
caaactacattggcatttaagccaatcaaaaaaggaaagaagagaaatccctggcctgattcagaatcagataggagcagtgac
gaaagtaattttgatgtccctccacgagaaacagagccacggagagcagcaacaaaaacaaaattcacaatggatttggattca
gatgaagatttctcagattttgatgaaaaaactgatgatgaagatttgtcccatcagatgctagtccacctaagaccaaaact
tccccaaaacttagtaacaaagaactgaaaccacagaaaagtgtcgtgtcagaccttgaagctgatgatgttaagggcagtgta
ccactgtcttcaagccctcctgctacacatttcccagatgaaactgaaattacaaacccagttcctaaaaagaatgtgacagtg
aagaagacagcagcaaaaagtcagtcttccacctccactaccggtgccaaaaaaagggctgccccaaaaggaactaaaagggat
ccagctttgaattctggtgtctctcaaaagcctgatcctgccaaaaccaagaatcgccgcaaaaggaagccatccacttctgat
gattctgactctaattttgagaaaattgtttcgaaagcagtcacaagcaagaaatccaagggggagagtgatgacttccatatg
gactttgactcagctgtggctcctcgggcaaaatctgtacgggcaaagaaacctataaagtacctggaagagtcagatgaagat
gatctgttttaaaatgtgaggcgattattttaagtaattatcttaccaagcccaagactggttttaaagttacctgaagctctt
aacttcctcccctctgaatttagtttggggaaggtgttttagtacaagacatcaaagtgaagtaaagcccaagtgttctttag
cttt
<212> Type : DNA
<211> Length : 4792
      SequenceName : J04088
      SequenceDescription :

Sequence
--------
<213> OrganismName : HUMAN
<400> PreSequenceString :
cccggggaggggaccggggaacagagggccgagaggcgtgcggcagggggggagggtaggagaaagaagggcccgactgtaggag
ggcagcggagcattacctcatcccgtgagcctccgcgggcccagagaagaatcttctagggtggagtctccatggtgacgggcg
ggcccgccccccctgagagcgacgcgagccaatgggaaggccttggggtgacatcatgggctatttttaggggttgactggtagc
agataagtgttgagctcgggctggataagggctcagagttgcactgagtgtggctgaagcagcgaggcgggagtggaggtgcgc
ggagtcaggcagacagacagacacagccagccagccaggtcggcagtatagtccgaactgcaaatcttattttcttttcacctt
ctctctaactgcccagagctagcgcctgtggctcccgggctggtggttcgggagtgtccagagagccttgtctccagccggccc
cgggaggagagccctgctgcccaggcgctgttgacagcggcggaaagcagcggtaccccacgcgcccgccggggggacgtcggcg
agcggctgcagcagcaaagaactttcccggcggggaggaccggagacaagtggcagagtcccggagcgaacttttgcaagcctt
tcctgcgtcttaggcttctccacggcggtaaagaccagaaggcggcggagagccacgcaagagaagaaggacgtgcgctcagct
tcgctcgcaccggttgttgaacttgggcgagcgcgagccgcggctgccgggcgccccctcccccctagcagcggaggaggggaca
agtcgtcggagtccgggcggccaagacccgccgccggccggccactgcagggtccgcactgatccgctccgcggggagagccgc
tgctctgggaagtgagttcgcctgcggactccgaggaaccgctgcgcccgaagagcgctcagtgagtgaccgcgacttttcaaa
gccgggtagcgcgcgcgagtcgacaagtaagagtgcgggaggcatcttaattaaccctgcgctccctggagcgagctggtgagg
agggcgcagcggggacgacagccagcgggtgcgtgcgctcttagagaaactttccctgtcaaaggctccgggggggcgcgggtgt
cccccgcttgccagagccctgttgcggccccgaaacttgtgcgcgcacgccaaactaacctcacgtgaagtgacggactgttct
atgactgcaaagatggaaacgaccttctatgacgatgccctcaacgcctcgttcctcccgtccgagagcggaccttatggctac
agtaaccccaagatcctgaaacagagcatgaccctgaacctggccgacccagtggggagcctgaagccgcacctccgcgccaag
aactcggacctcctcacctcgcccgacgtggggctgctcaagctggcgtcgcccgagctggagcgcctgataatccagtccagc
aacgggcacatcaccaccacgccgacccccacccagttcctgtgccccaagaacgtgacagatgagcaggaggggttcgccgag
ggcttcgtgcgcgccctggccgaactgcacagccagaacacgctgcccagcgtcacgtcggcggcgcagccggtcaacggggca
ggcatggtggctcccgcggtagcctcggtggcagggggcagcggcagcggcggcttcagcgccagcctgcacagcgagccgccg
gtctacgcaaacctcagcaacttcaacccaggcgcgctgagcagcggcggcggggcgccctcctacggcgcggccggcctggcc
tttcccgcgcaaccccagcagcagcagcagccgccgcaccacctgccccagcagatgcccgtgcagcacccgcggctgcaggcc

ctgaaggaggagcctcagacagtgcccgagatgcccggcgagacaccgcccctgtcccccatcgacatggagtcccaggagcgg
atcaaggcggagaggaagcgcatgaggaaccgcatcgctgcctccaagtgccgaaaaaggaagctggagagaatcgcccggctg
gaggaaaaagtgaaaaccttgaaagctcagaactcggagctggcgtccacggccaacatgctcagggaacaggtggcacagctt
aaacagaaagtcatgaaccacgttaacagtgggtgccaactcatgctaacgcagcagttgcaaacattttgaagagagaccgtc
gggggctgaggggcaacgaagaaaaaaaataacacagagagacagacttgagaacttgacaagttgcgacggagagaaaaaaga
agtgtccgagaactaaagccaagggtatccaagttggactgggttcggtctgacggcgcccccagtgtgcacgagtgggaagga
cttggtcgcgccctcccttggcgtggagccagggagcggccgcctgcgggctgccccgctttgcggacgggctgtccccgcgcg
aacggaacgttggactttcgttaacattgaccaagaactgcatggacctaacattcgatctcattcagtattaaagggggggagg
gggaggggggttacaaactgcaatagagactgtagattgcttctgtagtactccttaagaacacaaagcggggggagggttgggg
aggggcggcaggagggaggtttgtgagagcgaggctgagcctacagatgaactctttctggcctgctttcgttaactgtgtatg
tacatatatatatttttaatttgattaaagctgattactgtcaataaacagcttcatgcctttgtaagttatttcttgtttgt
ttgtttgggtatcctgcccagtgttgtttgtaaataagagatttggagcactctgagtttaccatttgtaataaagtatataat
tttttatgttttgtttctgaaaattccagaaaggatatttaagaaaatacaataaactattggaaagtactcccctaacctct
tttctgcatcatctgtagatcctagtctatctaggtggagttgaaagagttaagaatgctcgataaaatcactctcagtgcttc
ttactattaagcagtaaaaactgttctctattagacttagaaataaatgtacctgatgtacctgatgctatgtcaggcttcata
ctccacgctcccccagcgtatctatatggaattgcttaccaaaggctagtgcgatgtttcaggaggctggaggaaggggggttg
cagtggagagggacagcccactgagaagtcaaacatttcaaagtttggattgcatcaagtggcatgtgctgtgaccatttataa
tgttagaaattttacaataggtgcttattctcaaagcaggaattggtggcagattttacaaaagatgtatccttccaatttgga
atcttctctttgacaattcctagataaaaagatggcctttgtcttatgaatatttataacagcattctgtcacaataaatgtat
tcaaataccaataacagatcttgaattgcttccctttactactttttgttcccaagttatatactgaagttttattttagt
tgctgaggtt
<212> Type : DNA
<211> Length : 3622
      SequenceName : J04111
      SequenceDescription :

Sequence
--------
<213> OrganismName : HUMAN
<400> PreSequenceString :
tgtcggggacggtaaccgggacccgtgctctgctcctgtcgccttcgcctcctgaatccctagccatatgcgtgagtgcatctc
catccacgttggccaggctggtgtccagattggcaatgcctgctgggagctctactgcctggaacacggcatccagcccgatgg
ccagatgccaagtgacaagaccattgggggaggagatgactccttcaacaccttcttcagtgagacgggcgctggcaagcacgt
gccccgggctgtgtttgtagacttggaacccacagtcattgatgaagttcgcactggcacctaccgccagctcttccaccctga
gcagctcatcacaggcaaggaagatgctgccaataactatgcccgagggcactacaccattggcaaggagatcattgaccttgt
gttggaccgaattcgcaagctggctgaccagtgcacccgtcttcagggcttcttggttttccacagctttggtgggggaactgg
ttctgggttcacctccctgctcatggaacgcctgtcagttgattatggcaagaaatccaagctggagttctccatttacccggc
accccaggtttccacagctgtagttgagccctacaactccatcctcaccacccacaccaccctggagcactctgattgtgcctt
catggtagacaatgaggccatctatgacatctgtcgtagaaacctcgatatcgagcgcccaacctacactaaccttaaccgcct
tattagccagattgtgtcctccatcactgcttccctgagatttgatggagccctgaatgttgacctgacagaattccagaccaa
cctggtcccctaccccgcatccacttccctctggccacatatgcccctgtcatctctgctgagaaagcctaccatgaacagct
ttctgtagcagacatcaccaatgcttgctttgagccagccaaccagatggtgaaatgtgaccctggccatggtaaatacatggc
ttgctgcctgttgtaccgtggtgacgtggttcccaaagatgtcaatgctgccattgccaccatcaaaaccaagcgcacgatcca
gtttgtggattggtgccccactggcttcaaggttggcatcaactaccagcctcccactgtggtgcctggtggagacctggccaa
ggtacagagagctgtgtgcatgctgagcaacaccacagccattgctgaggcctgggctcgcctggaccacaagtttgacctgat
gtatgccaagcgtgcctttgttcactggtacgtgggtgaggggatggaggaaggcgagttttcagaggcccgtgaagatatggc
tgcccttgagaaggattatgaggaggttggtgtggattctgttgaaggagagggtgaggaagaaggagaggaatactaattatc
cattccttttggccctgcagcatgtcatgctcccagaatttcagcttcagcttaactgacagatgttaaagctttctggttaga
ttgttttcacttggtgatcatgtcttttccatgtgtacctgtaatatttttccatcatatctcaaagtaaagtcattaacatca
<212> Type : DNA
<211> Length : 1596
      SequenceName : K00558
      SequenceDescription :

Sequence
--------
<213> OrganismName : HUMAN
<400> PreSequenceString :
aaaggagcaggggcctttggctactttgaggtcacacatgacattaccaaatactccaaggcaaaggtatttgagcatattgga
aagaagactcccatcgcagttcggttctccactgttgctggagaatcgggttcagctgacacagttcgggaccctcgtgggttt
gcagtgaaattttacacagaagatggtaactgggatctcgttggaaataacacccccatttcttcatcagggatcccatattg
tttccatcttttatccacagccaaaagagaaatcctcagacacatctgaaggatccggacatggtctgggacttctggagccta
cgtcctgagtctctgcatcaggtttctttcttgttcagtgatcggggattccagatggacatcgccacatgaatggatatgga

tcacatactttcaagctggttaatgcaaatgggaggcagtttattgcaaattccattataagactgaccagggcatcaaaaac
ctttctgttgaagatgcggcgagactttcccaggaagatcctgactatggcatccgggatctttttaacgccattgccacagga
aagtacccctcctggacttttacatccaggtcatgacatttaatcaggcagaaacttttccatttaatccattcgatctcacc
aaggtgagtcagtaaacaactatattgtttctttttaagtctcttcttacctaattagaaaaaaatctagtcaaacaatta
taataatggggaagtcatatacaaaatacagagggtaccacttcagagtgtcctaagctgtgaatgagtgcttaccagcatctt
acttccacgttcctgtttgtcatttcattgagtatgtgtatgtggcttcatatattgttattaacagggaacagattatgaaaa
gctgatgtactttttcctggggaaactgtcagtatttaccacttactattgtgaaagatttaactaaggcactcatcttaaatt
cttatgttttattggatttaaaaattattttcattggcttgattgtatttgaaatctggtatttttgtgggtagctttgatttc
cttcagttgattgcctggtaattgtgaatatgacatcattttcaggtttggcctcacaaggactaccctctcatcccagttggt
aaactggtcttaaaccggaatccagttaattactttgctgaggttgaacagatagccttcgacccaagcaacatgccacctggc
attgaggccagtcctgacaaaatgcttcagggccgcctttttgcctatcctgacactcaccgccatcgcctgggacccaattat
cttcatatacctgtgaactgtccctaccgtgctcgagtggccaactaccaacgtgacggcccgatgtgcatgcaggacaatcag
ggtggtgctccaaattactaccccaacagctttggtgctccggaacaacagccttctgccttggagcacagcatccaatattct
ggagaagtgcggagattcaacactgccaatgatgataacgttactcaggtgcgggcattctatgtgaacgtgctgaatgaggaa
cagaggaaacgtctgtgtgagaacattgccggccacctgaaggatgcacaaattttcatccagaagaaagcggtcaagaacttc
actgaggtccaccctgactacgggagccacatccaggctcttctggacaagtacaatgctgagaagcctaagaatgcgattcac
acctttgtgcagtccggatctcacttggcggcaagggagaaggcaaatctgtgaggccggggccctgcacctgtgcatgaagct
<212> Type : DNA
<211> Length : 1848
SequenceName : K02400
SequenceDescription :

Sequence
--------
<213> OrganismName : HUMAN
<400> PreSequenceString :
atatgtggccaaatcattccttggaatttcccgttggttatgctcatttggaagatagggcctgcactgagctgtggaaacaca
gtggttgtcaaaccagcagagcaaactcctctcactgctctccacgtggcatctttaataaaagaggcagggtttcctcctgga
gtagtgaatattgttcctggttatgggcctacagcaggggcagccatttcttctcacatggatatagacaaagtagccttcaca
ggatcaacagaggttggcaagttgatcaaagaagctgccgggaaaagcaatctgaagagggtgaccctggagcttggaggaaag
agcccttgcattgtgttagctgatgccgacttggacaatgctgttgaatttgcacaccatggggtattctaccaccagggccag
tgttgtatagccgcatccaggatttttgtggaagaatcaatttatgatgagtttgttcgaaggagtgttgagcgggctaagaag
tatatccttggaaatcctctgaccccaggagtcactcaaggccctcagattgacaaggaacaatatgataaaatacttgacctc
attgagagtgggaagaaagaaggggccaaactggaatgtggaggaggcccgtgggggaataaaggctactttgtccagcccaca
gtgttctctaatgttacagatgagatgcgcattgccaaagaggagatttttggaccagtgcagcaaatcatgaagtttaaatct
ttagatgacgtgatcaaaagagcaaacaatactttctatggcttatcagcaggagtgtttaccaaagacattgataaagccata
acaatctcctctgctctgcaggcaggaacagtgtgggtgaattgctatggcgtggtaagtgcccagtgcccctttggtggattc
aagatgtctggaaatggaagagaactgggagagtacggtttccatgaatatacagaggtcaaaacagtcacagtgaaaatctct
cagaagaactcataaagaaaatacaagagtggagagaagctcttcaatagctaagcatctccttacagtcactaatatagtaga
ttttaaagacaaaatttttcttttcttgatttttttaaacataagctaaatcatattagtattaatactacccatagaaaact
tgacatgtagcttcttctgaaagaattatttgccttctgaaatgtgacccccaagtcctatcctaaataaaaaaagacaaattc
ggatgtatgatctctctagctttgtcatagttatgtgatttcctttgtagctacttttgcaggataataattttatagaaaag
gaacagttgcatttagcttctttcccttagtgactcttgaagtacttaacatacacgttaactgcagagtaaattgctctgttc
ccagtagttataaagtccttggactgttttgaaaagtttcctaggatgtcatgtctgcttgtcaaaagaaataatccctgtaat
atttagctgtaaactgaatataaagcttaataaaaacaaccttgcata
<212> Type : DNA
<211> Length : 1560
SequenceName : K03000
SequenceDescription :

Sequence
--------
<213> OrganismName : HUMAN
<400> PreSequenceString :
ctcgagagctccgccatggccgctctcacccgggacccccagttccagaagctgcagcaatggtaccgcgagcaccgctccgag
ctgaacctgcgccgcctcttcgatgccaacaaggaccgcttcaaccacttcagcttgaccctcaacaccaaccatgggcatatc
ctggtggattactccaagaacctggtgacggaggacgtgatgcggatgctggtggacttggccaagtccaggggcgtggaggcc
gcccgggagcggatgttcaatggtgagaagatcaactacaccgagggtcgagccgtgctgcacgtggctctgcggaaccggtca
aacacacccatcctggtagacggcaaggatgtgatgccagaggtcaacaaggttctggacaagatgaagtctttctgccagcgt
gtccggagcggtgactggaaggggtacacaggcaagaccatcacggacgtcatcaacattggcattgtcggctccgacctggga
cccctcatggtgactgaagcccttaagccatactcttcaggaggtccccgcgtctggtatgtctccaacattgatggaactcac
attgccaaaaccctggcccagctgaacccggagtcctccctgttcatcattgcctccaagacctttactacccaggagaccatc
acgaatgcagagacggcgaaggagtggtttctccaggcggccaaggatccttctgcagtggcgaagcactttgttgccctgtct

actaacacaaccaaagtgaaggagtttggaattgaccctcaaaacatgttcgagttctgggattgggtgggaggacgctactcg
ctgtggtcggccatcggactctccattgccctgcacgtgggttttgacaacttcgagcagctgctctcgggggctcactggatg
gaccagcacttccgcacgacgcccctggagaagaacgcccccgtcttgctggccctgctgggtatctggtacatcaactgcttt
gggtgtgagacacacgccatgctgccctatgaccagtacctgcaccgctttgctgcgtacttccagcagggcgacatggagtcc
aatgggaaatacatcaccaaatctggaacccgtgtggaccaccagacaggccccattgtgtgggggggagccagggaccaatggc
cagcatgcttttaccagctcatccaccaaggcaccaagatgataccctgtgacttcctcatcccggtccagacccagcacccc
atacggaagggtctgcatcacaagatcctcctggccaacttcttggcccagacagaggccctgatgaggggaaaatcgacggag
gaggcccgaaaggagctccaggctgcgggcaagagtccagaggaccttgagaggctgctgccacataaggtctttgaaggaaat
cgcccaaccaactctattgtgttcaccaagctcacaccattcatgcttggagccttggtcgccatgtatgagcacaagatcttc
gttcagggcatcatctgggacatcaacagctttgaccagtggggagtggagctgggaaagcagctggctaagaaaatagagcct
gagcttgatggcagtgctcaagtgacctctcacgacgcttctaccaatgggctcatcaacttcatcaagcagcagcgcgaggcc
agagtccaataaactcgtgctcatctgcagcctcctctgtgactcccctttctcttctcgtccctcctccccggagccggcact
gcatgttcctggacaccacccagagcaccctctggttgtgggcttggaccacgagcccttagcagggaaggctggtctcccccca
gcctaacccccagcccctccatgtctatgctccctctgtgttagaattggctgaagtgtttttgtgcagctgacttttctgacc
catgttcacgttgttcacatcccatgtagaaaaacaaagatgccacggaggaggt
<212> Type : DNA
<211> Length : 1987
      SequenceName : K03515
      SequenceDescription :

Sequence
--------
<213> OrganismName : HUMAN
<400> PreSequenceString :
caagagctccagcaaagactttcactgtagcttgacttgacctgagattaactagggaatcttgagaataaagatgagctctga
aaattgtttcgtagcagagaacagctctttgcatccggagagtggacaagaaaatgatgccaccagtccccatttctcaacacg
tcatgaagggtccttccaagttcctgtcctgtgtgctgtaatgaatgtggtcttcatcaccattttaatcatagctctcattgc
cttatcagtgggccaatacaattgtccaggccaatacacattctcaatgccatcagacagccatgtttcttcatgctctgagga
ctgggttggctaccagaggaaatgctactttatttctactgtgaagaggagctggacttcagcccaaaatgcttgttctgaaca
tggtgctactcttgctgtcattgattctgaaaaggacatgaactttctaaaacgatacgcaggtagagaggaacactgggttgg
actgaaaaaggaacctggtcacccatggaagtggtcaaatggcaaagaatttaacaactggttcaacgttacagggtctgacaa
gtgtgtttttctgaaaaacacagaggtcagcagcatggaatgtgagaagaatttatactggatatgtaacaaaccttacaaata
ataaggaaacgtgttcacttattgactattatagaatggaactcaaggaaatctgtgtcagtggatgctgctctgtggtccgaa
gtcttccatagagactttgtgaaaaaaaatttatagtgtcttgggaatttcttccaaacagaactatggaaaaaaaggaaga
aattccaggaaaatctgcacttgtggcttttattgccatgagctagaagcatcacaggttgaccaataaccatgcccaagaatg
agaagaatgactatgcaacctttggatgcactttatattatttgaatccagaaataatgaaataactaggcgtggacttacta
ttaattgctgaatgactaccaacagtgagagcccttcatgcatttgcactattggaaggagttagatgttggtactagatactg
aatgtaaacaaaggaattatggctggtaacatagttttagtctaattgaatcccttaaactcagggagcatttataaatggca
aatgcttatgaaactaagatttgtaatatttctctctttttagagaaatttgccaatttactttgttattttccccaaaaaga
atgggatgatcgtgtatttatttttttacttcctcagctgtagacaggtccttttcgatggtacatatttctttgcctttataa
tcttttatacagtgtcttacagagaaaagacataagcaaagactatgaggaatatttgcaagacatagaatagtgttggaaaat
gtgcaatatgtgatgtggcaaatctctattaggaaatattctgtaatcttcagacctagaataatactagtcttataataggtt
tgtgactttcctaaatcaattctattacgtgcaatacttcaatacttcatttaaaatatttttatgtgcaataaaatgtatttg
tttgtatttgtgttcagtacaattataagctgtttttatatatgtgaaataaaagtagaataaac
<212> Type : DNA
<211> Length : 1662
      SequenceName : L07555
      SequenceDescription :

Sequence
--------
<213> OrganismName : HUMAN
<400> PreSequenceString :
gatgctggggctggtcctggccttgctgtcctccagctctgctgaggagtacgtgggcctgtctgcaaaccagtgtgccgtgcc
ggccaaggacagggtggactgcggctacccccatgtcacccccaaggagtgcaacaaccggggctgctgctttgactccaggat
ccctggagtgccttggtgtttcaagcccctgactaggaagacagaatgcaccttctgaggcacctccagctgcccctgggatgc
aggctgagcacccttgcccggctgtgattgctgccaggcactgttcatctcagtttttctgtccctttgctcccggcaagcttt
ctgctgaaagttcatatctggagcctgatgtcttaacgaataaaggtcccatgctccacccg
<212> Type : DNA
<211> Length : 398
      SequenceName : L08044
      SequenceDescription :

Sequence
--------
<213> OrganismName : HUMAN
<400> PreSequenceString :
ggcgagatggcggccaccgagggggtcggggaggctgcgcaagggggcgagcccgggcagccggcgcaacccccgccccagccg
cacccaccgccgccccagcagcagcacaaggaagagatggcggccgaggctggggaagccgtggcgtcccccatggacgacggg
tttgtgagcctggactcgccctcctatgtcctgtacagggacagagcagaatgggctgatatagatccggtgccgcagaatgat
ggccccaatcccgtggtccagatcatttatagtgacaaatttagagatgtttatgattacttccgagctgtcctgcagcgtgat
gaaagaagtgaacgagcttttaagctaacccgggatgctattgagttaaatgcagccaattatacagtgtggcatttccggaga
gttcttttgaagtcacttcagaaggatctacatgaggaaatgaactacatcactgcaataattgaggagcagcccaaaaactat
caagtttggcatcataggcgagtattagtggaatggctaagagatccatctcaggagcttgaatttattgctgatattcttaat
caggatgcaaagaattatcatgcctggcagcatcgacaatgggttattcaggaatttaaactttgggataatgagctgcagtat
gtggaccaacttctgaaagaggatgtgagaaataactctgtctggaaccaaagatacttcgttatttctaacaccactggctac
aatgatcgtgctgtattggagagagaagtccaatacactctggaaatgattaaactagtaccacataatgaaagtgcatggaac
tatttgaaagggattttgcaggatcgtggtctttccaaatatcctaatctgttaaatcaattacttgatttacaaccaagtcat
agttcccctacctaattgcctttcttgtggatatctatgaagacatgctagaaaatcagtgtgacaataaggaagacattctt
aataaagcattagagttatgtgaaatcctagctaaagaaaaggacactataagaaaggaatattggagatacattggaagatcc
cttcaaagcaaacacagcacagaaaatgactcaccaacaaatgtacagcaataacaccatccagaagaacttgatggaatgctt
ttatttttattaagggaccctgcaggagtttcacacgagagtggtccttccctttgcctgtggtgtaaaagtgcatcacacag
gtattgctttttaacaagaactgatgctccttgggtgctgctgctactcagactagctctaagtaatgtgattcttctaaagca
aagtcattggatgggaggaggaagaaaaagtcccataaaggaacttttgtagtcttatcaacatataatctaatcccttagcat
cagctcctccctcagtggtacatgcgtcaagatttgtagcagtaataactgcaggtcacttgtatgtaatggatgtgaggtagc
cgaagtttggttcagtaagcagggaatacagtcgttccatcagagctggtctgcacactcacattatcttgctatcactgtaac
caactaatgccaaaagaacggttttgtaataaaattatagctgtatct
<212> Type : DNA
<211> Length : 1644
      SequenceName : L10413
      SequenceDescription :

Sequence
--------
<213> OrganismName : HUMAN
<400> PreSequenceString :
gaattcctgggctcagaggtcctcccaccttagccttctgagtagctaggactacagacaccagctaccacatgaggctttgta
gaaatggggtcttactatgttgcccaggctgatttgaactcctggtctcaagcaatctttccaccttagccttccaaagtgct
ggaattacaggagtgggccactgcacctggctctattaacatttttatttgctttatcacatatttatcaatccatctcactt
ttaaatatcttttaaaattacaaatatcagtacattttacatctaaacccttcagaagcttaacattgactggagttcagtatt
tatttccccatttcttttctggcctgaggaaggcaaattttacatacaaatctcaagtcagtactcttttttttttttgagacg
gagtcttgctctgttgcccaggctggagtccagtggtgtgatcttggctcactgcaacctctgccttctgggtacaagcgattc
tcctgtctcagcctcccaagtagctgggactacaggtttgtgccaccatatccagctaatttttgtatttttaatggagaaggg
gtttcaccatgttggccaggctggtctcaaactcttgacctcaagtgatccacctgccttggtctcctaaagtgctgggattat
aggtgtgagccatctcgcctggcctaatactgttttgtttgtttgtttttgtttttaagacagagtcttgttcttgtcacccag
gctggagtgcaatggcatgatttcggctcactgcaacttccgcctcctgggttcaagtgattctcctgcctcagcctcccaagt
agctggaattaaaggtgcctaccaccacgccccgctaatttttatatttttagtagagatggggtttcaccatgttgatcaggc
tgctctcgagctccttacctcagatgatccaccttccttggcctcccaaagtgctggtattataggcaagagccactgcgccca
gccccagtattcagtttttaaactgtcttgttatcaaggctctggagccagatgcctgggttcaaattctggttctgccactga
ctctgtgagctccataagtttcttaacctctctgtacctcagtttcctcttagggtttttgtcaggattataattattggctgg
gcatgatggctcatgcttgtaatcccagcactttaggaggccaacacgggcagatcacgtgagtccaggagtttgagcccagcc
tgggcaatgtggcaaaaatccatctctacaaaaaatgcaaaaattagctgggcatggtggcatgtgcctatagtcccagctatt
caggaggctgaggtaggtgaatccatagatcctgggaggtcaaggctgcagtgagccatgatcctgccattgcattccagtctg
ggtgacatagcgagaccctgtctcaaaaaaaaaaattattaaagtgtgtaaatcagtggcataaacatgttaagtgcattttgt
gggtcagctatattattattagtattacggaaacacatagagatgttaccaagaaggggagatgattggagccacttccagctt
ccttggacctggtctttcttcccttgactcttttttttttttttttttttttttgagagagagagtctcagcctgttgcccag
gctggagtgcaatggtgcaatcttggctcactgcaacctctgcctcccaggtttaagtgattctcctgcctcagcctcctgtgt
agctggaattacaggcgcgtgccaccacgcccggctaacttttgtatctttagtagagacagggtttcaccatgttggccagg
ctggtctcgaactcctgacctcaagtgatccacctgcctcagcctcccaaagtgttgggattacaggtgtaagccactaccccg
gctactcccttgactcttaaccactcatgctgcctacatctaccattcatgtggtccttgctgctttgttttggttattcctgc
atttatttgtccttttattcatttatgtataaacatttagtaagcacctactaatggatagggctcattgtagacttggaagct
ctctgagggtgggagtatgcctcgtccatctgtctttacttttgtagcaagggaggtaaagctccatttccatccctccttag
tgagtcagtagtcagtggtgaggctaaggcttacctctccctttctcactcagcacagggggctggagatgagcaagggaacgg
gaggaggtcagcccagtatgggaatcagttcttctcagggaacccagacatccatccctcaagattccagtccttgtcctagtc
cggcccttgacctcagagacgggatcagctcttcctccagcacctaccttgagggtatagaagaatgcaaaccacattggaaac
ctggagatctgtgttctcatttcagctctgctgactggcttcctgcaagctaccttccctccctgggcctcagtttctctctct

gctgagccagaagatgtctaaagacccctttggttccaccctgagagcctgtctccctaacctcaacttcttccccagttcaga
gaacccaggcatccagctgccccaccccagctctgggtaaacaggaagctgggtgaggggagcaggggtgtgcggaaagtccca
gccaggtgtgcaggtctacagggaggggggtgggcccgtccctgaggtatgaaagccccctgctctggctctggttcagtctcaa
tggggcactggggctggagggcaggggtgggaggctccaggggaggggttccctcctgctagctgtggcaggagccacttctc
tggtgaccttgttgctggcggtgcctatcactgtcctggctgtgctggccttagtgccccaggatcagggaggactggtgagtg
gctgcaacaggccctggtggagagttgtatcttgcggatgcttggctccctctggttgtgcctgtggtcttttgccccctctgg
ctcagctggctcggctgtccctggtggggatgtcttgtctctttgctgactctctttccatgttcctgtgatgttgtgcttgtg
tcccgacataagccccttgtgtctcctctccctcttcccgaggtacatctgtttctccgcccaagtacctatgccttgcttgttc
tcccttctaaggaggtgtgtgttggggatggtgctggtaggagaaaccccaggcctgcagcttgggtccactttcagaggggta
ggggtgacatgagctgaatctgaactctgggcactgtgaccccacccaaccaggtaacggagacggccgaccccggggcacagg
cccagcaaggactgggtaagagcagactgtctctccttccccgcttcagaccctcaggggctcccagctccctgctgcgtcccc
agatacctcttcctctaggaatccaggctccccatccctgcgccctgttctctcaagggtagcctgcatgggtggctgccctgc
ccccaatcgtggactctttgccccttccagggtttcagaagctgccagaggaggagccagaaacagatctcagccccgggctcc
cagctgcccacctcataggtaaggacctccaagacctgaataagagtgtaaataatccgaaggttccagttctgctcgcccaga
gtccttcggctccatgattccagtgctcggtttcccacccgcttcacgaccttttgtcgctcgtgcccactcttacgctcgtcc
ccgcagtgtagtttcttcttccctccggtgcaagcaaaagccggcctggaggtccccactacagcgttctgcaccccacatccg
tgttccctcggcccccaactcgcactcatcccagaaacagcaccatccctcctcccccggcccggctcggctcccgcaggggct
aaaagccgccacttccccagaagtcccaagcctttaggatcgcattcccaagagcgcgtcggcccgtgtctccgcaggcgctcc
gctgaaggggcaggggctaggctgggagacgacgaaggaacaggcgtttctgacgagcgggacgcagttctcggacgccgaggg
gctggcgctcccgcaggacggcctctattacctctactgtctcgtcggctaccggggccgggcgccccctggcggcggggaccc
ccagggccgctcggtcacgctgcgcagctctctgtaccgggcgggggcgcctacgggccgggcactcccgagctgctgctcga
gggcgccgagacggtgactccagtgctggacccggccaggagacaagggtacgggcctctctggtacacgagcgtggggttcgg
cggcctggtgcagctccggaggggcgagagggtgtacgtcaacatcagtcaccccgatatggtggacttcgcgagagggaagac
cttctttggggccgtgatggtggggtgagggaatatgagtgcgtggtgcgagtgcgtgaatattgggggcccggacgcccagga
ccccatggcagtgggaaaaatgtaggagactgtttggaaattgattttgaacctgatgaaaataaagaatggaaagcttcagtg
ctgccgataaagatgctgagttgcgacacacgtcttaattcagggtgggtgcacgggtgcgggttaaatattctcagtactctt
ctggttgcttgaaacaattcatcacaacacagtgtatggcctttgctcctagggatgatggtctgcctgtcccacccccctccct
gcctctgaatggccaggccccaccattagcccagttggagggtgggaggaaggggggacttctcaaactccgaagcttctctagg
catcctgattttcagggccacatggtcccaaccagactctgcaccatactcttttctcttgggtacccccccaacagtgagaggg
gtcattacagagcccagcaagcaccactcagaaaggcccagcagcagagtaagcccctatcatgacagaggaatgaagcctgga
ggggccccgcacttctcccccctagagctgcctgaaggcctctctgtctcctacccgacagtcaactcttctcctccaaggagct
taattcaaggctcatggggtctgaagggaggaggctgaaggagaaagaaggggagaatattagagagagatggggatggcagga
aggagcctgtggtgcctgaaaacaccaggaagttctggggaggaggaaaaaccgatgccccacttagggtgtcccatttagggt
gagacggaaaatcctcacctttttttcacactttaggtccccttcccaaaagtgagtaagtgtgggtgcttctgggatgagta
acagtgtcccccattacttcatggctgactttcagccacaggctggaggaggcagagggtgacccaaggccctatctaggtcac
cccaatgggtcaccctacccctcagcctaccacatggttttctcctgcctggcaccccagggctggaggtaaagcctaatttc
cgaactcagtgggggctcccagtctagggggggctcaatttccgtctccatatttgtttttggaattattatttttttgagacag
ggtctcgttctgtcacccagacgggggtacagtggcatgatcatagcttactgtaacctcaaactcctgggcttgagtgatcct
cctgcctcagcctcctgaggagctaggattacaggcatgcaccactacacctgactaatctttaatttttttctagaaacaag
gtcttgctatgttgcacaggctggtcttgaactagtgggctcaagtggtcctcccacctcagcctcccaaagtgttgggataac
aggcatgagccactgcgccccacccttatttgtctttgactctctccagaagagccttcatccagggaggggggtgcttttctct
ttccggattacccacctctcacctctcccctccttcaccacaaagaccagtgggaccaagccggcatgtgagtccttcacccac
atcttattcctatgtttcattctttttaaaaaatagagacaggatctcactatgttgcccaggttgctctggaactcctgggt
tcaagcgatcctctcaccttggccttgcaaagtggtaggattacaggtgcatgccaccacgtccggcagttcggttccttgttc
tttattgtcctcagtctcttcgatttcacccactgagagaatggaaggggatagaacagctggaaactggttgaaggaagccag
aattc
<212> Type : DNA
<211> Length : 6305
      SequenceName : L11016
      SequenceDescription :

Sequence
--------
<213> OrganismName : HUMAN
<400> PreSequenceString :
aattagagtagaagttgtcggggtccgctcttaggacgcagccgcctcatggggtccaggggctctggaagctgctggagtgc
tccgggcggcaggtcagccccgaagcgctggaagggaagatcctggctgttgatattagcatttggttaaaccaagcacttaaa
ggagtccgggatcgccacgggaactcaatagaaaatcctcatcttctcactttgtttcatcggctctgcaaactcttattttt
cgaattcgtcctattttgtgtttgatggggatgctccactattgaagaaacagactttggtgaagagaaggcagagaaaggac
ttagcgtccagtgactccaggaaaacgacagagaagcttctgaaaacattttgaaaagacaagccatcaaaactgccttcaga
agcaaaagagatgaagcactacccagtcttacccaagttcgaagagaaaacgacctctatgttttgcctcctttacaagaggaa
gaaaaacacagttcagaagaggaagatgaaaaagaatggcaagaaagaatgaatcaaaaacaagcattacaggaagagttcttt
cataatcctcaagcgatagatattgagtctgaggacttcagcagcctgcccccctgaagtaaagcatgaaatcttgactgatatg

aaagagttcaccaagcgcagaagaacattatttgaagcaatgccagaggagtctgatgacttttcacagtaccaactcaaaggc
ttgcttaaaaagaactatctgaaccagcatatagaacatgtccaaaaggaagtgaatcagcaacattcaggacacatccgaagg
cagtatgaagatgaaggggctttctgaaggaggtagagtcaaggagagtggtctctgaagacacttcacattacatcttgata
aaaggtattcaagctaagacagttgcagaagtggattcagagtctcttccttcttccagcaaaatgcacggcatgtcttttgac
gtgaagtcatctccatgtgaaaaactgaagacagagaaagagcctgatgctacccctccttctccaagaactttactagctatg
caagctgccctgctgggaagtagctcagaagaggagctggagagtgaaaatcgaaggcaggcccgtgggaggaacgcacctgct
gctgtagacgaaggctccatatcaccccggactctttcagccattaagagagctcttgacgatgacgaagatgtaaaagtgtgt
gctggggatgatgtgcagacgggagggccaggagcagaagaaatgcgtataaacagctccaccgagaacagtgatgaaggactt
aaagtgagagatggaaaaggaataccgtttactgcaacacttgcgtcatctagtgtgaactctgcagaggagcacgtagccagc
actaatgaggggagagagcccacagactcagttccaaaagaacaaatgtcacttgttcacgtggggactgaagcctttccgata
agtgatgagtctatgattaaggacagaaaagatcggctgcctctggagagtgcagtggttagacatagtgacgcacctgggctc
ccgaatggaagggaactgacaccggcatctccaacttgtacaaattctgtgtcaaagaatgaaacacatgctgaagtgcttgag
cagcagaacgaactttgcccatatgagagtaaattcgattcttctcttctttcaagtgatgatgaaacaaaatgtaaaccgaat
tctgcttctgaagtcattggccctgtcagtttgcaagaaacaagtagcatagtaagtgtcccttcagaggcagtagataatgtg
gaaaatgtggtgtcatttaatgctaaagagcatgagaattttctggaaaccatccaagaacagcagaccactgaatctgcaggc
caggatttaatttccattccaaaggccgtggaaccaatggaaattgactcggaagaaagtgaatctgatggaagtttcattgaa
gtgcaaagtgtgattagtgatgaggaacttcaagcagaattccctgaaacttccaaacctccctcagaacaaggcgaagaggaa
ctggtaggaactagggagggagaagcccctgctgagtccgagagcctcctgagggacaactctgagagggacgacgtggatggt
gagccacaggaagctgagaaagatgcggaagattcgctccatgaatggcaagatattaatttggaggagttggaaactctggag
agcaacctcttagcacagcagaattcactgaaagctcaaaaacagcagcaagaacggatcgctgctactgtcaccggacagatg
ttcctggaaagccaggaactcctgcgcctgttcggcattccctacatccaggctcccatggaagcagaggcgcagtgcgccatc
ctggacctgactgatcagacttccggaaccatcactgatgacagtgatatctggctgtttggagcgcggcatgtctatagaaac
tttttaataaaaataagtttgtagaatattatcaatatgtggactttcacaatcaattgggattggaccggaataagttaata
aatttggcttatttgcttggaagtgattataccgaaggaataccaactgtgggttgtgtaaccgccatggaaattctcaatgaa
ttccctgggcatggcctggaacctctcctaaaattctcagaatggtggcatgaagctcaaaaaaatccaaagataagacctaat
cctcatgacaccaaagtgaaaaaaaaattacggacattgcaactcacccctggctttcctaacccagctgttgccgaggcctac
ctcaaacccgtggtggatgactcgaagggatcctttctgtgggggaaacctgatctcgacaaaattagagaattttgtcagcgg
tatttcggctggaacagaacgaagacagatgaatctctgtttcctgtattaaagcaactcgatgcccagcagacacagctccga
attgattccttctttagattagcacaacaggagaaagaagatgctaaacgtattaagagccagagactaaacagagctgtgaca
tgtatgctaaggaaagagaaagaagcagcagccagcgaaatagaagcagtttctgttgccatggagaaagaatttgagctactt
gataaggcaaaacgaaaaacccagaagagaggcataacaaataccttagaagagtcatcaagcctgaaaagaaagaggctttca
gattctaaacgaaagaatacatgcggtggatttttgggggagacctgcctctcagaatcatctgatggatcttcaagtgaagat
gctgaaagttcatctttaatgaatgtacaaaggagaacagctgcgaaagagccaaaaaccagtgcttcagattcgcagaactca
gtgaaggaagctcccgtgaagaatggaggtgcgaccaccagcagctctagtgatagtgatgacgatggagggaaagagaagatg
gtcctcgtgaccgccagatctgtgtttgggaagaaaagaaggaaactaagacgtgcgaggggaagaaaaaggaaaacctaatta
aaaaatatgtatcctctataattagttatgacagccatttgtaatgaatttgtcgcaaagacgtaataaaattaactggtagca
cggtca
<212> Type : DNA
<211> Length : 3702
      SequenceName : L20046
      SequenceDescription :

Sequence
--------
<213> OrganismName : HUMAN
<400> PreSequenceString :
attgcggcggcgccagagctgctggagcgctcggggtccccgggcggcggcggcggcgcagaggaggaggcaggcggcggcccc
ggtggctccccccggacggtgcgcggcccggcccgtctcgcgaactcgcggtggtcgcgcggccccgcgctgctccgaccccg
ggcccctccgccgccgccatggctcggccgctagtgcccagctcgcagaaggcgctgctgctggagctcaaggggctgcaggaa
gagccggtcgagggattccgcgtgacactggtggacgagggcgatctatacaactgggaggtggccattttcgggcccccaac
acctactacgagggcggctacttcaaggcgcgcctcaagttccccatcgactacccatactctccaccagcctttcggttcctg
accaagatgtggcaccctaacatctacgagacgggggacgtgtgtatctccatcctccacccgccggtggacgacccccagagc
ggggagctgccctcagagaggtggaaccccacgcagaacgtcaggaccattctcctgagtgtgatctccctcctgaacgagccc
aacaccttctcgcccgcaaacgtggacgcctccgtgatgtacaggaagtggaaagagagcaaggggaaggatcgggagtacaca
gacatcatccggaagcaggtcctggggaccaaggtggacgcggagcgtgacggcgtgaaggtgcccaccacgctggccgagtac
tgcgtgaagaccaaggcgccggcgcccgacgagggctcagacctcttctacgacgactactacgaggacggcgaggtggaggag
gaggccgacagctgcttcggggacgatgaggatgactctggcacggaggagtcctgacaccaccagaataaacttgccgagttt
acctcactagggccggacccgtggctccttagacgacagactacctcacggaggttttgtgctggtccccgtctcctctggttg
tttcgttttggctttttctccctccccatgtctgttctgggttttcacgtgcttcagagaagaggggctgccccaccgccactc
acgtcactcggggctcggtggacgggcccagggtgggagcggccggcccacctgtcccctcgggaggggagctgagcccgactt
ctaccggggtcccccagcttccggactggccgcaccccggaggagccacgggggcgctgctgggaacgtgggcgggggccgtt
tcctgacactaccagcctgggaggcccaggtgtagcggtccgagggcccggtcctgcctgtcagctccaggtcctggagccac
gtccagcactgagtggacggattcaccaat

<212> Type : DNA
<211> Length : 1374
      SequenceName : L22005
      SequenceDescription :

Sequence
--------
<213> OrganismName : HUMAN
<400> PreSequenceString :
atggacgtgttcctcatgatccggcgccacaagaccaccatcttcacggacgccaaggagtccagcacggtgttcgaactgaag
cgcatcgtcgagggcatcctcaagcggcctcctgacgagcagcggctgtacaaggatgaccaactcttggatgatggcaagaca
ctgggcgagtgtggcttcaccagtcaaacagcacggccacaggccccagccacagtggggctggccttccggcagatgacacc
tttgaggccctgtgcatcgagccgttttccagcccgccagagctgcccgatgtgatgaagccccaggactcgggaagcagtgcc
aatgaacaagccgtgcagtga
<212> Type : DNA
<211> Length : 357
      SequenceName : L42856
      SequenceDescription :

Sequence
--------
<213> OrganismName : HUMAN
<400> PreSequenceString :
gccagggttgcctgcgggagccaggcgtccgctctccacacctttcacagccccagccctcagagcaacctcagcccagcccag
cccagctccagctccagctccagcccgggccccatcatggccaaggactttcaagatatccagcagctgagctcggaggaaaat
gaccatcctttccatcaaggtgaggggccaggcactcgcaggctgaatcccaggagaggaaatccattttgaaagggccacct
cctgcccagcccctggcacagcgtctctgctccatggtctgcttcagtctgcttgccctgagcttcaacatcctgctgctggtg
gtcatctgtgtgactgggtcccaaagtgagggtcacagaggtgcacagctgcaagccgagctgcggagcctgaaggaagctttc
agcaacttctcctcgagcaccctgacggaggtccaggcaatcagcacccacggaggcagcgtgggtgacaagatcacatcccta
ggagccaagctggagaaacagcagcaggacctgaaagcagatcacgatgccctgctcttccatctgaagcacttccccgtggac
ctgcgcttcgtggcctgccagatggagctcctccacagcaacggctcccaaaggacctgctgccccgtcaactgggtggagcac
caaggcagctgctactggttctctcactccgggaaggcctgggctgaggcggagaagtactgccagctggagaacgcacacctg
gtggtcatcaactcctgggaggagcagaaattcattgtacaacacacgaaccccttcaatacctggataggtctcacggacagt
gatggctcttggaaatgggtggatggcacagactataggcacaactacaagaactgggctgtcactcagccagataattggcac
gggcacgagctgggtggaagtgaagactgtgttgaagtccagccggatggccgctggaacgatgacttctgcctgcaggtgtac
cgctgggtgtgtgagaaaaggcggaatgccaccggcgaggtggcctgaccccagcacacctctggctaacccataccccacacc
tgcccagctctggcttctctgttgaggattttgaggaaaggaagaaacactgagacaggggtatggggaagagctgagcaaaga
gagaaaggaggtagtttaagagtccctgaccctggaggactgagatcccacctccttctgtaattcattgtaattattataatc
gtcagcctcttcaatggcgtaggaaagaagaaacaaatgcttgaatctc
<212> Type : DNA
<211> Length : 1309
      SequenceName : M11025
      SequenceDescription :

Sequence
--------
<213> OrganismName : HUMAN
<400> PreSequenceString :
gttggccccccgttacttttcctctgggaaatatggcgcacgctgggagaacagggtacgataaccgggagatagtgatgaagta
catccattataagctgtcgcagaggggctacgagtgggatgcgggagatgtgggcgccgcgcccccgggggccgcccccgcgcc
gggcatcttctcctcgcagcccgggcacacgccccatacagccgcatcccgggacccggtcgccaggacctcgccgctgcagac
cccggctgccccggcgccgccgcggggcctgcgctcagcccggtgccacctgtggtccacctgaccctccgccaggccggcga
cgacttctcccgccgctaccgccgcgacttcgccgagatgtccaggcagctgcacctgacgcccttcaccgcgcggggacgctt
tgccacggtggtggaggagctcttcagggacggggtgaactgggggaggattgtggccttctttgagttcggtggggtcatgtg
tgtggagagcgtcaaccgggagatgtcgcccctggtggacaacatcgccctgtggatgactgagtacctgaaccggcacctgca
cacctggatccaggataacggaggctgggatgcctttgtggaactgtacggccccagcatgcggcctctgtttgatttctcctg
gctgtctctgaagactctgctcagtttggccctggtgggagcttgcatcaccctgggtgcctatctgggccacaagtgaagtca
acatgcctgccccaaacaaatatgcaaaaggttcactaaagcagtagaaataatatgcattgtcagtgatgttccatgaaacaa
agctgcaggctgtttaagaaaaaataacacacatataaacatcacacacacagacagacacacacacacacaacaattaacagt
cttcaggcaaaacgtcgaatcagctatttactgccaaagggaaatatcatttatttttacattattaagaaaaaagatttat
ttatttaagacagtcccatcaaaactcctgtctttggaaatccgaccactaattgccaagcaccgcttcgtgtggctccacctg
gatgttctgtgcctgtaaacatagattcgctttccatgttgttggccggatcaccatctgaagagcagacggatggaaaaagga
cctgatcattggggaagctggctttctggctgctggaggctggggagaaggtgttcattcacttgcatttctttgccctgggg

ctgtgatattaacagagggagggttcctgtgggggaagtccatgcctccctggcctgaagaagagactctttgcatatgactc
acatgatgcatacctggtgggaggaaaagagttgggaacttcagatggacctagtacccactgagatttccacgccgaaggaca
gcgatgggaaaaatgcccttaaatcataggaaagtatttttttaagctaccaattgtgccgagaaaagcattttagcaatttat
acaatatcatccagtaccttaagccctgattgtgtatattcatatatttggatacgcacccccaactcccaatactggctct
gtctgagtaagaaacagaatcctctggaacttgaggaagtgaacatttcggtgacttccgcatcaggaaggctagagttaccca
gagcatcaggccgccacaagtgcctgcttttaggagaccgaagtccgcagaacctgcctgtgtcccagcttggaggcctggtcc
tggaactgagccggggccctcactggcctcctccagggatgatcaacagggcagtgtggtctccgaatgtctggaagctgatgg
agctcagaattccactgtcaagaaagagcagtagaggggtgtggctgggcctgtcaccctggggccctccaggtaggcccgttt
tcacgtggagcatgggagccacgacccttcttaagacatgtatcactgtagagggaaggaacagaggccctgggcccttcctat
cagaaggacatggtgaaggctgggaacgtgaggagaggcaatggccacggcccattttggctgtagcacatggcacgttggctg
tgtggccttggcccacctgtgagtttaaagcaaggctttaaatgactttggagagggtcacaaatcctaaaagaagcattgaag
tgaggtgtcatggattaattgacccctgtctatggaattacatgtaaaacattatcttgtcactgtagtttggttttatttgaa
aacctgacaaaaaaaaagttccaggtgtggaatatggggggttatctgtacatcctggggcattaaaaaaaaatcaatggtggg
gaactataaagaagtaacaaaagaagtgacatcttcagcaaataaactaggaaatttttttttcttccagtttagaatcagcct
tgaaacattgatggaataactctgtggcattattgcattatataccatttatctgtattaactttggaatgtactctgttcaat
gtttaatgctgtggttgatatttcgaaagctgctttaaaaaaatacatgcatctcagcgtttttttgttttaattgtatttag
ttatggcctatacactatttgtgagcaaaggtgatcgttttctgtttgagatttttatctcttgattcttcaaaagcattctga
gaaggtgagataagccctgagtctcagctacctaagaaaaacctggatgtcactggccactgaggagctttgtttcaaccaagt
catgtgcatttccacgtcaacagaattgtttattgtgacagttatatctgttgtccctttgaccttgtttcttgaaggtttcct
cgtccctgggcaattccgcatttaattcatggtattcaggattacatgcatgtttggttaaacccatgagattcattcagttaa
aaatccagatggcaaatgaccagcagattcaaatctatggtggtttgacctttagagagttgctttacgtggcctgtttcaaca
cagacccacccagagccctcctgccctccttccgcggggggctttctcatggctgtccttcagggtcttcctgaaatgcagtggt
gcttacgctccaccaagaaagcaggaaacctgtggtatgaagccagacctccccggcgggcctcagggaacagaatgatcagac
ctttgaatgattctaattttaagcaaaatattattttatgaaaggtttacattgtcaaagtgatgaatatggaatatccaatc
ctgtgctgctatcctgccaaaatcattttaatggagtcagtttgcagtatgctccacgtggtaagatcctccaagctgctttag
aagtaacaatgaagaacgtggacgcttttaatataaagcctgttttgtcttctgttgttgttcaaacggattcacagagtatt
tgaaaaatgtatatatattaagaggtcacggggctaattgctggctggctgccttttgctgtggggtttgttacctggtttt
aataacagtaaatgtgcccagcctcttggccccagaactgtacagtattgtggctgcacttgctctaagagtagttgatgttgc
attttccttattgttaaaaacatgttagaagcaatgaatgtatataaaagcctcaactagtcatttttttctcctcttctttt
tttcattatatctaattattttgcagttgggcaacagagaaccatccctattttgtattgaagagggattcacatctgcatctt
aactgctctttatgaatgaaaaaacagtcctctgtatgtactcctctttacactggccagggtcagagttaaatagagtatatg
cactttccaaattggggacaagggctctaaaaaaagccccaaaggagaagaacatctgagaacctcctcggccctcccagtcc
ctcgctgcacaaatactccgcaagagaggccagaatgacagctgacagggtctatggccatcgggtcgtctccgaagatttggc
aggggcagaaaactctggcaggcttaagatttggaataaagtcacagaatcaaggaagcacctcaatttagttcaaacaagacg
ccaacattctctccacagctcacttacctctctgtgttcagatgtggccttccatttatatgtgatctttgttttattagtaaa
tgcttatcatctaaagatgtagctctggcccagtgggaaaaattaggaagtgattataaatcgagaggagttataataatcaag
attaaatgtaaataatcagggcaatcccaacacatgtctagctttcacctccaggatctattgagtgaacagaattgcaaatag
tctctatttgtaattgaacttatcctaaaacaaatagtttataaatgtgaacttaaactctaattaattccaactgtacttta
aggcagtggctgtttttagactttcttatcacttatagttagtaatgtacacctactctatcagagaaaaacaggaaaggctcg
aaatacaagccattctaaggaaattagggagtcagttgaaattctattctgatcttattctgtggtgtcttttgcagcccagac
aaatgtggttacacacttttaagaaatacaattctacattgtcaagcttatgaaggttccaatcagatctttattgttattca
atttggatctttcagggatttttttttaaattattatgggacaaaggacatttgttggaggggtgggagggaggaacaatttt
taaatataaaacattcccaagtttggatcagggagttggaagttttcagaataaccagaactaagggtatgaaggacctgtatt
ggggtcgatgtgatgcctctgcgaagaaccttgtgtgacaaatgagaaacattttgaagtttgtggtacgacctttagattcca
gagacatcagcatggctcaaagtgcagctccgtttggcagtgcaatggtataaatttcaagctggatatgtctaatgggtattt
aaacaataaatgtgcagttttaactaacaggatatttaatgacaaccttctggttggtagggacatctgtttctaaatgtttat
tatgtacaatacagaaaaaaatttataaaattaagcaatgtgaaactgaattggagagtgataatacaagtcctttagtctta
cccagtgaatcattctgttccatgtctttggacaaccatgaccttggacaatcatgaaatatgcatctcactggatgcaaagaa
aatcagatggagcatgaatggtactgtaccggttcatctggactgccccagaaaaataacttcaagcaaacatcctatcaacaa
caaggttgttctgcataccaagctgagcacagaagatgggaacactggtggaggatggaaaggctcgctcaatcaagaaaattc
tgagactattaataaataagactgtagtgtagatactgagtaaatccatgcacctaaacccttttggaaaatctgccgtgggccc
tccagatagctcatttcattaagtttttccctccaaggtagaatttgcaagagtgacagtggattgcatttcttttggggaagc
tttcttttggtggttttgtttattataccttcttaagttttcaaccaaggtttgcttttgttttgagttactggggttattttt
gttttaaataaaaataagtgtacaataagtgttttgtattgaaagcttttgttatcaagattttcatactttttaccttccatg
gctctttttaagattgatacttttaagaggtggctgatattctgcaacactgtacacataaaaaatacggtaaggatactttac
atggttaaggtaaagtaagtctccagttggccaccattagctataatggcactttgtttgtgttgttggaaaagtcacattgc
cattaaactttccttgtctgtctagttaatattgtgaagaaaaataaagtacagtgtgagatactg
<212> Type : DNA
<211> Length : 6030
      SequenceName : M14745
      SequenceDescription :

Sequence

--------
<213> OrganismName : HUMAN
<400> PreSequenceString :
ttccggtttttctcaggggacgttgaaattattttgtaacgggagtcgggagaggacggggcgtgccccgcgtgcgcgcgcgt
cgtcctccccggcgctcctccacagctcgctggctcccgccgcggaaaggcgtcatgccgcccaaaacccccgaaaaacggcc
gccaccgccgccgctgccgccgcggaaccccggcaccgccgccgccgcccctcctgaggaggacccagagcaggacagcggc
ccggaggacctgcctctcgtcaggcttgagtttgaagaaacagaagaacctgatttactgcattatgtcagaaattaaagata
ccagatcatgtcagagagagagcttggttaacttgggagaaagtttcatctgtggatggagtattgggaggttatattcaaaag
aaaaaggaactgtggggaatctgtatctttattgcacgagttgacctagatgagatgtcgttcactttactgagctacagaaaa
acatacgaaatcagtgtccataaattctttaacttactaaaagaaattgataccagtaccaaagttgataatgctatgtcaaga
ctgttgaagaagtatgatgtattgtttgcactcttcagcaaattggaaaggacatgtgaacttatatatttgacacaacccagc
agttcgatatctactgaaataaattctgcattggtgctaaaagtttcttggatcacattttttattagctaaaggggaagtatta
caaatggaagatgatctggtgatttcatttcagttaatgctatgtgtccttgactattttattaaactctcacctcccatgttg
ctcaaagaaccatataaaacagctgttatacccattaatggttcacctcgaacacccaggcgaggtcagaacaggagtgcacgg
atagcaaaacaactagaaaatgatacaagaattattgaagttctctgtaaagaacatgaatgtaatatagatgaggtgaaaaat
gtttatttcaaaaattttatacctttatgaattctcttggacttgtaacatctaatggacttccagaggttgaaaatctttct
aaacgatacgaagaaatttatcttaaaaataaagatctagatcgaagattatttttggatcatgataaaactcttcagactgat
tctatagacagttttgaaacacagagaacaccacgaaaaagtaaccttgatgaagaggtgaatataattcctccacacactcca
gttaggactgttatgaacactatccaacaattaatgatgattttaaattctgcaagtgatcaaccttcagaaaatctgatttcc
tattttaacaactgcacagtgaatccaaaagaaagtatactgaaaagagtgaaggatataggatacatctttaaagagaaattt
gctaaagctgtgggacagggttgtgtcgaaattggatcacagcgatacaaacttggagttcgcttgtattaccgagtaatggaa
tccatgcttaaatcagaagaagaacgattatccattcaaaattttagcaaacttctgaatgacaacattttttcatatgtcttta
ttggcgtgcgctcttgaggttgtaatggccacatatagcagaagtacatctcagaatcttgattctggaacagatttgtctttc
ccatggattctgaatgtgcttaatttaaaagcctttgattttacaaagtgatcgaaagttttatcaaagcagaaggcaacttg
acaagagaaatgataaaacatttagaacgatgtgaacatcgaatcatggaatcccttgcatggctctcagattcacctttattt
gatcttattaaacaatcaaaggaccgagaaggaccaactgatcaccttgaatctgcttgtcctcttaatcttcctctccagaat
aatcacactgcagcagatatgtatctttctcctgtaagatctccaaagaaaaaaggttcaactacgcgtgtaaattctactgca
aatgcagagacacaagcaacctcagccttccagacccagaagccattgaaatctacctctctttcactgttttataaaaaagtg
tatcggctagcctatctccggctaaatacactttgtgaacgccttctgtctgagcacccagaattagaacatatcatctggacc
cttttccagcacaccctgcagaatgagtatgaactcatgagagacaggcatttggaccaaattatgatgtgttccatgtatggc
atatgcaaagtgaagaatatagaccttaaattcaaaatcattgtaacagcatacaaggatcttcctcatgctgttcaggagaca
ttcaaacgtgttttgatcaaagaagaggagtatgattctattatagtattctataactcggtcttcatgcagagactgaaaaca
aatattttgcagtatgcttccaccaggccccctaccttgtcaccaatacctcacattcctcgaagcccttacaagtttcctagt
tcacccttacggattcctggagggaacatctatatttcacccctgaagagtccatataaaatttcagaaggtctgccaacacca
acaaaaatgactccaagatcaagaatcttagtatcaattggtgaatcattcgggacttctgagaagttccagaaaataaatcag
atggtatgtaacagcgaccgtgtgctcaaaagaagtgctgaaggaagcaaccctcctaaaccactgaaaaaactacgctttgat
attgaaggatcagatgaagcagatggaagtaaacatctcccaggagagtccaaatttcagcagaaactggcagaaatgacttct
actcgaacacgaatgcaaaagcagaaaatgaatgatagcatggatacctcaaacaaggaagagaaatgaggatctcaggacctt
ggtggacactgtgtacacctctggattcattgtctctcacagatgtgactgtataactttcccaggttctgtttatggccacat
ttaatatcttcagctctttttgtggatataaaatgtgcagatgcaattgtttgggtgagtcctaagccacttgaaatgttagtc
attgttatttatacaagattgaaaatcttgtgtaaatcctgccatttaaaaagttgtagcagattgtttcctcttccaaagtaa
aattgctgtgctttatggatagtaagaatggccctagagtgggagtcctgataacccaggcctgtctgactactttgccttctt
ttgtagcatataggtgatgtttgctcttgtttttattaatttatatgtatatttttttaatttaacatgaacacccttagaaaa
tgtgtcctatctatcttccaaatgcaatttgattgactgcccattcaccaaaattatcctgaactcttctgcaaaaatggatat
tattagaaattagaaaaaaattactaattttacacattagattttattttactattggaatctgatatactgtgtgcttgtttt
ataaaattttgcttttaattaaataaaagctggaagcaaagtataaccatatgatactatcatactactgaaacagatttcata
cctcagaatgtaaaagaacttactgattattttcttcatccaacttatgtttttaaatgaggattattgatagtactcttggtt
tttataccattcagatcactgaatttataaagtacccatctagtacttgaaaaagtaaagtgttctgccagatcttaggtatag
aggaccctaacacagtatatcccaagtgcactttctaatgtttctgggtcctgaagaattaagatacaaattaattttactcca
taaacagactgttaattataggagccttaatttttttttcatagagatttgtctaattgcatctcaaaattattctgccctcct
taatttgggaaggtttgtgttttctctggaatggtacatgtcttccatgtatcttttgaactggcaattgtctatttatctttt
atttttttaagtcagtatggtctaacactggcatgttcaaagccacattatttctagtccaaaattacaagtaatcaagggtca
ttatgggttaggcattaatgtttctatctgattttgtgcaaaagcttcaaattaaaacagctgcattagaaaaagaggcgcttc
tcccctcccctacacctaaaggtgtatttaaactatcttgtgtgattaacttatttagagatgctgtaacttaaaataggggat
atttaaggtagcttcagctagcttttaggaaaatcactttgtctaactcagaattatttttaaaaagaaatctggtcttgttag
aaaacaaaatttttatttgtgctcatttaagtttcaaacttactattttgacagttattttgataacaatgacactagaaaact
tgactccatttcatcattgtttctgcatgaatatcatacaaatcagttagttttaggtcaagggcttactatttctgggtctt
ttgctactaagttcacattagaattagtgccagaatttaggaacttcagagatcgtgtattgagatttcttaaataatgcttc
agatattattgctttattgctttttgtattggttaaaactgtacatttaaaattgctatgttactatttctacaattaatag
tttgtctattttaaataaattagttgttaagagtc
<212> Type : DNA
<211> Length : 4740
      SequenceName : M15400

SequenceDescription :

Sequence
--------
<213> OrganismName : HUMAN
<400> PreSequenceString :
aaagtatgtgcatgtataaatctgtgtgtcttccactttgtcccacatatactaaatttaaacattcttctaacgtgggaaaat
ccagtattttaatgtggacatcaactgcacaacgattgtcaggaaaacaatgcatatttgcactggtgatacatttgcaaaatc
tgtcatagtttgctactccttgcccttccatgaaccagagaattatctcagtttattagtcccctcccctaagaagcttccacc
aatactcttttcccctttcctttaacttgattgtgaaatcaggtattcaacagagaaatttctcagcctcctacttctgctttt
gaaagccataaaaacagcgagggagaaactggcagataccaaacctcttcgaggcacaaggcacaacaggctgctctgggattc
tcttcagccaatcttcattgctcaagtatgactttaatcttccttacaactaggtgctaagggagtctctctgtctctctgcct
ctttgtgtgtatgcatattctctctctctctctctttctttctctgtctctccctctccttccctctctgcctccctctctcag
ctttttgcaaaaagccaggtgtaatataatgcttatgactcgggaaatattctgggaatggatactgcttatctaacagctgac
accctaaaggttagtgtcaaagcctctgctccagctctcctagcctaatacattgctagttggggtttggtttagcaaatgctt
ttctctagacccaaaggacttctctttcacacattcattcatttactcagagatcatttctttgcatgactgccatgcactgga
tgctgagagaaatcacacatgaacgtagccgtcatggggaagtcactcattttctcctttttacacaggtgtctgaagcagcca
tggcagaagtacctgagctcgccagtgaaatgatggcttattacaggtcagtggagacgctgagaccagtaacatgagcaggtc
tcctctttcaagagtagagtgttatctgtgcttggagaccagatttttcccctaaattgcctctttcagtggcaaacagggtgc
caagtaaatctgatttaaagactactttcccattacaagtccctccagccttgggacctggaggctatccagatgtgttgttgc
aagggcttcctgcagaggcaaatggggagaaaagactccaagcccacaatacaaggaatccctttgcaaagtgtggcttggagg
gagagggagagctcagattttagctgactctgctgggctagaggttaggcctcaagatccaacagggagcncccagggtgccca
cctgccaggcctagaatctgccttctggactgttctgcgcatatcactgtgaaacttgccaggtgtttcaggcagctttgagag
gcaggctgtttgcagtttcttatgaacagtcaagtcttgtacacagggaaggaaaaataaacctgtttagaagacataattgag
acatgtccctgtttttattacagtggcaatgaggatgacttgttctttgaagctgatggccctaaacagatgaaggtaagacta
tgggtttaactcccaacccaaggaagggctctaacacagggaaagctcaaagaagggagttctgggccactttgatgccatggt
attttgttttagaaagactttaacctcttccagtgagacacaggctgcaccacttgctgacctggccacttggtcatcatatca
ccacagtcactcactaacgttggtggtggtggccacacttggtggtgacaggggaggagtagtgataatgtttcccatttcata
gtaggaagacaaccaagtcttcaacataaatttgattatccttttaagagatggattcagcctatgccaatcacttgagttaaa
ctctgaaaccaagagatgatcttgagaactaacatatgtctaccccttttgagtagaatagtttttttgctacctggggtgaagc
ttataacaacaagacatagatgatataaacaaaaagatcaattgagacttgaaagaaaaccattcacttgctgtttgaccttga
caagtcattttacccgctttggacctcatctgaaaaataaagggctgagctggatgatctctgagattccagcatcctgcaacc
tccagttctgaaatattttcagttgtagctaagggcatttgggcagcaaatggtcatttttcagactcatccttacaaagagcc
atgttatattcctgctgtcccttctgttttatatgatggctcagtagccttcctagtggcccagccatcagcctagctaggtca
gttgtgcaggttggaggcagccacttttctctggctttatttattccagtttgtgatagcctcccctagcctcataatccagt
cctcaatcttgttaaaaacatatttctttagaagttttaagactggcataacttgttggctgcagctgtgggaggagcccattg
gcttgtctgcctggcctttgccccattgcctcttccagcagcttggcgctgctccaggcaggaaattctctcctgctcaactt
tcttttgtgcacttacaggtctctttaactgtctttcaagcctttgaaccattatcactgccttaaggcaacctcagtgaagcc
ttaatacggagcttctctgaataagaggaaagtggtaacatttcacaaaaagtactctcacaggatttgcagaatgcctatgag
acagtgttatgaaaaaggaaaaaaaagaacagtgtagaaaaattgaatacttgctgagtgagcataggtgaatggaaaatgtta
tggtcatctgcatgaaaaagcaaatcatagtgtgacagcattagggatacaaaaagatatagagaaggtatacatgtatggtgt
aggtggggcatgtacaaaaaagatgaacaaagtagaaatgggatttattctaaagaatagcctgtaaggtgtcagaaagcccac
attctagtcttgagtctgtttctaacctgctgtgtgcccttgagtacacacttaacctcttgagcttcagagagggataatctt
tttattttattttattttattttgttttgttttgttttgttttgttttatgagacagagtctcactctgttgcccaggctggag
tgcagtggtacaatcttggcttactgcatcctccacctcctgagttcaagcgattctccttcctcagtctcctgaatagctagg
attacaggtgcaccccaccacacccagctaattttgtatttttagtagagaaggggtttcgccatgttggccaggctggtttt
gaagtcctgacctaaatgattcatccacctcggcttcccaaagtgctgggattacaggcatgagccaccacgcctggcccagag
agggatgatctttagaagctcgggattctttcaagccctttcctcctctctgagctttctactctctgatgtcaaagcatggtt
cctggcaggaccacctcaccaggctccctccctcgctctctccgcagtgctccttccaggacctggacctctgccctctggatg
gcggcatccagctacgaatctccgaccaccactacagcaagggcttcaggcaggccgcgtcagttgttgtggccatggacaagc
tgaggaagatgctggttccctgcccacagaccttccaggagaatgacctgagcaccttctttcccttcatctttgaagaaggta
gttagccaagagcaggcagtagatctccacttgtgtcctcttggaagtcatcaagccccagccaactcaattcccccagagcca
aagccctttaaaggtagaaggcccagcggggagacaaaacaaagaaggctggaaaccaaagcaatcatctctttagtggaaact
attcttaaagaagatcttgatggctactgacatttgcaactccctcactctttctcaggggcctttcacttacattgtcaccag
aggttcgtaacctccctgtgggctagtgttatgaccatcaccatttacctaagtagctctgttgctcggccacagtgagcagt
aatagacctgaagctggaacccatgtctaatagtgtcaggtccatgttcttagccaccccactcccagcttcatccctactggt
gttgtcatcagactttgaccgtatatgctcagtgtcctccaagaaatcaaatttgccgcctcgcctcacgaggcctgccccttc
tgattttatacctaaacaacatgtgctccacatttcagaacctatcttcttcgacacatgggataacgaggcttatgtgcacga
tgcacctgtacgatcactgaactgcacgctccgggactcacagcaaaaaagcttggtgatgtctggtccatatgaactgaaagc
tctccacctccagggacaggatatggagcaacaaggtaaatggaaacatcctggtttccctgcctggcctcctggcagcttgct
aattctccatgttttaaacaaagtagaaagttaatttaaggcaaatgatcaacacaagtgaaaaaaaatattaaaaaggaatat
acaaactttggtcctagaaatggcacatttgattgcactggccagtgcatttgttaacaggagtgtgaccctgagaaattagac
ggtcaagcactcccaggaccatgtccacccaagtctcttgggcatagtgcaatgtcaattcttccacaatatcccctcatttga

tggacatggcctaactgcctgtgggttctctcttcctgttgttgaggctgaaacaagagtgctggagcgataatgtgtccatcc
cctccccagtcttccccccttgccccaacagtccgtcccacccaatgcaggtggttcttgtagggaaatttaccgcccagcag
gaacttatatctctccgctgtaacgggcaaaagtttcaagtgcggtgaacccatcattagctgtggtgatctgcctggcatcgt
gccacagtagccaaagcctctgcacaggagtgtgggcaactaaggctgctgactttgaaggacagcctcactcagggggaagct
atttgctctcagccaggccaagaaaatcctgtttctttggaatcgggtagtaagagtgatcccagggcctccaattgacactgc
tgtgactgaggaagatcaaaatgagtgtctctctttggagccactttcccagctcagcctctcctctcccagtttcttcccatg
ggctactctctgttcctgaaacagttctggtgcctgatttctggcagaagtacagcttcacctctttcctttccttccacattg
atcaagttgttccgctcctgtggatgggcacattgccagccagtgacacaatggcttccttccttccttccttcagcatttaaa
atgtagaccctctttcattctccgttcctactgctatgaggctctgagaaacctcaggcctttgaggggaaaccctaaatcaac
aaaatgaccctgctattgtctgtgagaagtcaagttatcctgtgtcttaggccaaggaacctcactgtgggttcccacagaggc
taccaaattacatgtatcctactcatggggcctaggggttggggtgaccctgcactgctgtgtccctaaccacaagaccccctt
ctttcttcagtggtgttctccatgtcctttgtacaaggagaagaaagtaatgacaaaatacctgtggccttgggcctcaaggaa
aagaatctgtacctgtcctgcgtgttgaaagatgataagcccactctacagctggaggtaagtgaatgctatggaatgaagccc
ttctcagcctcctgctaccacttattcccagacaaccaccttctccccgcccccatccctaggaaaagctgggaacaggtctat
ttgacaattttgcattaatgtaaataaatttaacataatttttaactgcgtgcaaccttcaatcctgctgcagaaaattaaatc
attttgccgatgttattatgtcctaccatagttacaaccccaacagattatatattgttagggctgctctcatttgatagacac
cttgggaaatagatgacttaaagggtcccattatcacgtccactccactcccaaaatcaccaccactatcacctccagctttct
cagcaaaagcttcatttccaagttgatgtcattctaggaccataaggaaaaatacaataaaaagcccctggaaactaggtactt
caagaagctctagcttaattttcacccccaaaaaaaaaaattctcacctacattatgctcctcagcatttggcactaagttt
tagaaaagaagagggctcttttaaataaattcacacagaaagttgggcccagttacaactcaggagtctggctcctgatcatgt
gacctgctcgtcagtttcctttctggccaacccaaagaacatctttcccatagcatctttgtcccttgccccacaaaaattctt
ctttctctttcgtgcagagtgtagatcccaaaaattacccaaagaagaagatggaaaagcgatttgtcttcaacaagatagaaa
tcaataacaagctggaatttgagtctgcccagttccccaactggtacatcagcacctctcaagcagaaaacatgcccgtcttcc
tgggagggaccaaaggcggccaggatataactgacttcaccatgcaatttgtgtcttcctaaagagagctgtacccagagagtc
ctgtgctgaatgtggactcaatccctagggctggcagaaagggaacagaaaggtttttgagtacggctatagcctggactttcc
tgttgtctacaccaatgcccaactgcctgccttagggtagtgctaagaggatctcctgtccatcagccaggacagtcagctctc
tcctttcagggccaatccccagcccttttgttgagccaggcctctctcacctctcctactcacttaaagcccgcctgacagaaa
ccacggccacatttggttctaagaaaccctctgtcattcgctcccacattctgatgagcaaccgcttccctatttatttattta
tttgtttgtttgttttattcattggtctaatttattcaaagggggcaagaagtagcagtgtctgtaaaagagcctagtttttaa
tagctatggaatcaattcaatttggactggtgtgctctctttaaatcaagtcctttaattaagactgaaaatatataagctcag
attatttaaatgggaatatttataaatgagcaaatatcatactgttcaatggttctgaaataaacttcactgaagaaaaaaaa
gggtctttcctgatcattgacttgtcttggatttgacactgaacagtaaagacaaacagggctgtgagagttcttgggggacta
aagcccacctcctcattgctgagtgctgcaaagtcacctagaaatatcccttggccaccgaagactatcctcctcacccatccc
ctttatttctgttgttcaacagaaggatattcagtgcacatctggaacaggatcagctgaagcactgcagggagtcaggactgg
tagtaacagctaccagtgatttatctatcaatgcaccaaacatctgttgagcaagcgctatgtacgaggagctgggagtacaga
gatgagaacagtcacaagtccctcctcagataggagaggcagctagttataagcagaaacaaggtaacatgacaagtagagtaa
gataaagaacaa
<212> Type : DNA
<211> Length : 7824
      SequenceName : M15840
      SequenceDescription :

Sequence
--------
<213> OrganismName : HUMAN
<400> PreSequenceString :
ggttcctggtcctcgcctcctccgctgtctccctggagttcttgcaagtcggccaggatgtctcaggctgagtttgagaaagct
gcagaggaggttaggcaccttaagaccaagccatcggatgaggagatgctgttcatctatggccactacaaacaagcaactgtg
ggcgacataaatacagaacggcccgggatgttggacttcacgggcaaggccaagtgggatgcctggaatgagctgaaagggact
tccaaggaagatgccatgaaagcttacatcaacaaagtagaagagctaaagaaaaaatacgggatatgagagactggatttggt
tactgtgccatgtgtttatcctaaactgagacaatgccttgtttttttctaataccgtggatggtggaa
<212> Type : DNA
<211> Length : 405
      SequenceName : M15887
      SequenceDescription :

Sequence
--------
<213> OrganismName : HUMAN
<400> PreSequenceString :
cgaggcgctagggggaacgctggcctctgaaactagctctgggaccggggtctgcggccggcccctagctggccccgtctccca
tccccagaagggtattcactggggattctgagctttggctactccagtttcccacgacacgatgttccctttctacagctgctg
gaggactggactgctactactactcctggctgtggcagtgagagaatcctggcagacagaagaaaaacttgcgacttggtagg

agaaaagggtaaagagtcagagaaagagttggctctagtgaagaggctgaaaccactgtttaataaaagctttgagagcactgt
gggccagggttcagacacatacatctacatcttcagggtgtgccgggaagctggcaaccacacttctggggcaggcctggtgca
aatcaacaaaagtaatgggaaggagacagtggtagggagactcaacgagactcacatcttcaacggaagtaattggatcatgct
gatctataaagggggtgatgaatatgacaaccactgtggcaaggagcagcgtcgtgcagtggtgatgatctcctgcaatcgaca
caccctagcggacaattttaaccctgtgtctgaggagcgtggcaaagtccaagattgtttctacctctttgagatggatagcag
cctggcctgttcaccagagatctcccacctcagtgtgggttccatcttacttgtcacgtttgcatcactggttgctgtttatgt
tgttgggggggttcctataccagcgactggtagtgggagccaaaggaatggagcagtttccccacttagccttctggcaggatct
tggcaacctggtagcagatggctgtgactttgtctgccgttctaaacctcgaaatgtgcctgcagcatatcgtggtgtgggggga
tgaccagctgggggaggagtcagaagaaagggatgaccatttattaccaatgtagattgcactttatatgtccagcctcttcct
cagtcccccaaaccaaagctacacagccagatttctcaagcagtctcaactccagtccctcatctcacccttactattgctctt
gctttccagtttgcttttgatttgcatcttctcactagtaaaactgccttccctttgttccttattttctgtttttctctaga
gaggtacagttgtaagtcagagttaatataatagggcctgtgaaaacagaggcttttgcattgtctcttgacatcagaagttac
aataggcatatgggcaaaatggtgtagcaggctcactggccgtttgtttttaaacacattttcacaagtttttgagacactgg
atttctttaattaaaaaaaaatgccaagaaacattatttatacagggttgattgctttcatgttgttattctgtaccctatag
tagcctccatgagaatctggtatttcttgctgcttggaactactttgcagtgattacttggttgcagtccaagtactctcgttt
agtctgagcctggagatgttctagacttgcttctcccacctctgagattaggacaggaaaaatgtgaaatttcccaattacagg
attatacggtaccatcacatcatttgtggaaattggggtgactgtatagctgggattgggctaaggactgtggtcttatctgtc
cacatacagccaaaatgcctatccagaaatccagttcgttggaaaggaaaattggtactcctgtgccacaggggttccagaaaa
gggaagtcactttaccttgcggtggtgggatcctgatgtctttcatccatttgtagtaaaagctggtaaagcttttcttactcc
tggttccctaccagtatttctaaacatgtcgcactttctccacaggcatgtggttttgaccttttttcaatcttctagaaagg
gaacggaagcagaagtgggacatcgagggctctgctgtcctctgcgctgggtgtggaatgctgctgcacctgtcccttctgctg
gctcagggaagtgtcttcttgcccacatttctgtggggaaaggttttaatcctctgatgcttccatcttcctgtttaggccat
gtgcccagaaacctggactgatctttctttaatagtgaacccctgggccactgaagagtaacatggctccactggacacaaaag
agggatggaatcaacaggcagggggccttttataagccttaggaaaagaaaatgaaactatttcatctttggacttttcaatac
tattggagtgatttttttctttctaaacagggaaaataatgttacaaaagcatctttttgttatttgtttgcatccctccccc
acaccctggtgttttaaaatgaagaaaaaaaaccatcaccttttgtacaaaaactcttaatgattaaaaaacaaac
<212> Type : DNA
<211> Length : 2428
SequenceName : M16985
SequenceDescription :

Sequence
--------
<213> OrganismName : HUMAN
<400> PreSequenceString :
acaaactttcagagacagcagagcacacaagcttctaggacaagagccaggaagaaaccaccggaaggaaccatctcactgtgt
gtaaacatgacttccaagctggccgtggctctcttggcagccttcctgatttctgcagctctgtgtgaaggtgcagttttgcca
aggagtgctaaagaacttagatgtcagtgcataaagacatactccaaacctttccaccccaaatttatcaaagaactgagagtg
attgagagtggaccacactgcgccaacacagaaattattgtaaagctttctgatggaagagagctctgtctggaccccaaggaa
aactgggtgcagagggttgtggagaagtttttgaagagggctgagaattcataaaaaaattcattctctgtggtatccaagaat
cagtgaagatgccagtgaaacttcaagcaaatctacttcaacacttcatgtattgtgtgggtctgttgtagggttgccagatgc
aatacaagattcctggttaaatttgaatttcagtaaacaatgaatagtttttcattgtaccatgaaatatccagaacatactta
tatgtaaagtattatttatttgaatctacaaaaaacaacaaataatttttgaatataaggattttcctagatattgcacgggag
aatatacaaatagcaaaattgggccaagggccaagagaatatccgaactttaatttcaggaattgaatgggtttgctagaatgt
gatatttgaagcatcacataaaaatgatgggacaataaatttttgccataaagtcaaatttagctggaaatcctggatttttttc
tgttaaatctggcaaccctagtctgctagccaggatccacaagtccttgttccactgtgccttggtttctcctttatttctaag
tggaaaaagtattagccaccatcttacctcacagtgatgttgtgaggacatgtggaagcactttaagtttttcatcataacat
aaattattttcaagtgtaacttattaacctatttattatttatgtatttatttaagcatcaaatatttgtgcaagaatttggaa
aaatagaagatgaatcattgattgaatagttataaagatgttatagtaaatttattttattttagatattaaatgatgttttat
tagataaatttcaatcagggtttttagattaaacaaacaaacaattgggtacccagttaaattttcatttcagataaacaacaa
ataatttttagtataagtacattattgtttatctgaaatttaattgaactaacaatcctagtttgatactcccagtcttgtc
attgccagctgtgttggtagtgctgtgttgaattacggaataatgagttagaactattaaaacagccaaaactccacagtcaat
attagtaatttcttgctggttgaaacttgtttattatgtacaaatagattcttataatattatttaaatgactgcatttttaaa
tacaaggctttatattttaactttaagatgttttatgtgctctccaaatttttttactgtttctgattgtatggaaatata
aaagtaaatatgaacatttaaaatataatttgttgtcaaagt
<212> Type : DNA
<211> Length : 1639
SequenceName : M17017
SequenceDescription :

Sequence
--------
<213> OrganismName : HUMAN

<400> PreSequenceString :
atgaactggcatctcccctcttcctcttggcctctgtgacgctgccttccatctgctcccacttcaatcctctgtctctcgag
gaactaggctccaacacggggatccaggttttcaatcagattgtgaagtcgaggcctcatgacaacatcgtgatctctcccat
gggattgcgtcggtcctggggatgcttcagctgggggcggacggcaggaccaagaagcagctcgccatggtgatgagatacggc
gtaaatggagttggtaaaatattaaagaagatcaacaaggccatcgtctccaagaagaataaagacattgtgacagtggctaac
gccgtgtttgttaagaatgcctctgaaattgaagtgcctttgttacaaggaacaaagatgtgttccagtgtgaggtccggaat
gtgaactttgaggatccagcctctgcctgtgattccatcaatgcatgggttaaaaacgaaaccagggatatgattgacaatctg
ctgtccccagatcttattgatggtgtgctcaccagactggtcctcgtcaacgcagtgtatttcaagggtctgtggaaatcacgg
ttccaacccgagaacacaaagaaacgcactttcgtggcagccgacgggaaatcctatcaagtgccaatgctggcccagctctcc
gtgttccggtgtgggtcgacaagtgcccccaatgatttatggtacaacttcattgaactgccctaccacggggaaagcatcagc
atgctgattgcactgccgactgagagctccactccgctgtctgccatcatcccacacatcagcaccaagaccatagacagctgg
atgagcatcatggtccccaagagggtgcaggtgatcctgcccaagttcacagctgtagcacaaacagatttgaaggagccgctg
aaagttcttggcattactgacatgtttgattcatcaaaggcaaattttgcaaaaataacaaggtcagaaaacctccatgtttct
catatcttgcaaaaagcaaaaattgaagtcagtgaagatggaaccaaagcttcagcagcaacaactgcaattctcattgcaaga
tcatcgcctccctggtttatagtagacagaccttttctgtttttcatccgacataatcctacaggtgctgtgttattcatgggg
cagataaacaaaccc
<212> Type : DNA
<211> Length : 1191
      SequenceName : M17783
      SequenceDescription :

Sequence
--------
<213> OrganismName : HUMAN
<400> PreSequenceString :
gacaatatcaggtgagctgtggaggtggggtccttggaagctggatgacagcagctggcaaggggataagagagcagtgagccc
ctccctcaaggaggtctggctttatccatagacagggccctctgaggtggggctgaggtacaaaggggggattgagcagcccagg
agaagagagatgggggttcccttcttctcttctctcagatgcatggtggacttaggaccttgctgggctgggggtctcactgca
gagatgaagctgcttctggccctagcagggctcctggccattctggccacgccccagccctctgaaggtgctgctccagctgtc
ctgggggaggtggacacctcgttggtgctgagctccatggaggaggccaagcagctggtggacaaggcctacaaggagcggcgg
gaaagcatcaagcagcggcttcgcagcggctcagccagccccatggaactcctatcctacttcaagcagccggtggcagccacc
aggacggcggtgagggccgctgactacctgcacgtggctctagacctgctggagaggaagctgcggtccctgtggcgaaggcca
ttcaatgtcactgatgtgctgacgcccgcccagctgaatgtgttgtccaagtcaagcggctgcgcctaccaggacgtgggggtg
acttgcccggagcaggacaaataccgcaccatcaccgggatgtgcaacaacagacgcagccccacgctgggggcctccaaccgt
gcctttgtgcgctggctgccggcggagtatgaggacggcttctctcttccctacggctggacgcccggggtcaagcgcaacggc
ttcccggtggctctggctcgcgcggtctccaacgagatcgtgcgcttccccactgatcagctgactccggaccaggagcgctca
ctcatgttcatgcaatggggccagctgttggaccacgacctcgacttcacccctgagccggccgcccgggcctccttcgtcact
ggcgtcaactgcgagaccagctgcgttcagcagccgccctgcttcccgctcaagatcccgcccaatgacccccgcatcaagaac
caagccgactgcatcccgttcttccgctcctgcccggcttgccccgggagcaacatcaccatccgcaaccagatcaacgcgctc
acttccttcgtggacgccagcatggtgtacggcagcgaggagcccctggccaggaacctgcgcaacatgtccaaccagctgggg
ctgctggccgtcaaccagcgcttccaagacaacggccgggccctgctgccctttgacaacctgcacgatgacccctgtctcctc
accaaccgctcagcgcgcatccccctgcttcctggcaggggacacccgttccagtgagatgcccgagctcacctccatgcacacc
ctcttacttcgggagcacaaccggctggccacagagctcaagagcctgaaccctaggtgggatggggagaggctctaccaggaa
gcccggaagatcgtgggggccatggtccagatcatcacttaccgggactacctgcccctggtgctggggccaacggccatgagg
aagtacctgcccacgtaccgttcctacaatgactcagtggacccacgcatcgccaacgtcttcaccaatgccttccgctacggc
cacaccctcatccaacccttcatgttccgcctggacaatcggtaccagcccatggaacccaacccccgtgtccccctcagcagg
gtctttttgcctcctggagggtcgtgctggaaggtggcattgaccccatcctccggggcctcatggccacccctgccaagctg
aatcgtcagaaccaaattgcagtggatgagatccgggagcgattgtttgagcaggtcatgaggattgggctggacctgcctgct
ctgaacatgcagcgcagcagggaccacggcctcccaggatacaatgcctggaggcgcttctgtgggctcccgcagcctgaaact
gtgggccagctgggcacggtgctgaggaacctgaaattggcgaggaaactgatggagcagtatggcacgcccaacaacatcgac
atctggatgggcggcgtgtccgagcctctgaagcgcaaaggccgcgtgggcccactcctcgcctgcatcatcggtacccagttc
aggaagctccgggatggtgatcggttttggtgggagaacgagggtgtgttcagcatgcagcagcgacaggccctggcccagatc
tcattgccccggatcatctgcgacaacacaggcatcaccaccgtgtctaagaacaacatcttcatgtccaactcatatccccgg
gactttgtcaactgcagtacacttcctgcattgaacctggcttcctggagggaagcctcctagaggccaggtaaggggggtgcag
cagtgaggggtatatctgggctggccagttggaaccacggagatctccttgccctagatgagcccagccctgttctgggtgcag
ctgagaaaatgagtgactagacgttcatttgtgtgctcatgtatgtgcgaagtatataaattggcttttcatgcgtgtgtgttg
tctgaacatggggagtgtttcatgggttatgtgtatgtgccatttatgtgagtgtgtgtttgtgctgatgagaatactgagtat
gtggaaggcagcagagcggactggtgaggagcacagctcaggaactagactgcctgggttccaatcctggctctgtggcttgct
agctatgtgaccttgagcaaattaccctccttaaacaagagttttcttccttgtaaattacatctgtcatggtttcttggaggg
cccacttgtatcctctggttcttcatttattgagcacctactacatgcaaggcactgtactaggcgtgagaagcatatagaggc
aagaaagagataccaagatgccatctgtgtcctggttagcagagctggaccagtggtgccttggagggataagccagctgcagc
tgggctgtgtggttgacttatgggcccagccagccaggctcaggccatggctcccctttttcttcctcaccctgatttcttgct
tattcactgaagttctcctgaagaggaactgggcctgttgccctttctgtaccatttatttgctcccaatgtttatgataataa

aggcaccgctgatggggacctcc
<212> Type : DNA
<211> Length : 3215
      SequenceName : M19507
      SequenceDescription :

Sequence
--------
<213> OrganismName : HUMAN
<400> PreSequenceString :
ggcggtcccctgttctccccgctcaggtgcggcgctgtggcaggaagccacccctcggtcggccggtgcgcggggctgttgcg
ccatccgctccggctttcgtaaccgcaccctgggacggcccagagacgctccagcgcgagttcctcaaatgttttcctgcgttg
ccaggaccgtccgccgctctgagtcatgtgcgagtgggaagtcgcactgacactgagccgggccagagggagaggagccgagcg
cggcgcggggccgagggactcgcagtgtgtgtagagagccgggctcctgcggatggggctgccccggggcctgagcccgcct
gcccgcccaccgccccgccccgcccctgccacccctgccgcccggttcccattagcctgtccgcctctgcgggaccatggagtg
gtagccgaggaggaagcatgctggccgtcggctgcgcgctgctggctgccctgctggccgcgccgggagcggcgctggccccaa
ggcgctgccctgcgcaggaggtggcaagaggcgtgctgaccagtctgccaggagacagcgtgactctgacctgcccgggggtag
agccggaagacaatgccactgttcactgggtgctcaggaagccggctgcaggctcccaccccagcagatgggctggcatgggaa
ggaggctgctgctgaggtcggtgcagctccacgactctggaaactattcatgctaccgggccggccgcccagctgggactgtgc
acttgctggtggatgttccccccgaggagccccagctctcctgcttccggaagagcccccctcagcaatgttgtttgtgagtggg
gtcctcggagcaccccatccctgacgacaaaggctgtgctcttggtgaggaagtttcagaacagtccggccgaagacttccagg
agccgtgccagtattcccaggagtcccagaagttctcctgccagttagcagtcccggagggagacagctctttctacatagtgt
ccatgtgcgtcgccagtagtgtcgggagcaagttcagcaaaactcaaacctttcagggttgtggaatcttgcagcctgatccgc
ctgccaacatcacagtcactgccgtggccagaaaccccgctggctcagtgtcacctggcaagacccccactcctggaactcat
ctttctacagactacggtttgagctcagatatcgggctgaacggtcaaagacattcacaacatggatggtcaaggacctccagc
atcactgtgtcatccacgacgcctggagcggcctgaggcacgtggtgcagcttcgtgcccaggaggagttcgggcaaggcgagt
ggagcgagtggagcccggaggccatgggcacgccttggacagaatccaggagtcctccagctgagaacgaggtgtccacccccca
tgcaggcacttactactaataaagacgatgataatattctcttcagagattctgcaaatgcgacaagcctcccagtgcaagatt
cttcttcagtaccactgcccacattcctggttgctggagggagcctggccttcggaacgctcctctgcattgccattgttctga
ggttcaagaagacgtggaagctgcgggctctgaaggaaggcaagacaagcatgcatccgccgtactctttggggcagctggtcc
cggagaggcctcgacccaccccagtgcttgttcctctcatctccccaccggtgtcccccagcagcctggggtctgacaatacct
cgagccacaaccgaccagatgccagggacccacggagcccttatgacatcagcaatacagactacttcttccccagatagctgg
ctgggtggcaccagcagcctggaccctgtggatgacaaaacacaaacgggctcagcaaaagatgcttctcactgccatgccagc
ttatctcaggggtgtgcggcctttggcttcacggaagagccttgcggaaggttctacgccaggggaaaatcagcctgctccagc
tgttcagctggttgaggtttcaaacctccctttccaaatgcccagcttaaaggggttagagtgaacttgggccactgtgaagag
aaccatatcaagactctttggacactcacacggacactcaaaagctgggcaggttggtgggggcctcggtgtggagaagcggct
ggcagcccacccctcaacacctctgcacaagctgcaccctcaggcaggtgggatggatttccagccaaagcctcctccagccgc
catgctcctggcccactgcatcgtttcatcttccaactcaaactcttaaaacccaagtgcccttagcaaattctgtttttctag
gcctggggacggcttttacttaaacgccaaggcctgggggaagaagctctctcctccctttcttccctacagttcaaaaacagc
tgagggtgagtgggtgaataatacagtatgtcagggcctggtcgttttcaacagaattataattagttcctcattagcagtttt
gcctaaatgtgaatgatgatcctaggcatttgctgaatacagaggcaactgcattggctttgggttgcaggacctcaggtgaga
agcagaggaaggagaggagaggggcacagggtctctaccatcccctgtagagtgggagctgagtggggatcacagcctctgaa
aaccaatgttctctcttctccacctcccacaaaggagagctagcagcagggagggcttctgccatttctgagatcaaaacggtt
ttactgcagctttgtttgttgtcagctgaacctgggtaactagggaagataatattaaggaagacaatgtgaaaagaaaaatga
gcctggcaagaatgcgtttaaacttggtttttaaaaactgctgactgttttctcttgagagggtggaatatccaatattcgct
gtgtcagcatagaagtaacttacttaggtgtggggggaagcaccataactttgtttagcccaaaaccaagtcaagtgaaaaagga
ggaagagaaaaaatattttcctgccaggcatggaggcccacgcacttcgggaggtcgaggcaggaggatcacttgagtccagaa
gtttgagatcagcctgggcaatgtgataaaaccccatctctacaaaaagcataaaaattagccaagtgtggtagagtgtgcctg
aagtcccagatacttgggggctgaggtgggaggatctcttgagcctgggaggtcaaggctgcagtgagccgagattgcaccac
tgcactccagcctggggtgacagagcaagtgagaccctgtctc
<212> Type : DNA
<211> Length : 3319
      SequenceName : M20566
      SequenceDescription :

Sequence
--------
<213> OrganismName : HUMAN
<400> PreSequenceString :
cttgttcggggcgctccgctggcttcttggacaattgcgccatgtgtgctgctcggctagcggcggcggcggcccagtcggtgt
atgccttctcggcgcgcccgttggccggcggggagcctgtgagcctgggctccctgcggggcaaggtactacttatcgagaatg
tggcgtccctctgaggcaccacggtccgggactacacccagatgaacgagctgcagcggcgcctcggaccccggggcctggtgg
tgctcggcttcccgtgcaaccagtttgggcatcaggagaacgccaagaacgaagagattctgaattccctcaagtacgtccggc

ctggtggtgggttcgagcccaacttcatgctcttcgagaagtgcgaggtgaacggtgcgggggcgcaccctctcttcgccttcc
tgcgggaggccctgccagctcccagcgacgacgccaccgcgcttatgaccgaccccaagctcatcacctggtctccggtgtgtc
gcaacgatgttgcctggaactttgagaagttcctggtgggccctgacggtgtgcccctacgcaggtacagccgccgcttccaga
ccattgacatcgagcctgacatcgaagccctgctgtctcaagggcccagctgtgcctagggcgcccctcctaccccggctgctt
ggcagttgcagtgctgctgtctcgggggggttttcatctatgagggtgtttcctctaaacctacgagggaggaacaccttgatc
ttacagaaaataccacctcgagatgggtgctggtcctgttgatcccagtctctgccagaccaaggcgagtttccccactaataa
agtgccgggtgtcagc
<212> Type : DNA
<211> Length : 856
      SequenceName : M21304
      SequenceDescription :

Sequence
--------
<213> OrganismName : HUMAN
<400> PreSequenceString :
atgaagcttctcacgggcctggttttctgctccttggtcctgagtgtcagcagccgaagcttcttttcgttccttggcgaggct
tttgatggggctcgggacatgtggagagcctactctgacatgagagaagccaattacatcggctcagacaaatacttccatgct
cggggggaactatgatgctgccaaaaggggacctgggggtgcctgggccgcagaagtgatcagcaatgccagagagaatatccag
agactcacaggccatggtgcggaggactcgctggccgatcaggctgccaataaatggggcaggagtggcagagaccccaatcac
ttccgacctgctggcctgcctgagaaatactga
<212> Type : DNA
<211> Length : 369
      SequenceName : M23699
      SequenceDescription :

Sequence
--------
<213> OrganismName : HUMAN
<400> PreSequenceString :
tagacgcaccctctgaagatggtgactccctcctgagaagctggaccccttggtaaaagacaaggccttctccaagaagaatat
gaaagtgttactcagacttatttgtttcatagctctactgatttcttctctggaggctgataaatgcaaggaacgtgaagaaaa
aataatttagtgtcatctgcaaatgaaattgatgttcgtccctgtcctcttaacccaaatgaacacaaaggcactataacttg
gtataaagatgacagcaagacacctgtatctacagaacaagcctccaggattcatcaacacaaagagaaactttggtttgttcc
tgctaaggtggaggattcaggacattactattgcgtggtaagaaattcatcttactgcctcagaattaaaataagtgcaaaatt
tgtggagaatgagcctaacttatgttataatgcacaagccatatttaagcagaaactacccgttgcaggagacggaggacttgt
gtgcccttatatggagtttttaaaatgaaaataatgagttacctaaattacagtggtataaggattgcaaacctctacttct
tgacaatatacactttagtggagtcaaagataggctcatcgtgatgaatgtggctgaaaagcatagagggaactatacttgtca
tgcatcctacacatacttgggcaagcaatatcctattacccgggtaatagaatttattactctagaggaaaacaaacccacaag
gcctgtgattgtgagcccagctaatgagacaatggaagtagacttgggatcccagatacaattgatctgtaatgtcaccggcca
gttgagtgacattgcttactggaagtggaatgggtcagtaattgatgaagatgacccagtgctaggggaagactattacagtgt
ggaaaatcctgcaaacaaaagaaggagtaccctcatcacagtgcttaatatatcggaaattgaaagtagattttataaacatcc
atttacctgttttgccaagaatacacatggtatagatgcagcatatatccagttaatatatccagtcactaatttccagaagca
catgattggtatatgtgtcacgttgacagtcataattgtgtgttctgttttcatctataaaatcttcaagattgacattgtgct
ttggtacagggattcctgctatgatttttctcccaataaaagcttcagatggaaagacctatgacgcatatatactgtatccaaa
gactgttggggaagggtctacctctgactgtgatatttttgtgtttaaagtcttgcctgaggtcttggaaaaacagtgtggata
taagctgttcatttatggaagggatgactacgttggggaagacattgttgaggtcattaatgaaaacgtaaagaaaagcagaag
actgattatcattttagtcagagaaacatcaggcttcagctggctgggtggttcatctgaagagcaaatagccatgtataatgc
tcttgttcaggatggaattaaagttgtcctgcttgagctggagaaaatccaagactatgagaaaatgccagaatcgattaaatt
cattaagcagaaacatggggctatccgctggtcaggggactttacacagggaccacagtctgcaaagacaaggttctggaagaa
tgtcaggtaccacatgccagtccagcgacggtcaccttcatctaaacaccagttactgtcaccagccactaaggagaaactgca
aagagaggctcacgtgcctctcgggtagcatggagaagttgccaagagttctttaggtgcctcctgtcttatggcgttgcaggc
caggttatgcctcatgctgacttgcagagttcatggaatgtaactatatcatcctttatccctgaggtcacctggaatcagatt
attaagggaataagccatgacgtcaatagcagcccagggcacttcagagtagagggcttgggaagatcttttaaaaaggcagta
ggcccggtgtggtggctcacgcctataatcccagcactttgggaggctgaagtgggtggatcaccagaggtcaggagttcgaga
ccagcccagccaacatggcaaaaccccatctctactaaaaatacaaaaatgagctaggcatggtggcacacgcctgtaatccca
gctacacctgaggctgaggcaggagaattgcttgaaccggggagacggaggttgcagtgagccgagtttgggccactgcactct
agcctggcaacagagcaagactccgtctcaaaaaaagggcaataaatgccctctctgaatgtttgaactgccaagaaaaggcat
ggagacagcgaactagaagaaagggcaagaaggaaatagccaccgtctacagatggcttagttaagtcatccacagcccaaggg
cggggctatgccttgtctggggaccctgtagagtcactgaccctggagcggctctcctgagaggtgctgcaggcaaagtgagac
tgacacctcactgaggaagggagacatattcttggagaactttccatctgcttgtattttccatacacatccccagccagaagt
tagtgtccgaagaccgaatttatttacagagcttgaaaactcacttcaatgaacaaagggattctccaggattccaaagttt
tgaagtcatcttagctttccacaggagggagagaacttaaaaaagcaacagtagcagggaattgatccacttcttaatgctttc

ctccctggcatgaccatcctgtcctttgttattatcctgcatttacgtctttggaggaacagctccctagtggcttcctccgt
ctgcaatgtcccttgcacagcccacacatgaaccatccttcccatgatgccgctcttctgtcatcccgctcctgctgaaacacc
tcccaggggctccacctgttcaggagctgaagcccatgctttcccaccagcatgtcactcccagaccacctccctgccctgtcc
tccagcttcccctcgctgtcctgctgtgtgaattcccaggttggcctggtggccatgtcgcctgcccccagcactcctctgtct
ctgctcttgcctcgacccttcctcctcctttgcctaggaggccttctcgcattttctctagctgatcagaattttaccaaaatt
cagaacatcctccaattccacagtctctgggagactttccctaagaggcgacttcctctccagccttctctctctggtcaggcc
cactgcagagatggtggtgagcacatctgggaggctggtctccctccagctggaattgctgctctctgagggagaggctgtggt
ggctgtctctgtccctcactgccttccaggagcaatttgcacatgtaacatagatttatgtaatgctttatgtttaaaaacatt
ccccaattatcttatttaatttttgcaattattctaattttatatatagagaaagtgacctatttttaaaaaaatcacactct
aagttctattgaacctaggacttgagcctccatttctggcttctagtctggtgttctgagtacttgatttcaggtcaataacgg
tccccccctcactccacactggcacgtttgtgagaagaaatgacatttgctaggaagtgaccgagtctaggaatgcttttattc
aagacaccaaattccaaacttctaaatgttggaattttcaaaaattgtgtttagattttatgaaaaactcttctactttcatct
attctttccctagaggcaaacatttcttaaaatgtttcattttcattaaaaatgaaagccaaatttatatgccaccgattgcag
gacacaagcacagttttaagagttgtatgaacatggagaggacttttggtttttatatttctcgtatttaatatgggtgaacac
caacttttatttggaataataatttttcctcctaaacaaaaacacattgagtttaagtctctgactcttgcctttccacctgctt
tctcctgggcccgctttgcctgcttgaaggaacagtgctgttctggagctgctgttccaacagacagggcctagctttcatttg
acacacagactacagccagaagcccatggagcagggatgtcacgtcttgaaaagcctattagatgtttacaaatttaatttg
cagattattttagtctgtcatccagaaaatgtgtcagcatgcatagtgctaagaaagcaagccaatttggaaacttaggttagt
gacaaaattggccagagagtgggggtgatgatgaccaagaattacaagtagaatggcagctggaatttaaggagggacaagaat
caatggataagcgtgggtggaggaagatccaaacagaaaagtgcaaagttattccccatcttccaagggttgaattctggagga
agaagacacattcctagttccccgtgaacttcctttgacttattgtccccactaaaacaaaacaaaaaactttaatgccttcc
acattaattagatttcttgcagtttttttatggcatttttttaaagatgccctaagtgttgaagaagagtttgcaaatgcaac
aaaaatatttaattaccggttgttaaaactggtttagcacaatttatatttccctctcttgcctttcttatttgcaataaaag
gtattgagccatttttttaaatgacattttgataaattatgtttgtactagttgatgaaggagtttttttaacctgtttatat
aatttgcagcagaagccaaatttttgtatattaaagcaccaaattcatgtacagcatgcatcacggatcaatagactgtact
tattttccaataaaattttcaaactttgtactgttaaa
<212> Type : DNA
<211> Length : 4910
      SequenceName : M27492
      SequenceDescription :

Sequence
--------
<213> OrganismName : HUMAN
<400> PreSequenceString :
atgcaggccatcaagtgtgtggtggtgggagacggagctgtaggtaaaacttgcctactgatcagttacacaaccaatgcattt
cctggagaatatatccctactgtctttgacaattattctgccaatgttatggtagatggaaaaccggtgaatctgggcttatgg
gatacagctggacaagaagattatgacagattacgcccccctatcctatccgcaaacagatgtgttcttaatttgcttttccctt
gtgagtcctgcatcatttgaaaatgtccgtgcaaagtggtatcctgaggtgcggcaccactgtcccaacactcccatcatccta
gtgggaactaaacttgatcttagggatgataaagacacgatcgagaaactgaaggagaagaagctgactcccatcacctatccg
cagggtctagccatggctaaggagattggtgctgtaaaatacctggagtgctcggcgctcacacagcgaggcctcaagacagtg
tttgacgaagcgatccgagcagtcctctgcccgcctcccgtgaagaagaggaagagaaaatgcctgctgttgtaa
<212> Type : DNA
<211> Length : 579
      SequenceName : M29870
      SequenceDescription :

Sequence
--------
<213> OrganismName : HUMAN
<400> PreSequenceString :
atgcaggccatcaagtgtgtggtggtgggagatggggccgtgggcaagacctgccttctcatcagctacaccaccaacgccttt
cccggagagtacatccccaccgtgtttgacaactattcagccaatgtgatggtggacagcaagccagtgaacctggggctgtgg
gacactgctgggcaggaggactacgaccgtctccggccgctctcctatccacagacggacgtcttcctcatctgcttctccctc
gtcagcccagcctcttatgagaacgtccgcgccaagtggttcccagaagtgcggcaccactgccccagcacacccatcatcctg
gtgggcaccaagctggacctgcgggacgacaaggacaccatcgagaaactgaaggagaagaagctggctcccatcacctacccg
cagggcctggcactggccaaggagattgactcggtgaaatacctggagtgctcagccctcacccagagaggcctgaaaaccgtg
ttcgacgaggccatccgggccgtgctgtgccctcagcccacgcggcagcagaagcgcgcctgcagcctcctctag
<212> Type : DNA
<211> Length : 579
      SequenceName : M29871
      SequenceDescription :

Sequence
--------
<213> OrganismName : HUMAN
<400> PreSequenceString :
acatggcgaacgaggttatcaagtgcaaggctgcagttgcttgggaggctggaaagcctctctccatagaggagatagaggtgg
cacccccaaaggctcatgaagttcgaatcaagatcattgccactgcggtttgccacaccgatgcctataccctgagtggagctg
atcctgagggttgttttccagtgatcttgggacatgaaggtgctggaattgtggaaagtgttggtgagggagttactaagctga
aggcgggtgacactgtcatcccactttacatcccacagtgtggagaatgcaaattttgtctaaatcctaaaactaacctttgcc
agaagataagagtcactcaagggaaaggattaatgccagatggtaccagcagatttacttgcaaaggaaagacaattttgcatt
acatgggaaccagcacatttctgaatacacagttgtggctgatatctctgttgctaaaatagatcctttagcacctttggata
aagtctgccttctaggttgtggcatttcaaccggttatggtgctgctgtgaacactgccaagttggagcctggctctgtttgtg
ccgtctttggtctgggaggagtcggattggcagttatcatgggctgtaaagtggctggtgcttcccggatcattggtgtggaca
tcaataaagataaatttgcaagggccaaagagtttggagccactgaatgtattaaccctcaggattttagtaaacccatccagg
aagtgctcattgagatgaccgatggaggagtggactattcctttgaatgtattggtaatgtgaaggtcatgagagcagcacttg
aggcatgtcacaagggctggggcgtcagcgtcgtggttggagtagctgcttcaggtgaagaaattgccactcgtccattccagc
tggtaacaggtcgcacatggaaaggcactgcctttggaggatggaagagtgtagaaagtgtcccaaagttggtgtctgaatata
tgtccaaaaagataaaagttgatgaatttgtgactcacaatctgtcttttgatgaaatcaacaaagcctttgaactgatgcatt
ctggaaagagcattcgaactgttgtaaagatttaattcaaaagagaaaaataatgtccatcctgtcgtgatgtgataggagcag
cttaacaggcagggagaagcgcctccaacctcacagcctcgtagagcttcacagctactccagaaaatagggttatgtgtgtca
ttcatgaatctctataatcaaggacaaggataattcagtcatgaacctgttttctggatgctcctccacataaataattgctag
tttattaaggaatattttaacataataaaagtaatttctacatttgtgtggaaattgtcttgttttatgctgtcatcattgtca
cggttgtctgccattatcttcattctgcaagggaaagggaaaggaagcagggcagtggtgggtgtctgaaacctcagaaacata
acgttgaacttttaagggtctcagtccccgttgattaaagaacagatcctagccatcagtgacaaagttaatcaggacccaagt
ctgcttctgtgatattatcttgaagggaggtactgtgccttgttcatacctgtaccccaaattcctaggatgcatctgccttca
gggggcactaaaatgtattattgaaacagcattctgggcttaaataggtgtatgtatgtgttggttgtgactgtactattctag
tatagtgaactacatactgaatatccaagttctcagcacctacttttgtcaaatcttaacattttgccacttcgagatcacatt
gccttcctcccctccaagaggtaacaattatccacaatttgatgtttatcattcctgtgttgttgtactttcactgtgtataac
ctaaaccatctactctttagtactgttttatatattttaagcctcatacttgctcattctacagctttttcactcattattg
tataattatatctgaagctctcgttcattaatttagtcctgtgtagcagaattcaattacgggaaactaccataatttatctg
ttctccagtccagttgaaggcatgaagttgttgccagtttctgtattataacactgtagtggaacattcttctgcattgggcta
ctcgcgtgttacctaagacgtatcacagaataaacacatttagccttatagacattgccaaattgctcttcaaagtaaatgtga
gtttttgtg
<212> Type : DNA
<211> Length : 2277
SequenceName : M30471
SequenceDescription :

Sequence
--------
<213> OrganismName : HUMAN
<400> PreSequenceString :
agacgttcgcacacctgggtgccagcgccccagaggtcccgggacagcccgaggcgccgcgcccgccgccccgagctccccaag
ccttcgagagcggcgcacactcccggtctccactcgctcttccaacacccgctcgttttgcggcagctcgtgtcccagagaccg
agttgccccagagaccgagacgccgccgctgcgaaggaccaatgagagccccgctgctaccgccggcgccggtggtgctgtcgc
tcttgatactcggctcaggccattatgctgctggattggacctcaatgacacctactctgggaagcgtgaaccattttctgggg
accacagtgctgatggatttgaggttacctcaagaagtgagatgtcttcagggagtgagatttcccctgtgagtgaaatgcctt
ctagtagtgaaccgtcctcgggagccgactatgactactcagaagagtatgataacgaaccacaaatacctggctatattgtcg
atgattcagtcagagttgaacaggtagttaagcccccccaaaacaagacggaaagtgaaaatacttcagataaacccaaaagaa
agaaaaagggaggcaaaaatggaaaaaatagaagaaacagaaagaagaaaaatccatgtaatgcagaatttcaaaatttctgca
ttcacggagaatgcaaatatatagagcacctggaagcagtaacatgcaaatgtcagcaagaatatttcggtgaacggtgtgggg
aaaagtccatgaaaactcacagcatgattgacagtagtttatcaaaaattgcattagcagccatagctgcctttatgtctgctg
tgatcctcacagctgttgctgttattacagtccagcttagaagacaatacgtcaggaaatatgaaggagaagctgaggaacgaa
agaaacttcgacaagagaatggaaatgtacatgctatagcataactgaagataaaattacaggatatcacattggagtcactgc
caagtcatagccataaatgatgagtcggtcctctttccagtggatcataagacaatggaccctttttgttatgatggttttaaa
ctttcaattgtcacttttatgctatttctgtatataaaggtgcacgaaggtaaaaagtatttttcaagttgtaaataattta
tttaatatttaatggaagtgtatttattttacagctcattaaactttttaacc
<212> Type : DNA
<211> Length : 1230
SequenceName : M30704
SequenceDescription :

Sequence
--------

<213> OrganismName : HUMAN
<400> PreSequenceString :
tagtctggagctatggtggtggtggcagccgcgccgaacccggccgacgggacccctaaagttctgcttctgtcggggcagccc
gcctccgccgccggagccccggcggccaggctgccgctcatggtgccagcccagagaggggccagcccggaggcagcgagcggg
gggctgccccaggcgcgcaagcgacagcgcctcacgcacctgagccccgaggagaaggcgctgaggaggaaactgaaaaacaga
gtagcagctcagactgccagagatcgaaagaaggctcgaatgagtgagctggaacagcaagtggtagatttagaagaagagaac
caaaaacttttgctagaaaatcagcttttacgagagaaaactcatggccttgtagttgagaaccaggagttaagacagcgcttg
gggatggatgccctggttgctgaagaggaggcggaagccaaggggaatgaagtgaggccagtggccgggtctgctgagtccgca
gcactcagactacgtgcacctctgcagcaggtgcaggcccagttgtcacccctccagaacatctccccatggattctggcggta
ttgactcttcagattcagagtctgatatcctgttgggcattctggacaacttggacccagtcatgttcttcaaatgcccttccc
cagagcctgccagcctggaggagctcccagaggtctacccagaaggacccagttccttaccagcctccctttctctgtcagtgg
ggacgtcatcagccaagctggaagccattaatgaactaattcgttttgaccacatatataccaagcccctagtcttagagatac
cctctgagacagagagccaagctaatgtggtagtgaaaatcgaggaagcacctctcagcccctcagagaatgatcaccctgaat
tcattgtctcagtgaaggaagaacctgtagaagatgacctcgttccggagctgggtatctcaaatctgctttcatccagccact
gcccaaagccatcttcctgcctactggatgctacagtgactgtggatacggggttccctttccccattcagtgacatgtcctc
tctgcttggtgtaaacattcttgggaggacacttttgccaatgaactctttccccagctgattagtgtctaaggaatgatccaa
tactgttgcccttttccttgactattacactgcctggaggatagcagagaagcctgtctgtacttcattcaaaaagccaaaata
gagagtatacagtcctagagaatccctctatttgttcagatctcatagatgacccccaggtattgccttttgacatccagcagt
ccaaggtattgagacatattactggaagtaagaaatattactataattgagaactacagcttttaagattgtactttttaagatt
gtacttttatcttaaaagggtggtagttttccctaaaatacttattatgtaagggtcattagacaaatgtcttgaagtagacat
ggaatttatgaatggtctttatcatttctcttcccccttttttggcatcctggcttgcctccagttttaggtcctttagtttgct
tctgcaagcaacgggaacacctgctgaggggctctttccctcatgtatacttcaagtaagatcaagaatcttttgtgaaatta
tagaaatttactatgtaaatgcttgatggaatttttcctgctagtgtagcttctgaaaggtgctttctccatttatttaaaaa
ctacccatgcaattaaaaggtacaatgcaaaaaaaaaaaaaaaaaatttttt
<212> Type : DNA
<211> Length : 1818
      SequenceName : M31627
      SequenceDescription :

Sequence
--------
<213> OrganismName : HUMAN
<400> PreSequenceString :
gaattccggcccgccgtcccccaccccgccgccccgcccggcgaattgcgccccgcgcccctcccctcgcgcccccgagacaaa
gaggagagaaagtttgcgcggccgagcggggcaggtgaggagggtgagccgcgcgggaggggcccgcctcggccccggctcagc
ccccgcccgcgcccccagcccgccgccgcgagcagcgcccggacccccccagcggcggcccccgcccgcccagcccccggcccg
ccatgggcgccgcggcccgcaccctgcggctggcgctcggcctcctgctgctggcgacgctgcttcgcccggccgacgcctgca
gctgctccccggtgcacccgcaacaggcgttttgcaatgcagatgtagtgatcagggccaaagcggtcagtgagaaggaagtgg
actctggaaacgacatttatggcaaccctatcaagaggatccagtatgagatcaagcagataaagatgttcaaagggcctgaga
aggatatagagtttatctacacggcccccctcctcggcagtgtgtggggtctcgctggacgttggaggaaagaaggaatatctca
ttgcaggaaaggccgaggggggacggcaagatgcacatcaccctctgtgacttcatcgtgccctgggacaccctgagcaccaccc
agaagaagagcctgaaccacaggtaccagatgggctgcgagtgcaagatcacgcgctgccccatgatcccgtgctacatctcct
ccccggacgagtgcctctggatggactgggtcacagagaagaacatcaacgggcaccaggccaagttcttcgcctgcatcaaga
gaagtgacggctcctgtgcgtggtaccgcggcgcggcgcccccccaagcaggagtttctcgacatcgaggacccataagcaggcc
tccaacgcccctgtggccaactgcaaaaaaagcctccaagggtttcgactggtccagctctgacatcccttcctggaaacagca
tgaataaaacactcatccccggaattc
<212> Type : DNA
<211> Length : 1035
      SequenceName : M32304
      SequenceDescription :

Sequence
--------
<213> OrganismName : HUMAN
<400> PreSequenceString :
ctttggggttcaagatcactgggaccaggccgtgatctctatgcccgagtctcaaccctcaactgtcaccccaaagcacttggg
acgtcctggacagaccgagtcccgggaagccccagcactgccgctgccacactgccctgagcccaaatgggggagtgagaggcc
atagctgtctggcatgggcctctccaccgtgcctgacctgctgctgccgctggtgctcctggagctgttggtgggaatataccc
ctcaggggttattggactggtccctcacctaggggacagggagaagagagatagtgtgtgtccccaaggaaaatatatccaccc
tcaaaataattcgatttgctgtaccaagtgccacaaaggaacctacttgtacaatgactgtccaggcccggggcaggatacgga
ctgcagggagtgtgagagcggctccttcaccgcttcagaaaaccacctcagacactgcctcagctgctccaaatgccgaaagga
aatgggtcaggtggagatctcttcttgcacagtggaccgggacaccgtgtgtggctgcaggaagaaccagtaccggcattattg
gagtgaaaaccttttccagtgcttcaattgcagcctctgcctcaatgggaccgtgcacctctcctgccaggagaaacagaacac

cgtgtgcacctgccatgcaggtttctttctaagagaaaacgagtgtgtctcctgtagtaactgtaagaaaagcctggagtgcac
gaagttgtgcctaccccagattgagaatgttaagggcactgaggactcaggcaccacagtgctgttgcccctggtcattttctt
tggtctttgccttttatccctcctcttcattggtttaatgtatcgctaccaacggtggaagtccaagctctactccattgtttg
tgggaaatcgacacctgaaaaagagggggagcttgaaggaactactactaagcccctggccccaaacccaagcttcagtcccac
tccaggcttcacccccaccctgggcttcagtcccgtgcccagttccaccttcacctccagctccacctataccccggtgactg
tcccaactttgcggctccccgcagagaggtggcaccaccctatcaggggctgaccccatccttgcgacagccctcgcctccga
ccccatccccaaccccttcagaagtgggaggacagcgcccacaagccacagagcctagacactgatgaccccgcgacgctgta
cgccgtggtggagaacgtgcccccgttgcgctggaaggaattcgtgcggcgcctagggctgagcgaccacgagatcgatcggct
ggagctgcagaacgggcgctgcctgcgcgaggcgcaatacagcatgctggcgacctggaggcggcgcacgccgcggcgcgaggc
cacgctggagctgctgggacgcgtgctccgcgacatggacctgctgggctgcctggaggacatcgaggaggcgctttgcggccc
cgccgccctcccgcccgcgcccagtcttctcagatgaggctgcgccctgcgggcagctctaaggaccgtcctgcgagatcgcct
tccaaccccacttttttctggaaaggaggggtcctgcaggggcaagcaggagctagcagccgcctacttggtgctaacccctcg
atgtacatagcttttctcagctgcctgcgcgccgccgacagtcagcgctgtgcgcgcggagagaggtgcgccgtgggctcaaga
gcctgagtgggtggtttgcgaggatgagggacgctatgcctcatgcccgttttgggtgtcctcaccagcaaggctgctcggggg
cccctggttcgtccctgagcctttttcacagtgcataagcagttttttttgttttgttttgttttgttttgtttttaaatcaa
tcatgttacactaatagaaacttggcactcctgtgccctctgcctggacaagcacatagcaagctgaactgtcctaaggcaggg
gcgagcacggaacaatggggccttcagctggagctgtggactttgtacatacactaaaattctgaagtta
<212> Type : DNA
<211> Length : 2087
      SequenceName : M33294
      SequenceDescription :

Sequence
--------
<213> OrganismName : HUMAN
<400> PreSequenceString :
cgcatctgcacctgcaggcccggctggtactgcgcgctgagcaagcaggaggggtgccggctgtgcgcgccgctgccgaagtgc
cgcccgggcttcggcgtggccagaccaggaactgaaacatcagacgtggtgtgcaagccctgtgccccggggacgttctccaac
acgacttcatccacggatatttgcaggccccaccagatctgtaacgtggtggccatccctgggaatgcaagcagggatgcagtc
tgcacgtccacgtcccccacccggagtatggccccaggggcagtacacttaccccagccagtgtccacacgatcccaacacacg
cagccaactccagaacccagcactgctccaagcacctccttcctgctcccaatgggccccagcccccagctgaagggagcact
ggcgacttcgctcttccagttggactgattgtgggtgtgacagccttgggtctactaataataggagtggtgaactgtgtcatc
atgacccaggtgaaaaagaagcccttgtgcctgcagagagaagccaaggtgcctcacttgcctgccgataaggcccggggtaca
cagggccccgagcagcagcacctgctgatcacagcgccgagctccagcagcagctccctggagagctcggccagtgcgttggac
agaagggcgcccactcggaaccagccacaggcaccaggcgtggaggccagtggggccggggaggcgaggaccagcaccgggagc
tcagattcttcccctggtggccatgggacccaggtcaatgtcacctgcatcgtgaacgtctgtagcagctctgaccacagctca
cagtgctcctcccaagccagctccacaatgggagacacagattccagcccctcggagtccccgaaggacgagcaggtccccttc
tccaaggaggaatgtgcctttcggtcacagctggagacgccagagaccctgctggggagcaccgaagagaagcccctgcccctt
ggagtgcctgatgctgggatgaagcccagttaaccaggccggtgtgggctgtgtcgtagccaaggtgggctg
<212> Type : DNA
<211> Length : 1080
      SequenceName : M35857
      SequenceDescription :

Sequence
--------
<213> OrganismName : HUMAN
<400> PreSequenceString :
gagaaatttttcattttctttcttgtttgactgtatgcaaacatcaaactaagaatggcaaagatgaatctctcttcctatata
ttaatactaacttttctttgttttctcaaggtatttactttcagcatccaagtccataagaaatttagatgatgacatggta
tttaatacattcaggttggggaaaggctttcagaaggaagacactgcagaaaaatcagttattgctccttccctggaacaatat
aaaaatgatgagagcagtttcatgaacgaagaggaaaataaagtttcaaagaacacaggctccaaacataatttcttaaatcat
ggtctgccactcaatctggctataaaggatatcaagcactaaaaggatctgtagatttcccagctgagaatggagttcagaat
actgaatcaacacaagaaaagagagaaattggggatgaagaaaactcagctaaatttcctataggaaggagagattttgacatg
ctcagatgtatgctcggaagagtctaccgaccctgttggcaagtctgatacctgttggtccacatcatcttttcagaagaaaat
aaaagcatttaattgccaatgggaggagaagcccatactgctactataacttgtgtatgttaaatgtctgttttaaaagaaagt
agtgttaaatgtatcagtaactgaaatgatatgctttctctgtgcattaaatttgtgaaaattctgcat
<212> Type : DNA
<211> Length : 741
      SequenceName : M57703
      SequenceDescription :

Sequence

--------
<213> OrganismName : HUMAN
<400> PreSequenceString :
gctcagggcacatgcctcccctccccaggccgcggcccagctgaccctcggggctcccccggcagcggacagggaagggttaaa
ggcccccggctccctgcccccctgccctggggaacccctggccctgtggggacatgaactgtgtttgccgcctggtcctggtcgt
gctgagcctgtggccagatacagctgtcgcccctgggccaccacctggccccctcgagtttccccagaccctcggccgagct
ggacagcaccgtgctcctgacccgctctctcctggcggacacgcggcagctggctgcacagctgagggacaaattcccagctga
cggggaccacaacctggattccctgcccaccctggccatgagtgcgggggcactgggagctctacagctcccaggtgtgctgac
aaggctgcgagcggacctactgtcctacctgcggcacgtgcagtggctgcgccgggcaggtggctcttccctgaagaccctgga
gcccgagctgggcaccctgcaggcccgactggaccggctgctgcgccggctgcagctcctgatgtcccgcctggccctgcccca
gccacccccggacccgccggcgcccccgctggcgccccctcctcagcctgggggggcatcagggccgcccacgccatcctggg
ggggctgcacctgacacttgactggccgtgaggggactgctgctgctgaagactcggctgtgacccggggcccaaagccacca
ccgtccttccaaagccagatcttatttatttatttatttcagtactgggggcgaaacagccaggtgatcccccgccattatct
ccccctagttagagacagtccttccgtgaggcctgggggacatctgtgccttatttatacttatttatttcaggagcaggggtg
ggaggcaggtggactcctgggtccccgaggaggaggggactggggtcccggattcttgggtctccaagaagtctgtccacagac
ttctgccctggctcttccccatctaggcctgggcaggaacatatattatttatttaagcaattacttttcatgttggggtgggg
acggaggggaaagggaagcctgggttttgtacaaaaatgtgagaaacctttgtgagacagagaacagggaattaaatgtgtca
tacatatcc
<212> Type : DNA
<211> Length : 1185
SequenceName : M57765
SequenceDescription :

Sequence
--------
<213> OrganismName : HUMAN
<400> PreSequenceString :
gaattcccgccattgccgccatcgtcgtggggcttctggggcagctagggctggccgcgctgcctgcgccggaccggggcgggt
ccagtcccgggcgggccgtcgcgggagagaaataacatctgctttgctgccgagctcagaggagaccccagacccctcccgcag
ccagagggctggagcctgctcagaggtgctttgaagatgccggaggccccgcctctgctgttggcagctgtgttgctgggcctg
gtgctgctggtggtgctgctgctgcttctgaggcactggggctggggcctgtcgcttatcggctggaacgagttcatcctgcag
cccatccacaacctgctcatgggtgacaccaaggagcagcgcatcctgaaccatgtgctgcagcatgcggagcccgggaacgca
cagagcgtgctggaggccattgacacctactgcgagcagaaggagtggccatgaacgtgggcgacaagaaaggcaagatcgtg
gacgccgtgattcaggagcaccagccctccgtgctgctggagctgggggcctactgtggctactcagctgtgcgcatggcccgc
ctgctgtcaccaggggcgaggctcatcaccatcgagatcaaccccgactgtgccgccatcacccagcggatggtggatttcgct
ggcatgaaggacaaggtcacccttgtggttggagcgtcccaggacatcatccccagctgaagaagaagtatgatgtggacaca
ctggacatggtcttcctcgaccactggaaggaccggtacctgccggacacgcttctcttggaggaatgtggcctgctgcggaag
gggacagtgctactggctgacaacgtgatctgcccaggtgcgccagacttcctagcacacgtgcgcgggagcagctgctttgag
tgcacacactaccaatcgttcctggaatacagggaggtggtggacggcctggagaaggccatctacaagggcccaggcagcgaa
gcagggccctgactgcccccccggcccccctctcgggctctctcacccagcctggtactgaaggtgccagacgtgctcctgct
gaccttctgcggctccgggctgtgtcctaaatgcaaagcacacctcgccgagcctgcgccctgacatgctaacctctctgaact
gcaacactggattgttctttttaagactc
<212> Type : DNA
<211> Length : 1206
SequenceName : M58525
SequenceDescription :

Sequence
--------
<213> OrganismName : HUMAN
<400> PreSequenceString :
tgccttgaccaggacttgggactttgcgaaaggatcgcggggcccggagaggtgttggagagcacaatggctgaacaagtcctt
cctcaggctttgtatttgagcaatatgcggaaagctgtgaagatacgggagagaactccagaagacattttaaacctactaat
gggatcattcatcattttaaaaccatgcaccgatacacactggaaatgttcagaacttgccagttttgtcctcagtttcgggag
atcatccacaaagccctcatcgacagaaacatccaggccaccctggaaagccagaagaaactcaactggtgtcgagaagtccgg
aagcttgtggcgctgaaaacgaacggtgacggcaattgcctcatgcatgccacttctcagtacatgtggggcgttcaggacaca
gacttggtactgaggaaggcgctgttcagcacgctcaaggaaacagacacacgcaactttaaattccgctggcaactggagtct
ctcaaatctcaggaatttgttgaaacggggctttgctatgatactcggaactggaatgatgaatgggacaatcttatcaaaatg
gcttccacagacacacccatggcccgaagtggacttcagtacaactcactggaagaaatacacatatttgtcctttgcaacatc
ctcagaaggccaatcattgtcatttcagacaaaatgctaagaagtttggaatcaggttccaatttcgcccctttgaaagtgggt
ggaatttacttgcctctccactggcctgcccaggaatgctacagataccccattgttctcggctatgacagccatcattttgta
cccttggtgaccctgaaggacagtgggcctgaaatccgagctgttccacttgttaacagagaccggggaagatttgaagactta
aaagttcacttttttgacagatcctgaaaatgagatgaaggagaagctcttaaaagagtacttaatggtgatagaaatccccgtc

caaggctgggaccatggcacaactcatctcatcaatgccgcaaagttggatgaagctaacttaccaaaagaatcaatctggta
gatgattactttgaacttgttcagcatgagtacaagaaatggcaggaaaacagcgagcaggggaggagagaggggcacgcccag
aatcccatggaaccttccgtgccccagctttctctcatggatgtaaaatgtgaaacgcccaactgccccttcttcatgtctgtg
aacacccagcctttatgccatgagtgctcagagaggcggcaaaagaatcaaaacaaactcccaaagctgaactccaagccgggc
cctgaggggctccctggcatggcgctcggggcctctcggggagaagcctatgagcccttggcgtggaaccctgaggagtccact
ggggggcctcattcggccccaccgacagcacccagcccttttctgttcagtgagaccactgccatgaagtgcaggagccccggc
tgccccttcacactgaatgtgcagcacaacggattttgtgaacgttgccacaacgcccggcaacttcacgccagccacgcccca
gaccacacaaggcacttggatcccgggaagtgccaagcctgcctccaggatgttaccaggacatttaatgggatctgcagtact
tgcttcaaaaggactacagcagaggcctcctccagcctcagcaccagcctccctccttcctgtcaccagcgttccaagtcagat
ccctcgcggctcgtccggagcccctccccgcattcttgccacagagctggaaacgacgcccctgctggctgcctgtctcaagct
gcacggactcctggggacaggacggggacgagcaagtgcagaaaagccggctgcgtgtattttggactccagaaaacaagggc
ttttgcacactgtgtttcatcgagtacagagaaaacaaacattttgctgctgcctcagggaaagtcagtcccacagcgtccagg
ttccagaacaccattccgtgcctggggagggaatgcggcacccttggaagcaccatgtttgaaggatactgccagaagtgtttc
attgaagctcagaatcagagatttcatgaggccaaaaggacagaagagcaactgagatcgagccagcgcagagatgtgcctcga
accacacaaagcacctcaaggcccaagtgcgcccgggcctcctgcaagaacatcctggcctgccgcagcgaggagctctgcatg
gagtgtcagcatcccaaccagaggatggccctggggcccaccggggtgagcctgcccccgaagacccccccaagcagcgttgc
cgggcccccgcctgtgatcatttggcaatgccaagtgcaacggctactgcaacgaatgctttcagttcaagcagatgtatggc
taaccggaaacaggtgggtcacctcctgcaagaagtggggcctcgagctgtcagtcatcatggtgctatcctctgaacccctca
gctgccactgcaacagtgggcttaagggtgtctgagcaggagaggaaagataagctcttcgtggtgcccacgatgctcaggttt
ggtaacccgggagtgttcccaggtggccttagaaagcaaagcttgtaactggcaagggatgatgtcagattcagcccaaggttc
ctcctctcctaccaagcaggaggccaggaacttctttggacttggaaggtgtgcggggactggccgaggcccctgcaccctgcg
catcaggactgcttcatcgtcttggctgagaaagggaaaagacacacaagtcgcgtgggttggagaagccagagccattccacc
tccccctcccccagcatctctcagagatgtgaagccagatcctcatggcagcgaggccctctgcaagaagctcaaggaagctcag
ggaaaatggacgtattcagagagtgtttgtagttcatggtttttccctacctgcccggttcctttcctgaggacccggcagaaa
tgcagaaccatccatggactgtgattctgaggctgctgagactgaacatgttcacattgacagaaaaacaagctgctctttata
atatgcaccttttaaaaaattagaatatttactgggaagacgtgtaactctttgggttattactgtctttacttctaaagaag
ttagcttgaactgaggagtaaaagtgtgtacatatataatataccccttacattatgtatgagggatttttttaaattatattga
aatgctgccctagaagtacaataggaaggctaaataataataacctgttttctggttgttgttggggcatgagcttgtgtatac
actgcttgcataaactcaaccagctgcctttttaaagggagctctagtcctttttgtgtaattcactttatttattttattaca
aacttcaagattatttaagtgaagatatttcttcagctctggggaaaatgccacagtgttctcctgagagaacatccttgcttt
gagtcaggctgtgggcaagttcctgaccacagggagtaaattggcctctttgatacacttttgcttgcctccccaggaaagaag
gaattgcatccaaggtatacatacatattcatcgatgtttcgtgcttctccttatgaaactccagctatgtaataaaaaactat
actctgtgttctgttaatgcctctgagtgtcctacctccttggagatgagatagggaaggagcagggatgagactggcaatggt
cacagggaaagatgtggccttttgtgatggttttatttctgttaacactgtgtcctgggggggctgggaagtcccctgcatcc
catggtaccctggtattgggacagcaaaagccagtaaccatgagtatgaggaaatctctttctgttgctggcttacagtttctc
tgtgtgctttgtggttgctgtcatatttgctctagaagaaaaaaaaaaaaggaggggaaatgcattttccccagagataaaggc
tgccattttgggggtctgtacttatggcctgaaaatatttgtgatccataactctacacagcctttactcatactattaggcac
actttccccttagagcccccctaagtttttcccagacgaatctttataatttcctttccaaagataccaaataaacttcagtgtt
ttcatctaattctcttaaagttgatatcttaatatttgtgttgatcattatttccattcttaatgtgaaaaaaagtaattatt
tatacttattataaaaagtatttgaaatttgcacatttaattgtccctaatagaaagccacctattctttgttggatttcttca
agtttttctaaataaatgtaacttttcacaagagtcaacattaaaaaataaattattt
<212> Type : DNA
<211> Length : 4426
      SequenceName : M59465
      SequenceDescription :

Sequence
--------
<213> OrganismName : HUMAN
<400> PreSequenceString :
ggggggaaggccaagcagtgaccaggaagagggagaaacttctgctcagaccgagcgtgtgccactggacctcagtcctcgcct
ggaggagacgctggacttcggcgaggtggaagtggagcccagtgaggtggaagacttcgaggctcgtgggagccgcttctccaa
gtctgctgatgagagacagcgcatgctggtcgcagcgtaaggacgaactcctccagcaagctcgcaaacgtttcttgaacaaaa
gttctgaagatgatgcggcctcagagagcttcctcccctcggaaggtgcgtcctctgaccccgtgaccctgcgtcgaaggatgc
tggctgccgcgcggaacggaggcttcagaagcagcagacctcctagcgctcccttgccttcctcagctgcctcctgcgccctgt
gcccgactgactggaggaggcctgtcccaattctgccgctccatggaaaagcgggcttgactgcattgccgctgtataaagcat
gtggtcttatagtgtttggacagctgataaatttaatccttctttgtaatactttctatgtgacatttctcttccccttagaaa
cactgcaaattttaactgtaggtatgatctcttctggtgttgactggactgcttggggtgggggacgatcaggaggaagtgagc
cagtcgcctgcctgcagcaggcagcttctactcctgcctcatgcatacgtcccacaaatgcaggtgtcctgagcaccacaccca
gtgggaagagtgtgggggaggcgcacagtgtgagcccgcccccacgtcgtggggtaacatctgttatcaaactgctgtcgttgt
tgtggaagcatgtagactgtgccagagccagacccacgggctcatgcacccctgagcagcagggcatcttggaaaaggaactct
tggttcgatacctggagcagaggaggggaaagtccagggctatagggtgtgatgaagtcacccctttctgtcccactacatctg
ggactgactttccgagcctccagtccaaagccggcttgatttccgtgaactctggtgctcctgcatctcatgagtgtgccccat

gggtcccctcccctctcagcatttccttgtcccgtctggacctggggagtggttaggcagcaagctttggtttatggttttcat
tcattggtgaagtaaattaggcagtgctaaagcctgtgggtttggtccttgaacaagatgtgggccttgcaagatgggagagta
aaccttgaagggctttattaaagaaataaaaaagaacttttgtatcttttatcctgggagcactgcgttttcctagctgtgtta
ttcctggtttaattcagcagagaaggtaaggtgtgaacctacctgccttggagaggcccaggtcccaaatctcttcaaattctt
cacatgtttaactttaaggatttgaaccatgaagtcataggttacagacctcagttttatgccccattggattactttttttt
tttttttttttttttactctttgaaagctttgttttgtggtagtcgcttttgggaagaatccagtattatctacaattattggc
aaagtttaaatgtatttacataacggaaagtttttagaatgttgaaaagtaattgaaaaaggtgataggtaaattttaggca
aagataatttatttcaataaatctttcaaaagccttaccttgaaatgctgttagtaaatttctgtgcatttttttttttaatt
tgttttgctgagagcatagctatttgtttttattgtaaacccgccc
<212> Type : DNA
<211> Length : 1810
      SequenceName : M63175
      SequenceDescription :

Sequence
--------
<213> OrganismName : HUMAN
<400> PreSequenceString :
gcgaccgctctcctacccggacaccgacgtcattctcatgtgcttctcggtggacagcccggactcgctggagaacatccccga
gaagtgggtccccgaggtgaagcacttctgtcccaatgtgcccatcatcctggtggccaacaanaaagacctgcgcaggacgag
catgtccgcacagagctggcccgcatgaagcaggaacccgtgcgcacggatgacggccgcgcatggccgtgcgcatccaagcct
acgactacctcgagtgctctgccaagaccaaggaaggcgtgcgcgaggtcttcgagacggccacgcgcgccgnntgcaagaaag
cgttacggctcccagaacggctgcatcaactgctgcaaggtgctatgagggccgcgc
<212> Type : DNA
<211> Length : 393
      SequenceName : N41062
      SequenceDescription :

Sequence
--------
<213> OrganismName : HUMAN
<400> PreSequenceString :
ttttcatagcagcttcttgtccagctttattgggaggccagcttgcctggagggggccaggcatgaggtggggtaggagagca
ctgagaatactgtccctttttaagcaacctacaggggaagccctggagattgcaggacccaaggagctggcaggatggataggca
ggtggacttggggtattgaggtctcaggcagccacggctgaaagttggtctgacctcagggtccaaatcccagaactcagagaa
cttgtccttaacggtgctccagtagtctttcagggaactgaagccatcggtcacccagcccctggctgcttgggcacctgggac
tcctgcacgctgctcagtgcatccttggcggtcttggtggcgtgcttcatgtagccctgcatgaagctgagaagggaggcatcc
tcggcctctgaagctcgggcagaggcaggagcgccaggagggcaacaacaaggagta
<212> Type : DNA
<211> Length : 477
      SequenceName : N53169
      SequenceDescription :

Sequence
--------
<213> OrganismName : HUMAN
<400> PreSequenceString :
tttttttcgcattcaacttaaatgcttttattgacaatgtcttggaacaataagcaaacaatgcttaaattttcattcaaatt
cactttccacatgtcaaaagacctcaaggtagaaaaaaataaaataaaaatataaatatctgagaatccatcttaataaataaa
ttaaaaacacaataaaacgttttcatggaaaactgttaatgtcagaacattcagaccacctcaacaatgcatgatcagtaacat
tacaatgaacattgatgttgaagaaaaactacagtacatggatatagctatttatttctatctaccagaaaataaagtcgtatc
ttttcttagtataatattgggtcatttctaatcagaacacactattgccaggaacacagtagttattgttaaaatcagccgcac
tagataccatttggaaatatccagcaccaggttaattcccataatgnaccccatagg
<212> Type : DNA
<211> Length : 477
      SequenceName : N67917
      SequenceDescription :

Sequence
--------
<213> OrganismName : HUMAN
<400> PreSequenceString :
ntttttcagttacacttttccttgctttaatatttgttgtcttttctgcttttgaaatgagctctcaattacaaaataccaa

actacactcaaaagagggtgtagaaaaagccagccattggaccagcatggagaagcccacaagtaaggacagatagcagcagca
ggtggcaagaggtgggaggtgactgaagcagtggttacaaatgttcacaggaagctaaaactatccaacaggttgtgccctaat
gttagcacaggttagactagttactaccttgctaggtggcctcagtgtgttcagattctacgcagcaatacaatttcttccagt
taagagttcttgacttgcaccgatcacagcatggttgaagggtactgggaccagtcttgttagtttttcaaaatgctgtcgtag
anctggatagatgtattgagagacttcaggagctctacacttcagtgacag
<212> Type : DNA
<211> Length : 471
      SequenceName : N72918
      SequenceDescription :

Sequence
--------
<213> OrganismName : HUMAN
<400> PreSequenceString :
agcacattcaatccaatttggacttctctccagtaaacagtgcttctagtgaagaaaatgtgaagtactccagttctcagccag
aaccccgcacaggtctttccttatgggataccagcccctcatacattgataaattggtacaagggatcagcttttcccagccca
catgtcctgatcatatgcttttgaatagtcagttacttggcaccccaggatcctcacagaancctgggacncggtgttggt
<212> Type : DNA
<211> Length : 249
      SequenceName : N73242
      SequenceDescription :

Sequence
--------
<213> OrganismName : HUMAN
<400> PreSequenceString :
ctctccttggaggacctttaggtaaactccctagtctccacctgagaccctgggagagaagtttgaagcacaactcccttaagg
tctccaaaccagaccggtgacgcctgcgggaccatctggggcacctgcttccacccgtctctctgcccactcgggtctgcagaa
ctggtttccaattgaggccctttgcaggatggaantacggggcttacaggagcttttgtgtgcctggtagaaactatttctgtt
ccagtcacattgccatcactcttgtactgcctgccaccgcggaggaggctggtgacaggccaaagnaggttggaagaaacatcc
tttcatctcagagtccactgt
<212> Type : DNA
<211> Length : 357
      SequenceName : N75719
      SequenceDescription :

Sequence
--------
<213> OrganismName : HUMAN
<400> PreSequenceString :
gcgtggtgaccatcatcaaggggaaggtggaggaggtggagctcccagtggagaaggtggacatcatcatcagcgagtggatgg
gctactgctcttctacgagtccatgctcaacaccgtgctctatgcccgggacaagtggctggcgccgatggcctcatcttccca
gaccggccacgctgtatgtgacggccatcgaggaccggcagtacaaagactacaagatccactggtgggagaacgtgtatggc
ttcgacatgtcttgcatcaaagatgtggccattaaggagcccctagtggatgtcgtggaccccaaacagctggtcaccaacgcc
ttgccttcataaaggaggttggacatctataccgtcaaggtggaagaccttgacctttcanctcccccgtttctgngccttgca
agttgaanccggaattgacttacgtgcacggccctggtgggctactttcaacatcgaggttnacacgctggcaaa
<212> Type : DNA
<211> Length : 495
      SequenceName : N76967
      SequenceDescription :

Sequence
--------
<213> OrganismName : HUMAN
<400> PreSequenceString :
gtgtaaaattgttaaccttgtaagatggataatgtgtataagaatgtaggccttaactatttcacatgagtcaaaacaaagcag
ctttaaaaaaataattggaagcacaagtgcatggcntgactgaatgctgttaatatttctaaaagtttccacantccagntaat
atggcctgattcatagtaaaatacctctaataaacactgttttatagaaaacctgacttcagtgaatattttgtattttacat
gggccagtttatatactgctactttacactattatttcctatagctacatgttctttgtaccttttgtagttttatttgtatta
ctagattcataccttgatggtaacgctctatctggttttgggtgtttttcatgttttagcat
<212> Type : DNA
<211> Length : 398
      SequenceName : N77754

SequenceDescription :

Sequence
--------
<213> OrganismName : HUMAN
<400> PreSequenceString :
ttattttcaagattacatttaagctctagaatctcattccctttttctcttccaagagccagaagttcatcagttttggttttc
aactctgtattcagccatgtattctgactatgtagcaattcctttttcttgctccaagcgtttttctcgatacttaacagaaaca
tcagaagcttgaagttcatccaatttaactgaagttcaccctttgttgtattgctttctttaagtttttcattcagacgttta
acatcctctgttaagtattcaagttcttgagatagtctctcattgggttctaattaagtctcttttctcagcttctaattcttc
ctttgttcttgtaaattggctctgaatgggcaatattgcgatcctgaggcaatttcaagttctttgtttttctcaggtaggggc
cttcag
<212> Type : DNA
<211> Length : 426
SequenceName : R11490
SequenceDescription :

Sequence
--------
<213> OrganismName : HUMAN
<400> PreSequenceString :
aagtcttcctaaattttattggcactgaaatagaacatggattgagcataaaccactccaaaaacaatttttaaaaaacccaa
aaagtacacaaaaaacccctgantacaaaatctaacctttcccccagcctccctaagggtaagttactgactttaaggcagct
attaatagattgccccacaattccagggnttcaatttaggccaatatagggacatatcaccaagtgaggctaattcacaggcaa
tgcacacaagactcctcaagggcanggccagagtggggggttgttggcngggggggatttggggngggtttta
<212> Type : DNA
<211> Length : 328
SequenceName : R22977
SequenceDescription :

Sequence
--------
<213> OrganismName : HUMAN
<400> PreSequenceString :
gtaccntgatcaagtttattgcatgcctttcttttcctatttctttttttaagggttagtattaacaaatggcaatgagtagaa
aagttaacatgaagattttagaaggagagaacttacaggacacagatttgtgattctttgactgtgacactattggatgtgatt
ctaaaagctttattgagcattgtcaaatttgtaagcttcataggggatgggncatccatatctataatgccccttcctatatg
gtgcctacccataggatgtggccatttttgnccccttaatatccggnctt
<212> Type : DNA
<211> Length : 302
SequenceName : R26434
SequenceDescription :

Sequence
--------
<213> OrganismName : HUMAN
<400> PreSequenceString :
tttttttttttttttttttttttaccaggggngaaacaattttatttcacanttgtctttaaaaccacaagacacctatgttc
ttcanataaaaacattagtatagatacccatactttctagaaaatgattatacagaccctttcaaagaaaaatgtattccatta
tataggnttcagtgaggttgcttggggaaaaactcacatctggggctgattcctaaaacat
<212> Type : DNA
<211> Length : 229
SequenceName : R27585
SequenceDescription :

Sequence
--------
<213> OrganismName : HUMAN
<400> PreSequenceString :
ggatctggtctcccacaatgaaggtcttgcctccctggttctgggacagcagggtctcaaaaggcttcagttgcccgggcagtg
cttcacatagtcatccttgcccgcctcatagttggtgtagatgagggagatgtatttgcagcggaggtcctccacgccgtcatt
caccatgtccaccagggctgcctcctgctggtccttcccatagagcccaagggtgcgggcccagggtgacgcaggatggtattg
gactggtacagggtgaggtctccgtcctgggaacttnggggagctgcccgtattaggcanggaggcttttgagttgagccctcc

ttncggccgcaagcttatttcccttttagttgagggttaantttaagtttggcaattggccttcttttaaaaacttcgtgatt
tgggaaaanctgggntttaaccaattta
<212> Type : DNA
<211> Length : 448
SequenceName : R33755
SequenceDescription :

Sequence
--------
<213> OrganismName : HUMAN
<400> PreSequenceString :
ttttttttgtgtgcaagtgtttatttggaatcccttctatttattagaaacagaaacagtaatttcaccagtaggaattgcg
tgtgctctcaatacaagtaagtttgccactccttcaattgttgtccattgcagacactttggattcaaggttaagaatccaaat
gagaaataagaaatatccggtccctgatgattcgtttaagtcctgttcaactcgatggaaagcttccacccgaaggaaggagtt
actgttcctcctgggctgggctttgtgtttctttcagtgctctaaaggaactttgtatttggggcagctgtgctctggtcatgt
caggctggctgggacagggagtttggatggcttacgggcggccgctggaccgggggctggctttttacttgaaggcttcactg
ggggtgttccattcaattcacaaagtggggcgttntgcaggccngtggaagggttttgcngggggntt
<212> Type : DNA
<211> Length : 488
SequenceName : R43734
SequenceDescription :

Sequence
--------
<213> OrganismName : HUMAN
<400> PreSequenceString :
tttttttttgcttttataccactttattccaacctgagcacctcaatataaaactaaacactggtgaaccgttttcctaatt
ctgcattatcataaatgtacaagttctcctagcagtccaacacttaaatagattaaatcatctctgacacatggtagctttcat
ataatgaaaatacctaaaataattagttgcaatatactgnaactggatcaaaataaaatggaactttgggnaagggngggctgg
caggattgttacttaacatattggcaatactttatggttacaanttcgggggnccattgntttattttggggttctggccctgg
ggggggccacctgtagg
<212> Type : DNA
<211> Length : 353
SequenceName : R45525
SequenceDescription :

Sequence
--------
<213> OrganismName : HUMAN
<400> PreSequenceString :
ggacagaatttcatnattattggataaatacatgcaggcactgtgcttggcctggggctccaagttagcagacgtggttcccac
cttgagctcagtctttctgtgggggccactgcacaggcgtgatgggtcctgggagagggtntggagctagggcagggcagagct
gnagaagacagcctccaagggtggggatntcacgggcccgtgcccctnttttattttatttttccttgagacggagntcgnc
tgtgttgcccaggctggagtgcaatggcgcaatcttggctcactgcaacctccacctcccaggttcaagcaattctcctacctc
aacctcctgagtagctggggactacagggtntgtgccaccacgcctggatttgtattttagtagagatggggttttcgcca
gggtggnctttaactcccgaccccangggatccaccctncttgggctcccaaagtgttgggattaa
<212> Type : DNA
<211> Length : 487
SequenceName : R48796
SequenceDescription :

Sequence
--------
<213> OrganismName : HUMAN
<400> PreSequenceString :
caagagtagattctnttnttcatttctctttttttttcttaaaaaaaaaagtattcatttggtataaaaaataaatattttaaat
atgacattgantaaataaaaataatctgtcagtatgaaacatccccacagggtacattcatcaaagaggattttgtcacccaag
gccatgtgcttttcagtggaagggaaggagggaaacctctaaggccgcacggtgggcccacggagctagcacgtgggcggggac
tganggctggatgcccggnttgagggtggggaactagagatgactntaagggcagggaacatctgtaccntctgcagggaaatt
gctaccctccccgggtgccngaggttccaccccacaaantttgtctac
<212> Type : DNA
<211> Length : 384
SequenceName : R50354

SequenceDescription :

Sequence
--------
<213> OrganismName : HUMAN
<400> PreSequenceString :
gggtaggaaaggtccccagaagcatgggggctgagtcagtcagcaaatgcataaggcctaggcacagaggagcaggttagggca
cattcaccttntcaggattctgtggctccctcattggagaagggagagagcatcttgggggcgcaattccaagagcagcgaggg
cagaggttagagatggctgagagcccctaacctgaggcggcaccacantaggcagcaacaactntntggaaagctggatgaact
ggtcagtagcggaaantgggagggggcactgggttggcctnttggggaggggtccaaccttggcttggatgagctcatgagant
acccagttttcccaagcagagggggcagagcaaagcccttttntgcaggcctcctttaaggagaaaaagg
<212> Type : DNA
<211> Length : 409
SequenceName : R76314
SequenceDescription :

Sequence
--------
<213> OrganismName : HUMAN
<400> PreSequenceString :
ttaacaggagacaggggttttattattactcaaatcagcctccctgaaaatttggaggctagggtttttaaaggtagtttggc
gggcaggggttggaggtagagcaatgtcatttagcttgctcacttccatctgccagtttggnagcttcttggctganagatggc
gccgggcatgcttggtcaaatggtcactcctcatgaaccgccggtcacacatggggcacgcaaatttcttctcacccgtgtggg
ttcgcctgtgtctggacagttcancagaacgggcaaa
<212> Type : DNA
<211> Length : 289
SequenceName : R79935
SequenceDescription :

Sequence
--------
<213> OrganismName : HUMAN
<400> PreSequenceString :
nnnnnagggaaaaaaaatggaaaatttattaattagacagtatgtgggcatcctgtnccacatgggaatgagaagatgctatag
gtnctctaagtattgcacagtctgaaaaaataacaaaaaaagggaaggggaggaaaaaaaatcacatgatattggganccatc
tcacattatgantantctaccaagaaacatttaaaaaagaaancccctttgtttctacagtaggctttaagtttatagttcttgg
gantgactgtattccattgaaggacatctcaggtaacagggaaggctgttttaggcaatccccatgtggcaaatattaataaaa
natatatantttttgccaattcatctctngccttcaccccgggcaatcatgacatttncgag
<212> Type : DNA
<211> Length : 399
SequenceName : R83270
SequenceDescription :

Sequence
--------
<213> OrganismName : HUMAN
<400> PreSequenceString :
acctcagtctgacgacattgaagctagccgaatgaagcgagcagctgcaaagattctaatagaacgctactaccaccagttaac
tgagggctgtggaaatgaagcctgcacgaatgagttttgtgcttcctgtccaacttttcttcgtatggataataatgcagcagc
tattaaagccctcgagctttataagattaatgcaaaactctgtgatcctcatccctccaagaaaggagcaagctcagcttacct
tgagaactcgaaagggtgcccccaacaactcctgctctgaggataaaaatgaacaagaaaggcgctagaattgattttaaagat
gtgacttactttaacagaagngaagggtatatggaaattcttgaattatgtaggaggaaagagagggttatttcccctntaatc
cctgttctttgggaggagtttttcta
<212> Type : DNA
<211> Length : 447
SequenceName : R85213
SequenceDescription :

Sequence
--------
<213> OrganismName : HUMAN
<400> PreSequenceString :
gcacacgggaagatatcagaaacatcctaggatcaggacaccccagatcttctcaactggaaccacgaaggctgtttcttccac

acagcactttgatctccatttaagcaggcacctctgtcctgcgttccggagctgcgttcccgatggtcctcctttggctcacgc
tgctcctgatcgccctgccctgtctcctgcaaacgaaggaagatccaaacccaccaatcacgaacctaaggatgaaagcaaagg
ctcagcagttgacctgggaccttaacagaaatgtgaccgatatcgagtgtgttaaagatgccgactattctatgccggcagtga
acaatagctattgccagtttggagcaatttccttatgtgaagtgaccaactacaccgtccgagtggccaacccaccattctcca
cgtggatcctcttccctgagaacagtgggaagccttgggcaggtgcggagaatctgacctgctggattcatgacgtggatttct
tgagctgcagctgggcggtaggcccgggggcccccgcggacgtccagtacgacctgtacttgaacgttgccaacaggcgtcaac
agtacgagtgtcttcactacaaaacggatgctcagggaacacgtatcgggtgtcgtttcgatgacatctctcgactctccagcg
gttctcaaagttcccacatcctggtgcggggcaggagcgcagccttcggtatcccctgcacagataagtttgtcgtcttttcac
agattgagatattaactccacccaacatgactgcaaagtgtaataagacacattcctttatgcactggaaaatgagaagtcatt
tcaatcgcaaatttcgctatgagcttcagatacaaaagagaatgcagcctgtaatcacagaacaggtcagagacagaacctcct
tccagctactcaatcctggaacgtacacagtacaaataagagcccgggaaagagtgtatgaattcttgagcgcctggagcaccc
cccagcgcttcgagtgcgaccaggaggagggcgcaaacacacgtgcctggcggacgtcgctgctgatcgcgctggggacgctgc
tggccctggtctgtgtcttcgtgatctgcagaaggtatctggtgatgcagagactctttccccgcatccctcacatgaaagacc
ccatcggtgacagcttccaaaacgacaagctggtggtctgggaggcgggcaaagccggcctggaggagtgtctggtgactgaag
tacaggtcgtgcagaaaacttgagactggggttcagggcttgtgggggtctgcctcaatctccctggccgggccaggcgcctgc
acagactggctgctggacctgcgcacgcagcccaggaatggacattcctaacgggtggtgggcatgggagatgcctgtgtaatt
tcgtccgaagctgccaggaagaagaacagaac
<212> Type : DNA
<211> Length : 1460
      SequenceName : M74782
      SequenceDescription :

Sequence
--------
<213> OrganismName : HUMAN
<400> PreSequenceString :
atctgttgtcagcacagttttatttgctgtggaatccatgagagccggaagcatcgttggggccgtggctagcagagctcatgg
tgaccagtcctgggcctgaccaatgggtgattacatttaaaaaccaaaacaaaacaaaacaaaataccaagaacagatcacttg
ccatggacatcagtaatctattggtaatggtgaaaatttcatgaaaatttcccctaaaccataacaaaaactgtcctccttacc
ccaaaagtgctggagggaaagatggttgcatggctttgacctctctttgaacttgaaatgctaccttcctacccggaaaatgcg
gcacactatactt
<212> Type : DNA
<211> Length : 349
      SequenceName : M78706
      SequenceDescription :

Sequence
--------
<213> OrganismName : HUMAN
<400> PreSequenceString :
tggcatcctggtcctctcacatggctccttaaaattggctgtggcttgtttttgtgatgtttgcttggttgcctgttcccttt
tctacctgcagagcacggttcccataagggcggcgagatcagcctcctgtctcatctggaagaccaccactctggggtctcaga
ggaatgatggaagccttggggtttctaaaattggaagtgaatggccccatggtgacggtggccctgtcagtggctctcttggcc
ctcctgaaatggtactccacatcagcattctcaagactggagaagttaggcctcagacatcccaagccttctcctttcattgga
aacttgacattttttccgccagggtttttgggaaagccaaatggagctcagaaagctgtatggacctctgtgtgggtactatctt
ggtcgtcggatgtttattgttatttctgagccagacatgatcaagcaggtgttggttgagaacttcagtaactttaccaacaga
atggcgtcgggtttggagttcaagtcggtagccgacagcgttctgtttttacgtgacaaaagatgggaagaggtcagaggtgcc
ctgatgtctgctttcagtcctgaaaagctgaacgagatggttcccctcatcagccaagcctgcgaccttctcctggctcattta
aaacgctatgcggaatctggggacgcatttgacatccagaggtgctactgcaattacaccacagatgtggttgccagcgtcccg
tttggcaccccggtggactcctggcaggcccctgaggatccctttgtgaaacactgcaagcgtttcttcgaattctgcatcccc
agacctatcctggtttactcttatcatttccatccataatggtcccactggcccggattttgcccaataagaaccgagacgaa
ctgaatggctttttaacaaactcattaggaatgtgattgccttgcgggaccagcaagctgccgaagagaggcggagagacttc
ctccaaatggtcctggatgcccgacattctgcaagtcccatgggcgtgcaagactttgacatcgtcagagacgttttctcctct
actgggtgcaagccgaacccttcccggcaacaccagcccagccctatggccaggcctttgactgtggatgagattgtgggccag
gccttcatcttcctcatcgctggctatgaaatcatcaccaacacactttcttttgccacctacctactggccaccaaccctgac
tgccaagagaagcttctgagagaggtagacgtttttaaggagaaacacatggcccctgagttctgcagcctcgaggaaggcctg
ccctatctggacatggtgattgcagagacgctgaggatgtacccgccagctttcagattcacacgggaggcagctcaggactgc
gaggtgctggggcagcgcatccccgcaggcgctgtgctagagatggccgtgggtgccctgcaccatgaccctgagcactggcca
agcccggagaccttcaaccctgaaaggttcacggctgaggcccggcagcagcaccggcccttcacgtacctgcccttcggggcc
ggcccacggagctgcctcggggtgcgtctagggctgcttgaggtcaagttgacactgctccacgtgctgcacaagttccggttc
caagcctgccctgagacccaggtaccgctgcagctagaatccaaatctgccctaggtccaaaaaatggtgtctatatcaagatc
gtatcccgctgacacagaaggctgccgggtgggggagggcacccccaaattcaaagaaaccctaagtgtggatgttcagaat
tttggaaaaatgtcactgaagtgattgaaagccg

<212> Type : DNA
<211> Length : 1882
SequenceName : M80647
SequenceDescription :

Sequence
--------
<213> OrganismName : HUMAN
<400> PreSequenceString :
atggcggccttcagcaagtacttgacggcgcggaactcctcgctggctggtgccgcgttcctgctgctctgcctgctccacaag
cggcgccgcgccctcggcctgcacggtaagaaaagtggaaaaccaccattacagaacaatgagaaagaggggaaaaaagagcga
gctgtggtggacaaggtgtttttctcaaggctcatacagattctgaaaatcatggtccctagaacatttgtaaagagacaggt
tacttggtacttattgctgttatgctggtgtctcgaacatattgtgatgtttggatgattcaaaatgggacactaattgaaagt
ggtatcattggtcgtagcaggaaagatttcaagagatacttactcaacttcatcgctgccatgcctcttatctctctggttaat
aacttcttgaagtatgggttaaatgagcttaaactgtgcttccgagtaaggctcactaaatacctctatgaggagtatcttcaa
gctttcacatattataaaatggggaatctggacaacagaatagctaatccagaccagctgcttacacaagatgtagaaaaattt
tgtaacagtgtagtcgatctgtattcaaatcttagtaagccattttagacatagtttgtatatctttaagttaacgagtgca
attggagctcagggcccagcgagcatgatggcctacttggttgtttctgggctattcctaactcgacttcgaagacccattggt
aagatgacaataactgagcaaaagtatgaaggagaatatagatatgttaattctcggctcatcacaaacagtgaagaaattgcc
ttttacaatgggaataaaagagaaaagcagacagtccactcagtcttccgaaaactggtggaacacctacataatttcatttg
tttcggttttcaatgggcttcattgatagtattattgccaaataccttgccactgttgttggttacctagttgtcagtcgccct
ttcttagatttgtctcatcctcgacatctcaagagtacacattcggaacttctagaggattactaccaaagtggaagaatgctt
ttgcgaatgtctcaagctctgggtcgaatagttttggctgggcgtgaaatgactagattggccggttttactgctcggattaca
gaattaatgcaagtactgaaggatttaaatcatggcaaatatgagcgcacaatggtctcacaacaggaaaagggtattgaagga
gtacaagtcattcccttgatacctggtgctggagaaatcattattgcagataacattataaagtttgatcatgttcctttagca
acgccaaatggagatgttttgatccgagaccttaattttgaagttcgatctggggctaatgttctaatttgtggtccaaatggc
tgcggaaagagttcacttttccgtgttcttggtgaattatggcctctttttggaggacgtctaactaaacctgaaagaggaaaa
ttattttatgttcctcagagaccttacatgacccttggaacacttcgagatcaagtgatatatccagatggacgagaagatcag
aaaaggaagggaatttctgacctagtactgaaggaatacttagacaatgtccagttgggtcatatccttgaacgtgaaggaggc
tgggacagtgttcaggattggatggacgtactcagtggtggagaaaagcaaagaatggcgatggcaagattattttatcataaa
ccccagtttgccattttggatgaatgcacaagtgcagttagtgtcgacgtggaaggctacatttatagtcattgtcgaaaggtt
ggcatcactctcttcactgtgtctcataggaaatctctttggaaacatcatgagtactacctgcatatggatggcagaggcaac
tatgaattcaaacagataacagaagatacagttgagtttggctcttagagaaatctggagaactatacctgcttcagtgaaata
attacagaatatacttagaaaggcaaagtacattgtaaaataaagttgagcttagtttttttaaaaaaaaaaacaaagcaac
aaattaactagatacagaataatggagaacaagttgttaaaacatttaatattatataggatattgctaattgtgtatatgttg
gtttaattaataatatgtactaagaatgtccttattcttgtggttaaaaacctgcctaaattaaattgggcttcaatcactgta
acctgattcatcctgggatgtaaaccattcgaagtcagctaattggacttttatggctctatcttttccttcagtgaagaaccc
tatttaaaactgggtcatcatttgtcctgttctagcaagatagtcttcagtttcattttcctgtgccctgtggtagttggaaac
aaatcataatgtattatttaaatgtttaacatcattgcataacagcgtttattatacagtggcagatttctttagctgccacag
taatactcattccttgtgtgtgtcttggagtgcatttgactccaggaaaagccattttggttttccttaactaaatgataaatg
taccccctctcagtctgcagtattgagttgtttaaagtatatgtgcagtcttgcttacaaggaggggttaccatgtatcacacct
aatcttcccaatgtttgggatattaaaacacaaagtccttaacatgccaggctcaaggtcttataagagttctagattttaag
agaattagacaaatttgtgtgtgttagaagcccattcattagaagtgtggtggttatttggtattaaactccaaatgagccata
ggaaggcactacatgaaataatgcactgagtatgcaatgctatcactgtctttgactgtgatttatgtttaaaaagtatgttc
taaaattattatatatacatgggtgaattatgtttccgaggcactgttttatctctgtgaatcttgaataactttttatattt
gggttatgatgtcaaacgatcctaagcgaagatgatttcagttcatcaaatcatcattaatgactttatgtattatttgcacag
ggagaattgaaactgagtataatcaataagctagatacgaaatcagtttctcaaactgagcttcagaaaggggcattttgtact
cttgtttttgcataactggttttgtttttttgcagaattaactataacaatcactggctaccgaagtaaactgatgtactgaat
tccataatacataacattcaattttaccacttctgtttagcgaacttgtatacttattttctgttcagattaaaaaaaaaaa
aaaaaaaactcagatatcctataaaaaaaaaaggaat
<212> Type : DNA
<211> Length : 3397
SequenceName : M81182
SequenceDescription :

Sequence
--------
<213> OrganismName : HUMAN
<400> PreSequenceString :
attccaggagcctatcaacccagataaagcgggacctcctctggtagaggtgcagggggcagtactcaacatgatcacagag
ggagcgcaggcccctcgactgttgctgccgccgctgctcctgctgctcaccctgccagccacaggctcagaccccgtgctctgc
ttcacccagtatgaagaatcctccggcaagtgcaagggcctcctgggggggtggtgtcagcgtggaagactgctgtctcaacact
gcctttgcctaccagaaacgtagtggtgggctctgtcagccttgcaggtccccacgatggtccctgtggtccacatgggccccc

tgttcggtgacgtgctctgagggctcccagctgcggtaccggcgctgtgtgggctggaatgggcagtgctctggaaaggtggca
cctgggaccctggagtggcagctccaggcctgtgaggaccagcagtgctgtcctgagatgggcggctggtctggctgggggccc
tgggagccttgctctgtcacctgctccaaagggacccggacccgcaggcgagcctgtaatcaccctgctcccaagtgcgggggc
cactgcccaggacaggcacaggaatcagaggcctgtgacacccagcaggtctgccccacacacggggcctgggccacctggggc
ccctggacccccctgctcagcctcctgccacggtggaccccacgaacctaaggagacacgaagccgcaagtgttccgcacctgag
ccctcccagaaacctcctgggaagccctgcccggggctagcctacgagcagcggaggtgcaccggcctgccaccctgcccagtg
gctgggggctgggggccttggggccctgtgagcccctgccctgtgacctgtggcctgggccagaccatggaacaacggacgtgc
aatcaccctgtgccccagcatggggggccccttctgtgctggcgatgccacccggacccacatctgcaacacagctgtgccctgc
cctgtggatggggagtgggactcgtggggggagtggagcccctgtatccgacggaacatgaagtccatcagctgtcaagaaatc
ccgggccagcagtcacgcgggaggacctgcaggggccgcaagtttgacggacatcgatgtgccgggcaacagcaggatatccgg
cactgctacagcatccagcactgccccttgaaaggatcatggtcagagtggagtacctgggggctgtgcatgccccccctgtgga
cctaatcctacccgtgcccgccagcgcctctgcacacccttgctccccaagtacccgcccaccgtttccatggtcgaaggtcag
ggcgagaagaacgtgaccttctggggggagaccgctgccacggtgtgaggagctacaagggcagaagctggtggtggaggagaaa
cgaccatgtctacacgtgcctgcttgcaaagaccctgaggaagaggaactctaacacttctctcctccactctgagcccccctga
ccttccaaacctcaataaactagcctcttcg
<212> Type : DNA
<211> Length : 1543
      SequenceName : M83652
      SequenceDescription :

Sequence
--------
<213> OrganismName : HUMAN
<400> PreSequenceString :
gccgctgtgtaggaaagaagctaaagcacttccagagcctgtccggagctcagaggttcggaagacttatcgaccatggagcgc
gcgtcctgcttgttgctgctgctgctgccgctggtgcacgtctctgcgaccacgccagaaccttgtgagctggacgatgaagat
ttccgctgcgtctgcaacttctccgaacctcagcccgactggtccgaagccttccagtgtgtgtctgcagtagaggtggagatc
catgccggcggtctcaacctagagccgtttctaaagcgcgtcgatgcggacgccgacccgcggcagtatgctgacacggtcaag
gctctccgcgtgcggcggctcacagtgggagccgcacaggttcctgctcagctactggtaggcgccctgcgtgtgctagcgtac
tcccgcctcaaggaactgacgctcgaggacctaaagataaccggcaccatgcctccgctgcctctggaagccacaggacttgca
ctttccagcttgcgcctacgcaacgtgtcgtgggcgacagggcgttcttggctcgccgagctgcagcagtggctcaagccaggc
ctcaaggtactgagcattgcccaagcacactcgcctgccttttcctgcgaacaggttcgcgccttcccggcccttaccagccta
gacctgtctgacaatcctggactgggcgaacgcggactgatggcggctctctgtccccacaagttcccggccatccagaatcta
gcgctgcgcaacacaggaatggagacgcccacaggcgtgtgcgccgcactggcggcggcaggtgtgcagccccacagcctagac
ctcagccacaactcgctgcgcgccaccgtaaaccctagcgctccgagatgcatgtggtccagcgccctgaactccctcaatctg
tcgttcgctgggctggaacaggtgcctaaaggactgccagccaagctcagagtgctcgatctcagctgcaacagactgaacagg
gcgccgcagcctgacgagctgcccgaggtggataacctgacactggacgggaatcccttcctggtccctggaactgccctcccc
cacgagggctcaatgaactccggcgtggtcccagcctgtgcacgttcgaccctgtcggtgggggtgtcgggaaccctggtgctg
ctccaaggggcccggggctttgcctaagatccaagacagaataatgaatggactcaaactgccttggcttcaggggagtcccgt
caggacgttgaggacttttcgaccaattcaaccctttgccccacctttattaaaatcttaaacaacg
<212> Type : DNA
<211> Length : 1327
      SequenceName : M86511
      SequenceDescription :

Sequence
--------
<213> OrganismName : HUMAN
<400> PreSequenceString :
gccgcctccgcctctctctttactgcggcgcggggcaaggtgtgcgggcgggaaggggcacgggcacccccgcggtccccggga
ggctagagatcatggaagggaagtggttgctgtgtatgttactggtgcttggaactgctattgttgaggctcatgatggacatg
atgatgatgtgattgatattgaggatgaccttgacgatgtcattgaagaggtagaagactcaaaaccagataccactgctcctc
cttcatctcccaaggttacttacaaagctccagttccaacaggggaagtatattttgctgattcttttgacagaggaactctgt
cagggtggattttatccaaagccaagaaagacgataccgatgatgaaattgccaaatatgatggaaagtgggaggtagaggaaa
tgaaggagtcaaagcttccaggtgataaaggacttgtgttgatgtctcgggccaagcatcatgccatctctgctaaactgaaca
agcccttcctgtttgacaccaagcctctcattgttcagtatgaggttaatttccaaaatggaatagaatgtggtggtgcctatg
tgaaactgctttctaaaacaccagaactcaacctggatcagctccatgacaagaccccttatacgattatgtttggtccagata
aatgtggagaggactataaactgcacttcatcttccgacacaaaaaccccaaaacgggtatctatgaagaaaaacatgctaaga
ggccagatgcagatctgaagacctattttactgataagaaaacacatctttacacactaatcttgaatccagataatagttttg
aaatactggttgaccaatctgtggtgaatagtggaaatctgctcaatgacatgactcctcctgtaaatccttcacgtgaaattg
aggacccagaagaccggaagcccgaggattgggatgaaagaccaaaaatcccagatccagaagctgtcaagccagatgactggg
atgaagatgccccctgctaagattccagatgaagaggccacaaaacccgaaggctggttagatgatgagcctgagtacgtacctg
atccagacgcagagaaacctgaggattgggatgaagacatggatggagaatgggaggctcctcagattgccaaccctagatgtg

agtcagctcctggatgtggtgtctggcagcgacctgtgattgacaaccccaattataaaggcaaatggaagcctcctatgattg
acaatcccagttaccagggaatctggaaacccaggaaaataccaaatccagatttctttgaagatctggaacctttcagaatga
ctcctttagtgctattggtttggagctgtggtccatgacctctgacatttttttgacaactttatcatttgtgctgatcgaa
gaatagttgatgattgggccaatgatggatggggcctgaagaaagctgctgatggggctgctgagccaggcgttgtggggcaga
tgatcgaggcagctgaagagcgcccgtggctgtgggtagtctatattctaactgtagcccttcctgtgttcctggttatcctct
tctgctgttctggaaagaaacagaccagtggtatggagtataagaaaactgatgcacctcaaccggatgtgaaggaagaggaag
aagagaaggaagaggaaaaggacaagggagatgaggaggaggaaggagaagagaaacttgaagagaaacagaaaagtgatgctg
aagaagatggtggcactgtcagtcaagaggaggaagacagaaaacctaaagcagaggaggatgaaattttgaacagatcaccaa
gaaacagaaagccacgaagagagtgaaacaatcttaagagcttgatctgtgatttcttctccctcctccccctgcaagagtggtc
ctaggagaggacctggcacaccttaggttgacattcagaaacttcaagacatcaccatcagcaggctccagttgaacactagt
ctgtgtaactttaaacatctagcagtaaatacttgcagttgtgatataaaggaccctgtttctgtagaaaagaaaacatttaac
ataatggttgtgaaatgtaacatgaagcaaactaacttttttttttaacatctttgtttttaaaatagaatgatagaacttt
gccagtctttaagatcttggcttaatttaatgtattaatctgtttgtgcaaacataataccaccatttaaaaatgttagggaga
tgagttgcagtttttataatagattttttttaaagtttggtattgtaaaacattcacacctctgtccctcaaaattgataatta
cgtttaaagtgcagtcatttgtggttagaatcttgttttgtttgcttccattattgagttcctcctaaggaaattgaggagagg
gactgaatagaagcccaaattcatataaaagttgcgtttaagttgtattaaaaatagatatataagaaaaaattctttcacttg
atgtttgttagaccagaaagtgtgtgtgttctgtagctcagttcccagacagctttttaggtagtggaggaggtggcttcatgt
ggcacttgggcatttatattccacttgggagggtcaggctgtggccttctggagcaggtggcttgttaaggaacgctagcaggg
catggcacgtgagctccggaatagatgtcttcatcacttcttccactgtgtgttgacactgttttccttacctatttcctcaga
tccccagctttctcctctgctatgcattttcttcacagtgcagcttgcagtccgttgctgaaaatgattataagccctgcataa
tgttaagctttattgtgattacgtgtatgtttcttctttcttttaagcagacccatacctttccagggtcaaagtacagaatag
aatacattgatacaaagtacagaaaaatactttgatttttatccatttcttttactctgtgtaaagacttgagaagtctaattc
acaggcaaaccaatacagaattgactgcagttgaacagactagaagtatttgtgggaggagtgacatgaagcatgagttatctg
atttttttgtagctgctatatattttaagccttcatttgcaattcatgtaacagttgtgtcataaattacacaataaagcagt
cctgttcaaattttttttaacgtggcttgtagaattttaaaaaagtgatcttaggtttgtttttcatgcgggatgcagatg
ggtgctatcagagcctctcccacaccactatagtgtaataatgttattattactctacactgaaacgtattcagagttagatat
tattttagcttcagttgttctttagaggctttcaaatgtaccgatgatactgtttcttgcactgaatatataaacactccacag
tgtttatattgggaagatattgggaaggaaatatatttgtaaaagatgaaggctgtatctattttttttttcttttaaagtttg
ttcacttaaattcttttgaggatgggatgtatttttcttgctgttcagtgctttttccttttcatctgttgttctgtggtcaca
gtgaccttagctacatagcagactttcccaaatgtattgattacaaataaacagttgttacttagcaagacctgaaaatatgtc
tgcaggtttctccttgaagcaaatgtgtgggatcattgcatttccagaaatctgcctccttcaccctccgttgacagtatatgt
catgcctcactttcttctagctgagctttaaatcattagagcttaaattgtcagatcgttcattgcctttccagggttatttag
taaagtttgttgaaaac
<212> Type : DNA
<211> Length : 3881
      SequenceName : M94859
      SequenceDescription :

Sequence
--------
<213> OrganismName : HUMAN
<400> PreSequenceString :
cagccagggccaccccaggggctataagagcaactagatttctggagcagctcggggatgggtgccatttgagcccagcttggc
tccccctcctggctggcctccttcctgcccttctgcctgcctgtgtctgctgagattctgcaaagaggaacgttggcaccatgt
ctctgctcaaccctgtcctgctgcccccaaggtgaaggcctatctgagccaaggggagcgcttcatcaaatgggatgatgaaa
ctacagttgcctctccagttatcctccgtgtggatcctaagggctactacttatactggacgtatcaaagtaaggagatggagt
ttctggatatcaccagcatccgggatactcgctttgggaagtttgccaagatgcccaagagccagaagctccgggacgtcttca
acatggactttcctgataacagtttcctgctgaagacactcacggtggtgtccggcccggacatggtggacctcaccttccaca
acttcgtctcctacaaggagaacgtgggcaaggcctgggctgaggacgtactggccctagtcaaacatccgctgacggccaacg
cctcccgcagcaccttcctggacaagatccttgtgaagctcaagatgcagctcaactctgaagggaagattccggtgaagaact
tttccagatgtttcctgctgaccgcaagcgggtggaagctgctctcagtgcctgccacctccccaaaggcaaaaatgacgcca
tcaatcctgaggacttcccagaacctgtctacaagagtttcctcatgagcctctgtcctcggccagaaatagatgagatcttca
cttcttaccatgctaaggccaaaccctacatgacgaaggagcacctgaccaaattcatcaaccagaaacagcgggactcccggc
ttaactccctgctgttcccgccagcacggcctgaccaggtgcagggcctcatcgacaagtatgagcccagtggcatcaatgcac
agaggggccagctgtcacctgaaggcatggtctggtttctctgtgggccagagaacagcgtgctggcccaggacaagctgctgc
tccaccacgacatgacgcagccactcaatcattacttcatcaactcgtcccacaacacctacctgacagccggccagttctcag
gcctctcctcggctgagatgtaccgccaggtgctgctctctggctgccgttgcgtggagctagactgctggaaggggaaacccc
ctgacgaggagcccattatcacccatggcttcaccatgaccacagacatcttcttcaaagaagcaattgaggctattgcagaaa
gtgcctttaagacctcccccctatcccatcatcctgtcgtttgagaaccatgtggactcaccccgccagcaggctaagatggctg
agtattgccggacgatctttggggatatgctgctcacagagcccctggaaaagttcccactaaaaccaggtgtccccctgccca
gccctgaggatctcaggggcaagatcctcatcaagaacaagaagaaccagttttctggccccacctcctccagtaaggataccg
gtggggaggctgagggcagcagcccacccagtgcccctgcagtgtgggctggcgaggaagggactgagctggaggaggaggagg
tggaagaggaagaggaggaggagtcaggaaacctggatgaagaagagattaagaagatgcagtcggatgagggcacagcgggcc

tggaagtgacggcttatgaggagatgtccagcctagtcaattacatccagcccaccaagttcgtctcctttgagttctctgccc
aaaagaaccgaagttatgtcatctcgtccttcacagagctcaaggcatatgacctgctctccaaggcctcggtgcagtttgtgg
actacaacaagcgccagatgagccgcatttaccccaagggaacccgcatggactcctccaactacatgccccagatgttctgga
atgctggatgccagatggttgccctcaacttccagacgatggacttgcccatgcagcagaacatggcagtatttgagttcaacg
ggcagagcggctacctcctcaagcatgagttcatgcgccggccggacaagcagttcaaccccttctcagtggaccgcatcgacg
tggtggtggccaccaccctttccattacggtgatctctggcagttcctgtcagaacgcagcgtgcgcacctatgtagaagtgg
agctgtttggccttcctggggaccccaagaggcgctatcgaactaagctgtcacccagtactaactccatcaatcctgtctgga
aggaggagccctttgtctttgagaagatcttgatgcctgagctggcctccctcagagtggctgtgatggaggaaggcaacaagt
ttcttggacaccgcatcatccccatcaatgccctaaattctgggtaccaccacctgtgcctgcacagtgagagcaacatgcccc
tcaccatgcctgcgctcttcatcttcctggagatgaaggactacatacctggtgcttgggcagatctcactgtggccctcgcca
accccattaagttcttcagtgcccatgacacgaagtctgtgaagctcaaggaggccatgggaggtctgcctgagaagcccttcc
cactggcgagtccagttgccagccaggtcaatggggcgttggccccaacgagcaatgggtcaccagcagccagggccggggcca
gggaagaggctatgaaagaagctgcggagccgcggaccgccagcctggaggagctccgggagctaaagggcgtggtgaagctgc
agcggcggcacgagaaggagctgcgagagttggagcggcgcggagcgcggcgctgggaggagctgctgcagcggggcgcggcgc
agctggcggagctcggcccaccgggcgtgggggcgtcggggcctgcaagctcggtcccggcaagggctctcgcaagaagagga
gcctgccccgcgaggagagcgccggagccgcgcccggcgagggccctgagggcgtggacggcgcgtgcgggagctgaaagaca
ggctggagctggagctgctgcggcagggcgaggagcagtacgagtgcgttctgaagcgcaaggagcagcacgtggccgagcaaa
tctccaaaatgatggagctggccagagagaaacaggcggcagagctgaaggccctgaaggagacgtcggagaacgacaccaaag
agatgaagaaaaagctggagacaaagagactggagcggatccagggcatgaccaaagtcaccacagacaagatggcccaggaga
ggttgaagagagagattaacaactcccacatccaggaagtagtgcaggtgatcaagcagatgacggagaacttggagaggcacc
aggagaagctggaggagaagcaggcggcttgcctggaacagatacgggagatggaaaagcagttccagaaggaggcgctggcag
agtacgaggccaggatgaagggtctggaggcagaggtgaaggagtcggtgagggcctgcctcaggacctgctttccctccgagg
ccaaggacaagcctgagagggcctgcgagtgcccccagagctgtgtgagcaggacccactcatagcaaaggcagatgcccagg
agagccgcctctgatgccccatcccactgggacatttagcaaggaggttcagccccttctctgggatgtggttctattccccc
aggaaaaaggagccccagccttctgaggctgtgggaacctgtggctgccttggacgctgcagcccccctcctcaacggccaggcc
agagtctgagacaggacccaggcaccctcacggcagggcctctctggggcctagaagtcttctcaagctgacttcctacctccc
cctccatctctagataagtgtcatatatttgttgagggcaaaagactatggactggaaggcagaaagtgggatcctggccccac
tctgcctttcctattgagcaaccagctctgggctcagtttcctcacctggagggttggacccactcacctccactctagccccg
aaccctcctgccccaggcttccaggccccatcaggcctgccctgagttggccttgtccactccttgaggcagatcctggcacta
cctcacagctccctggggacggccactccctggctgagggccctcccctcccctctgcctgtccggaacggaggctgaaatgg
aaaagctgccttggccctgcttggctgagtcacaaggggcagtgggctcttgggtgctgttccaccctgaccctggctcacccc
tcttcctaggcctgggggcaggcagttcctaccatgtacccctctcaggctgcctgcctgacaaggtcagcatcatttgctctc
ctgaatttatgaggtttatttattttctctttcctactcctattaaagaacctcgtcccagtgaaa
<212> Type : DNA
<211> Length : 4519
      SequenceName : M95678
      SequenceDescription :

Sequence
--------
<213> OrganismName : HUMAN
<400> PreSequenceString :
caaaatggcaccacactgtttatttaaacctgcaaacaatataaagaggtaaggacttctgacgccaaaccatgcaagtaata
acaaaggaacagagaannatattantaacaaaaagaaaatcattaaaataaaaaacaaagtacttcacatttcacagcagtg
ctttggcagtactggcangcatttctggtcaactgcttccnacagcaggaatatgaagaaacccccagatcttagcaaangtgt
aacaacattttctccctgacagcttacattccaagtcattccatnccataccgctccactccacagttttatanacctgatctt
aagatgtaactaggtactatgtgtgctcacaattaaaaacttaactaaanacaaatcagagggtagcagacaagtnaacaaagt
ttccagtaa
<212> Type : DNA
<211> Length : 429
      SequenceName : N20072
      SequenceDescription :

Sequence
--------
<213> OrganismName : HUMAN
<400> PreSequenceString :
gcgncncgagggtgngacaccttctcacaggggatcatgtactcgccctgaatcagcggcacggccccgatggccttctgcagc
tcgcgcacctcatccaccgggcccaccatgagggaagtgcctgtgtccacaatggcctcacagccctccttgcacagggtcagc
ccgctggccacctccacctggtccaggtggacctggccagtaggcttgcgggtgacattcaggtaggacagagaaccccttgtaa
tacttggagtctgtgccacccagcatcagctcacccccaggctgcgcatctgggtccctgctcaggtagaaggagaagatgttc
tggtccaccagcttctgctgcatcaggttgtccgaagaggggcagcacnttgttgacggagatgcggggtaggccatgcccag
gatgccatcgaacttggctgcgatgaaggtgatgcctggctgcttggtggctcccaaagacctgcta

<212> Type : DNA
<211> Length : 487
      SequenceName : N20475
      SequenceDescription :

Sequence
--------
<213> OrganismName : HUMAN
<400> PreSequenceString :
gacttttagctgctcactggccccgnttgtntggccgactcatccgcccgcgacccctaatcccctctgcctgccccaagatg
ctgaagccagccctggagccccgagggtntcntccttcgagaactgccaaagaaatncatcattggaacgcgtcctcccggggn
tcaaggtccctcacgcacgcaagaccgggaccaccatcgcgggcntgggtgttccaagacggggtcattctgggcgccgatacg
cgagccactaacgattcggtcgtgggcggacaaagagctgcgagaagntccacttcatcgcccccaaaatctaacttgctgtgg
gggctgggagtagccgcgggaagncgagnatgaaccacaaggatgggtgggcttccaagatggaagctaacaaggntttatcta
aggggc
<212> Type : DNA
<211> Length : 426
      SequenceName : T53775
      SequenceDescription :

Sequence
--------
<213> OrganismName : HUMAN
<400> PreSequenceString :
ataactctgtcttcgtgagctggatggcaagctctcacactttacccgcagctgtgctgtgacttcccacacagtgtctgagca
gaggcggcgtgggaccagaccctctcccacagccagcctggncaaggtggcacagaccagagctctgtgcagacaagcacccag
gggtctaggaatgctcccttctctccaggggccagaggggagaaccaagctgctcagccttccttgctctggccagcaggaggc
ccttntcctgggagcagccctcgtttctgcctgtcatgcccgagggacacaagggccatccacttgtgcttcctgcttacatgg
gggcacttgcttggggatgttgcttcccacttcttgaggtnttcccccacccaggggggcct
<212> Type : DNA
<211> Length : 397
      SequenceName : T55353
      SequenceDescription :

Sequence
--------
<213> OrganismName : HUMAN
<400> PreSequenceString :
ttgcattnatagatttattgtaaaacagagctgaagtcacaggaagtagggaactttgcacccaacatatacagcattcattc
gctgagtacctgccaagagcgaggctaggtgcttggccctgcaggaccntgagacatatacgagtcatgtttcctgctctgcna
gctcccagcccggtggggagacaggcagaggcacagcatctttcatacaagtntccaagtgccagaagctctacaatgtcaagg
agcaatttattccttcagcagaggaagaaagaacccccaggngccacatttgctcagagccttgggagctgagaaacttctgat
aggaggtaaagtactgcaggcagctgtaaaaaatctgggagtgggagatgggagcttnggaggctgggcagcagtgcctgggac
agagggantccagnttttggaagtgttttaaaagg
<212> Type : DNA
<211> Length : 455
      SequenceName : T72877
      SequenceDescription :

Sequence
--------
<213> OrganismName : HUMAN
<400> PreSequenceString :
aaagagctggaggcgcgcaggccggctccgctccggccccggacgatgcggcgcgcccaggatgctgccgtgcctcgtagtgct
gctggcggcgctcctcagcctccgtcttggctcagacgctcatgggacagagctgcccagccctccgtctgtgtggtttgaagc
agaattttccaccacatcctccactggacacccatcccaaatcagtctgaaagtacctgctatgaagtggcgctcctgaggta
tggaatagagtcctggaactccatctccaactgtagccagaccctgtcctatgaccttaccgcagtgaccttggacctgtacca
cagcaatggctaccgggccagagtgcgggctgtggacggcagccggcactccaactggaccgtcaccaacacccgcttctctgt
ggatgaagtgactctgacagttggcagtgtgaacctagagatccacaatggcttcatcctcgggaagattcagctacccaggcc
caagatggcccccgcgaatgacacatatgaaagcatcttcagtcacttccgagagtatgagattgccattcgcaaggtgccggg
aaacttcacgttcacacacaagaaagtaaaacatgaaaacttcagcctcctaacctctggagaagtgggagagttctgtgtcca
ggtgaaaccatctgtcgcttcccgaagtaacaaggggatgtggtctaaagaggagtgcatctccctcaccaggcagtatttcac
cgtgaccaacgtcatcatcttctttgcctttgtcctgctgctctccggagccctcgcctactgcctggccctccagctgtatgt

gcggcgccgaaagaagctacccagtgtcctgctcttcaagaagcccagccccttcatcttcatcagccagcgtccctccccaga
gacccaagacaccatccacccgcttgatgaggaggcctttttgaaggtgtccccagagctgaagaacttggacctgcacggcag
cacagacagtggctttggcagcaccaagccatccctgcagactgaagagccccagttcctcctccctgaccctcacccccaggc
tgacagaacgctgggaaacggggagcccccctgtgctgggggacagctgcagtagtggcagcagcaatagcacagacagcgggat
ctgcctgcaggagcccagcctgagccccagcacagggcccacctgggagcaacaggtggggagcaacagcaggggccaggatga
cagtggcattgacttagttcaaaactctgagggccgggctggggacacacagggtggctcggccttgggccaccacagtccccc
ggagcctgaggtgcctggggaagaagacccagctgctgtggcattccagggttacctgaggcagaccagatgtgctgaagagaa
ggcaaccaagacaggctgcctggaggaagaatcgcccttgacagatggccttggccccaaattcgggagatgcctggttgatga
ggcaggcttgcatccaccagccctggccaagggctatttgaaacaggatcctctagaaatgactctggcttcctcaggggcccc
aacgggacagtggaaccagcccactgaggaatggtcactcctggccttgagcagctgcagtgacctgggaatatctgactggag
ctttgcccatgaccttgcccctctaggctgtgtggcagccccaggtggtctcctgggcagctttaactcagacctggtcacccct
gcccctcatctctagcctgcagtcaagtgagtgactcgggctgagaggctgcttttgattttagccatgcctgctcctctgcct
ggaccaggaggagggccctggggcagaagttaggcacgaggcagtctgggcacttttctgcaagtccactggggctggcccagc
caggctgcagggctggtcagggtgtctggggcaggaggaggccaactcactgaactagtgcagggtatgtgggtggcactgacc
tgttctgttgactggggccctgcagactctggcagagctgagaagggcagggaccttctccctcctaggaactctttcctgtat
cataaaggattatttgctcaggggaaccatggggctttctggagttgtggtgaggccaccaggctgaagtcagctcagacccag
acctccctgcttaggccactcgagcatcagagcttccagcaggaggaagggctgtaggaatggaagcttcagggccttgctgct
ggggtcattttaggggaaaaggaggatatgatggtcacatggggaacctcccctcatcgggcctctggggcaggaagcttgt
cactggaagatcttaaggtatatatttctggacactcaaacacatcataatggattcactgaggggagacaaagggagccgag
accctggatggggcttccagctcagaacccatccctctggtgggtacctctggcacccatctgcaaatatctccctctctccaa
caaatggagtagcatcccccctggggcacttgctgaggccaagccactcacatcctcactttgctgccccaccatcttgctgaca
acttccagagaagccatggtttttgtattggtcataactcagccctttgggcggcctctgggcttgggcaccagctcatgcca
gccccagagggtcagggttggaggcctgtgcttgtgtttgctgctaatgtccagctacagacccagaggataagccactgggca
ctgggctggggtccctgccttgttggtgttcagctgtgtgatttggactagccacttgtcagagggcctcaatctcccatctg
tgaaataaggactccacctttaggggaccctccatgtttgctgggtattagccaagctggtcctgggagaatgcagatactgtc
cgtggactaccaagctggcttgtttcttatgccagaggctaacagatccaatgggagtccatggtgtcatgccaagacagtatc
agacacagccccagaagggggcattatgggccctgcctccccataggccatttggactctgccttcaaacaaaggcagttcagt
ccacaggcatggaagctgtgaggggacaggcctgtgcgtgccatccagagtcatctcagccctgcctttctctggagcattctg
aaaacagatattctggcccagggaatccagccatgacccccacccctctgccaaagtactcttaggtgccagtctggtaactga
actccctctggaggcaggcttgagggaggattcctcagggttcccttgaaagctttatttatttattttgttcatttatttatt
ggagaggcagcattgcacagtgaaagaattctggatatctcaggagccccgaaattctagctctgactttgctgtttccagtgg
tatgaccttggagaagtcacttatcctcttggagcctcagtttcctcatctgcagaataatgactgacttgtctaattcatagg
gatgtgaggttctgctgaggaaatgggtatgaatgtgccttgaacacaaagctctgtcaataagtgatacatgtttttattcc
aataaattgtcaagaccaca
<212> Type : DNA
<211> Length : 3632
      SequenceName : U00672
      SequenceDescription :

Sequence
--------
<213> OrganismName : HUMAN
<400> PreSequenceString :
atgtcggcgggcggccgtgacgaggagcggcggaagctggccgacatcatccaccactggaacgccaaccggctggacctgttc
gagatcagccagccgaccgaggatttggagttccatggagtgatgagattttattttcaagataaagctgctggaaactttgca
acaaaatgtattcggtctctagtactgccaccactcaagatgtaatcgaaacgctcgcggagaaatttcgacctgatatgcga
atgctgtcctctcccaagtattcactctatgaagtgcatgtcagcggagaaagaagattggatatagatgagaaacctctagtt
gtacaactgaattggaacaaagatgatcgggaaggcagatttgttcttaagaatgagaatgacgccattcctcctaaggctcaa
agtaatggacctgaaaagcaggaaaaagaaggggttatccagaacttcaagagaactctctcaaagaaagaaaagaaggaaaaa
aagaagagagagaaaaagaggcattgcgacaggcatctgataaagatgatagacctttccaaggggaggatgttgaaaattctcga
ctggctgctgaggtttacaaagacatgccggaaaccagctttactcgaaccatttctaatcctgaggtggttatgaaacgacgg
aggcagcaaaaattggaaaagagaatgcaggaatttcggagctcagatgggcggcctgattcaggtggaacattgagaatttat
gcagatagtttaaaaccaaatattccctacaagacaatcctgctgtctactacagatcctgcagactttgctgtggctgaagct
ttagagaagtatggtctggaaaaagaaaaccctaaggattactgcatcgcccgggttatgcttcctcctggagcccagcattct
gatgaaaagggtgctaaagaaattattcttgatgatgatgagtgtcctttacaaatcttcagggaatggccaagtgacaaaggg
atttagtctttcagttgaagaggaggccaccagaccacatcccaaagaaaaccaagaaacacttggaaggcaagacacccaag
ggaaaggagagagctgacgggtctgtctatggctccacccttcctccggagaagctgccctatttagtagagttaagcccagat
ggttctgactctagagataagccaaagctttaccgccttcagttaagtgttactgaagttgggacagaaaagttggatgacaac
tctatccagttgtttggcccaggaattcagccccatcactgtgaccttaccaacatggatggagtggtcactgtgacgcccaga
agtatggacgcagaaacctacgtggaaggccagcgcatctcagaaaccaccatgctgcagagtggcatgaaagtgcagtttggg
gcgtcccatgtatttaagtttgtggaccccagtcaggatcatgctcttgcaaaaagatctgtggatggaggcctgatggttaag
ggcccaagacataaacctggaattgttcaggagacaacttttgatttgggaggagatattcatagtgggacagcattaccgaca
agcaagagcaccactaggctggacagcgacagagtgtcgtctgcctctagcacagccgagcggggaatggtgaagccgatgatc

agagtagaacagcagccagattatcgcaggcaagaaagcagaacacaggatgcttctgggcctgagctgatactacctgcaagc
attgaattcagggaaagttctgaagattcattttgtctgccattataaattatactaatagctctacagtccactttaagttg
tcccctacatatgtattatatatggcatgccggtatgtattgtccaaccagtacagacctgacatcagccctacagagcgcaca
cataaagtcattgcagtcgtcaacaagatggtgagcatgatggagggtgtcatccagaaacagaagaatattgcaggggcactt
gccttctggatggcaaatgcatctgaacttctcaacttcattaagcaagaccgagaccttagtcggatcacactggatgctcaa
gatgttttagcacatttggttcaaatggcatttaaatacttggttcactgtcttcaatcagaacttaataattacatgccagcc
tttctagatgaccctgaagagaacagtctgcaacgaccaaaaatagatgatgtgctgcacacgctcacaggagccatgtccttg
ctacgacgctgcagagtcaatgccgccctgaccatccagctcttctctcagctcttccacttcatcaatatgtggctgttcaat
agattggtgaccgacccagattcggggctgtgctcccattactggggtgcgattatccgtcagcagttgggccatattgaagcc
tgggctgagaagcaggggctggaactggctgcggactgtcatctgagcaggatcgtgcaggcaacgactttgcttaccatggat
aagtatgcacctgatgacattccaaatataaacagcacctgctttaagttaaattcattacaacttcaagccttattacagaac
tatcactgtgcacctgatgagccttttatcccaacggatcttatagaaaatgtagtgactgtggctgaaaacactgccgatgag
ctggcccgcagtgatggaagggaagtgcagttggaggaggatcctgatctgcagctgccgtttcttttgccagaagatggttat
tcttgtgatgttgtcagaaacattccaaatggtttacaagaatttttagaccctctgtgccagagaggattttgcaggttaatt
cctcacacacgttcaccaggtacttggacaatatatttgaaggtgcagattatgaaagtcaccttctgcgtgagaacacagag
ctggctcagcctctgaggaaagaacctgaaataatcactgtgaccctaaaaaagcagaatggaatggccttagcattgttgca
gcaaagggtgctggtcaagataaactaggaatctacgtgaagtcggttgtgaaaggaggtgctgcagatgtggatggacgtcta
gctgcaggtgatcagctcctcagtgtggatggacgaagtctggttggactctctcaggaaagggcggcagaactcatgacaaga
acaagctctgtggtgacactggaagtagcaaagcagggtgccatctaccacggtctggccacccttctcaatcagccatccccc
atgatgcagagagaatttcagatcgtcgtggctcaggtaaaccccgaccaaagagtgaaggctttgagctctataataattcaact
caaaatgggtctcctgagagtcctcagctgccttgggcagaatatagtgaaccaaagaaattgcctggtgatgacagactgatg
aaaaatagagctgatcaccgttccagccccaacgtagcaaatcagcctcctagtcctggagggaaaagtgcatatgcctctgga
acaacagcgaagataacatctgtctctactggaaacctctgcactgaggagcagacgcctccgcctagacctgaagcctacccc
attcccactcagacgtacaccagagagtatttaccttcccagcttccaaatcccaggatcggatggctcctcctcagaaccag
tggccaaattatgaggaaaagccacatatgcacacagatagtaatcattccagtattgcaattcagcgtgttacacgttcccaa
gaagaacttcgagaagataaagcttaccaacttgagcggcatcgaatagaggcagctatggaccgaaagtctgatagtgatatg
tggataaatcagagctcctcactggactccagtacctctagccaggagcatctgaaccattcctctaagtcggtcacccctgct
tccacactgaccaaaagtggccctggccgttggaaaacaccagcagccataccggccacccctgtggccgtctcccagccaatc
cgaacagacctgcctccgccacccccgccacctccagtccactatgccggtgatttcgatggaatgtccatggatttgcctctc
ccaccacccccttccgccaaccagatagggctgccgtctgcgcaggtggctgctgctgaacggagaaagagagaagaacatcag
cgttggtatgagaaggagaaggccccctggaggaggagcgggagaggaagcggagagagcaggagaggaagttggccagatg
cgcactcagtccttaaaccctgctccgttttctcccctgactgcacagcagatgaagcccgaaaagccttccacactccagcgg
ccacaggaaacagtcattcgggagctgcagcctcagcagcagccccgcacgatcgagcgcagagacttgcagtacattacagtc
agcaaagaggagctttcctcgggggacagtctgtcccccgacccgtggaagcgggacgccaaggagaagctggagaagcagcag
cagatgcacatcgtggacatgctgagcaaggagatccaggagctccagagcaaaccggaccgcagcgccgaggagagcgaccgg
ctgcgcaagctcatgctggagtggcagttccagaagagactccaggagtcgaagcagaaggacgaagatgacgaggaggaggag
gacgatgatgtggacaccatgctgatcatgcagcgcctggaggctgaacgaagagcgagggtaaagggggggagtgctttggctg
tgcccatctgtggtccctatttagcttctgcgtgtttcccatggggatag
<212> Type : DNA
<211> Length : 4839
      SequenceName : U02478
      SequenceDescription :

Sequence
--------
<213> OrganismName : HUMAN
<400> PreSequenceString :
ggcgggaaacagcttagtgggtgtggggtcgcgcattttcttcaaccaggaggtgaggaggtttcgacatggcggtgcagccga
aggagacgctgcagttggagagcgcggccgaggtcggcttcgtgcgcttctttcagggcatgccggagaagccgaccaccacag
tgcgccttttcgaccggggcgacttctatacggcgcacggcgaggacgcgctgctggccgcccgggaggtgttcaagacccagg
gggtgatcaagtacatggggccggcaggagcaaagaatctgcagagtgttgtgcttagtaaaatgaattttgaatcttttgtaa
aagatcttcttctggttcgtcagtatagagttgaagtttataagaatagagctggaaataaggcatccaaggagaatgattggt
atttggcatataaggcttctcctggcaatctctctcagtttgaagacattctctttggtaacaatgatatgtcagcttccattg
gtgttgtgggtgttaaaatgtccgcagttgatggccagagacaggttggagttgggtatgtggattccatacagaggaaactag
gactgtgtgaattccctgataatgatcagttctccaatcttgaggctctcctcatccagattggaccaaaggaatgtgttttac
ccggaggagagactgctggagacatggggaaactgagacagataattcaaagaggaggaattctgatcacagaaagaaaaaaag
ctgacttttccacaaaagacatttatcaggacctcaaccggttgttgaaaggcaaaaagggagagcagatgaatagtgctgtat
tgccagaaatggagaatcaggttgcagtttcatcactgtctgcggtaatcaagttttagaactcttatcagatgattccaact
ttggacagtttgaactgactacttttgacttcagccagtatatgaaattggatattgcagcagtcagagcccttaacctttttc
agggttctgttgaagataccactggctctcagtctctggctgccttgctgaataagtgtaaaacccctcaaggacaaagacttg
ttaaccagtggattaagcagcctctcatggataagaacagaatagaggagagattgaatttagtggaagcttttgtagaagatg
cagaattgaggcagactttacaagaagatttacttcgtcgattcccagatcttaaccgacttgccaagaagtttcaaagacaag
cagcaaacttacaagattgttaccgactctatcagggtataaatcaactacctaatgttatacaggctctggaaaaacatgaag

gaaaacaccagaaattattgttggcagtttttgtgactcctcttactgatcttcgttctgacttctccaagtttcaggaaatga
tagaaacaactttagatatggatcaggtggaaaaccatgaattccttgtaaaccttcatttgatcctaatctcagtgaattaa
gagaaataatgaatgacttggaaaagaagatgcagtcaacattaataagtgcagccagagatcttggcttggaccctggcaaac
agattaaactggattccagtgcacagtttggatattactttcgtgtaacctgtaaggaagaaaaagtccttcgtaacaataaaa
actttagtactgtagatatccagaagaatggtgttaaatttaccaacagcaaattgacttctttaaatgaagagtataccaaaa
ataaaacagaatatgaagaagcccaggatgccattgttaaagaaattgtcaatatttcttcaggctatgtagaaccaatgcaga
cactcaatgatgtgttagctcagctagatgctgttgtcagctttgctcacgtgtcaaatggagcacctgttccatatgtacgac
cagccattttggagaaaggacaaggaagaattatattaaaagcatccaggcatgcttgtgttgaagttcaagatgaaattgcat
ttattcctaatgacgtatactttgaaaaagataaacagatgttccacatcattactggccccaatatgggaggtaaatcaacat
atattcgacaaactggggtgatagtactcatggcccaaattgggtgttttgtgccatgtgagtcagcagaagtgtccattgtgg
actgcatcttagcccgagtaggggctggtgacagtcaattgaaaggagtctccacgttcatggctgaaatgttggaaactgctt
ctatcctcaggtctgcaaccaaagattcattaataatcatagatgaattgggaagaggaacttctacctacgatggatttgggt
tagcatgggctatatcagaatacattgcaacaaagattggtgctttttgcatgtttgcaacccattttcatgaacttactgcct
tggccaatcagataccaactgttaataatctacatgtcacagcactcaccactgaagagaccttaactatgctttatcaggtga
agaaaggtgtctgtgatcaaagttttgggattcatgttgcagagcttgctaatttccctaagcatgtaatagagtgtgctaaac
agaaagccctggaacttgaggagtttcagtatattggagaatcgcaaggatatgatatcatggaaccagcagcaaagaagtgct
atctggaaagagagcaaggtgaaaaaattattcaggagttcctgtccaaggtgaaacaaatgccctttactgaaatgtcagaag
aaaacatcacaataaagttaaaacagctaaaagctgaagtaatagcaaagaataatagctttgtaaatgaaatcatttcacgaa
taaaagttactacgtgaaaaatcccagtaatggaatgaaggtaatattgataagctattgtctgtaatagttttatattgtttt
atattaa
<212> Type : DNA
<211> Length : 2947
      SequenceName : U04045
      SequenceDescription :

Sequence
--------
<213> OrganismName : HUMAN
<400> PreSequenceString :
atgaagctctgcgtgtctgccttctctcctcctgcttgtggccgccttctgcgattcagtgctgtcagcaccaataggctct
gaccctcccacttcctgctgcttctcttacacctcccggaagattcatcggaactttgtgatggattactatgagaccagcagc
ctttgctcccagccagctgtggtatttctgaccaaaaaggcaggcaaatctgtgccgaccccagcgagccctgggtcaatgag
tatgtgaatgacttggagttgaactga
<212> Type : DNA
<211> Length : 279
      SequenceName : U06434
      SequenceDescription :

Sequence
--------
<213> OrganismName : HUMAN
<400> PreSequenceString :
tcctcctctcctgaccgtattctaggatcctctctgctcttacctcctgccattccaaggcctccttctgaacctggcctcaac
ccagtcccaagtttgcaccgcccgaggcaggataggggtccctccattttgatttcctaaagagaataagactggctagttcag
aaccctaagtccagggcagcccaatcagcagtggaagaggctccacagcagtgggcctgaccaccttcccctctcctaggtgca
ggcggagctgaggcggaagtggcggcgctggcacctgcagggcgtcctgggctggaaccccaaataccggcacccgtcgggagg
cagcaacggcgccacgtgcagcacgcaggtttccatgctgacccgcgtcagcccaggtgcccgccgctcctccagcttccaagc
cgaagtctccctggtctgaccaccaggatcccaggggcccaaggcggcccctcccgccccttcccactcaccccggcagacgcc
ggggacagaggcctgcccgggcgcggccagccccggccctgggctcggaggctgcccccggcccccctggtctctggtccggaca
ctcctagagaacgcagccctagagcctgcctggagcgtttctagcaagtgagagagatgggagctcctctcctggaggattgca
ggtggaactcagtcattagactcctcctccaaaggcccccctacgccaatcaagggcaaaaagtctacatactttcatcctgact
ctgccccctgctggctcttctgcccaattggaggaaagcaaccggtggatcctcaaacaacactggtgtgacctgagggcagaa
aggttctgcccggggaaggtcaccagcaccaacaccacggtagtgcctgaaatttcaccattgctgtcaagttcctttgggtta
agcattaccactcaggcatttgactgaagatgcagctcactaccctattctctctttacgcttagttatcagctttttaaagtg
ggttattctggagtttttgtttggagagcacacctatcttagtggttccccaccgaagtggactggcccctgggtcagtctggt
gggaggacggtgcaacccaaggactgagggactctgaagcctctgggaaatgagaaggcagccaccagcgaatgctaggtctcg
gactaagcctacctgctctccaagtctcagtggcttcatctgtcaagtgggatctgtcacaccagccatacttatctctctgtg
ctgtggaagcaacaggaatcaagagctgccctccttgtccacccacctatgtgccaactgttgtaactaggctcagagatgtgc
acccatgggctctgacagaaagcagatacctcaccctgctacacatacaggatttgaactcagatctgtctgataggaatgtga
aagcacggactcttactgctaactttgtgtatcgtaaccagccagatcctcttggttatttgtttaccacttgtattattaat
gccattatcctgaattccccttgccaccccaccctccctggcgtgtggctgaggaggcctccatctcatgtatcatctggatag
gagcctgctggtcacagcctcctctgtctgcccttcaccccagtggccactcagcttcctacccacacctctgccagaagatcc
cctcaggactgcaacaggcttgtgcaacaataaatgttggcttggattggcttggttcccctggtggttgatattctcaatatg

gcctaaaatgggaccagcttccaacttggagccatcttcctccacccaaaggatgggtgatctaatacattactgaaaccagat
gcctcaactcttttggcttttgacagaaatccatatttcatatgaggcattcacagttctgagggccttcagctcccatcttt
ttgagtggtatcctctcctctgagtgtgatgaagaccaggtccccattgtgtccccccactgcatatgtatggacccactcc
agatggcacggggacgggggagtaggaaaagtggcatggtgagcagagccctggctatcaggtggcagatggcagagttggaa
ggaatcttg
<212> Type : DNA
<211> Length : 2109
      SequenceName : U11087
      SequenceDescription :

Sequence
--------
<213> OrganismName : HUMAN
<400> PreSequenceString :
gagctccaagctggtttgaacaagccctgggcatgtttggcgggaagttggcttagctcggctacctgtggccccgcagttttg
tagtccccgccttgtttctccccagaggcctctcaatcctccctccatgatcttcgcatagagcacagtaccccttcacacgga
ggacgcgatggctcccaagaaacgcccagaaacccagaagacctccgagattgtattacgccccaggaacaagaggagcaggag
tcccctggagctggagcccgaggccaagaagctctgtgcgaagggctccggtcctagcagaagatgtgactcagactgcctctg
ggtggggctggctggcccacagatcctgccaccatgccgcagcatcgtcaggaccctccaccagcataagctgggcagagcttc
ctggccatctgtccagcaggggctccagcagtccttttgcacactctggattcttaccggatattacaaaggctgccccctt
tgacaggagggctacatccttggcgtggcacccaactcaccccagcaccgtggctgtgggttccaaagggggagatatcatgct
ctggaattttggcatcaaggacaaacccaccttcatcaaagggattggagctggagggagcatcactgggctgaagtttaaccc
tctcaataccaaccagttttacgcctcctcaatggagggaacaactaggctgcaagactttaaaggcaacattctacgagtttt
tgccagctcagacaccatcaacatctggttttgtagcctggatgtgtctgctagtagccgaatggtggtcacaggagacaacgt
ggggaacgtgatcctgctgaacatggacggcaaagagctttggaatctcagaatgcacaaaaagaaagtgacgcatgtggccct
gaacccatgctgtgattggttcctggccacagcctccgtagatcaaacagtgaaaatttgggacctgcgccaggttagagggaa
agccagcttcctctactcgctgccgcacaggcatcctgtcaacgcagcttgtttcagtcccgatggagcccggctcctgaccac
ggaccagaagagcgagatccgagtttactctgcttcccagtgggactgccccctgggcctgatcccgcaccctcaccgtcactt
ccagcacctcacacccatcaaggcagcctggcatcctcgctacaacctcattgttgtggccgatacccagatcctaatttcaa
aagttgtaccccttatgaattgaggacgatcgacgtgttcgatggaaactcagggaagatgatgtgtcagctctatgacccaga
atcttctggcatcagttcgcttaatgaattcaatcccatgggggacacgctggcctctgcaatgggttaccacattctcatctg
gagccaggaggaagccaggacacggaagtgagagacactaaagaaggtgtgggccagacaaggccttggagcccacacatggga
tcaagtcctgcaagcagaggtggtgatttgttaaagggccaaaagtatccaaggttagggttggagcaggggtgctgggacctg
gggcactgtgggactgggacacttttatgttaatgctctggacttgcctccagagactgctccagagttggtgacacagctgtc
ccaagggcccctctgtatctagcctggaaccaaggttatcttggaactaaatgacttttctcctctcagtgggtggtagcagag
ggatcaagcagttatttgatttgtgctcttttgatatggccaataaaccataccg
<212> Type : DNA
<211> Length : 1820
      SequenceName : U18300
      SequenceDescription :

Sequence
--------
<213> OrganismName : HUMAN
<400> PreSequenceString :
aattcggcacgagggactttgaacatgtcggggatcgccctcagcagactcgcccaggagaggaaagcatggaggaaagaccac
ccatttggtttcgtggctgtcccaacaaaaaatcccgatggcacgatgaacctcatgaactgggagtgcgccattccaggaaag
aaagggactccgtgggaaggaggcttgtttaaactacggatgcttttcaaagatgattatccatcttcgccaccaaaatgtaaa
ttcgaaccaccattatttcacccgaatgtgtacccttcggggacagtgtgcctgtccatcttagaggaggacaaggactggagg
ccagccatcacaatcaaacagatcctattaggaatacaggaacttctaaatgaaccaaatatccaagacccagctcaagcagag
gcctacacgatttactgccaaaacagagtggagtacgagaaaagggtccgagcacaagccaagaagtttgcgccctcataagca
gcgaccttgtggcatcgtcaaaaggaagggattggtttggcaagaacttgtttacaacattttgcaaatctaaagttgctcca
tacaatgactagtcacctgggggggttgggcgggcgccatcttccattgccgccgcgggtgtgcggtctcgattcgctgaattg
cccgtttccatacagggtctcttccttcggtcttttgtattttgattgttatgtaaaactcgctttatttaatattgatgt
cagtatttcaactgctgtaaaattataaacttttatacttgggtaagtcccccagggcgagttcctcgctctgggatgcaggca
tgcttctcaccgtgcagagctgcacttggcctcagctggctgtatggaaatgcaccctccctcctgccgctcctctctagaacc
ttctagaacctgggctgtgctgcttttgagcctcagaccccagggcagcatctcggttctgcgccacttcctttgtgttatatg
gcgttttgtctgtgttgctgtttagagtaaataaactgtttatataaaaaaaaaaaaaaaaaactcga
<212> Type : DNA
<211> Length : 1077
      SequenceName : U29092
      SequenceDescription :

Sequence
--------
<213> OrganismName : HUMAN
<400> PreSequenceString :
tccctcgtctctctcgggcaacatggcgggcgtggaggaggtagcggcctccgggagccacctgaatggcgacctggatccaga
cgacagggaagaaggagctgcctctacggctgaggaagcagccaagaaaaaagacgaaagaagaagaagagcaaagggccttc
tgcagcaggggaacaggaacctgataaagaatcaggagcctcagtggatgaagtagcaagacagttggaaagatcagcattgga
agataaagaaagagatgaagatgatgaagatggagatggcgatggagatggagcaactggaaagaagaagaaaaagaagaagaa
gaagagaggaccaaaagttcaaacagaccctccctcagttccaatatgtgacctgtatcctaatggtgtatttcccaaaggaca
agaatgcgaatacccacccacacaagatgggcgaacagctgcttggagaactacaagtgaagaaaagaaagcattagatcaggc
aagtgaagagatttggaatgattttcgagaagctgcagaagcacatcgacaagttagaaaatacgtaatgagctggatcaagcc
tgggatgacaatgatagaaatctgtgaaaagttggaagactgttcacgcaagttaataaaagagaatggattaaatgcaggcct
ggcatttcctactggatgttctctcaataattgtgctgcccattatactcccaatgccggtgacacaacagtattacagtatga
tgacatctgtaaaatagactttggaacacatataagtggtaggattattgactgtgcttttactgtcacttttaatcccaaata
tgatacgttattaaaagctgtaaaagatgctactaacactggaataaagtgtgctggaattgatgttcgtctgtgtgatgttgg
tgaggccatccaagaagttatggagtcctatgaagttgaaatagatgggaagacatatcaagtgaaaccaatccgtaatctaaa
tggacattcaattgggcaatatagaatacatgctggaaaaacagtgccgattgtgaaaggaggggaggcaacaagaatggagga
aggagaagtatatgcaattgaaacctttggtagtacaggaaaaggtgttgttcatgatgatatggaatgttcacattacatgaa
aaattttgatgttggacatgtgccaataaggcttccaagaacaaaacacttgttaaatgtcatcaatgaaaactttggaaccct
tgccttctgccgcagatggctggatcgcttgggagaaagtaaatacttgatggctctgaagaatctgtgtgacttgggcattgt
agatccatatccaccattatgtgacattaaaggatcatatacagcgcaatttgaacataccatcctgttgcgtccaacatgtaa
agaagttgtcagcagaggagatgactattaaacttagtccaaagccacctcaacacctttattttctgagctttgttggaaaac
atgataccagaattaatttgccacatgttgtctgttttaacagtggacccatgtaatactttttatccatgtttaaaaaagaagg
aatttggacaaaggcaaaccgtctaatgtaattaaccaacgaaaaagctttccggacttttaaatgctaactgtttttcccctt
cctgtctaggaaaatgctataaagctcaaattagttaggaatgacttatacgttttgttttgaatacctaagagatactttttg
gatatttatattgccatattcttacttgaatgctttgaatgactacatccagttctgcacctataccctctggtgttgcttttt
aaccttcctggaatccattttctaaaaaataaagacacattcttctcagcacccacacaacacctattccaaaatcgaccacata
tttggaagtaaagctctcctcagcaaatgtaaaagaacagaaattataacaaactgtctctcagaccacagtataaccaaacta
gaactcaggattaagaaactcactcaaaaccacacaactacatggaaactgaacaacctgctcctgaatgactactggatacat
aacaaaatgaaggcagaaataaagatgttctttaaaaccaatgagaacaaagacacaacataccagaatctctgggacacattc
aaagcagtgtgtagagggaaatttatagcactaaatgcccacaagagaaagcaggaaatatctaaaattgacaccctaacatca
caattaaaagaactagagaagcaagagcaaacacattgaaaagctaagagaaggcaagaaataactaagatcagagcagaactg
aaggaaatagagacacaaaaaactcttcaaaaaatcaatgaatccaggagctggtttttttgaaacgatcaacaaaattgataga
cactagcaagactaataaagaagaaaggagagaagaatcaaatagaagcaataaaaaatgataaaggggatatcaccaccaatc
ccacagaaataaaccaccatcagagaatactacaaacacctctacgcaa
<212> Type : DNA
<211> Length : 2569
      SequenceName : U29607
      SequenceDescription :

Sequence
--------
<213> OrganismName : HUMAN
<400> PreSequenceString :
gagcatacaccgagagagtgaggagccagacgacaagcacacactatggcgctgaaacggattaataaggaacttagtgatttg
gcccgtgaccctccagcacaatgttctgcaggtccagttggggatgatatgtttcattggcaagccacaattatgggacctaat
gacagcccatatcaaggcggtgtattctttttgacaattcatttcctacagactaccccttcaaaccacctaaggttgcattt
acaacaagaatttatcatccaaatattaacagtaatggcagcatttgtctcgatattctaagatcacagtggtcgcctgcttta
acaatttctaaagttcttttatccatttgttcactgctatgtgatccaaacccagatgacccccctagtgccagagattgcacgg
atctataaaacagacagagataagtacaacagaatatctcgggaatggactcagaagtatgccatgtgatgctaccttaaagtc
agaataacctgcattatagctggaataaactttaaattactgttcctttttttgattttcttatccggctgctcccctgatcaga
cctcatctttttttaatttatttttttgtttacctccctccattcattcacatgctcatctgagaagacttaagttcttccagct
ttgacaataactgctttagaaactgtaaagtagttacaagagaacagttgg
<212> Type : DNA
<211> Length : 724
      SequenceName : U39318
      SequenceDescription :

Sequence
--------
<213> OrganismName : HUMAN
<400> PreSequenceString :
ggcggctgcggcacgggaaaggctcagtgactgaagctccaaaggccagcaggctggtggggacgtgaccgaagcgaggctctg

gttccctttcggtgggcgccatttgagcctcatctctggcttccccaggatgcgccggcagccggggagcggctccgggcgcga
ggtctgaggatgatctttcctgtcgcccgctacgcgctccggtggctgcgacggcccgaagaccgtgccttttcccgcgccgcc
atggagatggccctccgaggcgtgcggaaagtcctctgtgtggccgaaaaaaacgacgcggccaaggggatcgccgacctgctg
tcaaacggtcgcatgaggcggagagaaggactttcaaaattcaacaagatctatgaatttgattatcatctgtatggccagaat
gttaccatggtaatgacttcagtttctggacatttactggctcatgatttccagatgcagtttcgaaaatggcagagctgcaac
cctcttgtcctctttgaagcagaaattgaaaagtactgcccagagaattttgtagacatcaagaaaactttggaacgagagact
cgccagtgccaggctctggtgatctggactgactgtgatagagaaggcgaaaacatcgggtttgagattatccacgtgtgtaag
gctgtaaagcccaatctgcaggtgttgcgagcccgattctctgagatcacaccccatgccgtcaggacagcttgtgaaaacctg
accgagcctgatcagagggtgagcgatgctgtggatgtgaggcaggagctggacctgaggattggagctgcctttactaggttc
cagaccctgcggcttcagaggatttttcctgaggtgctggcagagcagctcatcagttacggcagctgccagttccccacactg
ggctttgtggtggagcggttcaaagccattcaggcttttgtaccagaaatcttccacagaattaaagtaactcatgaccacaaa
gatggtatcgtagaattcaactggaaaaggcatcgactctttaaccacacggcttgcctagttctctatcagttgtgtgtggag
gatcccatggcaactgtggtagaggtcagatctaagcccaagagcaagtggcggcctcaagccttggacactgtggagcttgag
aagctggcttctcgaaagttgagaataaatgctaaagaaaccatgaggattgctgagaagctctacactcaagggtacatcagc
tatccccgaacagaaacaaacattttcccagagacttaaacctgacggtgttggtggaacagcagacccccgatccacgctgg
ggggcctttgcccagagcattctagagcggggtggtcccaccccacgcaatgggaacaagtctgaccaagctcaccctcccatt
caccccaccaaatacaccaacaacttacagggagatgaacagcgactgtacgagtttattgttcgccatttcctggcttgctgc
tcccaggatgctcaggggcaggagaccacagtggagatcgacatcgctcaggaacgctttgtggcccatggcctcatgattctg
gcccgaaactatctggatgtgtatccatatgatcactggagtgacaagatcctccctgtctatgagcaaggatcccactttcag
cccagcaccgtggagatggtggacggggagaccagcccacccaagctgctcaccgaggccgacctcattgccctcatggagaag
catggcattggtacggatgccactcatgcggagcacatcgagaccatcaaagcccggatgtacgtgggcctcaccccagacaag
cggttcctccctgggcacctgggcatgggacttgtggaaggttatgattccatgggctatgaaatgtctaagcctgacctccgg
gctgaactggaagctgatctgaagctgatctgtgatggcaaaaaggacaaatttgtggttctaaggcagcaagtgcagaaatac
aagcaggttttcattgaagcggtggctaaagcaaagaaattggacgaggccttggcccagtactttgggaatgggacagagttg
gcccagcaagaagatatctacccagccatgccagagcccatcaggaagtgcccacagtgcaacaaggacatggtccttaagacc
aagaagaatggcgggttctacctcagctgcatgggtttcccagagtgtcgctcagctgtgtggcttcctgactcggtgctggag
gccagcagggacagcagtgtgtgtccagtttgtcagccacaccctgtgtacaggttaaagttaaagtttaagcgcggtagcctt
cccccgaccatgcctctggagtttgtttgctgcatcggcggatgcgacgacaccctgagggagatcctggacctgagatttca
gggggcccccccagggctagccagccctctggccgcctgcaggctaaccagtccctgaacaggatggacaacagccagcacccc
cagcctgctgacagcagacagactgggtcctcaaaggctctggcccagaccctcccaccacccacggctgctggtgaaagcaat
tctgtgacctgcaactgtggccaggaggctgtgctgctcactgtccgtaaggagggccccaaccggggccggcagttctttaag
tgcaacggaggtagctgcaacttcttcctgtgggcagacagccccaatccgggagcaggagggcctcctgccttggcatataga
cccctgggcgcctccctgggatgcccaccaggcccagggatccacctaggtgggtttggcaaccctggtgatggcagtggtagt
ggcacatcctgcctttgcagccagccctccgtcacacggactgtgcagaaggatggacccaacaaggggcgccagttccacaca
tgtgccaagccgagagagcagcagtgtggctttttccagtgggtcgatgagaacaccgctccagggacttctggagccccgtcc
tggacaggagacagaggaagaaccctggagtcggaagccagaagcaaaaggccccgggccagttcctcagacatggggtccaca
gcaaagaaaccccggaaatgcagcctttgccaccagcctggacacacccgtcccttttgtcctcagaacagatgagctcagggt
agggtagagaacgccactttctcagacctgtcccctttgtgtttagaaatgagttaaccaggaccaagtggccatttagtgtcc
tggaaacttagaggacagtgttggcctttggagtcgggccttcttgtgttaaggggcacaaggtccagatcactctggagcagg
ccagctctgctggacagtgaccctcttcccaggcctcaggagtgaccatagccactgctgaaaagtcacgcagctgctccctcg
gacccccccaaggatggttgctgttagcagaggattggtgcagtcccagctgaagcccactgtgtgccaaaggaagaagctccca
gggctgcttccttcacctgcagaaagccccaagtgagccaccagcactcatggggcagtccctgtccaggctgcccagggcttc
tcatagacgtcctgagaaggacggtgtaatgcaaggaaatggctgtggtaacactgatccttcagaagaagcttcattccctct
taatctagttaagccaggacatccagaattcattgctttaataaagaacccaggccggg
<212> Type : DNA
<211> Length : 3755
      SequenceName : U43431
      SequenceDescription :

Sequence
--------
<213> OrganismName : HUMAN
<400> PreSequenceString :
catgattcccaagcttggcacgagggtccgcaagcccggctgagagcgcgccatggggcaggcgggctgcaaggggctctgcct
gtcgctgttcgactacaagaccgagaagtatgtcatcgccaagaacaagaaggtgggcctgctgtaccggctgctgcaggcctc
catcctggcgtacctggtcgtatgggtgttcctgataaagaagggttaccaagacgtcgacacctccctgcagagtgctgtcat
caccaaagtcaagggcgtggccttcaccaacacctcggatcttgggcagcggatctgggatgtcgccgactacgtcattccagc
ccaggaaaagaacgtctttttttgtggtcaccaacctgattgtgacccccaaccagcggcagaacgtctgtgctgagaatgaagg
cattcctgatggcgcgtgctccaaggacagcgactgccacgctggggaagcggttacagctggaaacggagtgaagaccggccg
ctgcctgcggagagagaacttggccaggggcacctgtgagatctttgcctggtgcccgttggagacaagctccaggccggagga
gccattcctgaaggaggccgaagacttcaccattttcataaagaaccacatccgtttccccaaattcaacttctccaacaatgt
gatggacgtcaaggacagatctttcctgaaatcatgccactttggccccaagaaccactactgccccatcttccgactgggctc
cgtgatccgctgggccgggagcgacttccaggatatagccctggagggtggcgtgataggaattaatattgaatggaactgtga

tcttgataaagctgcctctgagtgccaccctcactattcttttagccgtctggacaataaactttcaaagtctgtctcctccgg
gtacaacttcagatttgccagatattaccgagacgcagccggggtggagttccgcaccctgatgaaagcctacgggatccgctt
tgacgtgatggtgaacggcaagggtgcctccttctgcgacctggtactcatctacctcatcaaaaagagagagttttaccgtga
caagaagtaccaggaagtgaggggcctagaagacagttcccaggaggccgaggacgaggcatcggggctggggctatctgagca
gctcacatctgggccagggctgctggggatgccggagcagcaggagctgcaggagccacccgaggcgaacgttggaagcagcag
tcagaaggggaacggatctgtgtgcccacagctcctggagccccacaggagcacgtgaattgcctctgcttacgttcaggccct
gtcctaaacccagccgtctagcacccagtgatcccatgcctttgggaatcccaggatgctgcccaacgggaaatttgtacattg
ggtgctatgaatgccacatcacagggaccagccatcacagagcaaagtgacctccacgtctgatgctggggtcatcaggacgga
cccatcatggctatctttttgccccacccccctgccgtcagttcttcctttctccgtggctggcttcccgcactagggaacgggt
tgtaaatggggaacatgacttccttccggagtccttgagcacctcagctaaggaccgcagtgccctgtagagttcctagattac
ctcactgggaatagcattgtgcgtgtccggaaaagggctccatttggttccagcccactcccctctgcaagtgccgcagcttcc
ctcaggcatactctccagtggatccaagtactctctctcctaaagacaccaccttcctgccagctgtttgcccttaggccagta
cacagaattgaaagtgggggaggtggcagacgctttctgggacctgcccaagatatgtattctctgacactcttatttggtcat
aaaacaataaatggtgtcaatttcaaaaaaaaaaaaaaaaaaaaaaaaaaaa
<212> Type : DNA
<211> Length : 1986
      SequenceName : U49395
      SequenceDescription :

Sequence
--------
<213> OrganismName : HUMAN
<400> PreSequenceString :
gcacgagcgaccatagctctccaggtcaatggccagcaaggagggggtccgagccggcggcggcggcagtggtggcagcg
ggagacaaatggaaacctccacagggcacagactccatcaagatggagaacgggcagagcacagccgccaagctggggctgcct
cccctgacgcccgagcagcaggaggcccttcagaaggccaagaagtacgccatggagcagagcatcaagagtgtgctggtgaag
cagaccatcgcgcaccagcagcagcagctcaccaacctgcagatggcggctcagcggcagggggcgctggccatcatgtgccgc
gtctacgtgggctctatctactatgagctgggggaggacaccatccgccaggcctttgcccccttggccccatcaagagcatc
gacatgtcctgggactccgtcaccatgaagcacaagggctttgccttcgtggagtatgaggtccccgaagctgcacagctggcc
ttggagcagatgaactcggtgatgctgggggggcaggaacatcaaggtgggcagacccagcaacatagggcaggcccagcccatc
atagaccagttggctgaggaggcacgggccttcaaccgcatctacgtggcctctgtgcaccaggacctctcagacgatgacatc
aagagcgtgtttgaggcctttggcaagatcaagtcctgcacactggcccgggaccccacaactggcaagcacaagggctacggc
ttcattgagtacgagaaggcccagtcgtcccaagatgctgtgtcttccatgaacctctttgacctgggtggccagtacttgcgg
gtgggcaaggctgtcacaccgcccatgcccctactcacaccagccacgcctggaggcctcccacctgccgctgctgtggcagct
gctgcagccactgccaagatcacagctcaggaagcagtggccggagcagcggtgctgggtaccctgggcacacctggactggtg
tccccagcactgaccctggcccagcccctgggcactttgccccaggctgtcatggctgcccaggcacctggagtcatcacaggt
gtgaccccagcccgtcctcctatcccggtcaccatcccctcggtgggagtggtgaaccccatcctggccagccctccaacgctg
ggtctcctggagcccaagaaggagaaggaagaagaggagctgtttcccgagtcagagcggccagagatgctgagcgagcaggag
cacatgagcatctcgggcagtagcgcccgacacatggtgatgcagaagctgctccgcaagcaggagtctacagtgatggttctg
cgcaacatggtggaccccaaggacatcgatgatgacctggaaggggaggtgacagaggagtgtggcaagttcggggccgtgaac
cgcgtcatcatctaccaagagaaacaaggcgaggaggaggatgcagaaatcattgtcaagatctttgtggagttttccatagcc
tctgagactcataaggccatccaggccctcaatggccgctggtttgctggccgcaaggtggtggctgaagtgtacgaccaggag
cgttttgataacagtgacctctctgcgtgacagtggtccctctccccggacttgcacttgttccttgtttcctctgggttttat
agtgatacagtggtgtccccgggccaggcgcgctctgcccagcccagcctacagtgcggataaaggtgcggatgctgctggcc
ctgaaaaaaaaaaaaaaaaaactcgag
<212> Type : DNA
<211> Length : 1791
      SequenceName : U51586
      SequenceDescription :

Sequence
--------
<213> OrganismName : HUMAN
<400> PreSequenceString :
tcttggtctcggctcctggcccagtgctctttcccatgtgtctgaatctgcatcttgggcaggggtccctgggccccactcctg
gacccccggactgacccccaccccatcttgtgcttagcagattcttcccctggtggccatgggacccaggtcaatgtcacctgc
atcgtgaacgtctgtagcagctctgaccacagctcacagtgctcctcccaagccagctccacaatgggagacacagattccagc
ccctcggagtccccgaaggacgagcaggtccccttctccaaggaggaatgtgcctttcggtcacagctggagacgccagagacc
ctgctggggagcaccgaagagaagcccctgccccttggagtgcctgatgctgggatgaagcccagttaaccaggccggtgtggg
ctgtgtcgtagccaaggtgggctgagccctggcaggatgaccctgcgaaggggccctggtccttccaggcccccaccactagga
ctctgaggctctttctgggccaagttcctctagtgccctccacagccgcagcctccctctgacctgcaggccaagagcagaggc
agcgagttggggaaagcctctgctgccatggtgtgtccctctcggaaggctggctgggcatggacgttcggggcatgctggggc
aagtccctgactctctgtgacctgccccgcccagctgcacctgccagcctggcttctggagcccttgggtttttgtttgtttg

tttgtttgtttgtttgtttctcccctgggctctgcccagctctggcttccagaaaccccagcatccttttctgcagaggggc
tttctggagaggagggatgctgcctgagtcacccatgaagacaggacagtgcttcagcctgaggctgagactgcgggatggtcc
tggggctctgtgtagggaggaggtggcagccctgtagggaacggggtccttcaagttagctcaggaggcttggaaagcatcacc
tcaggccaggtgcagtggctcacgcctatgatcccagcactttgggaggctgaggcgggtggatcacctgaggttaggagttcg
agaccagcctggccaacatggtaaaaccccatctctactaaaaatacagaaattagccgggcgtggtggcgggcacctatagtc
ccagctactcagaagcctgaggctgggaaatcgtttgaacccgggaagcggaggttgcagggagccgagatcacgccactgcac
tccagcctgggcgacagagcgagagtctgtctcaaaagaaaaaaaaaaagcaccgcctccaaatgctaacttgtccttttgta
ccatggtgtgaaagtcagatgcccagagggcccaggcaggccaccatattcagtgctgtggcctgggcaagataacgcacttct
aactagaaatctgccaatttttaaaaaagtaagtaccactcaggccaacaagccaacgacaaagccaaactctgccagccaca
tccaacccccccacctgccatttgcaccctccgccttcactccggtgtgcctgcagccccgcgcctccttccttgctgtcctagg
ccacaccatctcctttcagggaatttcaggaactagagatgactgagtcctcgtagccatctctctactcctacctcagcctag
accctcctcctcccccagaggggtgggttcctcttccccactccccaccttcaattcctgggccccaaacgggctgccctgcca
ctttggtacatggccagtgtgatcccaagtgccagtcttgtgtctgcgtctgtgttgcgtgtcgtgggtgtgtgtagccaaggt
cggtaagttgaatggcctgccttgaagccactgaagctgggattcctccccattagagtcagccttccccctcccagggccagg
gccctgcagaggggaaaccagtgtagccttgcccggattctgggaggaagcaggttgaggggctcctggaaaggctcagtctca
ggagcatggggataaaggagaaggcatgaaattgtctagcagagcaggggcagggtgataaattgttgataaattccactggac
ttgagcttggcagctgaactattggagggtgggagagcccagccattaccatggagacaagaagggttttccaccctggaatca
agatgtcagactggctggctgcagtgacgtgcacctgtactcaggaggctgaggggaggatcactggagcccaggagtttgagg
ctgcagcgagctatgatcgcgccactacactccagcctgagcaacagagtgagaccctgtctcttaaagaaaaaaaaagtcaga
ctgctgggactggccaggtttctgcccacattggacccacatgaggacatgatggagcgcacctgccccctggtggacagtcct
gggagaacctcaggcttccttggcatcacagggcagagccgggaagcgatgaatttggagactctgtggggccttggttccctt
gtgtgtgtgtgttgatcccaagacaatgaaagtttgcactgtatgctggacggcattcctgcttatcaataaacctgtttgttt
taaaaaaa
<212> Type : DNA
<211> Length : 2613
      SequenceName : U52165
      SequenceDescription :

Sequence
--------
<213> OrganismName : HUMAN
<400> PreSequenceString :
gacatggccaacatcgcggtgcagcgaatcaagcgggagttcaaggaggtgctgaagagcgaggagacgagcaaaaatcaaatt
aaagtagatcttgtagatgagaattttacagaattaagaggagaaatagcaggacctccagacacaccatatgaaggaggaaga
taccaactagagataaaaataccagaaacatacccatttaatccccctaaggtccggtttatcactaaaatatggcatcctaat
attagttccgtcacaggggctatttgtttggatatcctgaaagatcaatgggcagctgcaatgactctccgcacggtattattg
tcattgcaagcactattggcagctgcagagccagatgatccacaggatgctgtagtagcaaatcagtacaaacaaaatcccgaa
atgttcaaacagacagctcgactttgggcacatgtgtatgctggagcaccagtttctagtccagaatacaccaaaaaaatagaa
aacctatgtgctatgggctttgataggaatgcagtaatagtggccttgtcttcaaaatcatgggatgtagagactgcaacagaa
ttgcttctgagtaactgaggcatagagagctgctgatatagtcaagcttgcctcttcttgaggagcaccaacatctgttatttt
taggattctgcatagatttcttttaatctggcattctcgcctaatgatgttatctaggcaccattggagactgaaaaaaaaaa
tccctgctctgtaaataaagctaattaaacgtctgtgtaaatttaaaaaggggaaatactttaatttttttcttaatagtgta
aaaattccctgagctaagctaaaaccatggaagaaacatgctactttagtgtttagcagtgtaccaagactagcaagagtttgc
ttcaggatttggttgaataattaagataatatttggagtgtgtcagggccattcaaattgttggtgttgcatcacagctacctt
aactgtttttaacatggatcctctgtgcctgtgaatttacttgcatgcttgtacttgacttcttaggatgggtagctgaaaaga
ccaccattttaagcatttgagaattcttaaatatgaaatttattcagaattgaagatggtgacctattcagagcctttttgtcc
ttgtcaacagactgggacagtgtctgattccccttcacccccccccacccccgccttggcacacacagctaatattctaatgg
taaatttctctgtatcaggtggggaaatgtgctgaaggacagtatgtatcccttgcttcatttttaggtcgtaggtttggaatg
tcttgtcccagttcttcaaacactcttaaattttcttaagtaatgtaaaaatggaactgccaattttatttctcttgcaaaaa
tagtaaatacttgatgttacattattcccaggtttaatgaaagaacccaacttagtttttcagtgaatttgacacctattttt
agtgatgaaatttttctttgagaactggcaaggatgcagtcagctgtttgcagtttttagcctgattttggggtctatagagat
tgctttattggatacttcaagtcattcttgcttgcacttcccctattgacacatgaaagctgtgttggtgttttattgtacata
cttcagatgcacataggaatagaagtgtgttataaatctagctttctttatgatgtttctgataatacgagaattgaaaacttt
accttctcttgtacatagtcagactatttgtattaaatttacatttcattctaagttccaaaagtttgaaaattattagttttg
caagatcacacactaatgtaaccattttatgaaggttgaagtggatttatgcaggcagttctatatatagaaatncaattcttt
ttaaattttaggaccaatacaaaataacacaaatgtaatggaatcagactgaattaaagtaaggctgtatattgaaagtcata
ttataaaaggtttgctttctttaagtgttatttatcttaaattataatcgttaaatgtttggaagataatttttgaatcataac
gtcagcataacttcatttgacttctcaataatcttgtcgacgcggccgc
<212> Type : DNA
<211> Length : 2149
      SequenceName : U58522
      SequenceDescription :

Sequence
--------
<213> OrganismName : HUMAN
<400> PreSequenceString :
tcccagccggctgaggcgggcagggccgggcggggccgcgccacggagtccacagcccggcgctccctgccgcgccgccgccgc
accgcgccccacaggagaagacgaaccgggcccggcggccgaagcggcccgcgaggcgcgggaggcatgaagttgggcgcgcac
gggcctcgaagcggcggggagccgggagccgcccgcatctagagcccgcgaggtgcgtgcgccatggagctaggggggccccgga
gcgccgcggctgctgccgccgctgctgctgcttctggggaccggcctcctgcgcgccagcagccatgtggagacccgggcccac
gccgaggagcggctcctgaagaaactcttctccggttacaacaagtggtcccgacccgtggccaacatctcggacgtggtcctc
gtccgcttcggcctgtccatcgctcagctcattgacgtggatgagaagaaccagatgatgaccacgaacgtatgggtgaagcag
gagtggcacgactacaagctgcgctgggacccagctgactatgagaatgtcacctccatccgcatccctccgagctcatctgg
cggccggacatcgtcctctacaacaatgctgacggggacttcgcggtcacccacctgaccaaggcccacctgttccatgacggg
cgggtgcagtggactcccccggccatttacaagagctcctgcagcatcgacgtcaccttcttccccttcgaccagcagaactgc
accatgaaattcggctcctggacctacgacaaggccaagatcgacctggtgaacatgcacagccgcgtggaccagctggacttc
tgggagagtggcgagtgggtcatcgtggacgccgtgggcacctacaacaccaggaagtacgagtgctgcgccgagatctacccg
gacatcacctatgccttcgtcatccggcggctgccgctcttctacaccatcaacctcatcatcccctgcctgctcatctcctgc
ctcaccgtgctggtcttctacctgccctccgagtgtggcgagaagatcacgctgtgcatctccgtgctgctgtcgctcaccgtc
ttcctgctgctcatcaccgagatcatcccgtccacctcactggtcatcccactcatcggcgagtacctgctgttcaccatgatc
ttcgtcaccctgtccatcgtcatcacggtcttcgtgctcaacgtgcaccaccgctcgccacgcacgcacaccatgcccacctgg
gtacgcagggtcttcctggacatcgtgccacgcctgctcctcatgaagcggccgtccgtggtcaaggacaattgccggcggctc
atcgagtccatgcataagatggccagtgccccgcgcttctggcccgagccagaaggggagcccccctgccacgagcggcacccag
agcctgcaccctccctcaccgtccttctgcgtcccccctggatgtgccggctgagcctgggccttcctgcaagtcaccctccgac
cagctccctcctcagcagcccctggaagctgagaaagccagcccccacccctcgcctggaccctgccgcccgccccacggcacc
caggcaccagggctggccaaagccaggtccctcagcgtccagcacatgtccagccctggcgaagcggtggaaggcggcgtccgg
tgccggtctcggagcatccagtactgtgttccccgagacgatgccgcccccgaggcagatggccaggctgccggcgccctggcc
tctcgcaacacccactcggctgagctcccacccccagaccagccctctccgtgcaaatgcacatgcaagaaggagccctcttcg
gtgtccccgagcgccacggtcaagacccgcagcaccaaagcgccgcccccgcacctgcccctgtcgccggccctgacccgggcg
gtggagggcgtccagtacattgcagaccacctgaaggccgaagacacagacttctcggtgaaggaggactggaagtacgtggcc
atggtcatcgaccgcatcttcctctggatgttcatcatcgtctgcctgctggggacggtgggcctcttcctgccgccctggctg
gctggcatgatctaggaagggaccgggagcctgcgtggcctggggctgccgtgcacggggccagcatccatgcggccggcctgg
ggccgggctggcttctccctggactctgtggggccacacgtttgccaaattttccttcctgttctgtgtctgctgtaagacggc
cttggacggggacacggcctctggggagaccgagtgtggagctgcttccagttggactgtggcctcaggaggcagtggcttgga
gcagaggtgggggtcgccgccttctacctgcaggactcgggctaagtccagctctcccccctgcgcagccctccgcggcggacag
gaacaccagccccagcgagtctggagaccaggactctgccttccaggcgtagggccagggctctggcaggtggccagggctcca
cggggggctagtggcttcagcccctggggtacttctgtgttgtgattccccggagctgggaaggtcccgaatggagtccagacc
tgggccctggttcccccaggaccctgagggtttccaccttggcgcgcagcccgggagatccgccctgggctctgggttcgggaa
gaaggacttcctgctacagtagctgtggggagctggtgggggcatccttgaggacctccacctgggagatgctgggaccctcgg
ggcaggaagtccctgagaagcctcatgggagtcagggagccctggggtttccacacaggcccatgccctccgtcctggcagggc
aggcagagctcagcacagcctcacccctgcaggcggtatccagaggtgagggaggcctgaaatgtttccaggcatgaccctgga
gcccggcagtgcacccccctaaagatggcgcacccggcagcccccccattgtccccaggggcacacttccccccttgggatgggcac
agcctgccccacccctccatgattccaagggccaagaggggcggggccaggatggcttttcccctgcctgtgagtgacatcggt
tcaggaggagacagtcaggaagcctcctgctgagtggtccacattctgctgcccccagaccccatccagccaggggtggggatg
gggttgggctctgcgtcccactgagtctcattcctctgtccccgagccgagctctcctggccagggtctcgtcaggaggtgcc
tgagagcagaatgaataattgaggttaggaacccggcatgccgagtgccccagaaatgccgctgtgt
<212> Type : DNA
<211> Length : 3343
      SequenceName : U62433
      SequenceDescription :

Sequence
--------
<213> OrganismName : HUMAN
<400> PreSequenceString :
gaattccggggtggtctgagctggagccacgctttctgttggagggggcagctgaaggagaacagcaagacatatccggcggcc
ctacggactcggagagctggagggaccccggagatctcaaagggacaggaaaggcagcagcagccaccctctctcccagtcaag
tggtcaccagcaggactgaaggggacagcccctttgcagtggctcggcgaggagacccctgcaccctagggtcagtgcaggagc
ccagccgctgagccatgccgggtccccgggcggcctgcagcgagcccaacccctgcacccaggtagtcatgaacagccacagct
acaatggcagcgtggggcggccgctgggcagcgggccgggcgccctgggacgagaccctccggaccctgaggccggccaccccc
cacaacccccgcacagcccgggcctccaggtggtagtggccaagagtgagccagcccggccctcacccggcagcccccggggc
agccccaggaccaggacgatgacgaggatgatgaggaagatgaggccggcaggcagagagcctcggggaaccctcaaatgtgg
gccaccgcctggccaccggcggcgctcttcgagaagcggaagcgcctcagcgactatgccctcatttcggcatgtttggca
tcgtcgtcatggtgacggagaccgagctgtcctgggggtgtacaccaaggagtctctgtactcattcgcactcaaatgcctca
tcagcctctccacggccatcctgctgggtctcgttgtcctctaccatgcccgggagatccagctgttcatggtggacaacgggg

ctgatgactggcgcatcgccatgacctgcgagcgcgtgttcctcatctcgctagagctggcagtgtgcgccattcacccggtgc
ccggccactaccgcttcacgtggacggcgcggctggccttcacgtacgcgccctcggtggccgaggccgacgtggacgtgctgc
tgtccatccccatgttcctgcgcctctacctgctgggccgggtgatgctactgcacagcaaaatcttcacggacgcctcgagcc
gcagcatcggggccctcaacaagatcaccttcaacacgcgcttcgtcatgaagacactcatgaccatctgccccggcaccgtgc
tgctggtcttcagcatctcctcctggatcatcgcagcctggaccgtgcgcgtctgcgagaggtaccacgacaagcaggaagtga
ccagcaacttcctgggggccatgtggctgatttccatcaccttcctctccattggctacggcgacatggtgccccacacctact
gcgggaagggtgtgtgcctgctcactggcatcatgggagctggctgtaccgcgctcgtggtggctgtggtggctcggaagctgg
agctcaccaaggctgagaagcacgtgcacaacttcatgatggacactcagctcaccaagcgggtaaaaaacgccgctgctaacg
ttctcagggagacgtggctcatctacaaacataccaggctggtgaagaagccagaccaagcccgggttcggaaacaccagcgta
agttcctccaagccatccatcaggctcagaagctccggagtgtgaagatcgagcaagggaagctgaacgaccaggctaacacgc
ttaccgacctagccaagacccagaccgtcatgtacgaccttgtatcggagctgcacgctcagcacgaggagctggaggcccgcc
tggccaccctggaaagccgcttggatgcgctgggtgcctctctacaggccctgcctggcctcatcgcccaagccatacgcccac
cccgcctcccctgcctcccaggcccggccccggccccaagaccaggcagcccggagctcccccctgccggtggacgcccgtgg
cccccctcggactgcgggtgacggccctgcccgccaccagacccctaaatcttggccatcgtgtggccgccacctccgggaagcc
ttgtacagtggcgcctcttggagttcaagaagccgacgctgagtcaggctgagtggactgaggcctgccccgcccagactgccc
aggcagagggcagggctggaccatgggtgagggcaggggagcccggagcttcctctggtcacctggtcccccgactctccccag
gcccccggtgggcatggagcagcccggggaggggtccgtgctggttctgaataaagcaggacccgcctagtggctgcctgtgtg
catggctggaaggcactggtgatgtcccaggaggtagacctccagccctgggtaccaagatgaatgtgggaatcagaaaaacct
gttcccatcaccggcctagcctagaatcctagcctagaagccctctctccctctggctggagctcagtagggacaactctctag
ggacacctgtaccagccccacctggcgctgagatccctcagacagcatggcccagccctggccagaagcatcgctccccttcaa
ccaaccgcgttgatg
<212> Type : DNA
<211> Length : 2535
      SequenceName : U69883
      SequenceDescription :

Sequence
--------
<213> OrganismName : HUMAN
<400> PreSequenceString :
cactcctgcctccaccatgtccatcagggtgacccagaagtcctacaaggtgtccacctctggcccccgggccttcagcagccg
ctcctacacgagtgggcccggttcccgcatcagctcctcgagcttctcccgagtgggcagcagcaactttcgcggtggcctggg
cggcggctatggtggggccagcggcatgggaggcatcaccgcagttacggtcaaccagagcctgctgagccccccttgtcctgga
ggtggaccccaacatccaggccgtgcgcacccaggagaaggagcagatcaagaccctcaacaacaagtttgcctccttcataga
caaggtacggttcctggagcagcagaacaagatgctggagaccaagtggagcctcctgcagcagcagaagacggctcgaagcaa
catggacaacatgttcgagagctacatcaacaaccttaggcggcagctggagactctgggccaggagaagctgaagctggaggc
ggagcttggcaacatgcaggggctggtggaggacttcaagaacaagtatgaggatgagatcaataagcgtacagagatggagaa
cgaatttgtcctcatcaagaaggatgtggatgaagcttacatgaacaaggtagagctggagtctcgcctggaagggctgaccga
cgagatcaacttcctcaggcagctgtatgaagaggagatccgggagctgcagtcccagatctcggacacatctgtggtgctgtc
catggacaacagccgctccctggacatggacagcatcattgctgaggtcaaggcacagtacgaggatattgccaaccgcagccg
ggctgaggctgagagcatgtaccagatcaagtatgaggagctgcagagcctggctgggaagcacggggatgacctgcggcgcac
aaagactgagatctctgagatgaaccggaacatcagccggctccaggctgagattgagggcctcaaaggccagagggcttccct
ggaggccgccattgcagatgccgagcagcgtggagagctggccattaaggatgccaacgccaagttgtccgagctggaggccgc
cctgcagcgggccaagcaggacatggcgcggcagctgcgtgagtaccaggagctgatgaacgtcaagctggccctggacatcga
gatcgccacctacaggaagctgctggagggcgaggagagccggctggagtctgggatgcagaacatgagtattcatacgaagac
caccagcggctatgcaggtggtctgagctcggcctatgggggcctcacaagccccggcctcagctacagcctgggctccagctt
tggctctggcgcgggctccagctccttcagccgcaccagctcctccagggccgtggttgtgaagaagatcgagacacgtgatgg
gaagctggtgtctgagtcctctgacgtcctgcccaagtgaacagctgcggcagcccctcccagcctacccctcctgcgctgccc
cagagcctgggaaggaggccgctat
<212> Type : DNA
<211> Length : 1537
      SequenceName : U76549
      SequenceDescription :

Sequence
--------
<213> OrganismName : HUMAN
<400> PreSequenceString :
atggccctgctactggccctcagcctgctggttctctggacttccccagccccaactctgagtggcaccaatgatgctgaagac
tgctgcctgtctgtgacccagaaacccatccctgggtacatcgtgaggaacttccactaccttctcatcaaggatggctgcagg
gtgcctgctgtagtgttcaccacactgagggccgccagctctgtgcaccccccagaccagccctgggtagaacgcatcatccag
agactgcagaggacctcagccaagatgaagcgccgcagcagttaacctatgaccgtgcagagggagcccggagtccgagtcaag
cattgtgaattattacctaacctggggaaccgaggaccagaaggaaggaccaggcttccagctcctctgcaccagacctgacca

gccaggacagggcctggggtgtgtgagtgtgagtgtgagcgagagggtgagtgtggtctagagtaaagctgctccacccca
gattgcaatgctaccaataaagccgcctggtgtttacaact
<212> Type : DNA
<211> Length : 545
      SequenceName : U77180
      SequenceDescription :

Sequence
--------
<213> OrganismName : HUMAN
<400> PreSequenceString :
ggcacgagcttgctcgcggagagaggagctaggagcctcggccaatgggagccggcgttgttggaggccacggcggcgcagccc
caaagcgagcgaagctagggtcgccgccactgccgcaggaggcgtgaggggataaaaacattcagatggcagatcacagttttt
cagatggggttccttcagattccgtggaagctgctaaaaatgcaagtaacacagaaaagctcacagatcaggtgatgcagaatc
ctcgagttctggcagctttacaggagcgacttgacaatgtccctcacaccccttccagctacatcgaaactttacctaaagcag
taaaaagaagaattaatgcattgaaacaacttcaggtgagatgtgctcacatagaagccaagttctatgaagaggtacatgact
tggaaagaaagtatgcagcgctataccagcctctctttgacaagagaagagaatttatcaccggcgatgttgaaccaacagatg
cggaatcggaatggcacagtgaaaatgaagaggaagagaaattggctggagacatgaaaagtaaagtagtcgtcacagaaaaag
cagcggcaacggctgaagagccagatcccaaaggaattccagagttctggtttaccatcttcagaaatgtggacatgctgagtg
aattagtccaggaatatgatgaaccaatcttgaaacacctgcaggatattaaagtgaaattttctgaccctggacagcctatgt
cttttgtgttagagttccactttgaacccaacgactactttaccaactcagtcctgacaaaaacctacaagatgaaatcagaac
cagataaggctgatcccttttcctttgaaggtcctgagattgtggactgtgacgggtgtactattgactggaagaaaggaaaga
atgttactgtcaaaaccatcaagaaaaagcagaagcataagggtcgaggcactgttagaacaattacgaaacaagtacccaatg
agtcctttttcaacttcttcaatccattgaaagcatccggggatggagaatcactggatgaagattctgaattcacattagcct
ctgattttgaaattggacacttttccgtgagcggatagtcccgcgggctgtgctgtacttcactggggaggccatagaagatg
atgacaattttgaagaaggtgaagaaggagaagaggaggaattagaaggtgacgaggagggagaagacgaggatgatgcggaaa
ttaaccccaaggtgtaattttgtctgttaatcattcatacgtttctagaaggaacccagccagccggcggaatgcaagcagca
gtaggaagcggaggcgggtgcctggcagaccggctgtcgggactccaggcctgtgggcggggcctcggtccttgccgcagcaca
atcccgtggacagagcttactccatctaactcgttttcaagtgcatgattttcactttcactttccttttccttattatttt
gattaacttgtacagtggcaactgaaatgcatttcagaaataggaggtttcgtccagcaccctctgcagccttggtgcctgtag
ctctggacttccctgggcctttccctgtgggagggccctgtagacacatcagggtggggtgggggtcacttggcaaaaagggcc
gaggtctggtgatgtggttcccaggatcttggaacctctcccacccctcctgcagttggactgaattctaccctttcatccgaa
gaaacccacttgctgtttccagccgctgaatctgctgagtgtgcagcctgcatcacctgctgtatgccgatcatctcagaaagg
gctgtgtagagtagggccctgttctccttaggatgttgcttcttgatttttttttttttttaggggtgggtcagggttgtgaca
caccagcccaggtgagagctgctgcgggtcacctcatatttatttatcccttcttgcctgtgaggactgcggcttttcgctgtg
gctcgtccttaacgtttctgaaccaccttggtgccctgagcaggaagatgtgccacttcctagcaggcgcaaggcctgtgcgga
agaaacgccgctccctgccaccagggctgaagatgcgagcccgtcctcatgacgcaggcgccaccctgctgccggagccgggct
tcggcatcttctccactgagggactgggctgggaagtcctgcgtttcagtggagcgtatgagcgtcaagtcctgctttctcagt
agccccattgcggggccccaccattcatcctgtctgaaggtcctgggtttggtgtgaccgcttggcggctggtgggtggggttt
tcaagtgggtgacggcgctctccggcaaccggggatggccgtgtccgcactgaccaggcctgtggagagtgctcggcctaacct
tagaacacaattgtaactgaaaacagtgttttcaatttgtacagaatagttagaatattctaataaagtggtgaaacattgaaa
aaaaaanaaaaaaa
<212> Type : DNA
<211> Length : 2534
      SequenceName : U77456
      SequenceDescription :

Sequence
--------
<213> OrganismName : HUMAN
<400> PreSequenceString :
aattacattgatttattagaagcatttgtagtccttcatggtttcagaaaatcaattgttcagacctttttgaacatatctgat
tgtgatattcatactgtgtaactctgaatttaaatcaagttcagtggaaattaagatatgatcgtattatttttagtgtatgt
ttcgtatctgaattttgatgcagttaaaaaagccttaagaaaacgacaaaaaagctagagtgcagacaacctaaaatctctat
tttaaaacatcattttcctgaggccatgcagactcatatctaagtttttttcatttaaatagacttaattctaacaaatactta
ctttcataatggnaaaacttttaaaggtgggacagacatactagttacgaaatggaagttttcaatccacaggattccag
<212> Type : DNA
<211> Length : 417
      SequenceName : W15277
      SequenceDescription :

Sequence
--------

<213> OrganismName : HUMAN
<400> PreSequenceString :
tttttttttttatatttatatttatatttatatatatgtatatatatatatatgtnatgtacaaaagactttgagatatcagg
caccattaaaccacatttccccccttataaatgcaactgttcaagtacactgggaacagttttaaggtacacctgcagtacant
aggagaagcatgagtggataatctaaacacaggatcataacagtgatacgctgcaacacctctgtgaattccattanccaagtt
ctgtcattaaaacatnggaaaactactggctcctcaaaataaaaggttttaggnaaccaaaaatcccctaagtagtgaactgtt
ttccaagcagagctccctaatggttttcaatttcctggcctacaaccaaanggggaccccagttggaagctgccgtttgggaa
acgtgggccaggcatcagatcancaacacggggggaatccngagaggggcncattnttgaagaaggng
<212> Type : DNA
<211> Length : 489
      SequenceName : W49672
      SequenceDescription :

Sequence
--------
<213> OrganismName : HUMAN
<400> PreSequenceString :
gacgtgacccagggcagactggtagcaaagcccccacgcccagccaggagcaccgccgaggactccagcacaccgagggacatg
ctgggcctgcgcccccactgctcgccctggtgggnntgctctccctcgggtgcgtcctctctcaggagtgcacgaagttcaagg
tcagcagctgccgggaatgcatcgagtcggggcccggctgcacctggtncagaagctgggagcggctttgggtcctccagccca
cgccaccgcggcgaagcaagtccccggagangaagccgggtggctcccgtctgtcctcggactgagcagggtggccaaggccca
ggttggaaactaccttggtcaggctgggtttttncctcccctttctggaggttttcaagctcctcttgaaataaacaccttttt
caagtgaga
<212> Type : DNA
<211> Length : 429
      SequenceName : W68291
      SequenceDescription :

Sequence
--------
<213> OrganismName : HUMAN
<400> PreSequenceString :
ttgccgatccgccgcccgtccacacccgccgccagctcaccatggatgatgatatcgccgcgctcgtcgtcgacaacggctccg
gcatgtgcaaggccggcttcgcgggcgacgatgccccccgggccgtcttcccctccatcgtggggcgccccaggcaccagggcg
tgatggtgggcatgggtcagaaggattcctatgtgggcgacgaggcccagagcaagagaggcatcctcaccctgaagtacccca
tcgagcacggcatcgtcaccaactgggacgacatggagaaaatctggcaccacaccttctacaatgagctgcgtgtggctcccg
aggagcaccccgtgctgctgaccgaggcccccctgaaccccaaggccaaccgcgagaagatgacccagatcatgtttgagacct
tcaacaccccagccatgtacgttgctatccaggctgtgctatccctgtacgcctctggccgtaccactggcatcgtgatggact
ccggtgacggggtcacccacactgtgcccatctacgaggggtatgccctcccccatgccatcctgcgtctggacctggctggcc
gggacctgactgactacctcatgaagatcctcaccgagcgcggctacagcttcaccaccacggccgagcgggaaatcgtgcgtg
acattaaggagaagctgtgctacgtcgccctggacttcgagcaagagatggccacggctgcttccagctcctccctggagaaga
gctacgagctgcctgacggccaggtcatcaccattggcaatgagcggttccgctgccctgaggcactcttccagccttccttcc
tgggcatggagtcctgtggcatccacgaaactaccttcaactccatcatgaagtgtgacgtggacatccgcaaagacctgtacg
ccaacacagtgctgtctggcggcaccaccatgtaccctggcattgccgacaggatgcagaaggagatcactgccctggcaccca
gcacaatgaagatcaagatcattgctcctcctgagcgcaagtactccgtgtggatcggcggctccatcctggcctcgctgtcca
ccttccagcagatgtggatcagcaagcaggagtatgacgagtccggcccctccatcgtccaccgcaaatgcttctaggcggact
atgacttagttgcgttacaccctttcttgacaaaacctaacttgcgcagaaaacaagatgagattggcatggctttatttgttt
ttttgttttgttttggtttttttttttttttggcttgactcaggatttaaaaactggaacggtgaaggtgacagcagtcggt
tggagcgagcatcccccaaagttcacaatgtggccgaggactttgattgcacattgttgtttttttaatagtcattccaaatat
gagatgcattgttacaggaagtcccttgccatcctaaaagccaccccacttctctctaaggagaatggcccagtcctctcccaa
gtccacacaggggaggtgatagcattgctttcgtgtaaattatgtaatgcaaaatttttttaatcttcgccttaatactttttt
attttgttttattttgaatgatgagccttcgtgcccccccttccccctttttgtcccccaacttgagatgtatgaaggcttttg
gtctccctgggagtgggtggaggcagccagggcttacctgtacactgacttgagaccagttgaataaaagtgcacaccttta
<212> Type : DNA
<211> Length : 1761
      SequenceName : X00351
      SequenceDescription :

Sequence
--------
<213> OrganismName : HUMAN
<400> PreSequenceString :
tatattccgggggtctgcgcggccgaggaccccctgggtgcgctgctctcagctgccgggtccgactcgcctcactcagctcccc

tcctgcctcctgaagggcagcttcgccgacgcttggcgggaaaaagaagggaggggagggatcctgagtcgcagtataaaagaa
gcttttcgggcgtttttttctgactcgctgtagtaattccagcgagagacagagggagtgagcggacggttggaagagccgtgt
gtgcagagccgcgctccggggcgacctaagaaggcagctctggagtgagaggggctttgcctccgagcctgccgcccactctcc
ccaaccctgcgactgacccaacatcagcggccgcaaccctcgccgccgctgggaaactttgcccattgcagcgggcagacactt
ctcactggaacttacaatctgcgagccaggacaggactccccaggctccggggagggaatttttgtctatttggggacagtgtt
ctctgcctctgcccgcgatcagctctcctgaaaagagctcctcgagctgtttgaaggctggatttcctttgggcgttggaaacc
ccgcagacagccacgacgatgcccctcaacgtgaacttcaccaacaggaactatgacctcgactacgactccgtacagccctat
ttcatctgcgacgaggaagagaatttctatcaccagcaacagcagagcgagctgcagccgcccgcgcccagtgaggatatctgg
aagaaattcgagctgcttcccaccccgcccctgtccccgagccgccgctccgggctctgctctccatcctatgttgcggtcgct
acgtccttctccccaagggaagacgatgacggcggcggtggcaacttctccaccgccgatcagctggagatgatgaccgagtta
cttggaggagacatggtgaaccagagcttcatctgcgatcctgacgacgagaccttcatcaagaacatcatcatccaggactgt
atgtggagcggtttctcagccgctgccaagctggtctcggagaagctggcctcctaccaggctgcgcgcaaagacagcaccagc
ctgagccccgcccgcgggcacagcgtctgctccacctccagcctgtacctgcaggacctcaccgccgccgcgtccgagtgcatt
gacccctcagtggtctttccctacccgctcaacgacagcagctcgcccaaatcctgtacctcgtccgattccacggccttctct
ccttcctcggactcgctgctgtcctccgagtcctccccacgggccagccctgagcccctagtgctgcatgaggagacaccgccc
accaccagcagcgactctgaagaagagcaagaagatgaggaagaaattgatgtggtgtctgtggagaagaggcaaacccctgcc
aagaggtcggagtcgggctcatctccatcccgaggccacagcaaacctccgcacagcccactggtcctcaagaggtgccacgtc
tccactcaccagcacaactacgccgcaccccctccacaaggaaggactatccagctgccaagagggccaagttggacagtggc
agggtcctgaagcagatcagcaacaaccgcaagtgctccagccccaggtcctcagacacggaggaaaacgacaagaggcggaca
cacaacgtcttggaacgtcagaggaggaacgagctgaagcgcagcttttttgccctgcgtgaccagatccctgaattggaaaac
aacgaaaaggcccccaaggtagtgatcctcaaaaaagccaccgcctacatcctgtccattcaagcagacgagcacaagctcacc
tctgaaaaggacttattgaggaaacgacgagaacagttgaaacacaaactcgaacagcttcgaaactctggtgcataaactgac
ctaactcgaggaggagctggaatctctcgtgagagtaaggagaacggttccttctgacagaactgatgcgctggaattaaaatg
catgctcaaagcctaacctcacaaccttggctggggctttgggactgtaagcttcagccataattttaactgcctcaaacttaa
atagtataaaagaactttttttatgcttcccatctttttctttttccttttaacagatttgtatttaattgtttttttaaaaa
aatcttaaaatctatccaattttcccatgtaaatagggccttgaaatgtaaataactttaataaaacgtttataacagttacaa
aagattttaagacatgtaccataattttttt
<212> Type : DNA
<211> Length : 2300
      SequenceName : X01023
      SequenceDescription :

Sequence
--------
<213> OrganismName : HUMAN
<400> PreSequenceString :
ttccggtacctgtgagtcagctaggggagggcagctctcacccaggctgatagttcggtgacctggctttatctactggatgag
ttccgctgggagatggaacatagcacgtttctctctggcctggtactggctacccttctctcgcaagtgagccccttcaagata
cctatagaggaacttgaggacagagtgtttgtgaattgcaataccagcatcacatgggtagagggaacggtgggaacactgctc
tcagacattacaagactggacctgggaaaacgcatcctggacccacgaggaatatataggtgtaatgggacagatatatacaag
gacaaagaatctaccgtgcaagttcattatcgaatgtgccagagctgtgtggagctggatccagccaccgtggctggcatcatt
gtcactgatgtcattgccactctgctccttgctttgggagtcttctgctttgctggacatgagactggaaggctgtctggggct
gccgacacacaagctctgttgaggaatgaccaggtctatcagcccctccgagatcgagatgatgctcagtacagccaccttgga
ggaaactgggctcggaacaagtgaacctgagactggtggcttctagaagcagccattaccaactgtaccttcccttcttgctca
gccaataaatatatcctctttcactcaaaa
<212> Type : DNA
<211> Length : 702
      SequenceName : X01451
      SequenceDescription :

Sequence
--------
<213> OrganismName : HUMAN
<400> PreSequenceString :
ggggggggggggaccacttggcctgcctccgtcccgccgcgccacttggcctgcctccgtcccgccgcgccacttcgcctgcct
ccgtcccccgcccgccgcgccatgcctgtggccggctcggagctgccgcgccggcccttgcccccgccgcacaggagcgggac
gccgagccgcgtccgccgcacggggagctgcagtacctggggcagatccaacacatcctccgctgcggcgtcaggaaggacgac
cgcacgggcaccggcaccctgtcggtattcggcatgcaggcgcgctacagcctgagagatgaattccctctgctgacaaccaaa
cgtgtgttctggaagggtgttttggaggagttgctgtggtttatcaagggatccacaaatgctaaagagctgtcttccaaggga
gtgaaaatctgggatgccaatggatcccgagactttttggacagcctgggattctccaccagagaagaaggggacttgggccca
gtttatggcttccagtggaggcattttggggcagaatacagagatatggaatcagattattcaggacagggagttgaccaactg
caaagagtgattgacaccatcaaaaccaaccctgacgacagaagaatcatcatgtgcgcttggaatccaagagatcttcctctg
atggcgctgcctccatgccatgccctctgccagttctatgtggtgaacagtgagctgtcctgccagctgtaccagagatcggga

gacatgggcctcggtgtgcctttcaacatcgccagctacgccctgctcacgtacatgattgcgcacatcacgggcctgaagcca
ggtgactttatacacactttgggagatgcacatatttacctgaatcacatcgagccactgaaaattcagcttcagcgagaaccc
agacctttcccaaagctcaggattcttcgaaaagttgagaaaattgatgacttcaaagctgaagactttcagattgaagggtac
aatccgcatccaactattaaaatggaaatggctgtttagggtgctttcaaaggagcttgaaggatattgtcagtctttaggggt
tgggctggatgccgaggtaaaagttctttttgctctaaaagaaaaaggaactaggtcaaaaatctgtccgtgacctatcagtta
ttaatttttaaggatgttgccactggcaaatgtaactgtgccagttctttccataataaaaggctttgagttaactcactgagg
gtatctgacaatgctgaggttatgaacaaagtgaggagaatgaaatgtatgtgctcttagcaaaaacatgtatgtgcatttcaa
tcccacgtacttataaagaaggttggtgaatttcacaagctattttggaatatttttagaatattttaagaatttcacaagct
attccctcaaatctgaggagctgagtaacaccatcgatcatgatgtagagtgtggttatgaactttatagttgttttatatgt
tgctataataaagaagtgttctgc
<212> Type : DNA
<211> Length : 1536
      SequenceName : X02308
      SequenceDescription :

Sequence
--------
<213> OrganismName : HUMAN
<400> PreSequenceString :
ctacacacttcttgaaggcaaaggcaatgctgaagtcacctttcatgttcaaatcatattaaaaagttagcaagatgtaattat
cagtgtactatgtaaatctttgtgaatgatcaataattacatattttcattatatatatttagtagataatatttatatacat
tcaacattctaaatatagaaagtttacagagaaaaataaagcctttttttccaatcctgtcctccacctctgcatcccattctt
cttcacagaggcaactgattcaagtcattacatagttattgagtgttaactacaactatgttaagtacagctatatatgttaga
tgccgtagccacagaaatcagtttacaatctaatgcagtggatacagcatgtatacatataatataaggttgctacaaatgcta
tctgaggtagagctgtttgaaagaatactaatacttaaatgtttaattcaactgacttgattgacaactgattagctgagtgga
aaagatggatgagaaagattgtgagacttaattggctggtggtatggtgatatgattgacaataactgctaagtcagagaggga
tatattaaggaggagaagaaaagcaacaaatctggttttgatgtgttcactttgttataattattgattatttactgaatatga
atatttatctttgtttttgagtcaataaatatacctttgtaaagacagaattaaagtattagtatttctttcaaactggaggca
tttctcccactaacatatttcatcaaaacttataataagcttggttccagaggaagaaatgagggataaccaaaaatagagaca
ttaataatagtgtaacgcccagtgataaatctcaataggcagtgatgacagacatgttttcccaaacacaaggatgctgtaagg
gccaaacagaaatgatggcccctccccagcacctcatttgccccttccttcagctatgcctctactctcctttagatacaagg
gaggtggatttttctcttctctgagatagcttgatggaaccacaggaacaatgaagtgggctcctggctctttttctctgtggca
gatggggtgccatgcccaccttcagacaaagggaagattgagctcaaaagctccctgagaagtgagagcctatgaacatggttg
acacagagggacaggaatgtatttccagggtcattcattcctgggaatagtgaactgggacatgggggaagtcagtctcctcct
gccacagccacagattaaaaataataatgttaactgatccctaggctaaaataatagtgttaactgatccctaagctaagaaag
ttcttttggtaattcaggtgatggcagcaggacccatcttaaggatagactaggtttgcttagttcgaggtcatatctgtttgc
tctcagccatgtactggaagaagttgcatcacacagcctccaggactgccctcctcctcacagcaatggataatgcttcactag
cctttgcagataatttggatcagagaaaaaaccttgagctgggccaaaaaggaggagcttcaacctgtgtgcaaaatctggga
acctgacagtataggttgggggccaggatgaggaaaaaggaacgggaaagacctgcccaccccttctggtaaggaggccccgtga
tcagctccagccatttgcagtcctggctatcccaggagcttacataaagggacaattggagcctgagaggtgacagtgctgaca
ctacaaggctcggagctccgggcactcagacatcatgagttggtccttgcacccccggaatttaattctctacttctatgctct
tttatttctctcttcaacatgtgtagcagtaagtgtgctcttcacaaaacgttgtttaaaatggaaagctggaaaataaaacag
ataataaactagtgaaatttcgtatttttctcttttagtatgttgctaccagagacaactgctgcatcttagatgaaagatt
cgtaagtagtttttatgtttctccctttgtgtgtgaactggagaggggcagaggaatagaaataattccctcataaatatcatc
tggcacttgtaacttttaaaaacatagtctaggttttacctattttcttaatagatttaagagtagcatctgtctacattt
ttaatcactgttatattttcagggtagttattgtccaactacctgtggcattgcagatttcctgtctacttatcaaaccaaagt
agacaaggatctacagtctttggaagacatcttacatcaagttgaaaacaaaacatcagaagtcaaacagctgataaaagcaat
ccaactcacttataatcctgatgaatcatcaaaaccaagtgagaaaataaagactactgaccaaaaaataataataataatctg
tgaagttcttttgctgttgttttagttgttctatttgcttaaggattttatgtctctgatcctatattacagatatgatagac
gctgctactttgaagtccaggataatgttagaagaaattatgaaatatgaagcatcgattttaacacatgactcaagtattcgg
taaggattttgttttaatttgctctgcaagactgatttagtttttatttaatattctatacttgagtgaaagtaattttaat
gtgttttccccatttataatatcccagtgacattatgcctgattatgttgagcatagtagagatagaagttttagtgcaatat
aaattatactgggttataattgcttattaataatcacattgaagaaagatgttctagatgtcttcaaatgctagtttgaccata
tttatcaaaaatttttccccatcccccatttatcttacaacataaaatcaatctcataggaatttgggtgttgaaaataaaat
cctctttataaaaatgctgacaaattggtggttaaaaaaattagcaagcagaggcatagtaaggattttggctcctaaagtaaa
ttatattgaatgtggagcaggaagaaacatgtcttgagagactaagtgtggcaaatattgcaaagctcatattgatcattgcag
aatgaacctgcatagtctcttcccttcatttggaagtgaatgtctctgttaaagcttctcagggactcataaactttctgaaca
taaggtctcagatacagtttaatattttccccaattttttttctgaatttttctcaaagcagcttgagaaattgagataaa
tagtagctagggagaagtggcccaggaaagatttctcctctttttgctatcagagggcccttgttattattgttattattatta
cttgcattattattgtccatcattgaagttgaaggaggttattgtacagaaattgcctaagacaaggtagagggaaaacgtgga
caaatagtttgtctacccttttttacttcaaagaaagaacggtttatgcattgtagacagtttctatcattttggatatttg
caagccaccctgtaagtaactacaaaggagggtttttacttcccccagtccattcccaaagctatgtaaccagaagcattaaa
gaagaaaggggaagtatctgttgtttatttacatacaataacgttccagatcatgtccctgtgtaagttatattttagattg

aagcttatatgtatagcctcagtagatccacaagtgaaaggtatactccttcagcacatgtgaattactgaactgagcttttcc
tgcttctaaagcatcagggggtgttcctattaaccagtctcgccactcttgcaggttgctatctgctgtcccttatgcataaag
taaaaagcaaaatgtcaatgacatttgcttattgacaaggactttgttatttgtgttgggagttgagacaatatgccccattct
aagtaaaaagattcaggtccacattgtattcctgttttaattgatttttgatttgtttttctttttcaaaaagtttataattt
taattcatgttaatttagtaatataatttacatttcctcaagaatggaataatttatcagaaagcacttcttaagaaaatac
ttagcagtttccaaagaaaatataaaattactcttctgaaaggaatacttattttgtcttcttattttgttatcttatgttt
ctgtttgtagatatttgcaggaaatatataattcaaataatcaaaagattgttaacctgaaagagaaggtagcccagcttgaag
cacagtgccaggaaccttgcaaagacacggtgcaaatccatgatatcactgggaaaggtaactgatgaaggttatattgggatt
aggttcatcaaagtaagtaatgtaaaggagaaagtatgtactggaaagtataggaatagtttagaaagtggctacccattaagt
ctaagaatttcagttgtctagacctttcttgaatagctaaaaaaacagtttaaaaggaatgctgatgtgaaaagtaagaaaat
tattcttggaaaatgaatagtttactacatgttaaaagctatttttcaaggctggcacagtcttacctgcatttcaaaccacag
taaaagtcgattctccttctctagattgtcaagacattgccaataagggagctaaacagagcggctttactttattaaacctc
tgaaagctaaccagcaattcttagtctactgtgaaatcgatgggtctggaaatggatggactgtgtttcagaaggtaattttt
ccccaccatgtgtatttaataaattcctacattgtttctgccatatggcagatactttctaagcaccttgtgaaccgtagctc
atttaatccttgcaatagccctaagaggaaggtacttctgttactcctatttacagaaaaggaaactgaggcacacaaggttaa
ataacttgcccaagaccacataactaataagcaacagagtcagcatttgaacctaggcagtatagtttcagagtttgtgacttg
actctatattgtactggcactgactttgtagattcatggtggcacataatcatagtaccacagtgacaaataaaaagaaggaaa
ctcttttgtcaggtaggtcaagacctgaggtttcccatcacaagatgaggaagcccaacaccacccccccaccaccccaccacca
tcaccaccctttcacacaccagaggatacacttgggctgctccaagacaaggaacctgtgttgcatctgccacttgctgatacc
cactaggaatcttggctcctttactttctgtttacctcccaccactgttataactgtttctacagggggcgctcagagggaatg
aatggtggaagcattagttgccagacaccgattgagcaatgggttccatcataagtgtaagaatcagtaatatccagctagagt
tctgaagtcgtctaggtgtcttttaatattaccactcatttagaatttatgatgtgccagaaaccctcttaagtatttctctt
atattctctctcatgatccttgcagcaaccctaagaagtaaccatcatttttcctatttgatacatgaggaaactgaggtagct
tggccaagatcacttagttgggagttgatagaaccagtgctctgtattttgacaaaatgttgacagcattctctttacatgca
ttgatagtctatttctcctttgctcttgcaaatgtgtaattagagacttgatggcagtgtagatttcaagaaaaactggatt
caatataaagaaggatttggacatctgtctcctactggcacaacagaattttggctgggaaatgagaagattcatttgataagc
acacagtctgccatcccatatgcattaagagtggaactggaagactggaatggcagaaccaggtactgttttgaaatgacttcc
aactttttattgtaaagattgcctggaatgtgcactttccaactatcaatagacaatggcaaatgcagcctgacaaatgcaaac
agcacatccagccaccattttctccaggagtctgtttggttcttgggcaatccaaaaaggtaaattctattcaggatgaatcta
agtgtattggtacaatctaattaccctggaaccattcagagtaatagctaattactgaacttttaatcagtcccaggaattgag
cataaaattataatttatctagtctaaattactatttcatgaagcaggtattattattaatcccattttatagattaacttgc
tcaaagtcacattgctgataagtggtagaggtagaattcagactcaagtagtttaactttagagcctgtcctcttaacaactat
cctggttgaaaagcaaatacagcctcttcagacttctcagtgccttgatggccatttattctgtcaaatcatgagctaccctaa
aagtaaaccagctagctcttttgatgatctagaggcttctttttgcttgagatatttgaaggttttaagcattgttacctaatt
aaaatgcagaaaaatatccaaccctcttgttatgtttaaggaatagtgaaatatattgtcttcaaacacatggacttttttta
ttgcttggttggttttaatccagaaagtgctatagtcagtagaccttcttctaggaaaggaccttccatttcccagccactgg
agattagaaaataagctaaatatttctggaaatttctgttcattcattaaggcccatcctttcccccactctatagaagtgtt
gtccacttgcacaatttttccaggaaagaatctctctaactccttcagctcacatgctttggaccacacagggaagactttga
ttgtgtaatgccctcagaagctctccttcttgccactaccacactgatttgaggaagaaaatccctttagcacctaacccttca
ggtgctatgagtggctaatggaactgtacctccttcaagttttgtgcaataattaagggtcactcactgtcagatactttctgt
gatctatgataatgtgtgtgcaacacataacatttcaataaaagtagaaaatatgaaattagagtcatctacacatctggattt
gatcttagaatgaaacaagcaaaaaagcatccaagtgagtgcaattattagttttcagagatgcttcaaaggcttctaggccca
tcccgggaagtgttaatgagctgtggactggttcacatatctattgcctcttgccagatttgcaaaaaacttcactcaatgagc
aaatttcagccttaagaaacaaagtcaaaaattccaaggaagcatcctacgaaagagggaacttctgagatccctgaggagggt
cagcatgtgatggttgtatttccttcttctcagtactgcagactatgccatgttcaaggtgggacctgaagctgacaagtaccg
cctaacatatgcctacttcgctggtggggatgctggagatgcctttgatggctttgattttggcgatgatcctagtgacaagtt
tttcacatcccataatggcatgcagttcagtacctgggacaatgacaatgataagtttgaaggcaactgtgctgaacaggatgg
atctggttggtggatgaacaagtgtcacgctggccatctcaatggagtttattaccaaggtatgttttcctttcttagattcca
agttaatgtatagtgtatactattttcataaaaaataataaatagatatgaagaaatgaagaataatttataaagatagtaggg
atttatcatgttctttatttcaactaagttctttgaaactggaagtggataataccaagttcatgcctaaaattagcccttct
aaagaaatccacctgctgcaaaatatccagtagtttggcattatatgtgaaactatcaccatcatagctggcactgtgggttgt
gggatctcctttagacatacaacataaatgatctggatggattaacattactacatggatgcttgttgacacattaacctggct
tcccatgagctttgtgtcagatacacgcagtgaacaggtgtttggaggaacagaataaagagaaggcaagcactggtaagggca
ggggtttgtgaaagcttgagagaagagaccagtctgaggacagtagacacttatttaggatgggggttggatgaggaggctat
agtttgctataagcttggaatggtttggaacactggtttcactcacctacccagcagttatgtgtggggaagccttaccgatgc
taaaggatccatgttacaataatggcattatttggaaatcccagtggtattccatgaataaaaccactatgaagataatcccac
tcaacagactctccgttggagaaggacagcaacaccaccctgggaaagccaaacagtcagaccagacctgtttagcatcagtag
gacttccctaccatatctgctgggtagatgagtgaaaccagtgttccaaaccactccgggcttgtagcaaaccatagtctcctc
atctaccaagatgagcaaccttacctcctgatgtcctagccaatcaccaactaggaaactttgcacagtttatttaaagtaaca
gtttgattttcacaatatttttaaattggagaaacataacttatctttgcactcacaaaccacataatgagaagaaactctaag
ggaaaatgcttgatctgtgtgacccggggcgccatgccagagctgtagttcatgccagtgttgtgctctgacaagccttttaca
gaattacatgagatctgcttccctaggacaaggagaaggcaaatcaacagaggctgcactttaaaatggagacataaaataaca
tgccagaaccatttcctaaagctcctcaatcaaccaacaaaattgtgctttcaaataacctgagttgacctcatcaggaatttt

gtggctccttctcttctaacctgcctgaagaaagatggtccacagcagctgagtccgggatggataagcttagggacagaggcc
aattagggaactttgggtttctagccctactagtagtgaataaatttaaagtgtggatgtgactatgagtcacagcacagatgt
tgtttaataatatgtttattttataaattgatattttaggaatctttggagatattttcagttagcagataatactataaattt
tatgtaactggcaatgcacttcgtaatagacagctcttcatagacttgcagaggtaaaaagattccagaataatgatatgtaca
tctacgacttgttttaggtggcacttactcaaaagcatctactcctaatggttatgataatggcattatttggccacttggaa
aacccggtggtattccatgaagaaaaccactatgaagataatcccattcaacagactcacaattggagaaggacagcaacacca
cctgggggagccaaacaggtcagaccagagcaccctgcggaaacagaatatgactcactttaccctgaggatgatttgtagaa
aattaactgctaacttctattgacccacaaagtttcagaaattctctgaaagtttcttcctttttctcttactatatttattg
atttcaagtcttctattaaggacatttagccttcaatggaaattaaaactcatttaggactgtatttccaaattactgatatca
gagttatttaaaaattgtttatttgaggagataacatttcaactttgttcctaaatatataataataaaatgattgactttatt
tgcatttttatgaccacttgtcatttattttgtcttcgtaaattattttcattatatcaaatatttagtatgtacttaataaa
ataggagaacatttagagtttcaaattcccaggtattttccttgtttattacccctaaatcattcctatttaattcttctttt
taaatggagaaaattatgtcttttaatatggttttgttttgttatatattcacaggctggagacgtttaaaagaccgtttca
aaagagatttactttttaaaggactttatctgaacagagagatataatatttttcctattggacaatggacttgcaaagcttc
acttcattttaagagcaaaagaccccatgttgaaaactccataacagttttatgctgatgataatttatctacatgcatttcaa
taaacctttgtttcctaagactagatacatggtacctttattgaccattaaaaaaccaccactttttgccaatttaccaatta
caattgggcaaccatcagtagtaattgagtcctcatttatgctaaatgttatgcctaactctttgggagttacaaaggaaata
gcaattatggcttttgccctctaggagatacaggacaaatacaggaaaatacagcaacccaaactgacaatactctatacaaga
acataatcactaagcaggagtcacagccacacaaccaagatgcatagtatccaaagtgcagctgtgtgcaaattaacagtccta
actggtatagagcacctagtccagtgacctgctgggtaaactgtggatgatggttgcaaaagactaatttaaaaaataactacc
aagaggccctgtctgtacctaacgccctatttttgcaatggctatatggcaagaaagctggtaaactatttgtctttcaggacc
ttttgaagtagtttgtataacttcttaaaagttgtgattccagataaccagctgtaacacagctgagagacttttaatcagaca
aagtaattcctctcactaaactttacccaaaaactaaatctctaatatggcaaaaatggctagacacccattttcacattccca
tctgtcaccaattggttaatctttcctgatggtacaggaaagctcagctactgatttttgtgatttagaactgtatgtcagaca
tccatgtttgtaaaactacacatccctaatgtgtgccatagagtttaacacaagtcctgtgaatttcttcactgttgaaaatta
ttttaaacaaaatagaagctgtagtagccctttctgtgtgcaccttaccaactttctgtaaactcaaaacttaacatatttact
aagccacaagaaatttgatttctattcaaggtggccaaattatttgtgtaatagaaaactgaaaatctaatattaaaaatatgg
aacttctaatatatttttatatttagttatagtttcagatatatatcatattggtattcactaatctgggaaggaagggctac
tgcagctttacatgcaatttattaaaatgattgtaaaatagcttgtatagtgtaaaataagaatgattttagatgagattgtt
ttatcatgacatgttatatatttttgtaggggtcaaagaaatgctgatggataacctatatgatttatagtttgtacatgcat
tcatacaggcagcgatggtctcagaaaccaaacagtttgctctaggggaagagggagatggagactggtcctgtgtgcagtgaa
ggttgctgaggctctgacccagtgagattacagaggaagttatcctctgcctcccattctgaccacccttctcattccaacagt
gagtctgtcagcgcaggtttagtttactcaatctccccttgcactaaagtatgtaaagtatgtaaacaggagacaggaaggtgg
tgcttacatccttaaaggcaccatctaatagcgggttactttcacatacagccctcccccagcagttgaatgacaacagaagct
tcagaagtttggcaatagtttgcatagaggtaccagcaatatgtaaatagtgcagaatctcataggttgccaataatacactaa
ttcctttctatcctacaacaagagtttatttccaaataaaatgaggacatgtttttgtttctttgaatgctttttgaatgtta
tttgttattttcagtattttggagaaattatttaataaaaaaacaatcatttgctttttg
<212> Type : DNA
<211> Length : 12072
      SequenceName : X02415
      SequenceDescription :

Sequence
--------
<213> OrganismName : HUMAN
<400> PreSequenceString :
gaattcccgcggagcagcgtgcgcggggccccgggagacggcggcggtagcggcgcgggcagagcaaggacgcggcggatccca
ctcgcacagcagcgcactcggtgccccgcgcagggtcgcgatgctgcccggtttggcactgctcctgctggccgcctggacggc
tcgggcgctggaggtacccactgatggtaatgctggcctgctggctgaaccccagattgccatgttctgtggcagactgaacat
gcacatgaatgtccagaatgggaagtgggattcagatccatcagggaccaaaacctgcattgataccaaggaaggcatcctgca
gtattgccaagaagtctaccctgaactgcagatcaccaatgtggtagaagccaaccaaccagtgaccatccagaactggtgcaa
gcggggccgcaagcagtgcaagacccatccccactttgtgattccctaccgctgcttagttggtgagtttgtaagtgatgccct
tctcgttcctgacaagtgcaaattcttacaccaggagaggatggatgtttgcgaaactcatcttcactggcacaccgtcgccaa
agagacatgcagtgagaagagtaccaacttgcatgactacggcatgttgctgccctgcggaattgacaagttccgaggggtaga
gtttgtgtgttgcccactggctgaagaaagtgacaatgtggattctgctgatgcggaggaggatgactcggatgtctggtgggg
cggagcagacacagactatgcagatgggagtgaagacaaagtagtagaagtagcagaggaggaagaagtggctgaggtggaaga
agaagaagccgatgatgacgaggacgatgaggatggtgatgaggtagaggaagaggctgaggaaccctacgaagaagccacaga
gagaaccaccagcattgccaccaccaccaccaccaccacagagtctgtggaagaggtggttcgagaggtgtgctctgaacaagc
cgagacggggccgtgccgagcaatgatctcccgctggtactttgatgtgactgaagggaagtgtgccccattcttttacggcgg
atgtggcggcaaccggaacaactttgacacagaagagtactgcatggccgtgtgtggcagcgccattcctacaacagcagccag
taccccctgatgccgttgacaagtatctcgagacacctggggatgagaatgaacatgcccatttccagaaagccaaagagaggct
tgaggccaagcaccgagagagaatgtcccaggtcatgagagaatgggaagaggcagaacgtcaagcaaagaacttgcctaaagc
tgataagaaggcagttatccagcatttccaggagaaagtggaatctttggaacaggaagcagccaacgagagacagcagctggt

ggagacacacatggccagagtggaagccatgctcaatgaccgccgcctggccctggagaactacatcaccgctctgcaggc
tgttcctcctcggcctcgtcacgtgttcaatatgctaaagaagtatgtccgcgcagaacagaaggacagacagcacaccctaaa
gcatttcgagcatgtgcgcatggtggatcccaagaaagccgctcagatccggtcccaggttatgacacacctccgtgtgattta
tgagcgcatgaatcagtctctctccctgctctacaacgtgcctgcagtggccgaggagattcaggatgaagttgatgagctgct
tcagaaagagcaaaactattcagatgacgtcttggccaacatgattagtgaaccaaggatcagttacggaaacgatgctctcat
gccatctttgaccgaaacgaaaaccaccgtggagctccttcccgtgaatggagagttcagcctggacgatctccagccgtggca
ttcttttggggctgactctgtgccagccaacacagaaaacgaagttgagcctgttgatgcccgccctgctgccgaccgaggact
gaccactcgaccaggttctgggttgacaaatatcaagacggaggagatctctgaagtgaagatggatgcagaattccgacatga
ctcaggatatgaagttcatcatcaaaaattggtgttctttgcagaagatgtgggttcaaacaaaggtgcaatcattggactcat
ggtgggcggtgttgtcatagcgacagtgatcgtcatcaccttggtgatgctgaagaagaaacagtacacatccattcatcatgg
tgtggtggaggttgacgccgctgtcaccccagaggagcgccacctgtccaagatgcagcagaacggctacgaaaatccaaccta
caagttctttgagcagatgcagaactagaccccgccacagcagcctctgaagttggacagcaaaaccattgcttcactaccca
tcggtgtccatttatagaataatgtgggaagaaacaaacccgttttatgatttactcattatcgccttttgacagctgtgctgt
aacacaagtagatgcctgaacttgaattaatccacacatcagtaatgtattctatctctctttacattttggtctctatactac
attattaatgggttttgtgtactgtaaagaatttagctgtatcaaactagtgcatgaatagattctctcctgattatttatcac
atagccccttagccagttgtatattattcttgtggtttgtgacccaattaagtcctactttacatatgctttaagaatcgatgg
gggatgcttcatgtgaacgtgggagttcagctgcttctcttgcctaagtattcctttcctgatcactatgcattttaaagttaa
acatttttaagtatttcagatgctttagagagattttttttccatgactgcattttactgtacagattgctgcttctgctatat
ttgtgatataggaattaagaggatacacacgtttgtttcttcgtgcctgttttatgtgcacacattaggcattgagacttcaag
cttttctttttttgtccacgtatctttgggtctttgataaagaaaagaatccctgttcattgtaagcacttttacggggcgggt
ggggaggggtgctctgctggtcttcaattaccaagaattc
<212> Type : DNA
<211> Length : 3148
      SequenceName : X06989
      SequenceDescription :

Sequence
--------
<213> OrganismName : HUMAN
<400> PreSequenceString :
gtccgccaaaacctgcgcggatagggaagaacagcaccccggcgccgattgccgtaccaaacaagcctaacgtccgctgggccc
cggacgccgcgcggaaaagatgaatttacaaccaattttctggattggactgatcagttcagtttgctgtgtgtttgctcaaac
agatgaaaatagatgtttaaaagcaaatgccaaatcatgtggagaatgtatacaagcagggccaaattgtgggtggtgcacaaa
ttcaacatttttacaggaaggaatgcctacttctgcacgatgtgatgatttagaagccttaaaaaagaagggttgccctccaga
tgacatagaaaatcccagaggctccaaagatataaagaaaaataaaaatgtaaccaaccgtagcaaaggaacagcagagaagct
caagccagaggatattcatcagatccaaccacagcagttggttttgcgattaagatcaggggagccacagacatttacattaaa
attcaagagagctgaagactatcccattgacctctactaccttatggacctgtcttattcaatgaaagacgatttggagaatgt
aaaaagtcttggaacagatctgatgaatgaaatgaggaggattacttcggacttcagaattggatttggctcatttgtggaaaa
gactgtgatgccttacattagcacaacaccagctaagctcaggaacccttgcacaagtgaacagaactgcaccaccccatttag
ctacaaaaatgtgctcagtcttactaataaaggagaagtatttaatgaacttgttggaaaacagcgcatatctggaaatttgga
ttctccagaaggtggtttcgatgccatcatgcaagttgcagtttgtggatcactgattggctggaggaatgttacacggctgct
ggtgttttccacagatgccgggtttcactttgctggagatgggaaacttggtggcattgttttaccaaatgatggacaatgtca
cctggaaaataatatgtacacaatgagccattattatgattatccttctattgctcaccttgtccagaaactgagtgaaaataa
tattcagacaatttttgcagttactgaagaatttcagcctgtttacaaggagctgaaaaacttgatccctaagtcagcagtagg
aacattatctgcaaattctagcaatgtaattcagttgatcattgatgcatacaattccctttcctcagaagtcattttggaaaa
cggcaaattgtcagaaggagtaacaataagttacaaatcttactgcaagaacggggtgaatggaacaggggaaaatggaagaaa
atgttccaatatttccattggagatgaggttcaatttgaaattagcataacttcaaataagtgtccaaaaaaggattctgacag
ctttaaaattaggcctctgggctttacggaggaagtagaggttattcttcagtacatctgtgaatgtgaatgccaaagcgaagg
catccctgaaagtcccaagtgtcatgaaggaaatgggacatttgagtgtggcgcgtgcaggtgcaatgaagggcgtgttggtag
acattgtgaatgcagcacagatgaagttaacagtgaagacatggatgcttactgcaggaaagaaaacagttcagaaatctgcag
taacaatggagagtgcgtctgcggacagtgtgtttgtaggaagagggataatacaaatgaaatttattctggcaaattctgcga
gtgtgataatttcaactgtgatagatccaatggcttaatttgtggaggaaatggtgtttgcaagtgtcgtgtgtgtgagtgcaa
ccccaactacactggcagtgcatgtgactgttctttggatactagtacttgtgaagccagcaacggacagatctgcaatggccg
gggcatctgcgagtgtggtgtctgtaagtgtacagatccgaagtttcaagggcaaacgtgtgagatgtgtcagacctgccttgg
tgtctgtgctgagcataaagaatgtgttcagtgcagagccttcaataaaggagaaaagaaagacacatgcacacaggaatgttc
ctattttaacattaccaaggtagaaagtcgggacaaattaccccagccggtccaacctgatcctgtgtcccattgtaaggagaa
ggatgttgacgactgttggttctattttacgtattcagtgaatgggaacaacgaggtcatggttcatgttgtggagaatccaga
gtgtcccactggtccagacatcattccaattgtagctggtgtggttgctggaattgttcttattggccttgcattactgctgat
atggaagcttttaatgataattcatgacagaagggagtttgctaaatttgaaaaggagagaaaatgaatgccaaatgggacacggg
tgaaaatcctatttataagagtgccgtaacaactgtggtcaatccgaagtatgagggaaaatgagtactgcccgtgcaaatccc
acaacactgaatgcaaagtagcaatttccatagtcacagttaggtagctttagggcaatattgccatggttttactcatgtgca
ggttttgaaaatgtacaatatgtataattttttaaaatgttttattattttgaaaataatgttgtaattcatgccagggactgac
aaaagacttgagacaggatggttattcttgtcagctaaggtcacattgtgcctttttgacctttcttcctggactattgaaat

caagcttattggattaagtgatatttctatagcgattgaaagggcaatagttaaagtaatgagcatgatgagagtttctgttaa
tcatgtattaaaactgattttagctttacatatgtcagtttgcagttatgcagaatccaaagtaaatgtcctgctagctagtt
aaggattgttttaaatctgttattttgctatttgcctgttagacatgactgatgacatatctgaaagacaagtatgttgagagt
tgctggtgtaaaatacgtttgaaatagttgatctacaaaggccatgggaaaaattcagagagttaggaaggaaaaaccaatagc
tttaaaacctgtgtgccatttaagagttacttaatgtttggtaacttttatgccttcactttacaaattcaagccttagataa
aagaaccgagcaattttctgctaaaaagtccttgatttagcactatttacatacaggccatactttacaaagtatttgctgaat
ggggacctttgagttgaatttattttattattttatttgtttaatgtctggtgctttctatcacctcttctaatcttttaa
tgtatttgtttgcaattttggggtaagactttttatgagtactttttcttgaagtttagcggtcaatttgcctttttaatg
aacatgtgaagttatactgtggctatgcaacagctctcacctacgcgagtcttactttgagttagtgccataacagaccactgt
atgtttacttctcaccatttgagttgcccatcttgtttcacactagtcacattcttgttttaagtgcctttagttttaacagtt
ca
<212> Type : DNA
<211> Length : 3614
SequenceName : X07979
SequenceDescription :

Sequence
--------
<213> OrganismName : HUMAN
<400> PreSequenceString :
tgaagatcagctattagaagagaaagatcagttaagtcctttggacctgatcagcttgatacaagaactactgatttcaacttc
tttggcttaattctctcggaaacgatgaaatatacaagttatatcttggcttttcagctctgcatcgttttgggttctcttggc
tgttactgccaggacccatatgtaaaagaagcagaaaaccttaagaaatattttaatgcaggtcattcagatgtagcggataat
ggaactcttttcttaggcattttgaagaattggaaagaggagagtgacagaaaaataatgcagagccaaattgtctccttttac
ttcaaactttttaaaaactttaaagatgaccagagcatccaaaagagtgtggagaccatcaaggaagacatgaatgtcaagttt
ttcaatagcaacaaaaagaaacgagatgacttcgaaaagctgactaattattcggtaactgacttgaatgtccaacgcaaagca
atacatgaactcatccaagtgatggctgaactgtcgccagcagctaaaacagggaagcgaaaaaggagtcagatgctgtttcaa
ggtcgaagagcatcccagtaatggttgtcctgcctgcaatatttgaattttaaatctaaatctatttattaatatttaacatta
tttatatggggaatatattttagactcatcaatcaaataagtatttataatagcaacttttgtgtaatgaaaatgaatatcta
ttaatatatgtattatttataattcctatatcctgtgactgtctcacttaatcctttgttttctgactaattaggcaaggctat
gtgattacaaggctttatctcaggggccaactaggcagccaacctaagcaagatcccatgggttgtgtgtttatttcacttgat
gatacaatgaacacttataagtgaagtgatactatccagttactgccggtttgaaaatatgcctgcaatctgagccagtgcttt
aatggcatgtcagacagaacttgaatgtgtcaggtgaccctgatgaaaacatagcatctcaggagatttcatgcctggtgcttc
caaatattgttgacaactgtgactgtacccaaatggaaagtaactcatttgttaaaattatcaatatctaatatatatgaataa
agtgtaagttcacaact
<212> Type : DNA
<211> Length : 1193
SequenceName : X13274
SequenceDescription :

Sequence
--------
<213> OrganismName : HUMAN
<400> PreSequenceString :
gaattccgccgctgaccgaggcgtgcaaagactccagaattggaggcatgatgaagactctgctgctgtttgtggggctgctgc
tgacctgggagagtgggcaggtcctgggggaccagacggtctcagacaatgagctccaggaaatgtccaatcagggaagtaagt
acgtcaataaggaaattcaaaatgctgtcaacggggtgaaacagataaagactctcatagaaaaaacaaacgaagagcgcaaga
cactgctcagcaacctagaagaagccaagaagaagaaagaggatgccctaaatgagaccagggaatcagagacaaagctgaagg
agctcccaggagtgtgcaatgagaccatgatggccctctgggaagagtgtaagccctgcctgaaacagacctgcatgaagttct
acgcacgcgtctgcagaagtggctcaggcctggttggccgccagcttgaggagttcctgaaccagagctcgcccttctacttct
ggatgaatggtgaccgcatcgactccctgctggagaacgaccggcagcagacgcacatgctggatgtcatgcaggaccacttca
gccgcgcgtccagcatcatagacgagctcttccaggacaggttcttcacccgggagccccaggatacctaccactacctgccct
tcagcctgccccaccggaggcctcacttcttctttcccaagtcccgcatcgtccgcagcttgatgcccttctctccgtacgagc
ccctgaacttccacgccatgttccagcccttccttgagatgatacacgaggctcagcaggccatggacatccacttccacagcc
cggccttccagcacccgccaacagaattcatacgagaaggcgacgatgaccggactgtgtgccgggagatccgccacaactcca
cgggctgcctgcggatgaaggaccagtgtgacaagtgccgggagatcttgtctgtggactgttccaccaacaacccctcccagg
ctaagctgcggcgggagctcgacgaatccctccaggtcgctgagaggttgaccaggaaatacaacgagctgctaaagtcctacc
agtggaagatgctcaacacctcctccttgctggagcagctgaacgagcagtttaactgggtgtcccggctggcaaacctcacgc
aaggcgaagaccagtactatctgcgggtcaccacggtggcttcccacacttctgactcggacgttccttccggtgtcactgagg
tggtcgtgaagctctttgactctgatcccatcactgtgacggtccctgtagaagtctccaggaagaaccctaaatttatggaga
ccgtggcggagaaagcgctgcaggaataccgcaaaaagcaccgggaggagtgagatgtggatgttgcttttgcaccttacgggg
gcatcttgagtccagctcccccccaagatgagctgcagccccccagagagagctctgcacgtcaccaagtaaccaggccccagcc
tccaggccccccaactccgcccagcctctccccgctctggatcctgcactctaacactcgactctgctgctcatgggaagaacag

aattgctcctgcatgcaactaattcaataaaactgtcttgtgagctgaaa[?]aaaaaaaaaaaaaaaaaaaaaaggaattc
<212> Type : DNA
<211> Length : 1676
SequenceName : X14723
SequenceDescription :

Sequence
--------
<213> OrganismName : HUMAN
<400> PreSequenceString :
agcgagccgccacggtatgaccccaggggctctgctgatgctgctgggggcgctggggccgccgctcgccccaggcgtccgcgg
ctcggaggcggagggtcgactccgggagaaacttttctctggctatgatagctccgtgcggccagcgcgggaggtgggagaccg
tgtcagggtcagcgttggtctcatcctggcgcaactcatcagcctgaacgagaaggatgaagagatgagcacaaaggtgtactt
agacctggagtggactgactacaggctgagctgggaccctgcggagcacgacggcatcgattcgctccgcatcacggcggaatc
cgtgtggctccctgacgtggtgctactgaacaacaatgatgggaattttgacgtggctctggacattagcgtcgtggtgtcctc
cgacggctccgtgcgttggcaacccccgggcatctatcgcagcagctgcagcatccaggtcacctacttccccttcgactggca
gaattgcactatggtgttcagctcctacagctacgacagctcggaggtcagcctgcagacaggcctgggtcctgacgggcaagg
gcatcaggaaatccacattcatgaagggactttcattgagaatggccagtgggagaatatccacaagccctctcggctaatcca
gcctccaggcgatcctaggggagggagggaaggacagcgccaggaagtcatcttctacctcatcatccgccgcaagcctctctt
ctacctggtcaacgtcattgccccatgcatcctcatcactcttctggccatcttcgtcttctacctgccaccagatgcaggaga
gaagatggggctctcaatctttgccctgctgacccttactgtgttcctgctgctgctggctgacaaagtacctgagacctcact
atcagtacccattattatcaagtacctcatgtttaccatggtcctcgtcaccttctcagtcatccttagtgtcgtggttctcaa
cctgcaccaccgctcaccccacacccaccaaatgcccctttgggtccgtcagatcttcattcacaaacttccgctgtacctgcg
tctaaaaaggcccaaacccgagagagacctgatgccggagcccctcactgttcttctccaggaagtggctggggtcggggaac
agatgaatatttcatccggaagccgccaagtgattttctcttccccaaacccaataggttccagcctgaactgtctgcccctga
tctgcggcgatttatcgatggtccaaaccgggctgtggccctgcttccggagctacgggaggtcgtctcctctatcagctacat
cgctcgacagctgcaggaacaggaggaccacgatgcgctgaaggaggactggcagtttgtggccatggtagtggaccgcctctt
cctgtggactttcatcatcttcaccagcgttgggaccctagtcatcttcctggacgccacgtaccacttgccccctccagaccc
ctttccttgaagactggagggttgagaccaggcccctgccagttgaagtgagagagtttggtgatactgtcaagccctatcct
tctctgcctcttaactccttcacgaggaatctgggcctcttatttcgttctggg
<212> Type : DNA
<211> Length : 1650
SequenceName : X14830
SequenceDescription :

Sequence
--------
<213> OrganismName : HUMAN
<400> PreSequenceString :
ctaaagatctccctccaggcagcccttggctggtccctgcgagcccgtggagactgccagagatgtcctctttcggttacagga
ccctgactgtggccctcttcaccctgatctgctgtccaggatcggatgagaaggtattcgaggtacacgtgaggccaaagaagc
tggcggttgagcccaaagggtccctcgaggtcaactgcagcaccacctgtaaccagcctgaagtgggtggtctggagacctctc
taaataagattctgctggacgaacaggctcagtggaaacattacttggtctcaaacatctcccatgacacggtcctccaatgcc
acttcacctgctccgggaagcaggagtcaatgaattccaacgtcagcgtgtaccagcctccaaggcaggtcatcctgacactgc
aacccactttggtggctgtgggcaagtccttcaccattgagtgcagggtgcccaccgtggagcccctggacagcctcaccctct
tcctgttccgtggcaatgagactctgcactatgagaccttcgggaaggcagcccctgctccgcaggaggccacagccacattca
acagcacggctgacagagaggatggccaccgcaacttctcctgcctggctgtgctggacttgatgtctcgcggtggcaacatct
ttcacaaacactcagccccgaagatgttggagatctatgagcctgtgtcggacagccagatggtcatcatagtcacggtggtgt
cggtgttgctgtccctgttcgtgacatctgtcctgctctgcttcatcttcggccagcacttgcgccagcagcggatgggcacct
acggggtgcgagcggcttggaggaggctgccccaggccttccggccatagcaaccatgagtggcatggccaccaccacggtggt
cactggaactcagtgtgactcctcagggttgaggtccagccctggctgaaggactgtgacaggcagcagagacttgggacattg
ccttttctagcccgaatacaaacacctggactt
<212> Type : DNA
<211> Length : 1041
SequenceName : X15606
SequenceDescription :

Sequence
--------
<213> OrganismName : HUMAN
<400> PreSequenceString :
ggcccccccccccctagaaatgctcgaaccaggacggctcctggagtcctcgcgccctcgcagaaggactacggccccggcg
accccgggggcggggcttccggcgcgctgccttgtgggcacggtagttccgccgggtctggcttccgcctgccgagcggcccg

gaccgcaggccggactacacttcccgtcggcccgcctgctctcccgatgccgccttggcgcgagacgttggcaagcagagtgtc
tccaagatggccgcttggggaaggaggcgtcttggcccgggcagcagtggcggcagcgcccgagagagggtgagcttgtcggcc
acagactgctacattgtgcatgagatctacaatggggagaatgcccaagaccagtttgagtacgagctggagcaggccctggaa
gcccagtacaagtacattgtgattgagcccactcgcattggcgacgagacagcccgctggatcaccgtgggcaactgcctgcac
aagacggccgtgctggcgggcaccgcctgcctcttcaccccgttggcgctgcccttagattattcccactacatttccctgccc
gctggtgtgctgagcctggcctgctgcaccctctatgggatctcctggcagtttgacccttgctgcaagtaccaagtggagtac
gacgcctataaactgtcgcgcctgcctctgcacacactcacctcctccaccccggtggtgctggtccggaaggacgacctgcac
agaaagagactgcacaacacgatagcactggccgccctggtgtactgtgtaaagaagatttacgaactctatgccgtatgattt
cagtagaacagggagcgaagcaaaaccacccggcccacaagagacaacagagtattcagatcgccacactctgtgaggcagcag
agcctgggcaggtgtttggcttagtatttgttatttttaaaaaataacagatcacgggtgtacccagggtttttcagctcatta
cactaagatgtggatttccataacccaagaggggggtctgaggctgtggaagtccgactgggcagtggaatgctgatggaggca
gacgctgccgaggggggtgtggacgtgctttgggggaggtctttaagtctattgtttaactgtaccatccagagcccaccagaag
ctattgatcattaaaattatgagaatttcaactcc
<212> Type : DNA
<211> Length : 1211
      SequenceName : X51804
      SequenceDescription :

Sequence
--------
<213> OrganismName : HUMAN
<400> PreSequenceString :
tgcgttttggggggttcctggagtatcaatcatggatcaagaaactgtaggcaatgttgtcctgttggccatcgtcaccctcatc
agcgtggtccagaatggattctttgcccataaagtggagcacgaaagcaggacccagaatgggaggagcttccagaggaccgga
acacttgcctttgagcgggtctacactgccaaccagaactgtgtagatgcgtaccccactttcctcgctgtgctctggtctgcg
gggctactttgcagccaagttcctgctgcgtttgctggactgatgtacttgtttgtgcggcaaaagtactttgtcggttaccta
ggagagagaacgcagagcaccccctggctacatatttgggaaacgcatcatactcttcctgttcctcatgtccgttgctggcata
ttcaactattacctcatcttcttttcggaagtgactttgaaaactacataaagacgatctccaccaccatctcccctctactt
ctcatttcctaactctctgctgaatatggggttggt
<212> Type : DNA
<211> Length : 540
      SequenceName : X52195
      SequenceDescription :

Sequence
--------
<213> OrganismName : HUMAN
<400> PreSequenceString :
ggaattccgcttaggacagttggctgttaagtgacactgattctccccgccccgcctgacccccgagaaggaggcttgtccccc
gctgcgtgaggggggtggggaagtgggtagtgaattcggatctacctgggagggggggagtggaagttcccgccccggagagcggc
gaggcggcagccacagttgattatggaagattcccacaagagtaccacgtcagagacagcacctcaacctggttcagcagttca
gggagctcacatttctcatattgctcaacaggtatcatctttatcagaaagtgaggagtcccaggactcatccgacagcatagg
ctcctcacagaaagcccacgggatcctagcacggcgcccatcttacagaaaaatttgaaagacttatcttctgaagatacacg
gggcagaaaaggagacggagaaaattctggagtttctgctgctgtcacttctatgtctgttccaactcccatctatcagactag
cagcggacagtacattgccattgccccaaatggagccttacagttggcaagtccaggcacagatggagtacagggacttcagac
attaaccatgacaaattcaggcagtactcagcaaggtacaactattcttcagtatgcacagacctctgatggacagcagatact
tgtgcccagcaatcaggtggtcgtacaaactgcatcaggagatatgcaaacatatcagatccgaactacaccttcagctacttc
tctgccacaaactgtggtgatgacatctcctgtgactctcacctctcagacaactaagacagatgacccccaattgaaaagaga
aataaggttaatgaaaaacagagaagctgctcgagaatgtcgcagaaagaagaaagaatatgtgaaatgcctggaaaaccgagt
tgcagtcctggaaaatcaaaataaaactctaatagaagagttaaaaactttgaaggatctttattccaataaaagtgtttgatt
cctaagaaagaaaatattttgtggacatgcataaaaattaaatggatttcctagtggagttttataaattaaaaggtcaaaca
tgaagctttttatttaggcttttccaaatcaaggataaatatcttacgc
<212> Type : DNA
<211> Length : 1141
      SequenceName : X55544
      SequenceDescription :

Sequence
--------
<213> OrganismName : HUMAN
<400> PreSequenceString :
cgcgccaccgccgccgcccaggccatcgccaccctccgcagccatgtccaccaggtccgtgtcctcgtcctcctaccgcaggat
gttcggcggcccgggcaccgcgagccggccgagctccagccggagctacgtgactacgtccacccgcacctacagcctgggcag

cgcgctgcgccccagcaccagccgcagcctctacgcctcgtccccgggcggcgtgtatgccacgcgctcctctgccgtgcgcct
gcggagcagcgtgcccggggtgcggctcctgcaggactcggtggacttctcgctggccgacgccatcaacaccgagttcaagaa
cacccgcaccaacgagaaggtggagctgcaggagctgaatgaccgcttcgccaactacatcgacaaggtgcgcttcctggagca
gcagaataagatcctgctggccgagctcgagcagctcaagggccaaggcaagtcgcgcctgggggacctctacgaggaggagat
gcgggagctgcgccggcaggtggaccagctaaccaacgacaaagcccgcgtcgaggtggagcgcgacaacctggccgaggacat
catgcgcctccgggagaaattgcaggaggagatgcttcagagagaggaagccgaaaacaccctgcaatctttcagacaggatgt
tgacaatgcgtctctggcacgtcttgaccttgaacgcaaagtggaatctttgcaagaagagattgcctttttgaagaaactcca
cgaagaggaaatccaggagctgcaggctcagattcaggaacagcatgtccaaatcgatgtggatgtttccaagcctgacctcac
ggctgccctgcgtgacgtacgtcagcaatatgaaagtgtggctgccaagaacctgcaggaggcagaagaatggtacaaatccaa
gtttgctgacctctctgaggctgccaaccggaacaatgacgccctgcgccaggcaaagcaggagtccactgagtaccggagaca
ggtgcagtccctcacctgtgaagtggatgcccttaaaggaaccaatgagtccctggaacgccagatgcgtgaaatggaagagaa
ctttgccgttgaagctgctaactaccaagacactattggccgcctgcaggatgagattcagaatatgaaggaggaaatggctcg
tcaccttcgtgaataccaagacctgctcaatgttaagatggcccttgacattgagattgccacctacaggaagctgctggaagg
cgaggagagcaggatttctctgcctcttccaaacttttcctccctgaacctgagggaaactaatctggattcactccctctggt
tgatacccactcaaaaaggacacttctgattaagacggttgaaactagagatggacaggttatcaacgaaacttctcagcatca
cgatgaccttgaataaaaattgcacacactcagtgcagcaatatattaccagcaagaataaaaaagaaatccatatcttaaaga
aacagctttcaagtgcctttctgcagtttttcaggagcgcaagatagatttggaataggaataagctctagttcttaacaaccg
acactcctacaagatttagaaaaaagtttacaacataatctagtttacagaaaaatcttgtgctagaatactttttaaaaggta
ttttgaataccattaaaactgcttttttttccagcaagtatccaaccaacttggttctgcttcaataaatctttggaaaaac
ta
<212> Type : DNA
<211> Length : 1766
      SequenceName : X56134
      SequenceDescription :

Sequence
--------
<213> OrganismName : HUMAN
<400> PreSequenceString :
gaggcggctgcctgctgctctgcaggtaccatggagctgagctataggctcttcatctgcctcctgctctggggtagtactgag
ctgtgctacccccaacccctctggctcttgcagggtggagccagccatcctgagacgtccgtacagcccgtactggtggagtgt
caggaggccactctgatggtcatggtcagcaaagaccttttggcaccggaagctcatcagggctgctgacctcaccttgggc
ccagaggcctgtgagcctctggtctccatggacacagaagatgtggtcaggtttgaggttggactccacgagtgtggcaacagc
atgcaggtaactgacgatgccctggtgtacagcaccttcctgctccatgaccccgccccgtgggaaacctgtccatcgtgagg
actaaccgcgcagagattcccatcgagtgccgctaccccaggcagggcaatgtgagcagccaggccatcctgcccacctggttg
cccttcaggaccacggtgttctcagaggagaagctgactttctctctgcgtctgatggaggagaactggaacgctgagaagagg
tcccccaccttccacctgggagatgcagcccacctccaggcagaaatccacactggcagccacgtgccactgcggttgtttgtg
gaccactgcgtggccacaccgacaccagaccagaatgcctccccttatcacaccatcgtggacttccatggctgtcttgtcgac
ggtctcactgatgcctcttctgcattcaaagttcctcgacccgggccagatacactccagttcacagtggatgtcttccacttt
gctaatgactccagaaacatgatatacatcacctgccacctgaaggtcaccctagctgagcaggacccagatgaactcaacaag
gcctgttccttcagcaagccttccaacagctggttcccagtggaaggcccggctgacatctgtcaatgctgtaacaaaggtgac
tgtggcactccaagccattccaggaggcagcctcatgtcatgagccagtggtccacgtctgcttcccgtaaccgcaggcatgtg
acagaagaagcagatgtcaccgtgggggccactgatcttcctggacaggagtggtgaccatgaagtagagcagtgggctttgcc
ttctgacacctcagtggtgctgctgggcgtaggcctggctgtggtggtgtccctgactctgactgctgttatcctggttctcac
caggaggtgtcgcactgcctcccaccctgtgtctgcttccgaataaaagaagaaa
<212> Type : DNA
<211> Length : 1315
      SequenceName : X56777
      SequenceDescription :

Sequence
--------
<213> OrganismName : HUMAN
<400> PreSequenceString :
gggtacctgtgtggaaagaagcaaccaccactctatttgtgcatcagatgctaaagcatatgatacagaggtacataatgttt
gggccacacatgcctgtgtacccacagaccccaacccacaagaaatagtattgaaaaatgtgacagaaaattttaacatgtgga
aaaataacatggtagaacagatgcatgaggatataatcagtttatgggatcaaagcctaaaaccatgtgtaaaattaaccccac
tctgtgttactttaaattgcactgatttgacgaatgctactaataccactagtagtagcgggggaacgatggagggaggagaaa
taaaaaactgctctttcaatatcaccacaagcataagagataaggtgcagaaagaatatgcactgttttataaacttgatatag
taccaatagataataatactagctataggttgataagttgtaacacctcagtcatcacacaggcctgtccaaaggtatcctttg
agccaattcccatacattattgtgccccggctggttttgcgattctaaagtgtaaagataagaagttcaatggaaaaggaccat
gtacaaatgtcagcacagtacaatgtacacatggaattaggccagtagtatcaactcaactgctgttaaat
<212> Type : DNA

<211> Length : 659
      SequenceName : X57447
      SequenceDescription :

Sequence
--------
<213> OrganismName : HUMAN
<400> PreSequenceString :
gcggccgctttcgatttcgctttcccctaaatggctgagcttctcgccagcgcaggatcagcctgttcctgggactttccgaga
gccccgccctcgttccctcccccagccgccagtaggggaggactcggcggtacccggagcttcaggccccaccggggcgcggag
agtcccagacccggccgggaccgggacggcgtccgagtgccaatggctagctctaggtgtcccgctccccgcgggtgccgctgc
ctccccggagcttctctcgcatggctggggacagtactgctacttctcgccgactgggtgctgctccggaccgcgctgccccgc
atattctccctgctggtgcccaccgcgctgccactgctccgggtctgggcggtgggcctgagccgctgggccgtgctctggctg
gggcctgcggggtcctcagggcaacggttggctccaagagcgaaaacgcaggtgcccagggctggctggctgctttgaagcca
ttagctgcggcactgggcttggccctgccgggacttgccttgttccgagagctgatctcatggggagcccccgggtccgcggat
agcaccaggctactgcactggggaagtcaccctaccgccttcgttgtcagttatgcagcggcactgcccgcagcagccctgtgg
cacaaactcgggagcctctgggtgcccggcggtcagggcggctctggaaaccctgtgcgtcggcttctaggctgcctgggctcg
gagacgcgccgcctctcgctgttcctggtcctggtggtcctctcctctcttggggagatggccattccattctttacgggccgc
ctcactgactggattctacaagatggctcagccgataccttcactcgaaacttaactctcatgtccattctcaccatagccagt
gcagtgctggagttcgtgggtgacgggatctataacaacaccatgggccacgtgcacagccacttgcagggagaggtgtttggg
gctgtcctgcgccaggagacggagtttttccaacagaaccagacaggtaacatcatgtctcgggtaacagaggacacgtccacc
ctgagtgattctctgagtgagaatctgagcttatttctgtggtacctggtgcgaggcctatgtctcttggggatcatgctctgg
ggatcagtgtccctcaccatggtcaccctgatcaccctgcctctgcttttccttctgcccaagaaggtgggaaaatggtaccag
ttgctggaagtgcaggtgcgggaatctctggcaaagtccagccaggtggccattgaggctctgtcggccatgcctacagttcga
agctttgccaacgaggagggcgaagcccagaagtttagggaaaagctgcaagaaataaagacactcaaccagaaggaggctgtg
gcctatgcagtcaactcctggaccactagtatttcaggtatgctgctgaaagtgggaatcctctacattggtgggcagctggtg
accagtggggctgtaagcagtgggaaccttgtcacatttgttctctaccagatgcagttcacccaggctgtggaggtactgctc
tccatctaccccagagtacagaaggctgtgggctcctcagagaaaatatttgagtacctggaccgcacccctcgctgcccaccc
agtggtctgttgactcccttacacttggagggccttgtccagttccaagatgtctcctttgcctacccaaaccgcccagatgtc
ttagtgctacaggggctgacattcaccctacgccctggcgaggtgacggcgctggtgggacccaatgggtctgggaagagcaca
gtggctgccctgctgcagaatctgtaccagcccaccgggggacagctgctgttggatgggaagccccttccccaatatgagcac
cgctacctgcacaggcaggtggctgcagtgggacaagagccacaggtatttggaagaagtcttcaagaaaatattgcctatggc
ctgacccagaagccaactatggaggaaatcacagctgctgcagtaaagtctggggcccatagtttcatctctggactccctcag
ggctatgacacagaggtagacgaggctgggagccagctgtcaggggggtcagcgacaggcagtggcgttggcccgagcattgatc
cggaaaccgtgtgtacttatcctggatgatgccaccagtgccctggatgcaaacagccagttacaggtggagcagctcctgtac
gaaagccctgagcggtactcccgctcagtgcttctcatcacccagcacctcagcctggtggagcaggctgaccacatcctcttt
ctggaaggaggcgctatccgggagggggaacccaccagcagctcatggagaaaaaggggtgctactgggccatggtgcaggct
cctgcagatgctccagaatgaaagccttctcagacctgcgcactccatctccctcccttttcttctctctgtggtggagaacca
cagctgcagagtagcagctgcctccaggatgagttacttgaaatttgccttgagtgtgttacctcctttccaagctcctcgtga
taatgcagacttcctggagtacaaacacaggatttgtaattcctactgtaacggagtttagagccagggctgatgctttggtgt
ggccagcactctgaaactgagaaatgttcagaatgtacggaaagatgatcagctattttcaacataactgaaggcatatgctgg
cccataaacaccctgtaggttcttgatatttataataaaattggtgttttgt
<212> Type : DNA
<211> Length : 2824
      SequenceName : X57522
      SequenceDescription :

Sequence
--------
<213> OrganismName : HUMAN
<400> PreSequenceString :
cggctcgctggtaccggcagtgccatggcggccttcagcaagtacttgacggcgcgaaactcctcgctggctggtgccgcgttc
ctgctgctctgcctgctccacaagcggcgccgcgccctcggcctgcacggtaagaaaagtggaaaaccaccattacagaataat
gagaaagaaggaaaaaaagaacgagctgtggtggacaaagtgtttttctcaaggctcatacagatcctgaaaatcatggtccct
agaacatttgtaaagagacaggttacttggtacttattgctgttatgctggtgtctcgaacatattgtgatgtttggatgatt
caaaatgggacactaattgaaagtggtatcattggtcgtagcaggaaagatttcaagagatacttactcaacttcatcgctgcc
atgcctcttatctctctggttaataacttcttgaagtatgggttaaatgagcttaaactgtgcttccgagtaaggctcactaaa
tacctctatgaggagtatcttcaagccttcacatattataaaaaggggaatctggacaacagaatagctaatccagaccagctg
cttacacaagatgtagaaaaatttgtaacagtgtagtcgatctgtattcaaatcttagtaagccattttagacatagtttttg
tatatctttaagttaacgagtgcaattggagctcagggcccagcgagcatgatggcctacttggttgtttctggctattccta
actcgacttcgaagacccattggtaagatgacaataactgagcaaaagtatgaaggagaatatagatatgttaattctcggctc
atcacaaacagtgaagaaattgccttttacaatgggaataaaagagaaaagcagacagtccactcagtcttccgaaaactggtg
gaacacctacataatttcattttgtttcggttttcaatgggcttcattgatagtattattgccaaataccttgccactgttgtt

ggttacctagttgtcagtcgccctttcttagatttgtctcatcctcgacatctcaagagtacacattcggaacttctagaggat
tactaccaaagtggaagaatgcttttgcgaatgtctcaagctctgggtcgaatagttttggctgggcgtgaaatgactagattg
gccggttttactgctcggattacagaattaatgcaagtactgaaggatttaaatcatggcaaatatgagcgcacaatggtctca
caacaggaaaagggtattgaaggagtacaagtcattcccttgatacctggtgctggagaaatcattattgcagataacattata
aagtttgatcatgttcctttagcaacgccaaatggagatgttttgatccgagaccttaattttgaagttcgatctggggctaat
gttctaatttgtggtccaaatggctgcggaaagagttcacttttccgtgttcttggtgaattatggcctcttttggaggacgt
ctaactaaacctgaaagaagaaaattattttatgttcctcagagacccttacatgacccttggaacacttcgagatcaagtgata
tatccagatggacgagaagatcagaaaaggaagggaatttctgacctagtacagaaggaatacttagacaatgtccagttgggt
catatccttgaacgtgaaggaggctgggacagtgttcaggattggatggacgtactcagtggtggagaaaagcaaagaatggcg
atggcaagattattttatcataaaccccagtttgccattttggatgaatgcacaagtgcagttagtgtcgacgtggaaggctac
atttatagtcattgtcgaaaggttggcatcactctcttcactgtgtctcataggaaatctctttggaaacatcatgagtactac
ctgcatatggatggcagaggcaactatgaattcaaacagataacagaagatacagttgagtttggctcttagagaaatctggag
aactatacctgcttcagtgaaataattacagaatatacttagaaaggcaaagtacattgtaaaataaagttgagcttagttttt
tttaaaaaaaaaacaaagccaaccaaattatattagatacagaataatggagaacaagttgttaaaacatttaatattatata
ggatattgctaattgtgtatatgttggtttaattaatatatgtactaagaatgtccttattcttgtggttaaaaacctgccta
aattaaattgggcttcaatcatgtaacctgattcatcctgggatgtaaaccattcgaagtcagctaattggacttttatggctc
tatcttttccttcatgaagaaccctatttaaaactgggtcatcatttgtcctgttctagcaagatagtcttcagtttcatttcc
tgtgccctgtggtagttggaaaccatatcataatgtattatttaaatgtttaacatcattgcataacacgtttattatacagtg
gcagatttctttagctgccacagtaatactcattccttgtgtgtgtcttggagtgcatttgactccaggaaaagccattttggt
tttccttaactaaatgataaatgtacccctctcagtctgcagtattgagttgtttaaagtatatgtgcagtcttgcttacaagg
aggggttaccatgtatcacacctaatcttcccaatgtttgggaatattaaaacaccaacagtccttaacatgccaggctcaagg
tcttataagagttctagattttaagagaattagacaaatttgtgtgtgttagaagcccattcattagaagtgtggtggttatt
tggtattaaactcaaacagtgccaagcttgggaaggcactacaatgaaataatgcactgagtatgcaatgctatcactgtcttt
gactgtgatttatgtttaaaaagtatgttctaaaattattatatatacatgggtgaattatgtttccgaggcactgttttatc
tctgtgaatcttgaataactttttatatttgggttatgatgtcaaacgatcctaagcgaagatgatttcagttcatcaaatca
tcattaatgactttatgtattatttgcacagggagaattgaaactgagtataatcaataagctagatacgaaatcagtttctca
aactgagcttcagaaaggggcattttgtactcttgttttgcataactggttttgtttttttgcagaattaactataacaatca
ctggctacg
<212> Type : DNA
<211> Length : 3285
      SequenceName : X58528
      SequenceDescription :

Sequence
--------
<213> OrganismName : HUMAN
<400> PreSequenceString :
gcctcgctcgggcgcccagtggtcctgccgcctggtctcacctcgccatggttcgtctgcctctgcagtgcgtcctctggggct
gcttgctgaccgctgtccatccagaaccacccactgcatgcagagaaaaacagtacctaataaacagtcagtgctgttctttgt
gccagccaggacagaaactggtgagtgactgcacagagttcactgaaacggaatgccttccttgcggtgaaagcgaattcctag
acacctggaacagagagacacactgccaccagcacaaatactgcgaccccaacctagggcttcgggtccagcagaagggcacct
cagaaacagacaccatctgcacctgtgaagaaggctggcactgtacgagtgaggcctgtgagagctgtgtcctgcaccgctcat
gctcgcccggctttggggtcaagcagattgctacaggggtttctgataccatctgcgagccctgcccagtcggcttcttctcca
atgtgtcatctgctttcgaaaaatgtcacccttggacaagctgtgagaccaaagacctggttgtgcaacaggcaggcacaaaca
agactgatgttgtctgtggtccccaggatcggctgagagccctggtggtgatccccatcatcttcgggatcctgtttgccatcc
tcttggtgctggtctttatcaaaaaggtggccaagaagccaaccaataaggcccccaccccaagcaggaaccccaggagatca
atttcccgacgatcttcctggctccaacactgctgctccagtgcaggagactttacatggatgccaaccggtcacccaggagg
atggcaaagagagtcgcatctcagtgcaggagagacagtgaggctgcacccacccaggagtgtggccacgtggcaaacaggca
gttggccagagagcctggtgctgctgctgcaggggtgcaggcagaagcggggagctatgcccagtcagtgccagcccctc
<212> Type : DNA
<211> Length : 1004
      SequenceName : X60592
      SequenceDescription :

Sequence
--------
<213> OrganismName : HUMAN
<400> PreSequenceString :
atggataatctcagtgataccttgaagaagctgaagataacagctgttgacaagactgaggatagtttagaaggatgcttggat
tgtctgcttcaagccctggctcaaaataatacggaaacaagtgaaaaaatccaagcaagtggaatacttcagctgtttgcaagt
ctgttgactccacagtcttcctgcaaagccaaagtagctaacatcatagcagaagtagccaaaaatgagtttatgcgaattcca
tgtgtggatgctggattgatttcaccactggtgcagctgctaaatagcaaagaccaggaagtgctgcttcaaacgggcagggct
ctaggaaacatatgttacgatagccattcgcttcaagctcagcttatcaatatgggtgttattcctaccttagtgaaattactg

ggcatccactgccaaaatgcagctcttacagaaatgtgtcttgttgcatttggtaatttagcagaacttgagtcaagtaaagaa
cagtttgccagtacaaacattgctgaagagctagtaaaactcttcaagaaacaaatcgaacatgataagagagaaatgattttt
gaagttcttgctccattggcagaaaatgatgctattaaactacagctggttgaagcaggcctagtagagtgtctactagagatt
gttcagcaaaaagtggatagtgacaaagaagatgatattactgagctcaaaactggttcagatctcatggttttattacttctt
ggagatgaatccatgcagaagttatttgaaggaggaaaaggtagtgtatttcaaagggtactctcttggatcccatcaaataac
caccagctacagcttgctggagcattggcaattgcaaattttgccagaaatgatgcaaattgtattcatatggtagacaatggg
attgtagaaaaacttatggatttactggacagacatgtagaagatggaaatgtaacagtacagcatgcagcactaagtgccctc
agaaacctggccattccagttatagataaagcaaagatgttatcagctggggtcacagaggcagttttgaaatttcttaaatct
gaaatgcctcctgttcagttcaaacttctgggaacattaagaatgttaatagatgcacaagcagaagctgctgaacaattggga
aagaatgttaagttagtggagcgtttggtggaatggtgtgaagccaaagatcatgctggtgtgatgggggagtcaaacagactg
ctgtctgcccttatacgacacagtaaatcaaaagatgtaattaaaaccattgtgcagagtggtggcatcaagcatctagttacc
atggcaactagtgaacatgtaataatgcagaatgaagctcttgttgctttggcattaatagcagctttagaattggcactgct
gagaaagatctagaaagtgctaaacttgtacagattttacatagactgctagcagatgagagaagtgctcctgaaatcaaatat
aattccatggtcctgatatgtgctcttatgggatctgaatgtctacacaaggaagtacaggatttggcttttctagatgtcgta
tccaaacttcgcagtcatgagaacaaaagtgttcgccagcaggcctctctcacagagcagagacttactgtggaaagctga
<212> Type : DNA
<211> Length : 1677
      SequenceName : X63465
      SequenceDescription :

Sequence
--------
<213> OrganismName : HUMAN
<400> PreSequenceString :
ctaaaaataccattaagtaatagtattagcttttgtattctgagattcaacagcagcagtcacttccctccactcctatgtgta
tcccaggaccaccctggcggggagggctgaggtcagggaggtctgaagctggtcctgggctccgggggtgacagtgatgagga
actgggtgcacacatgagtggggcagccgggcctggccagagaagcaacacacacgtgcacagacatgtttatccacatacaca
tgtgcacgcatgtgcacaaacacattgcaggcaggcatgttgacgcctcaggcagcggaggaccctgactctgggccctgctga
cccgggcaaggcccattgtgatgcgtgccatgacctcagaatgtcactggtgcttagcacctatccgctctccagactgcgtct
gtgttctacggcagttacacacacgcagtggtattcacaagcggttttgtggactcaaaggtttctccctgagaggcataacc
caggccagctgattcatcagaatcaggtgagtgtgacctgctctcttccctccaggctgacttggggacagtggctatggtatg
ggcggtgttggcctctgggcagctacagaggagggtcatccctgagcactcaccggcgcccgttctacactgcccatgtagac
gattttctctttcgtcttcatggtggcttcgtagagtgggtgctgttcccaaatgtacccattcgacaggtgagccgtctgggg
tcagagaggcagtaactggcctgggaatccagacaagaccctgggttttgctctcagccctgctgtgtgccatgctagacttca
ggcctcaaccctgagacctccctgctctagatcccaaatctgcccagatttccgatccaatgggcagagcctggccctggcaga
gacactgggatggatccactgtgggtggggaggagggaagggtcctcagaacacacctggggcctaagctgggtcttgatggtc
actgtgggacccactggacacacacagtcccttgtctgggagtggcatggggagccttctgcccttgggcagttgtggaaagtg
aaggagccctggagagctggctgaggggagactatcttcccttgtgttcaaaggggtccaggcactggggctctccccaagtat
ttcttattctgtctggcctcgctttccttttgccctgagtattctcaggagggacggtccatctagatgtcctccaggagcaag
gacccactgttcttcatcagtgacccaggaaaatgaagcccccctcctgtggggacagctcagaatggtggagtccacagtccct
ccctgagagacatggtttccatgagcacagtggctgctttggagacagtaatcattttcatccccaaaaccaaacacactcctg
ctcaaatggtgttattgctaaagcagcttcactggttagactgaagggccatggtagcccaagtgatgagcggggtagaatgga
gcagtcaggagagatcttgttccccgtaggaaactgggcatctctgtggccctgaacatcccaggaggccgatcgtacagagac
ctctggtgcctgaccgcagttcacatccacatccctggaatagaccatcacaggctcttcacccttggcaggtggacaccattc
aacctgccggggcaggatggacatggtagagaatgcagatagtttgcaggcacaggagcggaaggacatacttatgaagtatga
caagggacaccgagctgggctgccagaggacaaggggcctgagcccgttggaatcaacagcagcattgatcgttttggcatttt
gcatgagacggagctgcctcctgtgactgcacgggaggcgaagaaaattcggcgggagatgacacgaacgagcaagtggatgga
aatgctgggagaatgggagacatataagcacagtagcaaactcatagatcgagtgtacaagggaattcccatgaacatccgggg
cccggtgtggtcagtcctcctgaacattcaggaaatcaagttgaaaaaccccggaagataccagatcatgaaggagaggggcaa
gaggtcatctgaacacatccaccacatcgacctggacgtgaggacgactctccggaaccatgtcttctttagggatcgatatgg
agccaagcagagggaactattctacatcctcctggcctattcggagtataacccggaggtgggctactgcagggacctgagcca
catcaccgccttgttcctcctttatctgcctgaggaggacgcattctgggcactggtgcagctgctggccagtgagaggcactc
cctgccaggattccacagcccaaatggtgggacagtccaggggctccaagaccaacaggagcatgtggtacccaagtcacaacc
caagaccatgtggcatcaggacaaggaaggtctatgcgggcagtgtgcctcgttaggctgcctt[?]tccggaacctgattgacgg
gatctctctcgggctcaccctgcgcctgtgggacgtgtatttggtggaaggagaacaggtgttgatgccaataaccagcattgc
tcttaaggttcagcagaagcgcctcatgaagacatccaggtgtggcctgtgggcacgtctgcggaaccaattcttcgatacctg
ggccatgaacgatgacaccgtgctcaagcatcttagggcctctacgaagaaactaacaaggaagcaaggggacctgccaccccc
aggcccaacagccctgggacgaaggtgtgtggcaggaagcccccagccagtctgaaccctgggggcagtcccaggagccaccca
ccatgccccaacggcttccccatgccaggcagcacacacccctccctctgggatcagcagactacaggcgtgtcgtcagtgtca
gaccacaggggccacacagagaccccaaggactccagagatgcagccaaacgcgagcaagggtccttggcacccaggcctgtgc
cggcttcacgtggtgggaagaccctctgcaaggggtataggcaggcccctccaggcccaccagcccagttccagcggcccattt
gctcagcttccccgccatgggcatctcgtttttccacgccctgtcctggtggggctgtccgggaagacacgtaccctgtgggca
ctcagggtgtgcccagcctggccctggctcagggaggacctcagggttcctggagattcctggagtggaagtcaatgccccggc

tcccaacggacctggatataggggcccttggttccccattatgatttgaacggagctgctgggtccgtgccatatcccagg
aggaccagctggccacctgctggcaggctgaacactgcggagaggttcacaacaaagatatgagttggcctgaggagatgtctt
ttacagcaaatagtagtaaaatagatagacaaaaggttcccacagaaaagggagccacaggtctaagcaacctgggaaacacat
gcttcatgaactcaagcatccagtgcgttagtaacacacagccactgacacagtatttatctcagggagacatctttatgaac
tcaacaggacaaatcccattggtatgaaggggcatatggctaaatgctatggtgatttagtgcaggaactctggagtggaactc
agaagagtgttgccccattaaagcttcggcggaccatagcaaaatatgctcccaagtttgatgggtttcagcaacaagactccc
aagaacttctggcttttctcttggatggtcttcatgaagatctcaaccgagtccatgaaaagccatatgtggaactgaaggaca
gtgatggccgaccagactgggaagtagctgcagaggcctgggacaaccatctaagaagaaatagatcaattattgtggatttgt
tccatggcagctaagatctcaagtcaaatgcaagacatgtgggcatataagtgtccgatttgaccctttcaatttttttgtctt
tgccactaccaatggacagttacatggacttagaaataacagtgattaagttagatggtactaccccctgtacggtatggactaa
gactgaatatggatgaaaagtacacaggtttaaaaaaacagctgagggatctctgtggacttaattcagaacaaatcctactag
cagaagtacatgattccaacataaagaactttcctcaggataaccaaaaagtacaactctcagtgagcggattttgtgtgcat
ttgaaattcctgtcccttcatctccaatttcagcttctagtccaacacaaatagatttctcctcttcaccatctacaaatggaa
tgttcaccctaactaccaatggggacctacccaaaccaatattcatccccaatggaatgccaaacactgttgtgccatgtggaa
ctgagaagaacttcacaaatggaatggttaatggtcacatgccatctcttcctgacagcccctttacaggttacatcattgcag
tccaccgaaaaatgatgaggacagaactgtatttcctgtcacctcaggagaatcgccccagcctctttggaatgccattgattg
ttccatgcactgtgcatacccagaagaaagacctatatgatgcggtttggattcaagtatcctggttagcaagaccactcccac
ctcaggaagctagtattcatgcccaggatcgtgataactgtatggctatcaatatccattcactctacgagttgtgcagaaag
atgggatctcctgtgcttggtgcccacagtatagattttgcagaggctgtaaaattgattgtggggaagacagagctttcattg
gaaatgcctatattgctgtggattggcaccccacagcccttcaccttcgctatcaaacatcccaggaaagggttgtagataagc
atgagagtgtggagcagagtcggcgagcgcaagccgagcccatcaacctggacagctgtctccgtgctttcaccagtgaggaag
agctaggggaaagtgagatgtactactgttccaagtgtaagacccactgcttagcaacaaagaagctggatctctggaggcttc
caccccttcctgattattcaccttaagcgatttcaatttgtaaatgatcagtggataaaatcacagaaaattgtcagatttcttc
gggaaagttttgatccgagtgctttttggtaccacgagacccggccctctgccagcataaaccactcacaccccaggggggatg
agctctccaagcccaggattctggcaagagaggtgaagaaagtggatgcgcagagttcggctggaaaagaggacatgctcctaa
gcaaaagcccatcttcactcagcgctaacatcagcagcagcccaaaaggttctccttcttcatcaagaaaaagtggaaccagct
gtccctccagcaaaaacagcagccctaatagcagcccacggactttggggaggagcaaagggaggctccggctgccccagattg
gcagcaaaaataagccgtcaagtagtaagaagaacttggatgccagcaaagagaatggggctggcagatctgtgagctggctg
acgccttgagccgagggcatatgcgggggggcagccaaccagagctggtcactcctcaggaccatgaggtagctttggccaatg
gattcctttatgagcatgaagcatgtggcaatggctgtggcgatggctacagcaatggtcagcttggaaaccacagtgaagaag
acagcactgatgaccaaagagaagacactcatattaagcctatttataatctatatgcaatttcatgccattcaggaattctga
gtgggggccattacatcacttatgccaaaaacccaaactgcaagtggtactgttataatgacagcagctgtgaggaacttcacc
ctgatgaaattgacaccgactctgcctacattcttttctatgagcagcaggggatagactacgcacaatttctgccaaagattg
atggcaaaaagatggcagacacaagcagtacggatgaagactctgagtctgattacgaaaagtactctatgttacagtaaagct
accactctggctgctagacagcttggtggcgagggagatgactccttgtagctgatacttggcaaaagtgtcactgaaagacaa
gctaaatgtagttattttatcctgttagaacaaaaattctaattaaaatagttaacttgaagagtagaaacaattgtattttga
agtctcatacaagctgtctgatagagaactttcaggcagatcccaccattagcctgtaaacaaaaggtgtggcaccagccacct
gggaccaaataagaattgaattgtgcttgtccagatatgaacaaatatgtagtgagtatagagtttaccaataatcataacaaa
tattaaagatttccttggagtcagaggaaaaaacaaacaattataatgttgtctagggacgacatgatacgctacctcctttt
cctgaagttttattccattatattgacaagatggagaaagcaagatcatgaaggtgtgcaaatgattcttacggcatggacaag
gatttttcaatttatttttaaactgtttccataccctttctttttcttgcttttgtttttgccattgtgtttacgtttgaga
cacaaccagtcattggtggcaggggcatagagtggtcagtctgaaagggaggctctcttaagagctatgtgccttccaaccaga
gggagacccagtagaaagaaaaacatcctgggaaatccagctaccagggccctcccagtggaggcatcttacatttaggctact
tcaagtatcctcagaaatgtattctgcaccccggccccgcccatgctgagggaaggggagcagttgccaatatttgcaccatc
ttcacatgcacatgttgcaacaagagcttctgggaaggtaagcggcatcggagctagatcacgtttcacaattagtggttattc
ttttctgtgtttgttttgcactttaaaaagagagaacacatgcaaatgaacttgcttgtgtgtatttgatggctctaagggct
ataaattacaaacaaaacacatcccagacattaggagttcataagtatatttaatgaaattggtggttttaggaagtcaacttt
agttttgctttgtttgcatgtccactggtttttttatttgatatttgtctttttttaaattttacagtagtcattgaaagtta
tgtttctttgcttacttcatttttttccctctaattatttaagattggaacaaaagtataaatattatttatttgaggtagaatt
tttttcatgtagtttcttaatatatacttgaaggaaatgtttcaccttatttttggtctttgtttattcatttagaccctgcaa
gttgattctcattgccagattccattaccctttcttcctcataggtagtaattaccaatgtaactaagcatttgtgttctgata
tctgaggccagtaactattaatatctagttctcagagcatttggaaaggttatcttaaatggctacctaaattgaaatcctttt
cagaaaaaatataattgcaagtaggtaggagtggcctaaattgtctaatgtaataaagtcagacaaaatgcacactttatagtt
tcaagattttcagtaaataaaatctgtccattcctacctggacatgtcccattaaaaagtggaagattttaaataatttcttta
cagatgttttatttaaacaggtagcacaatctactaatgttgtgtgatttgtgttatactggttgtaattaatttttttaattc
atgaactagcggaaaatttattaaattaactattaactacattcaccttgtaaattactgtataaaacttgttgacaatgcact
gactttagaaagatgttaatgtacataaatagagtgtaaataaaatagtgttgatgtactgaaatatgaactgtatcaaaagta
ttggtaattgtatatggggtgtacctgtttatctgttaactattatccaaacaaattaaatactgtggttgcctctatgtgctg
tttttcctcatacaagtaaacacagaaagtcaaaaaaaaaaaaaaaaaaaa
<212> Type : DNA
<211> Length : 8201
SequenceName : X63547
SequenceDescription :

Sequence
--------
<213> OrganismName : HUMAN
<400> PreSequenceString :
ttcccaccaggataatcccctttcaagagcgccaccacgacggtcatgaaccccaagtttgctgagagttaggagcacttggt
gaagacaaggccgtcaggacccaccatgtctgccccatcacgcggccgagacatggcttgccacagctcttgaggatgtcacca
attaaccagaaatccagttattttccgccctcaaaatgacagccatggccggccgggtgcttctgggggctcgtcgggggaca
gctccactctgactggcacagtctttgcatggagacttgaggagggagggcttgaggttggtgaggttaggtgcgtgtttcctg
tgcaagtcaggacatcagtctgattaaaggtggtgccaatttatttacatttaaacttgtcagggtataaaatgacatcccatt
aattatattgttaatcaatcacgtgtatagaaaaaaataaaacttcaatacaggctgtccatggaaactgggcactgtgtccg
ctgtattcccccgactggcaaggtggccaggcacacgtgggccctgtcctgcccgctgacctttgccacacaggcaca
<212> Type : DNA
<211> Length : 582
      SequenceName : X63926
      SequenceDescription :

Sequence
--------
<213> OrganismName : HUMAN
<400> PreSequenceString :
cgtccctcagtcctctctcaggagtgcacgaagttcaaggtcagcagctgccgggaatgcatcgagtcggggcccggctgcacc
tggtgccagaagctggtaagtgcct
<212> Type : DNA
<211> Length : 109
      SequenceName : X64073
      SequenceDescription :

Sequence
--------
<213> OrganismName : HUMAN
<400> PreSequenceString :
ccctccccagaacttcacagggccgggggatcctgactccattcgctgcgacacccggccacagctgctcatgaggggctgtgc
ggctgacgacatcatggaccccacaagcctcgctgaaacccaggaagaccacaatgggggccagaagcagctgtccccacaaaa
agtgacgctttacctgcgaccaggtaggcttgg
<212> Type : DNA
<211> Length : 201
      SequenceName : X64074
      SequenceDescription :

Sequence
--------
<213> OrganismName : HUMAN
<400> PreSequenceString :
cgtcttccagggagggggctctgcttctgcgggaagtgccgctgccacccgggctttgagggctcagcgtgccagtgcgagagg
accactgagggctgcctgaacccgcggcgtgttgagtgtagtggtcgtggccggtgccgctgcaacgtatgcgagtgccattca
ggctaccagctgcctctgtgccaggagtgccccggctgcccctcaccctgtggcaagtacatgtgagtgcag
<212> Type : DNA
<211> Length : 240
      SequenceName : X64083
      SequenceDescription :

Sequence
--------
<213> OrganismName : HUMAN
<400> PreSequenceString :
ctaccattttccctctcgattctatatgtacactcgggacaagttctcctgatcgaaaacggcaaaactaaggccccaagtagg
aatgccttagttttcggggttaacaatgattaacactgagcctcacacccacgcgatgccctcagctcctcgctcagcgctctc
accaacagccgtagcccgcagccccgctggacaccggttctccatccccgcagcgtagcccggaacatggtagctgccatcttt
acctgctacgccagccttctgtgcgcgcaactgtctggtcccgccccgtcctgcgcgagctgctgcccaggcaggttcgccggt
gcgagcgtaaaggggcggagctaggactgccttgggcggtacaaatagcagggaaccgcgcggtcgctcagcagtgacgtgaca
cgcagcccacggtctgtactgacgcgccctcgcttcttcctctttctcgactccatcttcgcggtagctgggaccgccgttcag
gtaagaatggggccttggctggatccgaagggcttgtagcaggttggctgcggggtcagaaggcgcgggggaaccgaagaacg

gggcctgctccgtggccctgctccagtccctatccgaactccttgggaggcactggccttccgcacgtgagccgccgcgaccac
catcccgtcgcgatcgtttctggaccgctttccactcccaaatctcctttatcccagagcatttcttggcttctcttacaagcc
gtcttttctttactcagtcgccaatatgcagctctttgtccgcgcccaggagctacacaccttcgaggtgaccggccaggaaac
ggtcgcccagatcaaggtaaggctgcttggtgcgccctgggttccattttcttgtgctcttcactctcgcggcccgagggaacg
cttacgagccttatctttccctgtaggctcatgtagcctcactggagggcattgccccggaagatcaagtcgtgctcctggcag
gcgcgcccctggaggatgaggccactctgggccagtgcggggtggaggccctgactaccctggaagtagcaggccgcatgcttg
gaggtgagtgagagaggaatgttctttgaagtaccggtaagcgtctagtgagtgtggggtgcatagtcctgacagctgagtgtc
acacctatggtaatagagtacttctcactgtcttcagttcagagtgattcttcctgtttacatccctcatgttgaacacagacg
tccatgggagactgagccagagtgtagttgtatttcagtcacatcacgagatcctagtctggttatcagcttccacactaaaaa
ttaggtcagaccaggccccaaagtgctctataaattagaagctggaagatcctgaaatgaaacttaagatttcaaggtcaaata
tctgcaactttgttctcattacctattgggcgcagcttctctttaaaggcttgaattgagaaaagaggggttctgctgggtggc
accttcttgctcttacctgctggtgccttcctttcccactacaggtaaagtccatggttccctggcccgtgctggaaaagtgag
aggtcagactcctaaggtgagtgagagtattagtggtcatggtgttaggactttttttcctttcacagctaaaccaagtccctg
ggctcttactcggtttgccttctccctccctggagatgagcctgagggaagggatgctaggtgtggaagacaggaaccagggcc
tgattaaccttcccttctccaggtggccaaacaggagaagaagaagaagaagacaggtcgggctaagcggcggatgcagtacaa
ccggcgctttgtcaacgttgtgcccacctttggcaagaagaagggccccaatgccaactcttaagtcttttgtaattctggctt
tctctaataaaaagccacttagttcagtcatcgcattgtttcatctttacttgcaaggcctcagggagaggtgtgcttctcgg
<212> Type : DNA
<211> Length : 2016
      SequenceName : X65921
      SequenceDescription :

Sequence
--------
<213> OrganismName : HUMAN
<400> PreSequenceString :
gccacggcgcggacgccatgcacacggaccctgactactcggctgcctatgtcgtcatagaaactgatgcagaagatggaatca
aggggtgtggaattaccttcactctgggaaaaggcactgaagttgattggtccagaaaagggcgtggtgcacctggcgacagcg
gccgtcctaaacgcggtgtgggacttgtgggccaagcaggagggaaagcctgtctggaagttacttgtggacatggatcccagg
atgctggtatcctgcatagatttcaggtacatcactgatgtcctgactgaggaggatgccctagaaatactgcagaaaggtcaa
attggtaaaaaagaaagagagaagcaaatgctggcacaaggataccctgcttacacgacatcgtgcgcctggctggggtactca
gatgacacgttgaagcagctctgtgcccaggcgctgaaggatggctggaccaggtttaaagtaaaggtgggtgctgatctccag
gatgacatgcgaagatgccaaatcatccgagacatgattggaccggaaaagactttgatgatggatgccaaccagcgctgggat
gtgcctgaggcggtggagtggatgtccaagctggccaagttcaagccattgtggattgaggagccaacctcccctgatgacatt
ctggggcacgccaccatttccaaggcactggtcccattaggaattggcattgccacaggagaacagtgccacaatagagtgata
tttaagcaactcctacaggcgaaggccctgcagttcctccagattgacagttgcagactgggcagtgtcaatgagaacctctca
gtattgctgatggccaaaaagtttgaaattcctgtttgccccatgctggtggagttggcctctgtgaactggtgcagcacctg
attatatttgactacatatcagtttctgcaagccttgaaaatagggtgtgtgagtatgttgaccacctgcatgagcatttcaag
tatcccgtgatgatccagcgggcttcctacatgcctcccaaggatcccggctactcaacagaaatgaaggaggaatctgtaaag
aaacaccagtatccagatggtgaagtttggaagaaactccttcctgctcaagaaaattaagtgctcagccccaacaactttttt
ctttctgaagtgaaagggcttaaaatttcttggaaatagttttacaaaaatggatttaaaaaatcctaccgatcaagatgagtt
cagctagaagtcataccaccctcaggaatcagctaaagcaaaaagaacttttacctcggcatccagcccaacccctaaagactg
acaatatccttcaagctcctttgaaagcaccctaaacagccatttccattttaatagttggatgcggattgtacccttcaatct
gaaagtcttcagctttgaagtcatcaatttctcaacttttcgaagaatcctgagctttgggaaaggtctgggttctcgctgaa
gctaaaaacaaaataaggccattattttgccataattgtacgacctgttgtaattgctcctcatgtccatgaaacaagtacaca
ggatgtgatcaacaaagttctatttacaggagtatgatcctgtcgataccttgccgtagttatgtaacatgattggagcgcaa
ccagctgttctcttgaccacagatcgagagtgaggggtattttgtgacattacacagcatcaggagcctggtgcctcatcaggt
gtaagttcttataaccactcttggcaaatttattaaagacaggaacacagtca
<212> Type : DNA
<211> Length : 1817
      SequenceName : X67098
      SequenceDescription :

Sequence
--------
<213> OrganismName : HUMAN
<400> PreSequenceString :
gctgcaggcggccctggccaggcttgacgatgaaatcgctcagaagaacaatgccctgaagaagatccgggagctggagggcca
catctcagacctccaggaggacctggactcagagcgggccgccaggaacaaggctgaaaagcagaagcgagacctcggcgagga
gctggaggccctaaagacagagctggaagacacactggacagcacagccactcagcaggagctcagggccaagagggagcagga
ggtgacggtgctgaagaaggccctggatgaagagacgcggtcccatgaggctcaggtccaggagatgaggcagaaacacgcaca
ggcggtggaggagctcacagagcagcttgagcagttcaagagggccaaggcgaacctagacaagaataagcagacgctggagaa
agagaacgcagacctggccggggagctgcgggtcctgggccaggccaagcaggaggtggaacataagaagaagaagctggaggc

gcaggtgcaggagctgcagtccaagtgcagcgatgggagcgggcccgggcggagctcaatgacaaagtccacaagctgcagaa
tgaagttgagagcgtcacagggatgcttaacgaggccgaggggaaggccattaagctggccaaggacgtggcgtccctcagttc
ccagctccaggacacccaggagctgcttcaagaagaaacccggcagaagctcaacgtgtctacgaagctgcgccagctggagga
ggagcggaacagcctgcaagaccagctggacgaggagatggaggccaagcagaacctggagcgccacatctccactctcaacat
ccagctctccgactcgaagaagaagctgcaggactttgccagcaccgtggaagctctggaagaggggaagaagaggttccagaa
ggagatcgagaacctcacccagcagtacgaggagaaggcggccgcgtatgataaactggaaaagaccaagaacaggcttcagca
ggagctggacgacctggttgttgatttggacaaccagcggcaactcgtgtccaacctggaaaagaagcagaggaaatttgatca
gttgttagccgaggagaaaaacatctcttccaaatacgcggatgagagggacagagctgaggcagaagccagggagaaggaaac
caaggccctgtccctggctcgggcccttgaagaggccttggaagccaaagaggaactcgagcggaccaacaaaatgctcaaagc
cgaaatggaagacctggtcagctccaaggatgacgtgggcaagaacgtccatgagctggagaagtccaagcgggccctggagac
ccagatggaggagatgaagacgcagctggaagagctggaggacgagctgcaagccacggaggacgccaaactgcggctggaagt
caacatgcaggcgctcaagggccagttcgaaagggatctccaagcccgggacgagcagaatgaggagaagaggaggcaactgca
gagacagcttcacgagtatgagacggaactggaagacgagcgaaagcaacgtgccctggcagctgcagcaaagaagaagctgga
aggggacctgaaagacctggagcttcaggccgactctgccatcaaggggagggaggaagccatcaagcagctacgcaaactgca
ggctcagatgaaggactttcaaagagagctggaagatgcccgtgcctccagagatgagatctttgccacagccaaagagaatga
gaagaaagccaagagcttggaagcagacctcatgcagctacaagaggacctcgccgccgctgagagggctcgcaaacaagcgga
cctcgagaaggaggaactggcagaggagctggccagtagcctgtcgggaaggaacgcactccaggacgagaagcgccgcctgga
ggcccggatcgcccagctggaggaggagctggaggaggagcagggcaacatggaggccatgagcgaccgggtccgcaaagccac
acagcaggccgagcagctcagcaacgagctggccacagagcgcagcacggcccagaagaatgagagttcccggcagcagcttga
gcggcagaacaaggagctccggagcaagctccacgagatggagggggccgtcaagtccaagttcaaatccaccatcgcggcgct
ggaggccaagattgcacagctagaggagcaggtcgagcaggaggccagagagaaacaggcggccaccaagtcgctgaagcagaa
agacaagaagctgaaggaaatcttgctgcaggtggaggacgagcgcaagatggccgagcagtacaaggagcaggcagagaaagg
caatgccagggtcaagcagctcaagaggcagctggaggaggcagaggaggagtcccagcgcatcaacgccaaccgcaggaagct
gcagcgggagctggatgaggccacggagagcaacgaggccatgggccgcgaggtgaacgcactcaagagcaagctcaggcgagg
aaacgagacctctttcgttccttctagaaggtctggaggacgtagagttattgaaaatgcagatggttctgaggaggaaacgga
cactcgagacgcagacttcaatggaaccaaggccagtgaataagcaactttctacagttttgcaccacggcaagaaaaccaaaa
accaaaacaaacaaacaaaaaaaaacccaacaacaacccagaacaaagcaaaacccagcagactgtacttagcattgtctaaat
ccattctcaaattccaaatatcacagacncctcacacaaggaatataaaaaccaccaccctccagcctgggcaacgtagtaaaa
cctcatctatacaagaatttaaaaataagctgggcgtggtggtacacacctgtggtcccagctactagggaggctgagccagga
agaacgctccagcccaggacttcgaggctgcaatgagctataattgcatcattgcactccagcctgggcaacagagaccctgtc
tcaaccaccaccaccaccaccaccccctactacccctgtattcaaggtaaaaattgaagtttgtatgatgtaagagatgagaaaa
acccaacaggaaacacagacacatcctccagttctatcaatggattgtgcagacactgagtttttagaaaaacatatccacggt
aaccggtccctggcaattctttttacatgaaatggggagaaagtcaccgaaatgggtgccgccggcccccactcccaattcatt
ccctaacctgcaaacctttccaacttctcacgtcaggcctttga
<212> Type : DNA
<211> Length : 3320
      SequenceName : X69292
      SequenceDescription :

Sequence
--------
<213> OrganismName : HUMAN
<400> PreSequenceString :
ctgtcagaatggccaccatggtaccatccgtgttgtggcccagggcctgctggactctgctggtctgctgtctgctgaccccag
gtgtccaggggcaggagttccttttgcgggtggagccccagaaccctgtgctctctgctggagggtccctgtttgtgaactgca
gtactgattgtcccagctctgagaaaatcgccttggagacgtccctatcaaaggagctggtggccagtggcatgggctgggcag
ccttcaatctcagcaacgtgactggcaacagtcggatcctctgctcagtgtactgcaatggctcccagataacaggctcctcta
acatcaccgtgtacgggctcccggagcgtgtggagctggcacccctgcctccttggcagccggtgggccagaacttcaccctgc
gctgccaagtggagggtgggtcgccccggaccagcctcacggtggtgctgcttcgctgggaggaggagctgagccggcagcccg
cagtggaggagccagcggaggtcactgccactgtgctggccagcagagacgaccacggagcccctttctcatgccgcacagaac
tggacatgcagccccaggggctgggactgttcgtgaacacctcagcccccgccagctccgaacctttgtcctgcccgtgaccc
cccgcgcctcgtggcccccggttcttggaggtggaaacgtcgtggccggtggactgcaccctagacgggcttttccagcct
cagaggcccaggtctacctggcgctgggggaccagatgctgaatgcgacagtcatgaaccacggggacacgctaacggccacag
ccacagccacggcgcgcgcggatcaggagggtgcccgggagatcgtctgcaacgtgaccctagggggcgagagacgggaggccc
gggagaacttgacggtctttagcttcctaggacccattgtgaacctcagcgagcccaccgcccatgaggggtccacagtgaccg
tgagttgcatggctggggctcgagtccaggtcacgctggacggagttccggccgcggccccggggcagccagctcaacttcagc
taaatgctaccgagagtgacgacggacgcagcttcttctgcagtgccactctcgaggtggacggcgagttcttgcacaggaaca
gtagcgtccagctgcgagtcctgtatggtcccaaaattgaccgagccacatgcccccagcacttgaaatggaaagataaaacga
gacacgtcctgcagtgccaagccaggggcaacccgtaccccgagctgcggtgtttgaaggaaggctccagccgggaggtgccgg
tggggatcccgttcttcgtcaacgtaacacataatggtacttatcagtgccaagcgtccagctcacgaggcaaatacaccctgg
tcgtggtgatggacattgaggctgggagctcccactttgtccccgtcttcgtggcggtgttactgaccctgggcgtggtgacta
tcgtactggccttaatgtacgtcttcagggagcaccaacggagcggcagttaccatgttagggaggagagcacctatctgcccc
tcacgtctatgcagccgacagaagcaatgggggaagaaccgtccagagctgagtgacgctgggatccgggatcaaagttggcgg

gggcttggctgtgccctcagattccgcaccaataaagccttcaaactccctaaaaaaaaaaaaaaaaaaaaaaaaaaaaa
aaaaaaaaaaaaaaaaaaaaaaaaaaaaaaaaaaaaaaaaaaaaaaa
<212> Type : DNA
<211> Length : 1817
      SequenceName : X69819
      SequenceDescription :

Sequence
--------
<213> OrganismName : HUMAN
<400> PreSequenceString :
gcgtgcaacccgacgatagagagctcggaggtgatccacaaatccaagcacccagagatccattgggatccttggcagatggac
atcagtgtcatttactaaccagcaggatggagacgacgcccttgaattctcagaagcagctatcagcgtgtgaagatggagaag
attgtcaggaaaacggagttctacagaaggttgttcccaccccaggggacaaagtggagtccgggcaaatatccaatgggtact
cagcagttccaagtcctggtgcgggagatgacacacggcactctatcccagcgaccaccaccaccctagtggctgagcttcatc
aaggggaacgggagacctggggcaagaaggtggatttccttctctcagtgattggctatgctgtggacctgggcaatgtctggc
gcttcccctacatatgttaccagaatggagggggggcattcctcctccccctacaccatcatggccattttggggaatcccgc
tctttacatggagctcgcactgggacagtaccaccgaaatggatgcatttcaatatggaggaaaatctgcccgattttcaaag
ggattggttatgccatctgcatcattgccttttacattgcttcctactacaacaccatcatggcctgggcgctatactacctca
tctcctccttcacggaccagctgccctggaccagctgcaagaactcctggaacactggcaactgcaccaattacttctccgagg
acaacatcacctggaccctccattccacgtcccctgctgaagaattttacacgcgccacgtcctgcagatccaccggtctaagg
ggctccaggacctgggggcatcagctggcagctggccctctgcatcatgctgatcttcactgttatctacttcagcatctgga
aaggcgtcaagacctctggcaaggtggtgtgggtgacagccaccttcccttatatcatcctttctgtcctgctggtgaggggtg
ccaccctccctggagcctggaggggtgttctcttctacttgaaacccaattggcagaaactcctggagacaggggtgtggatag
atgcagccgctcagatcttcttctctcttggtccgggctttggggtcctgctggcttttgctagctacaacaagttcaacaaca
actgctaccaagatgccctggtgaccagcgtggtgaactgcatgacgagcttcgtttcgggatttgtcatcttcacagtgctcg
gttacatggctgagatgaggaatgaagatgtgtctgaggtggccaaagacgcaggtcccagcctcctcttcatcacgtatgcag
aagcgatagccaacatgccagcgtccactttctttgccatcatcttctttctgatgttaatcacgctgggcttggacagcacgt
ttgcaggcttggagggggtgatcacggctgtgctggatgagttcccacacgtctgggccaagcgccgggagcggttcgtgctcg
ccgtggtcatcacctgcttctttggatccctggtcaccctgacttttggaggggcctacgtggtgaagctgctggaggagtatg
ccacggggcccgcagtgctcactgtcgcgctgatcgaagcagtcgctgtgtcttggttctatggcatcactcagttctgcaggg
acgtgaaggaaatgctcggcttcagcccggggtggttctggaggatctgctgggtggccatcagccctctgtttctcctgttca
tcatttgcagttttctgatgagcccgccacaactacgacttttccaatataattatccttactggagtatcatcttgggttact
gcataggaacctcatctttcatttgcatccccacatatatagcttatcggttgatcatcactccagggacatttaaagagcgta
ttattaaaagtattaccccggagacaccaacagaaattccttgtggggacatccgcttgaatgctgtgtaacacactcaccgag
aggaaaaaggcttctccacaacctcctcctccagttctgaggaggcacgcctgccttctcccctccgagtgaatgagtttgcc
<212> Type : DNA
<211> Length : 2099
      SequenceName : X70697
      SequenceDescription :

Sequence
--------
<213> OrganismName : HUMAN
<400> PreSequenceString :
ggcctggagtgcgacggcaaggtcaacatctgctgtaagaaacagttctttgtcagtttcaaggacatcggctggaatgactgg
atcattgctccctctggctatcatgccaactactgcgagggtgagtgcccgagccatatagcaggcacgtccgggtcctcactg
tccttccactcaacagtcatcaaccactacgcatgcggccatagcccctttgccaacctcaaatcgtgctgtgtgcccaccaag
ctgagacccatgtccatgttgtactatgatgatggtcaaaacatcatcaaaaaggacattcagaacatgatcgtggaggagtgc
gggtgctcctaa
<212> Type : DNA
<211> Length : 348
      SequenceName : X72498
      SequenceDescription :

Sequence
--------
<213> OrganismName : HUMAN
<400> PreSequenceString :
ctcgagagttgccggggaggagcggagcggtgcggaggctctgctcggatcgaggtctgcagcgcagcttcgggagcatgagtg
ctgcagtgactgcagggaagctggcacgggcaccggccgaccctgggaaagccggggtccccggagttgcagctcccggagctc
cggcggcggctccaccggcgaaagagatcccggaggtcctagtggacccacgcagccggcggcgctatgtgcggggccgctttt
tgggcaagggcggctttgccaagtgcttcgagatctcggacgcggacaccaaggaggtgttcgcgggcaagattgtgcctaagt

ctctgctgctcaagccgcaccagagggagaagatgtccatggaaatatccattcaccgcagcctcgcccaccagcacgtcgtag
gattccacggcttttcgaggacaacgacttcgtgttcgtggtgttggagctctgccgccggaggtctctcctggagctgcaca
agaggaggaaagccctgactgagcctgaggcccgatactacctacggcaaattgtgcttggctgccagtacctgcaccgaaacc
gagttattcatcgagacctcaagctgggcaaccttttcctgaatgaagatctggaggtgaaaataggggattttggactggcaa
ccaaagtcgaatatgacggggagaggaagaagaccctgtgtgggactcctaattacatagctcccgaggtgctgagcaagaaag
ggcacagtttcgaggtggatgtgtggtccattgggtgtatcatgtataccttgttagtgggcaaaccaccttttgagacttctt
gcctaaaagagacctacctccggatcaagaagaatgaatacagtattcccaagcacatcaaccccgtggccgcctccctcatcc
agaagatgcttcagacagatcccactgcccgcccaaccattaacgagctgcttaatgacgagttctttacttctggctatatcc
ctgcccgtctccccatcacctgcctgaccattccaccaaggttttcgattgctcccagcagcctggaccccagcaaccggaagc
ccctcacagtcctcaataaaggcttggagaacccccctgcctgagcgtccccgggaaaaagaagaaccagtggttcgagagacag
gtgaggtggtcgactgccacctcagtgacatgctgcagcagctgcacagtgtcaatgcctccaagccctcggagcgtgggctgg
tcaggcaagaggaggctgaggatcctgcctgcatccccatcttctgggtcagcaagtgggtggactattcggacaagtacggcc
ttgggtatcagctctgtgataacagcgtgggggtgctcttcaatgactcaacacgcctcatcctctacaatgatggtgacagcc
tgcagtacatagagcgtgacggcactgagtcctacctcaccgtgagttcccatcccaactccttgatgaagaagatcaccctcc
ttaaatatttccgcaattacatgagcgagcacttgctgaaggcaggtgccaacatcacgccgcgcgaaggtgatgagctcgccc
ggctgccctacctacggacctggttccgcacccgcagcgccatcatcctgcacctcagcaacggcagcgtgcagatcaacttct
tccaggatcacaccaagctcatcttgtgcccactgatggcagccgtgacctacatcgacgagaagcgggacttccgcacatacc
gcctgagtctcctggaggagtacggctgctgcaaggagctggccagccggctccgctacgcccgcactatggtggacaagctgc
tgagctcacgctcggccagcaaccgtctcaaggcctcctaatagctgccctcccctccggactggtgccctcctcactcccacc
tgcatctggggcccatactggttggctcccgcggtgccatgtctgcagtgtgcccccagccccggtggctgggcagagctgca
tcatccttgcaggtgggggttgctgtataagttattttgtacatgttcgggtgtgggttctacagccttgtccccctcccccct
caaccccaccatatgaattgtacagaatatttctatt
<212> Type : DNA
<211> Length : 2137
      SequenceName : X73458
      SequenceDescription :

Sequence
--------
<213> OrganismName : HUMAN
<400> PreSequenceString :
agtcctgcgtccgggccccgaggcgcagcagggcaccaggtggagcaccagctacgcgtggcgcagcgcagcgtccctagcacc
gagcctcccgcagccgccgagatgctgcgaacagagagctgccgccccaggtcgcccgccggacaggtggccgcggcgtccccg
ctcctgctgctgctgctgctgctcgcctggtgcgcgggcgcctgccgaggtgctccaatattacctcaaggattacagcctgaa
caacagctacagttgtggaatgagatagatgatacttgttcgtcttttctgtccattgattctcagcctcaggcatccaacgca
ctggaggagctttgctttatgattatgggaatgctaccaaagcctcaggaacaagatgaaaaagataatactaaaaggttctta
tttcattattcgaagacacagaagttgggcaagtcaaatgttgtgtcgtcagttgtgcatccgttgctgcagctcgttcctcac
ctgcatgagagaagaatgaagagattcagagtggacgaagaattccaaagtccctttgcaagtcaaagtcgaggatatttttta
ttcaggccacggaatggaagaaggtcagcagggttcatttaaaatggatgccagctaattttccacagagcaatgctatggaat
acaaaatgtactgacatttgttttcttctgaaaaaatccttgctaaatgtactctgttgaaaatccctgtgttgtcaatgtt
ctcagttgtaacaatgttgtaaatgttcaatttgttgaaaattaaaaaatctaaaaataaa
<212> Type : DNA
<211> Length : 817
      SequenceName : X76029
      SequenceDescription :

Sequence
--------
<213> OrganismName : HUMAN
<400> PreSequenceString :
gcctccagctgacctctggctcctgtcctctggctccacctgcaccgccctgctcttcctaaggggccaggaagcccccagaag
ctctaccatcgacgtgggtggtggcacccggctcaccctgagagcagagggcgtgcagggggctcagttctgagcccagccggc
ccaccatggcacggcggttccaggaggagctggccgccttcctcttcgagtatgacacccccgcatggtgctggtgcgtaata
agaaggtgggcgttatcttccgactgatccagctggtggtcctggtctacgtcatcgggtgggtgtttctctatgagaagggct
accagacctcgagcggcctcatcagcagtgtctctgtgaaactcaagggcctggccgtgacccagctccctggcctcggccccc
aggtctgggatgtggctgactacgtcttcccagcccaggggacaactccttcgtggtcatgaccaatttcatcgtgaccccga
agcagactcaaggctactgcgcagagcacccagaagggggcatatgcaaggaagacagtggctgtacccctgggaaggccaaga
ggaaggcccaaggcatccgcacgggcaagtgtgtggccttcaacgacactgtgaagacgtgtgagatctttggctggtgccccg
tggaggtggatgacgacatcccgcgccctgcccttctccgagaggccgagaacttcactcttttcatcaagaacagcatcagct
ttccacgcttcaaggtcaacaggcgcaacctggtggaggaggtgaatgctgcccacatgaagacctgcctctttcacaagaccc
tgcacccccctgtgcccagtcttccagcttggctacgtggtgcaagagtcaggccagaacttcagcaccctggctgagaagggtg
gagtggttggcatcaccatcgactggcactgtgacctggactggcacgtacggcactgcagacccatctatgagttccatgggc
tgtacgaagagaaaaatctctccccaggcttcaacttcaggtttgccaggcactttgtggagaacgggaccaactaccgtcacc

tcttcaaggtgtttgggattcgctttgacatcctggtggacggcaaggccgggaagtttgacatcatccctacaatgaccacca
tcggctctggaattggcatctttggggtggccacagttctctgtgacctgctgctgcttcacatcctgcctaagaggcactact
acaagcagaagaagttcaaatacgctgaggacatggggccaggggcggctgagcgtgacctcgcagctaccagctccaccctgg
gcctgcaggagaacatgaggacatcctgatgctcggccccaactcctgactgggtgcagcgtgaggcttcagcctggagccct
ggtgggtcccagccagggcagaggggcctccccaggaagtctcctaccctctcagccaggcagagagcagtttgccagaagctc
agggtgcatagtaggagagacctgtgcaaatctgagctccggctccgaccccacacaccctgagggaggcctaccctagcctca
gccgctcctggtgggggaatggctggggggttgggcaggaccctcccacacacctgcaccctagcttcgtgcttctctctccgga
ctctcattatccaacccgctgcctccatttctctagatctgtgctctccgatgtggcagtcagtaaccataggtgactaaatta
aactaaaataaaatagaatgaaacacaaaattcaattcctcggctgaactagccacatttcaactgctcagtagatacgtgtgg
ttagtggctgccatactggacagctcggggcattttcactgtcaaagaaagttctattagacagccctgcttgagccctgtttc
ttcctggcttcggtttccctggggaacttatcgacaatgcaagctcctgggcccacccccagacctcctgaaccaaaagctcca
gggctggccgtatgatctgtgtggatggcaaactccccaggccattctgggacctaagtttaagaagtgccgtcctcgaacttt
ctgactctaagctcctgagcgggagtcagacttagccctgagcctgcacttcctgttcaggtgcagacactgaacagggtctca
aacaccttcagcatgtgtgttgtgtgctcacgtgccacacagtgtctcatgcacacaacccagtgtacacaccacctacgtgca
cacagcatccttccacactgtgtatgtgaacagcttgggccctgcaaacacaaccatctacacacatctacacccccaagcaca
cacacatggtccgtgccatgtcacctccatagggaaaggcttctctccaagtgtgccaggccaggacagccctcccagccatga
atccttactcagctacctcgggttggggtgggagccccagccaaatcctgggctccctgcctgtggctcagccccagctcccaa
ggcctgcctggctctgtctgaacagaaggtctgggggaagcgaggggtggagtacaataaagggaatgaggacaaacaaaaaaa
aaaaaaaaaaaaaaaaaaaaaaaaaaaaaaaaaaa
<212> Type : DNA
<211> Length : 2643
      SequenceName : X83688
      SequenceDescription :

Sequence
--------
<213> OrganismName : HUMAN
<400> PreSequenceString :
tggcactaggggcagccactcagcccttcgagctgtaggggaggcgctgcgcccccggccccagccgatcgggggctcctggcg
ctactgccctccagagtccctcctgccgtctgacttgagtccctgctgctgcagtgcacgcccccctttcggcagccagggtgg
ggcccagacccaacctggcccctcctgcccccaccccgcctttgggtcgctgacccccaggctgtggtgagggtctgagagctg
gtaccacggagcctgggcaacctcctccgcccacccatgcccaccccgcatgaggctgagaagcagatcacagggccagaggag
gcggaccggccccccttcaatgtccagtcatgatacagcctctccagcggcccccagccgcaacccctgctgcctgtgctggtgc
tgctgctgtagctgctcctggaaccaagagcggcggcgcgcgtggcaggcctcccgggagagcaagctgcagcccctccccagc
tgtgaagtatgtgccacgccaagtcctgaggaggtgcagagctgggcgcagtcttttgacaagctgatgcacagcccagcggga
cgcagcgtgttccgggcgttcctgcggacagagtacagcgaggagaacatgctcttctggttggcctgcgaggagctgaaggcc
gaggccaaccagcatgtggtagacgagaaggcgaggctcatctacgaggactacgtatccatcctgtcccccaaggaggtgagc
ctggactcccgtgtgcgggagggcatcaacaagaagatgcaggagccgtccgcacacacgttcgacgacgcgcagctgcagatc
tacacgctcatgcaccgggactcctaccccgcttcctcagctctcccacctaccgtgccctgctgctgcaggggccatcacag
tcctcctccgaggcctaggccgcccccagcagcacagaccccgccgcctcctacggccgactctgggttcccttcaggtgttgg
tgtcggcagggctgtcagggcacatgcctggccggctggggtccccacccgcggagacggtcctagacccagtggggagtgctg
tgtctcctgggtctgcccacccgtggccaagcaggaactccaggtgcagggcgggcctccaggtgcagggcgggccagaagccc
cctcatcggcccgcagctggctggcccagtgctcctggctggggctcttgcgtggtgagagtaggggtcccccaggagccat
gctggacccatgagcctcgctggggcctcctccccaggcagccaatgggccccagacaagcctttggtggggaacagaacctcc
gcatcgtgtagttttgtgacataaggagacctcctacttgagctgtctgtaccccagaatcaaacacagaactcagaaccagat
ttaggccctcagaatcctgcactcaaggtggcaggacccaatttctgttttatatgttcatgaactttaaacctggaaacatgt
ccttactaggtgttttatcaaaaaaaagtttttattattgaagatattttaaagcaaaaaaaaaaaaaaaaa
<212> Type : DNA
<211> Length : 1587
      SequenceName : X91809
      SequenceDescription :

Sequence
--------
<213> OrganismName : HUMAN
<400> PreSequenceString :
gcgcggagcgggctccggagggaagtcccgagacaaagggaagcgccgccgccgccgccccgctcggtcctccacctgtccgct
acgctcgccggggctgcggccgcccgagggactttgaacatgtcggggatcgccctcagcagactcgcccaggagaggaaagca
tggaggaaagaccacccatttggtttcgtggctgtcccaacaaaaaatcccgatggcacgatgaacctcatgaactgggagtgc
gccattccaggaaagaaagggactccgtgggaaggaggcttgtttaaactacggatgcttttcaaagatgattatccatcttcg
ccaccaaaatgtaaattcgaaccaccattatttcacccgaatgtgtacccttcggggacagtgtgcctgtccatcttagaggag
gacaaggactggaggccagccatcacaatcaaacagatcctattaggaatacaggaacttctaaatgaaccaaatatccaagac
ccagctcaagcagaggcctacacgatttactgccaaaacagagtggagtacgagaaaagggtccgagcacaagccaagaagttt

gcgccctcataagcagcgaccttgtggcatcgtcagaaggaagggattggtttggcaagaacttgtttacaacatttttgcaaa
tctaaagttgctccatacaatgactagtcacctggggggttgggcggcgccatcttccattgccgccgcgggtgtgcggtct
cgattcgctgaattgcccgtttccatacagggtctcttccttcggtcttttgtattttgattgttatgtaaaactcgcttta
ttttaatattgatgtcagtatttcaactgctgtaaaattataaacttttatacttgggtaagtcccccaggggcgagttcctcg
ctctgggatgcaggcatgcttctcaccgtgcagagctgcacttggcctcagctggctgtatggaaatgcaccctccctcctgcc
gctcctctctagaaccttctagaacctgggctgtgctgcttttgagcctcagaccccagggcagcatctcggttctgcgccact
tcctttgtgtttatatggcgttttgtctgtgttgctgtttagagtaaataaactgtttatataaaaaaaa
<212> Type : DNA
<211> Length : 1163
      SequenceName : X96427
      SequenceDescription :

Sequence
--------
<213> OrganismName : HUMAN
<400> PreSequenceString :
aagcattgagtttactgcaaggtcagaaaagcatgcaaagcccttagagtggctgcaaagagctccaacaaaacaatttagaac
tttattaaggaataggggaagctaggaagaaactcaaaacatcaagatttaaatacgcttcttggtctccttgctataatta
tctgggataagcatgctgtttctgtctgtccctaacatgccctgtgattatccgcaaacaacacacccaagggcagaactttg
ttacttaaacaccatcctgtttgcttctttcctcaggaactgtggctgcaccatctgtcttcatcttcccgccatctgatgagc
agttgaaatctggaactgcctctgttgtgtgcctgctgaataacttctatcccagagaggccaaagtacagtggaaggtggata
acgccctccaatcgggtaactcccaggagagtgtcacagagcaggacagcaaggacagcacctacagcctcagcagcaccctga
cgctgagcaaagcagactacgagaaacacaaagtctacgcctgcgaagtcacccatcagggcctgagctcgcccgtcacaaaga
gcttcaacaggggagagtgttagagggagaagtgcccccacctgctcctcagttccagcctgaccccctcccatcctttggcct
ctgaccctttttccacaggggacctacccctattgcggtcctccagctcatctttcacctcacccccctcctcctccttggctt
taattatgctaatgttggaggagaatgaataaataaagtgaatctttgcacctgtggtttctctctttcctcatttaataatta
ttatctgttgttttaccaactactcaatttctcttataagggactaaatatgtagtcatcctaaggcgcataaccatttataaa
aatcatccttcattctattttaccctatcatcctctgcaagacagtcctccctcaaacccacaagccttctgtcctcacagtcc
cctgggccatggtaggagagacttgcttccttgtttcccctcctcagcaagccctcatagtccttt
<212> Type : DNA
<211> Length : 1075
      SequenceName : X96754
      SequenceDescription :

Sequence
--------
<213> OrganismName : HUMAN
<400> PreSequenceString :
cttttctcaagacaactacataagcagacaaaattgcaaagatctgccctgtgtcgagtatgacagccacgactcgtggctctc
cggtcggagggaatgacaaccagggccaggctcctgatggacagtctctgcccccccctccaacagaatcagacttcatcgcctg
attcttccaatgaaaattccccggcaactcccccagatgagcaaggtcaaggtgatgccccaccacagcttgaagatgaggaac
ctgcatttccacatactgacttggccaagttggatgacatgatcaacaggcctcgatgggtggttccagttttgccgaaagggg
aattagaagtgcttttagaagctgctattgatcttagtaaaaagggccttgatgttaaaagtgaagcatgtcagcgatttttcc
gtgatgggctaacaatatcattcactaaaattcttacagatgaagcagtgagtggctggaagtttgaaattcataggtgtctgg
tggagctatgtgtggccaagttgtcccaagactggtttccacttttagaacttcttgccatggccttaaatcctcattgcaaat
tccatatctacaatggtacacgtccatgtgaatcagtttcctcaagtgttcagttgcctgaagatgaactctttgctcgttctc
cagatcctcgatcaccaaagggttggctagtggatcttctcaacaaatttggcactttaaatgggttccagattttgcatgatc
gttttattaatggatcagcattaaacgttcaaataattgcagcccttattaaaccatttgggcaatgctatgagtttctcactc
ttcatacagtgaaaaagtactttcttccaataatagaaatggttccacagttttttagaaaacttaactgatgaagaactgaaaa
aagaagcaaagaatgaagccaaaaatgatgctctttcaatgattattaaatctttgaagaatttagcttcaagggttccaggac
aagaagaaactgttaaaaacttagaaatatttaggttaaaaatgatacttagattattgcaaatttcttctttcaatggaaaga
tgaatgcactgaatgaagttaataaggtgatatctagtgtatcatactatactcatcgacatggtaatcctgaggaggaagagt
ggctcacagctgaacgaatggcagaatggatacagcagaacaatatcttatccatagtgttgcgagatagtcttcatcagccac
agtatgtagaaaagttagagaagattcttcgttttgtcatcaaagaaaaagctctgaccttacaggatcttgataatatctggg
cagcacaggcagggaaacatgaagccattgtgaagaatgtacatgatctcctggcaaaattggcatgggattttctcctgaac
aacttgatcatccttttgattgttttaaggccagtcggacaaatgcgagtaaaaagcaacgtgaaaagctacttgagctgatac
gtcgtcttgcagaagatgataaagatggtgtgatggcacacagagtgttgaaccttctgtggaatctggctcacagtgatgatg
tgcctgtagatatcatggacctggctctcagtgcccacataaaaatactagattacagttgctcccaggaccgtgatacacaaa
agatccaatggatagatcgctttatagaagaacttcgcacaaatgacaaatgggttattcccgcactgaaacaaattagagaaa
tttgtagtttgtttggtgaagcgcctcaaaatttgagtcaaactcagcgaagtccccatgtgttttatcgccatgacttaatca
atcaacttcaacacaatcatgccctagttactttggtagcagaaaaccttgtaacttacatggaaagcatgagactatatgcta
gagaccatgaagattatgacccacaaactgtgaggctgggaagtagatatagtcatgttcaagaagttcaagaacggcttaact
tccttagattttattgaaggatggtcagctgtggctatgtgctcctcaggcaaaacaaatatggaaatgcttagctgagaatg

cagtttacctttgtgatcgtgaagcctgttttaagtggtattccaagttgatgggggatgaaccagacttagatcctgatatta
ataaggacttctttgaaagtaatgtgcttcagcttgatccttctctgttaactgaaaatggaatgaagtgttttgagcgattct
tcaaagctgtgaattgtcgagaaggaaaactagtagcaaaaaggagagcctatatgatggatgacttggagttaataggattag
attacctttggagggtcgtgattcagagtaatgatgatattgccagcagagctatagatctcctcaaagagatatacacgaacc
ttggtccaagactacaagtcaatcaggtggtgatccatgaagacttcattcagtcttgttttgatcgtctgaaggcttcctatg
acacattgtgtgttttggatggtgacaaagacagtgttaattgtgcaagacaggaagctgttcgaatggttcgagtattaactg
ttttaagggaatatataaatgaatgtgacagtgattatcatgaggaaagaacaattctccctatgtcgagagcattccgcggta
aacacctctcttttgtagttcgatttccaaaccagggcagacaggttgatgacttggaggtatggtctcatacaaatgatacaa
ttggttcagtacgacgatgtattctcaatcgtattaaagccaacgtagcccatacaaaaattgagctctttgtgggcggtgagc
tgatagatcctgcagatgatagaaagttgattggacaattaaacttaaaagataaatcgcttattacagccaaacttacacaga
taagttccaatatgccttcaagccctgatagctcttctgattcctccactggatctcctggaaaccatggtaatcattacagtg
atggtcccaatccagaagtggaaagctgtttgcctggagtgataatgtcactgcatcccagatacatctcttttctttggcaag
ttgcagacttaggtagcagcctaaatatgccaccccttagagatggagcaagagtacttatgaaacttatgccgccagatagca
caacgatagaaaaattaagagctatttgtttagaccatgccaaacttggagaaagcagccttagtccatctcttgactcacttt
tctttggtccttcagcctcacaagtgctatatctaacagaggtagtctatgccttgttaatgcctgctggtgcacctctggctg
atgattcctctgattttcagtttcacttcttgaaaagtggtggcctaccccttgtactgagtatgctaaccagaaataacttcc
taccgaatgcagatatggaaactcgaaggggtgcctacctcaatgctcttaaaatagccaagcttttgctaactgccattggct
atggtcatgttcgagctgtggcagaagcttgtcagccaggtgtagaaggtgtgaatcccatgacacagatcaaccaagttaccc
atgatcaagcagtggtgctacaaagtgcccttcagagcattcctaatccatcatccgagtgcatgcttagaaatgtgtcagttc
gtcttgctcagcagatatctgatgaggcttcaagatatatgcctgatatttgtgtaattagagctatacaaaaaattatctggg
catcaggatgtgggtcgttacagctagtatttagcccaaatgaagaaatcactaaaatttatgagaagaccaatgcaggcaatg
agccagacttggaagacgaacaggtttgctgtgaagcattggaagtgatgaccttatgttttgccttgattccaacagccttag
atgctcttagtaaagaaaaggcttggcagacattcatcattgacttactattgcactgtcacagcaaaactgttcgtcaggtgg
cacaggagcagttctttttaatgtgcaccagatgttgcatgggacaccggcctctactttcttcattactctactctttactg
ttttggggagcacagcaagagagagagagctaaacactcaggcgactactttactcttttaagacaccttcttaattacgcttaca
atagtaatattaatgtacccaatgctgaagttcttttcaataatgaaattgattggcttaaaagaattagggatgatgttaaa
gaacaggagaaacgggtattgaagagacgatcttagagggccaccttggagtgacaaaggagttactggcctttcaaacttctg
agaaaaaatttcatattggttgtgaaaaaggaggtgctaatctcattaaagaattaattgatgatttcatatttcctgcatcca
atgtttacctacagtatatgagaaatggagagcttccagctgaacaggctattccggtctgtggttcaccacctacaattaatg
ctggttttgaattacttgtagcattagctgttggctgtgtgaggaatctcaaacaaatagtagattctttgactgaaatgtatt
acattggcacagcaataactacttgtgaagcacttactgagtgggaatatctgccacctgttggaccccgcccacccaaaggat
tcgtggggctgaaaaatgccggtgctacttgttacatgaattctgtgattcagcaactctacatgattccttccattaggaacg
gtattcttgccattgaaggcacaggtagtgatgtagatgatgatatgtctggggatgagaagcaggacaatgagagcaatgttg
atcccagggatgatgtatttggatatcctcaacaatttgaagataaaccagcattaagtaaaactgaagatagaaaagagtaca
acattggtgtcctaagacaccttcaggtcatctttggtcatttagctgcttctcgactgcaatactatgtgcccagaggatttt
ggaaacagttcaggctttggggtgagcctgttaatctgcgtgaacaacacgatgctttagaatttttaattcattggtggata
gtttagatgaagctttaaaagctttaggacatccagctatgctaagtaaagtcttaggaggttcctttgctgatcagaagatct
gccaaggctgcccacataggtacgaatgtgaagaatcttttacgaccctaaacgtagacattagaaatcaccaaaatcttcttg
attctttggaacagtatgtcaaaggagatttactagaaggtgcaaatgcatatcattgtgaaaaatgcaataaaaaggttgata
ccgtaaagcgcttgctgattaaaaaattacctcctgttcttgctatacaactaaagcgatttgactatgactgggaaagagaat
gtgcaatcaagttcaatgattattttgaatttcctcgagagctggacatggaaccttacacagttgcaggtgtcgcaaagctgg
aaggggataatgtaaacccagagagtcagttgatacaacagagtgagcagtctgaaagtgagacagcaggaagcacaaaataca
gacttgtgggtgtgctcgtacacagtggtcaagcgagtgggggcattattattcttacatcatccaaaggaatggtggagatg
gtgagagaaatcgctggtataaatttgatgatggtgatgtaacagaatgtaaaatggatgatgacgaagaaatgaaaaaccagt
gttttggtggagagtacatgggagaagtgtttgatcacatgatgaagcgtatgtcatacaggcgccagaaaaggtggtggaatg
cttatatacctttttatgaacgaatggacacaatagaccaagatgatgagttgataagatatatatcagagcttgctatcacca
ccagacctcatcagattattatgccatcagccattgagagaagtgtacggaaacagaacgtacaattcatgcataaccgaatgc
agtacagtatggagtattttcagtttatgaaaaaactgcttacatgtaatggcgtttacttaaaccctcctcccggcaagatc
acctgttgcctgaagcagaagaaatcactatgatcagtattcaacttgctgctaggttcctctttactacaggatttcacacaa
agaaagtagtccgtggctctgccagtgattggtatgatgcattgtgtattctccttcgtcacagcaagaatgtacgtttttggt
ttgcaaaacttatagtctttattgcacatttttccttgcaagatggccatgtccttcaccttttgcctctcctggaccttcta
gtcaggcttatgacaacttaagcttgagtgatcacttactaagagcagtactaaatctcttgagaagggaagtttcagagcatg
ggcgtcatttacagcagtacttcaacctgtttgtaatgtatgccaatttaggtgtggcagagaagacacagcttctgaaattga
gtgtacctgctacttttatgcttgtgtctttagatgaaggtccaggtcctccaatcaaataccagtatgctgaattaggcaaat
tatactcagtagtgtcacagctgatccgctgttgcaatgtctcttcaagaatgcagtcttcaatcaatggtaatcctcctcttc
ccaatccttttggtgatcctaatttatcacaacctataatgccaattcagcagaatgtggcagacatttatttgtgagaacaa
gttatgtgaagaaaatcattgaagactgcagtaattcagaggaaaccgtcaaattgcttcgtttttgctgctgggagaatcctc
agttctcatctactgtcctcagtgaacttctctggcaggttgcatattcctatccctatgaactgcggccctatttggatctgc
ttttgcaaatcttactgattgaggactcctggcaaactcacagaattcataatgcactgaaaggaattccagatgaccgagatg
ggctgtttgacacaatccagcgctctaagaatcactatcaaaaaagagcataccagtgtataaaatgtatggtagctctattta
gtaactgtcctgttgcttaccaaatcctgcagggcaatggagatcttaaaagaaagtggacctgggcagtggaatggcttggag
atgaacttgaaagaagaccatatactggcaatcctcagtacacttacaacaattggtctcccccagtgcaaagcaatgaaacgt

ccaatggttatttcttggagagatcacatagtgctaggatgacacttgcaaaagcttgtgaactctgtccagaggaggagccag
atgaccaagatgctccagatgaacatgagtcgcctccacctgaagatgccccattgtaccccattcacctggatctcagtatc
aacagaataaccatgtgcatggacagccatatacaggcccagcagcacatcacatgaacaaccctcagagaactggccaacgag
cacaagaaaattatgaaggcagtgaagaagtatccccacctcaaaccaaggatcaatgaaatgcacataattaactggttccat
caagactgtgcacccaggccttacagtccaacctttttctgtgtctggctaatatttaaaactagaaaaactattcctaatcaa
catggagtggagagtttattcactgtcttatctgcagaaatttgctgtcaatatataacccgcctgcagtggaaagtgtatagt
gttttgtaataaatggcctgatgctaatgtgtaaatggcaaaggtgtatatagtatattaatgttgactgttaattcttaagca
agaaactttttcttgatgagactcacagatctacacaaactacaaaagttaattttctgttacacccactgcactcttaacca
gtgttgctgcctcatggcagtggatcagctcc
<212> Type : DNA
<211> Length : 8096
      SequenceName : X98296
      SequenceDescription :

Sequence
--------
<213> OrganismName : HUMAN
<400> PreSequenceString :
ccaggaggcacgctggttttccggggccgctccatcgcgccttcctcctgcgcctcgcttctccggtccagccgccatcttcct
ttccgcacaggggccgccgagcggggccatgcagccaacgctgcttctcagcctcctgggagccgtggggctggcggctgtcaa
ttccatgccagtggataacaggaaccacaatgaaggaatggtgactcgctgcatcattgaggtcctctcaaatgccttgtcgaa
gtccagcgctccacccatcacccctgagtgccgccaagtcctgaagacgagtagaaaagacgtcaaagacaaagagacaactga
aaatgaaaacacaaagtttgaagtaagattgttaagagacccagctgatgcctcggaagcccacgagtcctccagcaggggaga
ggcaggagccccaggggaggaggacatccaaggcccaacaaaggcagacacagagaaatgggcagagggaggcgggcacagccg
agagcgagcggatgagccccagtggagcctctatccctccgacagccaagtctctgaagaagtgaagacacgccattctgagaa
gagccagagagaggatgaggaggaggaggagggagagaactatcaaaaaggggagcgaggggaagatagcagtgaagagaaaca
ccttgaagagccaggagagacacaaaacgctttttctcaatgaaagaaagcaggcttcagctataaaaaaagaggagttagtggc
cagatcggaaacacatgctgccgggcattctcaggagaagacacatagccgagagaagagtagccaggagagtggagaggaggc
agggagccaggagaatcacccccaggagtctaaaggccaaccccgaagccaggaagaatctgaggaaggtgaggaagatgccac
ctctgaggtggacaaacgacgcacgaggcccagacaccaccacgggaggagcaggcccgacaggtcctctcaaggagggagtct
tccctctgaggaaaagggacacccccaggaggaatctgaggagtcaaacgtcagcatggccagtttaggggaaaagagggacca
ccattcaacccactacagggcttcagaggaagaacctgaatatggagaagaaataaagggttatccaggcgtccaggcccctga
ggacctggagtgggagcgctataggggcagaggaagtgaagaatacagggctccaagacctcagagtgaggagagttgggatga
ggaggacaagagaaactaccccagcttagagcttgataagatggcacatggatatggtgaagaaagtgaggaagagaggggcct
tgagccgggaaagggacgccatcacagaggcaggggaggggagccacgtgcctatttcatgtctgacaccagagaagagaaaag
gttcttgggtgaaggacaccaccgtgtccaagaaaaccagatggacaaggcaaggaggcatccacaaggtgcgtggaaagagct
ggacagaaattatctcaactacggtgaggaaggagccccagggaagtggcagcagcagggagacctgcaggacactaaagaaaa
cagggaggaagctaggtttcaagataaacaatatagctcccatcacacagctgaaaagaggaagagattaggggaactgttcaa
cccatactacgaccctctccagtggaagagcagccattttgaaagaagagacaacatgaatgacaatttctcgagggtgagga
ggaaaatgagctgaccttgaacgagaagaatttcttcccagaatacaactatgactggtgggagaaaaagcccttctctgagga
tgtgaactgggggtatgagaagagaaacctcgccagggtccccaagctggacctgaaaaggcaatatgacagggtggcccaact
ggaccagctccttcactacaggaagaagtcagctgagtttccagacttctatgattctgaggagccggtgagcacccaccagga
ggcagaaaatgaaaaggacagggctgaccagacagtcctgacagaggacgagaaaaaagaactcgaaaacttggctgcaatgga
tttggaactacagaagatagctgagaaattcagccaaaggggctgactgtcattggagcggtgggcactgttaagaagcagcca
tcacatgatctgtttttcaccacttcactgaaagacaccatttatatacccaagggcagaaagtagaacttactattcattaaa
tgtttgacacaattggaattgtctttaatttctgtcagaatgctattgaaaatgtgaattgcatgacttgtagcatattctttt
ctgcaaaatagacatattaacatgcttatgacaatgactgtgctactgtctttggaaaaatgtttgtctcagttggaaataata
aaagattcacctgagacc
<212> Type : DNA
<211> Length : 2454
      SequenceName : Y00064
      SequenceDescription :

Sequence
--------
<213> OrganismName : HUMAN
<400> PreSequenceString :
cccggacgctcgcgggtctcggaacgcatcccgccgcgggggcttcggccgtggcatgggcgccgcgggcctgctcggggtttt
cttggctctcgtcgcaccgggggtcctcgggatttcttgtggctctcctccgcctatcctaaatggccggattagttattattc
taccccattgctgttggtaccgtgataaggtacagttgttcaggtaccttccgcctcattggagaaaaaagtctattatgcat
aactaaagacaaagtggatggaacctgggataaacctgctcctaaatgtgaatatttcaataaatattcttcttgccctgagcc
catagtaccaggaggatacaaaattagaggctctacaccctacagacatggtgattctgtgacatttgcctgtaaaaccaactt
ctccatgaacggaaacaagtctgtttggtgtcaagcaaataatatgtgggggccgacacgactaccaacctgtgtaagtgtttt

cccctctcgagtgtccagcacttcctatgatccacaatggacatcacacaagtgagaatgttggctccattgctccaggattgtc
tgtgacttacagctgtgaatctggttacttgcttgttggagaaaagatcattaactgtttgtcttcgggaaaatggagtgctgt
ccccccacatgtgaagaggcacgctgtaaatctctaggacgatttcccaatggaaggtaaaggagcctccaattctccgggt
tggtgtaactgcaaacttttctgtgatgaagggtatcgactgcaaggcccaccttctagtcggtgtgtaattgctggacaggg
agttgcttggaccaaaatgccagtatgtgaagaaatttttgcccatcacctcccctattctcaatggaagacatataggcaa
ctcactagcaaatgtctcatatggaagcatagtcacttacacttgtgacccggacccagaggaaggagtgaacttcatccttat
tggagagagcactctccgttgtacagttgatagtcagaagactgggacctggagtggccctgccccacgctgtgaactttctac
ttctgcggttcagtgtccacatccccagatcctaagaggccgaatggtatctgggcagaaagatcgatatacctataacgacac
tgtgatatttgcttgcatgtttggcttcaccttgaagggcagcaagcaaatccgatgcaatgcccaaggcacatgggagccatc
tgcaccagtctgtgaaaaggaatgccaggcccctcctaacatcctcaatgggcaaaaggaagatagacacatggtccgctttga
ccctggaacatctataaaatatagctgtaaccctggctatgtgctggtgggagaagaatccatacagtgtacctctgaggtgtg
gacacccctgtaccccaatgcaaagtggcagcgtgtgaagctacaggaaggcaactcttgacaaaaccccagcaccaatttgt
tagaccagatgtcaactcttcttgtggtgaagggtacaagttaagtgggagtgtttatcaggagtgtcaaggcacaattccttg
gtttatggagattcgtctttgtaaagaaatcacctgcccaccaccccctgttatctacaatggggcacacaccgggagttcctt
agaagattttccatatggaaccacggtcacttacacatgtaaccctgggccagaaagaggagtggaattcagcctcattggaga
gagcaccatccgttgtacaagcaatgatcaagaaagaggcacctggagtggccctgctcccctgtgtaaactttccctccttgc
tgtccagtgctcacatgtccatattgcaaatggatacaagatatctggcaaggaagccccatatttctacaatgacactgtgac
attcaagtgttatagtggatttactttgaagggcagtagtcagattcgttgcaaagctgataacacctgggatcctgaaatacc
agtttgtgaaaaagaaacatgccagcatgtgagacagagtcttcaagaacttccagctggttcacgtgtggagctagttaatac
gtcctgccaagatgggtaccagttgactggacatgcttatcagatgtgtcaagatgctgaaaatggaatttggttcaaaaagat
tccactttgtaaagttattcactgtcaccctccaccagtgattgtcaatggaagcacacaggcatgatggcagaaaactttct
atatggaaatgaagtctcttatgaatgtgaccaaggattctatctcctgggagagaaaaaattgcagtgcagaagtgattctaa
aggacatggatcttggagcgggccttccccacagtgcttacgatctcctcctgtgactcgctgccctaatccagaagtcaaaca
tgggtacaagctcaataaaacacattctgcatattcccacaatgacatagtgtatgttgactgcaatcctggcttcatcatgaa
tggtagtcgcgtgattaggtgtcatactgataacacatgggtgccaggtgtgccaacttgtatcaaaaaagccttcatagggtg
tccacctccgcctaagacccctaacggaaccatactggtggaaacatagctcgatttctcctggaatgtcaatcctgtacag
ctgtgaccaaggctacctgctggtgggagaggcactccttctttgcacacatgagggaacctggagccaacctgcccctcattg
taaagaggtaaactgtagctcaccagcagatatggatggaatccagaaagggctggaaccaaggaaaatgtatcagtatggagc
tgttgtaactctggagtgtgaagatgggtatatgctggaaggcagtccccagagccagtgccaatcggatcaccaatggaaccc
tcccctggcggtttgcagatcccgttcacttgctcctgtcctttgtggtattgctgcaggtttgatacttcttaccttcttgat
tgtcgttaccttatacgtgatatcaaaacacagagcacgcaattattatacagatacaagccagaaagaagcttttcatttaga
agcacgagaagtatattctgttgatccatacaacccagccagctgatcagaagacaaactggtgtgtgcctcattgcttggaat
tcagcggaatattgattagaaagaaactgctctaatatcagcaagtctctttatatggcctcaagatcaatgaaatgatgtcat
aagcgatcacttcctatatgcacttattctcaagaagaacatctttatggtaaagatgggagcccagtttcactgccatatact
cttcaaggactttctgaagcctcacttatgagatgcctgaagccaggccatggctataaacaattacatggctctaaaaagttt
tgcccttttttaaggaaggcactaaaaagagctgtcctggtatctagacccatcttcttttttgaaatcagcatactcaatgttac
tatctgcttttggttataatgtgtttttaattatctaaagtatgaagcattttctggggttatgatggccttacctttattagg
aagtatggttttattttgatagtagcttcctcctctggttggtgttaatcatttcattttttacccttacttggtttgagtttct
ctacattactgtatatactttgcctttccataatcactcagtgattgcaatttgcacaagtttttttaaattatgggaatcaag
atttaatcctagagatttggtgtacaattcaggctttggatgtttctttagcagttttgtgataagttctagttgcttgtaaaa
tttcacttaataatgtgtacattagtcattcaataaattgtaattgtaaagaaaacaaa
<212> Type : DNA
<211> Length : 3923
      SequenceName : Y00649
      SequenceDescription :

Sequence
--------
<213> OrganismName : HUMAN
<400> PreSequenceString :
cgctcctcgggctgcccctcggttgacaatggtctccaggatggtctctaccatgctatctggcctactgttttggctggcatc
tggatggactccagcatttgcttacagcccccggacccctgaccgggtctcagaagcagatatccagaggctgcttcatggtgt
tatggagcaattgggcattgccaggccccgagtggaatatccagctcaccaggccatgaatcttgtgggcccccagagcattga
aggtggagctcatgaaggacttcagcatttgggtccttttggcaacatccccaacatcgtggcagagttgactggagacaacat
tcctaaggactttagtgaggatcaggggtacccagaccctccaaatccctgtcctgttggaaaaacagatgatggatgtctaga
aaacacccctgacactgcagagttcagtcgagagttccagttgcaccagcatctctttgatccggaacatgactatccaggctt
gggcaagtggaacaagaaactcctttacgagaagatgaagggaggagagagacgaaagcggaggagtgtcaatccatatctaca
aggacagagactggataatgttgttgcaaagaagtctgtccccccattttcagatgaggataaggatccagagtaaagagaaga
tgctagacgaaaacccacattacctgttaggcctcagcatggcttatgtgcacgtgtaaatggagtccctgtgaatgacagcat
gtttcttacatagataattatggatacaaagcagctgtatgtagatagtgtattgtcttcacaccgatgattctgctttttgct
aaattagaataagagctttttttgtttcttgggttttaaaatgtgaatctgcaatgatcataaaaattaaaatgtgaatgtcaa
caataaaaagcaagactatgaaaggctcagatttcttgcagtttaaaatggtgtctgaggttgtactatttggccaagtctgt
agaaagctgtcatttgattttgattatgtagttcatccagcccttgggcattgttatacaccagtaaagaaggctgtactcaag

aggaggagctgacacatttcacttggctgcgtcttaataaacatgaatgcaagcattggc
<212> Type : DNA
<211> Length : 1152
      SequenceName : Y00757
      SequenceDescription :

Sequence
--------
<213> OrganismName : HUMAN
<400> PreSequenceString :
cgtggtttgtagatgtgcttggggagaatggggcctcttctccaagaagcccggagcctgtcggccgccggcgcccggtctc
cccttctgctgcggaggatccctgctggcggttgtggtgctgcttgcgctgccggtggcctggggtcaatgcaatgccccagaa
tggcttccatttgccaggcctaccaacctaactgatgagtttgagtttcccattgggacatatctgaactatgaatgccgccct
ggttattccggaagaccgttttctatcatctgcctaaaaaactcagtctggactggtgctaaggacaggtgcagacgtaaatca
tgtcgtaatcctccagatcctgtgaatggcatggtgcatgtgatcaaaggcatccagttcggatcccaaattaaatattcttgt
actaaaggataccgactcattggttcctcgtctgccacatgcatcatctcaggtgatactgtcatttgggataatgaaacacct
atttgtgacagaattccttgtgggctacccccaccatcaccaatggagatttcattagcaccaacagagagaattttcactat
ggatcagtggtgacctaccgctgcaatcctggaagcggagggagaaaggtgtttgagcttgtgggtgagccctccatatactgc
accagcaatgacgatcaagtgggcatctggagcggccccgcccctcagtgcattatacctaacaaatgcacgcctccaaatgtg
gaaaatggaatattggtatctgacaacagaagcttattttccttaaatgaagttgtggagtttaggtgtcagcctggctttgtc
atgaaaggaccccgccgtgtgaagtgccaggccctgaacaaatgggagccggagctaccaagctgctccagggtatgtcagcca
cctccagatgtcctgcatgctgagcgtacccaaagggacaaggacaacttttcacctgggcaggaagtgttctacagctgtgag
cccggctacgacctcagaggggctgcgtctatgcgctgcacaccccagggagactggagccctgcagcccccacatgtgaagtg
aaatcctgtgatgacttcatgggccaacttcttaatggccgtgtgctatttccagtaaatctccagcttggagcaaaagtggat
tttgtttgtgatgaaggatttcaattaaaaggcagctctgctagttactgtgtcttggctggaatggaaagcctttggaatagc
agtgttccagtgtgtgaacaaatcttttgtccaagtcctccagttattcctaatgggagacacacaggaaaacctctggaagtc
tttccctttggaaaagcagtaaattacacatgcgacccccacccagacagagggacgagcttcgacctcattggagagagcacc
atccgctgcacaagtgaccctcaagggaatggggtttggagcagccctgcccctcgctgtggaattctgggtcactgtcaagcc
ccagatcattttctgtttgccaagttgaaaacccaaaccaatgcatctgactttcccattgggacatctttaaagtacgaatgc
cgtcctgagtactacgggaggccattctctatcacatgtctagataacctggtctggtcaagtcccaaagatgtctgtaaacgt
aaatcatgtaaaactcctccagatccagtgaatggcatggtgcatgtgatcacagacatccaggttggatccagaatcaactat
tcttgtactacagggcaccgactcattggtcactcatctgctgaatgtatcctctcgggcaatgctgcccattggagcacgaag
ccgccaatttgtcaacgaattccttgtgggctacccccaccatcgccaatggagatttcattagcaccaacagagagaatttt
cactatggatcagtggtgacctaccgctgcaatcctggaagcggagggagaaaggtgtttgagcttgtgggtgagccctccata
tactgcaccagcaatgacgatcaagtgggcatctggagcggcccggcccctcagtgcattatacctaacaaatgcacgcctcca
aatgtggaaaatggaatattggtatctgacaacagaagcttattttccttaaatgaagttgtggagtttaggtgtcagcctggc
tttgtcatgaaaggaccccgccgtgtgaagtgccaggccctgaacaaatgggagccggagctaccaagctgctccagggtatgt
cagccacctccagatgtcctgcatgctgagcgtacccaaagggacaaggacaacttttcacccgggcaggaagtgttctacagc
tgtgagcccggctatgacctcagaggggctgcgtctatgcgctgcacaccccagggagactggagccctgcagcccccacatgt
gaagtgaaatcctgtgatgacttcatgggccaacttcttaatggccgtgtgctatttccagtaaatctccagcttggagcaaaa
gtggattttgtttgtgatgaaggatttcaattaaaaggcagctctgctagttattgtgtcttggctggaatggaaagcctttgg
aatagcagtgttccagtgtgtgaacaaatcttttgtccaagtcctccagttattcctaatgggagacacacaggaaaacctctg
gaagtctttccctttggaaaagcagtaaattacacatgcgacccccacccagacagagggacgagcttcgacctcattggagag
agcaccatccgctgcacaagtgaccctcaagggaatggggtttggagcagccctgcccctcgctgtggaattctgggtcactgt
caagccccagatcattttctgtttgccaagttgaaaacccaaaccaatgcatctgactttcccattgggacatctttaaagtac
gaatgccgtcctgagtactacgggaggccattctctatcacatgtctagataacctggtctggtcaagtcccaaagatgtctgt
aaacgtaaatcatgtaaaactcctccagatccagtgaatggcatggtgcatgtgatcacagacatccaggttggatccagaatc
aactattcttgtactacagggcaccgactcattggtcactcatctgctgaatgtatcctctcaggcaatactgcccattggagc
acgaagccgccaatttgtcaacgaattccttgtgggctacccccaaccatcgccaatggagatttcattagcaccaacagagag
aattttcactatggatcagtggtgacctaccgctgcaatcttggaagcagagggagaaaggtgtttgagcttgtgggtgagccc
tccatatactgcaccagcaatgacgatcaagtgggcatctggagcggccccgcccctcagtgcattatacctaacaaatgcacg
cctccaaatgtggaaaatggaatattggtatctgacaacagaagcttattttccttaaatgaagttgtggagtttaggtgtcag
cctggctttgtcatgaaaggaccccgccgtgtgaagtgccaggccctgaacaaatgggagccagagttaccaagctgctccagg
gtgtgtcagccgcctccagaaatcctgcatggtgagcataccccaagccatcaggacaacttttcacctgggcaggaagtgttc
tacagctgtgagcctggctatgacctcagaggggctgcgtctctgcactgcacaccccagggagactggagccctgaagccccg
agatgtgcagtgaaatcctgtgatgacttcttgggtcaactccctcatggccgtgtgctatttccacttaatctccagcttggg
gcaaaggtgtcctttgtctgtgatgaagggtttcgcttaaagggcagttccgttagtcattgtgtcttggttggaatgagaagc
ctttggaataacagtgttcctgtgtgtgaacatatcttttgtccaaatcctccagctatccttaatgggagacacacaggaact
ccctctggagatattccctatggaaaagaaatatcttacacatgtgacccccacccagacagagggatgaccttcaacctcatt
ggggagagcaccatccgctgcacaagtgaccctcatgggaatggggtttggagcagccctgcccctcgctgtgaactttctgtt
cgtgctggtcactgtaaaaccccagagcagtttccatttgccagtcctacgatcccaattaatgactttgagtttccagtcggg
acatctttgaattatgaatgccgtcctgggtattttgggaaaatgttctctatctcctgcctagaaaacttggtctggtcaagt
gttgaagacaactgtagacgaaaatcatgtggacctccaccagaacccttcaatggaatggtgcatataaacacagatacacag

tttggatcaacagttaattattcttgtaatgaagggtttcgactcattggttccccatctactacttgtctcgtctcaggcaat
aatgtcacatgggataagaaggcacctatttgtgagatcatatcttgtgagccacctccaaccatatccaatggagacttctac
agcaacaatagaacatcttttcacaatggaacggtggtaacttaccagtgccacactggaccagatggagaacagctgtttgag
cttgtgggagaacggtcaatatattgcaccagcaaagatgatcaagttggtgtttggagcagccctccccctcggtgtatttct
actaataaatgcacagctccagaagttgaaaatgcaattagagtaccaggaaacaggagtttcttttccctcactgagatcatc
agatttagatgtcagcccgggtttgtcatggtagggtcccacactgtgcagtgccagaccaatggcagatgggggcccaagctg
ccacactgctccagggtgtgtcagccgcctccagaaatcctgcatggtgagcataccctaagccatcaggacaacttttcacct
gggcaggaagtgttctacagctgtgagcccagctatgacctcagaggggctgcgtctctgcactgcacgccccagggagactgg
agccctgaagcccctagatgtacagtgaaatcctgtgatgacttcctgggccaactccctcatggccgtgtgctacttccactt
aatctccagcttggggcaaaggtgtcctttgtttgcgatgaagggttccgattaaaggcaggtctgctagtcattgtgtcttg
gctggaatgaaagccctttggaatagcagtgttccagtgtgtgaacaaatcttttgtccaaatcctccagctatccttaatggg
agacacacaggaactccctttggagatattccctatggaaaagaaatatcttacgcatgcgacacccacccagacagagggatg
accttcaacctcattggggagagctccatccgctgcacaagtgaccctcaagggaatggggtttggagcagccctgcccctcgc
tgtgaactttctgttcctgctgcctgcccacatccacccaagatccaaaacgggcattacattggaggacacgtatctctatat
cttcctgggatgacaatcagctacacttgtgaccccggctacctgttagtgggaaagggcttcattttctgtacagaccaggga
atctggagccaattggatcattattgcaaagaagtaaattgtagcttcccactgtttatgaatggaatctcgaaggagttagaa
atgaaaaaagtatatcactatggagattatgtgactttgaagtgtgaagatgggtatactctggaaggcagtccctggagccag
tgccaggcggatgacagatgggaccctcctctggccaaatgtacctctcgtgcacatgatgctctcatagttggcactttatct
ggtacgatcttctttatttactcatcattttcctctcttggataattctaaagcacagaaaaggcaataatgcacatgaaaac
cctaaagaagtggctatccatttacattctcaaggaggcagcagcgttcatccccgaactctgcaaacaaatgaagaaaatagc
agggtccttccttgacaaagtactatacagctgaagaacatctcgaatacaatttggtgggaaaggagccaattgatttcaac
agaatcagatctgagcttcataaagtctttgaagtgacttcacagagacgcagacatgtgcacttgaagatgctgccccttccc
tggtacctagcaaagctcctgcctctttgtgtgcgtcactgtgaaaccccccaccttctgcctcgtgctaacgcacacagtat
ctagtcaggggaaaagactgcatttaggagatagaaaatagtttggattacttaaaggaataaggtgttgcctggaatttctgg
tttgtaaggtggtcactgttcttttttaaaatatttgtaatatggaatgggctcagtaagaagagcttggaaaatgcagaaagt
tatgaaaaataagtcacttataattatgctacctactgataaccactcctaatatttgattcattttctgcctatcttctttc
acatatgtgtttttttacatacgtacttttccccccttagtttgtttccttttattttatagagcagaaccctagtcttttaaa
cagtttagagtgaaatatatgctatatcagtttttactttctctagggagaaaaattaatttactagaaaggcatgaaatgatc
atgggaagagtggttaagactactgaagagaaatatttggaaaataagatttcgatatcttcttttttttgagatggagtctg
gctctgtctcccaggctggagtgcagtggcgtaatctcggctcactgcaacgtccgcctcccg
<212> Type : DNA
<211> Length : 6951
      SequenceName : Y00816
      SequenceDescription :

Sequence
--------
<213> OrganismName : HUMAN
<400> PreSequenceString :
aagcggggactgtgccgtgtggaacgtgtagctgttgagaggtggactctgttaccattgaggatgtttggaggatgagtatg
tgtggcagaggcacacataaacaggcagagaccctttgcccctgcctttctcccccaacccaaggctgacctgtgttctcccag
gtctgggattctaagtgacctgctctgtgtttggtctctctcaggatgagcacaagcctgggagatggcagtgatggaaatggc
ctgcccaggtgcccctggctcagcagtggggcagcagaaggaactccccaagccaaaggagaagacgccgccactggggaagaa
acagagctccgtctacaagcttgaggccgtggagaagagccctgtgttctgcggaaagtgggagatcctgaatgacgtgattac
caagggcacagccaaggaaggctccgaggcagggccagctgccatctctatcatcgcccaggctgagtgtgagaatagccaaga
gttcagccccacctttcagaacgcattttcatcgctgggtccaaacagtacagccagtccgagagtcttgatcagatccccaa
caatgtggcccatgctacagagggcaaaatggcccgtgtgtgttggaagggaaagcgtcgcagcaaagcccggaagaaacggaa
gaagaagagctcaaagtccctggctcatgcaggagtggccttggccaaacccctccccaggacccctgagcaggagagctgcac
catcccagtgcaggaggatgagtctccactcggcgccccatatgttagaaacaccccgcagttcaccaagcctctgaaggaacc
aggccttgggcaactctgttttaagcagcttggcgagggcctacggccggctctgcctcgatcagaactccacaaactgatcag
ccccttgcaatgtctgaaccacgtgtggaaactgcaccacccccaggacggaggcccccctgcccctgcccacgcaccccttccc
ctatagcagactgcctcatcccttcccattccaccctctccagccctggaaacctcaccctctggagtccttcctggcaaact
ggcctgtgtagacagccagaaacccttgcctgacccacacctgagcaaactggcctgtgtagacagtccaaagcccctgcctgg
cccacacctggagcccagctgcctgtctcgtggtgcccatgagaagttttctgtggaggaatacctagtgcatgctctgcaagg
cagcgtgagctcaagccaggcccacagcctgaccagcctggccaagacctgggcagcacggggctccagatcccgggagcccag
ccccaaaactgaggacaacgagggtgtcctgctcactgagaaactcaagccagtggattatgagtaccgagaagaagtccactg
ggccacgcaccagctccgcctgggcagaggctccttcggagaggtgcacaggatggaggacaagcagactggcttccagtgcgc
tgtcaaaaaggtgcggctggaagtatttcgggcagaggagctgatggcatgtgcaggattgacctcacccagaattgtcccttt
gtatggagctgtgagagaagggccttgggtcaacatcttcatggagctgctggaaggtggctccctgggccagctggtcaagga
gcaggctgtctcccagaggaccgggccctgtactacctgggccaggccctggagggtctggaatacctccactcacgaaggat
tctgcatggggacgtcaaagctgacaacgtgctcctgtccagcgatgggagccacgcagccctctgtgactttggccatgctgt
gtgtcttcaacctgatggcctgggaaagtccttgctcacaggggactacatccctggcacagagacccacatggctccggaggt
ggtgctgggcaggagctgcgacgccaaggtggatgtctggagcagctgctgtatgatgctgcacatgctcaacggctgccaccc

ctggactcagttcttccgagggccgctctgcctcaagattgccagcgagcctccgcctgtgagggagatcccaccctcctgcgc
ccctctcacagcccaggccatccaagaggggctgaggaaagagcccatccaccgcgtgtctgcagcggagctgggagggaaggt
gaaccgggcactacagcaagtgggaggtctgaagagcccttggaggggagaatataaagaaccaagacatccaccgccaaatca
agccaattaccaccagaccctccatgcccagccgagagagctttcgccaagggccccagggccccggccagctgaggagacaac
aggcagagcccctaagctccagcctcctctcccaccagagcccccagagccaaacaagtctcctcccttgactttgagcaagga
ggagtctgggatgtgggaacccttacctctgtccctcctggagccagcccctgccagaaaccccagctcaccagagcggaaagc
aaccgtcccggagcaggaactgcagcagctggaaatagaattattcctcaacagcctgtcccagccattttctctggaggagca
ggagcaaattctctcgtgcctcagcatcgacagcctctccctgtcggatgacagtgagaagaacccatcaaaggcctctcaaag
ctcgcgggacaccctgagctcaggcgtacactcctggagcagccaggccgaggctcgaagctccagctggaacatggtgctggc
ccgggggcggcccaccgacaccccaagctatttcaatggtgtgaaagtccaaatacagtctcttaatggtgaacacctgcacat
ccgggagttccaccgggtcaaagtgggagacatcgccactggcatcagcagccagatcccagctgcagccttcagcttggtcac
caaagacgggcagcctgttcgctacgacatggaggtgccagactcgggcatcgacctgcagtgcacactggcccctgatggcag
cttcgcctggagctggagggtcaagcatggccagctggagaacaggccctaaccctgccctccaccgccggctccacactgccg
gaaagcagccttcctgctcggtgcacgatgctgccctgaaaacacaggctcagccgttcccaggggattgccagcccccggct
cacagtgggaaccagggcctcgcagcagcaaggtgggggcaagcagaatgcctcccaggatttcacacctgagccctgccccac
cctgctgaaaaaacatccgccacgtgaagagacagaaggaggatggcaggagttacctggggaaacaaaacagggatctttttc
tgcccctgctccagtcgagttggcctgacccgcttggatcagtgaccatttgttggcagacaggggagagcagcttccagcctg
ggtcagaaggggtgggcgagcccttcggcccctcaccctccaggctgctgtgagagtgtcaagtgtgtaagggcccaaactcag
gttcagtgcagaaccaggtcagcaggtatgcccgcccgtaggttaaggggccctctaaaccccttgcctggcctcacctggcc
agctcaccccttttgggtgtaggggaaaagaatgcctgaccctgggaaggctccctggtagaatacaccacacttttcaggttg
ttgcaacacaggtcctgagttgacctctggttcagccaaggaccaaagaaggtgtgtaagtgaagtggttctcagtccccagac
atgtgcccctttgctgctggctaccactcttccccagagcagcaggccccgagccccttcaggcccagcactgccccagactcg
ctggcactcagttccctcatctgtaaaggtgaagggtgatgcaggatatgcctgacaggaacagtctgtggatggacatgatca
gtgctaaggaaagcagcagagagagacgtccggcgccccagccccactatcagtgtccagcgtgctggttccccagagcacagc
tcagcatcacactgacactcaccctgccctgccccctggccagagggtactgccgacggcactttgcactctgatgacctcaaag
cactttcatggctgccctctggcagggcagggcagggcagtgacactgtaggagcatagcaagccaggagatggggtgaaggga
cacagtcttgagctgtccacatgcatgtgactcctcaaacctcttccagatttctctaagaatagcacccccttccccattgcc
ccagcttagcctcttctcccaggggagctactcaggactcacgtagcattaaatcagctgtgaatcgtcaggggggtgtctgcta
gcctcaacctcctggggcaggggacgccgagactccgtgggagaagctcattcccacatcttgccaagacagcctttgtccagc
tgtccacattgagtcagactgctcccggggagagagccccggcccccagcacataaagaactgcagccttggtactgcagagtc
tgggttgtagagaactctttgtaagcaataaagtttggggtgatgacaaatgttaaaaaa
<212> Type : DNA
<211> Length : 4596
      SequenceName : Y10256
      SequenceDescription :

Sequence
--------
<213> OrganismName : HUMAN
<400> PreSequenceString :
aggatcagaacaatgcctccaagaccatcatcaggtgaactgtggggcatccacttgatgcccccaagaatcctagtggaatgt
ttactaccaaatggaatgatagtgactttagaatgcctccgtgaggctacattagtaactataaagcatgaactatttaaagaa
gcaagaaaataccctctccatcaacttcttcaagatgaatcttcttacatttcgtaagtgttacccaagaagcagaaagggaa
gaatttttgatgaaacaagacgactttgtgatcttcggctttttcaaccattttaaaagtaattgaaccagtaggcaaccgt
gaagaaaagatcctcaatcgagaaattggttttgctatcggcatgccagtgtgcgaatttgatatggttaaagatcctgaagta
caggacttccgaagaaatattcttaatgtttgtaaagaagctgtggatcttagggatcttaattcacctcatagtagagcaatg
tatgtctatccgccacatgtagaatcttcaccagagctgccaaagcacatatataataaattggatagaggccaaataatagtg
gtgatttgggtaatagtttctccaaataatgacaagcagaagtatactctgaaaatcaaccatgactgtgtgccagaacaagta
attgctgaagcaatcaggaaaaaaactagaagtatgttgctatcatctgaacaattaaaactctgtgttttagaatatcagggc
aagtacattttaaaagtgtgtggatgtgatgaatacttcctagaaaaatatcctctgagtcagtataagtatataagaagctgt
ataatgcttgggaggatgcccaatttgaagatgatggctaaagaaagcctttattctcaactgccaatggactgttttacaatg
ccatcttattccagacgcatttccacagctacaccatatatgaatggagaaacatctacaaaatccctttgggttataaataga
gcactcagaataaaaattctttgtgcaacctacgtgaatctaaatattcgagacattgacaagatttatgttcgaacaggtatc
taccatggaggagaacccttatgtgacaatgtgaacactcaaagagtaccttgttccaatcccaggtggaatgaatggctgaat
tatgatatatacattcctgatcttcctcgtgctgctcgactttgcctttccatttgctctgttaaaggccgaaagggtgctaaa
gaggaacactgtccattggcatggggaaatataaacttgtttgattacacagacactctagtatctggaaaaatggctttgaat
ctttggccagtacctcatggattagaagatttgctgaaccctattggtgttactggatcaaatccaaataaagaaactccatgc
ttagagttggagtttgactggttcagcagtgtggtaaagttcccagatatgtcagtgattgaagagcatgccaattggtctgta
tcccgagaagcaggatttagctattcccacgcaggactgagtaacagactagctagagacaatgaattaagggaaaatgacaaa
gaacagctcaaagcaatttctacacgagatcctctctctgaaatcactgagcaggagaaagatttctatggagtcacagacac
tattgtgtaactatccccgaaattctacccaaattgcttctgtctgttaaatggaattctagagatgaagtagcccagatgtat
tgcttggtaaaagattggcctccaatcaaacctgaacaggctatggaacttctggactgtaattacccagatcctatggttcga
ggttttgctgttcggtgcttggaaaaatatttaacagatgacaaactttctcagtatttaattcagctagtacaggtcctaaaa

tatgaacaatatttggataacttgcttgtgagatttttactgaagaaagcattgactaatcaaaggattgggcactttttcttt
tggcatttaaaatctgagatgcacaataaaacagttagccagaggtttggcctgcttttggagtcctattgtcgtgcatgtggg
atgtatttgaagcacctgaataggcaagtcgaggcaatggaaaagctcattaacttaactgacattctcaaacaggagaggaag
gatgaaacacaaaaggtacagatgaagttttagttgagcaaatgaggcgaccagatttcatggatgccctacagggcttgctg
tctcctctaaaccctgctcatcaactaggaaacctcaggcttaaagagtgtcgaattatgtcttctgcaaaaaggccactgtgg
ttgaattgggagaacccagacatcatgtcagagttactgtttcagaacaatgagatcatctttaaaaatggggatgatttacgg
caagatatgctaacacttcaaattattcgtattatggaaaatatctggcaaaatcaaggtcttgatcttcgaatgttaccttat
ggttgtctgtcaatcggtgactgtgtgggacttattgaggtggtgcgaaattctcacactattatgcaaattcagtgcaaaggc
ggcttgaaaggtgcactgcagttcaacagccacacactacatcagtggctcaaagacaagaacaaaggagaaatatatgatgca
gccattgacctgtttacacgttcatgtgctggatactgtgtagctaccttcattttgggaattggagatcgtcacaatagtaac
atcatggtgaaagacgatggacaactgtttcatatagatttggacactttttggatcacaagaagaaaaaatttggttataaa
cgagaacgtgtgccatttgttttgacacaggatttcttaatagtgattagtaaaggagcccaagaatgcacaaagacaagagaa
tttgagaggtttcaggagatgtgttacaaggcttatctagctattcgacagcatgccaatctcttcataaatcttttctcaatg
atgcttggctctggaatgccagaactacaatcttttgatgacattgcatacattcgaaagaccctagccttagataaaactgag
caagaggctttggagtatttcatgaaacaaatgaatgatgcacatcatggtggctggacaacaaaaatggattggatcttccac
acaattaaacagcatgcattgaactgaaagataactgagaaaatgaaagctcactctggattccacactgcactgttaataact
ctcagcaggcaaagaccgattgcataggaattgcacaatccatgaacagcattagatttacagcaagaacagaaataaaatact
atataatttaaataatgtaaacgcaaacagggtttgatagcacttaaactagttcatttcaaaa
<212> Type : DNA
<211> Length : 3424
      SequenceName : Z29090
      SequenceDescription :

Sequence
--------
<213> OrganismName : HUMAN
<400> PreSequenceString :
gggcccggcgctcgctgctcccgcggcccgcgccatgccctcctacacggtcaccgtggccactggcagccagtggttcgccgg
cactgacgactacatctacctcagcctcgtgggctcggcgggctgcagcgagaagcacctgctggacaagcccttctacaacga
cttcgagcgtggcgcggtggattcatacgacgtgactgtggacgaggaactgggcgagatccagctggtcagaatcgagaagcg
caagtactggctgaatgacgactggtacctgaagtacatcacgctgaagacgccccacggggactacatcgagttcccctgcta
ccgctggatcaccggcgatgtcgaggttgtcctgagggatggacgcgcaaagttggcccgagatgaccaaattcacattctcaa
gcaacaccgacgtaaagaactggaaacacggcaaaaacaatatcgatggatggagtggaaccctggcttccccttgagcatcga
tgccaaatgccacaaggatttaccccgtgatatccagtttgatagtgaaaaaggagtggactttgttctgaattactccaaagc
gatggagaacctgttcatcaaccgcttcatgcacatgttccagtcttcttggaatgacttcgccgactttgagaaaatctttgt
caagatcagcaacactatttctgagcgggtcatgaatcactggcaggaagacctgatgtttggctaccagttcctgaatggctg
caaccctgtgttgatccggcgctgcacagagctgcccgagaagctcccggtgaccacggagatggtagagtgcagcctggagcg
gcagctcagcttggagcaggaggtccagcaagggaacattttcatcgtggactttgagctgctggatggcatcgatgccaacaa
aacagacccctgcacactccagttcctggccgctcccatctgcttgctgtataagaacctggccaacaagattgtccccattgc
catccagctcaaccaaatcccgggagatgagaaccctattttcctcccttcggatgcaaaatacgactggcttttggccaaaat
ctgggtgcgttccagtgacttccacgtccaccagaccatcacccaccttctgcgaacacatctggtgtctgaggttttggcat
tgcaatgtaccgccagctgcctgctgtgcaccccattttcaagctgctggtggcacacgtgagattcaccattgcaatcaacac
caaggcccgtgagcagctcatctgcgagtgtggcctctttgacaaggccaacgccacagggggcggtgggcacgtgcagatggt
gcagagggccatgaaggacctgacctatgcctccctgtgctttcccgaggccatcaaggcccggggcatggagagcaaagaaga
catcccctactacttctaccgggacgacgggctcctggtgtgggaagccatcaggacgttcacggccgaggtggtagacatcta
ctacgagggcgaccaggtggtggaggaggacccggagctgcaggacttcgtgaacgatgtctacgtgtacggcatgcggggccg
caagtcctcaggcttccccaagtcggtcaagagccgggagcagctgtcggagtacctgaccgtggtgatcttcaccgcctccgc
ccagcacgccgcggtcaacttcggccagtacgactggtgctcctggatccccaatgcgcccccaaccatgcgagccccgccacc
gactgccaagggcgtggtgaccattgagcagatcgtggacacgctgcccgaccgcggccgctcctgctggcatctgggtgcagt
gtgggcgctgagccagttccaggaaaacgagctgttcctgggcatgtacccagaagagcattttatcgagaagcctgtgaagga
agccatggcccgattccgcaagaacctcgaggccattgtcagcgtgattgctgagcgcaacaagaagaagcagctgccatatta
ctacttgtccccagaccggattccgaacagtgtggccatctgagcacactgccagtctcactgtgggaaggccagctgccccag
ccagatggactccagcctgcctggcaggtgtctggccaggcctcttggcagtcacatctcttcctccgaggccagtacctttcc
atttattctttgatcttcagggaactgcatagattgatcaaagtgtaaacaccatagggacccattctacacagagcaggactg
cacagcgtcctgtccacacccagctcagcatttccacaccaagcagcaacagcaaatcacgaccactgatagatgtctattctt
gttggagacatgggatgattattttctgttctatttgtgcttagtccaattccttgcacatagtaggtacccaattcaattact
attgaatgaattaagaattggttgccataaaaataaatcagttcattt
<212> Type : DNA
<211> Length : 2484
      SequenceName : J03571
      SequenceDescription :

Sequence

--------
<213> OrganismName : HUMAN
<400> PreSequenceString :
agtctctgctcttcccagcctctccggcgcgctccaagggcttcccgtcgggaccatgcgcggcagtgagctcccgctggtcct
gctggcgctggtcctctgcctagcgccccgggggcgagcggtcccgctgcctgcgggcggagggaccgtgctgaccaagatgta
cccgcgcggcaaccactgggcggtggggcacttaatgggaaaaagagcacaggggagtcttcttctgtttctgagagagggag
cctgaagcagcagctgagagagtacatcaggtgggaagaagctgcaaggaatttgctgggtctcatagaagcaaaggagaacag
aaaccaccagccacctcaacccaaggccttgggcaatcagcagccttcgtgggattcagaggatagcagcaacttcaaagatgt
aggttcaaaaggcaaagttggtagactctctgctccaggttctcaacgtgaaggaaggaacccccagctgaaccagcaatgata
atgatggcctctctcaaaagagaaaaacaaaacccctaagagactgagttctgcaagcatcagttctacggatcatcaacaaga
tttccttgtgcaaaatatttgactattctgtatctttcatccttgactaaattcgtgattttcaagcagcatcttctggtttaa
acttgtttgctgtgaacaattgtcgaaaagagtcttccaattaatgctttttatatctaggctacctgttggttagattcaag
gccccgagctgttaccattcacaataaaagcttaaacacat
<212> Type : DNA
<211> Length : 797
      SequenceName : K02054
      SequenceDescription :

Sequence
--------
<213> OrganismName : HUMAN
<400> PreSequenceString :
acggcatccagtacagaggggctggacttggacccctgcagcagccctgcacaggagaagcggcatataaagccgcgctgcccg
ggagccgctcggccacgtccaccggagcatcctgcactgcagggccggtctctcgctccagcagagcctgcgcctttctgactc
ggtccggaacactgaaaccagtcatcactgcatctttttggcaaaccaggagctcagctgcaggaggcaggatggtctggaggc
tggtcctgctggctctgtgggtgtggcccagcacgcaagctggtcaccaggacaaagacacgaccttcgaccttttcagtatca
gcaacatcaaccgcaagaccattggcgccaagcagttccgcgggcccgaccccggcgtgccggcttaccgcttcgtgcgctttg
actacatcccaccggtgaacgcagatgacctcagcaagatcaccaagatcatgcggcagaaggagggcttcttcctcacggccc
agctcaagcaggacggcaagtccaggggcacgctgttggctctggagggccccggtctctcccagaggcagttcgagatcgtct
ccaacggccccgcggacacgctggatctcacctactggattgacggcacccggcatgtggtctccctggaggacgtcggcctgg
ctgactcgcagtggaagaacgtcaccgtgcaggtggctggcgagacctacagcttgcacgtgggctgcgacctcataggaccag
ttgctctggacgagcccttctacgagcacctgcaggcggaaaagagccggatgtacgtggccaaaggctctgccagagagagtc
acttcaggggtttgcttcagaacgtccacctagtgtttgaaaactctgtggaagatattctaagcaagaagggttgccagcaag
gccagggagctgagatcaacgccatcagtgagaacacagagacgctgcgcctgggtccgcatgtcaccaccgagtacgtgggcc
ccagctcggagaggaggcccgaggtgtgcgaacgctcgtgcgaggagctgggaaacatggtccaggagctctcggggctccacg
tcctcgtgaaccagctcagcgagaacctcaagagagtgtcgaatgataaccagtttctctgggagctcattggtggccctccta
agacaaggaacatgtcagcttgctggcaggatggccggttctttgcggaaaatgaaacgtgggtggtggacagctgcaccacgt
gtacctgcaagaaatttaaaaccatttgccaccaaatcacctgcccgcctgcaacctgcgccagtccatcctttgtggaaggcg
aatgctgcccttcctgcctccactcggtggacggtgaggagggctggtctccgtgggcagagtggacccagtgctccgtgacgt
gtggctctgggacccagcagagaggccggtcctgtgacgtcaccagcaacacctgcttggggccctcgatccagacacgggctt
gcagtctgagcaagtgtgacacccgcatccggcaggacggcggctggagccactggtcaccttggtcttcatgctctgtgacct
gtggagttggcaatatcacacgcatccgtctctgcaactccccagtgccccagatgggggcaagaattgcaaagggagtggcc
gggagaccaaagcctgccagggcgccccatgcccaatcgatggccgctggagcccctggtccccgtggtcggcctgcactgtca
cctgtgccggtgggatccgggagcgcacccgggtctgcaacagccctgagcctcagtacggagggaaggcctgcgtgggggatg
tgcaggagcgtcagatgtgcaacaagaggagctgccccgtggatggctgtttatccaacccctgcttcccgggagcccagtgca
gcagcttccccgatgggtcctggtcatgcggcttctgccctgtgggcttcttgggcaatggcacccactgtgaggacctggacg
agtgtgccctggtccccgacatctgcttctccaccagcaaggtgcctcgctgtgtcaacactcagcctggcttccactgcctgc
cctgcccgccccgatacagagggaaccagcccgtcggggtcggcctggaagcagccaagacggaaaagcaagtgtgtgagcccg
aaaacccatgcaaggacaagacacacaactgccacaagcacgcggagtgcatctacctgggtcacttcagcgaccccatgtaca
agtgcgagtgccagacaggctacgcgggcgacggctcatctgcggggaggactcggacctggacggctggcccaacctcaatc
tggtctgcgccaccaacgccacctaccactgcatcaaggataactgcccccatctgccaaattctggcaggaagactttgaca
aggacgggattggcgatgcctgtgatgatgacgatgacaatgacggtgtgaccgatgagaaggacaactgccagctcctcttca
atccccgccaggctgactatgacaaggatgaggttggggaccgctgtgacaactgcccttacgtgcacaaccctgcccagatcg
acacagacaacaatggagagggtgacgcctgctccgtggacattgatggggacgatgtcttcaatgaacgagacaattgtccct
acgtctacaacactgaccagagggacacggatggtgacggtgtgggggatcactgtgacaactgccccctggtgcacaaccctg
accagaccgacgtggacaatgaccttgttggggaccagtgtgacaacaacgaggacatagatgacgacggccaccagaacaacc
aggacaactgcccctacatctccaacgccaaccaggctgaccatgacagagacggccagggcgacgcctgtgaccctgatgatg
acaacgatggcgtccccgatgacagggacaactgccggcttgtgttcaacccagaccaggaggacttggacggtgatggacggg
gtgatatttgtaaagatgattttgacaatgacaacatcccagatattgatgatgtgtgtcctgaaaacaatgccatcagtgaga
cagacttcaggaacttccagatggtccccttggatcccaaagggaccacccaaattgatcccaactgggtcattcgccatcaag
gcaaggagctggttcagacagccaactcggaccccggcatcgctgtaggttttgacgagtttgggtctgtggacttcagtggca
cattctacgtaaacactgaccgggacgacgactatgctggcttcgtctttggttaccagtcaagcagccgcttctatgtggtga
tgtggaagcaggtgacgcagacctactgggaggaccagcccacgcgggcctatggctactccggcgtgtccctcaaggtggtga

actccaccacggggacgggcgagcacctgaggaacgcgctgtggcacacggggaacacgccggggcaggtgcgaaccttatggc
acgaccccaggaacattggctggaaggactacacggcctataggtggcacctgactcacaggcccaagaccggctacatcagag
tcttagtgcatgaaggaaaacaggtcatggcagactcaggacctatctatgaccaaacctacgctggcgggcggctgggtctat
ttgtcttctctcaagaaatggtctatttctcagacctcaagtacgaatgcagagatatttaaacaagatttgctgcatttccgg
caatgccctgtgcatgccatggtccctagacacctcagttcattgtggtccttgcggcttctctctctagcagcacctcctgtc
ccttgaccttaactctgatggttcttcacctcctgccagcaaccccaaacccaagtgccttcagaggataaatatcaatggaac
tcagagatgaacatctaacccactagaggaaaccagtttggtgatatatgagactttatgtggagtgaaaattggcatgccat
tacattgctttttcttgtttgtttaaaaagaatgacgtttacatataaaatgtaattacttattgtatttatgtgtatatggag
ttgaagggaatactgtgcataagccattatgataaattaagcatgaaaaatattgctgaactacttttggtgcttaaagttgtc
actattcttgaattagagttgctctacaatgacacacaaatcccgctaaataaattataaacaagggtcaattcaaatttgaag
taatgttttagtaaggagagattagaagacaacaggcatagcaaatgacataagctaccgattaactaatcggaacatgtaaaa
cagttacaaaaataaacgaactctcctcttgtcctacaatgaaagccctcatgtgcagtagagatgcagtttcatcaaagaaca
aacatccttgcaaatgggtgtgacgcggttccagatgtggatttggcaaaacctcatttaagtaaaaggttagcagagcaaagt
gcggtgctttagctgctgcttgtgccgttgtggcgtcggggaggctcctgcctgagcttccttccccagctttgctgcctgaga
ggaaccagagcagacgcacaggccggaaaaggcgcatctaacgcgtatctaggctttggtaactgcggacaagttgcttttacc
tgatttgatgatacatttcattaaggttccagttataaatattttgttaatatttattaagtgactatagaatgcaactccatt
taccagtaacttattttaaatatgcctagtaacacatatgtagtataatttctagaaacaaacatctaataagtatataatcct
gtgaaaatatgaggcttgataatattaggttgtcacgatgaagcatgctagaagctgtaacagaatacatagagaataatgagg
agtttatgatggaaccttaatatataatgttgccagcgatttagttcaatatttgttactgttatctatctgctgtatatgga
attcttttaattcaaacgctgaaaacgaatcagcatttagtcttgccaggcacacccaataatcagtcatgtgtaatatgcaca
agtttgtttttgtttttgtttttttttgttggttggtttttttgctttaagttgcatgatctttctgcaggaaatagtcactcat
cccactccacataaggggtttagtaagagaagtctgtctgtctgatgatggatagggggcaaatctttttcccctttctgttaa
tagtcatcacatttctatgccaaacaggaacgatccataactttagtcttaatgtacacattgcattttgataaaattaatttt
gttgtttcctttgaggttgatcgttgtgttgttttgctgcactttttactttttgcgtgtggagctgtattcccgagacaacg
aagcgttgggatacttcattaaatgtagcgactgtcaacagcgtgcaggttttctgtttctgtgttgtggggtcaaccgtacaa
tggtgtgggaatgacgatgatgtgaatatttagaatgtaccatatttttgtaaattatttatgtttttctaaacaaatttatc
gtataggttgatgaaacgtcatgtgttttgccaaagactgtaaatatttatttatgtgttcacatggtcaaaatttcaccactg
aaaccctgcacttagctagaacctcatttttaaagattaacaacaggaaataaattgtaaaaaggttttct
<212> Type : DNA
<211> Length : 5784
      SequenceName : L12350
      SequenceDescription :

Sequence
--------
<213> OrganismName : HUMAN
<400> PreSequenceString :
accaccatgaatccactcctgatccttacctttgtggcagctgctcttgctgcccccttttgatgatgatgacaagatcgttggg
ggctacaactgtgaggagaattctgtcccctaccaggtgtccctgaattctggctaccacttctgtggtggctccctcatcaac
gaacagtgggtggtatcagcaggccactgctacaagtcccgcatccaggtgagactgggagagcacaacatcgaagtcctggag
gggaatgagcagttcatcaatgcagccaagatcatccgccacccccaatacgacaggaagactctgaacaatgacatcatgtta
atcaagctctcctcacgtgcagtaatcaacgcccgcgtgtccaccatctctctgcccaccgcccctccagccactggcacgaag
tgcctcatctctggctggggcaacactgcgagctctggcgccgactacccagacgagctgcagtgcctggatgctcctgtgctg
agccaggctaagtgtgaagcctcctaccctggaaagattaccagcaacatgttctgtgtgggcttccttgagggaggcaaggat
tcatgtcagggtgattctggtggccctgtggtctgcaatggacagctccaaggagttgtctcctggggtgatggctgtgcccag
aagaacaagcctggagtctacaccaaggtctacaactacgtgaaatggattaagaacaccatagctgccaatagctaaagcccc
cagtatctcttcagtctctataccaataaagtgaccctgttctc
<212> Type : DNA
<211> Length : 800
      SequenceName : M22612
      SequenceDescription :

Sequence
--------
<213> OrganismName : HUMAN
<400> PreSequenceString :
gaattccgggaacgaaagagaagctctatctcccctccaggagcccagctatgaactccttctccacaagcgccttcggtccag
ttgccttctccctggggctgctcctggtgttgcctgctgccttccctgccccagtacccccaggagaagattccaaagatgtag
ccgccccacacagacagccactcacctcttcagaacgaattgacaaacaaattcggtacatcctcgacggcatctcagccctga
gaaaggagacatgtaacaagagtaacatgtgtgaaagcagcaaagaggcactggcagaaaacaacctgaaccttccaaagatgg
ctgaaaaagatggatgcttccaatctggattcaatgaggagacttgcctggtgaaaatcatcactggtcttttggagtttgagg
tatacctagagtacctccagaacagatttgagagtagtgaggaacaagccagagctgtgcagatgagtacaaaagtcctgatcc
agttcctgcagaaaaaggcaaagaatctagatgcaataaccacccctgacccaaccacaaatgccagcctgctgacgaagctgc

aggcacagaaccagtggctgcaggacatgacaactcatctcattctgcgcagctttaaggagttcctgcagtccagcctgaggg
ctcttcggcaaatgtagcatgggcacctcagattgttgttgttaatgggcattccttcttctggtcagaaacctgtccactggg
cacagaacttatgttgttctctatggagaactaaaagtatgagcgttaggacactattttaattattttaatttattaatatt
taaatatgtgaagctgagttaatttatgtaagtcatatttatattttaagaagtaccacttgaaacattttatgtattagttt
tgaaataataatggaaagtggctatgcagtttgaatatcctttgtttcagagccagatcatttcttggaaagtgtaggcttacc
tcaaataaatggctaacttatacatatttttaaagaaatatttatattgtatttatataatgtataaatggtttttataccaat
aaatggcattttaaaaaattc
<212> Type : DNA
<211> Length : 1113
      SequenceName : M54894
      SequenceDescription :

Sequence
--------
<213> OrganismName : HUMAN
<400> PreSequenceString :
gctacgcggccacgctgctggctggcctgacctaggcgcgcggggtcgggcggccgcgcgggcgggctgagtgagcaagacaa
gacactcaagaagagcgagctgcgcctgggtcccggccaggcttgcacgcagaggcgggcggcagacggtgcccggcggaatct
cctgagctccgccgcccagctctggtgccagcgcccagtggccgccgcttcgaaagtgactggtgcctcgccgcctcctctcgg
tgcgggaccatgaagctgctgccgtcggtggtgctgaagctctttctggctgcagttctctcggcactggtgactggcgagagc
ctggagcggcttcggagagggctagctgctggaaccagcaacccggaccctcccactgtatccacggaccagctgctacccta
ggaggcggccgggaccggaaagtccgtgacttgcaagaggcagatctggaccttttgagagtcactttatcctccaagccacaa
gcactggccacaccaaacaaggaggagcacgggaaaagaaagaagaaaggcaaggggctagggaagaagagggacccatgtctt
cggaaatacaaggacttctgcatccatggagaatgcaaatatgtgaaggagctccgggctccctcctgcatctgccacccgggt
taccatggagagaggtgtcatgggctgagcctcccagtggaaaatcgcttatatacctatgaccacacaaccatcctggccgtg
gtggctgtggtgctgtcatctgtctgtctgctggtcatcgtggggcttctcatgtttaggtaccataggagaggaggttatgat
gtggaaaatgaagagaaagtgaagttgggcatgactaattcccactgagagagacttgtgctcaaggaatcggctggggactgc
tacctctgagaagacacaaggtgatttcagactgcagaggggaaagacttccatctagtcacaaagactccttcgtccccagtt
gccgtctaggattgggcctcccataattgctttgccaaaataccagagccttcaagtgccaaacagagtatgtccgatggtatc
tgggtaagaagaaagcaaaagcaagggaccttcatgcccttctgattcccctccaccaaaccccacttcccctcataagtttgt
ttaaacacttatcttctggattagaatgccggttaaattccatatgctccaggatctttgactgaaaaaaaaaaagaagaagaa
gaaggagagcaagaaggaaagatttgtgaactggaagaaagcaacaaagattgagaagccatgtactcaagtaccaccaaggga
tctgccattgggaccctccagtgctggatttgatgagttaactgtgaaataccacaagcctgagaactgaattttgggacttct
acccagatggaaaaataacaactattttttgttgttgttgtttgtaaatgcctcttaaattatatatttattttattctatgtat
gttaatttatttagtttttaacaatctaacaataatatttcaagtgcctagactgttactttggcaatttcctggccctccact
cctcatccccacaatctggcttagtgccacccacctttgccacaaagctaggatggttctgtgacccatctgtagtaatttatt
gtctgtctacatttctgcagatcttccgtggtcagagtgccactgcgggagctctgtatggtcaggatgtaggggttaacttgg
tcagagccactctatgagttggacttcagtcttgcctaggcgattttgtctaccatttgtgttttgaaagcccaaggtgctgat
gtcaaagtgtaacagatatcagtgtctccccgtgtcctctccctgccaagtctcagaagaggttgggcttccatgcctgtagct
ttcctggtccctcaccccccatggccccaggccacagcgtgggaactcactttcccttgtgtcaagacatttctctaactcctgc
cattcttctggtgctactccatgcaggggtcagtgcagcagaggacagtctggagaaggtattagcaaagcaaaaggctgagaa
ggaacagggaacattggagctgactgttcttggtaactgattacctgccaattgctaccgagaaggttggaggtggggaaggct
ttgtataatcccacccacctcaccaaaacgatgaaggtatgctgtcatggtcctttctggaagtttctggtgccatttctgaac
tgttacaacttgtatttccaaacctggttcatatttatactttgcaatccaaataaagataacccttattccataaaaaaaaaa
aaaaaaaa
<212> Type : DNA
<211> Length : 2360
      SequenceName : M60278
      SequenceDescription :

Sequence
--------
<213> OrganismName : HUMAN
<400> PreSequenceString :
ctctaaaggttcgggggtggaatccttgggccgctgggcaagcggcgagacctggccagggccagcgagccgaggacagagggc
gcacggagggccgggccgcagccccggccgcttgcagaccccgccatggacccgttcctggtgctgctgcactcggtgtcgtcc
agcctgtcgagcagcgagctgaccgagctcaagttcctatgcctcgggcgcgtgggcaagcgcaagctggagcgcgtgcagagc
ggcctagacctcttctccatgctgctggagcagaacgacctggagcccgggcacaccgagctcctgcgcgagctgctcgcctcc
ctgcggcgccacgacctgctgcggcgcgtcgacgacttcgaggcgggggcggcggccggggccgcgcctggggaagaagacctg
tgtgcagcatttaacgtcatatgtgataatgtggggaaagattggagaaggctggctcgtcagctcaaagtctcagacaccaag
atcgacagcatcgaggacagataccccgcaacctgacagagcgtgtgcgggagtcactgagaatctggaagaacacagagaag
gagaacgcaacagtggcccacctggtgggggctctcaggtcctgccagatgaacctggtggctgacctggtacaagaggttcag
caggcccgtgacctccagaacaggagtggggccatgtccccgatgtcatggaactcagacgcatctacctccgaagcgtcctga

tgggccgctgctttgcgctggtggaccacaggcatctacacagcctggactttggttctctccaggaaggtagcccagcactgt
gaagacccagcaggaagccaggctgagtgagccacagaccacctgcttctgaactcaagctgcgtttattaatgcctctcccgc
accaggccgggcttgggccctgcacagatatttccatttcttcctcactatgacactgagcaagatcttgtctccactaaatga
gctcctgcgggagtagttggaaagttggaaccgtgtccagcacagaaggaatctgtgcagatgagcagtcacactgttactcca
cagcggaggagaccagctcagaggcccaggaatcggagcgaagcagagaggtggagaactgggatttgaaccccgccatcctt
caccagagcccatgctcaaccactgtggcgttctgctgcccctgcagttggcagaaaggatgttttgtcccatttccttggagg
ccaccgggacagacctggacactagggtcaggcggggtgctgtggtggggagaggcatggctggggtgggggtggggagacctg
gttggccgtggtccagctcttggcccctgtgtgagttgagtctcctctctgagactgctaagtaggggcagtgatggttgccag
gacgaattgagataatatctgtgaggtgctgatgagtgattgacacacagcactctctaaatcttccttgtgaggattatgggt
cctgcaattctacagtttcttactgttttgtatcaaaatcactatctttctgataacagaattgccaaggcagcgggatctcgt
atctttaaaaagcagtcctcttattcctaaggtaatcctattaaaa
<212> Type : DNA
<211> Length : 1642
SequenceName : U24231
SequenceDescription :

Sequence
--------
<213> OrganismName : HUMAN
<400> PreSequenceString :
agagacttaagtctaaggcactgagcgtatcatgttaaagatgagcgggtggcagcgacagagccaaaatcagagctggaacct
gaggagagagtgttcaagaaggaagtgtatcttcatacatcaccacacctgaaagcagatgtgcttttccagactgatccaact
gcagagatggcagctgagtcattgcctttctcttcggacactgtcagctgggagctggaagcctggtatgaggacctgcaagag
gtcctgtcttcagatgaaaatggggtacctatgtttcacctcctggaaatgaagaggaagaatcaaaaatcttcaccactctt
gaccctgcttctctggcttggctgactgaggaggagccagaaccagcagaggtcacaagcacctcccagagccctcactctcca
gattccagtcagagctccctggctcaggaggaagaggaggaagaccaagggagaaccaggaaacggaaacagagtggtcattcc
ccagcccgggctggaaagcagcgcatgaaggagaaagaacaggagaatgaaaggaaagtggcacagctagctgaagagaatgaa
cggctcaagcaggaaatcgagcgcctgaccagggaagtagaggcgactcgccgagctctgattgaccgaatggtgaatctgcac
caagcatgaacaattgggagcatcagtcccccacttgggccacactacccacctttcccagaagtggctactgactaccctctc
actagtgccaatgatgtgaccctcaatcccacatacgcaggggggaaggcttggagtagacaaaaggaaaggtctcagcttgtat
atagagattgtacatttatttattactgtccctatctattaaagtgactttctat
<212> Type : DNA
<211> Length : 895
SequenceName : S40706
SequenceDescription :

Sequence
--------
<213> OrganismName : HUMAN
<400> PreSequenceString :
gtgggcatctacantggnacccacgtcatccctaatgacaggaagatcatctggccaggcggagagacagagaagtctcgaggg
taccagatgtccaccagactgaagattgtgacgatccaccaggagcccttcgtgtacgtcaagcccacgctgagtgatggaca
tgcaaggaggagttcacagtcaacggcgacccagtcaagaaggtgatctgcaccgggcccaacgacacgtcgccgggcagcccc
cgccacacggtgcctcagtgttgctacggcttttgcatagacctgctcatca
<212> Type : DNA
<211> Length : 304
SequenceName : S57708
SequenceDescription :

Sequence
--------
<213> OrganismName : HUMAN
<400> PreSequenceString :
ctactcctag atatttggca tgatcttcag tatgatcttg tgctgtgcta tccgcaggaa 60
ccgcgagatg gtctaga 77
<212> Type : DNA
<211> Length : 77
SequenceName : S60489
SequenceDescription :

Sequence
--------
<213> OrganismName : HUMAN

<400> PreSequenceString :
ttcacacgtgacccaggcctctctccggtctcacatgggagttgtgccccaagacactgtcctctttaatgacaccatcgccga
caatatccgttacggccgtgtcacagctgggaatgatgaggtggaggctgctgctcaggctgcaggcatccatgatgccattat
ggctttccctgaagggtacaggacacaggtgggcgagcggggactgaagctgagcggcggggagaagcagcgcgtcgccattgc
ccgcaccatcctcaaggctccgggcatcattctgctggatgaggcaacgtcagcgctggatacatctaatgagagggcca
<212> Type : DNA
<211> Length : 332
      SequenceName : 3188135H1
      SequenceDescription :

Sequence
--------
<213> OrganismName : HUMAN
<400> PreSequenceString :
ccaggattccatgctactagtgctgactgctgcatctcctacaccccacgaagcatcccgtgttcactcctggagagttacttt
gaaacgaacagcgagtgctccaagccgggtgtcatcttcctcaccaagaaggggcgacgtttctgtgccaaccccagtgataag
caagttcaggtttgcatgagaatgctgaagctggacacacggatcaagaccaggaagaattgaacttgtcaaggtgaagggaca
caagttgccagccaccaactttcttgcctcaactaccttcctgaattattttttaagaagcatttattcttgtgttctggatt
tagagcaattcatctaataaacagtttctcactta
<212> Type : DNA
<211> Length : 371
      SequenceName : 319095H1
      SequenceDescription :

Sequence
--------
<213> OrganismName : HUMAN
<400> PreSequenceString :
gctcttcgtctcagtcccagacacgagctggaccacgcaagtatcaaggggctctgcagaaggagctctgagagaaacagctgg
ctcagagggtcctggccccacgaggccacctgtctgtccacagcggccatcatgtttgctgccaacctgactctggggctgtac
atccactttggccccaggcctctgagccccaacagcactgcgggcctggaaagcgagtcctggggggacttggcgcaccctggc
agcacccgctggctacctcaccctggtgcccctgctggccaccatgctcttcatcatgggctacgccgtgggctggggtcccat
cacctggctgctcatgtctgaggtcctgcccctgcgtgcccgtggcgtggcctcagggctctgcgtgctggccagctggctcac
cgccttcgtcctcaccaagtccttcctgccagtggtgagcacttcggcctccaggtgcctttcttcttcttcgcggcattgttg
gtga
<212> Type : DNA
<211> Length : 508
      SequenceName : 551234R6
      SequenceDescription :

Sequence
--------
<213> OrganismName : HUMAN
<400> PreSequenceString :
ctttcacacaaagaaaaagttgtctgtgtgcgcaaatccaaaacagacttgggtgaaatatattgtgcgtctcctcagtaaaaa
agtcaagaacatgtaaaaactgtggcttttctggaatggaattggacatagcccaagaacagaaagaaccttgctggggttgga
ggtttcacttgcacatcatggagggtttagtgcttatctaatttgtg
<212> Type : DNA
<211> Length : 215
      SequenceName : 556963H1
      SequenceDescription :

Sequence
--------
<213> OrganismName : HUMAN
<400> PreSequenceString :
tggaataattttgagagtaatttgaagaattagatgcaataaatttgaaaacctttaaaaagaaaccaatggcctaactgacc
caagaagggaaagaaaatctaaacagactgataaatacaaaatgaaattcagacacttacagagc
<212> Type : DNA
<211> Length : 149
      SequenceName : 859993H1
      SequenceDescription :

Sequence
--------
<213> OrganismName : HUMAN
<400> PreSequenceString :
ggaaaacgccgtcctttgaataacaattctgaaattgcactgtcactaagcagaaataaagttttgtctttttgatctgact
gtagatgatcagtcagtttatcctaaggcattaagagatgaatacatcatgtcaaaaactcttggaagtggtgcctgtggagag
gtaaagctggctttcgagaggaaaacatgtaagaaagtagccataaagatcatcagcaaaaggaagtttgctattggttcagca
<212> Type : DNA
<211> Length : 252
      SequenceName : 864164H1
      SequenceDescription :

Sequence
--------
<213> OrganismName : HUMAN
<400> PreSequenceString :
catatagcagacattccataaaataacattttccaaattaattatgcatacatatacatctgctaattgaatgacactggtaga
tacatcttcattaagatgtgagttttatgcttgttactgcatattttcaattatgaattaagggcaagaatcaccgtcttcctg
tgaatacattcccataatttaagaattccactaagctacattctgtctaccagtttgtaagcaaatgtctctacagacctttca
agtatttatagttggtgcacagtaactccacttattttgtataatgttgctcaaccgaatgtgggatactataattatagcata
attattcatagttacagtaaaactctaaaccatttatttagaatatttcagcagtgaacagaacctacatgaaagtgtgccaa
aataatttgtgattgtttactgaaatttatgagacttgtaataggctctgtctgaacacatatgaaaaatgtagatagtgatag
tttctgtatatgggctggcttacagcttccctacattcatatttntctat
<212> Type : DNA
<211> Length : 554
      SequenceName : 2676931T6
      SequenceDescription :

Sequence
--------
<213> OrganismName : HUMAN
<400> PreSequenceString :
gtgatctcatatgattctccaaacttttgagattaattatctttataaatgaggtaatgagattttaaagagtttagatgact
tgcctaactgacaacactgtcagtggcaaagttgggatttgaactcagtctgatgggcaaaaagagttgtcttctaccagccaa
gttgccataaaagaaaactatgcattgtgcatagttttactcttgttaagtgtaagaatgataaggtaaggctattaacttgct
ttgtgaacaggctatgttctgg
<212> Type : DNA
<211> Length : 274
      SequenceName : 2704982H1
      SequenceDescription :

Sequence
--------
<213> OrganismName : HUMAN
<400> PreSequenceString :
cagggcccaggaatggctgtccaagaggaaggggctggaccgtccactccctgggttcccaaccaccccttcccctcctggtcac
ctttcggcttggcatcaggagggcttgaagaagactcctcagccccacctgcagacctagctagttgccaaccacctggggttg
tgcccagtcctcagaactcacctgcccatggtcatccgagccgttcccacaaaagccttccttgggatcctcagccgtctgggg
ggcctccttactggggtcacctcaaaaacagct
<212> Type : DNA
<211> Length : 285
      SequenceName : 2760110H1
      SequenceDescription :

Sequence
--------
<213> OrganismName : HUMAN
<400> PreSequenceString :
atgatgcggaggcagggtctgtggaggagcacaagagcgttatcatgcatctcttgtcgcaggttagacttggaatggatctta
ctaaggtagttcttccaacgtttattcttgaaagaagatctcttttagaaatgtatgcagactttttgcacatccggacctgt
ttgtgagcattagtgaccagaaggatcccaaggatcgaatggttcaggttgtgaaatggtacctctcagcctttcatgcgg
<212> Type : DNA
<211> Length : 249

SequenceName : 2803571H1
SequenceDescription :

Sequence
--------
<213> OrganismName : HUMAN:
GTTAAACTGACTATTCAGAAAGAGGTGATGAATGGTGCTATCCTTCAACAAGTGTTTGTG
GTGGATTATGTTGTTCAGTCCCAAATGTGTGGAGATTGCCATAGAGTAGAAGCTAAGGAT
TTCTGGAAGGCTGTGATTCAAGTGAGGCAAAAGACTTTGCATAAAAAAACTTTCTACTAT
CTGGAACAGTTAATTCTGAGATATGGAATGCATCAGAATACACTTCGTATCAAAGAGATT
CA

<3097063H1;DNA;HUMAN
<400> PreSequenceString :
gtttgccattttctctttcctgatagagtacagctgagacccggacactggttagagggctaggtcgggtgttggccacttgga
agataagattaggtttgccatccatgtgagctactactgctatgtcagtaaagcgaattgaaaaagctcgatttttggccggg
caatcttcgccacaaaagcacctaaataagaaattattgatttttttagaatgaagactttaaatatcaatactttttctga
atgacaagtgtatatcaaatatttacacatttcttggtgccatgcctttcagtgagtca
<212> Type : DNA
<211> Length : 311
SequenceName : 2997457H1
SequenceDescription :

Sequence
--------
<213> OrganismName : HUMAN
<400> PreSequenceString :
actgaaatacggaatcaagttaaaaaagagatgatccttgccaaacgtttttctttatagtatttactgatgcattatgctgg
atacccattttgtagtgaaatttctttcactgcttcaggtagaaataccaggtaccataacctcttgggtagtgatttttatt
ctgcccattaacagtgctttgaacccaattctctatactctgaccacaagaccatttaaagaaatgattcatcggttttggtat
aactacagacaaagaaaatctatggacagcaaaggt
<212> Type : DNA
<211> Length : 288
SequenceName : 3556218H1
SequenceDescription :

Sequence
--------
<213> OrganismName : HUMAN
<400> PreSequenceString :
caaagttggagatctgcaacctcacggccgatgctctgcagtctgacaagcttcagacctggacagtgtctgtgaacgagggcc
gctgggtacggggttgctctgccggaggtcgccgcaacttcccagatactttctggaccaaccctcagtaccgtctgaagctcc
tggaggaggacgatgaccctgatgactcggaggtgatttgcagcttcctggtggccctgatg
<212> Type : DNA
<211> Length : 230
SequenceName : 3576337H1
SequenceDescription :

Sequence
--------
<213> OrganismName : HUMAN
<400> PreSequenceString :
tgcagccccaaatctggaggaattatcacgtcttacagcttgaaagtacaggtgaaaatgtcaaacctcaccatcaattganaa
acacagtgactgtggataatactgtgggcaacgacactatgtttctagttacgtggcaggccagtggtcctcctgagattatat
tatttgatcctgatggacgaaaatactacacaaataattttatcaccaatctaacttttcggacagctagtctttggattccag
gaacagctaagcctgggcactggacttacaccctgaacaatacccatcattctctgcaagccctgaaagtgacagtgacctctc
gcgcctccaactcagctgtgcccccagccactgtggaagcctttgtggaaagagacagcctccatttcctcatcctgtgatga
tttatgccaatgtgaaacagggattttatcccattcttaatgccactgtcactgccacagttgagccagagactggagatctgt
tacgctgagactccttgatgatggagcaggtgctgatgttat
<212> Type : DNA
<211> Length : 546
SequenceName : 3618886F6
SequenceDescription :

Sequence
--------
<213> OrganismName : HUMAN
<400> PreSequenceString :
ttttaaaatatgaaaagaaactggccaaaatgtgcttttaatgatattcaccttcctggtctgttggatgccttatatcgtga
tctgcttcttggtggttaatggtcatggtcacctggtcactccaacaaatctattgtttcgtacctctttgctaaatcgaacac
tgtatacaatccagtgatttatgtcttcatgatcagaaagtttcgaagatcccttttgcagcttctgtgc
<212> Type : DNA
<211> Length : 238
      SequenceName : 4540025H1
      SequenceDescription :

Sequence
--------
<213> OrganismName : HUMAN
<400> PreSequenceString :
gcctcgtttctggctggggtctgctggcgaacggcagaatgcctaccgtgctgcagtgcgtgaacgtgtcggtggtgtctgagg
aggtctgcagtaagctctatgacccgctgtaccaccccagcatgttctgcgccggcggagggcaagaccagaaggactcctgca
acggtgactctgggggcccctgatctgcaacgggtacttgcagggccttgtgtctttcggaaaagccccgtgtggccaagttg
g
<212> Type : DNA
<211> Length : 253
      SequenceName : 1810447H1
      SequenceDescription :

Sequence
--------
<213> OrganismName : HUMAN
<400> PreSequenceString :
gcggggcagagctgtgggctgcttgtcctggnnggagatgaagaaagcaatttccaacctnacaaatccggcagccccaaanta
agccgggccagcaagatgaaacagggcaccccagctnccctggtactnaggttg
<212> Type : DNA
<211> Length : 138
      SequenceName : 194370H1
      SequenceDescription :

Sequence
--------
<213> OrganismName : HUMAN
<400> PreSequenceString :
cttctcaaggccacacgactggaaagaggcagaacaaggacttcacccgagtcgtctgtctttttttctgaatgctcgcaacgc
cctggtggtctccttcgcagccctggttgcgtactccttcgaggtgactggataccagcctttaatcctaacaggggagacagc
tgaggggctccctccagtccggatcccgcccttctcagtgaccacagcca
<212> Type : DNA
<211> Length : 218
      SequenceName : 198381H1
      SequenceDescription :

Sequence
--------
<213> OrganismName : HUMAN
<400> PreSequenceString :
cccagaggctcatcaggacttgtaaacagcagacagaagagctatgaccactcaaggcaccatcatgggagcagcattgctgga
ggactggtgaagggggctttgtctgttgctgcctctgcatacaaggccctgtttgctggccaccagtcactgcacagccaata
atttctgaagatcagacagcagccctgatggcccatctctttgaaatgggattctgtgaca
<212> Type : DNA
<211> Length : 229
      SequenceName : 2134847H1
      SequenceDescription :

Sequence
--------

<213> OrganismName : HUMAN
<400> PreSequenceString :
gcgctgccgccgccgcgtctgctgaagcctccgagatgccggcgcgtaccgccccagcccgggtgcccacactggccgtcccgg
ccatctcgctgcccgacgatgtccgcaggcggctcaaagatttggaaagagacagcttaacagaaaggaatgtgtgaaggaga
aattgaatctcttgcacgaatttctgcaaacagaaataaagaatcagttatgtgacttggaaaccacattacgtaaagaagaat
tatccgag
<212> Type : DNA
<211> Length : 260
      SequenceName : 2159360H1
      SequenceDescription :

Sequence
--------
<213> OrganismName : HUMAN
<400> PreSequenceString :
ggcacccagagtacaacagacccttgctcgctaacgacctcatgctcatcaagttggacgaatccgtgtccgagtctgacacca
tccggagcatcagcattgcttcgcagtgccctaccgcggggaactcttgcctcgtttctggctggggtctgctggcgaacggtg
agctcacggggtgactctgggggcccctgatctgcaacgggtacttgcagggccttgtgtctttcggaaaagccccgtgtggc
caagttggcgt
<212> Type : DNA
<211> Length : 263
      SequenceName : 2185556H1
      SequenceDescription :

Sequence
--------
<213> OrganismName : HUMAN
<400> PreSequenceString :
ggcgcctgcggtgggtgagcgcacgcganggcggcgaggcggcacgtgtttctaggtcgtggcgtcgggcttccggagctttgg
cggcagctaggggaggatggcggagtcttcggataagctctatcgagtcgagtacgccaagagcgggcgcgcctcttgcaagaa
atgcagcgagagcatccccaaggactcgctccggatggccatcatggtgcagtcgcccatgtttgatggaaaagtcccacactg
gtaccacttctcctgcttctggaaggtgggccatccatccggcaccctgacgttgaggtggatgggttctctgagcttcggtg
<212> Type : DNA
<211> Length : 335
      SequenceName : 2403512H1
      SequenceDescription :

Sequence
--------
<213> OrganismName : HUMAN
<400> PreSequenceString :
gtgtagcaagaggcagattttggtgccaaatagatttgaatcctggttctgcttcttcctttgtagagtatgatattggntct
ttcctcccaaagctattataaagactaaatatgtacacaaatctttggggatgtctgacatataaatgcttaacaataggtatt
tgctggtattattacaaatgaatttgcttattttgagccacttctatgtctgtccattaaaccaaaatgtgttctgc
<212> Type : DNA
<211> Length : 246
      SequenceName : 2445101F6
      SequenceDescription :

Sequence
--------
<213> OrganismName : HUMAN
<400> PreSequenceString :
cccctccaaaaacccagccaacagaggacaacaagttcaaattctgtttgagctgtgcccgtttggctgagatgaggcaaaaag
aaatccccagggtcctggtgcagctcgaggacctggatagccgggtcctctactactcagccaccaagaacggcatcctgtacc
gatttggtgatggtgtgtacctgccccctgaggccttcacgttcaacatcaagctgtccagtcccgtgaaacggccacggaagg
ag
<212> Type : DNA
<211> Length : 254
      SequenceName : 264639H1
      SequenceDescription :

Sequence

--------
<213> OrganismName : HUMAN
<400> PreSequenceString :
gctgggcaatgacatcgcccttatgaagctggccgggccactcacgttcaatgcagaaatgatccagcctgtgtgcctgcccaa
ctctgaagagaacttccccgatggaaaagtgtgctggacgtcaggatggggggccacagaggatggaggtgacgcctcccctgt
cctgaaccacgcggccgtccctttgatttccaacaagatctgcaaccacagggacgtgtacggtggcatcatctcccccctccat
gctctgcgcgggctacctgacgggtggcgtggacagctgccagggggacagcggggggcccctggtgtgtcaagagaggaggct
gtggaagttagtgggagcgaccagctttggcatcggctgcgcagaggtgaacaagcctggggtgtacacccgtgtcacctcctt
cctggactggattcacgagcagatggagagagacctaaaaancttgaagaggaaggggacaagtagccacctgagttcctgagg
tgatgaagacagcccgattctcccctggactcccgtgtagga
<212> Type : DNA
<211> Length : 546
      SequenceName : 1001514R6
      SequenceDescription :

Sequence
--------
<213> OrganismName : HUMAN
<400> PreSequenceString :
atctggagcacatgaattggataggctgaaaattatattcatagatctgaatataatgatctgaaagtggcagtactatcagaa
aaaaaccctgtgaggcttattttggtaatacaatttacttataaaggctgaatcaacaataaaaagttgtggtcttattttaat
gaaataaaagcagagaaacaaaaaaatgcatgccctcctacatccctgactatattgaaccataaacattcctatcatgtttta
aaatagatattacaaatcctatagagcatgcaattggttttaaaaggctaaagactaaattagaaaactcattagcagaattaa
atatgatgttgggattgcccatctcgcctgaccatgctgtgatgtgtcacacgattcagcagagagcagctgggcaagtctagc
acttccttgccatggccggggaaggaacatagctgagtagcatgcaggccagtcaaaattcatttaggaacgtgattgtacatg
ta
<212> Type : DNA
<211> Length : 506
      SequenceName : 1266202T6
      SequenceDescription :

Sequence
--------
<213> OrganismName : HUMAN
<400> PreSequenceString :
gtgagttacacttcttcctccccaccaggtgctctctgcagctctggaaaaatggtgtcctctttgttgtcccaccagggggcg
ccacctccagccccgccccagcctcatacccagttcttcagctcggccagcggtaactgaagcctcccagaatcctggatccgg
gcccctagtaccctctttcccagggacccaggagtcctgcctccagtcgcctgcacttgtaactgagagctggaggtcgtccat
agcagcatagtgagagtgtttttgatgagggtatgcagagtgggggtgaccatgttcccacctggggcctcaggtggccaagg
cctacccactttagccagcgtcccctccagcagccatcagcaagccaacccactccaagccagggcccccttttggtccttgcac
ttgaggtgctttgttcagggctgggtcaggagtggcagagacgatgtccaacaacctcagtactgggggaaaagtagcctgg
<212> Type : DNA
<211> Length : 502
      SequenceName : 1322305T6
      SequenceDescription :

Sequence
--------
<213> OrganismName : HUMAN
<400> PreSequenceString :
actgggcataatttcctcattatctacagcctttaccctgaacaatggagtttaatcaccaaaaaaattagatactaggctc
cagatgataggatggcccaagttatttaacgttttcttgtttaaaaggtgggcccattggcacttaagaaaataaatcagaaaa
taatatggcttcaagtggacaaataaatatgaatttctaaggaagtcttcgcattatctattggtttcctcttgatttggttcc
atggataatttctctccaagttctctaataagttctgctaggtcttctgtattctgagatttttcttcaaggagttcatagcgg
agacttgagatgtcctgcttaatttccttcagttccccttcgttcacttcatcactctccttatctatc
<212> Type : DNA
<211> Length : 405
      SequenceName : 1392516T6
      SequenceDescription :

Sequence
--------
<213> OrganismName : HUMAN

<400> PreSequenceString :
attcttcactcctgctccaagggctgggctgtgggtgggatactgggattttgggccactggatttccctaaatttgtccccc
ctttactctccctctatttttctctccttagactccctcagacctgtaaccagctttgtgtctttttttcctttctctcttta
aaccatgcattataactttgaaa
<212> Type : DNA
<211> Length : 191
      SequenceName : 1539060H1
      SequenceDescription :

Sequence
--------
<213> OrganismName : HUMAN
<400> PreSequenceString :
atcgagatcctgcgcgacgacgtggtcgtggccatcctaggaaagaatgacatctttggggaacccgtcagcctccatgcccag
ccaggcaagtccagtgcagacgtgcgggctctgacctactgcgacctgcacaagatccagcgggcagatctgctggaggtgctg
gacatgtacccggcctttgcggagagcttctggagtaagctggagg
<212> Type : DNA
<211> Length : 214
      SequenceName : 1540906H1
      SequenceDescription :

Sequence
--------
<213> OrganismName : HUMAN
<400> PreSequenceString :
ccggtgtttggactctacacttctttcttccccgtcctcatctacagcttgctaggtactgggagacacctgtccacaggaact
ttcgccatactcagcctcatgacaggctcggccgtcgagcggctggtgccggancccctcgtggggaatctgagcggaatcgag
aaggagcagctggacgctcaacgggttggggtagccgcggccgtggccttcgggagcggggcgttgatgctggggatgttcgtg
ctgcagctcggcgtcttgtccaccttttttgtccgagcctgtggtcaaggcgctgaccagcggggccgcgctgcacgtgctcttg
tcccagctgccgagcctcttggggttgtccctcccgcgccagatcggctgcttctctctcttcaanacgctggcctccttgctg
actacgctgcctcggancagtccggccgaactgaccatctccgcgctcagcctggcgctgctcgtgccggtaaagggaattgaa
cgtgagattccgagaccggctaacccac
<212> Type : DNA
<211> Length : 532
      SequenceName : 1654210F6
      SequenceDescription :

Sequence
--------
<213> OrganismName : HUMAN
<400> PreSequenceString :
acagatcctaagccgcccccgcccgcctgctcggacagtttccgttccctcctagtctctgttcgctcgcttaagctgttttga
agccaagtgaagagtgtggtttgcatccaagagaaaacaccacactgtgatgtcatcgggaaatgatctcccaagtcgctgcca
gacaccacccactgcttcgccggacaatgtcgaagtccagtctgtgccggggaaggcactggttaggaaggatgttcaaacggt
cccacccaagcctgtcacctctgctcatcctccaagaccacccagctttctcccagccacagccaagattcccaaagtcaaggc
ccaaagatttccaaggttcccaaagtcaaggccaggccaaggcctggttgggtccttggggcgggcaagggcggnctcttcctc
tgctgagaatccccacttggtgtaaggggaga
<212> Type : DNA
<211> Length : 452
      SequenceName : 1671033F6
      SequenceDescription :

Sequence
--------
<213> OrganismName : HUMAN
<400> PreSequenceString :
ctttttttgaggtttggatgttgcagctagtaaaggatatttttgctctgttcagcagttctaaaaattgctgaagtaggggcc
aggtcactggtagttatagtatggaatgggagaagtgaaagttcagttatagaactttccatacttccaagtttactgcaagtt
tttatgcttgagagagatgctttctaatataagactgatgtgttgattttactgattgtactgtacatctattaaagccttaga
ttattacattacgggttggaacccataccaatgtaatttcaatcgtgttaagaaagtaatngtgacttcacatgttattgtagt
tagttacattata
<212> Type : DNA
<211> Length : 349

SequenceName : 000527H1
SequenceDescription :

Sequence
--------
<213> OrganismName : HUMAN
<400> PreSequenceString :
atttgaaaaaatgtatttaaaagtccaacaacttttaatataaattacgactctcaaacccattcccatcactttattagtga
tggtagcatacatattagagaaggtagctaaaggcaagagagcaccaaaggaaaaagactgtccaaagaacaggtattagaatg
aggccgaagatcacggtgaccagagatttctaggagtctctaacctttccaccctatcctgttaaccctttagatctctagtat
aacactcag
<212> Type : DNA
<211> Length : 261
SequenceName : 002783H1
SequenceDescription :

Sequence
--------
<213> OrganismName : HUMAN
<400> PreSequenceString :
tgtacatctattaaagccttagattattacattacgggttggaacccataccaatgtaatttcaatcgtgttaagaaagtaatg
gtgacttcacatgttattgtagttagttacattatagaatattacttattttctttgttaaaatgtagtttttcatttcctaca
tttattagattttcattttctattaacaattgaataccatttcatnttatagactt
<212> Type : DNA
<211> Length : 224
SequenceName : 059476H1
SequenceDescription :

Sequence
--------
<213> OrganismName : HUMAN
<400> PreSequenceString :
tgcaaatatgtagtccaaaacaacaaaaaaagaaaatgtcttatgtgacttagcacgaacagtttccacagcctttcacactat
tgacaatttcttcttgtagtttctttctttcctcctctttggatatattttctttctttgcttcccatttggggttatcatggt
tctgcacacggctgctctgtgtatgccacatttctgaatagatactatgagggttagcaagcaaaccatggtgggtaccacgtt
cggctaccttaccctgatccaagacaatgatttcatctgcatcaaccactgttgacaatctgtgtgcaatgaaaatagaagttc
tgtgtttgaccacatccttcatggcaccaagaatagtctcttcagtaatcgaatctaacgatgaagtagcttcatcatagagta
tgactgggggggccttcaaaatggntcttgcaattgctaactctttggcttttctcctc
<212> Type : DNA
<211> Length : 479
SequenceName : 1360938T6
SequenceDescription :

Sequence
--------
<213> OrganismName : HUMAN
<400> PreSequenceString :
gttataactaatctgcctgaagacttctcatgatggaaaatcagccaaggactaagcttccatagaaatacactttgtatctgg
acctcaaaattatgggaacatttacttaaacggatgatcatagctgaaaataatgatactgtcaatttgagatagcagaagttt
cacacatcaaagtaaaagatttgcatatcattatactaaatgcaaatgagtcgcttaacccttgacaaggtcaaagaa
<212> Type : DNA
<211> Length : 246
SequenceName : 1445982H1
SequenceDescription :

Sequence
--------
<213> OrganismName : HUMAN:
TGGATGTTCGTGGAATTGCTGATCCATCCAAGGGCGTCCTTTGGAGCCAGTGGAGCCTGC
CGGCGCATCTGAGGGGCAGAATGCTGCTAGCACTTGAATCTGGGATCTCGCCTTATTCTC
AAGTAGCAAGGCATCTCGACAAGCATGGTCTAGGTCTGGTGGCCAGCTTGCCAGTACCTG
AGCCGGTCGGGTCATCTGCCTCTGAGGGACCGTCCTCACCGAG

<1525902F6;DNA;HUMAN
<400> PreSequenceString :
ggagctgttcctgaaggttgccggcctgggccctgtggagtacttgtcttccggatggaacttgtttgacttctccgtgacagt
gttcgccttcctgggactgctggcgctggccctcaacatggagcccttctatttcatcgtggtcctgcgcccccctccagctgct
gaggttgtttaagttgaaggagcgctaccgcaacgtgctggacaccatgttcgagctgctgccccggatggccagcctgggcct
caccctgctcatcttttactactccttcgccatcgtgggcatggagttcttctgcgggatcgtcttccccaactgctgcaacac
gagtacagtggcagatgcctaccgctggcgcaaccacaccgtgggcaacaggaccgtggtggaagaaggtactattatctcaat
aattttgacaacatcctcaacagcttt
<212> Type : DNA
<211> Length : 447
      SequenceName : 1489075H1
      SequenceDescription :

Sequence
--------
<213> OrganismName : HUMAN
<400> PreSequenceString :
agctgcctga gccagccagt gacagcgttt cctcagggca cttggattgg tgaagacata      60
ttccctcctc tgctgcctcc cactgtaatc aggacct                               97
<212> Type : DNA
<211> Length : 97
      SequenceName : 1548205H1
      SequenceDescription :

Sequence
--------
<213> OrganismName : HUMAN
<400> PreSequenceString :
cacagatgaaggacgtgttcttcttcctcttcttcctcggcgtgtggctggtagcctatggcgtggccacggaggggctcctga
ggccacgggacagtgacttcccaagtatcctgcgccgcgtcttctaccgtccctacctgcagatcttcgggcagattccccagg
aggacatggacgtggccctcatggagcacagcaactgctcgt
<212> Type : DNA
<211> Length : 210
      SequenceName : 1693028H1
      SequenceDescription :

Sequence
--------
<213> OrganismName : HUMAN
<400> PreSequenceString :
atgagaacggctacaacttcaagtccgacatctggtccttgggctgtctgctgtacgagatggcagccctccagagccccttct
atggagataagatgaatctcttctccctgtgccagaagatcgagcagtgtgactacccccccactccccggggagcactactccg
agaagttacgagaactggtcagcatgtgcatctgccctgacccccaccagagacctgacatcggatacgtgcaccaggtggcca
agcagatgcacatctggatgtccagcacctgagcgtggatgcaccgtgccttatcaaagccagcaccactttgccttacttgag
tcgtcttctcttcgagtggccacctggtagcctagaacagctaagaccacagggttcagcaggttccccaaaaagctgcccagc
cttacagcagatgctga
<212> Type : DNA
<211> Length : 437
      SequenceName : 1931159F6
      SequenceDescription :

Sequence
--------
<213> OrganismName : HUMAN
<400> PreSequenceString :
ccgctgacagaggagctggtgttccgggcctgtatgctgcccatgttagcaccgtgcatgggcctgggcctgctgtgttcacct
gcccgctctttttggagttgcccattttcaccatattattgagcagctg
<212> Type : DNA
<211> Length : 134
      SequenceName : 195337H1
      SequenceDescription :

Sequence

--------
<213> OrganismName : HUMAN
<400> PreSequenceString :
gtcaaggagaagtccataaggatcatcatcacgctgctggtgcaggtgctcgtctgctttatgcccttccacatctgtttcgct
ttcctgatgctgggaacgggggagaacagttacaatccctggggagcctttaccaccttcctcatgaacctcagcacgtgtctg
gatgtgattctctactacatcgtttcaaaacaatttcaggctcgagtcattagtgtcatgctataccgtaa
<212> Type : DNA
<211> Length : 239
      SequenceName : 2005142H1
      SequenceDescription :

Sequence
--------
<213> OrganismName : HUMAN
<400> PreSequenceString :
cagaggcttggaggggatgtgcagagccccaactgcccatctgaggatgtagtcatcactccagaaagctttggaagagattca
tccctcacatgcctggctgggaatgtcagtgcatgtgacgcccctattctctcctcttccaggtccctagactgcagggagagt
ggcaagaatgggcctcatgtgtaccaggacctcctgcttagccttgggactacaaacagcacgctgccccctccatttctctc
caatctggaatcctgacattgaacccagttgctcagggtcagcccattcttacttccctgggatcaaatcaagaagaagcatat
gtcaccatgtccagcttctaccaaaaccagtgaagtgtaagaaaaccccagaactgaacttaccgtgagcgaccaaagatgatt
taaaaagggaagtctagagttcctagtctccctaacagcaccagagaagaca
<212> Type : DNA
<211> Length : 472
      SequenceName : 205581R6
      SequenceDescription :

Sequence
--------
<213> OrganismName : HUMAN
<400> PreSequenceString :
gctcgcgtcgcatttggccgcctccctaccgctccaagcccagccctcagccatggcatgccccctggatcaggccattggcct
cctcgtggccatcttccacaagtactccggcagggagggtgacaagcacaccctgagcaagaaggagctgaaggagctgatcca
gaaggagctcaccattggctcgaagctgcaggatgctgaaattgcaaggctgatggaagacttggaccggaacaaggaccagga
ggtgaacttccaggagtatgtcaccttcctgggggc
<212> Type : DNA
<211> Length : 288
      SequenceName : 2135769H1
      SequenceDescription :

Sequence
--------
<213> OrganismName : HUMAN
<400> PreSequenceString :
tttggtcaagttgtttccattaaaaagtactgattttaaaaactaataacttaaaactgccacacgcaaaaaagaaaaccaaag
tggtccacaaaacattctcctttccttctgaaggttttacgatgcattgttatcattaaccagtcttttactactaaacttaaa
tggccaattgaaacaaacagttctgagaccgttcttccaccactgattaagagnggggnggcaggcantagggananagtcan
ttngccttcnnagcnttctgggcagacntggcgaccncnncagcncnagcagccntctcgtccacggctntgtntgacacccac
cgcaacggncngnctctatancacgaacagcaaagcgacccaaaggng
<212> Type : DNA
<211> Length : 384
      SequenceName : 2206642T6
      SequenceDescription :

Sequence
--------
<213> OrganismName : HUMAN
<400> PreSequenceString :
gggtgaaggtggcttcgggggtcgtggtggaggcagaggcggctttggaggacgaggtggtggtagaggaggccgaggaggatt
tggtggcagaggccggggaggctttggagggcgaggaggcttccnnnnnnnnnnnnnnnnnnnnnnnnaccacaagccacaagg
aaagaagacgaagtttgaatagcttctgtccctctgctttcccttttccatttgaaagaaaggactctggggtttttactgtta
cctg
<212> Type : DNA
<211> Length : 256

SequenceName : 2294719H1
SequenceDescription :

Sequence
--------
<213> OrganismName : HUMAN
<400> PreSequenceString :
cttctttatggcgtcgctcctgtgctgtgggccgaagctggccgcctgcggcatcgtcctcagcgcctggggagtgatcatgtt
gataatgctcggaatatttttcaatgtccattccgctgtgttgattgatgacgttcccttcacggagaaagattttgagaatgg
cccccagaacatatacaacctttacgagctagtcagctacaactgtttcatcgctgcaggcctttacctcctcctcggaggctt
ctctttctgccaagttcggctcaataagcgcaaggaatacatggtg
<212> Type : DNA
<211> Length : 298
SequenceName : 2515486H1
SequenceDescription :

Sequence
--------
<213> OrganismName : HUMAN
<400> PreSequenceString :
gaggaacagcgccatgtgctccgggctcctggagctcctgctgcccatctggctctcctggaccctggggacccgaggctctga
gccccgcagtgtgaacgatcccgggaacatgtcctttgtgaaggagacggtggacaagctgttgaaaggctacgacattcgcct
aagacccgacttcgggggtcccccggtctgcgtggggatgaacatcgacatcgccagcatcgacatggtttccgaagtcaacat
gagattctggctgcaggaaaggggaacgaagacagtggtctgtgcgttccaggggtgtctctgcggtttttccaaggctgcctc
ctggactgggagacccgggcccggcaccgccagtctctgtccgaggtgctgacaggcttccgctgcttcagagagcgggaggcc
gcgcctaggcgggctctcagaggagccgctctaccgggagagtcggaggctggtgacccagtgtcacttaggtcttctgtgaat
gcagactggattcaatattctgacctgtgggaagcg
<212> Type : DNA
<211> Length : 540
SequenceName : 257274R6
SequenceDescription :

Sequence
--------
<213> OrganismName : HUMAN
<400> PreSequenceString :
aagaggaccaatgaaaagggtgccttctggggcctgatctcgggcctgctcctgggcttggttaggctggtcctggactttatt
tacgtgcagcctcgatgcgaccagccagatgagcgcccggtcctggtgaagagcattcactacctctacttctccatgatcctg
tccacggtcaccctcatcactgtctccaccgtgagctggttcacagagccaccctccaaggagatggtcagccacctgacctgg
tttactcgtcacgaccccgtggtccagaaggaacaagcaccaccagcagctcccttgtctcttaccctctctcagaacgggatg
ccagaggcnnnnnnnnnnnnnnnnngtccagttcgagatggttcaagaaaacacgtctaaaanccacagctgtgacatgacccca
aagcagtccaaagtgtgaangccatctgtggntctgtggaatacaggagaagggcaaagnagagctcccggcnagagcagaagc
natc
<212> Type : DNA
<211> Length : 508
SequenceName : 2707270F6
SequenceDescription :

Sequence
--------
<213> OrganismName : HUMAN
<400> PreSequenceString :
ttcaggagtgggcgaantctatgagtttgttcacagttttcaggatgagaagcgtcantggncttaccaggttttgggcctctc
agaaggggcaacaaatgaagaaatacatcggagttaccaggagctagtgaaggtctggcacccagaccacaacctggaccagac
agaggaggcacagaggcacttcctggagatccaggctgcgtatgaagtcctgagtcaacccaggaagccctggggatcccggag
gtgaaaagaaacttccccctgaggactgactcttcctagcagagctgggcaacttgtcccaaatctagctttgcccacgaatgg
catcccaacagagttaaagaaactgcttgcaggggctgggcgtggtnnnnnnnnnnnnnnnnnnnnnnnnnnnnnnnnnnnnn
nnnnnnnnnnnnnnnnnnnnnnnnnnnnnnnnnnnnnnnnnnnnnnnnnnnnnnnnnnnnnnnnntggatatatttaattta
ctcaaatgggaactactgataagcagttcttattttccagcataagca
<212> Type : DNA
<211> Length : 553
SequenceName : 2792728F6
SequenceDescription :

Sequence
--------
<213> OrganismName : HUMAN
<400> PreSequenceString :
tgctataagcaaaaacatccaattgggtgatgagaagggcttaaaattcccctctgtttgggactggtctctccagtttacagc
aaaggatcgcacccttttccataacccccttctacattggaaagagcacaccttgtatacagaatggctccgtgaagtcttttaa
acggacaaaggtaaatcacagctaacaaaacgtgatgttggctcacacgtaaccaaacacctctttttcagaacagagagcgtt
aaaagtaaaggcagttccaagagtaacactgctaatgtgggtttctgaggggtcattccctttttaactcaaatgactgtatcc
caactttcttcctggtgtctgaggcccacaaagtctcagtacctgagagtgggcagattgcagctttgagcctgcaagcctgat
ttactaaagccccatttatccatttcttgatgattcaagccgccactgtggcagggaatgccgcctggctgtgatgtagtcatg
gcctcctgactgctatantcttgtcctaataacattcattggttgnccttttaataantccccaataaat
<212> Type : DNA
<211> Length : 574
      SequenceName : 2821685T6
      SequenceDescription :

Sequence
--------
<213> OrganismName : HUMAN
<400> PreSequenceString :
gcccctgcctgcctgcctcaacccactgctgtacctgctcttcaacccccacttccgggatgaccttcggcggcttcggccccg
cgcaggggactcagggcccctagcctatgctgcggccggggagctggagaagagctcctgtgattctacccaggccctggtagc
cttctctgatgtggatctcattctggaagcttctgaagctgggcggcccctgggctggagacctatggcttcccctcagtgac
cctcatctc
<212> Type : DNA
<211> Length : 261
      SequenceName : 3038910H1
      SequenceDescription :

Sequence
--------
<213> OrganismName : HUMAN
<400> PreSequenceString :
taaacatcccaaaactggagttttcgaagagaaacatgccaaacctccagatgtagaccttaaaaagttctttacagacaggaa
gactcatctttataccctgtgatgaatccagatgacacatttgaggtgttagttgatcaaacagttgtaaacaaaggaagcct
cctagaggatgtggttcctcctatcaaacctcc
<212> Type : DNA
<211> Length : 201
      SequenceName : 3107995H1
      SequenceDescription :

Sequence
--------
<213> OrganismName : HUMAN
<400> PreSequenceString :
ccgccgccgacgccgcttgtgggcccgactccgctctgtctgcttcgccaccttctccccgagcactgcccggccggccgccat
ggctaacgtggctgacacgaagctgtacgacatcctgggcgtcccgcccggcgccagcgagaacgagctgaagaaggcatacag
aaagttagccaaggaatatcatcctgataagaatccaaatgcaggagacaaatttaaagaaataagttttgcatatgaagtact
atcaaatcctgagaagcgtgagttatatgacaga
<212> Type : DNA
<211> Length : 286
      SequenceName : 3118530H1
      SequenceDescription :

Sequence
--------
<213> OrganismName : HUMAN
<400> PreSequenceString :
tggcgtggcctcagggctctgcgtgctggccagctggctcaccgccttcgtcctcaccaagtccttcctgccagtggttagcac
cttcggcctccaggtgcctttcttcttcttcgcggccatctgcttggtgagcctggtgttcacaggctgctgtgtgcccgagac
caagggacggtccctggagcagatcgagtccttcttccgcacggggagaaggtccttcttgcgctaggtcaaggtccccgcctg
gagggggccaaacccccagtggctgggcctctgtgt

<212> Type : DNA
<211> Length : 288
      SequenceName : 3141315H1
      SequenceDescription :

Sequence
--------
<213> OrganismName : HUMAN
<400> PreSequenceString :
atcacacacgaagagagccatcttccagaaataagtttatacactctctcctctaattgcatcaggactttaccagataatgtt
cttccagatctgaaaaggaaaatgcctaaaagagcttccaaactcattttgaataatactaggctacaaagaattacactgtg
aattcat
<212> Type : DNA
<211> Length : 175
      SequenceName : 335737H1
      SequenceDescription :

Sequence
--------
<213> OrganismName : HUMAN
<400> PreSequenceString :
aggcttgacaccaagcacgtgcctgcctcgtcaggctacaacttcaggtttgccaaatactacaagatcaatggcaccaccacc
cgcacgctcatcaaggcctacgggatccgcattgacgtcattgtgcatggacaggccgggaagttcagcctgattcccaccatt
attaatctggccacagctctgacttccgtcggggtggtaaggaaccctctctggggtcccagcgggtgcgggggtccaccagg
cccttacacaccggtctct
<212> Type : DNA
<211> Length : 271
      SequenceName : 3363678H2
      SequenceDescription :

Sequence
--------
<213> OrganismName : HUMAN
<400> PreSequenceString :
ctgtcaccaggagagcacgccaaggacgaatatgtgctatactattactccaatctcagtgtgcctattgggcgcttccagaac
cgcgtacacttgatgggggacaacttatgcaatgatggctctctcctgctccaagatgtgcaagaggctgaccagggaacctat
atctgtgaaatccgcctcaaaggggagagccaggtgttcaagaaggcggtggtactgcatgtgctt
<212> Type : DNA
<211> Length : 234
      SequenceName : 3422674H1
      SequenceDescription :

Sequence
--------
<213> OrganismName : HUMAN
<400> PreSequenceString :
angnncnggacatgnanctganagntannnnaangggtnnaaggantgnncaaatncngacnctagcagggatgtgnncactg
tcanggggccagnataactgnggntgttgaagancctcttttcctcagtntggntcttctgccancagtcagatgcanggangc
ccggtgcagaagcaccaggcactaataatcaggagaggcatggctagattccactcacttccaacatttncttctggtctcttt
tcttagggactcacccctttggttgggaacattctccagcagtacagccacagangggccagagatgccagaagcagcagaatc
cagacacccaagagcatggcctgaagggttctgaacaaggccttcagcatgtcccatgaaggaagtacataggtgtaagcttct
gactttcttctggactcttgagtgggg
<212> Type : DNA
<211> Length : 447
      SequenceName : 346874T6
      SequenceDescription :

Sequence
--------
<213> OrganismName : HUMAN
<400> PreSequenceString :
cgcagccgcccgccgcccgcgccgccatgggagtggagggctgcaccaagtgcatcaagtacctgctcttcgtcttcaatttcg
tcttctggctggctggaggcgtgatcctgggtgtggccctgtggctccgccatgacccgcagaccaccaacctcctgtatctgg

agctgggagacaagcccgcgcccaacaccttctatgtaggcatctacatcctcatcgctgtgggcgctgtcatgatgttcgttg
gcttcctgggctgctacggggccatccaggaatcccagtgcctgctggggacg
<212> Type : DNA
<211> Length : 305
      SequenceName : 3801801H1
      SequenceDescription :

Sequence
--------
<213> OrganismName : HUMAN
<400> PreSequenceString :
ctggaagacttagacaatgctaaggaaaaatttaatgatggaaatatcgacaaatattcaacatcatttaaaaacaaagtagct
tctcttatttcacatagcttagtttgggatagaaatagaactaatgtttacaatgattcttacatttagcattaatcagaaacg
agggtttgttaagtgcccttgctgnnnnnnnnnnnnnnnnnnnnnnnnnnnnnnnnnnnnnnnnnnnnnnnnnnnnnnnnnnnnn
nnnnnnnnnnnnnnnnnnnnnnnnnnnnnnnnnnnnnnnnnnnnnnngtttgagaaccgctggattgattctttgacagtttttg
aagtccgctgctcaccatatcacttcatcctcttataaactttcactatgtgattgcctaagagcccctgcttgttggctgctc
cacattttcacacagttgtactatttctttctgaaatgacattgtttgagggacagtgggtggaacgtgtgagctcctcactga
aggctgcctccagaattccctgaatggactgccttgctttcttcctaaaatctttccccaagagggcataaaggaagggattaa
agcaactattggcagatgctagagcaatgcatacatg
<212> Type : DNA
<211> Length : 625
      SequenceName : 391237F1
      SequenceDescription :

Sequence
--------
<213> OrganismName : HUMAN
<400> PreSequenceString :
caatgctaaggaaaaatttaatgatggaaatatcgacaaatattcaacatcatttaaaaacaaagtagcttctcttatttcaca
tagcttagtttgggatagaaatagaactaatgtttacaatgattcttacatttagcattaatcagaaacgagggtttgttaagt
gcccttgctgnnnnnnnnnnnnnnnnnnnnnnnnnnnnnnnnnnnnnnnnnnnnnnnnnnnnnnnnnnnnnnnnnnnnnnnnnnn
nnnnnnnnnnnnnnnnnnnnnnnnnnnnnnnnngtttgagaaccgctggattgattctttgacagtttttgaagtccgctgctca
ccatatcacttcatcctcttataaactttcactatgtgattgcctaagagcccctgcttgttggctgctccacattttcacaca
gttgtactatttctttctgaaatgacattgtttgagggacagtgggtggaacgtgtgagctcctcactgaaggctgcctccaga
attccctgaatggactgccttgctttcttcctaaaatctttccccaagagggcataaaggaagggattaaggcaactattggc
agatgctaggagcaatgcataca
<212> Type : DNA
<211> Length : 611
      SequenceName : 510997F1
      SequenceDescription :

Sequence
--------
<213> OrganismName : HUMAN
<400> PreSequenceString :
gtggtcgggggagcggccggagaggagctgccgggagttcgtgccctgcaggacatgacaccagtggcatatcacggccatggg
gtctcagcattccgctgctgctcgcccctcctcctgcaggcgaaagcaagaagatgacagggacggtttgctggctgaacgaga
gcaggaagaagccattgctcagtacccatatgtggaattcaccgggagagatagcatcacctgtctcacgtg
<212> Type : DNA
<211> Length : 240
      SequenceName : 5122087H1
      SequenceDescription :

Sequence
--------
<213> OrganismName : HUMAN
<400> PreSequenceString :
ggctgcttcctagaaggtcgtgtcacgtggaacctcttaatctcagcatccggagctccaggaagggaaaatttcaagtcagat
agaattctatatataccatttctttggaaccttcagccctcaagattccaacatcatgacctcagtttcaacacagttgtcctt
agtcctcatgtcactgcttttggtgctgcctgttgtggaagcagtag
<212> Type : DNA
<211> Length : 215
      SequenceName : 5164454H1

SequenceDescription :

Sequence
--------
<213> OrganismName : HUMAN
<400> PreSequenceString :
aaagttttggcaggagcgggagaattctgcggagctgcgggacggcggcggtggcgccgtaggagccgggacagtgttgtacag
tgttttgggcatgcacgtgatactcacacagt
<212> Type : DNA
<211> Length : 116
SequenceName : 5849807H1
SequenceDescription :

Sequence
--------
<213> OrganismName : HUMAN
<400> PreSequenceString :
gagaaccagagtgtcagagcaaaacctcctctatctgcacatcctggggacgaaccgggcagccggagagctgcggccggccca
gtcccgatccggctttgaagggtaaaacccaaggcggggccttggttctggcagaagggacgctatgaccgcagaattcctctc
cctgctttgcctcgggctgtgtctgggctacgaagatgagaaaaagaatgagaaacc
<212> Type : DNA
<211> Length : 225
SequenceName : 656258H1
SequenceDescription :

Sequence
--------
<213> OrganismName : HUMAN
<400> PreSequenceString :
gtcagtgcatgtgacgcccctattctctcctcttccaggtccctagactgcagggagagtggcaagaatgggcctcatgtgtac
caggacctcctgcttagccttgggactacaaacagcacgctgccccctccatttctctccaatctggaatcctgacattgaac
ccagttgctcagggtcagcccattcttacttccctgggatcaaatcaagaagaagcatatgtcaccatgtccagcttctaccaa
aaccagtgaagtgtaagaaacccagactgaacttaccgtgagcgacaaagatgatttaaaagggaagtctagagttcctagtct
ccctcacagcacagagaagacaaaattagcaaaaccccactacacagtctgcaagattctgaaacattgctttgaccactcttc
ctgagttcagtggcactcaacatgagtcaagagcatcctgcttctaacatgtggatttggtcacaaggnttaaggtgacccaat
g
<212> Type : DNA
<211> Length : 505
SequenceName : 736837R6
SequenceDescription :

Sequence
--------
<213> OrganismName : HUMAN
<400> PreSequenceString :
gcgactatatggatgaggtgacatatggggagctggagaaggaggaagctcagcccattgtcactaagtacaaggaggtggcag
aggtggtggcagaggcggtggttttagaggtggaagaggaggtggaggtgggggcttcagaggaggaagaggtggtggtttcag
agggagaggacattaagtgaaacagttgacagacatcaccagttgacttctgcattaacctgcatgatctgtttctactatg
<212> Type : DNA
<211> Length : 250
SequenceName : 767295H1
SequenceDescription :

Sequence
--------
<213> OrganismName : HUMAN
<400> PreSequenceString :
atttgtttagagtatctatagcaaaataggttacatgtagtttgaaataaggtatcctgagttactttggcaacaagttctttt
acccttacccgtggtatttgaaaaaaatcaaggtaactgtctgaatactttaatatcagcttgttttgtgaattctctgaatac
tgtcaacactcttatctaagtttgcctttatgatgcagtggcagcattttgaattacttttcaaagaatactgt
<212> Type : DNA
<211> Length : 242
SequenceName : 778372H1

SequenceDescription :

Sequence
--------
<213> OrganismName : HUMAN
<400> PreSequenceString :
cagagaggtcgtggaggtggatctggcaattttatgggtcgcggagggaactttggaggtggtggaggtaattttggccgtggt
ggaaactttggtggaagaggaggctatggtggtggaggtggtggcagcagaggtagttatggaggaggtgatggtggatataat
ggatttggaggtgatggtggcaactatggcggtggtcctggttatagtagtagagggggctatggtggtggtggaccaggatat
ggaaaccaaggtggtggatatggtggaggtggag
<212> Type : DNA
<211> Length : 286
      SequenceName : 780099H1
      SequenceDescription :

Sequence
--------
<213> OrganismName : HUMAN
<400> PreSequenceString :
tttatcaaataaaactatgaaaatgcaagtgggctcctatgccagaaaaacccacctggcgttccagatgcaagagggccagag
cagaggcctggttctggggaagcctcaggatgctgcccaccaaggagtgatttccaaagagtaatccagggtgcccttttccct
tctggggaagtgtggagaggtagagccccagaggagaatgtaaacaagcagccagcacctctgtataggcccggcctggatcag
agagaggggagaactctgcagggtgtgggattgggctcagggacctccgagtgaggcagggactccctgctggctgctctgcag
cagctgctggaacagggagtggggctggttgcgcagggtggccggggagtccagctctaccactctccccgcttgtagcaccag
cacccggtctgagttcaggatcgtgttgagcctatgggcaatggtcagcactgtcttgttggcaaagcgtttgcagatggtctg
ctggagcagctggtctgtcttctggtccacacttgctgtggccttcatcgatacacaggatcttggcatctgtgaagagaancc
tggccaaacacaacagctgcctctgccccaagaagataa
<212> Type : DNA
<211> Length : 627
      SequenceName : 809631T6
      SequenceDescription :

Sequence
--------
<213> OrganismName : HUMAN
<400> PreSequenceString :
ttccgggaagttcaatagtggtgagttggtggccattatgggtccttccggggccgggaagtccacgctgatgaacatcctggc
tggatacagggagacgggcatgaagggggccgtcctcatcaacggcctgccccgggacctgcgctgcttccggaaggtgtcctg
ct
<212> Type : DNA
<211> Length : 170
      SequenceName : 917064H1
      SequenceDescription :

Sequence
--------
<213> OrganismName : HUMAN
<400> PreSequenceString :
gctgcaccgtgagcgccgaggacaaggcggcggccgagcgctctaagatgatcgacaagaacctgcgggaggacggagagaagg
cggcgcgggaggtgaagttgctgctgttgggtgctggggagtcagggaagagcaccatcgtcaagcaggtgtaggtcattcccg
gggggttgcttattccggggggggattcccgcagtacgcgcggttgtctacagcaacaacatccagtccatcatggccattgtcaa
agccatgggcaacctgcagatcgactttgccgacccct
<212> Type : DNA
<211> Length : 290
      SequenceName : 938765H1
      SequenceDescription :

Sequence
--------
<213> OrganismName : HUMAN
<400> PreSequenceString :
agcctctgnagggtaaacttttatttcttggcctgnacaggnacttccacacccatgcctcctcctgggcaccttttactctgc
ccaagcagtggcagncccagttccaaaaggtgcctgccatggnccaaaggggttggggagtcacttcacattccaggttggagg

agtgagaggacttggggtactcctcccaccccattgccagcaagaggccatgactcacctcaaccccaagtcctgcatcctgg
gtgggaagtgacaatgcctcctcccagctntccatacctaatggggtcatgaccagacagggtaggacccaggcccccagcttc
agttggtaacggtggcctccacggctgcccctgtccaggtgacaaccccagcccccatgatgg
<212> Type : DNA
<211> Length : 400
      SequenceName : G922698
      SequenceDescription :

Sequence
--------
<213> OrganismName : HUMAN
<400> PreSequenceString :
atagagaacccacagttcagtttattcagagcaaagaaaaaagaaactttccatcatactagaagaaacttggccgtaagttt
gggatctgtgctcaaactacacatgcaatgaggacaatattctgctaatacaattgacttgctgctgcatttgtctactgtttg
tctaatattaacaattataaacagagcagtgcaactagtatttggaacaatccttacagtgttacagtgtcaggcacaacattt
cacttctcttcacaccactggttctctttgcgtacctgggcttcctcctcttcagtaaagtcattttttgatattgaaggtcttg
cgaatctcctcaggagttttccccttgatcatattggcaacagtcttgcatgtaacatcaagcaaaccttn
<212> Type : DNA
<211> Length : 407
      SequenceName : AA136533
      SequenceDescription :

Sequence
--------
<213> OrganismName : HUMAN
<400> PreSequenceString :
gtagtcagctatttaattaggttcttaagacatttagaacaccaatttgtgaggataaattccattcgtcagagcaaacacaga
tcgcaggtagccctggagctgaggaatagctttgattttggtaaaatttgtgagtccacagctttctgatcaatcttgcgctg
ctccgtaatctcatatttctcttttctgtgtcgaagatctcaccttcctggtgtctgggcttccgcagcttcttcttcttgaa
gtaagcatcagtaagatgttttgggatttttacattgctgatatcgattttggttgaagtggcaatgacaaatttctggtgtgt
tcttcgtagaggaactcgattgaggaccagaggcgntncaacaagtaataagccactagccagctgcttcaggaaaaccacccct
cttgcccctgtggcgtccagtgaggatgatcagaat
<212> Type : DNA
<211> Length : 456
      SequenceName : AA629808
      SequenceDescription :

Sequence
--------
<213> OrganismName : HUMAN
<400> PreSequenceString :
taatactaagggaaaaggctgttctttatttatttattttcctgagtcctcacgtttttcttctctgacaaatgtttgaaat
tcagtgaaataccaaaggagtcaaggatgaaggggacacaggatggaatcagaaaaaacataaatggaatccaggcagttctat
gagacaacactgatatatctccttgataaagaaaaaatgtacagaatatttaatgagtctgtcttgccccaaaagggaaaaaca
caagtagctaagccatttgcagagaggaaaaatgtcatggaaaaatgaaaaatctctcctaatgtctatagcacatgaaatatc
taagtaggtgggtgcagtggttcatgcctggtattgggagaccaaggcaggcagatcacttgacgtcaggagttcaagaccagc
ctggcccaatggngaaaccccgtctcctattaaaaataccaaatttagctgggcatggtggtgggtgccctgtaaccccancac
ttaggaagcntaggccgggagaatccatgttgaccccggaggcggaggt
<212> Type : DNA
<211> Length : 554
      SequenceName : AA088861
      SequenceDescription :

Sequence
--------
<213> OrganismName : HUMAN
<400> PreSequenceString :
ttttttacatttctcaacttttattgtgggcccttcaaccatttttgctgctgggtttcatattttcggattgtttcacggatc
cacaggacgtatcttgagacacgggtgtagacaccaggccggtcaggttgcccacatggaagtctccccaggagagaatgcca
tacagtgttctgttacagaccagggggcccccagagtcaccctcacaggagtctttgccaccctcttttgtgccggcacacaac
atgttgtcagtgatctttcctgggtagacttgacgacactcctcatctgagcgaagttggatgttggcacaattgtagagtttt
ggggtaattcactcagaccttccctcccatctctgcccctgtttccccagccactcctatggggcctcaggcacctgtgtgggt
gcatacctgggggctggtggtggtgcccagccagacacccgacagtggtgccaggggttaggcggttgttgtgggaaaggggca

gggtttggatgtagc
<212> Type : DNA
<211> Length : 519
      SequenceName : AA401397
      SequenceDescription :

Sequence
--------
<213> OrganismName : HUMAN
<400> PreSequenceString :
catacttaatttttatttgaaattccctattccctctatacaagaacctttgctgaaacagtttccacaaaggcagcagtga
attttcagaacatttacatttttccccctcagcaaaaagataaatcacagtgtaaattatgttgttctgctgtcatctttggc
tggggttagacccagaagttggtgactagcaaaccaatatagccaaatgttgctagtgctccttaggcctttaagctataaact
cagcaaggaatgtctgcattttatctctttaaggtaccaccagggggtatgacagcattaacttccataaacttttatagaca
aatggaaaaaatctacaaaatttgctagtaatattacacagcaatacacactttttatactctacacaaaaccaaaattcttg
gtcttaatggcgagtaacaatttctgtttgagaatctgtttagaggaatagagtgggacgtaaagtcgagaaatgcaagtattt
gcagtaggaagatgttggtcatctggtggtcttattgtgattgagggcataggctggaatccttcttcactggctggcagtgat
gggcttgt
<212> Type : DNA
<211> Length : 596
      SequenceName : AA434064
      SequenceDescription :

Sequence
--------
<213> OrganismName : HUMAN
<400> PreSequenceString :
ttcgtgcagagccagatactttattcatcacttgggcgatttgaaactgccaactccgtccctccctcccccaccccttgattct
agagtgacataagaatggtgataagtctgaactctttcacctcttaaatgccatatcattgcccacagttgccaaatacaaaat
atcaagtcctctgctgaagaagactgaggtcccatcgtttcctgtgttgcaggtcacttcttcaccaggtggctgactgcatca
ttgtgaggcagagcatgctgtgcaagtgtcataagtcatcccactttcaactctcaggaacactcgcagccagtaccacgctgt
cctcgccatccaatattgatag
<212> Type : DNA
<211> Length : 358
      SequenceName : AA488341
      SequenceDescription :

Sequence
--------
<213> OrganismName : HUMAN
<400> PreSequenceString :
ttttagtcataaaaccgattatttaattgaagctataaaaaaggtagtataagtgataaaataattaggaaagaatatttagca
tgtttcaaaacatttaaaataggagcagaacattttacaaaaagttgtacaggaaattaaattcttaaactatcagtacaaaca
tgacattacagagtatcttgggaagtacgggtccggatcggaaaggactatgatgctttaagtctgaaaactattggccaaata
tttaggtttagatttacagttcctgggtatgagaatcatattactatatacatctcccaaaccagtaggtagtattttccaatt
aaccatgtgtggtatcatcttctacaaagtctttggccatctctgctgtgatcacatcaatatgactagccttatttctggact
ttacaccataggatttggcagctgactcaagcagttc
<212> Type : DNA
<211> Length : 457
      SequenceName : AA463411
      SequenceDescription :

Sequence
--------
<213> OrganismName : HUMAN
<400> PreSequenceString :
cgagtctcagaaaataccaggaatgactctatccagttctgtttgtcaagtaaactgttgtgccagagaaacttcacttgcgga
gaacatttggcaggaacaacctcattctaaaggtcccagtgtacctttctccatttttgatgagtttcttctttcagaaaagaa
gaacaaaagtcctcctgcagatcccccacgagttttagctcaacgaagaccccttgcagttctcaaaacctcagaaagcatcac
ctcaaatgaagatgtgtctccagatgtttgtgatgaatttacaggaattgaacccttgagcgaggatgccattatcacaggctt
cagaaatgtaacaatttgtcctaacccagaagacacttgtgactttgccagagcagctcgttttgcatccactccttttcatga
gataatgtccttgaaggatct
<212> Type : DNA

<211> Length : 441
      SequenceName : AA488324
      SequenceDescription :

Sequence
--------
<213> OrganismName : HUMAN
<400> PreSequenceString :
gaccagacaggattaaatttattgaattacagttacaagtggaaaaaatgactgacttactgcatggagaagtattatgtcaa
cctgttaaaagctatagaaattaaaatgttcatgtatatacaaaatataaatgacatgtaaggtaaaattacagtgatcaattt
acaacaaagaaattaacagtagttttccagcttgtttgattaatcaagcaggtttactcttgaatccatagcgtttctacaatt
taatttggacaaagttctattccagatcagccaggttttaaaactgaaatgtgttaagggtggattgttagcttccccaggatt
ggtgtggatgataaatactttttttaaaaattaattattattgtttataaga
<212> Type : DNA
<211> Length : 388
      SequenceName : AA452802
      SequenceDescription :

Sequence
--------
<213> OrganismName : HUMAN
<400> PreSequenceString :
gccgacagagcaagactctgtctcaaaaaaaaaaaaaaatacaattaagaggtgtatactgatgattcttcccactcatttgtt
tgtccagaagtaaataaatgtcggaagttttccaagtaactatttgtttttgggggtttttagttagagtcttggtctgtc
acccaggctggagtacagtggcaggatctcagcttgctgcagcttccatctcccaggttcaagcgatcatgtgcctaagcctcc
tgagtagctgggatcacgggtgtgtaccaccacgcccagctgattttgtatttttagtaaagactttcgccatgttgtccagg
ctggtctcgaactcctggcctaatgatctgcccgcctctgcctcccaaagtgctgggatcacaggcatgagcaccggaaccaag
ctttatccgcttaaatttaacgtgtttttgacttcttaattaacttggtcatataccaaatgtgatctctgtggattaccaca
tgcattaaaaaattttcaagctgggcccagtggcgcacgctgcaatcccacactttgggaggccagagagggagattgctgag
cccagagtt
<212> Type : DNA
<211> Length : 597
      SequenceName : AA430653
      SequenceDescription :

Sequence
--------
<213> OrganismName : HUMAN
<400> PreSequenceString :
ccacagagtaataataaactcattcaacattataaaaatacttttccaactacttttacatgacttaatgtttccaattatttg
cgaaaccatgtccccgcaggtcttagctggtccttgtttaagattcttaatttcattttggcaatatctaaatttttatttaca
tacatgtgtactaaagcattacctatttttatggttaaaacatgaactttagctgggcatggtggctcacatctgtaatcccag
cactttcagagatcaaagcgggaagactgcttgaggccaggagatcaagaccagtctgggcaacatagtgagacctcatcgcta
caaaaaatcttaacaattagctggatgcagtggcacatgcctgtagtcccagctactcgcaggctgaggtgggaggattccttc
agctcaggagctcaaggcagcagtgagctatgatccgtca
<212> Type : DNA
<211> Length : 460
      SequenceName : AA283874
      SequenceDescription :

Sequence
--------
<213> OrganismName : HUMAN
<400> PreSequenceString :
tataaaagcttggggctggggccgagcactggggactttgagggtggccaggccagcgtaggatcctgctgggagcggggaact
gagggaagcgacgccgagaaagcaggcgtaccacggagggagagaaaagctccggaagcccagcagcgcctttacgcacagctg
ccaactggccgctgccgaccgtctccagctcccgaggacgcgcgaccggacaccgggtcctgccacagccgaggacagctcgcc
gctcgccgcagcgaggcccggggcggcccttcaggggacctttcccagatcgcccaggccgcccggatgtgcacgaaaatgga
acagcctttctatcacgacgactcttacgcagcggcgggatacggtcggagccctggcagcctgtctctacacgactacaaact
cctgaaacccaccttggcgctcaacctggcggatccctatcggggtctcaagggtcctggggcgcggggtccaggcccggaggg
cagtggggcaggcagctacttttcgggtcagggatcagacacaggcgcatctctgaagctagcctccacggaactggagcgctt
gatcgtccccaacagcaacggcgtgatcacgacgacgcccacgcctccgggacagtacttttaccccgtgggggtggcagcgg
tggaggtacagggggcggcgtcaccgaggagcaggagggctttgcggacggttttgtcaaagccctggacgacctgcacaagat

gaaccacgtgacgcccccaacgtgtccctgggcgccagcgggggtccccaggccgg[?]ccaggggcgtctatgctggtccgga
gccgcctcccgtctacaccaacctcagcagttactctccagcctctgcaccctctggaggctccgggaccgccgtcgggactgg
gagctcatacccgacggccaccatcagctacctcccacatgcaccaccctttgcgggcggccacccggcacagctgggtttgag
tcgtggcgcttccgcctttaaagaggaaccgcagaccgtaccggaggcacgcagccgcgacgccacgccgcctgtgtcccccat
caacatggaagaccaggagcgcatcaaagtggagcgaaagcggctgcggaacaggctggcggccaccaagtgccggaagcggaa
gctggagcgcatcgcgcgcctggaggacaaggtgaagacactcaaggctgagaacgcggggctgtcgagtgctgccggtctcct
aagggagcaagtggcgcagctcaagcagaaggtcatgacccatgtcagcaacggctgccagttgctgctaggggtcaagggaca
cgccttctgagagcctcccttgccccatacggacacccccagccttgaaggctgggcgcctgccccccactggggtgaggggggg
caggcgatgggcacccgccaaaaggcctggggcgcagctcacacactggactccggcccgcccgcctgcgcccagtccttccac
ctcgaggtttacatggccccttccagcgtattttgtatgtttttttttctgcaaagagactgaattcatattgaatataata
tatttgtgtatttaacagggaggggagaagggggctgtcgcggcggagctggccccgccgcttggtactcagcctgcggggata
ctagggagggacctccgcccctgccctcccctctgcatagtactgtggagaagaaacacgcacttcgtgtctaaagtctatt
ttaagatgtgtttgtgtgtgtgtgtttgacttttattgaatctatttaagta
<212> Type : DNA
<211> Length : 1817
SequenceName : J03236
SequenceDescription :

Sequence
--------
<213> OrganismName : HUMAN
<400> PreSequenceString :
caggactgggtctcctttccagggagaggaagagcccttcatggaaccgtctgcagcggctcagtcagtctcggctgtccagcc
agggtgtttggtggtgaggattcaggctccgtccagacaaggcagtggcctgaggctcagggcccccagcccctccctcccag
tccatcagcgtcactccccagccccgagctggaccgcacaccttgggacacggttttccacttcctcaggacgagccccagact
ggaggagaggtcggaggaggtggcgttggctcttcacgaggaccccggcggcgggcccgggggaggcggccgaagcggcggc
ggccgggagcgacatggccgaggaacaagacctatcagaggtggagctgagccctgtgggctcggaggaaccccgctgcctgtc
cccaggcagcgcgccgtcgctgggacccgacggcggcggcggtggctcgggcttgcgagccagcccggggcccggtgaactggg
caaggtcaagaaggaacagcaggacggcgaggcggacgatgacaagttccccgtgtgcatccgcgaggcggtcagccaggtgct
cagcggctacgactggacgctggtgcccatgcccgtgcgcgtcaacggtgccagcaagagcaagccgcacgtcaagaggcccat
gaacgccttcatggtgtgggcacaggcggcacgcagaaagctagccgaccagtaccctcacctccacaatgctgagctcagcaa
gacactaggcaagctctggaggttgctgaacgaaagtgacaagcgccccttcattgaggaggctgagaggctccggatgcagca
caaaaaggaccatccggactacaagtaccaacctcggcggcggaagaacgggaaggcagcccagggggaggcagaatgcccagg
cggggaagccgagcaaggagggggctgctgctattcaggctcactacaagagtgcccacctggaccaccggcacccagaagaagg
ctcccccatgtcagatgggaacccagagcacccctcaggccagagccatggcccccaacccctccaaccaccccaaagacaga
gctgcagtccggcaaggcagaccccaaaagggatggcgctccttggggggagggcgggaagccccacatcgacttcggcaacgt
ggacatcggggagatcagccacgaggtaatgtccaacatggaaacctttgatgtgactgagctggaccaatacctgccacccaa
cgggcacccaggccatgtgggtagctactcggcagctggctacgggctgggcagtgccctggctgtggccagtggacactctgc
ctggatctccaagccaccaggtgtggctctgcccacggtctcgccccctggtgtggatgccaaagcccaggtgaagacagagac
cacaggcccccagggacccccacactacaccgaccagccgtccacttcccagatcgcctacacttccctcagtctgccccacta
cggctccgccttcccctccatctcacgaccccagtttgactattctgaccatcagccctcaggaccctattatggccatgcagg
ccaggcctctggcctctattcagccttttcttacatggggccctcccagcggcccctctacactgccatctctgaccccagccc
ctcagggccccaatcccacagtcccacacactgggagcagccagtatatacgactctatcccgaccttagagggccctgtcac
caccagtgcccacagggccttggggccccttcccccagcctgtgtgccctgctcctcatcagcctcaggcttggcagtggagga
ggctgcagaggctgacagcagcaggaaggcttctgccagctcctgccctgatgacctccacccaccccggctctggaggccaag
ccctgactgagctggcaaaggaagtttggtggctctcccagactgagggtgaggggccacccttccccagaaatgaccctctat
cccaggacctgagagtcctgctcagtccttcagggaggcaggaagggttagggtaggaggcctcactgctcctgttatctcctt
ggagagcaaggggcccgtgtgctactgtgtcattgcctaagagctaagctgcccagggacctgcctcaaagcctggagctggtt
ctgtcctgtcactaaggacgcactgaggacagctttgaacagctttgtaaagagcagggtgggacactctgatcctttctccgc
cccctcttcacctgaatgtgggaactggtcgaggtccccagatccagttccgtgtcaataacctcatcccttgcctaactgagg
agagtccccatgttcttcccatcccaccccaggtgaggagccaggggctccaggaaagagtcagaggcaatccacctagccttc
ttctcgccttctgtcagcctcaagccccccaggctcagagttagcatggcacggccctagcccctgggttgctggcagcaggctg
gacactaaacccctgccatgtacagtctacctttgccttgcaccctttgctccagcgataccttaataaagtacgtagctctgt
ctccccatctcctgtctgcagcccagtccacatgtaacacatcgctgcccctttatttatctccatagtcgccatgttcctatc
cctctagctactccagccccgattaacttgcattaagcactccaagtaataaaatcaatgcttctggtttaaa
<212> Type : DNA
<211> Length : 2761
SequenceName : AF017182
SequenceDescription :

Sequence
--------
<213> OrganismName : HUMAN

<400> PreSequenceString :
cggccttggatcctggaagagaatcgctaaacacagagcagacccagtaagtgagcaggtgttttggacaatggactggttgag
cccatctctattataaaatgtctcagagcaaccgggagctggtggtcgactttctctcctacaagctttcccagaaaggatac
agctggagtcagtttagtgatgtcgaagagaataggactgaggccccagaagaaactgaagcagagagggagaccccccagtgcc
atcaatggcaacccatcctggcacctggcggatagcccggccgtgaatggagccactggccacagcagcagtttggatgcgcgg
gaggtgattcccatggcagcagtgaagcaagcgctgagagaggcaggcgatgagtttgaactgcggtaccggagagcgttcagt
gatctaacatcccagcttcacataaccccagggaccgcgtatcagagctttgagcaggtagtgaatgaactctttcgggatgga
gtaaactggggtcgcatcgtggcctttttctcctttggcggggcactgtgcgtggaaagcgtagacaaggagatgcaggtattg
gtgagtcggattgcaagttggatggccacctatctgaatgaccacctagagccttggatccaggagaacggcggctgggacact
tttgtggatctctacgggaacaatgcagcagccgagagccggaaaggcaaggagggcttcaaccgctggtttctgacgggcatg
actgtggctggtgtggttctgctgggctcactcttcagtcggaagtgaccagacactgaccgtccactcacctctcacctccca
ccctgcccccaccacaactctctcttcagccaccattgctaccaggagaaccactacatgcaactcacgccccttgccctatca
cagggttggcctagacggagtcccctgcaattagctttctaggatctaccacacttctgtgaaagccaccttcccccacatc
tcagttctcttggcctcaaaactcacaaggttttcccctaaaccagctccttggaggctggcaggaacagagaggggtgtgtag
ggaggagagcctgtcttgttggtgggaccctaattaccccctgagccttctcgggaatgcttttccagcagggagctcttcccct
gaaggagactagcttgccttggttttgatgtgtgtggcctcagacgtgatcatttcccatccgctgtgtccctgggtctccctc
ctccccatctccacccccccaagagccattgagtgaggtgcttttagcccttttgactaattagaaattcaggctgcttgggata
atgagacaaggaccagaacctcctccccacctgtggcctggccaaaacccccacttgtggtctgaatgttctcctgagcgctct
ggctagagtctacgctcacccagggggaggggcttagg
<212> Type : DNA
<211> Length : 1466
      SequenceName : U51278
      SequenceDescription :

Sequence
--------
<213> OrganismName : HUMAN
<400> PreSequenceString :
aagcttcttcccagaacctggaagcccagcaagcaaatgccctgcccccacccgacctccgccctcgttggcttcgcaaagctg
tgatctgtggccagtagagggcacactttactttcgcaaatctgagagccacaacccgggggagggggcggggaaagagtctct
gcagcaaaatgcagcctagggattggtggctcttggcgtttgaggcaaaatcctagaggctgtagtcattttgcaatccttaaa
gctgaattgtgcaatgagctcgatgaaggaagatactatcatccaacagctgaatcctaaattgcaaactcaatggctaatgac
tactttgaacaatgcgcaccttacacacgctacacgctactgtagtctcttctttttttaggggggtagcagtgagaggtttctt
aaaatgccttggggcgaggagtcctgaatgaggagactgctttgggttttgatgcgtaggataagcaaatcccgaggaaatatg
cactatataataaatctagaaccaaaacacagagcaaaagattcatgtttcacgttggttaataaactaggttatcgtgtatat
attaagtgtgtatacctttggggactctacaaaatgcacggcatggtattgaggaaaaagtaaattgggaaattagtgtaaaaa
ttagagtcggcttttaattagtttaacacacacatacgaaggcaaagacacaacgttactttgatctgaccggggccgaccttt
ttttaagtgcataattacgattccagtaataaaaggggaaagtttggggtttgtcctgagaggaaggggttaacggtttccttt
attctagggtctccgcaggctccccagatctgggttggcaattctactcctcccccctttctgggaagtccgggtttcccccaac
ccccaatccatggcatattctcgcgtctagcgccttgattcccgcccccccacacccccaacctctaagccggagtctggtgca
aactggctccacaggggcaaagaggatttgcctttagtggaaactggctgtggaactggaactctgagtggaggtgcatggggt
gtagactggcagagactcgaccgggaggatccgggtagagcgcgcccaccgccgcagtcctactctactccagctctggaacgc
gcagaaagacctacggggcaaggagccgcctccaccccaccccgcctggtggaaggtccgaacggaggtgttggagtggaggg
cctttctggcagcaaattggggggattcattctgtaaagaatgtgcccagtcaacatagctgtacgcccaaacgcaaaatacaca
ctgccctccccgagagatgcagtggctgtttattcctaagtggctctccaagtatacgtggcattgagttgctgagcaatttta
atgattccaggcatcgtttttctgctcagacctcatctgtcgtaggtcttcaactatcactccacactgagcaagggctcctac
ataactcttttttttttttcgtccttccctttttaaattctgtttccccgaccttagagagacgactggccgcccgagacgtgcg
tgacgcggctcagggtacacggcgtattgtgtggagcgaggcagctgttccacctgcggtgactaatacacgcaggacaagaac
acagttcagccgagcgctgcgcccgaacaactgtacagaaagggaaaggaccgacgcgcgagcaggagaaaatggtcggcgcg
cagttaattcatgctgcgctattactgtttacaccccgagtcggagtactgggctgcgggactgaggctcctcctctttccccg
gctccccactagtgcccctcccgagttcccaaagcagagggcgggggagcgagaggaggaaaaaaatagaggtcggagggggag
ggagaaagagattctctggcctaatccccgcccacccgccctttataatccgggggtctgcgcggctaaggacccccggctgc
gctgctctccgctgccgcctccgccgcgcccactccgctcgcctcctgcctccaaaagggcaggcgttcgccgaggcttggcgg
gaaaaagaagcgaggggagggatccggagtcgcagtataaaagaagcttttcgggcgtttttttctgactcgctgtagtaatt
ccagcgagagacagagggagtgagcggcgggttggaagagcccagtgtgcagagccccactccgggcttcctaggaaggcagc
tctggagtgagaagggctttgcctccaggcttgctgcctcctcgacccaatcctcccgctgacccaacatcagcggtcgcaacc
ctcgccgcctctgggaaactttgcccattgcaacggcagacacttctcactggaacttacaatctgcgagccaggacaggact
ccccaggcgcaggggagggaattttgtctatttggggacagtgttctctgcctctgcccgcgatcggctcccctgaaaagagc
tcctcgcgttatttgaagcctgaatttcctttgggaggtggaaaacccggtaagcacagatctgttggtcttctactgttttct
ctacgtcctgagtgtagcgaccggttagaacagtctttattcaatttctgtgcttttgacactttactcagagtagttggggta
ggccggggcggagttggggtggatctgagtcggggtagaacgacttgtcaagatgacaggaaaggggaaggaaaaaaccggggt
gcatttttgaaactgtcttccagaggttaccgtgggctgacgctgacccgggccggttggacattcttgctgtgctacattaatt
gatatttatgtcctttgggggggggtgtcaaagccggaggtctcttcgcggtggccaaaggaagcccttcgtatcctgaggtc

tttgggaaagggattacctttttgcgtgttctccgcaaaaagcgagaaggctccgtagcctctgacttcccagtctttgggaggg
catttaaatttcggcttggtgcatttctgacagcctgagaccaactcgggggtgcgtcccgcccgccaatccccggcggcgatt
gcaaccggtccctgatccttttaagaagttgccattggctttaaaaatagtgatcatagtaaatttaagcctaaccctccgc
ggtattaggacttggtgttggactagcgcactgaggagaggcaaaactggggcagggatgtgacagattcgttgacttggggaa
aaccagagggaatcctcacattcctggttgggatccgcgggtattccccgcgcccctgaattgctagggagactgcggtgagtc
ttgatctgagctgttcggtaacagctgctaccctcggcggggagagggaagacgccctgcaccgggtgctgaatcgctgtgggg
tctctggcgcagtggcgtcgcggtttagagtgtagcagggaggtgtctcttattatttgacacccccctcccctttggagaggct
tgttagagcctgagactgagctctcccctccaagtcagcaataggaaagaaaagccggcaaaggaaggaagggggggcgcgctgg
gggtggagaaagaggagggcggagaggggcggcggcgccggctgggtaagagccgggcgacggcgcgagtagggacccggaccc
ggtcggcggcgcaaagagccggcaccggagggggccgagcaccgcggcgcctctcgcctttctccttcaggtggcgcaaaactt
tgcgcctaggcttttggcagactgtattccctacagtcgcctccctcagcctctgaaggaggccaaggccgatggcgattcctg
ggcgtctgcagtgctaagtccctgctctaaggaggcggggactccgagcagctgctagtccgaccagcgtcactgatagtaggg
agtaaaagagtgcattccccccaaccacacacacacacacacacacacacacacacacacacacacacacacacacacttggaa
gtacagcaggctgaaaggggagtggttcaggattggggtacgcgctgcgccaggtttccgcatcaaccagagctgggtaactct
agacttgcttcccttgctgtgcccctccaacagacagtcacgacgatgcccctcaacgtgagcttcgctaacaggaactatga
cctcgactacgactcggtgcagccctatttcatctgcgacgaggaagagaatttctatcaccagcaacagcagagcgagctgca
gccgcccgcacccagtgaggatatctggaagaaattcgagctgctgcccaccccgcccctgtccccagccgccgctccgggct
ctgctctccgtcctatgtcgcggtcgctacgtccttctccccaagggaggacgatgacggtggcggtggcaacttctccaccgc
cgatcagctggagatgatgaccgagctacttggaggagacatggtgaatcagagcttcatctgcgatcctgacgatgagacctt
catcaagaacatcatcatccaggactgtatgtggagcggcttctcggccgctgccaaactggtctccgagaagctggcctctta
ccaggctgcgcgcaaagacagcaccagcctgagccccgcccgcgggcacagcgtctgctccacctccagcctgtacctgcagga
cctcaccgccgcagcgtccgagtgcatcgacccctcagtggtcttcccctacccgctcaacgacagcagctcgcccaaatcctg
tacctcgtccgattccacggccttctcttcttcctcggactcgctgctgtcctccgagtcctccccacgggccacccctgagcc
cctagtgctgcatgaagagacaccgcccaccaccagcagcgactctggtaagccgccccaacccagcagggtaggaagccaga
ggttggatggacctccttctcattggcattaattcaattggcccccgggctcaccctttctttcccttcggtctaagctcttca
tccctggactcctgtgcttcagatatccctccccttcccccagaccgccctggttgtcagccccacccccacccttcccacccc
tcaggaatttcatttgggtttttaaatcttctggcttatctttcaactcaatccactccctttacccctcctaggcattttaat
tacccagagaagggtgtgaaggaggatagggaactgatcctgagaagagtgaatgaattacctgcttttctttgacttcagaag
aatatttgaatttaatgtctacaagacccaagagaagcaatgacagaagcaggacagcctttagcctcagagccaggcactagt
gaaagatcctgaagagttgaagagctgttttccctccatcccgttttgaacacttcaaagcaaatcctcttcagtttggatttt
cgtacccctccctccccaaatgctcgccccaccactgccaccaccaggacaggacgtttggcaaaaccacaagactttactat
tgcacttccagtaatatagggagttggtaaagtcatagcaagatatttgcagctatcgcctacaggacctgaagggttcttggt
gaagtcccttaaaaatgggaggtgcttgggaatgtgctttgctttgggtgtgtctgaagcctcattaagtcttaggtaagaatt
ggcaacgatgccatatcctggtacatggtaattttctcacctgtgccctaaccctgttctacctttctgggaaacgggaggatg
gtgtctagatctggttcttacttctcaatcctttccaacttggcacttggatagcatcggtcaaaccccccatctgggattcagg
aggcattgctaactaatcttccacttccttccttacagaagaagaacaagatgatgaggaagaaattgatgtggtgtctgtgga
aaagaggcaacccccctgccaagaggtccgagtcagggtcatccccatcaagaggccacagcaaacctccacacagcccactggt
cctcaagaggtgccatgtctctactcaccagcacaattatgcagcacccccctccacaaggaaggactatccagctgccaagag
ggccaagttggacagtggcagggtcctgaaacagatcagcaacaaccgcaaatgctccagccccaggtcctcagacaccgagga
aaacgacaagaggcggacacacaacgtcttggaacgtcagaggagaaacgagctgaagcgtagctttttttgccctgcgcgacca
gatccctgagttggaaaacaacgaaaaggcccccaaggtagttatcctcaaaaaagccaccgcctacatcctgtccgttcaagc
agatgagcacaaactcatctcagaaaaggacttactgaggaaacggcgagaacagttgaaacacaaactcgaacagcttcgaaa
ctctggtgcataaactgaccggaagtgaggaggagctggaatctcgagtgtaaggagaacggttccttctgacagaacttggac
ttcaaaaaatgcatgctcaaagcctaacctcacaaccttggctggggctttgggacttcagccataatgttaactgcctcaaag
ttaaggcataaaagaactttttttatgcttcccatcttctttctttttcctttaacagatttgtatttaattgtttttttaa
aaaaatcttccggtgtacatagggcctttaaatgtaaataactttaataaaacgtttataacagttatacaagattttaagaca
tgtatgataaaccataatttttttatttaaagacctttcatttttaaagttgatttttttctattgttttagaaaaaataa
aataattggaaaaaatataattgagccaactcttaaatggtgtttctttttttttttcctccaatttctcttcctcaactat
agatgaacagaactgaccgtcttcccaggacgggtttacaagggtggaaggggttatcttaaaatggggcgggggagggggggt
gcctcaagacctctaagttcccagtgttatgagatcctttctgtggatgctgataggatgagagttgagaaagggcaaaaggca
agtgaggttagatacaaggaacagggaagcaagaggttaaagatagcagacagggaagagatggcagaatgaatctcctgcagt
t
<212> Type : DNA
<211> Length : 7225
      SequenceName : Y00396
      SequenceDescription :

Sequence
--------
<213> OrganismName : HUMAN
<400> PreSequenceString :
cggccaccgccgtctgggaggtgaggggcgcctctgcccggccgcccctactgggaagtgaggagcccctctgcccagccacca
ccccgtctgggaggtgtgcccaacagctcattgagaacggccaggatgacaatggcggctttgtggaatagaaaggcgggaaa

ggtggggaaaagattgagaaatcggatggttgccgtgtctgtgtagaaagaagtagacatgggagacttttcattttgttctgc
actaagaaaaattcctctgccttgggatcctgttgatctgtgaccttaccccaaccctgtgctctctgaaacatgtgctgtgt
ccactcagggttaaatggattaagggcggtgcaagatgtgctttgttaaacagatgcttgaaggcagcatgctcgttaagagtc
atcaccactccctaatctcaagtaatcagggacacaaacactgcggaaggccgcagggtcctctgcctaggaaa
<212> Type : DNA
<211> Length : 494
      SequenceName : AA426103
      SequenceDescription :

Sequence
--------
<213> OrganismName : HUMAN
<400> PreSequenceString :
cagatggctccataatgacagcttcataatggcagtgggtgagcccctggtgcacatcagggtcactcttctgctgctctggtt
ggggatgttttgtctatttctggccactctcaggccaggccctcccagtatttcacttctccagaagtggtgatccctttgaa
ggtgatcagcaggggcagaggtgcaaaggctcctggatggctctcctatagcctgcggtttgggggacagagatacattgtcca
catgagggtaaataagctgttgtttgctgcacaccttcctgtgttcacctacacagagcagcatgccctgctccaggatcagcc
cttcatccaggatgactggtactaccatggttatgtggaggggtccctgagtccttggttgcccttagtacctgttctgggg
ctttcttggaatgctacagataaatgaccttgtttatgaaatcaagccaattagtgtttctgccacatttgaacacctagtata
taagatagacagtgatgatacacagtttccacctatgagatgtgggttaacagaagagaaaatagcacaccagatggagttgca
attgtcatataatttcactctgaagcaaagttcttttgtgggctggtggacccatcagcggtttgttgagctggtagtggtcgt
ggataatattagatatcttttctctcaaagtaatgcaacaacagtgcagcatgaagtatttaacgttgtcaatatagtggattc
cttctatcatcctttggaggttgatgtaattttgactggaattgatatatggactgcatcaaatccacttcctaccagtggaga
cctagataatgttttagaggacttttctatttggaagaattataaccttaataatcgactacaacatgatgttgcacatctttt
cataaaagacacacaaggcatgaagcttggtgttgcctatgttaaaggaatatgccagaatccttttaatactggagttgatgt
tttgaagacaacaggttggtcgtttttgcaattactttgggccacgagcttggtcataatttgggtatgcaacatgacaccca
gtggtgtgtgtgcgagctacagtggtgcataatgcatgcctatagaaaggtgacaactaaatttagcaactgcagttatgccca
atattgggacagtactatcagtagtggattatgtattcaaccgcctccatatccagggaatatatttagactgaagtactgtgg
gaatctagtggttgaagaaggggaggaatgtgactgtggaaccatacggcagtgtgcaaaagatccctgttgtctgttaaactg
tactctacatcctggggctgcttgtgcttttggaatatgttgcaaagactgcaaatttctgccatcaggaactttatgtagaca
acaagttggtgaatgtgaccttccagagtggtgcaatgggacatcccatcaatgcccagatgatgtgtatgtgcaggacgggat
ctcctgtaatgtgaatgccttctgctatgaaaagacgtgtaataaccatgatatacaatgtaaagagattttggccaagatgc
aaggagtgcatctcagagttgctaccaagaaatcaacacccaaggaaaccgtttcggtcactgtggtattgtaggcacaacata
tgtaaaatgttggacccctgatatcatgtgtgggagggttcagtgtgaaaatgtgggagtaattcccaatctgatagagcattc
tacagtgcagcagtttcacctcaatgacaccacttgctggggcactgattatcatttagggatggctatacctgatattggtga
ggtgaaagatggcacagtatgtggtccagaaaagatctgcatccgtaagaagtgtgccagtatggttcatctgtcacaagcctg
tcagcgtaagacctgcaacatgaggggaatctgcaacaacaaacaacactgtcactgcaaccatgaatgggcacccccatactg
caaggacaaaggctatggaggtagtgctgatagtggcccacctcctaagaacaacatggaaggattaaatgtgatgggaaagtt
gcgttacctgtcactattgtgccttcttcctttggttgctttttattattttgcttacatgtgcttttaagaaacgcacaaa
aagtaaagaagatgaagaaggataagagaaatgggaaaaagaaggagactaaactttatacttcattttaatatccaatttt
taatagaaaaatatgaagccatgtctcactgtttaaataaaacttcatggacatttcatgtcaggattgcaagcattagctatc
acagcaaaggattcctagcctattcttacttactttacagtgtcttaagcaatattaaaggttccttttcccaaaaaaaaaaa
aaaaaa
<212> Type : DNA
<211> Length : 2442
      SequenceName : AF029899
      SequenceDescription :

Sequence
--------
<213> OrganismName : HUMAN
<400> PreSequenceString :
acagggtcgctgagaccagtgcacgagcctcttcggaagatgcatcctggactcttggccgcagaagagaaagcggttggggtc
tcccagggactctttctaaagggtcagtccaccatggtaggggttaacgtgggtccacccgtatagggaaaggaaagaaattgt
atttgacacttcagggggcgggtggcggacgggtctcgctgcctgattggttttgcatttcatcccaattttttttctccac
tctctctccctcttttcagtttctctctctgatcccaggggaggggaaacgggaatagtgagagatcaaaaataaacctcttat
gtcaagccgggaaggaagtataaataagaagggctcggcagcccactcggtgcttcccggggtggggaggaggcaggaggcggc
tgcgcggggctggcgcgggcaggggcggaggacgggcgcacgcgggccggggccagctgctcgcgctcggctcgggggcggcgg
cgcgggaggagcgggctgcgccagccagaggggccgccgggggagcgctcccgccgccgggccccgcgggcggagtcggccttg
cacccgattgattccgttttcctggagagagcggccgggccgcgctcgggccgccagcacctgcccgcgcctggcgggtttttc
cttttcctgcgcgcagccaccgccgcagccccatgttccccgcccccgccgcccccggtggcttccgttcctgctgctgctg
ctgctgctgctgctgccgctggcccgcggcgccccggcccggcccgcagccgggggcaggcctcggagctggtggtgcccacg
cggttgcccggcagcgcgggcgagctcgcgctccacctgtccgccttcggcaagggcttcgtgttgcgcctggcgcccgacgac

agcttcctggcgcccgagttcaagatcgagcgcctcgggggctccggccgggcgaccgggggcgagcggggctgcgcggctgt
ttttttttccggcaccgtgaatggggagcccgagtcgctggcggcggtcagcctgtgccgcgggctgagcggctccttcctgctg
gacggcgaggagttcaccatccagccgcagggcgcgggggctccctggctcagccgcaccgcctgcagcgctggggtcccgcc
ggagcccgcccctcccgcgaggacccgagtggaggtggagacgggagagggtcagaggcaggagagaggagaccaccaggag
gacagcgaggaggagagccaagaagaggaggcagaaggcgctagcgagccgccaccgcccctgggggccacgagtaggaccaag
cggtttgtgtctgaggcgcgcttcgtggagacgctgctggtggccgatgcgtccatggctgccttctacgggccgacctgcag
aaccacatcctgacgttaatgtctgtggcagcccgaatctacaagcaccccagcatcaagaattccatcaacctgatggtggta
aaagtgctgatcgtagaagatgaaaaatggggcccagaggtgtccgacaatgggggcttacactgcgtaacttctgcaactgg
cagcggcgtttcaaccagcccagcgaccgccacccagagcactacgacacggccatcctgctcaccagacagaacttctgtggg
caggaggggctgtgtgacaccctgggtgtggcagacatcgggaccatttgtgaccccaacaaaagctgctccgtgatcgaggat
gaggggctccaggcggcccacaccctggcccatgaactagggcacgtcctcagcatgccccacgacgactccaagccctgcaca
cggctcttcggcccatgggcaagcaccacgtgatggcaccgctgttcgtccacctgaaccagacgctgccctggtcccccctgc
agcgccatgtatctcacagagcttctggacggcgggcacggagactgtctcctggatgcccctggtgcggccctgcccctcccc
acaggcctcccgggccgcatggccctgtaccagctggaccagcagtgcaggcagatctttgggccggatttccgccactgcccc
aacacctctgctcaggacgtctgcgcccagctttggtgccacactgatggggctgagcccctgtgccacacgaagaatggcagc
ctgccctgggctgacggcacgccgtgcgggcctgggcacctctgctcagaaggcagctgtctacctgaggaggaagtggagagg
cccaagcccgtggtagatggaggctgggcaccgtggggaccctggggagaatgttctcggacctgtggaggaggagtacagttt
tcacaccgtgagtgcaaggaccccgagcctcagaatggaggaagatactgcctgggtcggagagccaagtaccagtcatgccac
acggaggaatgcccccctgacgggaaaagcttcagggagcagcagtgtgagaagtataatgcctacaattacactgacatggac
gggaatctcctgcagtgggtccccaagtatgctggggtgtcccccgggaccgctgcaagttgttctgccgagcccgggggagg
agcgagttcaaagtgttcgaggccaaggtgattgatggcaccctgtgtgggccagaaacactggccatctgtgtccgtggccag
tgtgtcaaggccggctgtgaccatgtggtggactcgcctcggaagctggacaaatgcggggtgtgtgggggcaaaggcaactcc
tgcaggaaggtctccgggtccctcacccccaccaattatggctacaatgacattgtcaccatcccagctggtgccactaatatt
gacgtgaagcagcggagccacccgggtgtgcagaacgatgggaactacctggcgctgaagacggctgatgggcagtacctgctc
aacggcaacctggccatctctgccatagagcaggacatcttggtgaaggggaccatcctgaagtacagcggctccatcgccacc
ctggagcgcctgcagagcttccggcccttgccagagcctctgacagtgcagctcctgacagtcctggcgaggtcttcccccca
aaagtcaaatacaccttctttgttcctaatgacgtggactttagcatgcagagcagcaaagagagagcaaccaccaacatcatc
cagccgctgctccacgcacagtgggtgctggggactggtctgagtgctctagcacctgcggggccggctggcagaggcgaact
gtagagtgcagggacccctccggccaggcctctgccacctgcaacaaggctctgaaacccgaggatgccaagccctgcgaaagc
cagctgtgcccccctgtgattcagggggcaggggccagtcttgtgctcctggacatgcggtactgaggtgcagacaaggtctcc
actgtggtgactgggtcccttggccatatcaaggcagcacggcccacccaggcctcccattgccgcaacccctccagtactgca
caaattcctaaggggaagagaaaaggtatggggcggcaaaacctatcatcaactgtccawtgnaatggaacttgctcgggttc
aattaaaggcataagttaaagtaaattcattatgatcaacagacctcacntcatctgttgcangatacaactantaaaaaaaa
aaaaaaaaaaaaaaa
<212> Type : DNA
<211> Length : 3711
      SequenceName : AF060153
      SequenceDescription :

Sequence
--------
<213> OrganismName : HUMAN
<400> PreSequenceString :
tctggagccaagtaatggtgatactgatgcttcctttctttgccgcgctcggattctgagtttcacaagaatgtacctgggtg
ccccttagcgggatatgaatagcttcttcggaaccccagcggccagctggtgcctcctggaaagtgacgtctcatctgcaccgg
acaaggaggcggggagggagcgcagagcactcagcgtccagcagcgcggcgggccagcctggagcggaagcctggagtggagca
ggcagtccgcgggggacagacgtcggctgggattgagccggcagactgcgaaaagtagctggagccggagcagggacagaacct
gttgctgcagacgggcttggtggattctggttcctgccgccgacagggctcgccgggagaggttcatcatgaatgagaaatggg
acacaaactcttcagaaaactggcatcccatctggaatgtcaatgacacaaagcatcatctgtactcagatattaatattacct
atgtgaactactatcttcaccagcctcaagtggcagcaatcttcattatttcctactttctgatcttctttttgtgcatgatgg
gaaatactgtggtttgctttattgtaatgaggaacaaacatatgcacacagtcactaatctcttcatcttaaacctggccataa
gtgatttactagttggcatattctgcatgcctataacactgctggacaatattatagcaggatggccatttggaaacacgatgt
gcaagatcagtggattggtccagggaatatctgtcgcagcttcagtctttacgttagttgcaattgctgtagataggttccagt
gtgtggtctaccctttttaaaccaaagctcactatcaagacagcgtttgtcattattatgatcatctgggtcctagccatcacca
ttatgtctccatctgcagtaatgttacatgtgcaagaagaaaaatattaccgagtgagactcaactcccagaataaaaccagtc
cagtctactggtgccgggaagactggccaaatcaggaaatgaggaagatctacaccactgtgctgtttgccaacatctacctgg
ctcccctctccctcattgtcatcatgtatggaaggattggaatttcactcttcagggctgcagttcctcacacaggcaggaaga
accaggagcagtggcacgtggtgtccaggaaaaagcagaagatcattaagatgctcctgattgtggccctgctttttattctct
catggctgcccctgtggactctaatgatgctctcagactacgctgacctttctccaaatgaactgcagatcatcaacatctaca
tctaccctttttgcacactggctggcattcggcaacagcagtgtcaatcccatcatttatggtttcttcaacgagaatttccgcc
gtggtttccaagaagctttccagctccagctctgccaaaaaagagcaaagcctatggaagcttataccctaaaagctaaaagcc
atgtgctcataaacacatctaatcagcttgtccaggaatctacatttcaaaaccctcatggggaaaccttgctttataggaaaa
gtgctgaaaaaccccaacaggaattagtgatggaagaattaaaagaaactactaacagcagtgagatttaaaaagagctagtgt

gataatcctaactctactacgcattatatatttaaatccattgctttttgtggctttgcacttcaaatttttcaaagaatgttc
taaataaaacatttactgaaagccctctctggcaaaaaattaaaaataaacaaaaatggtcataagatcataaacaatcttat
gttgtataaaaatacgtagagtgacttagacatgtttgcatgaataaatatatttctagagaacagttaaaaaaaaaaaaaa
aaaa
<212> Type : DNA
<211> Length : 1936
      SequenceName : AF119815
      SequenceDescription :

Sequence
--------
<213> OrganismName : HUMAN
<400> PreSequenceString :
agcttgtttggccgttttagggtttgttggaatttttttcgtctatgtacttgtgaattatttcacgtttgccattaccggt
tctccatagggtgatgttcattagcagtggtgataggttaattttcaccatctcttatgcggttgaatagtcacctctgaacca
ctttttcctccagtaactcctctttcttcggaccttctgcagccaacctgaaagaataacaaggaggtggctggaaacttgttt
taaggaaccgcctgtccttcccccgctggaaaccttgcacctcggacgctcctgctcctgcccccacctgacccccgccctcgt
tgacatccaggcgcgatgatctctgctgccagtagagggcacacttactttactttcgcaaacctgaacgcgggtgctgcccag
agagggggcggagggaaagacgctttgcagcaaaatccagcatagcgattggttgctccccgcgtttgcggcaaaggcctggag
gcaggagtaatttgcaatccttaaagctgaattgtgcagtgcatcggatttggaagctactatattcacttaacacttgaacgc
tgagctgcaaactcaacgggtaataacccatcttgaacagcgtacatgctatacacgcacccctttcccccgaattgtttctc
ttttggaggtggtggagggagagaaaagtttacttaaaatgcctttgggtgagggaccaaggatgagaagaatgtttttgttt
ttcatgccgtggaataacacaaaataaaaaatcccgagggaatatacattatatattaaatatagatcatttcagggagcaaac
aaatcatgtgtggggctgggcaactagctaagtcgaagcgtaaataaaatgtgaatacacgtttgcgggttacatacagtgcac
tttcactagtattcagaaaaaattgtgagtcagtgaactaggaaattaatgcctggaaggcagccaaattttaattaactcaag
actccccccccccaaaaaaaggcacggaagtaatactcctctcctcttctttgatcagaatcgatgcatttttttgtgcatgac
cgcatttccaataataaaaggggaaagaggacctggaaaggaattaaacgtccggtttgtccggggaggaaagagttaacggtt
tttttcacaagggtctctgctgactcccccggctcggtccacaagctctccacttgcccctttaggaagtccggtcccgcggt
tcgggtacccccctgcccctcccatattctcccgtctagcacctttgatttctcccaaacccggcagcccgagactgttgcaaac
cggcgccacagggcgcaaaggggatttgtctcttctgaaacctggctgagaaattgggaactccgtgtgggaggcgtgggggtg
ggacggtggggtacagactggcagagagcaggcaacctccctctcgccctagtccagctctggaacaggcagacacatctcagg
gctaaacagacgcctcccgcacggggccccacggaagcctgagcaggcggggcaggaggggcggtatctgctgctttggcagca
aattgggggactcagtctgggtggaaggtatccaatccagatagctgtgcatacataatgcataatacatgactcccccccaaca
aatgcaatgggagtttattcataacgcgctctccaagtatacgtggcaatgcgttgctgggttattttaatcattctaggcatc
gttttcctccttatgcctctatcattcctccctatctacactaacatcccacgctctgaacgcgcgcccattaatacccttctt
tcctccactctccctgggactcttgatcaaagcgcggccctttccccagccttagcgaggcgccctgcagcctggtacgcgcgt
ggcgtggcggtgggcgcgcagtgcgttctcggtgtggagggcagctgttccgcctgcgatgatttatactcacaggacaaggat
gcggtttgtcaaacaatactgctacggaggagcagcagagaaagggagagggtttgagagggagcaaaagaaaatggtaggcgc
gcgtagttaattcatgcggctctcttactctgtttacatcctagagctagagtgctcggctgcccggctgagtctcctccccac
cttccccaccctccccaccctccccataagcgcccctcccgggttcccaaagcagagggcgtgggggaaaagaaaaaagatcct
ctctcgctaatctccgcccaccggccctttataatgcgagggtctggacggctgaggacccccgagctgtgctgctcgcggccg
ccaccgccgggccccggccgtccctggctcccctcctgcctcgagaagggcagggcttctcagaggcttggcgggaaaaagaac
ggagggagggatcgcgctgagtataaaagccggttttcggggctttatctaactcgctgtagtaattccagtgagaggcagagg
gagcgagcgggcggccggctagggtggaagagccgggcgagcagagctgcgctgcgggcgtcctgggaagggagatccggagcg
aataggggggcttcgcctctggcccagacctcccgctgatcccccaggcagcggtccgcaacccttgccgcatccacgaaacttt
gcccatagcagcgggcggacactttgcactggaactgacaacacccgaccaaggacgcgactctcccgacgcggggaggctatt
ctgcccatttggggacacttccccgccgctgccaggacccgcttctctgaaaggctctccttgcagctgcttagatactgaatt
tttttcgggaagtggaaaaccaggtaagcatcgaagtccacttgtcttttaatttactttttatcagtttaattctgagatga
gtcgaatgcctaaatagggtgtcttttctcccaatcctgcgttattgacacctttctcggggtgggggttattccagaattgga
tcggggtacagtgacttgtcaagatgggggaggagaagacagagggaaaacggaatggttttacgattatcctttcgagatt
tctgccttatgaatatattcacgtcgactcccggccggtcggacattcctactttattgtgttaattgttctctgggttttggg
gggcgggggggttgctttgcggtgggcagaaagtcccttgcatcttgagctccttggagaagggaccgcatatcgcctgtgtgag
ccagatcgctccgcaggcgctgacttgtccccgtctccgggagggcagttaaatctcgactcaccgcatttctgacagccggag
acggacactgcggcgcgtcccgcccgcctgtccccgcggcgattccaacccgccctgattcttttaagaagttgacatttggct
ttttaaaaagcaataaaacaatttaaaacctgggtctctagaagtgttaggacgtggtgttgggtaggcgcaggcaggggaaaa
gggaggcgaggatgtgtccgattctcctgcaatcgttgacttggaaaaaccagggcgaatctccgcacccagtcctgactcccc
tgccgcggccgccctcgggtgtcctcgcgcccgagatgcggaggaactgcgaggagcggggctctgggcggttccaaaacagct
gctacccttggtggggtggctccgggggaggtatcgcagcggggtctctggcgcagttgcatctccgtattgagtgcgaaggga
ggtgcccctattattatttgacacccccccttgtatttatggaggggtgttaaagtccgcggctgagctcgccactccagccggc
gagagaaagaagaaaagctggcaaaaggagtgttggacgggggcagtactggggggtggggacgggggcggtggagagggaaggt
tgggaggggctgcggtgccggcgggggtaggagagcgcctagggcgcgagtgggaacagccgcagcggaggggccccggcgcgg
agcggggttcacgcagccgctttcgcccaggcgcctttcgccttctccttcaggtggcgcaaaactttgtgccttggatttgg
caaattgtattcctcaccgccacctcccgcggcttcttaagggcgccagggccgatttcgattcctctgccgctgcggggccga

ctcccgggctttgcgctccgggctcccgggggagcgggggctcggcgggtaccaagacgctggttcactaagtgcgtctccgag
atagcaggggactgtccaaaggggggtgaaagggtgctccctttattccccccaccaagaccacccagccgctttaggggatagct
ctgcaaggggagaggttcgggactgtggcgcgcactgcgcgctgcgccaggtttccgcaccaagacccctttaactcaagactg
cctcccgctttgtgtgccccgctccagcagcctcccgcgacgatgcccctcaacgttagcttcaccaacaggaactatgacctc
gactacgactcggtgcagccgtatttctactgcgacgaggaggagaacttctaccagcagcagcagcagagcgaactgcagccc
ccggcgcccagcgaggatatctggaagaaattcgagctgctgcccaccccgcccctgtcccctagccgccgctccgggctctgc
tcgccctcctacgttgcggtcacacccttctcccttcggggagacaacgacggcggtggcgggagcttctccacggccgaccag
ctggagatggtgaccgagctgctgggaggagacatggtgaaccagagtttcatctgcgacccggacgacgagaccttcatcaaa
aacatcatcatccaggactgtatgtggagcggcttctcggccgccgccaagctcgtctcagagaagctggcctcctaccaggct
gcgcgcaaagacagcggcagcccgaaccccgcccgcggccacagcgtctgctccacctccagcttgtacctgcaggatctgagc
gccgccgcctcagagtgcatcgacccctcggtggtcttcccctaccctctcaacgacagcagctcgcccaagtcctgcgcctcg
caagactccagcgccttctctccgtcctcggattctctgctctcctcgacggagtcctccccgcagggcagccccgagcccctg
gtgctccatgaggagacaccgcccaccaccagcagcgactctggtaagcgaagcccgcccaggcctgtcaaaagtgggcggctg
gatacctttcccattttcattggcagcttatttaacggccactcttattaggaaggagagatagcagatctggagagatttgg
gagctcatcacctctgaaaccttgggctttagcgtttcctcccatcccttccccttagactgcccatgtttgcagccccccctcc
ccgtttgtctcccacccctcaggaatttcatttaggttttaaaccttctggcttatcttacaactcaatccacttcttcttac
ctcccgttaacattttaattgccctggggcggggtggcagggagtgtatgaatgaggataagagaggattgatctctgagagtg
aatgaattgcttccctcttaacttccgagaagtggtgggatttaatgaactatctacaaaaatgaggggctgtgtttagaggct
aggcagggcctgcctgagtgcgggagccagtgaactgcctcaagagtgggtgggctgaggagctgggatcttctcagcctattt
tgaacactgaaaagcaaatccttgccaaagttggactttttttttcttttattccttccccgccctcttggacttttggcaaa
actgcaattttttttttttttatttttcatttccagtaaaatagggagttgctaaagtcataccaagcaatttgcagctatcat
ttgcaacacctgaagtgttcttggtaaagtccctcaaaaataggaggtgcttgggaatgtgctttgctttgggtgtgtccaaag
cctcattaagtcttaggtaagaattggcatcaatgtcctatcctgggaagttgcacttttcttgtccatgccataacccagctg
tctttccctttatgagactcttaccttcatggtgagaggagtaagggtggctggctagattggttctttttttttttttttcct
tttttaagacggagtctcactctgtcactaggctggagtgcagtggcgcaatcaacctccaacccccctggttcaagagattctc
ctgcctcagcctcccaagtagctgggactacaggtgcacaccaccatgccaggctaattttgtaatttagtagagatggggt
ttcatcgtgttggccaggatggtctctcctgacctcacgatccgcccacctcggcctcccaaagtgctgggattacaggtgtga
gccagggcaccaggcttagatgtggctctttggggagataatttgtccagagacctttctaacgtattcatgccttgtatttg
tacagcattaatctggtaattgattatttaatgtaaccttgctaaaggagtgatttctatttcctttcttaaagaggaggaac
aagaagatgaggaagaaatcgatgttgtttctgtggaaaagaggcaggctcctggcaaaaggtcagagtctggatcaccttctg
ctggaggccacagcaaacctcctcacagcccactggtcctcaagaggtgccacgtctccacacatcagcacaactacgcagcgc
ctcccctccactcggaaggactatcctgctgccaagagggtcaagttggacagtgtcagagtcctgagacagatcagcaacaacc
gaaaatgcaccagccccaggtcctcggacaccgaggagaatgtcaagaggcgaacacacaacgtcttggagcgccagaggagga
acgagctaaaacggagcttttttgccctgcgtgaccagatcccggagttggaaaacaatgaaaaggcccccaaggtagttatcc
ttaaaaaagccacagcatacatcctgtccgtccaagcagaggagcaaaagctcatttctgaagaggacttgttgcggaaacgac
gagaacagttgaaacacaaacttgaacagctacggaactcttgtgcgtaaggaaaagtaaggaaaacgattccttctaacagaa
atgtcctgagcaatcgcctatgaacttgtttcaaatgcatgatcaaatgcaacctcacaaccttggctgagtcttgagactgaa
agatttagccataatgtaaactgcctcaaattggactttgggcataaaagaactttttttatgcttaccatcttttttttttctt
taacagatttgtatttaagaattgtttttaaaaaatttaagatttacacaatgtttctctgtaaatattgccattaaatgtaa
ataactttaataaaacgtttatagcagttacacagaatttcaatcctagtatatagtacctagtattataggtactataaaccc
taatttttttatttaagtacatttgcttttaaagttgatttttttctattgttttagaaaaaataaaataactggcaaat
atatcattgagccaaatcttaagttgtgaatgttttgtttcgtttcttccccctcccaaccaccaccatccctgtttgttttca
tcaattgccccttcagagggtggtcttaagaaaggcaagagttttcctctgttgaaatgggtctgggggccttaaggtctttaa
gttcttggaggttctaagatgcttcctggagactatgataacagccagagttgacagttagaaggaatggcagaaggcaggtga
gaaggtgagaggtaggcaaaggagatacaagaggtcaaaggtagcagttaagtacacaaagaggcataaggactggggagttgg
gaggaaggtgaggaagaaactcctgttactttagttaaccagtgccagtcccctgctcactccaaacccaggaatt
<212> Type : DNA
<211> Length : 8056
      SequenceName : D10493
      SequenceDescription :

Sequence
--------
<213> OrganismName : HUMAN
<400> PreSequenceString :
ctagcactctgacctagcagtcaacatgaaggctctcattgttctggggcttgtcctcctttctgttacggtccagggcaaggt
ctttgaaaggtgtgagttggccagaactctgaaaagattgggaatggatggctacaggggaatcagcctagcaaactggatgtg
tttggccaaatgggagagtggttacaacacacgagctacaaactacaatgctggagacagaagcactgattatgggatatttca
gatcaatagccgctactggtgtaatgatggcaaaaccccaggagcagttaatgcctgtcatttatcctgcagtgctttgctgca
agataacatcgctgatgctgtagcttgtgcaaagagggttgtccgtgatccacaaggcattagagcatgggtggcatggagaaa
tcgttgtcaaaacagagatgtccgtcagtatgttcaaggttgtggagtgtaactccagaattttccttcttcagctcattttgt
ctctctcacattaagggagtaggaattaagtgaaaggtcacactaccattatttccccttcaaacaaataatatttttacagaa
gcaggagcaaaatatggcctttcttctaagagatataatgttcactaatgtggttattttacattaagcctacaacatttttca

gtttgcaaatagaactaatactggtgaaaatttacctaaaaccttggttatcaaatacatctccagtacattccgttcttttt
tttttgagacagtctcgctctgtcgcccaggctggagtgcagtggcgcaatctcggctcactgcaacctccacctcccgggtt
cacgccattctcctgcctcagcctcccgagtagctgggattacgggcgcccgccaccacgcccggctaatttttgtattttta
gtagagacagggtttcaccgtgttagccaggatggtctcgatctcctgaccttgtgatccacccacctcggcctcccaaagtgc
tgggattacaggcgtgagccactgcgcccggccacattcagttcttatcaaagaataacccagacttaatcttgaatgatacg
attatgcccaatattaagtaaaaaatataagaaaaggttatcttaaatagatcttaggcaaaataccagctgatgaaggcatct
gatgccttcatctgttcagtcatctccaaaaacagtaaaaataaccacttttgttgggcaatatgaaatttttaaaggagtag
aataccaaatgatagaaacagactgcctgaattgagaatttgatttcttaaagtgtgtttctttctaaattgctgttccttaa
tttgattaatttaattcatgtattatgattaaatctgaggcagatgagcttacaagtattgaaataattactaattaatcacaa
atgtgaagttatgcatgatgtaaaaaatacaaacattctaattaaaggctttgcaacac
<212> Type : DNA
<211> Length : 1487
      SequenceName : J03801
      SequenceDescription :

Sequence
--------
<213> OrganismName : HUMAN
<400> PreSequenceString :
gttgtgctgtcagtgcaccagagacacaaggcttgaagctctgtcctgggaaaatatggcaaacagtgcctctcctgaacagaa
tcaaaatcactgttcagccatcaacaacagcatcccactgatgcagggcaacctccccactctgaccttgtctggaaagatccg
agtgacggttactttcttcctttttctgctctctgcgacctttaatgcttctttcttgttgaaacttcagaagtggacacagaa
gaaagagaaagggaaaaagctctcaagaatgaagctgctcttaaaacatctgaccttagccaacctgttggagactctgattgt
catgccactggatgggatgtggaacattacagtccaatggtatgctggagagttactctgcaaagttctcagttatctaaagct
ttctccatgtatgccccagccttcatgatggtggtgatcagcctggaccgctccctggctatcacgaggcccctagctttgaa
aagcaacagcaaagtcggacagtccatggttggcctggcctggatcctcagtagtgtctttgcaggaccacagttatacatctt
caggatgattcatctagcagacagctctggacagacaaaagtttctctcaatgtgtaacacactgcagtttttcacaatggtg
gcatcaagcatttataactttttcaccttcagctgcctcttcatcatccctcttttcatcatgctgatctgcaatgcaaaaat
catcttcaccctgacacgggtccttcatcaggaccccccacgaactacaactgaatcagtccaagaacaatataccaagagcacg
gctgaagactctaaaaatgacggttgcatttgccacttcatttactgtctgctggactccctactatgtcctaggaatttggta
ttggtttgatcctgaaatgttaaacaggttgtcagacccagtaaatcacttcttctttctctttgccttttaaacccatgctt
tgatccacttatctatggatatttttctctgtgattgatagactacacaagaagtcatatgaagaagggtaaggtaatgaatct
ctccatctgggaatgattaacacaaatgttggagcatgtttacatacaaacaaagtaggatttacacttaagttatcattcttt
tagaaactcagtcttcagagcctcaattattaaggaaaagctcttcaggaaaaatactaaaatatttctcttcctcataagctt
ctaaattaatctctgccttttctgacctcatataacacattatgtaggtttcttatcactttctctttgcataataatgtacta
atatttaaaataccttcagcctaaggcacaaggatgccaaaaaaacaaaggtgagaaaccacaacacaggtctaaactcagcat
gctttggtgagtttttctccaaaaggggcatattagcaattagagttgtatgctatataatacatagagcacagagccctttgc
ccataatatcaactttccctcctatagtt
<212> Type : DNA
<211> Length : 1541
      SequenceName : L03380
      SequenceDescription :

Sequence
--------
<213> OrganismName : HUMAN
<400> PreSequenceString :
ggggacttcttgaacttgcagggagaataacttgcgcaccccactttgcgccggtgcctttgccccagcggagcctgcttcgcc
atctccgagccccaccgcccctccactcctcggccttgcccgacactgagacgctgttcccagcgtgaaaagagagactgcgcg
gccggcacccgggagaaggaggaggcaaagaaaaggaacggacattcggtccttgcgccaggtcctttgaccagagtttttcca
tgtggacgctctttcaatggacgtgtccccgcgtgcttcttagacggactgcggtctcctaaaggtcgaccatggtggccggga
cccgctgtcttctagcgttgctgcttccccaggtcctcctgggcggcgcggctggcctcgttccggagctgggccgcaggaagt
tcgcggcggcgtcgtcgggccgcccctcatcccagccctctgacgaggtcctgagcgagttcgagttgcggctgctcagcatgt
tcggcctgaaacagagacccacccccagcagggacgccgtggtgccccccctacatgctagacctgtatcgcaggcactcaggtc
agccgggctcacccgccccagaccaccggttggagagggcagccagccgagccaacactgtgcgcagcttccaccatgaagaat
ctttggaagaactaccagaaacgagtgggaaaacaacccggagattcttctttaatttaagttctatccccacggaggagttta
tcacctcagcagagcttcaggttttccgagaacagatgcaagatgctttaggaaacaatagcagtttccatcaccgaattaata
tttatgaaatcataaaacctgcaacagccaactcgaaattccccgtgaccagacttttggacaccaggttggtgaatcagaatg
caagcaggtgggaaagttttgatgtcacccccgctgtgatgcggtggactgcacagggacacgccaaccatggattcgtggtgg
aagtggcccacttggaggagaaacaaggtgtctccaagagacatgttaggataagcaggtctttgcaccaagatgaacacagct
ggtcacagataaggccattgctagtaacttttggccatgatggaaaagggcatcctctccacaaaagagaaaaacgtcaagcca
aacacaaacagcggaaacgccttaagtccagctgtaagagacaccctttgtacgtggacttcagtgacgtggggtggaatgact
ggattgtggctcccccggggtatcacgccttttactgccacggagaatgcccttttcctctggctgatcatctgaactccacta

atcatgccattgttcagacgttggtcaactctgttaactctaagattcctaaggcatgctgtgtcccgacagaactcagtgcta
tctcgatgctgtaccttgacgagaatgaaaaggttgtattaaagaactatcaggacatggttgtggagggttgtgggtgtcgct
agtacagcaaaattaaatacataaatatatatata
<212> Type : DNA
<211> Length : 1547
      SequenceName : M22489
      SequenceDescription :

Sequence
--------
<213> OrganismName : HUMAN
<400> PreSequenceString :
gaattccggacccggtaggagggggcggggccgggggagaaggcgccgcctccgccccttcgggacccggagggtgcccttgtt
tacctcccagtatttgtcacccgagacaaagagggaaggaaggaggagccttctgagggaaactccctcggcgggaagcatctt
actttcagaacgtttttaacaccctacggacgcgggaacggttccctcccggcccccgcgccctttccgaggttccctgcct
tgacttccccgagttctacgacaacctcagcgacagcgggaggagctgggcaatcggttttgcagggcaaactccaggctctcc
tcatttattattctctgggcggcgcacgggagaccctgcagggcaacagagcagagcgactacagctcccaggagccaacgctg
cagggctgagccgacgcggggacagacaggacctaaatggtggtcggaggaagggcaaggcagacctcttctgttaccttagc
gccagcaaaccgagccgccgccgtcgccaagccaggagaccaacccccagtctcccgactaagtatttttaaatctggcggggc
tttccttcccgagccaaccactcccaccgtttacagccccaaccagtcagcgtgaggcccgccattttcaaaccctcttcccg
ccgccaatcaggatcgagcagtacattcctctcctgaccggttctaacgggtctgggagttaacgacctgggcgacccaccgaa
cctgctaggctggggcgtggagggcgggtctggtaagacactgaccaatcgttagcctccgtggcaaggggggcggggactatct
gggtttgaataatcgtcagaaccaatcagattagtggggatgtggcccgtggcctagctcgtcaagttgccgtggcgcggagaa
ctctgcaaaacaagaggctgaggattgcgttagagataaaccagttcacgccggagccccgtgagggaagcgtctccgttgggt
ccggccgctctgcgggactctgaggaaaagctcgcaccaggcaagaataccctccaataccctcgggttcgtggacctgccttt
ccccattccctcagagcctctactcggtctcggcgcagtggctctcggggtctgacccggcgagcggcatttggggtgcgggcc
ggcgagggctgggtctgtggagggccggcgggcagtcggaggaggcggaaactgcccctgaccgggcccggttctgggagtttt
caatgtcggtcacgaggtgcttagcggttaagtgaggctcgcgacgtgggagctgggggacatttctgtcaggaaaggcgtttt
gagggcgcttgcggagtcaccggactcggtgaagggtgggcggcggcgtctcgggcccccgggagccacttgctggctgagccg
cggccgctcgggccgggaggaggaggaggaggagggtgtgcgccggggcgcgggcgggggcgcggcggccgcccttgggaggac
gccctcgggaggagggggcgcgggcagggcgggagcggatttgggcgggaagcggagccccgccagcgcccgccctggcagct
gcggcgtccgcgccgacctccggcttcccctctccccctcggccccgtcaggtggacgcggatctgtcaacatgggtaaagga
gaccccaacaagccgcggggcaaaatgtcctcgtacgccttcttcgtgcagacctgccgggaagagcacaagaagaaacacccg
gactcttccgtcaatttcgcggaattctccaagaagtgttcggagagatggaaggtgaggcaggagagggacggagctcagggg
gtgcaggtgtggttttcggctaggagggccttaggcaggtagtaggcaactcatgagcttcaactttgccaccccagtgactca
cctgaatatcttaagagttccaggcagacgatttaggtttttacttatataagacttcgaaagcaaaatgctgttaaggaaaa
tgtggtccttaaagatgatcttgtcgtctttggatgtttataggtaacaattttgctgtattttggctcttattttatgtcca
cagaccatgtctgcaaaggagaagtcgaagtttgaagatatggcaaaaagtgacaaagctcgctatgacagggagatgaaaaat
tacgttcctcccaaaggtgataagaaggggaagaaaaaggaccccaatgctcctaaaaggccaccgtaagtttaaaataaccca
aattgctccttggatttttccttcagtttattaaactctgttgcttcctttcagatctgccttcttcctgttttgctctgaaca
tcgcccaaagatcaaaagtgaacaccctggcctatccattggggatactgcaaagaaattgggtgaaatgtggtctgagcagtc
agccaaagataaacaaccatatgaacagaaagcagctaagctaaaggagaaatatgaaaaggtacagtgtcatctttttaaag
ccgtggataagactaggtataggtaataactgtagaaaacctgggaaatttagttaatcttgtattaatggttgtcagctatgt
tttgaaaaggcctaatgaaaattgtacacttcaacacaaggtaattgaaaccttccttttgactgaaaccagtgtttgtagcac
tagtatattcctgcagacagacttgtagttacttgtagttattgtatagtctgtatagtctgttatttttttttttaagcagag
agtcaaagaaattgttttatgtagatatatataaaatgtgaaggtacaggagggactatggcactgtgtgtgatgtaaaagggt
attggtagtgaaagtactgatactgctgtatcgcaacccttgtcattttacgtcattaacttgttaaagcctagtgggataagt
gctctaaaaacttagactggttaccttttagacagttattagggttattggtcaatcatcttagattgtttacaactaagtgg
tttttcacagttgagtaatgataccggatgctttattttttgacaatatttcaggatattgctgcatatcgtgccaagggcaa
aagtgaagcaggaaagaagggccctggcaggccaacaggctcaaagaagaagaacgaaccagaagatgaggaggaggaggagga
agaagaagatgaagatgaggaggaagaggatgaagatgaagaataaatggctatcctttaatgatgcgtgtggaatgtgtgtgt
gtgctcaggcaattatttgctaagaatgtgaattcaagtgcagctcaatactagcttcagtataaaaactgtacagattttttg
tatagctgataagattctctgtagagaaaatacttttaaaaaatgcaggttgtagcttttttgatgggctactcatacagttaga
ttttacagcttctgatgttgaatgttcctaaatatttaatggtttttttaatttcttgtgtatggtagcacagcaaacttgtag
gaattagtatcaatagtaaattttgggtttttttaggatgttgcatttcgtttttttaaaaaaaaattttgtaataaaattatgta
tattatttctattgtctttgtcttaatatgctaagttaattttcactttaaaaaagccatttgaagaccagagctatgttgatt
tttttcggtatttctgcctagtagtagttcttagacacagttgacctagtaaaatgtttgagaattaaaaccaaacatgctcat
atttgcaaaatgttctttaaaagttacatgttgaactcagtgaactttataagaatttatgcagttttacagaacgttaagttt
tgtacttgacgtttctgtttattagctaaattgttcctcaggtgtgtgtatatatatatacatatatatatatatatatatgta
tatatatacacatatatacgtatatatacatatatatgtatatggagtctcactctgttgcccaggctggagtgcagtggcacg
atcccagctcactgcaacctccgcctcccgggttcaagcgattcttctgcctcagcctccctggtagctggggctacagccatg
tgccaccaagcccagctaatttatatttttagtagagacagggtttcaccatgttggtgaggctggtctggaactgacctcaaa
tgatctgcccacctcagcctcccaaagtgttgggattaacaggtgtgagccaccacgcctgtcccagtatattgtttaacaagt

ttattttgggtgaaaaattctctttaatgggaagaagaggggctagaatgtgaattc
<212> Type : DNA
<211> Length : 4341
      SequenceName : M83665
      SequenceDescription :

Sequence
--------
<213> OrganismName : HUMAN
<400> PreSequenceString :
gacagcggcttccttgatccttgccacccgcgactgaacaccgacagcagcagcctcaccatgaagttgctgatggtcctcatg
ctggcggccctctcccagcactgctacgcaggctctggctgccccttattggagaatgtgatttccaagacaatcaatccacaa
gtgtctaagactgaatacaaagaacttcttcaagagttcatagacgacaatgccactacaaatgccatagatgaattgaaggaa
tgttttcttaaccaaacggatgaaactctgagcaatgttgaggtgtttatgcaattaatatatgacagcagtctttgtgattta
ttttaactttctgcaagacctttggctcacagaactgcagggtatggtgagaaaccaactacggattgctgcaaaccacacctt
ctctttcttatgtctttttactacaaactacaagacaattgttgaaacctgctatacatgtttattttaataaattgatggca
<212> Type : DNA
<211> Length : 503
      SequenceName : NM_002411
      SequenceDescription :

Sequence
--------
<213> OrganismName : HUMAN
<400> PreSequenceString :
gggccggctcgcgggcgctgccagtctcgggcggcggtgtccggcgcgcgggcggcctgctgggcgggctgaagggttagcgga
gcacgggcaaggcggagagtgacggagtcggcgagcccccgcggcgacaggttctctacttaaaagacaatgactactgatgaa
ggtgccaagaacaatgaagaaagccccacagccactgttgctgagcagggagaggatattacctccaaaaaagacaggggagta
ttaaagattgtcaaaagagtggggaatggtgaggaaacgccgatgattggagacaaagtttatgtccattacaaaggaaaattg
tcaaatggaaagaagtttgattccagtcatgatagaaatgaaccatttgtctttagtcttggcaaaggccaagtcatcaaggca
tgggacattggggtggctaccatgaagaaaggagagatatgccatttactgtgcaaaccagaatatgcatatggctcggctggc
agtctccctaaaattccctcgaatgcaactctctttttgagattgagctccttgatttcaaaggagaggatttatttgaagat
ggaggcattatccggagaaccaaacggaaaggagagggatattcaaatccaaacgaaggagcaacagtagaaatccacctggaa
ggccgctgtggtggaaggatgtttgactgcagagatgtggcattcactgtgggcgaaggagaagaccacgacattccaattgga
attgacaaagctctggagaaaatgcagcgggaagaacaatgtattttatatcttggaccaagatatggttttggagaggcaggg
aagcctaaatttggcattgaacctaatgctgagcttatatatgaagttacacttaagagcttcgaaaaggccaaagaatcctgg
gagatggataccaaagaaaaattggagcaggctgccattgtcaaagagaagggaaccgtatacttcaagggaggcaaatacatg
caggcggtgattcagtatgggaagatagtgtcctggttagagatggaatatggtttatcagaaaaggaatcgaaagcttctgaa
tcatttctccttgctgcctttctgaacctggccatgtgctacctgaagcttagagaatacaccaaagctgttgaatgctgtgac
aaggcccttggactggacagtgccaatgagaaaggcttgtataggaggggtgaagcccagctgctcatgaacgagtttgagtca
gccaagggtgactttgagaaagtgctggaagtaaacccccagaataaggctgcaagactgcagatctccatgtgccagaaaaag
gccaaggagcacaacgagcgggaccgcaggatatacgccaacatgttcaagaagtttgcagagcaggatgccaaggaagaggcc
aataaagcaatggcaagaagacttcagaaggggtcactaatgaaaaaggaacagacagtcaagcaatggaagaagagaaacct
gagggccacgtatgacgccacgccaaggagggaagagtcccagtgaactcggcccctcctcaatggctttcccccaactcagg
acagaacagtgtttaatgtaaagtttgttatagtctatgtgattctggaagcaaatggcaaaaccagtagcttcccaaaaacag
cccccctgctgctg
<212> Type : DNA
<211> Length : 1694
      SequenceName : U71321
      SequenceDescription :

Sequence
--------
<213> OrganismName : HUMAN
<400> PreSequenceString :
atgccagggaagcccaagcacttgggtgtccccaacgggcgcatggttctggctgtctcagatggagaactgaccagcactgca
ggttcccaggcccagggtgagggcagaggcagctctctcagcatccacagcctccctagtggccccagcagccccttctccacc
gaggagcagcctgtggccagctgggcccagtcctttgagcggctgctgcaagacccacggggtctggcttacttcactgagttc
ctgaagaaggaattcagcgcagagaacgtaactttctggaaagcctgcgaacgtttccagcagatcccagccagcgacaccaag
cagctagctcaggaggcccacaacatctaccacgagttcctatccagccaggcgctgagcccagtgaacatcgaccgacaggcc
tggcttagtgaggaggtgctggcccagccccggccagatatgttccgagcacagcagcttcagatcttcaatttgatgaagttc
gacagctatgcgcgcttcgtcaaatccccgctgtaccaagagtgcctgctggcggaggccgagggacgcccccctgcgggaacct
ggctcctcacacctcgggagcccggacacagcgaggaagaagccaaagctgaagcctgggaagtcactgccgctgggcgtggaa ,

gagttggggcagctgccactcgctgagggcccttgtggccgccctctccgcaagtcctttcgtagagagatgacaggtggagcc
atgaattcggccctgcgacgagagtctcaagggtccctgaattcttctgccagtctggacctgggtttccttgcctttgtgagc
agcaaatctgagagtcaccggaagagccttggaagtggagagagtgagagcgagagtcggccggggaagtattgctgcgtgtat
ctacctgacggcacggcttccttggccctggctcgacctggcctcaccatccgagacatgctggcaggcatctgtgagaagaga
ggcctctctctgcctgacattaaggtctacctggtgggcaatgaacagaaggccctggtcctggatcaggattgcaccgtgctg
gcagaccaggaagtgcgactggaaaacaggatcaccttccagctggagttggtcggcctggagcgagtggtccggatctcagct
aagcccaccaagcgtctgcaagaggcgctgcagcccatcctggctaagcatggcctgagcctggaccaggtggtactgcacagg
ccaggagagaagcagcccatggatttggagactccagtgagctcagtggcctcacagacactggttttggacactcctccggat
gcaaagatgagtgaagccagaagcatatccccctgccgcagtcagggatgcctcccaagaacccagaccaaggacagtcacctt
cccccatcgtcctccagtttgctggtagaagatgccagtagttctactgggaaccggcagacctgtgacattgaaggcctggtg
gagctgctgaatcgggtgcagagcagcggggcccacgatcagagaggacttcttcgcaaagaggacctggtccttccagaattt
ctgcagcttccttcccaaagaccaggctctcgggaggctccaccatagactgaatcaactattcagcccagggaggcccttcag
actccaccacccacccggtcctctggcatctgtgtaacaatccaggctatctgcatggtgcctgggcaaaccaagcatgccacg
ggtctgtgctgcatgccctatctgtaccatgagtgtctttggctcctttctgctatgggcaggcnnccagaaagggccaggagg
ggggggctaactgttctctcataggtttgcctacccattcctcccagtaggccatcaagaggaggtgggcctagttccttggca
taagcatagtgaacacaaatgggtgactttggcagtgccagcctcctcaacttgttctaggctttagcaggggcaggaaggag
gggctggcccctccttaaagagttgatataagtaagacctgggtgtactggtgagcaacccctccctccatccacagactctac
tgtacatatggatttatggttggcttggggcagctgggtttgtcctggatatatggtactgttatataataataattatc
attattaaaaaaaagctcgacca
<212> Type : DNA
<211> Length : 2208
      SequenceName : U85055
      SequenceDescription :

Sequence
--------
<213> OrganismName : HUMAN
<400> PreSequenceString :
ctccaccatgaaactcgctgtcaccctcaccctggtcacactggctctctgctgcagctccgcttctgcagagatctgcccgag
ctttcagcgtgtcatcgaaaccctcctcatggacacaccctccagttatgaggctgccatggaacttttcagccctgatcaaga
catgagggaggcaggggctcagctgaagaagctggtggacaccctcccccaaaagcccagagaaagcatcattaagctcatgga
aaaaatagcccaaagctcactgtgtaattagcatttagaagctgaagatccccaactgctccagcctctgccgctgccatgctt
tgagtccacgcccaccagccttgctctcttc
<212> Type : DNA
<211> Length : 367
      SequenceName : X13197
      SequenceDescription :

Sequence
--------
<213> OrganismName : HUMAN
<400> PreSequenceString :
gcgtgaggaaagtaccaaacagcagcggagttttaaactttaaatagacaggtctgagtgcctgaacttgccttttcattttac
ttcatcctccaaggagttcaatcacttggcgtgacttcactacttttaagcaaaagagtggtgcccaggcaacatgggtgactg
gagcgccttaggcaaactccttgacaaggttcaagcctactcaactgctggagggaaggtgtggctgtcagtacttttcatttt
ccgaatcctgctgctggggacagcggttgagtcagcctggggagatgagcagtctgcctttcgttgtaacactcagcaacctgg
ttgtgaaaatgtctgctatgacaagtctttcccaatctctcatgtgcgcttctgggtcctgcagatcatatttgtgtctgtacc
cacactcttgtacctggctcatgtgttctatgtgatgcgaaaggaagagaaactgaacaagaaagaggaagaactcaaggttgc
ccaaactgatggtgtcaatgtggacatgcacttgaagcagattgagataaagaagttcaagtacggtattgaagagcatggtaa
ggtgaaaatgcgaggggggttgctgcgaacctacatcatcagtatcctcttcaagtctatctttgaggtggccttcttgctgat
ccagtggtacatctatggattcagcttgagtgctgtttacacttgcaaaagagatccctgcccacatcaggtggactgtttcct
ctctcgccccacggagaaaaccatcttcatcatcttcatgctggtggtgtccttggtgtccctggccttgaatatcattgaact
cttctatgttttcttcaagggcgttaaggatcgggttaagggaaagagcgacccttaccatgcgaccagtggtgcgctgagccc
tgccaaagactgtgggtctcaaaaatatgcttatttcaatggctgctcctcaccaaccgctcccctctcgcctatgtctcctcc
tgggtacaagctggttactggcgacagaaacaattcttcttgccgcaattacaacaagcaagcaagtgagcaaaactggctaa
ttacagtgcagaacaaaatcgaatggggcaggcgggaagcaccatctctaactcccatgcacagccttttgatttccccgatga
taaccagaattctaaaaaactagctgctggacatgaattacagccactagccattgtggaccagcgaccttcaagcagagccag
cagtcgtgccagcagcagacctcggcctgatgacctggagatctagatacaggcttgaaagcatcaagattccactcaattgtg
gagaagaaaaaaggtgctgtagaaagtgcaccaggtgttaattttgatccggtggaggtggtactcaacagccttattcatgag
gcttagaaaacacaaagacattagaatacctaggttcactgggggtgtatggggtagatggtggagagggaggggataagaga
ggtgcatgttggtatttaaagtagtggattcaaagaacttagattataaataagagttccattaggtgatacatagataagggc
tttttctccccgcaaacaccccctaagaatggttctgtgtatgtgaatgagcgggtggtaattgtggctaaatattttgtttta
ccaagaaactgaaataattctggccaggaataaatacttcctgaacatcttaggtcttttcaacaagaaaaagacagaggattg

tccttaagtccctgctaaaacattccattgttaaaatttgcactttgaaggtaagctttctaggcctgaccctccaggtgtcaa
tggacttgtgctactatatttttttattcttggtatcagtttaaaattcagacaaggcccacagaataagatttccatgcatt
tgcaaatacgtatattctttttccatccacttgcacaatatcattaccatcacttttttcatcattcctcagctactactcacat
tcatttaatggtttctgtaaacatttttaagacagttgggatgtcacttaacatttttttttttgagctaaagtcagggaatc
aagccatgcttaatatttaacaatcacttatatgtgtgtcgaagagtttgttttgtttgtcatgtattggtacaagcagataca
gtataaactcacaaacacagatttgaaaataatgcacatatggtgttcaaatttgaacctttctcatggattttgtggtgtgg
gccaatatggtgtttacattatataattcctgctgtggcaagtaaagcacactttttttttctcctaaaatgtttttccctgtg
tatcctattatggatactggtttgttaattatgattctttatttctctcctttttttaggatatagcagtaatgctattact
gaaatgaatttcctttttctgaaatgtaatcattgatgcttgaatgatagaattttagtactgtaaacaggctttagtcattaa
tgtgagagacttagaaaaaatgcttagagtggactattaaatgtgcctaaatgaattttgcagtaactggtattcttgggtttt
cctacttaatacacagtaattcagaacttgtattctattatgagtttagcagtcttttggagtgaccagcaactttgatgtttg
cactaagatttatttggaatgcaagagaggttgaaagaggattcagtagtacacatacaactaatttatttgaactatatgtt
gaagacatctaccagtttctccaaatgcctttttaaaactcatcacagaagattggtgaaaatgctgagtatgacacttttct
tcttgcatgcatgtcagctacataaacagttttgtacaatgaaaattactaatttgtttgacattccatgttaaactacggtca
tgttcagcttcattgcatgtaatgtagacctagtccatcagatcatgtgttctggagagtgttctttattcaataaagttttaa
tttagtataaacat
<212> Type : DNA
<211> Length : 3038
      SequenceName : X52947
      SequenceDescription :

Sequence
--------
<213> OrganismName : HUMAN
<400> PreSequenceString :
atgaaggctgtcattattctggggcttgtcctcctttctgtcacggtccagggcaagatctttgaaaggtgtgagttggccaga
actctgaaaagattgggactggatggctacaggggaatcagcctagcaaactgggtgtgtttggccaaatgggagagtaattat
aacacacaagctacaaactacaatcctggagaccaaagcactgattatgggatatttcagatcaatagccactactggtgtaat
aatggcaaaaccccaggagcagttaatgcctgtcatatatcctgcaatgctttgctgcaagataacatcgctgatgctgtaact
tgtgcaaagagggttgtcagtgatccacaaggcattagagcatgggtggcatggagaaatcactgtcaaaacagagatgtcagt
cagtatgttcaaggttgtggagtgtaattgcagaattttccttcttcagctcattttcgtctctttctcacattaagggagtag
taattaagtgaaagatcacactaccattatttccccttcaaagaatatatttacagagcaggagcaaaatatggcttcttctag
agacgtatg
<212> Type : DNA
<211> Length : 597
      SequenceName : X60236
      SequenceDescription :

Sequence
--------
<213> OrganismName : HUMAN
<400> PreSequenceString :
ctcggaaggacaggatgccatgccatcgggaagacaggactccccaaggagaacaggatgccatcagggagacaggactccccc
gtggaagataggatacctccaggaagacagaatgccccccaggcagccctgggacagcgggaaacactagggaccaagctatcc
ctggttcactcgggattttgttgggttgtgggcggggctcaggaaagattgtccctgctggtcctgctccacgaccacccaccc
ggcaaggttccctcctagagaaccttgtcagagacagaacgggtccccacaggcgcattcttctttccaacacccatggcgcag
ttcctgagactttgcatctggctcgtagcgcttgggtcctgcctcctggctacagtgcaggcagactgcagccaggactgcgct
aaatgcagctaccgcctggtacgtcccggcgacatcaacttcctggcatgcacactcgaatgtgaagggcagctgccttctttc
aaaatctgggagacctgcaaggatctcctgcaggtgtccaagcccgagttcccttgggataacatcgacatgtacaaagacagc
agcaaacaggatgagagccacttgctagccaagaagtatggagggttcatgaaacggtatggaggcttcatgaagaagatggat
gagctttaccccgtggagccagaagaagaggccaatggaggcgagatccttgccaagaggtatggcggtttcatgaagaaggat
gcagatgagggagacaccttggccaactcctccgacctgctgaaagagctactgggaacaggagacaaccgtgcgaaagatagc
caccaacaggaaagcaccaacaatgatgaagacagcacgagcaagaggtatgggggcttcatgagaggcctcaaaagaagcccc
cagctggaagacgaagcaaaggagctgcagaagcgctatgggggcttcatgagaagggtcggcgccccgagtggtggatggac
tatcagaagagatacggaggcttcctgaagcgctttgctgagtctctaccctcggatgaagaaggcgaaagttactctaaagaa
gttcccgagatggaaaaaagatacggaggctttatgcggttttgaagcccttcccagcagtgatcccggcccccactagcctg
ctccatcccccgtgagcaactgccccatcagtgatgtttcttgtcatgtgctgcttgtgctgtacagttgccctcgtggtctgg
ataactgctgcctgaaagctgtgatttttagggggttgtctgtattcttttgagtcttgaagctcagtattggtctcttgtggc
tatgttatcatgctgaaacagtctgttacctcatcccttctgacaaaacgtcaataaatgcttatttgtatataaaaataataa
acccgtgaacccaactgc
<212> Type : DNA
<211> Length : 1446
      SequenceName : Y07503

SequenceDescription :

Sequence
--------
<213> OrganismName : HUMAN
<400> PreSequenceString :
aagagaaccagacccatgtttatccctttgaaagaaacctgaaccaagccattcctctggactgttcttccatccactggacca
ataactcatagtttgaaggatttttacttaatggaagtgagaaaacagccagatcaaaagaatttccaggagccaagacagaaa
acatgcagagcattagacagcctaaatggagctggctagatatgactc
<212> Type : DNA
<211> Length : 216
SequenceName : 1700438H1
SequenceDescription :

Sequence
--------
<213> OrganismName : HUMAN
<400> PreSequenceString :
tggacagcctggccggctggaagccctccgtgtactacgtcatgctgatcctatgcacagcctccatcctcatctcttgctgcg
cctcagccatgtacacccccattgaaggctggagctactttgactcactctacttctgttttgtggctttcagcaccattggct
ttggggacctggtcagcagccagaacgcccactatgagagccaaggcctctatcgctttgccaacttcgtcttcatcctcatgg
gtgtctgctgcatctac
<212> Type : DNA
<211> Length : 269
SequenceName : 3092423H1
SequenceDescription :

Sequence
--------
<213> OrganismName : HUMAN
<400> PreSequenceString :
ctcacctggtgcaggtggacacctgcgcaggtgtgtgccctccggcccctgaagcatggccagcagcggcatggctgacagcgc
caaccacctgcccatctttttcggcaacatcacccgggaggaggcagaag
<212> Type : DNA
<211> Length : 134
SequenceName : 3285272H1
SequenceDescription :

Sequence
--------
<213> OrganismName : HUMAN
<400> PreSequenceString :
cngncattgcagtanatnnagctcnatagaganatcgccgttggcaattgagtatgcggacgggnactgtgncgactctgtgcc
tcgctgaggaaaaataactaa
<212> Type : DNA
<211> Length : 105
SequenceName : 3633286H1
SequenceDescription :

Sequence
--------
<213> OrganismName : HUMAN
<400> PreSequenceString :
ctgggttcccgagtgcttgagctgtggctcccgctgtagctctgaccacgtggaaactcaagcctgcactcgggaacagaaccg
catctgcacctacaggcccggctggtactgcgcgctgagcaagcaggaggggtgccggctgtgcgcgccgctgcgcaagtgccg
cccgggcttcggcgtggccagaccaggaactgaaacatcagaactggtgtgcaa
<212> Type : DNA
<211> Length : 222
SequenceName : 5077322H1
SequenceDescription :

Sequence
--------

<213> OrganismName : HUMAN
<400> PreSequenceString :
gggagtccgctccagcggggcgctccagtccctcagacgtgggctgagcttgggacgagctgcgttccgccccaggccactgta
gggaacggcggtggcgcctccccagcaaaccggaccgactggg
<212> Type : DNA
<211> Length : 127
      SequenceName : 6300669H1
      SequenceDescription :

Sequence
--------
<213> OrganismName : HUMAN
<400> PreSequenceString :
ggcttcaccatgcacagctgggagcgcctggcagttctggtcctcctagtgagcggccgcctgcgcgggccgccccgtggtcgg
atcctgggcggcagaaaaggccaagggcgcacgcgcggccctacatggcgtcggtgcagctgaacggcgcgcacctgtgcggcg
gcgtcctggtggc
<212> Type : DNA
<211> Length : 181
      SequenceName : 6805226H1
      SequenceDescription :

Sequence
--------
<213> OrganismName : HUMAN
<400> PreSequenceString :
ctccgactggacgcagcagccaccgacgcgtccctctctccacgaggctgccggcttaggacccccagctccgacatgtcggcc
tctggtcggctgtgtcttctcaacatcgttggactgattctccccaccagaggacagacgttgaaagataccacgtccagttct
tcagcagacgcaactatcatggacattcaggtcccgacacgagccccagatgcagtc
<212> Type : DNA
<211> Length : 225
      SequenceName : 914979H1
      SequenceDescription :

Sequence
--------
<213> OrganismName : HUMAN
<400> PreSequenceString :
gactattacaaaagtttatttaacaaaaagtctaatatgaaaatgtacatgacctaattttacatcatagtaaaacaggccct
atggagagaggacatgggtttctctgctgaacagccattatttatactcgttccaaggcttctaacatgatgatactatttcct
cgtattaccaccattccaatattgttctgttgtccactagtcgccatctccacacattcatctatcacaaggttcataaagga
tcaaatccccgcaatattccttggacatgtctgccaccatttaatttcaatgataacttcttgtccataaatttttcaactcg
gggagggtgagctttgctcatnggtgtatactccgcgggctcacagatgcctttggaacgcaacgcacggctttcctcacgctc
ccgctgtaggcccggcgtcttgccctcgtgccgaatttcttggccccganggcaaa
<212> Type : DNA
<211> Length : 476
      SequenceName : A025432
      SequenceDescription :

Sequence
--------
<213> OrganismName : HUMAN
<400> PreSequenceString :
caaggtcctggtgatcactgtcttctgtggtctgactgtggctttccccctgtcagaattagtttcaatcaataaagaactaca
gaattcaatcattgacctactaaactcagtctttgaccaactgggatcatacagagggacaaaagctcctctagaggattatac
agatgatgatttaagcactgactctgagcagatcatggacttcacgccagccgcaaacaaacagaattctgagttctctactga
tgttgagacagtctcctccggctttctggaagaattcactngagaacacagacatcacagtgaaaattccattagctgggaatc
cagtctcccctacttcctgagaagactccaactttgaaataaacctgcgactgccattcaggatattgtctttgtagaaaatga
tata
<212> Type : DNA
<211> Length : 424
      SequenceName : AA070627
      SequenceDescription :

Sequence
--------
<213> OrganismName : HUMAN
<400> PreSequenceString :
ggggtgttcggtccttgcgggcttcacgagatcgattcctgactactttgctgtgaattgcacaactcacacagtaatgtagc
ttcacatacagcttgggaagcacataggcatcgaagacgctcgcttcagaaatgtccctgactgctgcggcctccactatgttt
cgaatgaccgaatttcttaatgggccttgtccttgggcacggcatcggggcacagttaagtgcagcgaataaggctgcacgtgg
gccgcgggcccttttttggcacgaccaattgttccttccttttccttggcaatcttgaaggaacgaaccgaagagaggaagacg
gtgcn
<212> Type : DNA
<211> Length : 341
      SequenceName : AA187162
      SequenceDescription :

Sequence
--------
<213> OrganismName : HUMAN
<400> PreSequenceString :
ccagaccccaagccngggccatcccgccactcgacgggtggtgctcgngacggcgtgcanntcccgcccggggactacagcacg
acncccggcggcacntcattcagcaccaccccggggangtaccaggatcatctatgaccggaaattc
<212> Type : DNA
<211> Length : 151
      SequenceName : AA190648
      SequenceDescription :

Sequence
--------
<213> OrganismName : HUMAN
<400> PreSequenceString :
ggctcggcgcgatggaggaggagacgcatactgacgccaaaatccgtgctgaaaatggaacagggtccagccctcggggtcctg
gctgcagcctccggcactttgcctgcgaacagaacctgctgtcgcggccagatggctctgcttccttcctgcaaggtgacacct
ctgtcctggcgggtgtgtacgggccggccgaggtgaaggtcagcaaagagattttcaacaaggccacactcgaagtgatcctga
ggccgaagattgggctgcctgcatggcattggtggatgcaggtgtgcccatgcgggctctcttctgtggggtcgcctgcgccct
ggactctgatgggaccctcgtgctggatcctacatccaagcaagaaaaggaggcccgggcagtcctgacctttgccctggacag
cgtggaacgaagctgctgatgt
<212> Type : DNA
<211> Length : 442
      SequenceName : AA428915
      SequenceDescription :

Sequence
--------
<213> OrganismName : HUMAN
<400> PreSequenceString :
tttttttttgagagattaagattcttttaatggtgaaaagatatacatatatttggaactagccagcttgactcagtttaggtg
atcccaattttggtggcaacaaccaaagcatcgtagtcaggagccagtcgaacatatgccttcctctctccatcagactgaatc
agagtgttgactttggccacatcaatgtcacaaacttcttcacagcctgtttgatctggtgcttgttggctttaacatccacag
tgaacacaagtaggctgttgtttctatcttcttcacagcctactcagtggtcagcggaaacttgatgataacatggtggtcaa
gcttatttctcctggggggtgctcttccaaggatatttgggctgcctccggagtcacagtgtcttgggccgccggaaggtgggtg
acatgtggatcttgtttttttgtgg
<212> Type : DNA
<211> Length : 446
      SequenceName : AA868186
      SequenceDescription :

Sequence
--------
<213> OrganismName : HUMAN
<400> PreSequenceString :
ccgctctccgctgcgggggaggccatggcggaaccttcccaggccccgaccccggccccggctgcgcagccccggccccttcag
tccccagcccctgccccaactccgactcctgcacccagcccggcttcagccccgattccgactcccaccccggcaccagcccct
gccccagctgcagccccagccggcagcacagggactgggggcccggggtaggaagtggggggccgggagcgggggggatccg
gctcggcctggcctgagccagcagcagcgcgccagtcagaggaaggcgcaagtccgggggctgccccgcgccaagaagcttgag

aagctaggggtcttctcggcttgcaaggccaatggaacctgtaagtgtaatggctggaaaaaccccaagcccccccactgcaccc
cgcatagatctgcagcagccagctgccaacctgagtgagctgtgccgcagttgtgagcaccccttggctgaccacgtatcccac
ttggagaatgtgtcagaggatgagataaaccgactgctggggatggtggtggatgtggagaatctcttcatgtctgttcacaag
gaagaggacacagacaccaagcaggtctatttctacctcttcaagctactgcggaaatgcatcctgcagatgacccggcctgtg
gtggaggggtccctgggcagccctccatttgagaaacctaatattgagcagggtgtgctgaactttgtgcagtacaagtttagt
cacctggctccccgggagcggcagacgatgttcgagctctcaaagatgttcttgctctgccttaactactgggagcttgagaca
cctgcccagtttcggcagaggtctcaggctgaggacgtggctacctacaaggtcaattacaccagatggctctgttactgccac
gtgccccagagctgtgatagcctcccccgctacgaaaccactcatgtctttggcgaagccttctccggtccattttcaccgtt
acccgccggcagctgctggaaaagttccgagtggagaaggacaaattggtgcccgagaagaggaccctcatcctcactcacttc
cccaaattcctgtccatgctggaggaggagatctatggggcaaactctccaatctgggagtcaggcttcaccatgccaccctca
gaggggacacagctggttccccggccagcttcagtcagtgcagcggttgttcccagcacccccatcttcagccccagcatgggt
gggggcagcaacagctccctgagtctggattctgcaggggccgagcctatgccaggcgagaagaggacgctcccagagaacctg
accctggaggatgccaagcggctccgtgtgatgggtgacatccccatggagctggtcaatgaggtcatgctgaccatcactgac
cctgctgccatgctggggcctgagacgagcctgctttcggccaatgcggcccgggatgagacagcccgcctggaggagcgccgc
ggcatcatcgagttccatgtcatcggcaactcactgacgcccaaggccaaccggcgggtgttgctgtggctcgtggggctgcag
aatgtcttttcccaccagctgccgcgcatgcctaaggagtatatcgcccgcctcgtctttgacccgaagcacaagactctggcc
ttgatcaaggatgggcgggtcatcggtggcatctgcttccgcatgtttcccacccagggcttcacggagattgtcttctgtgct
gtcacctcgaatgagcaggtcaagggttatgggacccacctgatgaaccacctgaaggagtatcacatcaagcacaacattctc
tacttcctcacctacgccgacgagtacgccatcggctacttcaaaaagcagggtttctccaaggacatcaaggtgcccaagagc
cgctacctgggctacatcaaggactacgagggagcgacgctgatggagtgtgagctgaatccccgcatcccctacacggagctg
tcccacatcatcaagaagcagaaagagatcatcaagaagctgattgagcgcaaacaggcccagatccgcaaggtctacccgggg
ctcagctgcttcaaggagggcgtgaggcagatccctgtggagagcgttcctggcattcgagagacaggctggaagccattgggg
aaggagaaggggaaggagctgaaggaccccgaccagctctacacaaccctcaaaaacctgctggcccaaatcaagtctcacccc
agtgcctggcccttcatggagcctgtgaagaagtcggaggcccctgactactacgaggtcatccgcttccccattgacctgaag
accatgactgagcggctgcgaagccgctactacgtgacccggaagctctttgtggccgacctgcagcgggtcatcgccaactgt
cgcgagtacaacccccccggacagcgagtactgccgctgtgccagcgccctggagaagttcttctacttcaagctcaaggaggga
ggcctcattgacaagtaggcccatctttgggccgcagccctgacctggaatgtctccacctcggattctgatctgatccttagg
gggtgccctggccccacggacccgactcagcttgagacactccagccaagggtcctccggacccgatcctgcagctctttctgg
accttcaggcacccccaagcgtgcagctctgtcccagccttcactgtgtgtgagaggtctcctgggttggggcccagcccctct
agagtagctggtggccagggatgaaccttgcccagccgtggtggcccccaggcctggtccccaagagctttggaggcttggatt
cctgggcctggcccaggtggctgtttccctgaggaccagaactgctcatttagcttgagtgatggcttcaggggttggaagtt
cagcccaaactgaagggggccatgccttgtccagcactgttctgtcagtctcccccaggggtgggggtatggggaccattcat
tccctggcattaatcccttagagggaataataaagcttttatttctctg
<212> Type : DNA
<211> Length : 3074
      SequenceName : AF029777
      SequenceDescription :

Sequence
--------
<213> OrganismName : HUMAN
<400> PreSequenceString :
atgctggttctctttgtggccacatggagtgacctgggcctctgcaagaagcgcccgaagcctggaggatggaacactgggggc
agccgatacccggggcagggcagccctggaggcaaccgctacccacctcagggcggtggtggctgggggcagcctcatggtggt
ggctgggggcagcctcatggtggtggctgggggcagccccatggtggtggctggggacagcctcatggtggtggctggggtcaa
ggaggtggcacccacagtcagtggaacaagccgagtaagccaaaaaccaacatgaagcacatggctggtgctgcagcagctggg
gcagtggtgggggggccttggcggctacgtgctgggaagtgccatgagcaggcccatcatacatttcggcagtgactatgaggac
cgttactatcgtgaaaacatgcaccgttaccccaaccaagtgtactacaggcccatggatgagtacagcagccagaacaacttt
gtgcacgactgcgtcaatatcacaatcaagcagcacacggtcaccacaaccaccaagggggagaacttcaccgagaccgacgtt
aagatgatggagcgcgtggttgagcagatgtgtatcacccagtacgagagggaatctcaggcctattaccagagaggatcgagc
atggtcctcttctcctctccacctgtgatcctcctgatctctttcctcatcttcctgatagtgggatgaggaaggtcttcctgt
t
<212> Type : DNA
<211> Length : 757
      SequenceName : AF030575
      SequenceDescription :

Sequence
--------
<213> OrganismName : HUMAN
<400> PreSequenceString :
tttttgcagtttgaatcctggtttatttaagtagcattcaggagttatactgtaaataaacatcattatggctaatgactaatt
aaaatcaccattcattacaaataccaaccaggttagataatactattaactatcttttccccttcggtctggggttcatattct

tcctttctggaagtcatttcatggataaaatatttagcatttttgttacctcttccttcaccaactgtactcaacacactt
ctgtaaacagagtgagaacctgggttcatggacaagctcttttccacttatcttcgggttaaaccaaaactttttcagcaactt
tgcccctgtccaagttttgcagaacaccagctgcctcttcctgttctaggcattctagggctagatacctgtgaacggacttca
agcattaaagaagtcatcagcatttcctatacctcacacacactcatt
<212> Type : DNA
<211> Length : 468
      SequenceName : AI147295
      SequenceDescription :

Sequence
--------
<213> OrganismName : HUMAN
<400> PreSequenceString :
tttttttccggtatccactggaagtttatttctttagggttctatcccaaccagtcgcttaaaaaccaagtaacacagacctg
aggggtgggggctggggactgcacctccctcctactcatggtggacagcagtggggactagggaggggcaggagaggtggctga
agcaaggcagcagtaatggggccacgacgccacagagccagctccgtcctctcccagaccctggtgggagtccctgggtggtga
aaaaaggggggggaaancaccccaggccctncctctcccttccttaaacagcctcctttcgggctcaacccatttctt
<212> Type : DNA
<211> Length : 330
      SequenceName : AI368583
      SequenceDescription :

Sequence
--------
<213> OrganismName : HUMAN
<400> PreSequenceString :
ttttaggattttagtattttaatataatacagttccaatcagtgtgtctggttcatttcagttccttcactgccaaaccggg
gggcagggactgcttcagtttctctgccttctctttgtcagtgatgaccagggtgtaaaggtatctgctgcatcgaactttaaa
cttcacgttgtccttattttcttgatcttgacagatttggcatcctttcgtcgggctgtgagcaggaagtccttgatttcctc
aattttccgaggcatggcgac
<212> Type : DNA
<211> Length : 273
      SequenceName : AI686653
      SequenceDescription :

Sequence
--------
<213> OrganismName : HUMAN
<400> PreSequenceString :
tgatgtaaaattcagctttattagaattctcttaaaatgtttgataatatacaagcagtctgactcccaatgctttgtgtcatt
atacaaataagaaaataacttgctttaattctaatgcagcatttttaacctgctaaatagaactaacttcttgtttctgaaat
cctgaacatatgcctcattgaaataccattttcaggacatcgttttcccagcaaaagcttctttagataacagaatcatttgg
tatacacagaacacgaatgatgactgcaaggggaggctgcagggctggaggagggttagagcaacacagtttccagttacagtt
gagtccgggaagccagcaccagaaagaggggaatgataagacccgccctttgttctgtataaagacttggggcttttctttgag
tagcggctgctgccgactttaaggcacagtcaggatgggtgctgtggtccacgcactgtaagagcttcactggtcggtaatcat
taatacagctgtactggaaacccatgtncttcagcctcttttnncagactntcatgctgncttccccgcaggattccacattgn
gtcccccaatnttcatgcatacccagat
<212> Type : DNA
<211> Length : 616
      SequenceName : AI700360
      SequenceDescription :

Sequence
--------
<213> OrganismName : HUMAN
<400> PreSequenceString :
ttttatttcttcagctaaaacagcggaagaggggatttattatatggttgttacactcggccacaaataaacacagaaatagt
ccagaatgtcacaggtccagggcagaggaccaacatgggcattttgtttatgagcaaggtgggtctcagaggtgatcggcgatc
agagggcgatgaagttctagatccattgagacaagctctagacagtag
<212> Type : DNA
<211> Length : 216
      SequenceName : AI707852
      SequenceDescription :

Sequence
--------
<213> OrganismName : HUMAN
<400> PreSequenceString :
tccaaggggctgtttaagatggcggaggtgcatgtcctggtgcttgatggtcgaggccatctcctgggccgcctggcggccatc
gtggctaaacaggtactgctgggccggaaggtggtggtcgtacgctgtgaaggcatcaacatttctggcaatttctacagaaac
aagttgaagtacctggctttcctccgcaagcggatgaacaccaacccttcccgaggcccctaccacttccgggcctccagccgc
atcttctggcggaccgtgcgaggtatgctgccccacaaaaccaagcgaggccaggccgctctggaccgtctcaaggtgtttgac
ggcatcccaccgccctacgacaagaaaaagcggatggtggttcctgctgccctcaaggtcgtgcgtctgaagcctacaagaaag
tttgcctatctgcggcgcctggctcacgaggttggctggaagtaccaggcagcgacagccaccctgtaggagaagaggaaagag
aaagccaagatccactaccggaagaagaac
<212> Type : DNA
<211> Length : 534
      SequenceName : AI878943
      SequenceDescription :

Sequence
--------
<213> OrganismName : HUMAN
<400> PreSequenceString :
gaactatagcagatgaaataaaatatatatctgttaaatgtcagagagcaagttagacacacattccactaagcatcaaaggcc
ctcgtagtctcggtggaaggacaaactccagctccacatcactggtttaagtttcttcctcttcttcttcctcttcttcctcct
cctcttcctcctcatcttcatcagagctgaaggtgccatcagggaggctgtagacacggatgacctgcttgttggggtccttga
ggatgaggtatttgccctcctccagcttcatgcagatgtcaatgacgcagcgtaaaatgccccaggcattctccacgctcaagg
ttgatctggctggcaaactcattaggcttgaactgctgggtgcctatgatgacctggcgtgaaagagtctttcaccgtggttac
cgagacacataacc
<212> Type : DNA
<211> Length : 434
      SequenceName : AW150605
      SequenceDescription :

Sequence
--------
<213> OrganismName : HUMAN
<400> PreSequenceString :
attataaaagtgtgctttattgtaaggaaaatttaaatataaccaacaatactgaaaataatgtacgtggcatttatagccttg
gtcaaagttctttattaaacaggcgtttgctgttccatgaatagttaacaattagtttatgcaacaaaatagataacttgaaaa
agtgagtaagaatacagaatgatgaaccaggaatctggagttcttttgtgtttttttcttgtttaacagacatggcttttaaaat
aaaatctgaaatataaaacaagtcatcttcatgtagagagctctcttttcctacaagaaggttttgcaaaaatgttaaacagtg
ttctaaaaatgtggctgaagttatactttacatactttaacttcttatatatacattatccaattaacattctaaccacatttt
catatacaagtgggaccataaacattctctagtaagactacactagcacagagtaatttacccatgtatatatattcatttga
aacctgtttccaaaatgaaatcactcatttaaaagcaaacaactggttagaattaatgtctctagacactgggtcatggggcct
agaaatcatgttagagtatatttgctttgttgaatattattaaattcatcttatgttgcancatact
<212> Type : DNA
<211> Length : 655
      SequenceName : AW467649
      SequenceDescription :

Sequence
--------
<213> OrganismName : HUMAN
<400> PreSequenceString :
ttgggtaccaaatttctttatttgaaggaatggtacaaatcaaagaacttaagtggatgttttggtacaacttatagaaaaggt
aaaggaaaccccaacatgcatgcactgccttggtgaccagggaagtcaccccacggctatggggaaattagcccgaggcttagc
tttcattatcactgtctcccagggtgtgcttgtcaaagagatattccgccaagccagattcgggcgctcccatcttgcgcaagt
tggtcacgtggtcacccaattctttgatggctttcacctgctcattcaggtaatgtgtctcaatgaagtcacacaaatggggggt
catttttgtcagtggccagtttgtgcagttccagtagtgactgattcacatttttttccaaatgtaatgcacactccattgcat
tcagcccg
<212> Type : DNA
<211> Length : 428
      SequenceName : AW519155
      SequenceDescription :

Sequence
--------
<213> OrganismName : HUMAN
<400> PreSequenceString :
agtccgagtggagagagcgagctgagtggttgtgtggtcgcgtctcggaaaccggtagcgcttgcagcatggctgaccaactga
ctgaagagcagattgcagaattcaaagaagctttttcactatttgacaaagatggtgatggaactataacaacaaaggaattgg
gaactgtaatgagatctcttgggcagaatcccacagaagcagagttacaggacatgattaatgaagtagatgctgatggtaatg
gcacaattgacttccctgaatttctgacaatgatggcaagaaaaatgaaagacacagacagtgaagaagaaattagagaagcat
tccgtgtgtttgataaggatggcaatggctatattagtgctgcagaacttcgccatgtgatgacaaaccttggagagaagttaa
cagatgaagaagttgatgaaatgatcagggaagcagatattgatggtgatggtcaagtaaactatgaagagtttgtacaaatga
tgacagcaaagtgaagaccttgtacagaatgtgttaaatttcttgtacaaaattgtttatttgccttttctttgtttgtaactt
atctgtaaaaggtttctccctactgtcaaaaaaatatgcatgtatagtaattaggacttcattcctccatgttttcttccctta
tcttactgtcattgtcctaaaaccttattttagaaaattgatcaagtaacatgttgcatgtggcttactctggatatatctaag
cccttctgcacatctaaacttagatggagttggtcaaatgagggaacatctgggttatgcctttttaaagtagttttctttag
gaactgtcagcatgttgttgttgaagtgtggagttgtaactctgcgtggactatggacagtcaacaatatgtacttaaaagttg
cactattgcaaaacgggtgtattatccaggtactcgtacactatttttttgtactgctggtcctgtaccagaaacattttcttt
tattgttacttgcttttttaaactttgtttagccacttaaaatctgcttatggcacaatttgcctcaaaatccattccaagttgt
atatttgttttccaataaaaaaattacaatttaccc
<212> Type : DNA
<211> Length : 1128
      SequenceName : D45887
      SequenceDescription :

Sequence
--------
<213> OrganismName : HUMAN
<400> PreSequenceString :
cgggagagcgcgctctgcctgccgcctgcctgcctgccactgagggttcccagcaccatgagggcctggatcttctttctcctt
tgcctggccgggagggccttggcagcccctcagcaagaagccctgcctgatgagacagaggtggtggaagaaactgtggcagag
gtgactgaggtatctgtgggagctaatcctgtccaggtggaagtaggagaatttgatgatggtgcagaggaaaccgaagaggag
gtggtggcggaaaatccctgccagaaccaccactgcaaacacggcaaggtgtgcgagctggatgagaacaacacccccatgtgc
gtgtgccaggaccccaccagctgcccagcccccattggcgagtttgagaaggtgtgcagcaatgacaacaagaccttcgactct
tcctgccacttctttgccacaaagtgcaccctggagggcaccaagaagggccacaagctccacctggactacatcgggccttgc
aaatacatcccccccttgcctggactctgagctgaccgaattcccctgcgcatgcgggactggctcaagaacgtcctggtcacc
ctgtatgagagggatgaggacaacaaccttctgactgagaagcagaagctgcgggtgaagaagatccatgagaatgagaagcgc
ctggaggcaggagaccaccccgtggagctgctggcccgggacttcgagaagaactataacatgtacatcttccctgtacactgg
cagttcggccagctggaccagcaccccattgacgggtacctctcccacaccgagctggctccactgcgtgctcccctcatcccc
atggagcattgcaccacccgctttttcgagacctgtgacctggacaatgacaagtacatcgccctggatgagtgggccggctgc
ttcggcatcaagcagaaggatatcgacaaggatcttgtgatctaaatccactccttccacagtaccggattctctctttaaccc
tccccttcgtgtttcccccaatgtttaaaatgtttggatggtttgttgttctgcctggagacaaggtgctaacatagatttaag
tgaatacattaacggtgctaaaaatgaaaattctaacccaagacatgacattcttagctgtaacttaactattaaggcctttttc
cacacgcattaatagtcccatttttctcttgccatttgtagctttgcccattgtcttattggcacatgggtggacacggatctg
ctgggctctgccttaaacacacattgcagcttcaacttttctctttagtgttctgtttgaaactaatacttaccgagtcagact
ttgtgttcatttcatttcagggtcttggctgcctgtgggcttccccaggtggcctggaggtgggcaaagggaagtaacagacac
acgatgttgtcaaggatggttttgggactagaggctcagtggtgggagagatccctgcagaatccaccaaccagaacgtggttt
gcctgaggctgtaactgagagaaagattctggggctgtcttatgaaaatatagacattctcacataagcccagttcatcaccat
ttcctcctttacctttcagtgcagtttcttttcacattaggctgttggttcaaacttttgggagcacggactgtcagttctctg
ggaagtggtcagcgcatcctgcagggcttctcctcctctgtcttttggagaaccagggctcttctcaggggctctagggactgc
caggctgtttcagccaggaaggccaaaatcaagagtgagatgtagaaagttgtaaaatagaaaaagtggagttggtgaatcggt
tgttctttcctcacatttggatgattgtcataaggtttttagcatgttcctccttttcttcaccctcccctttgttcttctatt
aatcaagagaaacttcaaagttaatgggatggtcggatctcacaggctgagaactcgttcacctccaagcatttcatgaaaaag
ctgcttcttattaatcatacaaactctcaccatgatgtgaagagtttcacaaatctttcaaaataaaagtaatgacttagaaa
ctgaaaaaaaaaaaaaaaaaaaaaaaaaaa
<212> Type : DNA
<211> Length : 2133
      SequenceName : J03040
      SequenceDescription :

Sequence
--------
<213> OrganismName : HUMAN
<400> PreSequenceString :

gcggccgcgtgaggcggaactctgagcaggtggtccattatggctgacatgcaaaatctggtagaaagattggagagggcagtg
ggccgcctggaggcagtatctcatacctctgacatgcaccgtgggtatgcagacagtccttcaaaagcaggagcagctccatat
gtgcaggcatttgactcgctgcttgctggtcctgtggcagagtacttgaagatcagtaaagagattgggggagacgtgcagaaa
catgcggagatggtccacacaggtttgaagttggagcgagctctgttggttacagcttctcagtgtcaacagccagcagaaaat
aagctttccgatttgttggcacccatctcagagcagatcaaagaagtgataacctttcgggagaagaaccgaggcagcaagttg
tttaatcacctgtcagctgtcagcgaaagtatccaggccctgggctgggtggctatggctcccaagcctggcccttatgtgaaa
gaaatgaatgatgccgccatgttttatacaaaccgagtcctcaaagagtacaaagatgtggataagaagcatgtagactgggtc
aaagcttatttaagtatatggacagagctgcaggcttacattaaggagttccataccaccggactggcctggagcaaaacgggg
cctgtggcaaaagaactgagcggactgccatctggaccctctgccggatcaggtcctcctcccctccaccaggcccccctcct
cccccagtctctaccagttcaggctcagatgagtctgcttcccgctcagcactgttcgcgcagattaatcagggggagagcatt
acacatgccctgaaacatgtatctgatgacatgaagactcacaagaaccctgccctgaaggctcagagtggtccagtacgcagt
ggccccaaaccattctctgcacctaaaccccaaaccagcccatcccccaaacgagccacaaagaaggagccagctgtacttgaa
ctggagggcaagaagtggagagtggaaaatcaggaaaatgtttccaacctggtgattgaggacacagagctgaaacaggtggct
tacatatacaagtgtgtcaacacgacattgcaaatcaagggcaaaattaactccattacagtagataactgtaagaaacttggc
ctggtattcgatgacgtggtgggcattgtggagataatcaacagtaaggatgtcaaagttcaggtaatgggtaaagtgccaacc
atatccatcaacaaaacagatggctgccatgcttacctgagcaagaattccctggattgtgaaatagtcagtgccaaatcttcc
gagatgaatgtcctcattcctacagaaggcggtgactttaatgaattcccagttcctgagcagttcaagaccctatggaacggg
cagaagttggtcaccacagtgacagaaattgctggataagcgaagtgccactgggttctttgccctcccttcacaccatgggat
aaatctgtatcaagacggttcttttctagatttcctctacctttttgctcttaaaactgcttctctgctctgagaagcacagct
acctgccttcactgaaatatacctcaggctgagatttggggtgggatagcaggtcagttgatcttctgcaggaaggtgcagctt
ttccatatcagctcaaccacgccgccagtccattcttaaggaactgccgactaggactgatgatgcattttagctttgagcttt
tgggggttattctaccaacaaacagtccattggaaagaaaacagtccctggaattaacagatcagaatgttcacactggttaat
ctttttttaacaatgagcatgaaggtagcagaagctggtgtgtttccagatggttcttctaaccaaactaatttttcactgttg
acaagcgaggcaagggttgcactggaccaaaggctgaggcttggccatctagcattccatacaaaattgtttcctataagcatt
ccttttattctctattctatcctgggtctgcctcaaccgtgagataggagagtctctggtactagctgctgtagcagtgcccctt
catccagggcagttaatggagtcttggaccctttctttctctgggatccctgcccagcaccttcctatagagatgactttaaaa
ggaaaaaaaaaaaaaaacccacatgatttcaaggagtctggcattcctgaatccttcttccctgccaggtgcctgtcacctgtc
ttcactgcctccttttccctgtcatgctcatcagcttatggcttctgtctaagcacctgaacagaggactgaaacctccactgc
aggctggttttaggtcttgaattatgtaagaatcttgcacagcactgctaatgtaaatttcagttgtttttccctctaggacaa
acacttaccaaaatatgcaactttttttggtgggaagagagattgtcctgtgatttctacccatttcctgaggcctgtggaaa
taaacctttatgtacttaaagttatacagaaaatagaataaagttaataccaaacttgaaaaagcggccgc
<212> Type : DNA
<211> Length : 2591
      SequenceName : L12168
      SequenceDescription :

Sequence
--------
<213> OrganismName : HUMAN
<400> PreSequenceString :
tggctggcaatggccttgctgacctcgagccgggcccacgtggggacctttggagcacagcctacgatcctggtgcaaggccgg
tggatgcagaggccagtccatataccacccaggcctgcgaggagcgtggtccccacccatccagcccatatgtgcaagtgccct
tgacagagaggctggtcatatccatggtgaccatttatggccacaacaggtccccatctgcgcagtgaaccctgtgctgagca
ccttgcagacgtgatcttgcttcgtcctgcagcactgtgcggggcaggaaaatccaagaggaagaaggatctacggatatcctg
catgtccaagccacccgcacccaaccccacacccccccggaacctggactcccggaccttcatcaccattggagacagaaactt
tgaggtggaggctgatgacttggtgaccatctcagaactgggccgtggagcctatggggtggtagagaaggtgcggcacgccca
gagcggcaccatcatggccgtgaagcggatccgggccaccgtgaactcacaggagcagaagcggctgctcatggacctggacat
caacatgcgcacggtcgactgtttctacactgtcaccttctacggggcactattcagagagggagacgtgtggatctgcatgga
gctcatggacacatccttggacaagttctaccggaaggtgctggataaaaacatgacaattccagaggacatccttggggagat
tgctgtgtctatcgtgcgggccctggagcatctgcacagcaagctgtcggtgatccacagagatgtgaagccctccaatgtcct
tatcaacaaggagggccatgtgaagatgtgtgactttggcatcagtggctacttggtggactctgtggccaagacgatggatgc
cggctgcaagccctacatggcccctgagaggatcaacccagagctgaaccagaagggctacaatgtcaagtccgacgtctggag
cctgggcatcaccatgattgagatggccatcctgcggttcccttacgagtcctggggggaccccgttccagcagctgaagcaggt
ggtggaggagccgtcccccagctcccagccgaccgtttctcccccgagtttgtggacttcactgctcagtgcctgaggaagaa
ccccgcagagcgtatgagctacctggagctgatggagcaccccttcttcaccttgcacaaaaccaagaagacggacattgctgc
cttcgtgaagaagatcctgggagaagactcataggggctgggcctcggaccccactccggccctccagagccccacagccccat
ctgcgggggcagtgctcacccacaccataagctactgccatcctggcccagggcatctgggaggaaccgagggggctgctccca
cctggctctgtggcgagccatttgtcccaagtgccaaagaagcagaccattggggctcccagccaggcccttgtcggccccacc
agtgcctctccctgctgctcctaggacccgtctccagctgctgagatcctggactgagggggcctggatgccccctgtggatgc
tgctgcccctgcacagcaggctgccagtgcctgggtggatgggccaccgccttgcccagcctggatgccatccaagttgtatat
ttttttaatctctcgactgaatggactttgcacactttggcccagggtggccacacctctatcccggctttggtgcggggtaca
caagaggggatgagttgtgtgaataccccaagactcccatgagggagatgccatgagccgcccaaggccttcccctggcactgg
caaacagggcctctgcggagcacactggctcacccagtcctgcccgccaccgttatcggtgtcattcacctttcgtgtttttt

taatttatcctctgttgatttttcttttgctttatgggtttggcttgtttttcttgcatggtttggagctgatcgcttctccc
ccaccccctagggg
<212> Type : DNA
<211> Length : 2030
SequenceName : L36719
SequenceDescription :

Sequence
--------
<213> OrganismName : HUMAN
<400> PreSequenceString :
cccaggcctcgcgctgcccggccggctcctcgtgtcccactcccggcgcacgccctcccgcgagtcccgggcccctcccgcgcc
cctcttctcggcgcgcgcgcagcatggcgcccccgcaggtcctcgcgttcgggcttctgcttgccgcggcgacggcgacttttg
ccgcagctcaggaagaatgtgtctgtgaaaactacaagctggccgtaaactgctttgtgaataataatcgtcaatgccagtgta
cttcagttggtgcacaaaatactgtcatttgctcaaagctggctgccaaatgtttggtgatgaaggcagaaatgaatggctcaa
aacttgggagaagagcaaaacctgaaggggccctccagaacaatgatgggctttatgatcctgactgcgatgagagcgggctct
ttaaggccaagcagtgcaacggcacctccacgtgctggtgtgtgaacactgctggggtcagaagaacagacaaggacactgaaa
taacctgctctgagcgagtgagaacctactggatcatcattgaactaaaacacaaagcaagagaaaaaccttatgatagtaaaa
gtttgcggactgcacttcagaaggagatcacaacgcgttatcaactggatccaaaatttatcacgagtattttgtatgagaata
atgttatcactattgatctggttcaaaattcttctcaaaaaactcagaatgatgtggacatagctgatgtggcttattattttg
aaaaagatgttaaaggtgaatccttgtttcattctaagaaaatggacctgacagtaaatggggaacaactggatctggatcctg
gtcaaactttaatttattatgttgatgaaaaagcacctgaattctcaatgcagggtctaaaagctggtgttattgctgttattg
tggttgtggtgatagcagttgttgctggaattgttgtgctggttatttccagaaagaagagaatggcaaagtatgagaaggctg
agataaaggagatgggtgagatgcatagggaactcaatgcataactatataatttgaagattatagaagaagggaaatagcaaa
tggacacaaattacaaatgtgtgtgcgtgggacgaagacatctttgaaggtcatgagtttgttagtttaacatcatacatttgt
aatagtgaaacctgtactcaaaatataagcagcttgaaactggctttaccaatcttgaaatttgaccacaagtgtcttatatat
gcagatctaatgtaaaatccagaacttggactccatcgttaaaattatttatgtgtaacattcaaatgtgtgcattaaatatgc
ttccacagtaaaatctgaaaaactgatttgtgattgaaagctgcctttctatttacttgagtcttgtacatacatactttttta
tgagctatgaaataaaacattttaaactg
<212> Type : DNA
<211> Length : 1457
SequenceName : M33011
SequenceDescription :

Sequence
--------
<213> OrganismName : HUMAN
<400> PreSequenceString :
ttggtacttggaaaaatggacaaggattgtgaaatgaaacgcaccacactggacagccctttggggaagctggagctgtctggt
tgtgagcagggtctgcacgaaataaagctcctgggcaaggggacgtctgcagctgatgccgtggaggtcccagcccccgctgcg
gttctcggaggtccggagcccctgatgcagtgcacagcctggctgaatgcctatttccaccagcccgaggctatcgaagagttc
cccgtgccggcacttcaccatcccgttttccagcaagagtcgttcaccagacaggtgttatggaagctgctgaaggttgtgaaa
ttcggagaagtgatttcttaccagcaattagcagccctggcaggcaaccccaaagccgcgcgagcagtgggaggagcaatgaga
ggcaatcctgtccccatcctcatcccgtgccacagagtggtctgcagcagcggagccgtgggcaactactccggaggactggcc
gtgaaggaatggcttctggcccatgaaggccaccggttggggaagccaggcttgggagggagctcaggtctggcaggggcctgg
ctcaagggagcgggagctacctcgggctccccgcctgctggccgaaactgagtatgtgcagtaggatggatgtttgagcgacac
acacgtgtaacactgcatcggatgcggggcgtggaggcaccgctgtattaaaggaagtggcagtgtcctggg
<212> Type : DNA
<211> Length : 744
SequenceName : M60761
SequenceDescription :

Sequence
--------
<213> OrganismName : HUMAN
<400> PreSequenceString :
gggcgaggggcggacaccggagagacacgggaaaggggtcgggacaggagcacgtggctcagacaccgacgccgggaggccgca
gaccccggacgtgtcaggcatccccgcaggcccggagcgatggcagccttgatgaccccgggaaccggggccccacccgcgcct
ggtgacttctccggggaagggagccagggacttcccgacccttcgccagagcccaagcagctcccggagctgatccgcatgaag
cgagacggaggccgcctgagcgaagcggacatcaggggcttcgtggccgctgtggtgaatgggagcgcgcagggcgcacagatc
ggggccatgctgatggccatccgacttcggggcatggatctggaggagacctcggtgctgacccaggccctggctcagtcggga
cagcagctggagtggccagaggcctggcgccagcagcttgtggacaagcattccacaggggggtgtgggtgacaaggtcagcctg
gtcctcgcacctgccctggcggcatgtggctgcaaggtgccaatgatcagcggacgtggtctggggcacacaggaggcaccttg

gataagctggagtctattcctggattcaatgtcatccagagcccagagcagatgcaagtgctgctggaccaggcgggctgctgt
atcgtgggtcagagtgagcagctggttcctgcggacggaatcctatatgcagccagagatgtgacagccaccgtggacagcctg
ccactcatcacagcctccattctcagtaagaaactcgtggaggggctgtccgctctggtggtggacgttaagttcggaggggcc
gccgtcttccccaaccaggagcaggcccgggagctggcaaagacgctggttggcgtgggagccagcctagggcttcgggtcgcg
gc[?]gcgctgaccgccatggacaagcccctgggtcgctgcgtgggccacgccctggaggtggaggaggcgctgctctgcatggac
ggcgcaggcccgccagacttaagggacctggtcaccacgctcgggggcgccctgctctggctcagcggacacgcggggactcag
gctcagggcgctgcccgggtggccgcggcgctggacgacggctcggcccttggccgcttcgagcggatgctggcggcgcagggc
gtggatcccggtctggcccgagccctgtgctcgggaagtcccgcagaacgccggcagctgctgcctcgcgcccgggagcaggag
gagctgctggcgcccgcagatggcaccgtggagctggtccgggcgctgccgctggcgctggtgctgcacgagctcggggccggg
cgcagccgcgctggggagccgctccgcctggggtgggcgcagagctgctggtcgacgtgggtcagaggctgcgccgtgggacc
ccctggctccgcgtgcaccgggacggccccgcgctcagcggcccgcagagccgcgccctgcaggaggcgctcgtactctccgac
cgcgcgccattcgccgcccctcgcccttcgcagagctcgttctgccgccgcagcaataaagctcctttgccgcg
<212> Type : DNA
<211> Length : 1587
      SequenceName : M63193
      SequenceDescription :

Sequence
--------
<213> OrganismName : HUMAN
<400> PreSequenceString :
gtgaagtgctcagaatggggcaggatgtcacctggaatcagcactaagtgattcagactttccttactttaaatgtgctgctc
ttcatttcaagatgccgttgcagctctgataaatgcaaactgacaaccttcaaggccacgacggagggaaaatcattggtgctt
ggagcatagaagactgcccttcacaaaggaaatccctgattattgtttgaaatgctgaggacgttgctgcgaaggagactttt
tcttatcccaccaaatactactttatggttcttgttttatccctaatcaccttctccgtttaaggattcatcaaaagcctgaa
tttgtaagtgtcagacacttggagcttgctggggagaatcctagtagtgatattaattgcaccaaagtttacagggtgatgta
aatgaaatccaaaaggtaaagcttgagatcctaacagtgaaatttaaaaagcgccctcggtggacacctgacgactatataaac
atgaccagtgactgttcttctttcatcaagagacgcaaatatattgtagaaccccttagtaaagaagaggcggagtttccaata
gcatattctatagtggttcatcacaagattgaaatgcttgacaggctgctgagggccatctatatgcctcagaatttctattgc
gttcatgtggacacaaaatccgaggattcctatttagctgcagtgatgggcatcgcttcctgttttagtaatgtctttgtggcc
agccgattggagagtgtggtttatgcatcgtggagccgggttcaggctgacctcaactgcatgaaggatctctatgcaatgagt
gcaaactggaagtacttgataaatctttgtggtatggattttcccattaaaaccaacctagaaattgtcaggaagctcaagttg
ttaatgggagaaaacaacctggaaacggagaggatgccatcccataaagaagaaaggtggaagaagcggtatgaggtcgttaat
ggaaagctgacaaacacagggactgtcaaaatgcttcctccactcgaaacacctctcttttctggcagtgcctacttcgtggtc
agtagggagtatgtggggtatgtactacagaatgaaaaaatccaaaagttgatggagtgggcacaagacacatacagccctgat
gagtatctctgggccaccatccaaaggattcctgaagtcccgggctcactccctgccagccataagtatgatctatctgacatg
caagcagttgccaggtttgtcaagtggcagtactttgagggtgatgtttccaagggtgctccctacccgccctgcgatggagtc
catgtgcgctcagtgtgcattttcggagctggtgacttgaactggatgctgcgcaaacaccacttgtttgccaataagtttgac
gtggatgttgacctctttgccatccagtgtttggatgagcatttgagacacaaagctttggagacattaaaacactgaccatta
cgggcaattttatgaacaagaagaaggatacacaaaacgtaccttatctgtttccccttccttgtcagcgtcgggaagatggta
tgaagtcctctttggggcagggactctagtagatcttcttgtcagagaagctgcatggtttctgcagagcacagttagctagaa
aggtgatagcattaaatgttcatctagagttaatagtgggaggagtaaaggtagccttgaggccagagcaggtagcaaggcatt
gtggaaagaggggaccagggtggctggggaagaggccgatgcataaagtcagcctgttccaagtgctcagggacttagcaaaat
gagaagatgtgacctgtgccaaaactattttgagaattttaaatgtgaccatttttctggtatgccaataaaddcttacagcaa
caaataatcaaagatacaattaatctgatattatatttgttgaaatagaaatttgattgtactataaatgattttgtaaataa
tttatattctgctctaatactgtactgtgtagtgtgtctccgtatgtcatctcagggagcttaaaatggcttgatttaacatt
gaaaaaaaat
<212> Type : DNA
<211> Length : 2110
      SequenceName : M97347
      SequenceDescription :

Sequence
--------
<213> OrganismName : HUMAN
<400> PreSequenceString :
ccgccagatttgaatcgcgggacccgttggcagaggtggcggcggcggcatgggtgccccgacgttgccccctgcctggcagcc
ctttctcaaggaccaccgcatctctacattcaagaactggcccttcttggagggctgcgcctgcaccccggagcggatggccga
ggctggcttcatccactgccccactgagaacgagccagacttggcccagtgtttcttctgcttcaaggagctggaaggctggga
gccagatgacgaccccatagaggaacataaaaagcattcgtccggttgcgctttcctttctgtcaagaagcagtttgaagaatt
aacccttggtgaattttgaaactggacagagaaagagccaagaacaaaattgcaaaggaaaccaacaataagaagaaagaatt
tgaggaaactgcgaagaaagtgcgccgtgccatcgagcagctggctgccatggattgaggcctctggccggagctgcctggtcc
cagagtggctgcaccacttccagggtttattccctggtgccaccagccttcctgtgggccccttagcaatgtcttaggaaagga

gatcaacattttcaaattagatgtttcaactgtgctcctgttttgtcttgaaagtggcaccagaggtgcttctgcctgtgcagc
gggtgctgctggtaacagtggctgcttctctctctctctcttttttgggggctcatttttgctgttttgattcccgggctta
ccaggtgagaagtgagggaggaagaaggcagtgtcccttttgctagagctgacagctttgttcgcgtgggcagagccttccaca
gtgaatgtgtctggacctcatgttgttgaggctgtcacagtcctgagtgtggacttggcaggtgcctgttgaatctgagctgca
ggttccttatctgtcacacctgtgcctcctcagaggacagtttttttgttgttgtgtttttttgtttttttttttggtagatg
catgacttgtgtgtgatgagagaatggagacagagtccctggctcctctactgtttaacaacatggctttcttattttgtttga
attgttaattcacagaatagcacaaactacaattaaaactaagcacaaagccattctaagtcattggggaaacggggtgaactt
caggtggatgaggagacagaatagagtgataggaagcgtctggcagatactccttttgccactgctgtgtgattagacaggccc
agtgagccgcggggcacatgctggccgctcctccctcagaaaaaggcagtggcctaaatcctttttaaatgacttggctcgatg
ctgtgggggactggctgggctgctgcaggccgtgtgtctgtcagcccaaccttcacatctgtcacgttctccacacgggggaga
gacgcagtccgcccaggtccccgctttctttggaggcagcagctcccgcagggctgaagtctggcgtaagatgatggatttgat
tcgccctcctccctgtcatagagctgcagggtggattgttacagcttcgctggaaacctctggaggtcatctcggctgttcctg
agaaataaaaagcctgtcatttc
<212> Type : DNA
<211> Length : 1619
SequenceName : NM_001168
SequenceDescription :

Sequence
--------
<213> OrganismName : HUMAN
<400> PreSequenceString :
gcttgtatataagattactttattcctgcatcttctcaatgggttcttccttgtatttgcccttttcctttccnacttggcgag
atttggctttccgttcgaggatcttttgcgggctttgtccagttttagcctagnganaaccaccttgctggggngaatgccta
cgngganagttgtgccattagccttttcccgntgcanccgttcaatgtagataacatatttcttccngtaaacctgggctactt
tgccaatttgctgaccnttatag
<212> Type : DNA
<211> Length : 275
SequenceName : R70234
SequenceDescription :

Sequence
--------
<213> OrganismName : HUMAN
<400> PreSequenceString :
ttctcctcggccaattcgcggaagaagtggctcacgccttccagagccacatcatcgcggtcgaaatagaagcccagagagagg
taggtgtaggaggcctgcaggtacaaattgaccaggctgttgacggctgcctccacgtcggtgaataattctgacgaatctggg
agctcatggttggttggcaagaaggagctaaccacaaaaacggtgctggcaggtcccagaagcaggagatggccgagaagatgg
tcccggagttgcaagcggagaggaaatcggagggcggtcggaggctggaagagagtccccggatctgttccgtccaaacacttt
ttgaagcaagagacagacccgcgggaccncctagtgccgaattnttggcttcg
<212> Type : DNA
<211> Length : 389
SequenceName : R81823
SequenceDescription :

Sequence
--------
<213> OrganismName : HUMAN
<400> PreSequenceString :
gccagggctcagtgggcaagggctctgtgccgtngncctgtacgactaccatgcagccgacgacacagagatctcctttgaccc
cgagaacctcatcacgggcatcgaggtgatcgacgaaggctggtggcgtggctatgggccggatcatntgttngcatgttccct
gccaactacgtggagctcattgagtgaggctgagggcacatcttgcccttcccctctcagacatggcttccttattgctggaag
aggaggcctggggagttgacattcagcactcttccagggaatagggacccccagttgaggattgaggcntcagggttccctccg
gnttgggcagattcagccttttcaccccaaatggcagcaattggcntgggtgatttcccacaaatcnttcctggcattccccg
acctttcccagacagtttggttt
<212> Type : DNA
<211> Length : 443
SequenceName : R82530
SequenceDescription :

Sequence
--------
<213> OrganismName : HUMAN

<400> PreSequenceString :
accatggcccctttgagcccctggcttctggcatcctgttgttgctgtggctgatagcccccagcagggcctgcacctgtgtc
ccaccccacccacagacggccttctgcaattccgacctcgtcatcagggccaagttcgtggggacaccagaagtcaaccagacc
accttataccagcgttatgagatcaagatgaccaagatgtataaagggttccaagccttaggggatgccgctgacatccggttc
gtctacacccccgccatggagagtgtctgcggatacttccacaggtcccacaaccgcagcgaggagtttctcattgctggaaaa
ctgcaggatggactcttgcacatcactacctgcagttttgtggctccctggaacagcctgagcttagctcagcgccggggcttc
accaagacctacactgttggctgtgaggaatgcacagtgtttccctgtttatccatcccctgcaaactgcagagtggcactcat
tgcttgtggacggaccagctcctccaaggctctgaaaagggcttccagtcccgtcaccttgcctgcctgcctcgggagccaggg
ctgtgcacctggcagtccctgcggtcccagatagcctgaatcctgccc
<212> Type : DNA
<211> Length : 636
SequenceName : S68252
SequenceDescription :

Sequence
--------
<213> OrganismName : HUMAN
<400> PreSequenceString :
gtttattcat ctgtttatga cacagtacac aggaggcaaa gtgttt 46
<212> Type : DNA
<211> Length : 46
SequenceName : T64335
SequenceDescription :

Sequence
--------
<213> OrganismName : HUMAN
<400> PreSequenceString :
natccatcagctcgttcaactttagcacctgtctcgnccccagtggcttttccagaactactgccttcaccatgaagcnccatg
agcttncccaattcaaacttgggctncttcagcatttttacttgnctaacgaagacancanggagagganaaatagantggcaa
gccttnncaangnctntaccaatgctgnctggaancaannnanngaccacnncnnacaagncaatagtctgnaccncncggg
<212> Type : DNA
<211> Length : 250
SequenceName : T80827
SequenceDescription :

Sequence
--------
<213> OrganismName : HUMAN
<400> PreSequenceString :
tccaaagaatttattatcaaaactaaaacaaaacttcagttgtacataaagtaataaactttacaacagctgttaagagaaagt
cgtgatgtaagtttatacatattaccgaagcaaagaggacaagcaaaggagagctccagttggaatgttgattacattcatgca
gcttcctcttgatttaaacactcatgacacattgaatgaaatcatcatatgggaaattcacaacaccttgctgagcagtatccc
gtcttcgaaagctgtctgtaagngccctcagtttgacgcagcaggngntgtagtcgtcgaaggtgatctttccattggngctgt
atcgttttgcaattggnttcacagcctgggg
<212> Type : DNA
<211> Length : 367
SequenceName : T87908
SequenceDescription :

Sequence
--------
<213> OrganismName : HUMAN
<400> PreSequenceString :
ggcacgagtggctgcttgcggctagtggatggtaattgcctgcctcgcgctagcagcaagctgctctgttaaaagcgaaaatga
aacaattgcctgcggcaacagttcgactcctttcaagttctcagatcatcacttcggtggtcagtgttgtaaaagagcttattg
aaaactccttggatgctggtgccacaagcgtagatgttaaactggagaactatggatttgataaaattgaggtgcgagataacg
gggagggtatcaaggctgttgatgcacctgtaatggcaatgaagtactacacctcaaaaataaatagtcatgaagatcttgaaa
atttgacaacttacggttttcgtggagaagccttggggtcaatttgttgtatagctgaggttttaattacaacaagaacggctg
ctgataattttagcacccagtatgttttagatggcagtggccacatactttctcagaaaccttcacatcttggtcaaggtacaa
ctgtaactgctttaagattatttaagaatctacctgtaagaaagcagttttactcaactgcaaaaaaatgtaaagatgaaataa
aaaagatccaagatctcctcatgagctttggtatccttaaacctgacttaaggattgtctttgtacataacaaggcagttattt
ggcagaaaagcagagtatcagatcacaagatggctctcatgtcagttctggggactgctgttatgaacaatatggaatcctttc

agtaccactctgaagaatctcagatttatctcagtggatttcttccaaagtgtgatgcagaccactctttcactagtctttcaa
caccagaaagaagtttcatcttcataaacagtcgaccagtacatcaaaaagatatcttaaagttaatccgacatcattacaatc
tgaaatgcctaaaggaatctactcgtttgtatcctgttttctttctgaaaatcgatgttcctacagctgatgttgatgtaaatt
taacaccagataaaagccaagtattattacaaaataaggaatctgttttaattgctcttgaaaatctgatgacgacttgttatg
gaccattacctagtacaaattcttatgaaaataataaaacagatgtttccgcagctgacatcgttcttagtaaaacagcagaaa
cagatgtgctttttaataaagtggaatcatctggaaagaattattcaaatgttgatacttcagtcattccattccaaaatgata
tgcataatgatgaatctggaaaaaacactgatgattgtttaaatcaccagataagtattggtgactttggttatggtcattgta
gtagtgaaatttctaacattgataaaaacactaagaatgcatttcaggacatttcaatgagtaatgtatcatgggagaactctc
agacggaatatagtaaaacttgttttataagttccgttaagcacacccagtcagaaaatggcaataaagaccatatagatgaga
gtggggaaaatgaggaagaagcaggtcttgaaaactcttcggaaatttctgcagatgagtggagcaggggaaatatacttaaaa
attcagtgggagagaatattgaacctgtgaaaattttagtgcctgaaaaaagtttaccatgtaaagtaagtaataataattatc
caatccctgaacaaatgaatcttaatgaagattcatgtaacaaaaaatcaaatgtaatagataataaatctggaaaagttacag
cttatgatttacttagcaatcgagtaatcaagaaacccatgtcagcaagtgctctttttgttcaagatcatcgtcctcagtttc
tcatagaaaatcctaagactagtttagaggatgcaacactacaaattgaagaactgtggaagacattgagtgaagaggaaaaac
tgaaatatgaagagaaggctactaaagacttggaacgatacaatagtcaaatgaagagagccattgaacaggagtcacaaatgt
cactaaaagatggcagaaaaaagataaaacccaccagcgcatggaatttggcccagaagcacaagttaaaaacctcattatcta
atcaaccaaaacttgatgaactccttcagtcccaaattgaaaaaagaaggagtcaaaatattaaaatggtacagatcccctttt
ctatgaaaaacttaaaaataaattttaagaaacaaaacaaagttgacttagaagagaaggatgaaccttgcttgatccacaatc
tcaggtttcctgatgcatggctaatgacatccaaaacagaggtaatgttattaaatccatatagagtagaagaagccctgctat
ttaaaagacttcttgagaatcataaacttcctgcagagccactggaaaagccaattatgttaacagagagtcttttaatggat
ctcattatttagacgttttatataaaatgacagcagatgaccaaagatacagtggatcaacttacctgtctgatcctcgtctta
cagcgaatggtttcaagataaaattgataccaggagtttcaattactgaaaattacttggaaatagaaggaatggctaattgtc
tcccattctatggagtagcagatttaaaagaaattcttaatgctatattaaacagaaatgcaaaggaagtttatgaatgtagac
ctcgcaaagtgataagttatttagagggagaagcagtgcgtctatccagacaattacccatgtacttatcaaaagaggacatcc
aagacattatctacagaatgaagcaccagtttggaaatgaaattaaagagtgtgttcatggtcgcccatttttcatcatttaa
cctatcttccagaaactacatgattaaatatgtttaagaagattagttaccattgaaattggttctgtcataaaacagcatgag
tctggttttaaattatctttgtattatgtgtcacatggttatttttaaatgaggattcactgacttgttttatattgaaaaa
agttccacgtattgtagaaaacgtaaataaactaataac
<212> Type : DNA
<211> Length : 3063
      SequenceName : U13695
      SequenceDescription :

Sequence
--------
<213> OrganismName : HUMAN
<400> PreSequenceString :
gaattcccctgtgttagacggtacaggtggaagaagactgctgtgtgcaggaggaatttgcagtgagacttcaaggaagagaat
aagctcgccacataaagaggaaacaaagagcaacccagagttcagacccaggaagcggccgggagggcaggagcgaatcgggcc
gccgccgccatggagctgagagtcgggaacaggtaccggctgggccggaagatcggcagcggctccttcggagacatctatctc
ggtacggacattgctgcaggagaagaggttgccatcaagcttgaatgtgtcaaaaccaaacaccctcagctccacattgagagc
aaaatctacaagatgatgcagggaggagtgggcatccccaccatcagatggtgtggggcagaggggggactacaacgtcatggtg
atggagctgctggggccaagcctggaggacctcttcaacttctgctccaggaaattcagcctcaaaaccgtcctgctgcttgct
gaccaaatgatcagtcgcatcgaatacattcattcaaagaacttcatccaccgggatgtgaagccagacaacttcctcatgggc
ctggggaagaagggcaacctggtgtacatcatcgacttcggcctggccaagaagtaccgggatgcacgcacccaccagcacatc
ccctatcgtgagaacaagaacctcacggggacggcgcggtacgcctccatcaacacgcaccttggaattgaacaatcccgaaga
gatgacttggagtctctgggctacgtgctaatgtacttcaacctgggctctctcccctggcaggggctgaaggctgccaccaag
agacagaaatacgaaaggattagcgagaagaaaatgtccacccccattgaagtgttgtgtaaaggctacccttccgaatttgcc
acatacctgaatttctgccgttccttgcgttttgacgacaagcctgactactcgtacctgcggcagcttttccggaatctgttc
catcgccagggcttctcctatgactacgtgttcgactggaacatgcttaaatttggtgccagccgggccgccgatgacgccgag
cgggagcgcagggaccgagaggagcggctgagacactcgcggaacccggctacccgcggcctcccttccacagactccggccgg
ctgcgggggacgcaggaagtggctccccccacacccctcacccctacctcacacacggctaacacctcccccggcccgtctcc
ggcatggagagagaacggaaagtgagtatgcggctgcaccgcggggcccccgtcaacatctcctcgtccgacctcacaggccga
caagatacctctcgcatgtccacctcacagattcctggtcgggtggcttccagtggtcttcagtctgtcgtgcaccgatgagaa
ctctccttattgctgtgaagggcagacaatgcatggctgatctactctgttaccaatggctttacctagtgacacgtcccccgg
tctaggatcgaaatgttaacaccgggagctctccaggccactcacccagcgacgctcgtgggggaaacatactaaacggacaga
ctccaagagctgccaccgctggggctgcactgcggcccccacgtgaactcggttgtaacggggctgggaagaaaagcagagag
agaattgcagagaatcagactcccttttccagggcctcagctccctccagtggtggccgccctgtactccctgacgattccactg
taactaccaatcttctacttggttaagacagttttgtatcattttgctaaaaattattggcttaaatctgtgtaaagaaaatct
gtctttttattgtttcttgtctgttttgcagtcttacaaaaaaaatgttgactaaggaattc
<212> Type : DNA
<211> Length : 1911
      SequenceName : U29171

SequenceDescription :

Sequence
--------
<213> OrganismName : HUMAN
<400> PreSequenceString :
tcgggagccgcggcttatggtgcagacatggccaagtccaagaaccacaccacacacaaccagtcccgaaaatggcacagaaat
ggtatcaagaaaccccgatcacaaagatacgaatctcttaagggggtggaccccaagttcttgaggaacatgcgctttgccaag
aagcacaacaaaaagggcctaaagaagatgcaggccaacaatgccaaggccatgagtgcacgtgccgaggctatcaaggccctc
gtaaagcccaaggaggttaagcccaagatcccaaagggtgtcagccgcaagctcgatcgacttgcctacattgcccaccccaag
cttgggaagcgtgctcgtgcccgtattgccaaggggctcaggctgtgccggccaaaggccaaggccaaggccaaggccaaggat
caaaccaaggcccaggctgcagccccagcttcagttccagctcaggctcccaaacgtacccaggcccctacaaaggcttcagag
tagatatctctgccaacatgaggacagaaggactggtgcgacccccaccccccgcccctgggctaccatctgcatggggctggg
gtcctcctgtgctatttgtacaaataaacctgaggcagga
<212> Type : DNA
<211> Length : 628
      SequenceName : U49083
      SequenceDescription :

Sequence
--------
<213> OrganismName : HUMAN
<400> PreSequenceString :
acccctgaagctctccaaggccgtgcataaggctgtgctgaccatcgacgagaaagggactgaagctgctggggccatgttttt
agaggccatacccatgtctatcccccccgaggtcaagttcaacaaaccctttgtcttcttaatgattgaacaaaataccaagtc
tcccctcttcatgggaaaagtggtgaatcccacccaaaaataactgcctctcgctcctcaacccctcccctccatccctggccc
cctccctggatgacattaaaga
<212> Type : DNA
<211> Length : 274
      SequenceName : V00496
      SequenceDescription :

Sequence
--------
<213> OrganismName : HUMAN
<400> PreSequenceString :
cggcccggccatggcggcccccgcccgtctcccgcgatctccgtttcggtctcggctccggctttttacgccccgcagaagaa
gttcggccctgtggtggccccaaagcccaaagtgaatcccttccggcccggggacagcgagcctcccccggcacccggggccca
gcgcgcacagatgggccgggtgggcgagattcccccgccgcccccggaagactttccccctgcctccacctccccttgctgggga
tggcgacgatgcagagggtgctctgggaggtgccttcccgccgcccctcccccgatcgaggaatcatttcccccctgcgcctct
ggaggaggagatcttcccttccccgccgcctcctccggaggaggagggagggcctgaggccccccataccgcccccaccacagcc
cagggagaaggtgagcagtattgatttggagatcgactctctgtcctcactgctggatgacatgaccaagaatgatcctttcaa
agcccgggtgtcatctggatatgtgcccccaccagtggccactccattcagttccaagtccagtaccaagcctgcagccgggg
cacagcaccccctgcctccttggaagtccccttccagctcccagcctctgccccaggttccggctccggctcagagccagacaca
gttccatgttcagccccagccccagcccaagcctcaggtccaactccatgtccagtcccagacccagcctgtgtctttggctaa
cacccagccccgagggcccccagcctcatctccggctccagcccctaagttttctccagtgactcctaagtttactcctgtggc
ttccaagttcagtcctggagccccaggtggatctgggtcacaaccaaatcaaaaattggggcaccccgaagctctttctgctgg
cacaggctcccctcaacctcccagcttcacctatgcccagcagagggagaagccccgagtgcaggagaagcagcaccccgtgcc
cccaccggctcagaaccaaaaccaggtgcgctcccctggggccccagggcccctgactctgaaggaggtggaggagctggagca
gctgacccagcagctaatgcaggacatggagcatcctcagaggcagaatgtggctgtcaacgaactctgcggccgatgccatca
acccctggcccgggcgcagccagccgtccgcgctctagggcagctgttccacatcgcctgcttcacctgccaccagtgtgcgca
gcagctccagggccagcagttctacagtctggagggggcgccgtactgcgagggctgttacactgacaccctggagaagtgtaa
cacctgcggggagcccatcactgaccgcatgctgagggccacgggcaaggcctatcacccgcactgcttcacctgtgtggtctg
cgcccgcccccctggagggcacctccttcatcgtggaccaggccaaccggccccactgtgtccccgactaccacaagcagtacgc
cccgaggtgctccgtctgctctgagcccatcatgcctgagcctggccgagatgagactgtgcgagtggtcgccctggacaagaa
cttccacatgaagtgttacaagtgtgaggactgcgggaagcccctgtcgattgaggcagatgacaatggctgcttcccccctgga
cggtcacgtgctctgtcggaagtgccacactgctagagcccagacctgagtgaggacaggccctcttcagaccgcagtccatgc
cccattgtggaccacccacactgagaccacctgcgcccacctcagttattgttttgatgtctagcccctcccatttccaacccc
tccctagcatcccaggtgccctgacccaggacccaacatggtctagggatgcaggatccccgccctggggtctggtcctcgccc
atcctgcaggattgcccaccgtcttccagacaccccacctgagggggcaccaggtttagtgctgctgctttcactgctgcac
ccgcgccctcggccggcccccgagcagcctttgtactctgcttgcggagggctgggagaccctccaggacattcccaccctcc
cccatgctgccaagttgtagctatagctacaaataaaaaaaaaccttgttttccaaaaaaaaaaa
<212> Type : DNA

<211> Length : 2166
SequenceName : X94991
SequenceDescription :

Sequence
--------
<213> OrganismName : HUMAN
<400> PreSequenceString :
gcacgctgaaggagccggcggaaccgggtggccatggggatgtgggcatcgctggacgctttgtgggagatgccggccgagaag
cgtatcttcggggccgtgctgctcttttcctggacagtgtatctttgggagaccttcctagcacagcggcagagaaggatatat
aaaacaacaactcatgtaccaccggagttaggacagatcatggattctgaaacatttgagaaatctcgactctatcaactggat
aaaagcactttcagcttctggtcaggactctattcagagactgaaggcactcttattcttctctttggaggaataccttatctc
tggagactttctggacggttctgtggttatgctggctttggaccagaatatgagatcactcagtccctggtgtttctgctgttg
gctacacttttcagtgcattgactggtttgccatggagtctttataatacttttgtgatagaagaaaaacatggcttcaatcaa
cagactttggggttcttcatgaaagatgcaatcaagaaatttgttgtgactcagtgtattttgttgcctgtgtcttcacttcta
ctttacattattaaaattggggggtgactatttttttatttatgcctggctgttcacattagttgtgtctctggttcttgtcaca
atctatgctgattatattgcccctttatttgacaaattcacacctctgcctgagggaaagcttaaagaagaaattgaagtaatg
gcaaagagtattgactttcctttgacgaaggtgtatgttgtggaaggatctaaacgctcttcccacagcaatgcttattttat
ggcttcttcaagaacaagcgaatagttttgtttgacactctactagaagagtactctgtactaaacaaagacatccaggaggat
tctggcatggaaccccgcaatgaggaagaagggaacagtgaagaaataaaagctaaagttaaaaataagaaacaaggatgtaaa
aatgaggaggtactcgctgtactaggccatgaactggggcactggaagttgggacatacagtcaaaaatatcattattagccag
atgaattctttcctgtgtttttttttatttgctgtattaattggtcgaaaggagctttttgctgcatttggttttatgatagc
caacccactcttattggactattgatcatcttccagtttatttttcaccttacaatgaggttctttctttttgcctaacagtc
ctaagccgcagatttgagtttcaagctgatgcatttgccaagaaacttgggaaggctaaagacttatattctgctttaatcaaa
cttaacaaagataacttgggattccctgtttctgactggttgttctcaatgtggcattattctcatcctccactgctagagaga
cttcaagctttgaaaactatgaagcaacactgagatgtccaggatctgtgactgaagacatttctgattatttctgtcctgggc
agcatgttccagctcttgatgttttaaactttttttagaagaaaaattaagtacagaaaagcccagatttaaatacatttaa
tatgtcattttaaaaatgatttaataattcatttcttaaaacactgaatgaattttgaagcttaatgttttaaaggcatagt
tttatctttgacatctaatttaccatcaagttgtaaaattatttggaaaaatacagaactcgttttatttgtatacttatatgg
aatctgcatgtgaggtgtttgagggcatatgtttgaaagagggagcatcaccacaggaatccttctgtgaggtggaaacagtgg
tcctgaatcattgtgctcacacctaacttgaaatctggtcttactttcatgctgttatgatttcacctggtgaatcagtgtttt
aaataagaaaggtaatagttggtaaggccaatgttatttaaatgaaagtagttagaaaaatgctctcctattctaccaaatttt
taatttctttcttcccctttcttgctacacagtgatcaagagtttctcatagtgctttgaagttagaaattatgtataggatat
tttaaatcattgagttttgtggggtttttttgtttgtttgtttcttttgtttttttggaaaatccgtgtctttatcttttttcc
cacgtggtagatatgatcccattggaggtaaattgtagcttcttctcattcatgcagtaaataatacatcctttcactcagcag
agatggccatattaaacacgttttgctatgttaaaagtggcagaacaggaaagacgaattaaaaataacatttttaagcgaca
taaggatgaaatactgatgaatctctgtgacattacagggaaaaaaatatagttttctatctctttcaagggcagaagagtttt
catttttattttgtaattttatctgtaagtcataaatattacttaatcaggcctgattctacttttgaaaattacagttcttg
aaatgcagataatgtttactttgaaaacaaatgtcatgaatgatttccagttttaaagctatatgtttcactgcttcatatct
ctgtccactttctgaatgagaacttatttgtgcctagagctctcactcactgataatgcttattaccttctgggcatttattc
caaagtgggatcaactgtacgcctttggtatctgaccataaagtctttcctccgctgacatttgggtgatgtcttcacatggaa
atataataaaaataaaaatctagtttaatactgcattatttattttcctaaggctaaagaggagcagtcctatgcttttattca
gcatcctttatctgtgacttcatgctctgataactgcctttccttccttctgtgcctttgaatacaaatttcagttctgcaaaa
gtgaaacattaaacattgccaacgca
<212> Type : DNA
<211> Length : 2966
SequenceName : Y13834
SequenceDescription :

Sequence
--------
<213> OrganismName : HUMAN
<400> PreSequenceString :
agaaaatccctaagacagatcttgccgtggactagcantacctgcaagtgctgccaatgggaactcaatttattcctgggaacc
taacgaggagagcccaggcctaggcaggaggctggaaccctcttggctaaggtgctgttcctgttcctgcaaggtctccagaac
ccctttggaaatggtgaaggaaccagcccaatagaagtacagagccagctgacaagtccttgtaaagctcactcctcagtcctt
ggcacagcatgttttgtcttctctcttggtatttcttctcccaactttaagccatttgccttggaatcatgattacaattt
tttcctttgcagatgccttcctgggggatactcctccccaccctaaagggtcgcctgcaacttaggcggattgggtctctctgc
tgtggcgttctctcttgagagaccctctgaatttagcacaaagtggcttctgtttcacagctgccacca
<212> Type : DNA
<211> Length : 490
SequenceName : AA043102
SequenceDescription :

Sequence
--------
<213> OrganismName : HUMAN
<400> PreSequenceString :
gtcactctgctgcggcacagacctgaattaacaactctagctagggctgacttcaaaaagcactttcgtttttaataaccaac
atcagctcagcaggcttcatttgggaaaagaaaccttgtcggattaccccgacattctccacctcctgggaggccagccattcc
caaatgccccaaggatcgaagaacggagacggtaagatgattaattgcactgcaggtttttgtttctgtatttttttccctcc
tttttttgggtttactttcacattaatgaggtatttaggaaagacaaatggaatctgataaaaggtaacgttatattgtttgtc
gaaacaacattttaaggatttcttgaagttatatggctctgtaggcatgtttcactttaagatgtgtttctgangtcatattag
gagaacgactggtgattatctatgggaagattaactatgaaaccatatgggaaatatccttaatacctgtaatataccttataa
ccctgaatagttacatgacttttgggaagtggtnagagaattgtatt
<212> Type : DNA
<211> Length : 551
      SequenceName : AA056652
      SequenceDescription :

Sequence
--------
<213> OrganismName : HUMAN
<400> PreSequenceString :
gtaatggggtccctcctntcctgccttttctggactgaggaaaatttcccatgaagacagtaaccaggaaaaattcagccacaa
ctattttcctatctcctgatgcttgaagatcggtgttacatatttgtcctactaggaaaccctacataatttattcattcaac
aaatacttattagagtacctctatgatctggacagtcttccaaggcattggtggacaaaatggacaaaagtctcttgcacctca
tgcaactcacatgcntataaaagta
<212> Type : DNA
<211> Length : 277
      SequenceName : AA121261
      SequenceDescription :

Sequence
--------
<213> OrganismName : HUMAN
<400> PreSequenceString :
tttgccggagatgtggtggtacagtgggagctattctgacatgtccactggaagttgtaaaaacacgactgcagtcatcttctg
tgacgctttatatttctgaagttcagctgaacaccatggctggagcagtgtcaaccgagtagtgtctcccggacctcttcattg
cctaaaggtgatcttggaaaaagaagggcctcgttccttgtttagaggactaggccccaatttagtgggggtagcccttccaga
gcaatatactttgctgcttattcaaactgcaaggaaaagttgaatgatgtatttgatcctgattctacccaagtacatatgatt
tcagctgcaatggcaggttttactgcaatcacagcaaccaaccccatttggcttataaagactcggttacagctttgatgcaag
gaaccgcggggaaaggcgaatgggtgcttttgaatgtgttccgtaagtgtatcagacagatggactaaaanggttttatagggg
catgtctgcctcatatgctggtatatcagan
<212> Type : DNA
<211> Length : 535
      SequenceName : AA160974
      SequenceDescription :

Sequence
--------
<213> OrganismName : HUMAN
<400> PreSequenceString :
ggaaggtcagagaacaccaaacagatttaacccaaataagatttccctaaggcatacaataatcaaactctcaaagttcaagga
caaagggaattctaataccagcaagagaaaagaagcaaataatatataaaggagttccaattcatttgtcaatnacattctcaa
cagaaaccattcagaactagatggagtgcaatgacattttcaaagtgctgaaagagaaaacacttccatacaagaatactctat
atagcaaagttatccttcaggtacgaaggagagataaagactttcccaaacaaaagctgagagaattc[?]ctagcaccaggccag
tcttacaagaaatgctaaagagagtttttctatttgaatgaaaanaaaaacactaataggaaaagaact
<212> Type : DNA
<211> Length : 406
      SequenceName : AA167461
      SequenceDescription :

Sequence
--------
<213> OrganismName : HUMAN

<400> PreSequenceString :
atgattcctcttgattcttttgttggccagagtgctgatggcaaaatggttcctataatccctggtggaaatagtatcccactc
acattttccaacaggaaggaatatgtggagagggccattgaatatcgacttcatgagatggacagacaggtggctgcagtccga
gaagggatgtcctggattgttcctgtgccgctgctgtccctcctcacagcaaaacaactggagcagatggtgtgtgggatgccc
gagatctctgtggaagtcttgaagaaagtggtgcggtaccgtgagtggatgagcagcatcagctggtgcagtggttctggcaca
cgctggaagagttctccaatgaggagcgggtgcttttcatgaggtttgtgtcaggaagatctcgactaccagccaacactgctg
acatttctcagagatttcaaatcatgaaggttgataggccttacgacagtctgcctacctcacagacctgcttcttccagctga
agctgccccccgtactcccagccagctggtcatggccgagcgcctgcctaattgcnaatcaacaactgccgct
<212> Type : DNA
<211> Length : 577
SequenceName : AA186542
SequenceDescription :

Sequence
--------
<213> OrganismName : HUMAN
<400> PreSequenceString :
cgagattgtgccactcgatccagcctgggcgacagagcgagactctgctcaagaaaaaaaaaaaaaagaaaataaaaaaaaga
ttttgataaattgataccaacaggaggcattgtagtaaggtggaaagaacattggattgggagttaggagagtcaggttctgat
gctggttctgccaccactcactttgttgttggttgattaattttaatttatgttttgagacagcgtctcactcccgtcgcccag
gctgcgagtgncgtacggtgttcacagctcactgcagattcaacttgggttcaggtgattcttccacttcagcctcccaagtag
ctgggactacaggagtgcaccaccacgcccagctaatttttgtatttagcagagacaaggtttcaccatgttgcccaggctg
atctcgaactcctgagctcaggtgatctgcctgcctt
<212> Type : DNA
<211> Length : 457
SequenceName : AA280754
SequenceDescription :

Sequence
--------
<213> OrganismName : HUMAN
<400> PreSequenceString :
caaatggggagcctgcgccttgtgttggcaggcaatgcagtcattttccttacaattcaagggtttttccttatatttggaaga
ggagatgattttagctgggagcagtggtagcactttattctgattacagtgcattgaatttcttagaactcatactatctgtat
acatgtgcacatgcggcattttactatgaaatttaatatgctgggtttttaatacctttatatatcatgttcactttaagaaa
gacttcataagtaggagatgagttttattctcagcaaatagacctgtcaaatttagattatgttactcaaattatgttacttgt
ttggctgttcatgtagtcacggtgctctcagaaaatatattaacacagtcttgtaggcagctgccaccttatgcagtgcatcga
aacctttttgcttggggatgtgcttggagaggcagataacgctgaagcaggcctctcatgacccaggaaggccggggtggatccc
ttttgtgttgtagtccatgcattaaaagtgtggcccacagaccaagagcctcaacatttcctagagccttattagaaatgcaga
atctgaagcccactctggacccaggacatttgatgagatccaangagttgtatgcncat
<212> Type : DNA
<211> Length : 647
SequenceName : AA149850
SequenceDescription :

Sequence
--------
<213> OrganismName : HUMAN
<400> PreSequenceString :
ctgagattagttcagatggtctaaccattgttctatatgtgcattttagttaatattgtgtattaaaggataagtcttaatgct
caaagtatgttaaaaatagatgtagtaaatcagtccctttgtgaatgtccttttgttagtttttaggaaggcctgtcctctggg
agtgacctttattagtccaccccttggagctagacatcctgtacttagtcacggggatggtggaagagggagaagaggaagggt
gaagggaagggctctttgctagtatctccatatctagacgatggttttagatgataaccacaggtctacaagagcgtttttagt
aaagtgcctgtgttcattgtggacaaagttattattttgcaacatctaagctttacgaatggggtgacaacttatgataaaaac
tagagctagtgaattagcctatttgtaaatacctttgttataattgataggatacatct
<212> Type : DNA
<211> Length : 479
SequenceName : AA237071
SequenceDescription :

Sequence
--------
<213> OrganismName : HUMAN

<400> PreSequenceString :
ctgtcaagctgttctttatttcagggagagggcaggggagtgtttgatttcggggtaggaatgtttctggttggagggcgcttt
atctcagggttggaatgtttctgattggaggtgtcatttgtggtttatggtcatgctgacagccattaggctgatttttgggg
gctggatttaggcggtttttaatcaaggggaacttaaaatgctgctgtttgtccaaaatgttgatgctcctgctttgtcaatcc
agaccctatagttataaaaggatgaggggcgacgtgctctttctggctacttcctg
<212> Type : DNA
<211> Length : 308
SequenceName : AA443719
SequenceDescription :

Sequence
--------
<213> OrganismName : HUMAN
<400> PreSequenceString :
cagatactgtgctgagtgtaataggctgcatcctgctgaggaaggagacttttgggcagagtcaagcatgttgggcctcaagat
cacctactttgcactgatggatggaaaggtgtatgacatcacagagtgggctggatgcagcgtgtaggtatctccccagatacc
cacagagtcccctatcacatctcatttggttctcggattccaggcaccagagggcggcagagagccaccccagatgcccctcct
gctgatcttcaggatttcttgagtcggatctttcaagtacccccagggcagatgccaatgggaacttctttgcagctcctcagc
ctgcccctggagccgctgcagcctctaagcccaacagcacagtacccaagggagaagccaaacctaagcggcggaagaaagtga
ggaggcccttccaacgttgatgccccttctctttcctcaaatcaatgtcagggagtcaaaagggctgtagcacaggatggagtt
tgatttatccctcctcccccaacacctaggaactgaatctttttcttttatttttgagatggagtcttgctctgttgcccag
<212> Type : DNA
<211> Length : 588
SequenceName : AA460131
SequenceDescription :

Sequence
--------
<213> OrganismName : HUMAN
<400> PreSequenceString :
tctatacggaatattaaacacattactgatatcccatataattctgaaggacaagccatagtggaaagaatgaatctctccctg
aaacagcagttgcagaagcaaaagggggaaaacaggtactacaggacaccccataagcaattgaatcgcattattaactttaaa
ttttttgagcctgcctaaaggccggatcttatcagcagctgaacagcatctacagaaaccagctgcaaagacagaagcagaaca
actggtttggtggagagacctgataacagaaagttgggaaataggtaaaataataacttggggtagaggttatgcttatgtttc
tccaggactgaaccatcaagacacctgaaaccttatcatgagcgaatgctgaggaagagattccgggagatcccgaggaccccc
ccagctgcagccatgtcaagactgatgctgaggaggaccccaactgtcacgagcaacacccgtcaaacacagccacccacctga
ggtcagatcaagaagctgtcacagatggc
<212> Type : DNA
<211> Length : 533
SequenceName : AA485961
SequenceDescription :

Sequence
--------
<213> OrganismName : HUMAN
<400> PreSequenceString :
tgtacggagaagaccttcaattatcattacaggatttaaatttaccctggcttttaaaggaatagggcacactttttttttttt
tttttttactatttctttttctttcttttgctctttgactccctctttgtctctctctgtctctttctctctctgactctc
tctctctccttctctctctctctccgtgtctctctcttagccattacaaacttggggccctggcaagggtgagtggggaatg
ggtcccacataactgcccattcgagagttatatacctaaattgggagagacaccagggataagactccctgggttataacctag
gtgcctaaggacacaacacagagcttccttagattcctttggagatacaacttgctagaggaaatg
<212> Type : DNA
<211> Length : 402
SequenceName : AA486141
SequenceDescription :

Sequence
--------
<213> OrganismName : HUMAN
<400> PreSequenceString :
gtcgacggacacagtggggaaacccagcaaggccactgtgcacaattccccttcccctgaggatagggcaggacccctcgggaa
tgagggtcttccagggaggagggagtgaccttgctaggttttatggctggctttgggagagaggagttctagtttctatgacct
gctgtgagaaaaagaattgtggtttctgggactcactttgcggcagaaaagtaggcaggaaccaggagggaaggacaaagacct

ggcttctgaggccttccagtctcctctgggtcagagtagtctgcatgccaaggtgccatactttcggggtttaatgagccctga
cagcaggtccttgcagatttcacaggcaacttgtgatcccagtaattgggtttcagtcccccaatttcagaggactttagcaaa
gcctggttga
<212> Type : DNA
<211> Length : 430
      SequenceName : AA508510
      SequenceDescription :

Sequence
--------
<213> OrganismName : HUMAN
<400> PreSequenceString :
tttttttttcacaagactagatggagaattaatgaagaagaaatagaaatgaggcttatttaaggtacaaaattcttgtatttg
taataacctagatttatagatataaaacaagtcttttaaagacttccctaaaatactcaatataaaacaggagttacttatcct
agatcatccttagttaggtacacatgattctctgtgcaactagctggtggacatgaataaaagctgctgggcctataatgtact
aaaacatttttctcccttcagcaagacaggtagggttgaaggctaaatgcatcagaggagtattttcttaggcattttggacaa
tctttttgcagtaggttccataggttttccccagcgaaagttgactcagaccagagtaactctgacacaagttgccaagaggga
aaaaatacttcatttgctagattacccaaatgtgtagcctcatttaattaccaaggtaagaatcatccccaaccacttttcagc
tgagaacacttgtcagagaatgtctctggatgggaatagatgtggtaccagctagcc
<212> Type : DNA
<211> Length : 561
      SequenceName : AA521213
      SequenceDescription :

Sequence
--------
<213> OrganismName : HUMAN
<400> PreSequenceString :
tctctgaccaccggtgcatgcagcccctgtcacataccgcctgcttgctcaaatcaatcatgaccctttcatgtgaaatcttta
gtattgtgagcccttaaaagggacggaaattgtgcatacgtggagctcggattttaaggcagtagctgcgtgatgctcccagct
gaataaagcccttccttctacaatttggtgtctgaggggttttgtctgcggctcgtcctgctacatttcttggttcctgaccag
gaaacgaggtaactgatggacagccgaggcagccccttaggcggcttaggctcccctgtggagcgtcactcgggctggcggagt
ccgctcagggatggtcg
<212> Type : DNA
<211> Length : 353
      SequenceName : AA526772
      SequenceDescription :

Sequence
--------
<213> OrganismName : HUMAN
<400> PreSequenceString :
ttaatgtccatttagtgaatttatttcattaggaagacagcaccatattgtactttcttcttcagctccgcttgttttcttcat
taatttatcctttgaattccacagctttgaaaccagccatggtacccaggaccacagctatcaccagtattaccaagcatagga
ttcccaaaattacagcaatgaggcgccaaggaggagatgcagcacacgatcctttctctgaaacaacagctatcctggtattgc
tttgagagtcgaagtgtaattgagtatatccatcttcatccaaattttctaaatcaggatgatattccattgttcttgagagcc
cctgaatagatatagcatttgggagctcttttctttctgctcctgagatgactgtctgtggacaaaagagaatctctgagtcaa
atcatgtgggctaggtacttaccggagt
<212> Type : DNA
<211> Length : 448
      SequenceName : AA535015
      SequenceDescription :

Sequence
--------
<213> OrganismName : HUMAN
<400> PreSequenceString :
aggggtcatttctantgcttcaggtttgtttgaaatccatggagcaacagtggttcccattgtgagtgtgctagccccggaga
agctgtcagccagcactaggaggcgctatgaaactcaggtacaaactcgacttcagacctcccttgccaacttacatcagaaaa
gcagtgaaattgaaattctggctgtggatctacccaaagaaacaatattacagtttttatcattagagtgggatgctgatgaac
aggcatttaacacaactgtgaagcagctgctgtcacgcctgccaaagcaaagatacctcaaattagtctgtgatgaaatttata
acatcaaagtagaaaaaaaggtgtctgtgctatttctgtacagctatagagatgactactacagaatcttattttaaccctaaa
gaactgtcgttaacctcattcaaacagacagaggcttatactggaataatggaatgttgtacattcaccataatttaaaattaa

atntaagaagaggctgggtgcagtggctcacacctttaatccagcactttgggaancnaggcaggaagactgttgaaccaggag
ttgagncagc
<212> Type : DNA
<211> Length : 598
      SequenceName : AA044653
      SequenceDescription :

Sequence
--------
<213> OrganismName : HUMAN
<400> PreSequenceString :
ctccttctttggaaactggtgttcaagataaagaaatgctgaaaaaatatggaaaatcatttagaaaaatgatttcattgtgcc
ttcaaaaagatccagaaaaaagaccaacagcagcagaactattaaggcacaaatttttccagaaagcaaagaataaagaatttc
ttcaagaaaaaacattgcagagagcaccaaccatttctgaaagagcaanaaaggttcggagagtaccaggttccagtgggcgtc
ttcataagacagaggatggaggctgggagtggagtgatgatgaatttgattgaaganagtgaggaagggaaagcagcaatttca
caactcaggtctccccngagtgngagantcaatatcaaattctgagctctttccaacaactgatcctgtgggtactttgctcca
agttccagaacagatctctgctcatctacc
<212> Type : DNA
<211> Length : 450
      SequenceName : AA047585
      SequenceDescription :

Sequence
--------
<213> OrganismName : HUMAN
<400> PreSequenceString :
agcccgtgtcacaggtatcaccccttcttgccctcagcccagctgctgtgcccctgccacccgcgccccctcagccccttgcg
cgtcgcatccaaggtcacttgtgctcgcagctccacctggagccgttgccactgctgctgctgcgcttccagtcagggtgggcc
gctggctcccactgggcgtcagtttggctcccaggccctgggcagtgcagcctctgggcccgtctgctgcgctgcgttgcntgg
ctgtgtgctgggctgtctttgctgtggggctgcagtgggggggggcggggtgtctggggacgcaggtgagtaggggagaaactg
gcagggtggtaagcttctgagcctccaggtaagtgcgtgcctgggagatgcacctgggagggacctcgctgccctcctgccgcc
tgcctcatcccttctctttccccttccagatccagaagatgaaagaaggaagtgagcat
<212> Type : DNA
<211> Length : 479
      SequenceName : AA176596
      SequenceDescription :

Sequence
--------
<213> OrganismName : HUMAN
<400> PreSequenceString :
agattccaagttcaggagaactcgaccgttggaatgccaagatgccttggaaacagcagcccgagccgaggnctctctcttgat
gcctccatgcattctcagctcagaatcctggatgaggagcatcccaagggaaagtaccatcatggcttgagtgctctgaagccc
atccggactacttccaaacaccagcacccagtggacaatgctgggctttttcctgtatgactttttcgtggctttcttctctg
gcccgtgtggccacaaagaagggggagctctcaatggaagacgtgtggtctctgtccaagcacgagtcttctgacgtgaactgc
agaaagacttagagaagactgtgggcaagaagagctgaatgaagtttgggccagaacgctgcnttccctgcgaagggtttgtgt
tggaatcttctgccgnaccaggctc
<212> Type : DNA
<211> Length : 445
      SequenceName : AA188256
      SequenceDescription :

Sequence
--------
<213> OrganismName : HUMAN
<400> PreSequenceString :
tagtagagactgggtttcgtcatgttggtcaggctggtcttgaactcctgacctcatgtgatccacccacctccgcctcccaa
agtgttgggattacaagcgtgaccactgcacccagcctgtaaaatattttaagagatttattctgagccaaatatgagtgaccg
tggcctgtgacacagccctcaggaggtcctgagaacatgtgcccaaggtggtcgggatacatcttggttttatgtattttagga
aggtatgagacatcaatcaaatacatataagaaatacattgatttggttcagaaaggcagaacaactcaaagcggggggcttcc
aggctataggtaaatttaaacatttctggttgacaattgtttgagtttgtctaaagacctgggatccatagaaaggaatgttc
agtttaaaagataaggnttat
<212> Type : DNA

<211> Length : 441
      SequenceName : AA188574
      SequenceDescription :

Sequence
--------
<213> OrganismName : HUMAN
<400> PreSequenceString :
atgcagtactgtaaggaagagggacctccagcttccacaaacaccatcttcagctgtatgaaagggacggttgtggtgaagttt
gtcaggcacggtaagcatgctgagtggcggggatcagaactctcctatctgaacctactgaggagcaaagcagcaattacatgg
gatcctgtggctctcccgttgcagaggccacaggaagataggatggaacgtgactggtctcctaaccaaggtgcactgagaagc
aatcaacgggtcggtcgtggccagtcctggggaggtctgagtggtggtctttgggataacctttggccttatggatttggactc
gaaattagaagagcctaccatttcagatgcaatcacttttggacatgcttttgcagacagtccttaatgctgaaaacacagaga
atgggtaattcaagaggcctttcttttaaaatagacttttgtgacccactaattgtaaggtattgcaaggtcactttgcgtgtg
tcataaagttgacttccttattggttgaaggtcacagaagtagtggtttgctttgat
<212> Type : DNA
<211> Length : 561
      SequenceName : AA279906
      SequenceDescription :

Sequence
--------
<213> OrganismName : HUMAN
<400> PreSequenceString :
atatttcagatataagatttcagttctcagtgagtctaagtgacagaaggaatggagaccctcttgggcctgcttatcctttgg
ctgcactgcaatgggtgagcagcaaacaggaggtgacgcagattcctgcagctctgagtgtcccagaaggagaaaacttggttc
tcaactgcagtttcactgatagcgctatttacaacctccagtggtttaggcaggaccctgggaaaggtctcacatctctgttgc
ttattcagtcaagtcagagagagcaaacaagtggaagacttaatgcctcgctggataaatcatcaggacgtagtactttataca
ttgcagcttctcagcctggtgactcagccacctacctctgtgctgtgagggacaatgacatgcgctttggagcagggaccagac
tgacagtaaaaccaaatatccagaaccctgaccctgccgtgtaccagctgagagactctaaatccagtgacaagtctgtctgcc
tattcaccgattttgattctcaaacaaatgtgtcccaagtaaggattctgatgtgtatatc
<212> Type : DNA
<211> Length : 565
      SequenceName : AA401046
      SequenceDescription :

Sequence
--------
<213> OrganismName : HUMAN
<400> PreSequenceString :
ttattagttcaagaaaaggcatacacatttattcaacatgtatacctggggagaaccagtcattaccccgatgttcattatttt
gatcaccacgatagagctgactactctgctatgtagtgcattttgggactgcacttatgtatgtttttgtttaacgtggacaaa
gacttacagataggtgcaaaaaataaatcctcttttgcaacccagaactcattgttcagtatgagttttgatacatataagaag
ggatattatgatacctgagacagttgattgatgggagtattgatagccataaaggttggttccaggccaggtgcaatgggtcaa
gcctgcaatccccacaccaaggtgggagtattgcttgaaaccaggagttcaagaccagcctaggcaagaaagtaagaaacacat
ctccacaaaaatttaaaaattggctgggtactggggcatgtgcttgtagttggagttactcaga
<212> Type : DNA
<211> Length : 484
      SequenceName : AA449040
      SequenceDescription :

Sequence
--------
<213> OrganismName : HUMAN
<400> PreSequenceString :
gcattaaggacttgaatgccaccttccagacagagaagatagggcacccccatgggctgcagatcacctacaggagagatggcc
acaccaggaacctgtttgtgtatcatgaaagtgggaaggagatagtggactggttcaatgccctccgtgcagcccgtctgcagt
acctaaaaatggcctttcctgaactcccagagtctgagctcgtgccattcctcaccaggaactacctcaaacaaggcttcatgg
aaaagactgggccaaagcagaaagaacctttcaagaaaaggtggttcgccctggattgccatgagcggaggctgctctattaca
agaacccactggatgccttcgagcagggccaggtttttcttgggaacaaggagcagggatatgaagcctacgaagacctgccca
aggcatccgaggaaatcgctggaaagccggactcaccattgtcaccccagagcggagatttgtcctcacttgccccagtgagaa
ggaacagcaggaatggctggaaagtttgcggggtgtcctgtccagccccttgacgcccctcaaccggct
<212> Type : DNA

<211> Length : 573
      SequenceName : AA625433
      SequenceDescription :

Sequence
--------
<213> OrganismName : HUMAN
<400> PreSequenceString :
ttctccccaaaatagccacattcttttatttgatgattcaatacattttgattcaaatagtcaccacataacctaggcacgat
attaagcattttacaagcaaaatattttactgattttcttttaaattgccagagtacaataagtgaactggttaagagacatac
ttctgtacataaaaatatccatgtatttatacaagtgtatggaacagagtttaagataataaaaccacaacatcacaataaata
ggatgtgttcctactgcacagcacagcacgctttatcaggaagctgccaagtctttttcaaggtgctttatgtttttctgagct
aggctcagatttccccaaaaaaggaataggttagtaatggctggatctgaatgtctactttcataagttgcttttttattttcat
ctccaataacaggaatggaagatatgataatttaattattttcaaaaagtctttgcaacttaccaattctattccaattcagtt
ttttaaaatgcagattaatctaactctagcaccatacgagaatgaaatacatgtatttttgt
<212> Type : DNA
<211> Length : 566
      SequenceName : AA631149
      SequenceDescription :

Sequence
--------
<213> OrganismName : HUMAN
<400> PreSequenceString :
accacgaggtggaggctgctgtgagccaagattgcaccactgcactccagcctgggcaacagggtgagaccgtgtctctagaaa
aaacaaaagccaaaaggatggcatttggtattaacaagatcttaacaagatctggggagttgggctatgggaacacacacctgg
ggggcctaagaccttccagagaccatggtgcttgagtaaagctgaaaggagctcgcaggcccagacaagtggtagtgtgtgtgc
gcttggttgcgagacaccatgtgcacaaacctggaggcacggtcgtcttgtgagagtgtggaggtcagcttggctcattagaaa
ccagggccatggccaggcgcggtggctcatgcctgtaatccg
<212> Type : DNA
<211> Length : 378
      SequenceName : AA634241
      SequenceDescription :

Sequence
--------
<213> OrganismName : HUMAN
<400> PreSequenceString :
gacagacacttcataagctttagatttagagaatttcacagcataaagctcatcgctgatgcagtaatgaaggaaatgttctac
aagttaatagatgagtgagagaagagggcattcatattagatttatttacttactgaactcagagccttgggttactgcttgac
ccccactgttatctagtttaagggtccttttatttttttgtttttttgaggtggagtttcactcttgttccccaggctggagtgc
agtggcccggtctctgctcactgcaatctccgcctcccagattctcctgtctcagcctcccaagtagctgggattataggtacc
caccaccacgccctgctagtttttgtatttttagtagagacagggtttcaccatgttggtcaggctggtctcgaactcctgacc
tcaggcagtctgcccacctcagcactttggga
<212> Type : DNA
<211> Length : 452
      SequenceName : AA652121
      SequenceDescription :

Sequence
--------
<213> OrganismName : HUMAN
<400> PreSequenceString :
ggttgctcagactgacacatgtagactaaatagaaacccctcagccctaaaatagaaaaaagaacagttctggactattgaaca
gcccattcatttatttctgttttgtttaccgacatattcagtattattcttgttttctgaattctgagtctcctgaacaaaagg
cttactatccatggtcttggaaaagattgtcccttctcttgctgttgtaatgcatctctgctacagcagtcattgtcatcctgt
gaccttatccctttttgtctcccatcaccttttccttcttcagcagcacctcacagtcagtcagacgctgcaggcaccactatca
ccaggagtggccaagtaaaccgagacaccagtaacagcagggctggagcttcctcagcgttttgggaaggaaagagatgttccc
ctg
<212> Type : DNA
<211> Length : 423
      SequenceName : AA682896
      SequenceDescription :

Sequence
--------
<213> OrganismName : HUMAN
<400> PreSequenceString :
tttttttttgaaggttaaattgcattatttcttttattttatgatttttttaataccaaaaaaaagagacaagattttgccat
gttgccgaggctggtctcaaactcctgacctcaactgatcctcccgtctcggcctcccaaagtgctgggatgacaggcgtgcac
tgccctgccttgtgctttatttctaaacacattttcctaatgtatttttctgatgactctcaaggggaggatggctgttttgg
ctgggtgtggtggtgcacacctgtagtcccagctactcaggaggctgagcggggaggatcgcttgagcccaggagtttgaggct
gcagtgagctatgatggtgccactgttctccagcccaggcaacagagcaaggccctgtctctaaaaaaataaatattataaaca
aaaaccaaaacaaaacaaaaacaaggaagagcaactgcatagctgcctgacaaaacgagagcgtggctcagccccacggccctg
cgt
<212> Type : DNA
<211> Length : 507
      SequenceName : AA714520
      SequenceDescription :

Sequence
--------
<213> OrganismName : HUMAN
<400> PreSequenceString :
ttttttttttagtgtcctctaattttattgatgcttcagttttcataaagtgcaataaacaacataaatagatgaaaaaagctt
tgaagatttatatacagttttgaggttaagataaattactgactatattcctttcagccttttaactctgtgaaagctgtaacg
tacattaaaagcacattgaactaggttaaataatgatctttccccttagatcaatctagtattaaggagtatataattatgca
agttcattctataacacgaggctagactaaaaggacaattttgtgctacagactaaatccagatacggtcaggtgctgagcag
tattcctggcttcaaaataggaggtatctgtttcatcatctggctgcgcgggatgaaaggcatagtctgatgctgcagattttc
ccagtccacatcactgaagagaggatgacgttttagctctttcattccagctctctttgtatcatcaatggttaaaagtatttc
tactgcactttgaggcattatcagataacttttcttcaccttctggccagggatatctcttttcagaatattctggaatacttg
ttgtggtgtttcatcattgaaaggggaattcctgttagaaattccaaccagcaaacttccagtggcca
<212> Type : DNA
<211> Length : 657
      SequenceName : AA745592
      SequenceDescription :

Sequence
--------
<213> OrganismName : HUMAN
<400> PreSequenceString :
cattgtcactgtatctgtcagaatcattacttccattctgccttgtattttctgctgaagaagaaattgttggcagaggagtag
aagatcttgattctgttccttgttccctcagtttcaaaagctttttctcatatagcttcctggttgttcccacaataggaccag
gattcactccgtatttcacaagctgatccaaaagatcttcattagtgagctctgttacatctagatcatctttatcttcttgtc
tgggtttatcagttttattcgtggctttcctgccgacggctgctcggctccggcccgcggggcgctcccagagccgaggaccgg
ggtgggctcgcgctcttcgtcactggagaagtccgggggccccttgctgttggtgccggcggggagcggcggcctggttgcgag
ccgtgaggtgctgcaggtagagctggacgtacacgtctttgcgctgctccccggccggcagcgtcacattgttggcgaccaact
cactct
<212> Type : DNA
<211> Length : 510
      SequenceName : AA777347
      SequenceDescription :

Sequence
--------
<213> OrganismName : HUMAN
<400> PreSequenceString :
ggaaatacaagggacctagaatagccaaaacaacctagaaaaaacaaacttaggactctcgctttcacaatttcctacaaagca
acagaaatcaaggcaatgtggtattggcataagaagatattatagatcattagaatacaattaagaatctataaataaacccat
acctctatagttgattttgacaatgggaaaaaaatcctttccataaatggtgctctgacaattgaatagccacgttagaagaa
cgaagtcagatctctctttcatatcatatatgaaaattaactcaaaatggatcaaagacctaaatgtaggagatataactataa
aattcttaaaacttagtcgtaaatattcataaccctggattaggcacaagcaaccaaagaaaaaataagttggacttaaaaaca
gtgtttcaaaggacattatggagaaagtgaaatgattcagagtccgtgtgattgtaatcacatgctgacaacactcagtctaag
tcagacttctgttgtattctcaccactcaatttgtgcttttt
<212> Type : DNA
<211> Length : 546

SequenceName : AA779424
      SequenceDescription :

Sequence
--------
<213> OrganismName : HUMAN
<400> PreSequenceString :
tttttttttaggagagacaggatttcaccatgttggccaggctggtctcaaactcttgacctcaagtgatctgcctgccttgg
cctcccaaagtgccaggaatacaggtatgagccactgcaaccatccacatttttatttttgtactatatactggggttctact
accaaaaaaaggaactactagcttataaagtttctttcaccatttaacattctatctctcacacatcaataccactcaggaaat
aggaagaaacgttgaagtgccatcaagcaagtgttacctgtgaatctgatcttgcctgcttgaccgtactggagccagaccatc
aatttcgtcaaaaaaaataattgatgggcgcatctgataggcctgatcaaacagcaatcgtagctgtctttcagattctcctac
ccatttacttagacaatc
<212> Type : DNA
<211> Length : 438
      SequenceName : AA828809
      SequenceDescription :

Sequence
--------
<213> OrganismName : HUMAN
<400> PreSequenceString :
attgccacaaagtttaacttcttcattcacaaccttgctcagcttcgtttttctggtcttccttctaatgatgagcccatcctt
tcattttcacctaaaacatactcctttagacaagatggtcgaatcaaggaagtctctgtttttacatatcataagaaatacaac
ccagataaacattatatttatgtagtccgaattttgagggaaggacagattgaaccatcatttgtcttccgaacatttgacgaa
tttcaggaacttcacaataagctcagtattattttccactttggaagttaccaggctttcctaataggatggttctaggaaga
acacacataaaagatgtagcagccaaaaggaaaattgagttaaacagttacttacagagtttgatgaatgcttccacggatgta
gcagagtgtgatcctgtttgtacctttcttcaccctttacttcgtgatgagaaagctgaggga
<212> Type : DNA
<211> Length : 483
      SequenceName : AA504415
      SequenceDescription :

Sequence
--------
<213> OrganismName : HUMAN
<400> PreSequenceString :
ggtcgacaaaagggtagcagtttattcatctgtgataggaaaataatacttatcttacaaggttgcaagagctcaaggagacca
tgtatgtaaagttcctgctgtaaatatgaactcccatcctaatacccttttacctctctgtgggtttgtcttgacctggaaatt
tgggctaaaacttagaaaaaattcttacatgataactcagtgatgcttactcatagtttttggtgtttctcatagataagatat
aaatcagctgggcgcggtggctcatgcctgtaatcccagcactttgggaggccgaggcgggcagatcacctggggtcgggaggt
cgagaccagcctgaccaacatggagaaacccgtctctactaaaatgcaaagttggctgggcgtggtggctcatgctgtaatccc
agctacttgggaagctgaggcaggagagttcgttgaacccaggaagccgaggtttatgttagcgaaatcgtgcatt
<212> Type : DNA
<211> Length : 496
      SequenceName : AA526337
      SequenceDescription :

Sequence
--------
<213> OrganismName : HUMAN
<400> PreSequenceString :
gaccatctctactaaaaatacaaaaattagctgggtgtggcggcacacgctgtagtcccagctacttgggaggctgaggcggga
gaatcacttgaacctgggaggcggaggttgcagtgagccaagattgcaccgctgcactccagcctggcaacagagcaagactcc
atctcaaaacaaacatacaaacaaaaaaaggtgcctctggtcctgtgtttctttttccttctgaaagttcatagaaacctggc
atatagagcttatttgctgttttaactggttggctccacaaatcctgctacctgacatcagtacagcttgaccgcagtcaagt
acttgatggcttgttcatgtttagttcacctctttagaaagaaagctttgtggccaggtgcagtggctcacgcctgtaatccca
acaccttgggcagctgaggtgagcagatcacctgaggtcaggagtt
<212> Type : DNA
<211> Length : 466
      SequenceName : AA578089
      SequenceDescription :

Sequence
--------
<213> OrganismName : HUMAN
<400> PreSequenceString :
ccaagccctttaatattcacttaaacagtttaggcacaagcaataataaaactgcatttttacgttacaaaaagattttgtat
gttatctgcatgatataccaattttctcaatctcttctgcaataacatctcattaacactctggtccacatgttgcactaatac
attattttccatttccaaagtttttagtaagtgtgcaacattagcacatggaaacgctcaagctgtgtattagttcactgaaa
tctgggtttttcgttctcacctgctgagcaccatttggccagcatcactacaacaagctgattacatcaccttgtaaaactt
aagatggggatgaatagtgtttggcaattacatttctgggagaaaaactgctgatttttctttaactcgacaca
<212> Type : DNA
<211> Length : 412
      SequenceName : AA682625
      SequenceDescription :

Sequence
--------
<213> OrganismName : HUMAN
<400> PreSequenceString :
tttttttttttattgagaaaagcacacacatttatttactgtaagttttacatgacaggaatcttcagaaatgaagacaaaaaa
gaaataggaaaacttgtgtatttttatgtacagtcttatgcagaagtataatcagaggacaaaaggacataatctaatggtaat
aaactgtggagaacttagcaagtcctatttgtccagattcttcttggtgtctctgtatctgtgaggataaagggtacctatgga
atgaaggttttatgatctacttcagggaacagtcacttagggtttatggcctgcttcaggggagaaggggtaaggggaaggtat
gagaaaccttgcttctgcagttttctcaaataacagggtgcaatatttggggttatgtgtcctgaatcccatcaacgttaaga
tggagagaccagcccaaatctttagttggattccagctaagtcttatacacctaagggacagaggagtagtcatatatatgc
<212> Type : DNA
<211> Length : 502
      SequenceName : AA744682
      SequenceDescription :

Sequence
--------
<213> OrganismName : HUMAN
<400> PreSequenceString :
gggtaacttcctgatgttgccatgacatttgtagactgtcatggcactgatgggagtacctcttagcatgctaataccttataa
ttagcatataatgagtagtgaggacgaccagaggcgactttcatcgccatcttggctttggtgggttttggcagccttctttac
taatgctgttttatcaggaaggtctttgtgaccttgtatcttgtgccgacctcctatctcatcttgtgacttagaatgcct
<212> Type : DNA
<211> Length : 249
      SequenceName : AA773114
      SequenceDescription :

Sequence
--------
<213> OrganismName : HUMAN
<400> PreSequenceString :
tttttttttttttttttttttttttttaaaaagtgcatattctttaatccaggaattactcaagaaatttatacatttttta
aaaagatcaccaaatgcatcccacatagttcctttacaataaacaaaagataaacaacctagcgtcctgtcctatatgcgctct
accagatgactcaattatattccatccatccatggaatcctagacaatcataaaaggacatttatgttaacatggaaagttaag
atacgctttgaagaataggctacaagagagaatacattacacaatcctactgctgtataaaacaaaacatctggaagaatctat
gcttaaatattaatagtgtgtcccgcaaagtggtaggataatagtgacttttattctttttacttatctctattttctcatgtt
tctgtaatgaacatgtttcataaaacaaaatgggtctgttataaaaatctgaaaacttgttttgaaagagagcagtattacag
gctgtagtg
<212> Type : DNA
<211> Length : 513
      SequenceName : AA805691
      SequenceDescription :

Sequence
--------
<213> OrganismName : HUMAN
<400> PreSequenceString :
catcaatgcgtacgggattaatccccgcttcagcagccatttcatgttgggcttcttcgctgatctcttcattgttcatcaaac
gggtgagcagtaactggaagtagtgggccagcggtgtgcgttcacctctgcgacaggctctgcgcattcggtcgcgtactcatc

ggcaatatcaaaatatttagcgggatatcgtggttcattatttgcccctggggttaatgcgctaagcataagggcggatgata
gcactcgtgatccgggtcatacatcttttatagttaatggcgcggcgcgtntaccctcaggcaggataatgcgccggcatccg
actattacgtttcactcaccggcgttctgtcaccatcatacaacggcactttgcgatagatgcggatcagataccataaatatg
cgccgccta
<212> Type : DNA
<211> Length : 429
      SequenceName : AA812265
      SequenceDescription :

Sequence
--------
<213> OrganismName : HUMAN
<400> PreSequenceString :
gtagcccggctgaagaacaacaacagacaagtgtgcattgacccgaagctaaagtggattcaggagtacctggagaaagcttta
aacaagtaagcacaacagccaaaaaggactttccgctagacccactcgaggaaaactaaaaccttgtgagagatgaaagggcaa
agacgtgggggagggggccttaaccatgaggaccaggtgtgtgtggggtgggcacattgatctgggatcgggcctgaggttt
gccagcatttagaccctgcatttatagcatacggtatgatattgcagcttatattcatccatgccctgtacctgtgcacgttgg
aactttttattactggggtttttctaagagagaaattgtattatcaacagcattttcaagcagttagttccttcatgatcatcac
aatcatcatcattctcatt
<212> Type : DNA
<211> Length : 439
      SequenceName : AA442810
      SequenceDescription :

Sequence
--------
<213> OrganismName : HUMAN
<400> PreSequenceString :
ccctgtttgcaacaaagtatgctttaaaatgtaaactgtggagccattttccttgcgttgtccagaaggaacttcgtcctgcgt
gacgctggattaatcatgagagagctcgtcaacattccactggtacatattcttactctggttgccttcagcggaactgagaaa
cttccaaaagctcctgtcatcaccactcctcttgaaacagtggatgccttagttgaagaagtggctactttcatgtgtgcagtg
gaatcctaccccccagcctgagatttcctggactagaaataaaattctcattaaactctttgacacccggtacagcatccgggag
aatgggcagctcctcaccatcctgagtgtggaagacagtgatgatggcatttactgctgcacggccaacaatggtgtaggagga
gctgtggagagttgtggagccctgcaagtgaagatgaaacctaaaataactcgtcctcccataaatgtggaaataatagaggga
ttaaaagcagtcctaccatgtactacaatgggtaatcccaaaccatcagtgtct
<212> Type : DNA
<211> Length : 558
      SequenceName : AA448972
      SequenceDescription :

Sequence
--------
<213> OrganismName : HUMAN
<400> PreSequenceString :
aattcggcagaggccccagaatgcacctttcagggcctccccaggctgagcaggaaacagggtcgcccccaccctcacccccgg
cgatgttgcgggccccaaaggcaggggcacttggctgttctgaatggcggatcgatgctgaggacccagaactcgctcaggatg
cagccgaaagccaggtgtgtgtactgggagtcctgcagcgacaccacccacgggaactccatgctgctgaccaagccctccttg
gggtcaggaccgtagaaaacggaagctttctggacgccacaacctgcggacgtgncnnggggcctcactgccggggcaaggaga
cctcaggaccctctctcctgtgggctgtctgtctcgatcatcactaacgctatccgcaacccctttttctttcttttgcctagtg
tgtcttcttcaccctaaaagatgctcagtggcatttttgaatcccaaaattatgttagact
<212> Type : DNA
<211> Length : 481
      SequenceName : AA460392
      SequenceDescription :

Sequence
--------
<213> OrganismName : HUMAN
<400> PreSequenceString :
ctctctcctgccatgtaaggttttgctaagaaatatgctgctagccttattggaactctcatatatgtgatttatttctattc
tcttgctgcttttaggattctctctttgtctttgatttttgatagtttgattataatacattttgttatagtcttgtttggata
gaatctgattggagacatttggtccttataccttgatattattcccca
<212> Type : DNA

<211> Length : 216
      SequenceName : AA489975
      SequenceDescription :

Sequence
--------
<213> OrganismName : HUMAN
<400> PreSequenceString :
gtagagacagggtctctttatgttgctcagacagtgagggttctctgtctttccactcacctccccttgttgcctcttcctcc
ccacgtcccacccccatgcatcttcccacccaggccagcagacctgtcaggatggactggtcaacccgcagcgtggtggatttg
atggcgaggattcggatgtctgcagggactttgtcccacactgtggagagagtagggaagagggaggtcacttgggaatggaat
tttcttccttccaaagaaaaacccaatctgtgtcttgactcgttctcctcatccatccttcaggtctcagggaggctcccagg
<212> Type : DNA
<211> Length : 335
      SequenceName : AA514978
      SequenceDescription :

Sequence
--------
<213> OrganismName : HUMAN
<400> PreSequenceString :
ttaataatgctattattaggagtattattattcgattcatatgtttactgttcaacaaatattgaatgataaacatatatgctg
ggtccggcatggtggcccatgcctgtaattccagcactttgggaggccaaggcgggcaggtcacttgaggtcaggagtttgaga
ccagcctggccaacgtggtggaaactccgtctgtgctaaaaatacaaaaattagccgggcatggtggtgggtgcctgtaatccc
agctactcgggaggctgagacgggagaatcacttgagcccaggagctggagtgcagtggtg
<212> Type : DNA
<211> Length : 313
      SequenceName : AA516206
      SequenceDescription :

Sequence
--------
<213> OrganismName : HUMAN
<400> PreSequenceString :
atctctgcccttacgtcgttctctatcccgagaccgactacgcgttcttctgtgactggacctttcactgctacgactatgaga
acggagacgaggtcttgacctggaccttcttgttctgctttttgacctagacctctgaactctataggatttccctcgacccct
tgaacgacttcgacttcgtttccttctggagccataagaagagctactgcttgatcgatgcctgcgtcttctatcataagaatg
tgaacgaggctgaagatctctggaccaagatcttgactttgatcttgatttccttcctcctttctttcggctgtatgttctact
atctgaagaactgctcgaggaggaccgtctacggtgtttctttttctcttgcttctgctttcttcttcagtatctgatgaccg
acgtcccatttctggggtcagacccttgggctcaaacttccgttgacgtgcaaaccccgagtccctgattcgttcttaaatcaa
ggggccccgt
<212> Type : DNA
<211> Length : 514
      SequenceName : AA527334
      SequenceDescription :

Sequence
--------
<213> OrganismName : HUMAN
<400> PreSequenceString :
ttagagtagttttagcaaaattgaatggaaggtacggagattcccatgtaccccctgtctcctccatcatcaacattctgcacca
gagcagtgcatttattacaactgatgaacctatttgatgcatcattgtcactcagagtccatagtttacagtagggttcactct
tggtgttgtacattctgtggatttggacgaatgtatagtgacgcatatccaccattattgtatcatacagaacagtttcacagc
cctaaaagtggggagattggcctcagttcgcctacctaagagactaaatgacttacccatatatggttgcctccatatgaagag
cagtgagaaccgccggccgctggtgtgtctcaaatgcctgtgaacagtgaaatcctcacttggatggtgg
<212> Type : DNA
<211> Length : 406
      SequenceName : AA552028
      SequenceDescription :

Sequence
--------
<213> OrganismName : HUMAN

<400> PreSequenceString :
tttttttcctaaataattcagtatttatttccttagaacaaggacaaatttgcataaccacagtagggttgtcaaaatcagcaa
atctaatattgatacactatacaatcctatgaccaatctcatctacaactttattcaaattttatagaggctgggattacaagc
atgaaccactctatccagctgattccatgttctctaacaccctatccacacaaatctgagatgggctcaaggatgaccacaggc
cttggagatctctgcagtgagcatctgtgcggctgaatctgagtaaaggattctgcttcaggctgctggccagcaaggtgtctc
aggagcaggctggtgcagggcagctatggagagacattagctctgcctgcacttacagcctgaggaccaagcaggctctggagg
cagagaaaggcctcagcaaagaagggcaggtcgacctgcaaggaagagtgagtggagggaatggggcggggcaccacagcctct
gctaaggtctcatggcacttagcagggtcctcagcgtctgctaagagctcaggaaatggtacctgctctcattcagaccgtttc
catcgt
<212> Type : DNA
<211> Length : 594
      SequenceName : AA576785
      SequenceDescription :

Sequence
--------
<213> OrganismName : HUMAN
<400> PreSequenceString :
tttcttggcttcacaaaacccacaccactacttcatggatggtatagccattaaccctgaggcttctgtcacacaaagcaaata
gccattcttctgtgctctcataatgttcaaccattcctttacttagagaattccagaaactgaccttaggagagctaaacatc
aaacaaggttgcgagtgtccttccttgggaagaaaaactgaaaaattgattactgcttcgctgcccccaccagaccaccaggtg
gttcattactcaagagaaccatcacaaccagacatgctgacctgcatcccctacccttcacatgctttgcccagcccagcctgc
atacactagtcttgaaatcaattcccatgttttgtctaattaaaaaaaaaaaaatccctaatggcgtttctttgggagctagc
cagaggatctttctcaccgttgctgtctcccttgcacttgagcataagccccataataaaagtcttatctggaaaatctgcttg
gccatgagtt
<212> Type : DNA
<211> Length : 514
      SequenceName : AA621370
      SequenceDescription :

Sequence
--------
<213> OrganismName : HUMAN
<400> PreSequenceString :
atctcttctttccttctctgaatctcatgcaggatttcacgttgctcctgctcttctttccgcttggcctccaacagcctctgc
tgctcctcctgagcccgcctttccagcatttcttcatgaaaagacttgggagggggcttgttatgctcgctgagaaatgactgc
acgtggtaagccagttcaaagatcatcacctccccacagtgtttcttggccagttcttctaggcgagattttaacaaattgaca
ctttcatttgatagacctttggcatttttaactctatttcaggaactacatctggataggtaggtgggcatttaaccctcaaa
tccacttttacatatacttcttcaccagttaggccttgagggtacaaaactaaattgatttcaggggggctctttgacccggtcg
caagcgtccggccgcagtccttggaagtccgggccgtaaaagggcctccaggcctgtaacccgtggtcctgtccgtgccggtaa
cc
<212> Type : DNA
<211> Length : 506
      SequenceName : AA926944
      SequenceDescription :

Sequence
--------
<213> OrganismName : HUMAN
<400> PreSequenceString :
tagggacagagtctatgttgcccagtggggctatccacaggtgtaatcatagctcactgcagcctcgaactcctgacctcaagt
gatcctgcctcctcagcctcccgagtagctgggaccacaggtgcatgccactgttcatgagttttacagacttttacattttaa
cttttgactcttttgtaattaacacaacacaaatacattgtacagctgcataaaaattatttctttatactcattctgtaagc
ttttttctattaattttcttttaaaactttttttcccttaaacactaagacagaaacacacacattagcctaggcctactca
ggatcttcaatatcactgtcttcccttctacatcttgtcccactggaaggccttcag
<212> Type : DNA
<211> Length : 393
      SequenceName : AA935151
      SequenceDescription :

Sequence
--------
<213> OrganismName : HUMAN

<400> PreSequenceString :
acgggttcatgatggtgaatgatatagcgggtgacagatggctacaaaccagtcataggccatcacactcaggagcatgtctct
cttccatgcctccaaaaatggcaaagagagacatctgagtcaggcagcctgcataggagatgactctgctgtgagactggatgt
ccacaatcatcttggggaccgtggtggaggtgaaaccgnatgtcaggcaaggacaggttggagaggaagaagtacatgggggtg
tggaggtgggagtcagggctgatggccaggatgatgagcaggttccccagcaccgtgaccaggcacatggacaggaacagccca
gcaaggactggctgcagttctggatcccctgagacttcaaca
<212> Type : DNA
<211> Length : 378
SequenceName : AA938905
SequenceDescription :

Sequence
--------
<213> OrganismName : HUMAN
<400> PreSequenceString :
gccaccatggccccgcccccaccgccactggctgccagcaccccgctcctccatggcgagtttggctcctacccagcccgaggc
ccacgctttgccctcacccttacatcgcaggccctgcacatacagcggctgcgccccaaacctgaagccaggccccggggtggc
ctggtcccgttggccgaggtctcaggctgctgcaccctgcgaagccgcagcccctcagactcagcggcctacttctgcatctac
acctaccctcggggccggcgcggggcccggcgcagagccactcgcaccttccgggcagatggggccgccacctacgaagagaac
cgtgccgaggcccagcgctgggccactgccctcacctgtctgctccgaggactgccactgcccggggatggggagatcacccct
gacctgctacctcggccgccccggttgcttctattggtcaatccctttgggggtcggggcctggcctggcagtggtgtaagaac
cacgtgcttcccatgatctctgaagctgggctgtccttcaacctcatccagacagaacgacagaaccacgcccgggagctggtc
caggggctgagcctgagtgagtgggatggcatcgtcacggtctcgggagacgggctgctccatgaggtgctgaacgggctccta
gatcgccctgactgggaggaagctgtgaagatgcctgtgggcatcctccccctgcggctcgggcaacgcgctggccggagcagtg
aaccagcacgggggatttgagccagccctgggcctcgacctgttgctcaactgctcactgttgctgtgccggggtggtggccac
ccactggacctgctctccgtgacgctggcctcgggctcccgctgtttctccttcctgtctgtggcctggggcttcgtgtcagat
gtggatatccagagcgagcgcttcagggccttgggcagtgcccgcttcacactgggcacggtgctgggcctcgccacactgcac
acctaccgcggacgcctctcctacctccccgccactgtggaacctgcctcgcccacccctgcccatagcctgcctcgtgccaag
tcggagctgaccctaaccccagacccagccccgcccatggcccactcacccctgcatcgttctgtgtctgacctgcctcttccc
ctgccccagcctgccctggcctctcctggctcgccagaacccctgcccatcctgtccctcaacggtgggggcccagagctggct
ggggactggggtggggctggggatgctccgctgtccccggacccactgctgtcttcacctcctggctctcccaaggcagctcta
cactcacccgtctccgaaggggccccgtaattcccccatcctctgggctcccacttcccacccctgatgcccgggtaggggcc
tccacctgcggcccgcccgaccacctgctgcctccgctgggcaccccgctgcccccagactgggtgacgctggagggggacttt
gtgctcatgttggccatctcgcccagccacctaggcgctgacctggtggcagctccgcatgcgcgcttcgacgacggcctggtg
cacctgtgctgggtgcgtagcggcatctcgcgggctgcgctgctgcgccttttcttggccatggagcgtggtagccacttcagc
ctgggctgtccgcagctgggctacgccgcggcccgtgccttccgcctagagccgctcacaccacgcggcgtgctcacagtggac
ggggagcaggtggagtatggccgctacaggcacagatgcaccctggcatcggtacactgctcactgggcctcctggctgcccg
gggcgggagccctgaaactaaacaagcttggtacccgccgggggcggggcctacattccaatggggcggagcttgagctagggg
gtgtggcctggctgctagagttgtggtggcaggggccctggccccgtctcaggattgcgctcgctttcatgggaccagacgtga
tgctggaaggtgggcgtcgtcacggttaaagagaaatgggctcgtcccgagggtagtgcctgatcaatgagggcggggcctggc
gtctgatctggggccgcccttacggggcagggctcagtcctgacgcttgccacctgctcctacccggccaggatggctgagggc
ggagtctattttacgcgtcgcccaatgacaggacctggaatgtactggctggggtaggcctcagtgagtcggccggtcagggcc
cgcagcctcgccccatccactccggtgcctccatttagctggccaatcagcccaggaggggcaggttccccggggccggcgcta
ggatttgcactaatgttcctctccccgc
<212> Type : DNA
<211> Length : 2380
SequenceName : AF245447
SequenceDescription :

Sequence
--------
<213> OrganismName : HUMAN
<400> PreSequenceString :
ttttcatccaaaataggtaaaaccttgttagccaaagcagtagcaaaccaaacctcagccactttcttgagagtggttggctct
gaacttattcagaagtacctaggtgatgggcccaaactcgtacgggaattgttccgagttgctgaagaacatgcaccgtccatc
gtgtttattgatgaaattgacgccattgggacaaaaaggtagactttctcatacttatttgccttgtttagtagggaacaccg
catagctcttctcttgagaatgggcaatctcaggggggctctccctatggccacagttcttgctgtgcgtggccttccatgcatg
ctttaggctctgctctcccaggagccagctaacaactgcccagtaactgtgaacgtctggagagactggtcagttatgaataac
catgtcttgagcacccgccctgatccaggaccatctggactctagggacacagttgtgagcacaagtctc
<212> Type : DNA
<211> Length : 490
SequenceName : AI027898
SequenceDescription :

Sequence
--------
<213> OrganismName : HUMAN
<400> PreSequenceString :
gagacagtttcactttgttgcccaggctggagtgcagtggcgcggtctcagctcaccgcaacaagagcgaaactccatctcaa
aaaataataataatgaggagaaggccttccagttatggcagcacaaagaggcaggcaatttctttctgtaaaaaaaaaaaaaa
agcaacaaccaaccaaaaaatatataaaactagacaaattatttatcaaaaccaaccatttcaggaaactggaaattgacaaa
atgcaggcaaaaaattgagcagcatttattcttgaaaaactactagatctttaggcaagtaggagattgtggctttttctgcct
gt
<212> Type : DNA
<211> Length : 338
      SequenceName : AI051860
      SequenceDescription :

Sequence
--------
<213> OrganismName : HUMAN
<400> PreSequenceString :
gcgccccgtttcccaggacgaagggcactccgcaccggaccccggtcccggcgcgcggcggggcacgcgccctcccgcgcgcgc
tctctgaccccgtctctctcttcctcgacttctctctggagctcttgctacctctttcctctttctgctggtttccaaaatcca
cagtgatttggggaagtagagcaatctccccaagccgtcggcccgattcaagtggggaatggcaagcaaaaataaattaaaacg
agaaacaatacagttttaaaaaaaaatgtttaagaaaaaagaagagggataaaacccggatcaatgaatatcaaattccagcac
cgagcgccctggccggtgagtccgctgaccggtccacctaaccgctgcgcctcccgacagagcgc
<212> Type : DNA
<211> Length : 401
      SequenceName : AI091579
      SequenceDescription :

Sequence
--------
<213> OrganismName : HUMAN
<400> PreSequenceString :
tttttctggctacaaattttattccgttacaaatagcaccgagcccttggggctgcccctgtggagagggatcccccctcctgga
cccccacctcctcccctgagctacccatgtagtgtctgggagctggcacacggcccagggtccctctggaagctgggccaggcc
aaggggttaggcccaggcccttcgtggggtgggcccagggccagctgtggcagatgtgcaggtaggtgagcggcagcctctcag
atgagcacactggccacgggcacgcgggtggagcggccccgctggggcaccacaatccggtcgtctgtggaccctgcttcgtgc
agcaggccctgcacgtgcagctgtgcccgccggcggtcggcctcaatgaagatggcggtgcccaggaaggctgcgccgcccagt
gccccaacaaacgcgcagagcatgagcgagaactgcagagcccggaactcggacaagaaggagggggccagttccggcgcagg
cggtcagagatcaagccaatgaggtaggggctcccagcatcacccagcaagtgggacagcacgatctggaaggcctggcggtgg
agcgtcgggtagggatcaccacgtacagcagaatgtctgccacgatggccccagtcatggacaggagggtctctccaatgaaga
tgaaaatataagtggccacgatgctaccacgggcgcatgcaagggacaggaagaggaggggtgcagagcccaggatgcc
<212> Type : DNA
<211> Length : 751
      SequenceName : AI126706
      SequenceDescription :

Sequence
--------
<213> OrganismName : HUMAN
<400> PreSequenceString :
gctctgcttccctttttaaatataagttctacctttttgtctttgctcccacatctgaatataagtggctagaagcagccacacca
cttcttgatcactttgctgcttagacatttcttctgccagataccctaaatcatcactctcaagttcaaacttccacagattcc
tagggcatgaacagaaggcagccaagctctttgctaaggcgttaacattgtgtgacctttgcaaagtgcttgggtgcattctgt
tcatgtcctagctatcagcatttggtcacaaccatttaaccagtccctaagaaattccaaactttccctcatctttctgtctt
cttctgagccctacaaactcttccaacttctgcctgtcaccaagttccaaagctgcttccacattttcaggtatctctacagca
atgactcacttctcagtaccagt
<212> Type : DNA
<211> Length : 443
      SequenceName : AI141943
      SequenceDescription :

Sequence

--------
<213> OrganismName : HUMAN
<400> PreSequenceString :
ttaacaagtcttgacaaatgtatgcagtccgataacagcgtgatcacaatgtagaacagttctcagataggcattgtttaaaag
tcaggaaagatgtatatcagaattagagactgagatttagctgatcttcacattttctgtattgttgaaatgcttataatgccc
atttgcatccaggattgaagaggctggcccagccagggatgaacttcgggtcccgggtgaagaggaggatggcaaacatgcag
aaaaggatgaaaacagcctgttcggcaaacttgatgggccccaggttctggtattcttcccgaattacagctcgagccctatct
tctgcattggttcttatctcagatttattcttcctccagcccctgaagctcagtccccgtacaggaaggagatccagagccag
cctgccaacaggaag
<212> Type : DNA
<211> Length : 435
      SequenceName : AA861140
      SequenceDescription :

Sequence
--------
<213> OrganismName : HUMAN
<400> PreSequenceString :
acgtttattaatactcttctgttgccctttggaggtctgagggggagtccttgcagggcctctggtctgtccctgttcatggaa
ggcagtgctcaggaggcagcagcaaagtgccaatgaagcagggcgttagtgccgatggagcagggagccagtaccggtggagct
gggcgttggtgccaatggagcgaggcactggtgcgggtggagcggggcgttagtgccgatggaatggggcaccagtgccgttgg
agctggacgtagtgatgatggagcagggtggtagtgccgacggagctggacatagtgatgatggagcggggtggtagtgccgat
ggagccggacgttagtgacaatggagtgggacaccagtgccgatggagcagggcgttagtgctgatggagtggggcac
<212> Type : DNA
<211> Length : 414
      SequenceName : AA861582
      SequenceDescription :

Sequence
--------
<213> OrganismName : HUMAN
<400> PreSequenceString :
ttatgtagtaattactctttattcaaatattataataatcttcactctacaatcataacctaggaaaaaccaggccatacagag
gtatgagctgaagggacatggtgagaagtgaccagaagacaagtgtgtgagcctctgttatgcccggacaggcgcaccagaggg
ctccttggtctagcggaacgttagcgtctgggaagacgctgttaccaagcggattgtggtctagcagtagcatcagtgccaagg
aaaagcacccactacttagcagaccagcaaagggagtctccctttccccgggggagtttagagaagactctactccaccatgtc
ttgtggagg
<212> Type : DNA
<211> Length : 345
      SequenceName : AA932129
      SequenceDescription :

Sequence
--------
<213> OrganismName : HUMAN
<400> PreSequenceString :
gcccctatacccaggtcggacgacgnatttgcacgtcaggaccgctacggacctccaccagagtttcctctggcttcgcactgc
ccaggcatagttcaccttgcccgaagacttcacacccttgaggatacagaggaacacgatgacccaggccagcagcaggcagag
gcagaggttccagcggatctccccagggctgccgatgccctggctgccttggatgtggaggacgtagcggctccagtactcctc
gctggggctgacggtgcaggtgaggttgaggggcagggctntngaccgtccttggagactctgtgctccaggcagagttctgtg
ttccaccagttgccacagtgctcccagggtaggtcgctggtgagggaggcgaagaggtagaagagcacgtaggcgatgatcatg
ttgtagtagatggtca
<212> Type : DNA
<211> Length : 436
      SequenceName : AA984230
      SequenceDescription :

Sequence
--------
<213> OrganismName : HUMAN
<400> PreSequenceString :
ctgttaaattcctcctagaagcaaagcacctgagagatgagatccaagtaaataagacccaaatcccggacacgtcacacaata
ctcggagctgtaccatgacttcaccaccacgcaggtgtgaatgcacgacattgtccgcagcttccggcagaccatggaccaata

cagcacggagcccggcagatacaggttcatggggactgaagcctatgcagagagtattgacaggaccgtgatgt
<212> Type : DNA
<211> Length : 242
      SequenceName : AA985407
      SequenceDescription :

Sequence
--------
<213> OrganismName : HUMAN
<400> PreSequenceString :
tttttttttcttccaggaggcaaggaccttttcctcgtttattcttatcaggaaaacttctattcaaggtagaaggacaataag
agtgaagccttcacagctgacagacactgcttgtccattggctatgtcatcctgggcaaagtcctcagcctcttggagcctgtt
tccttgcctgtaaaatgtggacagtaatagctaccctgctggttggttgggaggattagacgtaatctatcaaaagtgctgcta
aaactgccccctggaangtaatggacaatgaaaaaaatcactaaaactatttgtgtgggagaaaagggtttcacccagaattta
atccaactctactagtttgctcccaaagggttctgtcaatcacaaccaacttcagagtctaaaactgaacc
<212> Type : DNA
<211> Length : 407
      SequenceName : AA988469
      SequenceDescription :

Sequence
--------
<213> OrganismName : HUMAN
<400> PreSequenceString :
ggccgctgaatggccgagtcgctgagccgcggctgccggacgggacgggaccggctaggctgggcgcgccccccgggccccgcc
gtgggcatgggcgcactggcccgggcgctgctgctgcctctgctggcccagtggctcctgcgcgccgccccggagctggccccc
gcgcccttcacgctgcccctccgggtggccgcggccacgaaccgcgtagttgcgcccaccccgggacccgggacccctgccgag
cgccacgccgacggcttggcgctcgccctggagcctgccctggcgtcccccgcgggcgccgccaacttcttggccatggtagac
aacctgcaggggggactctggccgcggctactacctggagatgctgatcgggacccccccgcagaagctacagattctcgttgac
actggaagcagtaactttgccgtggcaggaaccccgcactcctacatagacacgtactttgacacagagaggtctagcacatac
cgctccaagggctttgacgtcacagtgaagtacacacaaggaagctggacgggcttcgttggggaagacctcgtcaccatcccc
aaaggcttcaatacttcttttcttgtcaacattgccactattttgaatcagagaatttctttttgcctgggattaaatggaat
ggaatacttggcctagcttatgccacacttgccaagccatcaagttctctggagaccttcttcgactccctggtgacacaagca
aacatccccaacgttttctccatgcagatgtgtggagccggcttgcccgttgctggatctgggaccaacggaggtagtcttgtc
ttgggtggaattgaaccaagtttgtataaaggagacatctggtataccccctattaaggaagagtggtactaccagatagaaatt
ctgaaattggaaattggaggccaaagccttaatctggactgcagagagtataacgcagacaaggccatcgtggacagtggcacc
acgctgctgcgcctgccccagaaggtgtttgatgcggtggtggaagctgtggcccgcgcatctctgattccagaattctctgat
ggtttctggactgggtcccagctggcgtgctggacgaattcggaaacaccttggtcttacttccctaaaatctccatctacctg
agagacgagaactccagcaggtcattccgtatcacaatcctgcctcagctttacattcagcccatgatgggggccggcctgaat
tatgaatgttaccgattcggcatttccccatccacaaatgcgctggtgatcggtgccacggtgatggagggcttctacgtcatc
ttcgacagagcccagaagagggtgggcttcgcagcgagcccctgtgcagaaattgcaggtgctgcagtgtctgaaatttccggg
cctttctcaacagaggatgtagccagcaactgtgtccccgctcagtctttgagcgagcccattttgtggattgtgtcctatgcg
ctcatgagcgtctgtggagccatcctccttgtcttaatcgtcctgctgctgctgccgttccggtgtcagcgtcgcccccgtgac
cctgaggtcgtcaatgatgagtcctctctggtcagacatcgctggaaatgaatagccaggcctgacctcaagcaaccatgaact
cagctattaagaaaatcacatttccagggcagcagccgggatcgatggtggcgctttctcctgtgcccacccgtcttcaatctc
tgttctgctcccagatgccttctagattcactgtcttttgattcttgattttcaagctttcaaatcctccctacttccaagaaa
aaaaaaaaaaaaa
<212> Type : DNA
<211> Length : 1862
      SequenceName : AF204944
      SequenceDescription :

Sequence
--------
<213> OrganismName : HUMAN
<400> PreSequenceString :
ttttcagattccacaaggatttattggcaccggacctagcctggtgcccgccccttcaaggaaagacactaacaaggaggagtg
ccggcaccgcaagagccctcccagaagcgcccagactgggggtcggggaggacgctgccattcgtggccaggaagggaagggag
attccggaggagggtgggcacggcgcagggaaaggccgggtggtgggatgggcagggaggcggggtaccggccccgctagaagg
ggaactcgcagacgtagtagagacgccgctggcagtcgtggtcccaccaggagccgtcgtcagaggcctgcgccacgcagttct
cgagcgtgccaccgttgggctggtccgggctgagcggatgcggcgaggcgctgggct
<212> Type : DNA
<211> Length : 393

SequenceName : AI016874
SequenceDescription :

Sequence
--------
<213> OrganismName : HUMAN
<400> PreSequenceString :
tttaagatggctgtttcataaattagttccctattcctaactttggagaacctggtttttcttacctttcaaggtccaaatat
aaaactataaactttcagtgttggacattcttgcttttgaaagcaaattcctcctcaattcatgacctgtgggccagctatca
tgaataatacacccatagcccttgggttttatgaatgtgacttctaaatcctcctgcataatagctttaggtgacttgaagagt
taaaagctgtcattgtcatcaatatttactgtacatcaaactcatttaaaatgctgttagaaattgctgactggcaagactgtc
actgacaaaaacaccagtttaggacagattccatgggaaacaaactagaataactgaaagctaaaatagagcttaatggtagag
aaagtctcagaaacaaggacagagatcacagaaaagacaaggaaggactcctattggtttttctgattttgaagagctctttgt
gcttacctatttatcctaaacaaaaaggcatgagtaaaaatttccaatgtattacaaagggtattctgtctaatgtcaggctgg
ctctcangatgttataaacctgcccaggtattagacttctgatgagactgtgccttaaggacaggaatggcactttctactaaa
aactactgccatctgggagcaaagactggggatngaaactacaagacaaacctgtctagtatgacttgtgatccagagcctntt
gactatgggactagacangtgcaacattctttcctttttcactgacccatanagaagttacagactttcattgtaatccaactt
accggttattcttggagatgcccagccaatctgtgccgatattggcccaggccaatactttagggacgtttaatcgatctatat
acc
<212> Type : DNA
<211> Length : 927
SequenceName : AI025519
SequenceDescription :

Sequence
--------
<213> OrganismName : HUMAN
<400> PreSequenceString :
ttttttgagggatgcacaagtctttattcctgctggcagaggaggtggtcctgtggctggcggagtgggcacctcaggctggca
gctctcacctcttgctggccggcagcttcagccacttgggcaccttgcccaggctcctcttcacgggctgggccgtccctggga
tgggctcagggggggaccagcgccccctcctcagcagctggctcagacttaggtgcagggtcaggggctggcaatggggaagggg
acccggcggccctggacatgtacctggccaccagcacgtcggccgcagatggggagatggcatgctccagcaggccaggctcca
ggtagacacctgccggctcctcggctcccaagtgcaccagagcggccgggaagaggttcgcccggcctgtccccacgtgcttgt
gcccagccttgactcgggagggatcgccactgctcggacgaggggatggtggagaggagggtcggcagactgggtggcatggag
gctgaccagtgcttggagaggctcg
<212> Type : DNA
<211> Length : 529
SequenceName : AI031531
SequenceDescription :

Sequence
--------
<213> OrganismName : HUMAN
<400> PreSequenceString :
tccgatgggcacagctttattctggaacggtgaacccagtggggaggatcctgcaggcggacggcagttggggtactatagatg
actaagtagggtccagtccatcgaggttgtagagtttgaggggtcaggttcttaataagaactgatcgtccagctagggtgtct
tcatatagttgggaatcctctactgtaagagttatccaaagctcagcatccgtaatggtccagggggcttccaaggcgatcagg
cagcatcagtcttcagctgctaagctgagaagatctgggaaggagtcagtcagagagcctttggccagagttctaggggccctg
ggagtggctgccgggttggacagtccgatttccagtggggtcccgatttgaaattcatgagatgttccttgggctggttggtct
gaggacccgaagtcgtaggt
<212> Type : DNA
<211> Length : 440
SequenceName : AI051176
SequenceDescription :

Sequence
--------
<213> OrganismName : HUMAN
<400> PreSequenceString :
ttttagagaaacgttgtgtttaatggtaaagcttaacacaccccagcaccaagaatggtgtggagttgcagtagcaacaggccg
gtgacccccccatggagcctcacatggcgaagaggatgaggaaggcgaccatcaaacagaaaagccccatggcctcagacagggc
aaagccaagaatggcataggagaagagctgctgcttgagagacgggttcctggcatagccaatgatcaagctgccaaacacggt
tccaatgccagcccctgaaccagccacaccaactgtggctgccccagcaccaataaacttggctgctgtgtcaatgtcccggga

gacaacactggtctggaactcccgtctggccacctggagtgggaagttgctgtaggaaggctgtttagatgaattcactgggct
attcaagaaggaggcagacacaggcctgattagacccctggtacaacagcggatcagagctggagaaatgaataatgcacaggc
ggtctgcatttttcagtctcac
<212> Type : DNA
<211> Length : 527
      SequenceName : AI186110
      SequenceDescription :

Sequence
--------
<213> OrganismName : HUMAN
<400> PreSequenceString :
tttttccaaataactggtaataattttattaagttttccctaatgcattttcaattctacatttcaagagtattgaactttt
ctcacttttgcactctaatcagaaaaaccagaattaaatttttatttaaaatacatttaataaaaataacccatagttttaca
tattttacatgtgtagaatatttacaaactcacttctacagcatttacttaatgtttactattttccattatttgccttttg
gctatttccctcagtagtaaaatgttgtccagtgttctctttaaaaaattagtttcttggcaatcctccttgttctaaggcct
ctgttcttttcctttttattgccaaagctggatttgattgctcctttaaggtttccactggacagtttggtcttggcagctgac
atccaagtgttggccctggcccgatactggaatcacttcattttcagtgcctgtgtggccgtaattcgagcactgaattaaata
agattaagccttgtatgagatctagtaagtcgtctcctgctgcactgaagatgtgatgcaaaggtattccagggaaactcttaa
atgtcacataatctggaagactacacatgtccggccactgttcctcagttgttgtgcccaaagtttcaaatattctggttagct
gatcaaggtctgaatctcctggcaaaaaaagaaccctttagagtacctctgctatatacagccaacagcccacattgtcacacc
tacccatacatcctagctcccaatggaactcgggggcc
<212> Type : DNA
<211> Length : 794
      SequenceName : AI189206
      SequenceDescription :

Sequence
--------
<213> OrganismName : HUMAN
<400> PreSequenceString :
tttttttttttttttacaatttactttttggcgggtatttaaatattttcttatctgcagagctatctcaggatatgaaatc
ataacatgctagcaactagtaatttaacattaaaacacttccctaaattattccatggaaaagtgcatttacaatataaacatg
tcatatatgaagctacaaatcataatctcactggagtggatataaaatttcaaattcagtccaaatgagtcaaagaaaaaagtg
ctaacagagcaaatctgaaacaagttcatggggattgggtaatgagtcatcgaaacgaaatcttttggaaaccatgctgctatc
ctctgggatattctctttgtcttctctatcaccacaggttccattacaggaggatttagaagtcttgtcttcagaggaccttac
agaatgatcctgagtttgagaggaactggaagtttcttcagggtgaaacaagctttcaaagtcccactgctgtagccaagaatg
agaaaggcatatctctgctgttggtcttttctctggatttttttactaagaggctctgaataaagtctgtggccagctgtgaaac
tgatgaaaaagtttcttccgaataatctacattaacttgagagatattgaggtatgtttcttgattatcttctcccacaaatgg
tgatgtgtgagttaacaacataattgctattataccaatatt
<212> Type : DNA
<211> Length : 714
      SequenceName : AI191941
      SequenceDescription :

Sequence
--------
<213> OrganismName : HUMAN
<400> PreSequenceString :
ttttgtttccaaaacaagaatgctgtttatttgctgatcaaaacttgtgggaaaaattaaagtaaaaaaatattgggagaagaa
tggaaagcctttggataaatgattagctggttaaataaatgagcctgtgttactgaatgagtatggattgcccggttactttttt
ttgtttcttgacactctttgctttgggtcgtttactttcaatgtttgctttagattcttcctgtccagtttttgctttagattc
ttcctgtccagtttttgcttcagattcttcctgtccagtttttgtttagattcttcctgtacagttttttgtttagattcttc
ctgttcagcttttgctttggatagtggcatcctttcctgtttgggttttaagtggtttcctatcacttgccggaaaaacacaaa
aaccactgaaaggattaataaaataaggcgaaacattatgatggacacaacccacattggcgaagaaatcgcccctctgagcag
tcctggaggcccactggcaatgcttccttcataccccacttcctcacagaatggaagtgcccacccatacatgcagtggcagct
ttttctggtgtt
<212> Type : DNA
<211> Length : 600
      SequenceName : AI214466
      SequenceDescription :

Sequence

--------
<213> OrganismName : HUMAN
<400> PreSequenceString :
tttttttttattttaatagtgtacatttattcaaaagtatataaaacaacactgtaaattatataatctcacataaaaacact
agaacttgatttgaataacaaaacaatctactaagaagtattgctcatgaagattttgtgcccacctttcctaactcagaggc
cctcatttatgtcgcaacagcaaggtggcagggaatcaaaactggcacacattctatatccttcacagcactgcagggcaggac
aagactccgtggcaatggttttggcctggctgttcttggcccaccaccttgtctgggttgtgatttttacagccacagctt
caggttcctttgcagcttttagcgcgttgtgtattttgaagtaaagacagagcacgacaactatcagcaggagcaaggccgcca
ggataccaccagcgatgacaagcataatcactgtatggaggtcaaaagacttcgatgtacctgcataaataatggctcctctgc
tccaggaagggcaagaaacatttcaaaaagagctccaatcatgtgtcagacatccccttgtcaggcatcct
<212> Type : DNA
<211> Length : 575
SequenceName : AI300541
SequenceDescription :

Sequence
--------
<213> OrganismName : HUMAN
<400> PreSequenceString :
aaacccgncactttattgccaatatatatttttaactagagatggcatctcgctccattatcaaagctggccctgaactcctgg
gctcacttgattctgcctcaggcttcagagtagctgggattacaggtgtgcgccactgtacctggcctcaccattcagtcttga
gagggctgtccattggttaggcctttaagacagagtacagggtctcgttctgggggctcttgaggggacggtggctgggaggtg
ctctgggtgaggtggagctggagagggcaaggaagcatagacgatgccgtcatccttgggatttagcttgggatctgtatttt
gtccttcattcctgatattctcatatggctcctctgtgttttggaagggttccctggctggggttgtggctttagtccgctgct
gaccctgngtgatggagagtttggtcccctcgatggactgccac
<212> Type : DNA
<211> Length : 464
SequenceName : AI338030
SequenceDescription :

Sequence
--------
<213> OrganismName : HUMAN
<400> PreSequenceString :
gtttttttttttgttttctcacttgatttcattaagctgatcttcagtctctgatatcctttcttccgtttgatcaatctggc
tattaatacttacgtatgtttcacgaagttctcgtgctgtgttttcagctccatcaggtcatttatgttcttctctaaaccgg
ttattctagttagcaattcctctaacctttttttaaggctcttagcttccttgcattgggttggaatatgctcctttagctcgg
aagagtttgttattacccaccttctgaagcctacttctgtcaattcatcaaactcattctccatccagttttgttcccatgctg
gcaaggagttgtgatcctttggaggagaagaggcgttctgggttttggaattttcagcctttttgcactggtctctccatcttc
atggatttatctacctttggtctttgatgttggtgacatttggatgaggtccctgagtggacatc
<212> Type : DNA
<211> Length : 485
SequenceName : AI061445
SequenceDescription :

Sequence
--------
<213> OrganismName : HUMAN
<400> PreSequenceString :
ggtagatgcatgacttgtgtgtgatgagagaatggagacagagtccccggctcctctactgtttaacaacatggctttcttatt
ttgtttgaattgttaattcacagaatagcacaaactacaattaaaactaagcacaaagccattctaagtcattggggaaacggg
gtgaacttcaggtggatgaggagacagaatagagtgataggaagcgtctggcagatactccttttgccactgctgtgtgattag
acaggcccagtgagccgcggggcacatgctggccgctcctccctcagaaaaaggcagtggcctaaatccttttaaatgacttg
gctcgatgctgtgggggactggctgggctgctgcaggccgtgtgtctgtcagcccaaccttcacatctgtcacgttctccacac
gggggaga
<212> Type : DNA
<211> Length : 428
SequenceName : AI074564
SequenceDescription :

Sequence
--------
<213> OrganismName : HUMAN

<400> PreSequenceString :
tttttgtatttttagtagagacggggtttccccgtgttaaacaggatggtctcgatctcctgacctcatgatccgcccgcctc
ggcgttggcctcccaaagtgctgagattacaggcatgagccaccgtgcccagccaacaacccctctcctttatgtatggaaca
gggcactgggaccagagaggacaaggggctagccggggtcagataattgggaccagaactctaacccttggccatgcccattcc
aaggagggaagccaggagatttgtgtctgcctgtcccagaggtaagaggccctccagccaggccactgtaaggaggctggccca
cctggtgcatattccatggcgatcataagggctttgtcttccaggaagttctcgtagtactcaatgacattggggtggttgagc
agcttgaggacctggcactcattctgggctgcctgccgctcttccttggtcatctgttccactggaatctgcttgatgatcacc
agcttctggtcagcctttcgcaggcacaggtgcacaatcccgaaagcacctcttcccaccactcggatccgctcgtacttctcc
at
<212> Type : DNA
<211> Length : 590
SequenceName : AI086865
SequenceDescription :

Sequence
--------
<213> OrganismName : HUMAN
<400> PreSequenceString :
ttttgcattttcactgaagcatttattatgcaaaataggaaggctttagcatgccagtataaattaaaacagtagaaaaaaagc
agagcagagagcgttttccatccatggcctgtgccctgtaatgacgcagcagtatcctagtacattgacgggtttttcctgtcc
tgccactgtccgctgggcagttataggtcccaagtgttgggtcttcctgcgaggccttcacaaagatgatttgtcattcgctg
aaagtggaagtgaatcaacggcctcctcttgctccgcagcactttattcacactgtgtaaccacatgtttttatgccactttg
tgcacagctcatcaaggtcagtctgttcatcttctggcccctgtagcacatggtacagttcaatggtgcggcacagaggcctga
g
<212> Type : DNA
<211> Length : 421
SequenceName : AI125073
SequenceDescription :

Sequence
--------
<213> OrganismName : HUMAN
<400> PreSequenceString :
tgttatttatttatttatttatttatttatttatttatttatttatagagacaagggcttgccatgttacccaggctggtcttg
aactcttgagctcgagcgatccccacctcccaaagtgctgggcttacaggtgtaagccatcatgtccagccgttcagatattct
agttgaattggagttggtgggctagtacaccttctaaattaaatgagtaaaggatttagaatggtgcctgacacacagtaggtg
ctacattcatgttagctactattataaacctttcctgcctctgactttcagggtcttgcccaccaccagcgatgcccagcccctt
ggtagagcttgaaccaccttctataaacaggatggcggtggagagacaggcccagtccctgagcccatgaggagtgtggcccct
tcaggcccaaagatggtgaacatcactgcagacaactcctcgatgagctgtaccatcgaccataccatncaccagacgctggnc
ccg
<212> Type : DNA
<211> Length : 507
SequenceName : AI126520
SequenceDescription :

Sequence
--------
<213> OrganismName : HUMAN
<400> PreSequenceString :
cctcgccgccttggggtctgggcgcgcggtgccgtgggggtcagcagggcggagcggcttttccaggagaaagggccctcacgg
gtgagcggggcgactgggctcccccgcggtgcagttgccccgcgggcgccggccccggctcaacggattcttctcgctcgctgc
ccggaaagaaccatttgggagagcccatggtgactgcgtgagtggagcccagctgtgtggatgccccagcatggatgactacat
ggtcctgagaatgattggggagggctccttcggcagagctcttttggttcagcatgaaagcagtaatcagatgtttgccatgaa
agaaataaggcttcccaagctttctctatacacagattctaggaggaggctgttctttagccaaatgaacaccctatattgtgc
cttcaagatc
<212> Type : DNA
<211> Length : 430
SequenceName : AI209040
SequenceDescription :

Sequence
--------
<213> OrganismName : HUMAN

<400> PreSequenceString :
ttttagtcgaaatgtttgctttaatagcccccaaagtacatattaagctttcaactctcccccgccccccctccccttttttttg
agacggagtcaccaggctggagtgcagtggggcgatctcggctcactgcaacctccgcctccagtcaaccccattttgaaaagg
gttttaaggggaaggagttagaaagggcccagtgaaggaggaggtggggctctgggggtgggggggaatggcctccgagcagggg
gagggagagacagaaacttccagcatttctaaatggcgtggggtttgccctggagccggcggcggtgcacgagtaggaagtcct
tta
<212> Type : DNA
<211> Length : 339
SequenceName : AI214335
SequenceDescription :

Sequence
--------
<213> OrganismName : HUMAN
<400> PreSequenceString :
gtggcggaggccagccccgagcttgcccagcggtgcttcaggcagggaggggccaggcccgccagccaggcgaaggcgcaggca
gcgcgcgcggggcgccgtaggcaccggccaggcgggcagccgccctgggcccgggaccgcgtacgaagccgcagcggcacacg
ctgatgagcgcggcgaagaccaccgccgcataggtggacacgtagtaggtggccccggccgcgtggcaggcggcctccgggcca
ggcccaggtagtaggtgagccacagctgcagcgtgagcgtgaagaactcatcaaccagagccaggttgaacaggtagagaagca
gggcctggcccaggcgcccgccgctgcggatgaacattgccagggccgncacgttggctggcagccccaggcccagtgccaagg
cataggccgcgggcaccacgatgaagcgagcagggtcatc
<212> Type : DNA
<211> Length : 460
SequenceName : AI264420
SequenceDescription :

Sequence
--------
<213> OrganismName : HUMAN
<400> PreSequenceString :
tgagatggagtctcgctctactgtagcccaggatggagtgcagtagtgtgatcttggctcactgcaacccctgcctcccgggtt
caaggaattcccctgccttagcctcctgagtagctgagattacaggcgcccaccaccacacctggctgatttttgtttcttagt
agagacagggtttcaccatgttggccaggctagtctcgaactgacctcaagtgatccatccgccttggtctcccaaagtgttgg
gattacaggtgtgagccactgtgcccagccaagttatatctctaaagcaatgtgcaaaaataaactgaacttgggttgattagg
tatattcaacatttgtcgggagagtagatgtttcatttatttcagtccctgtgtaatttgtcttctctaatgttaaatactat
gtagaatgtgtctgtgtaattttatagata
<212> Type : DNA
<211> Length : 450
SequenceName : AI299633
SequenceDescription :

Sequence
--------
<213> OrganismName : HUMAN
<400> PreSequenceString :
ttcactttacaaatagtttatttccaccttcacaaattcatgcgcctctgaccattatgcaaggcaacatgaattaaatactta
tgtcagaacatatttgtcttacattattcaagataaagtggattttaaaagcaagtgggtaccaatgatggggagatgaggaga
atagggcaggcagcaacagggcaacatgcatttttcaagagtgtttattaaaataggcagtaatcagtacatgtacatcatatg
agcagtttttcaaaattagcacttccaggagaggggctacatctcagtttttctgtctgtacagtaaaatgccaaaagtactt
ccctaaagtacaaaggcatttccctagtagtcttggtaccagtaacaatatgattactaaacatctccaatgtggttttcatta
caaagaaacat
<212> Type : DNA
<211> Length : 431
SequenceName : AI299795
SequenceDescription :

Sequence
--------
<213> OrganismName : HUMAN
<400> PreSequenceString :
tttttttttgagttggaaacaactttattgatacctgagaaaaagtgcaggaaaaatattcctcctgaccaggtggcctgtccc
ctgaggccagatcattctctcgcctctcccttaagccctgggtcatttgggacctcactggccagcacccagaggctgggtcac
aacacccccctgaggagaggctgtcactcaggtcagggccacctgaggtttcaggagtactccccactggccagggcaaaggtgg

gcccatggggctgagccccaaggatgtgtgagtctgaaaatctgtcagtgacaagaccttgccatcgagaaggaagccagtagc
agcatccctggcccggggtgacaagtactgggcagtggctgctcggtgctcagggtggaaccaaagagggttcccctccaggta
gagcttgcggagctcagccagcagccacagtggtgacagctcccggtgtccttccagcaggttgtatgccaaatccaggtgccg
cagattcctcagctgctctaggccatgcaggctccgaagctcattgcctcgcagtatcaggaccccagagcagcccctgaggg
tcccattcttggcaccaaatgcaggcgattataggagatgtccagatggtggagctcacacaaatccatc
<212> Type : DNA
<211> Length : 658
      SequenceName : AI307255
      SequenceDescription :

Sequence
--------
<213> OrganismName : HUMAN
<400> PreSequenceString :
ttttttttttgtcttcagcagtgactttatagaagtatctatatatatacatttagtacaaccgacctttggtttcttctctc
aatccccttttcaaacgtatataaaaatatccaaaggctcctcccagcccagtgtttccaggcagcctttcaaatcccctcttt
gccttagttggtcaaaagcctgtggatgcagcacccttccaggaagggagaggaagaaagttagggctccttcttggcagcctt
gggaaaccatcggggtagagccccaagcctagaggcgggagcctgccgtccttccatcttgccacacatgggcacatactttcc
tgttccctcttgccctggaaggctgagcctgcagtgagggaagtggagtccttttccctgcaggggtcaagagagaactccctt
cgccaggcgcggtggctcacgcctgtaatcccagcactttgggaggctgaggcaggcagatcacgaggtcaggagatcgagacc
attctggctaacatggtgaaaccgcgtatatactaaaaatacaaaataaatt
<212> Type : DNA
<211> Length : 556
      SequenceName : AI310138
      SequenceDescription :

Sequence
--------
<213> OrganismName : HUMAN
<400> PreSequenceString :
gattccaagatacacatttttattattgtaacttctctgaaatcagatattttgatggtgtcccatagtgtttttgtttctt
actatttcataaaataaaggtgcatcttaagaatgatggcatcatagatttgatacaaaaagtacttaggtgactcattgcca
gctcacagacatgtttgatgctcagtccagctcctggcgtggtggcacgtgcctgtaatccccactcctcaggaggctgaggca
ggaagatcacttgcgcccaggagtttgaggcttcagtgagttatgatcgtgccactgcactccagcctgggtggcagagtaaga
ccctgttttgcatctctcaaggatgtgccatgaagctgtagccaggtggaattgtaccacagccctgacatgaacacctgatgg
cagctgctgggttggagccttcaaggcctcactc
<212> Type : DNA
<211> Length : 454
      SequenceName : AI310451
      SequenceDescription :

Sequence
--------
<213> OrganismName : HUMAN
<400> PreSequenceString :
tttttttccgcataactcttttaataaataaagcatattcatggcttaaattttaaatcacatttacagatgagggaaaacttc
taggcctaaacaggtaacagtaaagcgactactgctggaaagagtggtgtgccacatgcgccttaagcctcaagagtggggact
gcagggccaaacaccaaatacatcacctttaggccagacggagagtggagacccaggaagcccatgcaaccaccatctcttgct
ctgaggactttgagtctcgcttgattcatcaaattggacatctgaattttcttctgctgctcaatctgtttctctttcttctca
taatattccataatctttagtctttgggtttgcacaagccgacctttctctatgttgaactcttcttctgcctttgcatctatt
tcttctgctttctcattgg
<212> Type : DNA
<211> Length : 439
      SequenceName : AI310990
      SequenceDescription :

Sequence
--------
<213> OrganismName : HUMAN
<400> PreSequenceString :
ttttttttttctgaggtaagactttattgtatacttgggtggggtagcccagatggatgcaaggttgcaggcatgggaagcagc
cctcttgggtgtcactctgaaaatgaagtagggtggtggccgcacctcgccagtgaattagtcccctaccaaggtcttacagac
ccacccttcctctcaccctccagttcccactgtcctccaaggagaagagccttgggtaagtgtccctccctccttcagagagtg

ggactgtctgcctcttgaaagcgaggattgatggtggtcgtgatggcacttttgtagagaggattactgtcctgcttccagttg
agttgttgctgctccttctcaaagcgactgtattcccggcggtcatagatttccaccgagagccggtaagccaggaccagcccc
agccccactgccacgatgcccctacgcagcccagcacaatggcctgcgtgtggtctgctccctttcttgggtctcactctga
gcacgaccgtgcctctggcgtcatcctccaccaagaagaagaacagctggttgtccagggtccgctcttttcaccaggcatcat
tcaggataagggccaaaggccaggtcacattgttatggcacaagctgtactggagttggtggcaaggggccgtcc
<212> Type : DNA
<211> Length : 663
      SequenceName : AI380522
      SequenceDescription :

Sequence
--------
<213> OrganismName : HUMAN
<400> PreSequenceString :
ttacattatcctagaaaacagaataattggctttatttggtatcttcaagaagcttctccttccgtaggagatgtaggtgttgt
gggagaaactgtaactggttttcgtgtaattctgtctagttctgcatgaacatctaagacaggcttctggcctgacttttggt
ggatggtggtaaaccactgcttccacctccagccccacctgcagggctaccaccactcacaccagggcctcctggagagccagt
ctttntactcaacaaactgttgaagaaatttgccagaacgccttcacttgtagctccagctttcatgtttggatcaattttttt
tgacccagcaggaatgggtgacacgctggcaacattagatgatacagatctatttggtgttcgtggggagcctcctgggactct
tggtgaggcatccacaggccttccagctgcagttggtggttgctttgctaaaagggactgtagcttcataagaaacacctgatc
atcttctgccataatttccttctcatgtacaaactttcgaacaggtggtttagttatgatgtcttcaaattatcttctgctttt
atgtttgaaaatttcatgtaatattcctattttcttatcattatcccacccctgctggataaatactgcatncttttncnnaca
cacagcagggatcttatagggaaatccatatagnttctgaacgatgtattatatactaagnctataattntgtngtctttactg
aagtgaaataagtgctgcaccatactgaaaaca
<212> Type : DNA
<211> Length : 789
      SequenceName : AI436551
      SequenceDescription :

Sequence
--------
<213> OrganismName : HUMAN
<400> PreSequenceString :
gccgtcgtctgtctggtgaacatctttctcaccgggagactcagcagtgcggttcctgcaccgctcatggacacaggcagcacc
agggcggccggagacaggcccccagtgcccccggccgtgcaccggaggtgggaccttcagggccaccccttgccctcggcctg
cgagacccagagtgcaggcccgtggacaaggacagataggtctgcagggagccactggcagacggcccagctcctgcagatgca
cctcgggagccctgctcagcccaggacccgcacccccaggtctccactgccagcagcaccctgggcccccactcgccagagcca
ctggggtctgcaggaacccccgccccctgctccccaatgctggaggccagcgatgatgaggccctgttggtctgttgaccg
ctgggctcgctggtgaccgccacagccccgct
<212> Type : DNA
<211> Length : 452
      SequenceName : AI652785
      SequenceDescription :

Sequence
--------
<213> OrganismName : HUMAN
<400> PreSequenceString :
tttgattgagagtgtgatcatgtttccaggtgagaccctattgcatctggcagcccgaattgggcaacaattcctgcaaataag
aagccactgctcttgttaggtaggtcatcgtcccataagccccactcggagcgctgtcataaaagaagtggcagattcctgtgg
cttggtctttctccagggtgtccatggcaccaattgacacctggtcctgcaagaacagctgactggccatctgcacgatctggt
ccacgtggatgatgcccccgatgctggtcatctccgtgtccgagagcagcgtcagcaccatgatggcttccaccagcagctgcc
ggtactcgggctgcggcacgcggttcagcaccgattcgacatggacagcaaacttgatctcatgcggggtcatctctcgggtcg
cgtaacta
<212> Type : DNA
<211> Length : 428
      SequenceName : AI311090
      SequenceDescription :

Sequence
--------
<213> OrganismName : HUMAN
<400> PreSequenceString :

gtgagcaatagtttttaatcacctggagtcagcaaaggagatggggtggggcagttttataggatttgggtaggtagtggaa
aaattacagttaacgtgcgttttctcttgtgggcaggggtgggggtaacaaggtgcttggtgaggagctcctgagactcattgt
ccaggagaaggaatgtcacaagatcaattgatcagttagggtggagcaggaacaaatcacaatggtggaatgtcatcagttaag
gcaggaactggctatttcacgtttatggttcttcagttgcttcaggccatctggatgtatatgtgcaggtcacagggttatgat
ggcttagcttgggctcaggggcctgacattgaggattctttttatcttcctctgatgctcttctataagaatgaatcggatga
agtgagggtctcaccccaacgcggccttagcttcctcggaaggaccgaacaccttggcggcagccgaggaaaggggttccacag
t
<212> Type : DNA
<211> Length : 505
      SequenceName : AI333013
      SequenceDescription :

Sequence
--------
<213> OrganismName : HUMAN
<400> PreSequenceString :
tttttttttcttctgggtattataatctttattacaaaacaagtattttatgttgaggtaatatttcaaatggtatttgtcaaa
aatcaattgaatcttacatacagaattttgctctgtaaaggactcagagaacagttttcttctagcattgcacgcaaactgatt
ttagaaaaaataaagctaaaaagtatctacgtaaagaggaatttctttctcccactcttcctgacccaatccttcttatcatca
ccagtgattagtaaggtgatgtttatgggagttctacaaaaaaaaaaatgatgaattggtaaattaattgggagaaaaagattt
tcttatccaaaacaactcactgtgccataatttcaaagtgcttttgcaaactaagtattgaatcctatagaatttactattatt
tatcccaagaatagaattccccggtctccaatgaggaaaccctatgcaaccttccaactc
<212> Type : DNA
<211> Length : 480
      SequenceName : AI371158
      SequenceDescription :

Sequence
--------
<213> OrganismName : HUMAN
<400> PreSequenceString :
acaaaaaaaaatttacaagtgaaatgttactacaaaacttttataaggaattttgcaaaacatttacattttaccatcaacta
tttctgttttaaaatcattatgtagatttaataccctatgctgcacatcaatttatgtgggatgacaacttagtgacatgcata
aaaaaacaccacaaggcattaaaatggagacttaaatacaaatattgttgcaataaaacagttataaaatttaaaaataggacc
taaacatcatgcttatctaagtttgacaaattaactttttctcctagattacacacttttattactgctctcatgagagtgtga
taaatgacttaacatcaagttctaatgtaatcagcaacacatacacaaacctaaccaaagaaagtatactgtaagaaaaaacta
ttctaacactggagttttaccatgataaatacatag
<212> Type : DNA
<211> Length : 456
      SequenceName : AI400473
      SequenceDescription :

Sequence
--------
<213> OrganismName : HUMAN
<400> PreSequenceString :
ttgtttttgtgcttcatgtttattttaggagagtattggggagcggtctggtctctcagggatggcactctgtgggtctgtatc
gcggtaggagttggaatgaggtgcagggtgggcttcaggaatggctggatccaggtatcatgttgctgactgtcatgattcggg
ctcatttgtaatacggcctctgtgtgttgaggtcgctgtagacatccgacctctgaccctggagctcctgataaggcgactcgg
tctcagtgatacgctgtttccgggtcgctgcctccgcagcccctcgcccccgagggaccagccggcccaggaagtacacggcca
gggcaatgagcactgtcagcaccaggtctcccatcacgatccctgccagcacgcccgggctcaccgtagagcaactg
<212> Type : DNA
<211> Length : 413
      SequenceName : AI423526
      SequenceDescription :

Sequence
--------
<213> OrganismName : HUMAN
<400> PreSequenceString :
tttttcaggcttccgggtctttaatggggcgggggctgggggcagttcatccagaggtggctccgaagctgaggtcaaccttgg
cggctggctcgcctactccagggccttcaccagggcctcgttggcctcgattcggatctggatctctgcccgcgtggcctcgtc
agctgtcctcaccagctccgcctgggccttctccaagtttgccttggctgcccccaggtccaacatgtccagcgtcacggcctc

ttcggccaacaactgcaccgaagaatcggcgttcactgcgatggaaccgctgctcacaaagtatttggaggtggtgccgtcctc
tgcatgcaccacgaccagccccggccgcaggacctgcagcgtgggcacgtgggccgccaggatgccgaaggctccggtcagcgt
gggcacgtccacctgccggacgttggcaccgttgaagaacacctgcgtgggagaggcgaaggtgaaggacatctggttgtggcc
agaggcggcagccggggcggcggcggcctcggcataggcacgggcgtggcggacgaggcggtcaagtccctggcggcggagcag
cgcggccggcagcatggcagcggctgtccggcaggatgcgggtagcttgctgacagcgggactccgcgcggcgcgggcggcctg
natggcga
<212> Type : DNA
<211> Length : 680
      SequenceName : AI436567
      SequenceDescription :

Sequence
--------
<213> OrganismName : HUMAN
<400> PreSequenceString :
atcttaactgcaaatttatttcattgattttaacattttgattttaacatataaatacaagaaaattaaataaatcagaaaag
aaatattgcaaagacaagtcagcactcacagaaatgtgtattattactatggttagtctgagatccaacattcacttgaaatat
atgcaagagaaccttagtgaaagacttaactaaaaggggtttgaaatgttcctaataaggatgtacttgaggcactgctcaagc
agatgtaggtaggaagcaagatagaccctgtatataactaggagcaatcaaggctcgatacatgtagcaacaatagcagtgtag
gacagttccctgttcccaatagctactccccttgtcactgtgcattcttcaagacacatttaaaaacaattatagctctggct
gggccagagtgtaccagtcataacaatcaacaatgtaccatgcagt
<212> Type : DNA
<211> Length : 466
      SequenceName : AI521155
      SequenceDescription :

Sequence
--------
<213> OrganismName : HUMAN
<400> PreSequenceString :
ttttagggctgagaaaatattcagtttattaaatattgtgaatgggtggaatatcagatacatcatctcaatatgtaaatatcc
tatcccatctcaacatcaaatttagattacctggatttcttctttgcttttctctatgattacaacagagatcattatgtattt
tattagtttcttcagtctttaagatatttttgaatgatgttcaaataaactccaagttatctccaactttttctgaacaaaat
atttccattcttaagatacaggcttgtaattcacatacttgatgctactcaatggcgtcttatctgtattttcttctcacattt
gactccagagtattcagtgcggcaggaacacacattgggaaatatgcatctgcctccataaagacattntgggttgcaaattgg
tatttgacaccgcactccttcccaggaggaaggacaatggcatatgctgggcgcaatgcattc
<212> Type : DNA
<211> Length : 483
      SequenceName : AI580830
      SequenceDescription :

Sequence
--------
<213> OrganismName : HUMAN
<400> PreSequenceString :
caggcactttttttataaaaacgcatagtctcatttttcactctttaaaatcacattcaaaaatcggcaaagaaacgccccgg
ggaatgagtagacgggaggggagggtgcgccaacccagccccgggggggagaaggcgcgacaccacacaggcagcgcgactggc
acgagggggcatgcggtgggcgaggcggcggcactgacaggacgtgggctgtggggctggccagcctccttgctgtgctgcccg
cccacctggccggccccgtcctgggcagatggggtcgcacccagcccagcatcagcgagacctcaggccaggcgaggccagtat
ggcccaacctgcaggtgccttgctgtggtccctgagcccgggtgctg
<212> Type : DNA
<211> Length : 383
      SequenceName : AI739095
      SequenceDescription :

Sequence
--------
<213> OrganismName : HUMAN
<400> PreSequenceString :
ttttgagtcatttcttgttctgtcaccaaggctagagtggagtggcacgatctcagctcactgcaacctctgcccccctcggtt
caagcaattctcctgcctttgcctcctgggtagctgggattacaggcgcctgccaccatggctgcctaatttttgtatttttgg
taaagacagggtttcaccatcttggccagtctggtcttggactcctgacctcatgatccagccatgtggccgcccaaagtgttg
ggatgagaggcgcaaaccaccatgccccgttctctcccacaatctttagagaaacattggcctcagacctcatggcctataccc

tgccaagtaacaactgtgccttgttgcagtacatagacgagctttctttgcctgccccaacctgagaggagtgttatcggggaa
cccaggatctcctccatctcctatagaaagcaagttacaaaatatcaaaaagaaagcccaaattttccatgaagaggttacat
atctaggtgtcatattgagccacgaggaacactggtttggctatgagaagaagcaagccatttgtgcacttctaaccccgacta
cgccgatgacaataggaagtttt
<212> Type : DNA
<211> Length : 611
      SequenceName : AI742120
      SequenceDescription :

Sequence
--------
<213> OrganismName : HUMAN
<400> PreSequenceString :
tttttttttttgtttctcactgcctgtataatcaggtctttattcaaaagaagctgtccaaaatgatttgacctttatggaata
atcaaatttaagagtttatgcagcaggctttcttctcctctgtataggtttttttttctgcaggcttcttttcaggggctggttt
cttggtagctgctgcctttttttcccaccagaggcttcttctgcttcttaacaccaacagcatccttctttcctttcttacctac
cacaggcttcttgcctgcaaccgccgccttctcatctgatttggcttgtagtgccgctgctgcagcagctgccttatccacccg
gagcttgtgattcctggcctggcgaaaaaatgtgttccggcgcatggtctttgcatatgggttagcttcaacatgattctcaag
ttttcagtgg
<212> Type : DNA
<211> Length : 430
      SequenceName : AI762926
      SequenceDescription :

Sequence
--------
<213> OrganismName : HUMAN
<400> PreSequenceString :
gggtttggagtagtacctgcggcgtgctcgagactccgacttcggtcttgcggcgcgctcgcgcccgcgggccatgccccactg
actctaagtgggcactgccccggctccgggagggcgagaccgagcgccggccatgggaagcttccagctggaagactttgcggc
gggctggatcggaggtgcagccagtgtcatcgtcggccaccctctggacacagtcaagactcgcctgcaggctggcgttggcta
cggaaacaccctcagctgcatccgcgtggtgtacaggagggagagtatgttcggcttcttcaagggcatgtccttcccccctcgc
cagcattgccgtctacaactccgtggtgtttggggtcttcagtaacacgcagcggttcctcagccagcaccgctgcggggagcc
agaggccagtcctccccgcacgctgtcagacctgctcctggccagcatggtg
<212> Type : DNA
<211> Length : 472
      SequenceName : AI791539
      SequenceDescription :

Sequence
--------
<213> OrganismName : HUMAN
<400> PreSequenceString :
cagatagcccaccaggggtggggagggtgagcagggacagggcgcccccactgacttgggaccctcctcctccaggcccacacc
tcagcacccaggacatctgggcccccgcccccagcgctgtctagtttggttgcctggccgtcactcccagcctggttcccact
cctgtgtcttctggggatggccctcaaggacagcatgttgacacatcaggcccagctctatcactggggagggagataggctgc
cagggacagaaagggctctttgagaaggccactctgcctggagtgggggcgccgggcactgtcccccaaggtcgcggcagagga
gataggggtctgtcctgcacaaacaccccaccttccactcggctcacttaaggcaggcagcccagcccctggcagcacccacga
tgcgggacctgcctctcaccagcctggccctagtgctgtctgccctgggggctctgctggagactgaggccctcagagcagagg
agccagctgtgggcaccagtggcctcatcttccgagagacttggactggcctccaggcatcccacaagagcctctgtgcctggt
ggcactggggcgggacagcaatggcagcagctccc
<212> Type : DNA
<211> Length : 623
      SequenceName : AI791949
      SequenceDescription :

Sequence
--------
<213> OrganismName : HUMAN
<400> PreSequenceString :
ctgttactcagggtaactgtctgctctttcattgtgcccagagaccacatgttgaaaaatgtccttgggagcttgaacttgtaa
ccacatggcagttctttctcttggtttccaccatccaacagataggaatttgggggttcatgtcatacttatccctaaaaatta
tcttgaacagttaaaagactttgcaagctcagaattggctgctctaaaatccttctgggaagaacaacggaaaccagccaatgc

tgtagctcaatagctaaggcttcaccattttacaatggtggcccagggttcaatcctggcttagggaatgaatcctttctggtt
taaaatctgtataacctttaccactggttaattctcttcctctccacaaaccatcttgaactttcctttctctgagcaaaaaat
atcggctgtttggcatggctaaagtcaggtaataagatatttaaaaggatttgagtgctatggttaaaagtcagtttgttcaag
ctctaacagcct
<212> Type : DNA
<211> Length : 516
      SequenceName : AI796718
      SequenceDescription :

Sequence
--------
<213> OrganismName : HUMAN
<400> PreSequenceString :
tttttttttttaaaggaagtcaaaaactttattcaggtgataaaattccatgttccaagaacatgctctaagcatgcaaaaagc
acaggacactgctcccgggtgggggctcaggtggggccacctctgaagctctgctgacccctgggccaaggccctgacagacta
gcaggcccagtggtcatgaaagcgtgacggccaccacatcacaagcgtgatggcacctgcntggtgcagctgcagtccctggtg
ggccgggcactcactctggcagccgccgtcccccacgccagccatggttgtcccgccatacgcagtagttgagtgtggtcgtga
aggccagccaggccaggtaggggtagagcaggcgggcggccagcgggctcacctggtaccaggccacggtagtggctgccgccg
ccccactgaccagcaggagatccaccaaggcccagcccatttgtcgggcaccaaagaagatgggggccatgcccagttcaggg
ccagctgcccagtgtagaggcccaggggaaccacagccttctctgtgaagcctcccagctctttccagaaccagtaggagccgt
accccatggctgagtaaagcgtgccccagacagggcccagcaccagtgggggcggtgccacgagggcttctgcagggccgcgta
cccacggagacccctcgccgtgacaaagcggagcccacgaagcacccccagctgggcggccacgtgaagcc
<212> Type : DNA
<211> Length : 743
      SequenceName : AI808621
      SequenceDescription :

Sequence
--------
<213> OrganismName : HUMAN
<400> PreSequenceString :
ttcagttcaacatacatttattaagcacagatggtataggatactgattgccccccagccctctgtgctccccccacgcacac
gctgtctatgtcgattaggctgctgtgccgtcggttcccttcttggtctagcagagccttcagctccttgatctgctcagccaa
ctccgggttcaagtcaaactccgctgggaaggcggagatccagtacctgaccaggtggcacgttttcacctgcagggaattgga
gttgtccttccgggattgttggtagatgtggagcagcttggccgccagcgaaggcttcgatgcacccgcggagcagctcctcca
ccgtgcagcccttgtccaggtccagggtgcctgccatggccgcccggcgcgggtgggctgggcccaggctgcgctccgggagcc
tcccaccgggctcggaccgaacccct
<212> Type : DNA
<211> Length : 446
      SequenceName : AI809036
      SequenceDescription :

Sequence
--------
<213> OrganismName : HUMAN
<400> PreSequenceString :
ttttttaaactagatctaagaaagaaaaagtcaacactgatatacatgttgcttgagccaaaagacataggaaaaaaagacaa
catataaccattaaattcctaagaaatatgaggtaaaaagatgaaatctttagataatttctaagtctgtacaaaaaagctaga
tttgctactctccaaaaagtggaaggacctattatataatatatggaaataatttaatgccatatcataagaatgtaactagag
ctgtgctaagcttcatattcgtgagggcatctaaaatgccactccacagcacaggtgcttctctcttctatccatttatgcggt
agttttcatggatttctggccgaatgtcacagacaaaagccaagaggttgtccaagacttcatccctgttctgccggaagtatg
tctggaggatggtcatcttctcctgggtctccttctccacttcagtgctgcaactgccacgggatcccaatgccgcagcttcct
tggccttgaattctttctccctctgcaggcggtactgttcaatttcagcctgagcttcttctttggcctgcttcagcctccggt
tctttcttttgcgggcctcggacaccttctcggctgcccgcttctcggcctgcagcagctgctgaatccnctgagactgactag
ccat
<212> Type : DNA
<211> Length : 676
      SequenceName : AI816398
      SequenceDescription :

Sequence
--------
<213> OrganismName : HUMAN

<400> PreSequenceString :
tttggccattcagtggccaatctcctctccaactgtccccagccgaactcttctccgtgttcacacactcctcttctctccgcc
aggccattctgccattctgctcttctgttcatctcatctgcttgtggagcctggggtttggggtttatacgggtacaggatagg
ggagcatggcaggtcaaaggcaactttgggggcgaaaacaggaatgcctgtcctcatttagggccgcaggtttccaggcttg
agggtgggcctttgccagggaactgccctcttctatccagtatttccctgtctcctgtgtgtatcagtgacaccctgtctctat
taaaaatacaaaaattagcctggcattgtggtgcatgcctgtagtcccagctactcaggaggctgaggcaggagaatcgcttga
acctgngaggtagaagtttgcagtgagctgagatggtgccactgcactccagcctgngtgacagagcanagctccatctca
<212> Type : DNA
<211> Length : 501
SequenceName : AI821337
SequenceDescription :

Sequence
--------
<213> OrganismName : HUMAN
<400> PreSequenceString :
agtagagacggggtttcaccgtgttagccaggatggtctcgacctcctgagctcatgatccgcctgccttggccttccaaagtg
ctgggattacaggtgtgagccgcttgcacctggcctaacatctttctatggaaggctgaatgcataattacaacaatctgatga
gccaaagtgttgatagcagtagagggcagacaaattcctaggcagacaggggcaggtccccaatgaaacctgatcttcaagcca
tagacagcctgaagcctgaaaaccaggcagccagctccaggtagagtccatgacctgagtgagaacttccttgatgcttttata
gccaggcaaatggtgctttttccaggactgcttatggaccaatcagcacacactcccccattctgagcccatgaaacccctgga
ctcagctacatgttgggactacccaccttcagatatgggttacccacttcgggtcccctctcatgtcaaaaact
<212> Type : DNA
<211> Length : 494
SequenceName : AI885557
SequenceDescription :

Sequence
--------
<213> OrganismName : HUMAN
<400> PreSequenceString :
ttttgagttcttcaaaattatttattgatgcgtggagggaagcacaacccagagaactgggcttggctctcagctgtgtgtgta
tagcacagtctcaaggctagctcttgacttctaggaaggtctttagggtcagtagggcagataattacagcaaagtatcattgt
atgtggatctactctccctctccaggcggaatgtttgaaccccaaccgggccccggcacacgaaagtcacatggctccccaggg
ggatcacggtgcctggctcagccgagatggagggtctgggcagatcttcctcctgcgtgtggatggtctgggccaggcagagca
ctaggcccaggagggcggtggggtggggagacatggcccaggtcccagcagtgcagcctggcctgaggcgcaccaatgcaagga
<212> Type : DNA
<211> Length : 420
SequenceName : AI921879
SequenceDescription :

Sequence
--------
<213> OrganismName : HUMAN
<400> PreSequenceString :
tttttcaggtatcaaggctgtttattgactcttcgcagccagcggctcttcctctgcccctctgggatgcctaatccccgtctc
ccccctggagccaagtggagtggctctgagcttgaccccaggcgccttcctcttgccaggcttccctgccccgcagccttggcc
ttcctggtttgccacaggggctgttactcctttgattctacatcctcctcgaagtgtgtgaggtaggccatctgagccgaatcg
cgcgagccatagagagtgttcatgctgtaggagctgccatggaggcacagggctttggtaacattgactgtgttggccgccaag
atgtccgcctgaatgctgtctattttcatgtacaagtcctcagcgttgctctgaggacaggagcaaggggggacccggctggggt
gccagggcagtctggacttggagttgggcttggcagagggatgt
<212> Type : DNA
<211> Length : 464
SequenceName : AI638342
SequenceDescription :

Sequence
--------
<213> OrganismName : HUMAN
<400> PreSequenceString :
ctgttgcacactgcaaatggaaagttctggattccacctagggcaccatcgggaggccaatggggtcatatggagacacacgca
gatcctgcagcaaagcttctaggttcttcctcagcatggcgtagggcggcttctcctcatacgtgagggccatcaccaccttca
ggtacttctgcagggtctctgagggcctgatccagtgaccgcagggtcccacgaagggccccggcttatcaacaaacttctgtt

tttgcttcatgatgtcctcagtgttgggaaggcaatttgtccatggcagaaacccgtagagccacttcagcatgcagtagccca
ggctctggaggtcgctgcggcgggagggcccgcatcccttgtgcaggtccatgctaatgaactcaaggtccccctcgtgagggc
tcctgctgccttccacgtaggccacgtgtttgccacttgggcaatagcggaaggcgaagccatagcctgccaaagtcacctgac
tctggtcctctggatccacaaagatattntcagctgtcacatttccatgaacatactcattctcatggaggaactccanggcat
ccagcagcccgcaggccacctgcagcacaga
<212> Type : DNA
<211> Length : 619
      SequenceName : AI651159
      SequenceDescription :

Sequence
--------
<213> OrganismName : HUMAN
<400> PreSequenceString :
cagcctggtggctccacaggatctggtccaagctacctttatggggtcactcaaccaaaacacctctcagcctccatgggtggc
tctgtggaaatccccttctccttctattaccccctgggagttagccacagctcccgacgtgagaatatcctggagacggggccac
ttccacgggcagtccttctacagcacaaggccgccttccattcacaaggattatgtgaaccggctctttctgaactggacagag
ggtcaggagagcggcttcctcaggatctcaaacctgcggaaggaggaccagtctgtgtatttctgccgagtcgagctggacacc
cggagatcagggaggcagcagttgcagtccatcaaggggaccaaactcaccatcacccaggctgtcacaaccaccaccacctgg
aggcccagcagcacaaccaccatagccggcctcagggtcacagaaagcaaagggcactcagaatcatggcacctaagtctggac
actgccatca
<212> Type : DNA
<211> Length : 514
      SequenceName : AI700673
      SequenceDescription :

Sequence
--------
<213> OrganismName : HUMAN
<400> PreSequenceString :
tggccctgaacggctggggtcactgatatttattgcatacaagacatgggggcagggtaaggagggtgaatcttctaagtgatt
gacaaggtgaagcaagtcatgtgattacaggataggcggcccttccccttttaggtagccgaagcagagagggaaggcagcatat
gtcagcgttttcttctctgcacttgtaagaaagatcgaagactttaagactttcactatttcttctaccgctatctactgcgct
cttcaaagaggaaccaggagtacgggaggagcatgaaagtggacaaggagtgggaccattgaagcacagcaccacagggaaggg
tttaagcctccggatgactgcgcgcaggcctggataatatccagccttccacaagaagctggtggagcagagtgttccccaact
cttccaaggaaaggagactccctttcgcggtctgctaattaacgggtgcattcccagacacttgcgttaccacttgaccaagga
gccctcaagcggcccttatgtgggcgtgacagagggctcacctcttgccttccaggtcacttctcacaatgtcccttcagcacc
tgaccctatacccaccggttattnct
<212> Type : DNA
<211> Length : 614
      SequenceName : AI732875
      SequenceDescription :

Sequence
--------
<213> OrganismName : HUMAN
<400> PreSequenceString :
ttgataatagttcattttattaaataatttataaagtcagaccagtgcattgggcttgttaggggaaaccattccttcctactg
tcacatagcctggcagattatatcaggtgccaaaacattcccttttattcaatttattaatgagagtgtggaattaaagtagat
ccttgcatgtcaggaaagctgcaacacatctggtttaagtggagcacaaaggtaagttattccacattcaccaacttccccact
tgacctcccaccagttcctgtctgctttttccatcaatcaactctaagatgccctaaacccaaacagttcccattcaactgtgc
acaaaggtatagatatggaagacatctcactccaactctcacctttggaattaaataaacctggaacagggaaggtgaactatg
gctgaaaccaagtgaagattttaagaagctttgacagaaacattaagaataacacctgaatgcanatccagaaaaaagcgcta
ctgggggaaaatagtcttagccaatgttctaaaaatgctcataagggagggttggggaattaccctttagacacaagctctaag
aactctggataccaacggaacttggatggatacagtctgggcctgctgggncagatgttccgagggcggcccggcaagcagcct
gtcttgcacatttgcactgactggcttatctacggcagagtcct
<212> Type : DNA
<211> Length : 716
      SequenceName : AI762738
      SequenceDescription :

Sequence
--------

<213> OrganismName : HUMAN
<400> PreSequenceString :
agacagagtctcgctctattgcgccactgcactccaggctgggcaacaaagcgagactccgcctcaaaagaaaaaaaaaagca
tgggtttgtgtaccaaacctcagtcctccatcatcctccctgtacctccttttcctttttgttgagacaaggtccagcctggg
tgacagagtgagactccatctgaaaaaaaaaaaaaaaagcatgggttttgtgtaccaanacctcagtcctccatcatcctcc
ctgtacctcctttttcctttttgttgagactccatctgaaaaaaaaaaaaaaaacgtgggggggccctccaacatc
<212> Type : DNA
<211> Length : 332
      SequenceName : AI766478
      SequenceDescription :

Sequence
--------
<213> OrganismName : HUMAN
<400> PreSequenceString :
gaacccgggaggcgaaggttgcagtgagccgagattggccactgcactccagcttgggcaagagcaaaactgtctcaaaaaaa
aaaaaaaaaaaaaaaaaaaaagctacaatactcccctcacaaactggaagcctcaagctcttcaccctaaaatagttctgttga
attttaccctggcaatgtaaactgatagcttatctgcacaggtgtgggacagaacccgtccctctgtacacctgagtcaaatgc
ctatctgactgcttcccctgccctatttatgtaaaaattcagagtcaatgagccagattaaggcataagtgactattcctctac
tccctctcatatataaattgtgtattcagtgaaaggctgatgagtcactcaaagaatgtgataatttattatctacctgtgacc
cggaagccccccaattccagtttttccacctttccagaccgaattaatgtacatcttatatgtattgattgatgtctcatgtct
ctgtaaaatatgtaaaaccaaactgcagcctgaaaacct
<212> Type : DNA
<211> Length : 543
      SequenceName : AI815593
      SequenceDescription :

Sequence
--------
<213> OrganismName : HUMAN
<400> PreSequenceString :
cttcacagattttcctaaagaaacttctgactccctttcccttaccctttcctcttcttaatgctctgttacgaaagaaaaagc
tgatgttgctgttgagagtctcataccaggactggagccccagttccccagcgcctcccatctcagacggaatctgtgacctc
gaatcgcacagattctctcaccggggaagattccctgcttgattgctctctgctctctaaccctactactgaccttctggaaga
gtttgcccccacagcaatctctgctccagtccataaagctgcagaagatagtaatctcatctcaggttttgatgtccctgaggg
ctcggacaaggtggctgaagatgagtttgaccctattcctgtattgataaccaaaaacccacaaggtgggcactctagaaacag
cagtgggagctctgagtccagtcttcccaacctagccaggtctttactgct
<212> Type : DNA
<211> Length : 471
      SequenceName : AI821217
      SequenceDescription :

Sequence
--------
<213> OrganismName : HUMAN
<400> PreSequenceString :
gcggccgcaagacccgccacgacccgctggccaaatccaagatcgagcgagtgaacatgccgcccgcggtggaccctgcggagt
tcttcgtgctgatggagcgttaccagcactaccgccagaccgtgcgcgccctcaggatggagttcgtgtccgaggtgcagagga
aggtgcacgaggcccgagccggggttctggcggagcgcaaggccctgaaggacgccgccgagcaccgcgagctgatggcctgga
accaggcggagaaccggcggctgcacgagctgcggatagcgaggctgcggcaggaggagcgggagcaggagcagcggcaggcgt
tggagcaggcccgcaaggccgaagaggtgcaggcctgggcgcagcgcaaggagcgggaagtgctgcagctgcaggaagaggtga
aaaacttcatcacccgagagaacctggaggcacgggtggaagcagcattggactcccggaagaactacaactgggccatcacca
gagagggg
<212> Type : DNA
<211> Length : 512
      SequenceName : AI824146
      SequenceDescription :

Sequence
--------
<213> OrganismName : HUMAN
<400> PreSequenceString :
gtgctgacttgcgggaccgcctggaggaggacccggcgtaggccagaccaggagctgaccgggagctggggccacgggcctagg

agcaccctggtcagggctaaggccatggccccgcccccaccgccactggctgccagcaccccgctcctccatggcgagtttggc
tcctacccagcccgaggcccacgctttgccctcacccttacatcgcaggccctgcacatacagcggctgcgccccaaacctgaa
gccaggccccggggtggcctggtcccgttggccgaggtctcaggctgctgcaccctgcgaagccgcagcccctcagactcagcg
gcctacttctgcatctacacctaccctcggggccggcgcggggcccggcgcagagccactcgcaccttccgggcagatgggcc
gccacctacgaagagaaccgtgccgaggcccagcgctgggccactgccctcacctgtctgctccgaggactgccactgcccggg
gatggggagatcacccctgacctgctacctcggccgccccggttgcttctattggtcaatccctttgggggtcggggcctggcc
tggcagtggtgtaagaaccacgtgcttcccatgatctctgaagctgggctgtccttcaacctcatccagacagaacgacagaac
cacgcccgggagctggtccaggggctgagcctgagtgagtgggatggcatcgtcacggtctcgggagacgggctgctccatgag
gtgctgaacgggctcctagatcgccctgactgggaggaagctgtgaagatgcctgtgggcatcctccccctgcggctcgggcaac
gcgctggccggagcagtgaaccagcacgggggcccgcgggaggactcagactccagcacttccagcagcgcctgccctctatgg
acgacagcccggagctgcccacgggctgcagccagtatgccagggagctgccctcttcttccacaacagctggctctggggttc
tcaagatttattcaggatcgtgttaacggaggcggtgggaggatagggtccctgacgtgccggggacacacacagagaaccctc
cccgcccccgcaagggagggggaggctcgctttttcttaaaatataaatgtatttatctgcaaaaaaaaaaaaaaaa
<212> Type : DNA
<211> Length : 1172
SequenceName : AK000599
SequenceDescription :

Sequence
--------
<213> OrganismName : HUMAN
<400> PreSequenceString :
tgacttggcccacagagctttattggagagatacacacaaaggctgtccactcacttccataatttcttgatggacatgttttt
ctcactgtccttctgcatgaccttggctactgccatctcaaagtcctcctgagtgacatggactcgccgttctcgcagggcata
catgccagcttctgtgcacacgcccttcacttcagcccctgatgctcctggcatgagctcagcaatttttctcaggttgatccc
ccgggtcaggttcatcttccgagaatgaatcttcaaaatgtccagccgggcctcctcattgggggtgggaattcaatttttct
gtcaatgcgccctgggcgaagcagtgccgagtccaggatatcaatcctattagtagccatgataaccttgatgttcttggtggc
ctcaaagccgtcgagctggttgagcaactccagcatcgtgcgctgcacttcactgtcccctccagaacccccctccagccgcga
ggagccgatggagtcgatttcgtccatgaagatgatagatggagcatgttcccgtgccatgacaaacagctccctcaccattct
tgccccttcccctatgaatttctgtaccagntcagagccagagacacgaaataacgtaca
<212> Type : DNA
<211> Length : 648
SequenceName : AW006368
SequenceDescription :

Sequence
--------
<213> OrganismName : HUMAN
<400> PreSequenceString :
tagcattccatcatgtttattgactcctggggggacaggtcacaaagtcagtttgtgggcaggccagactgccctagaaggaagt
caggggcctcaaggggtggcactcttccttaactcgtaactcttggaggcaagcttggaaggtgctttatttcccgctatgatt
ataccaaccctgtggcctgctccaggcttcaggatctttagggccttcttgcgaacctactggtgggggtgctgcaagcccct
ccccttgcccgaaggtgccaagccccatgtgggcaaggcagttgtctgttgcatagtcaagtagttgttgtctccaacttgcac
cccagagcagttgtatatgttaacgagcggtcgccctgttactggatttggctccggggtcctgcaggaccagttcatgccttg
tctctcagccccctgattccctcggggtccaggacttggtgttccagttga
<212> Type : DNA
<211> Length : 471
SequenceName : AW009940
SequenceDescription :

Sequence
--------
<213> OrganismName : HUMAN
<400> PreSequenceString :
tttaaaagaaaagacacctgggcctgggggaccactaccaccaagacgcggagaccagtagtggccccaaatgccaggctgcac
tgatatttattggatataagacaaaggggcagggtaaggaatgtgaaccatctccaataataggtaaggtcacatgggtcatgt
gtccactggacaggggggcccttccctgcctggcagcagaggcagagagagagagaagagagagagacagcttatgccattattt
ctgcatatcagacatttagtactttcactaatttgctcctgctatctaaaaggcagagccaggtatacaggatggaacatgaaa
gcggactangagcgtgaccactgaagcacagcatcacagggagacaggcctctgata
<212> Type : DNA
<211> Length : 393
SequenceName : AW078834
SequenceDescription :

Sequence
--------
<213> OrganismName : HUMAN
<400> PreSequenceString :
tttttttacttaaaagataaaacaactttattatgacatcaccattatatacagctgtgcttttcacatcaagttggcttagt
tcttggaggaaagtcttagtgactaaaattgtacacagatcacaagacaagagaactagtctaacaaattcaataagaaatcag
gacaactgtagactatataaaaactgtcttagaaaattatttacaactttatcatagtgtaaatactctaaatgtaaaataaat
ctatggattttatacaaaaatactctaaagtacaaatgtaagttaaatgctgaataaaacattttcaccttttccagtggatgc
tattactaagactataatctaggtgtcaaaacttaaagtgataaccacaaagcaatgttttcttaatcactcataattttacat
ttgttagtagtcatcacaaggtttagagttccactgaaaaagtctcattttacaaatgcaactcatacactntccttcatgga
ccctttctctg
<212> Type : DNA
<211> Length : 515
      SequenceName : AW129770
      SequenceDescription :

Sequence
--------
<213> OrganismName : HUMAN
<400> PreSequenceString :
cagattcctgggtgcaaggtggcctcattagtgcccggagaccgccccatctccagggagcagatagacagacaaggggggtgat
caggggcacagtgatccaaccctggcctctgaacgccgcagcggccattccttgggcccagcctggagacggccccccctgcagc
aggctaatcttagacttgcctttgtctggcctgggtgtggacgcaagtgcctgtcaattccccgccacctcagagcactataaa
ccccagacccctgggagtgggtcacaattgacagcctcaaggttcctggctttttgaaccaccacagacatctcctttcccggc
taccccaccctgagcgccagacaccatgaaccttccttccacaaaggtctcctgggccgccgtgacgctactgctgctgctgct
gctgctgccgcccgcgctgttgtcgtccggggcggctgcacagcccctgcccgactgctgtcgtcaaaagacttgctcttgccg
cctctacgagctgctgcacggcgcgggcaatcacgcggccggcatcctcacgcatgggcaagcgaggtccnggcaccccggcct
ccagggtcggctgcagcgcctactgcaggccagcggcaaccacgccgcgggcatcctgaccatgggcggncgcgcaagcgcaga
gcccagcgcgcgc
<212> Type : DNA
<211> Length : 685
      SequenceName : AW163686
      SequenceDescription :

Sequence
--------
<213> OrganismName : HUMAN
<400> PreSequenceString :
ttttttattggaagggggggtttaattgtttagaatttcaaaatagtattgggatgtgaaacaggatgattatcactctgataa
aaacaagactgaactgagtccttcactgtatttttctaaagaactcacttctagtgagtaagtgttcaataacataaagttagg
aaatctttatgttattaaaatttgaattttctttcactgtatcccaatattttaaatatgcgcttcaggacatttccagttaa
aataaaagtttaaataatatcctcactacattaaaaataagatctttaagtaaacgtatttccaaatcatattgtcattgtgca
ctgaagttaattctctgaagactaaattatatgcaattgaggttctgtttcccttctaaacgttcctttactaattttccaaa
gcgaaaagatgctcatctgcttcttctggcacaaggttcctaatagaatttgcaagttcaaaaagatattctgtatttcttcca
cttggaccagctgcattaaaaatttgttcagcaatgtcttccagaggtgcaggaccaaagatatcaggattatcacatgttcca
atatatag
<212> Type : DNA
<211> Length : 596
      SequenceName : AI867297
      SequenceDescription :

Sequence
--------
<213> OrganismName : HUMAN
<400> PreSequenceString :
ttcaataaaaagacagatttctttacacagatttaagccaataattttagcaaaatgatacaaaactgtcttaaccaagtag
aagattggtagttacagtggaatcgtcagggagtacagggcggccaccactggagggagctgaggccctggaaaaggagtctga
ttctctgcaattctctctctgcttttcttcccagccccgttacaaccgagttcacgtggggggccgcagtgcagccccagcggt
ggcagctcttggagtctgtccgtttagtatgtttcccccacgagcgtcgctgggtgagtggcctggagagctcccggtgttaac
atttcgatcctagaccgggggacgtgtcactagtaaagccattggtaacagagtagatcagccatgcattgtctgcccttcaca
gcaataaggagagttctcatcggtgcacgacagactgaagaccactggaagccacccgaccaggaatccagtgctgccttccga
tgggagacgagcagctacttgccgtggtgttcgaaaggaatgctattctgtgaggtggacatgcgagaggtatcttgtcggcct

gtgaggtcggacgaggagatgttgacgggggcnccgcggtgcagccgcatactcactttccgctctctctccatgccggagacg
gggccgggggggagtgtanccgtgtntgaaaggtaggggtanaagggttggaatncacttccaatactctcctaagcgagtcg
gaatgtgtaggagggaatgcgttagatacagg
<212> Type : DNA
<211> Length : 788
      SequenceName : AI884916
      SequenceDescription :

Sequence
--------
<213> OrganismName : HUMAN
<400> PreSequenceString :
ttctttctttttctttttctttctctctctctttatctttgtagctatcttctttaggaataaaatgtgaggcaaattgtctga
tgcaattcccaccttgactttttcatttggcttagtgatttggggtcctgagatttattttcctttcacaccatatacagaag
aagagtaaatataaaaacactttcttgaacagcaggctagcagcagggaacaggggcatgggtgtggaaagttttcaaaagagt
ttggcgtgaaagatgccatatatactgttccaacacttggaacacaataactagagacacagttgtgcatgcgtggtgcaatat
ctggcctgcaggtattctcagtgatgatgaagaacaaggtagtgactttgaaggattctgtatgtcaagttgaaaaaaaagtct
gacctcctgccatatgtgaaatatacaccttccgagtctgtcagtaggctgaaagaagaggatatggaagaagt
<212> Type : DNA
<211> Length : 494
      SequenceName : AI949694
      SequenceDescription :

Sequence
--------
<213> OrganismName : HUMAN
<400> PreSequenceString :
tctcattattcaggcgctccagctctgacacgtgcacttctttattggaaaagaataaagcagtcactgatgtggcaggggcag
gacacgagcagctgccgtcctcctcccagcgtgcctggcatggtcgcaggggagcgggtgcctggagtcccggtgacaccacgg
ggcacactgagggagctgaggagccggggccgcgcagcctcctggatgctcagcggatcgtgtacttgtcccacttcttttcag
ggtcgtagggttcccaccggctggcgggaaagatgtgcttgttcttctcgtaccagctcctcagcaccaccttgcctgcatggg
actcatccttctccacagtggcgtcactgagcaaccgcacatcgtcatgaacatcaaagttgaagagtggtccactcttccccc
gtgccttggtgacgatgaagtcgtagaagctgtgatgctgggagccaatggcaggaaagcggcgtcttgatggtccatctcggt
ccgtccccacacccccccgactgcgcacactgtccccatcagcctgagccagagccgaatgtgctgcacccactgcagatactgg
ggctgaagggactcacgtgagggatgatcaagtcctgctagatgtacatgagctgactcacccctgctgacctcaggctcactg
agtcttatccgaagatctcgagcgccttctg
<212> Type : DNA
<211> Length : 703
      SequenceName : AI962797
      SequenceDescription :

Sequence
--------
<213> OrganismName : HUMAN
<400> PreSequenceString :
tttacattttagaccatttaatggaggtatttgttgcacatgtggttttagtgtaacgtaaacacaacatctctccccagaaac
acttaacctgagttggaaatgaaatatgaactgaagagattcttaattcacacatacacttctcctcaaaatattagcaatgat
tccaactgaggattctgcttgaagttcaggactcggtcccaggacaggaatgaaggaaaactccgtgcagttcgaacgctctgc
tgaagtcaaaggactgcacctgcgggctcttaatcagagctagcttcctcaaacacccgcggaacgaggtctggctgcgcaggc
atttttgcttcacaccagcaggatagccaccaacataaatgggattgttggtgtccactgaggtagactgggtgtgtggacttt
cagcgccaactgcgttcccatcaacaatcagagtgatacggtgtttgcttttgttagcttgaagagtgtgccattntccatcac
agagcacagtggcggttntgggctcatatgcagctgttatcctgccagcaccattgttgacatggaacaagaccttgccgtcca
caagctctagtccaatggcatgcactatggcagtgctgatccccaggaagacgccatttctgcgagaggttcgaaacttccagt
gtgatgtcacatctgactggactttgtagccctctttgacaagagctgcatatncgctcccgcaaagtatgttctttctgggcc
actgcgtagcacctggtcaccgtgaa
<212> Type : DNA
<211> Length : 782
      SequenceName : AI990816
      SequenceDescription :

Sequence
--------
<213> OrganismName : HUMAN

<400> PreSequenceString :
gcacgaggattaatggaactactttgttttgctatgcttttaaagttctaagacaaccctcttctactaccaggttggtctag
ataactgtggtatgtcttcaagtttctagatgttcataagttcttttattacctaaaacttttcttcttaaatttcagaacaaa
acagaaaactttcacagtgcaggttgagtatgccatatctgaaatgtattggatcagtttttggattttggactttttcagat
tttggaatattcacagagtacataccagttgagcatcccaaattcaaaatctgaaatgctccagtgagcatttcttttgagcat
cacgttggcatttgaaaaagttatagaccgggcatggtggctcacgcctgtactcccagcactttgggag
<212> Type : DNA
<211> Length : 406
      SequenceName : AW022174
      SequenceDescription :

Sequence
--------
<213> OrganismName : HUMAN
<400> PreSequenceString :
tttgcttgagaggagtgtgtggtcattacacggcctcctggcccagctgctgtcacagcccccagtgcgagtgcccacggggc
tcggggtgtctacacctctagccccgccacctccgtcagcccaaggtgaaaagggccctgctggccctgggcggctgcaccta
ggtctccaggtactggtagagctcctccgctgggggcacgaggctcaggtggaccaaagcacaaccaggagggcggtcagccg
catggccaaggtctccagcctgagcgccacttcaaacttgagcatctcccagacgaccttggcgtggcgcaggaggctggtgcc
gtagacaccccgatacgtggcgcccgccaggcggctccagccccggacagcccttttagccataaggaagtagcggtccacggg
ggcgccgagggccacgttgatagcgcgcacggtggtgatgttgcgcagcaccagcagcatgggccg
<212> Type : DNA
<211> Length : 486
      SequenceName : AW027164
      SequenceDescription :

Sequence
--------
<213> OrganismName : HUMAN
<400> PreSequenceString :
cagagtgactcaatgtcatgttcacatggaagacaacagacaatatcgacatagtctaaaacaatacttccagggcaggtgcag
ctccctcatgggcctgttctaaaccagcctctaggagttgatccaaacaagacgactacagaaacccccaacaaaacgtgccac
gaccacatagcatctgaaaggcaaaacaagacgctggatgttgactttaacatataaaaatactggnaccaaaatgaaatacaa
atatatattagaatctgtaagtattctgtataaagagatacttttgcggttaggctaccagcaggcaaagaggagacaaagtcc
agccttttccacttcctgacaccttcaccgggagccccggccctgttgccagtggtgttgcaaggccacagctccctccggacg
caccctgctgtatctcttcatgcctaaaatgcagagcgaactaggggtcttcacaaagggggacaagatgtggagagaggactt
ctcagaggggtgattntgcagtagccaccttc
<212> Type : DNA
<211> Length : 536
      SequenceName : AW027188
      SequenceDescription :

Sequence
--------
<213> OrganismName : HUMAN
<400> PreSequenceString :
atctcctcgtctgccatttgctaagactgtgggaaaagcacagtgtttggcggggtgtactgattttccatgtacagtctgtc
acagcttcccttggctaggaaaggaaaatcccccgacccattgtgcttcctgggtgaggcgacgccctgtcatgcttcaccttg
ccctccgtgggctgcacccactgtccaaccagtcccagtgagatgaatcaggtacctcagttggtgatgcagaaatcacccgtc
ttctgcgtcgatcactctgggagctgcagaccagagctgttcctattcagctatcttggaacggaacccagccatttcttttta
atcatcacatgttaaatttgaaggaactggcttagttttagaagagtgatcggtctcattaaagtggagggaaaaataggacc
cttaaggaaaatcaaaagatagtgacttaatgtagctaga
<212> Type : DNA
<211> Length : 460
      SequenceName : AW630054
      SequenceDescription :

Sequence
--------
<213> OrganismName : HUMAN
<400> PreSequenceString :
gcttaactataagaccccaaattataaagctactgcaggaaaacataggggaaacacttcagaagaataggcaaatatatttta
gataagacctcaaagatattttcaataagggtcaacaacaaaaaaatagacaaattggactatattgaactaaaatgcttctgc

acagcaaaggaaacaatcaggcagagtgaagagacctgtaggaatggggagaaaatatttgcaaactctttcacctgaggaagg
ggactaattntccaggaatttaacaagggaaggcaaaaaaactttaataggcagtgcccttggcgggctccttaactgttattc
cccaggcacntggggagggcccaagttcagggagggttccantttgagggccagggggttttccaagac
<212> Type : DNA
<211> Length : 407
      SequenceName : H00249
      SequenceDescription :

Sequence
--------
<213> OrganismName : HUMAN
<400> PreSequenceString :
ttaatgttcaccttccatgtattaatttatgtcttngnttataattnctatctccctaaaatgtataaaattaaactataactt
gactacctcgggcactttctcaggacctcttgagactgtacctgagccatggttattcatattggctcagaataaacctcttta
aatattttacagagtttggctcctttggtcaatagctgggtggtagctggccaaggtgaatctgggctgggtaatttactgatt
tacaggagtcctgttcgacactagattctcatcttaacagcacagatgcagtgactagttccagattaatacttgcagctaact
tggaggtatggctggccgctagtgcttagctgtgcccttttgcatggcgccatccaggggaagnccaaagccccagctcagtcc
ccttcctgcttccgtaccctgttgtgncctctttgaagggccttttctgccatgcttcctccaaggcttcagcctctctcagcc
tntaaatctcacctcctgtnatatttcctcagaggg
<212> Type : DNA
<211> Length : 540
      SequenceName : H29666
      SequenceDescription :

Sequence
--------
<213> OrganismName : HUMAN
<400> PreSequenceString :
cagggtctctnaacagggcaggtcatcatagtacggggantggtcttncaagagccgaagcattttactgtgagcctgagggac
caggctncccatgctcctgtgacactcagggcctccttcgcagacagaactctggcctggatctcccgctgggggcagaagaaa
ctgatctcagccccccttcctcttttacccncagagattctttgaggtgctgctcctgttccaggagggagggctgaagctggcg
ctcaatgggcaggggctgggggccaccaagcaatgaaccagcaggccctggagcagctgcggngactccggatcagtggaagta
tccagctctactgtgtccactcctnaggatggttccaggggaaataccgcccagaaaancaagaaaggtcagcc
<212> Type : DNA
<211> Length : 411
      SequenceName : H30148
      SequenceDescription :

Sequence
--------
<213> OrganismName : HUMAN
<400> PreSequenceString :
ggagagctgtctattcagatcctttgcccatttaaaataaggatgtctttntattattgaattataagantcctttatatagt
ctaaatacaagtcccttgtcagatatgattggcaaatatgtnctcccattctgtgtgttgcctttccactttcttgatggtgtc
ctttacagtacaaaagtnttaaatttncataaagnctaatttgtntttaattttntaacttgtgcttttggtgtgataatctga
ggaagatgtttttaaccctactattgagcaagaatatgggatatcacagttaattcatagtaattgggtataggctcgtgaaat
gnattcggnccttgtc
<212> Type : DNA
<211> Length : 352
      SequenceName : H51856
      SequenceDescription :

Sequence
--------
<213> OrganismName : HUMAN
<400> PreSequenceString :
agaaacagcttgctaaagccctctccacttgcaagataacaaaactgactgaaattggctggaactaatatgaccaactagagt
ctgtgcagactgagcttgctgacatcacagcccaaatttccacatttcattccagctactccagaatttgcacatgtggcccat
aaagaggcatgaagagacaactgtgcacgtccaaggacttttcagacttcctctttccttccccagaatctacccccctaaacct
ttcctaataaacatactaccttaaggccaatttgcggggagacagatttgagctggactcctgtcttccttgttggttatctaa
gtaaagctttcctttcttcaaaaacctgggtgccccctaagtttgggaggttttgcttgagcccagggagttttgagatttagc
ctgnaggcaacatgggcaaanccccacggtgtta
<212> Type : DNA

<211> Length : 454
      SequenceName : H60498
      SequenceDescription :

Sequence
--------
<213> OrganismName : HUMAN
<400> PreSequenceString :
attgaggctttttgtggcctatggtctggtttatgtttaatgtacacttgagtagaatgtgtattgtgctgttgttgagtggg
gtgttctgtaaatgtctgttaggttttagtgtctgctgatcaagtttttctaactcctcgttgatcttctgcctcgctctcct
gtctgttaggaacctggccctgccaacaccttgattctggacttccagcctcccaaactgagacagttaatttcagtgtgaaac
caccccatttcttctgttacaaccaccctgacacaggctgattgagcttaacctactgtgggaaataagtggcaacctcagna
tagtgctttgaagggagggcggaacagattgggccattttgtgtgagtgcatgtgtgtcgttgtgtgttgcgtgcgtgcgtgt
gtgtgtgtngtg
<212> Type : DNA
<211> Length : 432
      SequenceName : H60969
      SequenceDescription :

Sequence
--------
<213> OrganismName : HUMAN
<400> PreSequenceString :
gaacttccaccttctcacttaaaggaagcacttgacggcttcttttggcacatccgaattgccggcatcactactctcgggct
ttggggtcattaagaagtaaaataagggctccttgaacacaagcactactagactacgacaattattgatacgcagagggctcc
tgagtggctaacaggcagggagtgcagacagtcagcgaatgtagacagacatcgtgaatagctggacaaaggatgcttctactc
tcggatggtatgagacancaggagatttcattgctctcctcagaaaggtntgaaatttcgccgggcccagtggcttatgcctgt
aatctcagcactttggggagaccgaggtngggcagatcacctgaggtca
<212> Type : DNA
<211> Length : 385
      SequenceName : H61066
      SequenceDescription :

Sequence
--------
<213> OrganismName : HUMAN
<400> PreSequenceString :
tctctgaaaggagaggaaatcgcctccaggaaccagttccttaatgacgtagataggccgtctttgtgtgtgaactcctatact
tttgacattactgggatgattatattgcttgaggattttggcttctagtaaaaatttaatttcagttcctggggaagacgttc
ttgacgtgttttaacagcaacagcaattttatcctttaatgtgaccttaaatgtggncancaaaattcccttgcctcttgtaac
gtggcacctttatgattgagaacccatttcttattctcctaatgggccatactgtgataccatggatggctctttaattgggaa
cattggactttttttttttttggcaattttaaacaattgggggtaaantccatataacatcaanttac
<212> Type : DNA
<211> Length : 405
      SequenceName : H63831
      SequenceDescription :

Sequence
--------
<213> OrganismName : HUMAN
<400> PreSequenceString :
actcttgcaaaaaatttagaacaaacctccaaattcatttcagaagtccagcattaccctgataccaaaatcaggcagatactt
taagaaacgaaaactacaaaccaatatccctgatgaatgtcgatgcaaaaatcttcagcaaaacactagcatgcttaattcaac
aacacattaagagttacaccaattactttagaatgcaagaagatggttcaatatatgaaaatcaaattaagacatcacattaa
caaaataatgggacaaaaaccacatagggccagggggcagtggcttcatgcctgttaatttccagtactttggggagcccgtgg
gtggggcagnttc
<212> Type : DNA
<211> Length : 349
      SequenceName : H73476
      SequenceDescription :

Sequence
--------

<213> OrganismName : HUMAN
<400> PreSequenceString :
tcggctcactacaacctccacctcctgggttcaagtgattctcctgcctcagcttcccgagtagctgggattacaggcgtgtgc
caccacgctgcctaatttatatttattagagatgaggtttctccatgttggtcaagctggtctcaaactcctgatctcagct
gatccactctccttggcctcccaatgtgctgggaatacacgcgtgagcaccgtgctcagcctaaaatctttattaccagacta
gccaggacagccaatatttctgggcttttggaattctaccacaggtaaacctcccatgtggaaattaattacatttttaactaa
gggtc
<212> Type : DNA
<211> Length : 341
      SequenceName : H81171
      SequenceDescription :

Sequence
--------
<213> OrganismName : HUMAN
<400> PreSequenceString :
gagactacatagggctcggcgtggctgagagcagctgtgtcggcagaaaggctcgagggcttgggcatcactggggtggccgct
ggtggggggtcaggctccccggatgtcagcactcagccggcgncatagtccacttcgctgcgctcccacccctcctcccggtct
tcactgaagctcctttcagcgaaggtaggagtgggctggacggccagggaactcagagctgctgtagaccttgctgaaccggtg
ggagcaggaggagaactggggggatctctgtggacgattcttcatcattggtctcgtcgtcctcggagcccagatggccggtaac
gttccgaacgtgtttcaaagtagctggtatc
<212> Type : DNA
<211> Length : 367
      SequenceName : R00285
      SequenceDescription :

Sequence
--------
<213> OrganismName : HUMAN
<400> PreSequenceString :
ggactttgccttttttcttcctataaaagggacaacagtgctgtccctgactactgcaaaaccagggagaccaggaggttccttc
ccctgcccgcttcctggattcagagttaatttccctgaagtctcctggtggtagaggggaaggggctcatttgatgtcagcagg
tacgaagggaagggttggagtgctgagaactgccccccacttgctgttctttttattgcagtggagttcacagggcccccacag
aagcccccgaggctngngcgcacagtccatccagcccacagctaacctgggaccggattgatgacctgggtgttaccttcaatg
ttcatgggagctggtt
<212> Type : DNA
<211> Length : 352
      SequenceName : R09288
      SequenceDescription :

Sequence
--------
<213> OrganismName : HUMAN
<400> PreSequenceString :
ccaaatgacttaatctagtctcaaaaaaaaaaaanaaaaaaaaaaaaaagaaatgatcattgtcagaaaagacgcttcattaag
tagtgccagtgtttttgtttgcggtctgcaattgtcagatggtcggtccctatnttcacagtctcgggaggcttgggagatata
ggtcancaccctttgtgctggctgctctctctgtagtcatgcttagctcagttgttttgtggggaagttacttaaacagggaa
attaaagtatttctgtgactacttctgctctnactactgcaaaacctttctcnttatagctctggggcttatttggccaaaat
tttgac
<212> Type : DNA
<211> Length : 342
      SequenceName : R10535
      SequenceDescription :

Sequence
--------
<213> OrganismName : HUMAN
<400> PreSequenceString :
tttttttttttgggactgttgaccttgctgggagaaaagagacaacgactgagcaagcactaccaccagcactgttactggga
attagaagacctgagtttctgtccagaccctcagtgcaaactgaggatgctccatccaaagtgaattatgtcctgtgcctcctg
attgctgagtgttcacctggaccttctgactaccttccctgtgctattccatcagcctacagacctggtacctggattttgcc
cgagatgattcctaccaccttactactgacgaagacacccattccagtggaccactgtgacccaggaggcattcagccatcatg
atgtggcctttacctccactcctgtcttgttctacccagattcagcacagccctttatagtgaagtcagagtcctcaagccaaa

tagctaaagctgtttatcacaacaaaggcctagtttgttccatgagtgtgcatttcatttcttc
<212> Type : DNA
<211> Length : 485
      SequenceName : AW170305
      SequenceDescription :

Sequence
--------
<213> OrganismName : HUMAN
<400> PreSequenceString :
tttccagagtgccttgattcaactgtttactaatcagaaaggtcggtatagtcggtcctttttctactgctggcaagtgatttt
tgtatatttattacaaataaaacaatattgcttgacagcatcatctgtcatcttacaaatatacaactacacagtcctaaagt
aatagtatgtctctagaactgagcttttcttaaaaacaaacaataactatactattattaagtcctgcaccctaaatctataca
gcagctttaatcaatgtggaaatgaaattatgatttattaatttacataaaattactttatacattaaagaataatttttcaaa
ttgttaaaaaaacaaagtctgaagctgtcattattgggaatcgtgggacgtggtttgctcttctatcacctgcttcacaatttc
tgccagttttagtcgatctactttatctgtgaatccacgaccattccagctgaagctctgcagagtatctcttaggagtttgca
ttcccggttatacttccaagcctcctgcattacttcattcagtttctcatcttcattctctcctttaccagcctggggggagaat
acactgagcaatcacatctcggagttntttccaaggcaacatttggaatcctcggctccattctcatggtcgtggatatagacac
tgtcctttggcttggtgcccagtaacttccttctccgcagaaatggggtgtcacatgatgccag
<212> Type : DNA
<211> Length : 736
      SequenceName : AW194791
      SequenceDescription :

Sequence
--------
<213> OrganismName : HUMAN
<400> PreSequenceString :
tataacattttaaactacttattttatttatatcagagtaacaaatacttatgaagtcctttgaagttcagcgcacaaatttc
tcgtgtggggcggtgggtgtggccatgttcttgctcttccttctttacacatttgagttgtgccttctgttcttaaagagattt
tcctttgttcaaaggatttattcctaccatttcacaaatccgaaaataattgaggaaacaggttacatcattccaattttgcct
tgggtttgaagagtctctcatggtggcacagtcctccagggtagctatgttgttgggctcccctacatcccagaagctcagaga
ctttgtcaaaggtgtgccgtccacccattgccactgaccctcgacaacctggtctgacagtccaataaaaaactctctcatttt
aggtttcttgtaggaaaggaattcctgctcctcctgtga
<212> Type : DNA
<211> Length : 459
      SequenceName : AW511388
      SequenceDescription :

Sequence
--------
<213> OrganismName : HUMAN
<400> PreSequenceString :
gcggccgctgttcctgggacgtccggttgaccgctcgtctgctgcagagaccatgtctgccgacggggcagaggctgatggcag
cacccaggtgacagtggaagaaccggtacagcagcccagtgtggtggaccgtgtggccagcatgcctctgatcagctccacctg
cgacatggtgtccgcagcctatgcctccaccaaggagagctacccgcacgtcaagactgtctgcgacgcagcagagaagggagt
gaggaccctcacggcggctgctgtcagcggggctcagccgatcctctccaagctggagccccagattgcatcagccagcgaata
cgcccacagggggctggacaagttggaggagaacctccccatcctgcagcagcccacggagaaggtcctggcggacaccaagga
gcttgtgtcgtctaatgtgtcgggggcccaagagatggtgtctagcgccaaggacacggtggccacccaattgtcggaggcggt
ggacgcgacccgcggtgctgtgcagagcggcgtggacaagacatagtccgtagtgaccggcggcgtncaatcagtcatgggctc
ccgcttgggccagatggtgctgagtngggtcgacacggtgctggggaagtctgaggagtgggcggacaaccacctgccccttac
ggatgccggactggcctcgcatcgcacatccctggatggcctcgacctcgcgtccgtgcagcagcagcagcaggaacagagcct
catcgtn
<212> Type : DNA
<211> Length : 763
      SequenceName : AW629084
      SequenceDescription :

Sequence
--------
<213> OrganismName : HUMAN
<400> PreSequenceString :
gctctcacccactttctcctgttacggcaggggaccgacctgtttgccgtggctgccatgagtttggccacgccctgggcctgg

gccactcctcagcccccaactccattatgaggcccttctaccagggtccggtgggcgaccctgacaagtaccgcctgtctcagg
atgaccgcgatggcctgcagcaactctatggtaggggagagggacctgccgcgaaaccatctttgccccatccagtgtcctct
gcagcagggccaggggacgcacacctgcctgactctttcctcacagggaaggcgccccaaaccccatatgacaagcccacaagg
aaacccctggctcctccgccccagcccccggcctcgcccacacacaggtgagtcccccaccaactcggagaccttgggtgacca
gctgcccagcctcagtgtcctctgagatggggatggtgggggtccctgccttggag
<212> Type : DNA
<211> Length : 476
      SequenceName : AW665782
      SequenceDescription :

Sequence
--------
<213> OrganismName : HUMAN
<400> PreSequenceString :
tttttgtgttaaaatatttattcttaaagttcttaaaggtggacatgttcaaggcttaaaaattaacttcaaaaagtcaagcc
aattcacaaacacctaaattaggtgaaggagaatctctcccaactttaaaatacacattaaataccttcccctcatgctgcttc
attatttttataattatctccagttgggtgtaggctatgtcttcttagaccgacagaaaatttggcttttctcagaatttatg
cagaatagcagaggttaagtgttagagtttggaagaggtcagttgttgtataagcagcttctggatcatcttccagcactctgg
agagaccaaaatcagaaactttgcatactaagttgctattgaccagtatattccgagccgctaggtctcgatgaacataaccca
tatcagaaagatacttcatgcctgatgcaatgcctcggagcattccgaccaactggatgactgtgaagtggccatc
<212> Type : DNA
<211> Length : 496
      SequenceName : AW665954
      SequenceDescription :

Sequence
--------
<213> OrganismName : HUMAN
<400> PreSequenceString :
acatacgtactaataaacatggaattctagggtcttaagagtgttcaggggaacaggcttcgctangctaagtgctcatgggcc
tccttttcacttgcggttctgcatttccttttacttatttttttcccaactctatcgaggtgtgattgacaaatgaaattgtaa
caaaggtgtacaaagtgctgaattgatacatgtatgtattgtgacatgattaccacacaagttagtgaacacatctgtcacctc
accttgttgcttgtgtgtgtagtgagagcagttgagacctactgttagcacatttcaggtgtacagtattgttgactctagtca
ccttgccgtccattaggagccccagaacttacctagttcaagtagagaggttgggctctgccttttaacgagtcttaaccacta
ggaggggcatttctggcattttcaggaacagaattggcatattaggccganggcttttcgtgttggcatcntgggggcagcact
nctcacccgnactcctgggaacccncaggggccggggtttgcnttcagaggggcaggtgagacccaaaag
<212> Type : DNA
<211> Length : 574
      SequenceName : R20939
      SequenceDescription :

Sequence
--------
<213> OrganismName : HUMAN
<400> PreSequenceString :
aacagtgtgcggtttcctcatgggcagatttgtgtaatctcaaccatagtcaacaaacagcccatctgcacaggatccttccag
ctgctcttccataacttcatctctcccctcaacacctgcaaccactaagccatttccccctacccgtctgtaattttgttatt
ttgagaatgccaggtaaatggaatcagagcggataatctttggagcatgattcccttgcattcacctgtgttgtttcgtgtatc
catagtttattctttttttaatctggggctcactggcaatctccgcctcctgggnttcaaggcggattctcctgtctcaggcct
ccccagaagctggggattacaggggggncccgncaccatggcccggctaaattttttttgtatttttagtagaggttgggggttt
caccngttgggccagggtggggtcttggaactcttggacctcaggtggatcccacccgggtggggcttccc
<212> Type : DNA
<211> Length : 491
      SequenceName : R53190
      SequenceDescription :

Sequence
--------
<213> OrganismName : HUMAN
<400> PreSequenceString :
gcaaatgagacacatatagatactgagacctgtgtgtacagtaggaccatgcactcacacccatctggagagggagcccccggt
ataccaagggagccagttgtgttcagacacacacatcacagcttgactcactaactgaggcctttccatagctccacagcttcc
ccttctcccctgtcctcaccatatgccttatccccattctactcccctgctatgcaagtgcccctgtggcttgtccccaacccc

ctcagcaacaaagctcagctggggaacgagagtaatttgaagaatgcttgaagtcagcgtcttccattccagaaagacccccat
tcttcctttggggtatgatgtgggaagctggtttcagcccagggacccaccactgagggagagggttttaggacaggtggggc
ttatttccaaggggncctttctggggcttggagaagggctttttacacacacacaacacatacacacacaca
<212> Type : DNA
<211> Length : 492
      SequenceName : R80718
      SequenceDescription :

Sequence
--------
<213> OrganismName : HUMAN
<400> PreSequenceString :
ttttnaaaacggagctcgctctgtcacccaggctggagtgcagtggcgggatctctagccttcatctttctcccatgctggatg
cttcctgccctcgaacatcaagaccccagtgtctttgggttttggaccgttggacctacaccagtgatttgccagggntcctgg
gaccttcgaccacagactgaaggccgcactctcagcttgcctacttttgaggttttgggccttggactggcttcctgggctcct
cagnttgcagacggcctattgtggggantttcaccttgtgatcgtgtgagtcaattatcctagtaaactccccttcatatacac
atcagtcctattcgttcttgtccctgtaggatcaccctgactttgaacaggcattgaaggacccaacggttg
<212> Type : DNA
<211> Length : 408
      SequenceName : T56441
      SequenceDescription :

Sequence
--------
<213> OrganismName : HUMAN
<400> PreSequenceString :
tgacttgtaggctataggggctataatatatcaagacaatctttatcctcatgaagcttacagttaagtaagagatagagatta
aataattataacaacagagtgaagaacagtgaagaaaaagtacagagttataatatatataatagggccaggactgcatgagga
aggtaggaaagacatttcggcaagaggttgtcagggaaaagacttgcttgagaaagagccaagttgtgggggtctggctgctta
gcaattgacccttaataccttaactttttgcttatttttacatggaagtaactaattttaaccctattgagggaaagtactac
tacccttcttaggttttacaggttaagtaaggccggagttaccggngaaggtttaaaactctttcacacgg
<212> Type : DNA
<211> Length : 408
      SequenceName : T56477
      SequenceDescription :

Sequence
--------
<213> OrganismName : HUMAN
<400> PreSequenceString :
gaataatgtcagagaaaataaccagaacattacaaataatactggaagttgtaccagaagaagataagttatgaactaatggnc
ttgagatcttggcaatctgcccaaggggagacacaaaatagggattttnacttcatttctgaaagtctagagaattacaactt
tggtgataaacaaaaggagtcagttattttattcatatatttagcatattcgaactaatttctaagaaatttagttatanct
ctatgtagttatagaaagtgaatatgcagttattctatgagtcgcacaattcttgagtctctgatacctaccnattggggttag
gagaaaagactagacanttactatgtggtcatntc
<212> Type : DNA
<211> Length : 371
      SequenceName : W00842
      SequenceDescription :

Sequence
--------
<213> OrganismName : HUMAN
<400> PreSequenceString :
aaaagaccaagaaacctacccaagattcttgtgagaatactgaagctcaccaaaagaaagccagtaagaagaaggcccctcctg
tgactaaagatccctcatcattaaaggcaaccccagggattaaggattcatcagcagcacttgccacttctacaagtctttctg
caaaaaatgttattaaaaagaagggagaaattatcattttatggacaagaaatgatgaccgggaaattttattggagtgtcaga
aaagagggccatcatttaaaacatttgcatatttagccgccaagttggataaaaatccaaatcaggtctcagaaagattccagc
agctaatgaagctctttgaaaagtcaaaatgcagataagttgctgaattcctgggactcttaactgatattgcatactacacag
gaagccttcaaatttcctcatgttttaaatgagcactagaggggagggtttatcaccctttttgggttaccagatggataatc
ccatanttggttaat
<212> Type : DNA
<211> Length : 519

SequenceName : W23427
SequenceDescription :

Sequence
--------
<213> OrganismName : HUMAN
<400> PreSequenceString :
aaccatggcagcatagaagtctctgactggaccaaatggactggtgataaagcctaaggtccagctctgtgatcttggataaat
ggttcaacccctcatcacctccgtcccttatctaaaatgcaggttagactcagtgattggtaaaggctctcatagttccttttt
ctctgactctgtacccagactcagggagcaaaactgtcatttgccttggtaggctttttgatatctcctgaaaaagcagcttcg
ggaggggatttagcttctgctaattcttcttcacaaagacacgtgaccatttctgaatgtctggctttaaaaagtgtaacaggt
ggttggactctgcagagacctcgggttagtctggcactgccccttaccacctatatgaccctgggggaattattcacctctctg
ctcccaagttttgtatattaagggtaa
<212> Type : DNA
<211> Length : 447
SequenceName : W90438
SequenceDescription :

Sequence
--------
<213> OrganismName : HUMAN
<400> PreSequenceString :
ttttttttttttttttttaaatagaaaacagctgggcctggggacactaccaccaaaacacgaagaccggtagtggccccg
aatggctgggcgcgctgatatttattgtatacaagacaaggggacaggcagggtaaggaaggtgggtcgtccaagtcgattga
taaggtcaagcaagtcacgtgatcataggacaggggtccctttccttttaggtagctgaagcagagatgggaaggcaagcatac
gtcagcgttttcttctgtgcatttatcagaaattcgatcaaagactttaagattttcactatttcttctaccgctatcttctaa
gaacttcaaagaggaaccaggagtacgggaagagcatgaaagtggacaaggagcgtgaccatggaagcacagcaccacagggag
gggtttaggcctccggatgactgcgggctt
<212> Type : DNA
<211> Length : 450
SequenceName : W96144
SequenceDescription :

Sequence
--------
<213> OrganismName : HUMAN
<400> PreSequenceString :
ggccacatggtggtctggccggtggcaacaaggctataaatcagttggtcagcattccttcctgaggtgagacactctgcaacc
ttggttccatattttggatctcctatggccaattcctg
<212> Type : DNA
<211> Length : 122
SequenceName : 1362587H1
SequenceDescription :

Sequence
--------
<213> OrganismName : HUMAN
<400> PreSequenceString :
tctcacctcggctgccagtccacagtgaacaccaccacagacacagtgaaaggctacttcctggctggaggggacatggtgtgg
tggccagtgggtgcatccttgtttgccaccaatgttggaagtggacatttcattggcctggcagggtcaggtgctgctacgggc
atttctgcaccagctcatgaacttaatggcttgttttctgtgctgatgttggc
<212> Type : DNA
<211> Length : 221
SequenceName : 1483347H1
SequenceDescription :

Sequence
--------
<213> OrganismName : HUMAN
<400> PreSequenceString :
agagaatgtatcttgagccaatgtattaatagtagattgctttgcttatgtagtaggcaatatatgggtatacattttcctgac
tattatcttaatgactattccaaattatgactaccttaaaaactaccaaaatcccctgctccagaattttcagtttcagatgag
gttcataattcatggcctcacttttcccacattctcctacaggttggacgtgataagtatgagcctgcagctgtttcagaacaa

ggtgataaaaagggcaaaaagggcaaaaagacagggacatggatgaactgaagaaagaagtttctatggtaagtactaggagg
aatattgtattccatccttattaaaatccatgattttaatcccccaggcctcactgtatcttcaaagaactgctatatatta
aaagagatcatagcagctgtacagatctcatctagtcgtagaggttaatgttgaa
<212> Type : DNA
<211> Length : 475
      SequenceName : 1512407F6
      SequenceDescription :

Sequence
--------
<213> OrganismName : HUMAN
<400> PreSequenceString :
ttttttcaggttcccacacagtttattgagggagccaaagccagtgagaggacgaaggagtcctacttcaacctacttcctta
aagctgtgtgcctccagcatgaagggaccttcccagagggcagggcctgccctataaggggccacatgaagccttgtggagact
cctgcctctcataccacagcctgtcctaccctaaagacccagacatgacagcagacataatccccaccccaggagggtgctgag
gaaacacccttacccatgcccctcccctagagacctcaggaagaaaggccaggacacctgtcctgtgattggccagttgaatcc
tcaacgtgaagacctggcaggcctggctgacaggtctgttcagatgtggcctcaggcatctactctaggatccaacccctaaca
ttcaaacatgattaaaaacacccaggacacttctcttgctggcagaggccacaccccagataatggcagtcctgccaaactctg
gccagagatagggacccaagctgactgggtgtgagcacgtaaggagaccactctgccccatggggagaatcttccagggctcag
ctga
<212> Type : DNA
<211> Length : 592
      SequenceName : BE218593
      SequenceDescription :

Sequence
--------
<213> OrganismName : HUMAN
<400> PreSequenceString :
tgatacttctgaactggtgaggtgtgctcacaatgaggtttcctcacaatgaggtttcctctaaaagttattttttactttct
tctgttagcaaagcagttgccgctacagattgaatgcatctgggccatctgcgggttactgggttaaggattttgataggaag
gctacgggttatcagtggcctcagtgctttcagaatacgcccttgtttacactgacaacaaagtggtattggagtcttataggg
ttatggagaataccttcaattatcaattataggttttaaatttaccctggcttttaaaggaatag
<212> Type : DNA
<211> Length : 317
      SequenceName : BE326380
      SequenceDescription :

Sequence
--------
<213> OrganismName : HUMAN
<400> PreSequenceString :
gagactggtttttccagccttggtttggaagcttgtctcaaaaaccattacaaaatcttaaccttcaaatacattctaaatca
ctttaatttagaagataggtgagcaacttgagtgatcccaaaccaaactaacagtgctggtttgtccgacacccaaagcccact
tcacacatttataaggacagagatttggtaactcatttgggtattttgtcagttttaacccttaatatacaggtatcccagctt
tgggcacatgtatgtccattcactttcaggaaggggtgtcagtactttctttctgtgtggtgaaaacataagccatggtgaact
gataatttttgtttttaaattatttatattactgaattgggcaaaattacagttcacactggcagagctagggttgtccnattt
ggccttaaaacaaaccnggcaacttt
<212> Type : DNA
<211> Length : 447
      SequenceName : H19371
      SequenceDescription :

Sequence
--------
<213> OrganismName : HUMAN
<400> PreSequenceString :
aacacacacacacacacacacacacacacacgtgcacacacacatccccccacaactacagccgccaaaataaccagcatggngtt
gtaacattatttctccatcttgcngttttctagccttgagttggggacatgagtgagaactgggggttttgaggagtaacccttt
gngaacacangtactccaggcagccgggnatcccagatctcct
<212> Type : DNA
<211> Length : 211
      SequenceName : H24679

SequenceDescription :

Sequence
--------
<213> OrganismName : HUMAN
<400> PreSequenceString :
cctaggttggccttgaacctcctggcctcaaatcattcctcctcagcatcttgagtaggtgggattgcagttgtgagccactgt
gcctggtcaattttagttaaacttttagataaggatatacagggtttgcatacacaaaaaatgcaaataattcttttgtttta
aatagcttccttttccctaaagagattgctgcaagtaactatgtactgtcttatatcttaagtgagctctacctacttctattc
atgactgttagttgatacagagtatgggntttcctaataagccattatatatttaactttataggaaggggaataacttgaagg
atccctgaggatacttggggtccnaatttgtaggaaggtgctgttaaaggg
<212> Type : DNA
<211> Length : 388
      SequenceName : H62827
      SequenceDescription :

Sequence
--------
<213> OrganismName : HUMAN
<400> PreSequenceString :
ngacaactgactttctgctttcacctttgcttgtcaactctcccttctcctcattctcttttaatgttaggccccttctccagt
cttttccttttactctggtcttttactcgttttgtatccattttatttagccccatcacatcattgcttaaccgctgctctc
ctcccattttcgcttcaccctctttacagcagcctgtccctctcccgatctcagtgatgatgctctaagtggttaagagttgat
tccggagccaggctgcctgggtttgaacccagatctatttattagcttggtgacccagagcaagttattctgcctgtgactcaa
ttttcctcacctttaaactggggatcatggttagtttagcattttcatagggattgttgtgaaatttngggtgagtgaatatt
tggaaacatttcatccagtggnttaggcatatgtagggagagttggctgtttcacatgattattncagtcccttagttttggt
ccaganctcatttttg
<212> Type : DNA
<211> Length : 521
      SequenceName : H63640
      SequenceDescription :

Sequence
--------
<213> OrganismName : HUMAN
<400> PreSequenceString :
tcataatagccattctaacaggtgtgagttaaagggtctgtagtactcttgcagtgtctttgctgatattctgtgtttgttttc
ctgttctttatttcatttatttccactctagtctttattattncttcattatttaggagtgttatttaatttccatatatttgt
gatttttccnttctttttttggtctgtattgagttctactttattccattgtggttggaaaacacactttgacntaatcattt
taaatttattgaggttcatttatagcctaacatatttgcctgctggagggatgttccattanaaccntaagtatgttacattc
tgggccagggcacggtggccgcacgtctntaaatcccagtcagtttgggg
<212> Type : DNA
<211> Length : 386
      SequenceName : H66015
      SequenceDescription :

Sequence
--------
<213> OrganismName : HUMAN
<400> PreSequenceString :
ttggcacagttgctggtggaggtccnggctctggtnggagtgcctccaattcccttgctttacgcagcttctcacgctttcgct
ggatggcagcaacccgttcacgtactgcacgggccactggctggttaatcggcttcacagaccaagcccagagccacctggaat
tggggtnggggatgaggaacacagcttgggaccccctatcaaagatccatcaggacgccttcttgggaactccagtctcgcctc
gggttgcaatngaccccaactgctcctgtgccnctgcaggtntctcctgcacctgcgtccanttc
<212> Type : DNA
<211> Length : 317
      SequenceName : H91370
      SequenceDescription :

Sequence
--------
<213> OrganismName : HUMAN
<400> PreSequenceString :

ggggagaaggtctcactatgttgcccaggcttgtctcaaactcccgggctcatagcaatcctcccactgtggcgtcccaaagtg
ctggggttacaggtgtgagccaccacacactgggctctgctctgcctttctgagtttggtttctgcttatggtggggagcttgt
tcccgttcttcccacaagaacccaggatgtggcacagcttccctgccgtcttccttcactcagttggctacgctncccatccta
gctccacttccagaactgccttcaccctaggctgggtccttgttttgtttttagagacagggtctcactgtgttctcccaggct
gggagtacagtgggcgtgatttcgggttcantgccaccttccacctnccgagtttcaaggcgatttttcctggccttcaggcc
tncccgagtaggttagggntttacagggc
<212> Type : DNA
<211> Length : 449
      SequenceName : R48756
      SequenceDescription :

Sequence
--------
<213> OrganismName : HUMAN
<400> PreSequenceString :
aaatcgtttctgttcttttaaaattaaattgtaagacagttgtttgtcagattttggattccatggaaaaagttttgtttagag
gtaggtaacacatgaacttttaaaatcaccttaaataagtagcttcttgcgtaagacaccaaatagtgttccctcctcatttg
ggaacatttcttctgggtacttgactttctttcatatgtttttcataataatagttccagtttataaaattacgttctctttt
ctagtcgccgagatgacatgggaatcattaggatatgtttgatgtattttaataggaaccagcctgccatggcaagggctaaa
gggtaaaccaggacttcacatttattcaggtttttctctctctttttaggaataaaaatatatattgggggggaggntagggtt
ttaagggtccccttctaggnctgggggttttnccggggtttaagggntttaacccctatttacntttttaccccttttncg
gg
<212> Type : DNA
<211> Length : 506
      SequenceName : R52476
      SequenceDescription :

Sequence
--------
<213> OrganismName : HUMAN
<400> PreSequenceString :
tttttttcagctttatctttttttattttcaaaaatatttgtttatttttaaaattgacatttaaaatggtacatatttatca
tgtacagtaccatactgtcagaaaaacagacacacagaacaatggaacagaatagagagcccagaaataaatccacatatttac
agccaacttattttttgacaaggtgccaagaacacacaaaaggcaaaggacagtctctgtaataaatattgttgggtacaactgg
gatatctacatgcaggaggaatgatatcagtctcttacctttcancatatacaaaantcaactcaaactgggntttaaaggncc
tggaaactagggnactactaggagggnaacatag
<212> Type : DNA
<211> Length : 370
      SequenceName : R59209
      SequenceDescription :

Sequence
--------
<213> OrganismName : HUMAN
<400> PreSequenceString :
agcctctgaaccctaggtagagctctttaaatgcttagtctagctacagaatttgacagtgaatcttacagtcccacgtgttac
tttctagttattttgttattcttgtcttcccaactaaattataagttccttaagggcaaggatcacatattatatttattta
<212> Type : DNA
<211> Length : 168
      SequenceName : 1594701F6
      SequenceDescription :

Sequence
--------
<213> OrganismName : HUMAN
<400> PreSequenceString :
acagcatggaaatatttggggtagggtggtcattcactctgcttaccacttttggtactatgtatgtgtacagtgtgtgaagtc
aagcacagtcacttgcatacgtagagctactcaacagtcagtggtaaatcatgtggtct
<212> Type : DNA
<211> Length : 143
      SequenceName : 1747028H1
      SequenceDescription :

Sequence
--------
<213> OrganismName : HUMAN
<400> PreSequenceString :
gccttcctgagaagatgcaaccaatcctgcttctgctggccttcctcctgctgcccagggcagatgcaggggagatcatcgggg
gacatgaggccaagccccactcccgcccctacatggcttatcttatgatctgggatcagaagtctctgaagaggtgcggtggct
tcctgatacaagacgacttcgtgctgacagctgctcactgttggggaagctccataaatgtcaccttgggggcccacaatatca
aagaacaggagccgacccagcagtttatccctg
<212> Type : DNA
<211> Length : 285
      SequenceName : 1876511H1
      SequenceDescription :

Sequence
--------
<213> OrganismName : HUMAN
<400> PreSequenceString :
gttcttgaactcctggcctcaagtgatccgcctacctctggccttccagattgctggaattacaggtgtgagctaccgcctctg
gccagtaagtggattctaaattgcttattgaaatgcaaaggacctagt
<212> Type : DNA
<211> Length : 132
      SequenceName : 1902928H1
      SequenceDescription :

Sequence
--------
<213> OrganismName : HUMAN
<400> PreSequenceString :
ccccacccggccagaacctttaaatttaaaattgtacagtgtacttgcctggccaagaagcgtaggacggangaaggaagtaat
gtttttacccgttttacggttaaagagcttggcttt
<212> Type : DNA
<211> Length : 120
      SequenceName : 1956982H1
      SequenceDescription :

Sequence
--------
<213> OrganismName : HUMAN
<400> PreSequenceString :
aaagtaaactatgctgtattaactccatattaggcagccagtaaaaataaatacaaccaccaaaagtaactcagaaatatgttt
aggaaacaatactaattgaaaaatatagaacttcaggttgtatatctaccatatttccaatctgtaaaatataagtacatttgg
atcaagattggaaaggaagacaaattagaataactcatcaataacatattt
<212> Type : DNA
<211> Length : 219
      SequenceName : 2291436H1
      SequenceDescription :

Sequence
--------
<213> OrganismName : HUMAN
<400> PreSequenceString :
agctgcactg cctacctgca agaacttcaa ggcatctaaa cgcaacatcc aacag          55

<212> Type : DNA
<211> Length : 55
      SequenceName : 2398102H1
      SequenceDescription :

Sequence
--------
<213> OrganismName : HUMAN
<400> PreSequenceString :
tttagagtacagattttataaacagcttggcagtgcaggtgaaggtctcccacaggtgtattactttggaccatgtgggaaatc

taatgccatggcgctggagctccttggccctagcttggaggacttgtttgacctctgtgaccgaacatttactttgaagacggt
gttaatgatagccatccagctgctttctcgaatggaatacgtgcactcaaagaacctcatttaccgagatgtcaagccagagaa
cttcctgattg
<212> Type : DNA
<211> Length : 263
      SequenceName : 2435705R6
      SequenceDescription :

Sequence
--------
<213> OrganismName : HUMAN
<400> PreSequenceString :
gcggcggcgttggcggcttgtgcagcaatggccaagatcaaggctcgagatcttcgcgggaagaagaaggaggagctgctgaaa
cagctggacgacctgaaggtggagctgtcccagctgcgcgtcgccaaagtgacaggcggtgcggcctccaagctctctaagatc
cgagtcgtccggaaatccattgcccgtgttctcacagttattaaccagactcagaaagaaaacctcagga
<212> Type : DNA
<211> Length : 238
      SequenceName : 2457652H1
      SequenceDescription :

Sequence
--------
<213> OrganismName : HUMAN
<400> PreSequenceString :
cggccctaggtcccggcagcggtggtgacggcggtgccggaggttgtccttggcaggttttcctcggcgcttctccatggagga
ggcggtgcgaacggcttcagccccgaatgctcgcatctcccactggacggcgacgaaggcggtggccgtgcgagcgcaggactg
ggcggcctgtgtgggggtgtgagccgcggtgcccaaggctgcaccggcgaggggaagccgcgc
<212> Type : DNA
<211> Length : 231
      SequenceName : 2526581H1
      SequenceDescription :

Sequence
--------
<213> OrganismName : HUMAN
<400> PreSequenceString :
cagaactttctgcctgggctgccagcccccacttgcctgatacaagatgtacttgatcatgctcaggtggtcccatggagttaa
ttgcagcattcttgctcctggaagtggggttgagggcccgaggagaagcagaggctgctcggcagagatcccggaacactttat
ccctctctcttgcccaggttttatgccgctgagataatgtgtggactgcagtttctacacagcaagggcatcatttacaggtgc
nggggtgagggcagcgggggctcttnggaggggaggctccagccccatcatatcttctgaaatgctccaagcaggatcccccca
actccagttccttctctgctggaaatccaatctttagctg
<212> Type : DNA
<211> Length : 376
      SequenceName : 2630745F6
      SequenceDescription :

Sequence
--------
<213> OrganismName : HUMAN
<400> PreSequenceString :
taaagtatattgatgtttttttattacagctagtatatatttaagaatgttagtataggattttgaataaattttgtttttaa
gagaagttataatttaattagatggggaggaaagtctttgaagaggaacctctactttacttttataaagtgattctcctgct
cagtctccccag
<212> Type : DNA
<211> Length : 180
      SequenceName : 2676312H1
      SequenceDescription :

Sequence
--------
<213> OrganismName : HUMAN
<400> PreSequenceString :
agccctttggaatagcagtgttccagtgtgtgaacgtgagttgaaagaactatgtagtttggatagctctccttattttcgttt

tccagcatgtttttccgcatggcatcacctgttgtctagatctttacttaactaaattatggataaaaatacttctttgttgga
agaatttcaagaagggtcttgtaggtcttctctgacatgtgttttgttactaatctgcaaatcacttagt
<212> Type : DNA
<211> Length : 239
      SequenceName : 2745317H1
      SequenceDescription :

Sequence
--------
<213> OrganismName : HUMAN
<400> PreSequenceString :
cgggggtggacctggtttgtataagtaccatgggtgccactctagtctccaggactttctctagcctagacctagtaaccacta
ccaggaactgctggggcncagctctggttctgtggcatagtgataacaaggactaactcgggacttccaaggaaagggcgctgt
ggtcactgggtggcagcctcaaatctctgagtcccttccccttgaccccttgacagtctgtggtatagctggccccagggccta
cttcacagntgtgtgnaaagagcgtcgaanggctgggaatgttgnctgactacactgtctggtccctccctgccctcagtg
<212> Type : DNA
<211> Length : 334
      SequenceName : 2794246F6
      SequenceDescription :

Sequence
--------
<213> OrganismName : HUMAN
<400> PreSequenceString :
gaaggcttctcaatggttaaaacagctgagctggaccttcaaagagaagtagcttaccagcagatatagagcattctaagcaga
aagaatagcaaggagggaggcatgcaagtgtttggcagggattcaggaaccttgagtcatctctaaggggggctagagtatagag
cgggggtgtccaagggtcagaatacagtcgtgggtgcttagtttctgtttctggttgggccagtaaagccccttcctcatcccg
gttttccact
<212> Type : DNA
<211> Length : 262
      SequenceName : 2832401H1
      SequenceDescription :

Sequence
--------
<213> OrganismName : HUMAN
<400> PreSequenceString :
ggagccacctttcaatgacctcccgcacataaggtctcagtctacctgtaggaggtcccctgttcctaattagctccangaggc
caagggaggagagggcactggccagggagtctggaagccagataccccttccactgtgtttccatagactgggaaggaaggagg
atggaggggtcccacccagactgtcccctgagaccctggcatgctctgtaccttgntagcctggccgaagcagcccttgcccag
cacctccccgtggatgangtcgacg
<212> Type : DNA
<211> Length : 277
      SequenceName : 2836805T6
      SequenceDescription :

Sequence
--------
<213> OrganismName : HUMAN
<400> PreSequenceString :
acgtatcagtatttagtcaaataattgatgagacctcattacagatctttggngctttgtgtgcagttcactctcctctgatat
tcagtcctgcgtattttaacattcttcttccagcaaactctcacctctgtctcctcaactcagttagatctgtggatgctgttt
gggttctctcttttgttctgtaacctggagactacctccagacagtaatctggagcaatcagagggtttgtctccattgttccc
cttctctcagggatcacagttttgtgctgcctgttgtcccagtgtctgaaagcttttgttttacatatatttttccagtattgt
agttggttatggcagaaggacactt
<212> Type : DNA
<211> Length : 361
      SequenceName : 2918417F6
      SequenceDescription :

Sequence
--------
<213> OrganismName : HUMAN

<400> PreSequenceString :
atccaggtgaacactcattctggaaactgatcgctttacccaaagttggccttatagccttcgtcatcaactcactcagctcgt
gttttggcttcctcgatcctactctgtctctctttgttttggagaagttcaatttaccagctggatatgtgggactagtattcc
tgggtatggcactgtcctatgccatctcttcaccactatttggtctcctaagtgataaaaggccacctctaaggaaatggcttc
tggtgtttggcaacttaatcacagccgggtgctacatgctcttaggggcctgtcccaatcttggcatattaaaagtcagctctg
ggctgctgggtgctgatattagttgtaagtgggc
<212> Type : DNA
<211> Length : 370
      SequenceName : R61661
      SequenceDescription :

Sequence
--------
<213> OrganismName : HUMAN
<400> PreSequenceString :
ctgtagaaaaanaggtctatgttgcccaggngagatcccgtctcaacaacaacaacaacagcaacaaaccaacaaaaaacgaaa
caaaagantccagataaaacttctagggacacaaattatacttgagataaaaatatgctaggtaggtttattagcagattagac
attac
<212> Type : DNA
<211> Length : 173
      SequenceName : R63067
      SequenceDescription :

Sequence
--------
<213> OrganismName : HUMAN
<400> PreSequenceString :
agagagaacccaccatggtgctgtctcctgccgacaagaccaacgtcaaggccgcctggggtaaggtcggcgcgcacgtggcga
gtatggtgcggaggccctggagaggatgttcctgtccttccccaccaccaagacctacttcccggtcaacttcaagctcctaag
ccactgcctgctggtgaccctggccgcccacctccccgccgagttcacccctgcggtgcacgcctccctggacaagttcctgg
gcttctgtgagcaccgtgctgacctccaaataccgttaagctggagcctcggtggccatgcttcttgccccttgggcttccccc
cagcccctcctnccccttcctgcacccgtgacccccg
<212> Type : DNA
<211> Length : 372
      SequenceName : R91899
      SequenceDescription :

Sequence
--------
<213> OrganismName : HUMAN
<400> PreSequenceString :
ttctgcagatggttatccaactttcccggcatcatttattgaatagaatgttctttcctgagcatatattttgttgacttcat
caaagatcagttggttgtaggtatgtggctctatttctgggttctccattgtgttccattgatctatgtgactatttttatttt
tatactagtaccttctgttttagtcactatagcttaggtaggtataattggaaggtcagggtaatataatgcctccagcattgt
tctttttgcttagggattgctttgactatttaaggctctttttgggntcccatattaatnttatggatttattcccctaattc
tgtggaaaaatggacattgggggaacncgatagggggntgccctgg
<212> Type : DNA
<211> Length : 382
      SequenceName : R92422
      SequenceDescription :

Sequence
--------
<213> OrganismName : HUMAN
<400> PreSequenceString :
atacnaaactatgtcttcaaaagtctgtgtgtattttacatttatagcgcatctcaatttggtttagccacattgcaagtggtc
aatactcatttgtggctagtggctaccatattggacagtacagttctacaggaaccactcaaaacacaattcaccctgggntag
caatttgctactagtctgctagtgcttgagtgagctgttttaaactgctgttagtgaggaccactaaataggtgcatcttac
ttgactcgtgttttccctctgaggctgtccngaaggcaaagttcaggaatttttatttacaggcatgacttggattgactcaag
ggtgggg
<212> Type : DNA
<211> Length : 343
      SequenceName : R93094

SequenceDescription :

Sequence
--------
<213> OrganismName : HUMAN
<400> PreSequenceString :
ggagtttcagtctccaactctcagcccaccaatgaaagccactctatcaaagccatcctgaaaaatatctcagtcctggctttc
tctgtctgcttcatcttcactatcaccattgggatgtttccagccgtgactgttgaggtcaagtccagcatcgcaggcagcagc
acctgggaacgttacttcattcctgtgtcctgtttcttgactttcaatatctttgactggttgggccggagcctcacagctgta
ttcatgtggcctgggaaggacagccgctggctgcccaagcctgggtgctggcccggctggtgtttgtgccactgctgctgctgt
gcaacattaaagccccgccgcttacctgactgtggtctttcgagcacgatngcctgggttcatcttcttcatggctgcctttgc
cttctccaacggctacctcgccagcctttgcatgtncttcggggnccaagaaagtgaagccagctgaggcagagancgcaggag
ccattcatgggctttcttnctgtntttggggtntggcantgggggnttgttttttt
<212> Type : DNA
<211> Length : 560
      SequenceName : R98589
      SequenceDescription :

Sequence
--------
<213> OrganismName : HUMAN
<400> PreSequenceString :
atatgtttgttggctgtttgtacancttcttttgagaattatctattcatgtcctacccacttttggaggagattgtttttttc
ttgctgatttgagttccttgtagattctggatattagtcctttgtaagatgatgatttgtgaatattgtctcctacactgtggg
ttgtctgtttactcttctgattatttcttttgttgtgaagaatttttaaaatttaattagganccctatttatttatttttgtnt
ttgttgcattngcttttgggggnctcaggacataaattctttgcccaggacccaatgtccaggaaatgtttttgctaggaatt
<212> Type : DNA
<211> Length : 335
      SequenceName : T55802
      SequenceDescription :

Sequence
--------
<213> OrganismName : HUMAN
<400> PreSequenceString :
tctgtagcacttggaattgaatttgtgcacaatctaaaactatagaaggagaaattacagttggccctctgtatctggattctg
catctgtggttggttgaatcgaaaatattgggagggaaaaatggatggttgtgtctatactgaacacaaagtctttttcttgt
cattccctaaacaatacagcttaacgactatttacgtaggcatttacgtcgtattaggtattatgagtaatctaggagatgatt
taagtatactgggagggtctgtgtgggattatatggcaaattcaccactttatgttaggaggacttgagcctctgtaggatttt
cgatatccnaggaacccattccccccacaggtggccaaagggtggacngtggccntagggtgttacngttggaacccgatgttt
ttg
<212> Type : DNA
<211> Length : 423
      SequenceName : T71391
      SequenceDescription :

Sequence
--------
<213> OrganismName : HUMAN
<400> PreSequenceString :
ttgatacagaaatatttctgaaggatttctaatcagaattgtaaaacaatgtgctatcatgaaatcgcagtcttcacctcatgg
ttcatggaacatttggttagtcccataaaatcctatgcaaaacaaagtagttcaagaattttaggtaggtagtcacatttata
aggtattcctcttactctttgggctttttcagtctgatttatttaaattttcatttagttgttttacttttggactaaggtgca
atacagtagnaggataacttcgttacatttatgttgtagggaaactaaggtgctgtctcctccccttcccttcccacaaaatc
tgtattcccccctattgctgaaatgtaacagacactacaaattttgtat
<212> Type : DNA
<211> Length : 384
      SequenceName : T77711
      SequenceDescription :

Sequence
--------
<213> OrganismName : HUMAN

<400> PreSequenceString :
cgcttaacagagcaagtgatgggagccttgttcctgggttctaccatgggagacgctgatcagcaggatnctggctccctttcc
caacccactgctcccaaggcctccagggccntgtgtacataaactggtgggttggaagttgctgggtaactctnatttcagaca
tgcgtgtggggtacctttttctgtgcatgctcagcctgggctctgtgcgtgtgtgtgtttctatgattntagaagggtaccaggc
acaggttctgtcctaagggcacttaccatttagtangggagatggaaccaacccaattaactctagcaatagcctccctaacct
gggctcctccattngattcagtgaaccttccaatgcatggctcataatttcaaaatacaggctggttagttactccctacctng
aaagccttcataaggtgcctctttgc
<212> Type : DNA
<211> Length : 446
SequenceName : W07570
SequenceDescription :

Sequence
--------
<213> OrganismName : HUMAN
<400> PreSequenceString :
atgctgtcatcaccagtaagataccccagcccggttggctaacccctagctcagccctgtttgtggttaggggagttctagaca
gaacataattagacccctgctacttcctgaaacctcagctaggactgcagggaggggtgcgaggctagccacgcaggcggggcc
ctgggtcattttaaactctcagagtgaacgtcttgataggaccgacaagacgcatgacatgtacttagaaagcttatcttagag
ccacactgagattggaacccgcaaaatatgccaggaaacgccaccccagtgaccaccactgnnccgcgcagggccgtggggctg
agccacgctctcgttttgtcaggcagctatgcagttgctcttccttgttttgtttttttttttttttgtttttaatatttatt
tttttagagacagggcttncctgttgcctggnctggagaacagtggc
<212> Type : DNA
<211> Length : 467
SequenceName : W15172
SequenceDescription :

Sequence
--------
<213> OrganismName : HUMAN
<400> PreSequenceString :
cgtacatggcacgtggacctgcatagacacgtgtttggtggaggctgtcttatacggattctgcacaactcaagtcccctaatg
ggaacagacttggggaggtcactcttgaggctttttgtggtattgtggctactgggagacctgaggagggttctggtcactagc
agactcatttcattcaccccttggaatctgcccaggacttggacttctgtgtaatagtaacttcacgccccaatgatcctccac
gttccttgctgatgctgtgattgctcctccaagttctccacctatgtccatctgagcacttggtatttctactctagtgtcaaa
aggtcctgttcccaagtctcctcccaaacatttattccaatttatcatccagatgcccgtcctttgccatccctctctgttcca
gcaaaggaca
<212> Type : DNA
<211> Length : 430
SequenceName : W32067
SequenceDescription :

Sequence
--------
<213> OrganismName : HUMAN
<400> PreSequenceString :
ccangtgaagaggtcattattcgagacagccctgttctccctgtcaccctgcagtgtaacctcacctccagctctcacacccctt
acatacagctactggacaaagaatggggtggaactgagtgccactcgtaagaatgccagcaacatggagtacaggatcaataag
ccgagagctgaggattcaggcgaataccactgcgtatatcactttgtcagcgctcctaaagcaaacgccaccattgaagtgaaa
gccgctcctgacatcactggccataaacggagtnngaacaagaatgaagggcaggatcacctatgtattgcaagtcagttggct
acccccacccagactggatatggcgcaagaaggagaacgggatgcccatggacattgtcaatacctctggcccgcttcttcatc
atcaacaaggaaaattacactgagttgaacattgtgaacctgcagatcacggaagaccctggcgagtatgaatgtaatgccacc
aacgccattggntccgcctctgttgtcactgtcctcagggtg
<212> Type : DNA
<211> Length : 546
SequenceName : W44673
SequenceDescription :

Sequence
--------
<213> OrganismName : HUMAN
<400> PreSequenceString :
actttaaatacttcactgtactctcttcttgcttgcatggtttcttaggggaagttggatataattttattttctctatacg

taaggtgtttccccccctccccagcttctttcaggaatgttctttatctttttatttctgtagtttgaaaatngaaatgccta
catgtaggttttggggcatttatcctgtttggtattctctgagcttcctggatctgtggtttggtgtctgatattaattctgg
gaatttctcagtcacttgtttcaaatatttcttatattatttgtttcttctctttctggtgttcttattacacatgttacacc
ttttatttattttagagacagggtctcactcttacccaagccgttctcccacctcagcctcccaaataatcgga
<212> Type : DNA
<211> Length : 411
      SequenceName : W92423
      SequenceDescription :

Sequence
--------
<213> OrganismName : HUMAN
<400> PreSequenceString :
gctgaggctggggctcgggctggggccgcgctgcgggagctctggggagccggcggcccggccattgcgcccctccccggcgag
cacacctgtgtggggctttcccccccgccccggacccttctgggcttgatagtgttccgggggaggctggagggtgttgggga
ggacggagaggtcatgggagtccccgctctgcctcaaccacctgacccaggctcccccggtttctctcctccggcggtcgcccg
cgctcggtggatgtggctggcagctgccgcccctccctcgctcgccgcctgctcttcctcggccctccgcctcctcccctcct
ccttctcgtcttcagcttctagaagggggggagagggtgagaacgagaatgttaatcccaggcagcctcgctgacttctcccc
gcctgctccagcccaggggct
<212> Type : DNA
<211> Length : 441
      SequenceName : 1274737F6
      SequenceDescription :

Sequence
--------
<213> OrganismName : HUMAN
<400> PreSequenceString :
acttctatcacttccttttgatttttcttatagcttctatctctgcttacgtcacccatcattcttgcgtgttgtatgctttt
cgcattagagccctttgcatgttaatcagttattttaaatttatgatctggtaattctaaaatgtctgccataactgagtctgg
ttctgatgcttgttttcttttcagactgtttttttgtcttttagcatgccttgtaatttttttgntgatagctagacatgatgta
ttgcataaataaaccagtaaactaaaaaaaaangcaaatta
<212> Type : DNA
<211> Length : 293
      SequenceName : 1338107F6
      SequenceDescription :

Sequence
--------
<213> OrganismName : HUMAN
<400> PreSequenceString :
caggaagacaggtaaattacccaacctcacacgttaagtcagaactgggagccataatttgtatccctggtataaatagacaa
tctctcgaagaaatgaagagatgaccatagaaaaacatcgagatatctccagctctaaaatcctttgtttcaatgttgtttggc
atatgttatctttggaatttagtgtctgagcctctgtctgttactgtagtatttaaaatgcatgtattataatcatataatcat
aactgttgtt
<212> Type : DNA
<211> Length : 262
      SequenceName : 1436240H1
      SequenceDescription :

Sequence
--------
<213> OrganismName : HUMAN
<400> PreSequenceString :
gaaacccactacggcccagatgggcagccagtggtgctggcccccaaccacacggatcattgccactaccaagggcgagtaagg
ngcttccccgantcctgggtagtcctctgcacctgctctgggatgantggcctgatnaccctcagcaggaatgccagctattat
ctgnntccctggnnaccccggggctccaaggacttntnaacccangagatctttcggatggngcagctgctcanntggaaagg
<212> Type : DNA
<211> Length : 251
      SequenceName : 1508571F6
      SequenceDescription :

Sequence

--------
<213> OrganismName : HUMAN
<400> PreSequenceString :
aaaaaagttaaacacatgacattggaccttatgaaatgtgtttaccctagagctgttgattgtatgataggtccctcctagata
gtattcaaggtttaatgcagaaacagagcaagactctgtttcaaaaataaaataaaataaaatagctggccatggtggcttgca
cctatagtaccagctgctc
<212> Type : DNA
<211> Length : 187
      SequenceName : 1569726H1
      SequenceDescription :

Sequence
--------
<213> OrganismName : HUMAN
<400> PreSequenceString :
ggcagattgatgacatactcagtgtggcctccgtgcgtccagctgtcttgccacccatacttggctcaaaatgagctaattgcc
cactgccaagcacgtggcctggaggtaactgcttatagccctttgggctcctctgatcgtgcatggcgtgatcctgatgagcct
gtcctgctggaggaaccagtagtcctggcattggctgaaaagta
<212> Type : DNA
<211> Length : 212
      SequenceName : 1634342H1
      SequenceDescription :

Sequence
--------
<213> OrganismName : HUMAN
<400> PreSequenceString :
tgttctctgactattcttttgtttatcatcttgctcttttatgcgaatagtcattctttatctctgaagatagaaactggag
gacatttgggaagggatatgtcctgtgcattgtttcttttctcttgcctattctgttcgcgtatttaatgttgacacttttctc
caatggcttgagatacatggatgtctgctttttattttaagagcaggccac
<212> Type : DNA
<211> Length : 219
      SequenceName : 2950021H1
      SequenceDescription :

Sequence
--------
<213> OrganismName : HUMAN
<400> PreSequenceString :
gagacaaggtagtggaagtgtgtccctcccattcactgaccaaggtgaggggactctagtcctttgctgctgaccttcagcctc
ccttcctcttaaaaagaggtcctagttggggagcgatggctgaagagttaccataaaataccaagtaggnnnnnnnnnnnnnn
nnnnnnnnnnnnnnnnnnnnnnnnnnnnnnnnnnnnnnnnnnnnnnnnnnnnnnnnnnnnnnnnnnnnnnnnnnnnnnnnnnnn
nnnnnnnnnnnnnnnnnnnnnnnnnnnnnnnnnnnnnnnnnnngtagggccatttagtatggtttcatctttattaaaagtcttaa
gttttagattttgaagttatcaccaataaaatatccagttgtggtcttgtgttgagtgaaaatgttatttaaatgcagatatt
taaaatatactacaattttgt
<212> Type : DNA
<211> Length : 442
      SequenceName : 3010621F6
      SequenceDescription :

Sequence
--------
<213> OrganismName : HUMAN
<400> PreSequenceString :
cgcctggttaaatattttggcaagagtacttcataggccaccctcaaaatttcagcatcactcgtaccaagtatctatcacaa
ggcagcatgtgtctacatctccaccccagtgctgttacagtgggacagtttctagtgtattgtcattgcgaccaggcttttaag
attcttagcatatctgtaatatccacatgcagatgtagaatccaaatcgtatttacagagaactttgcatggcagcttttatat
ggtagtgatacattcagtttcttacattctagattattcagggaggttctggagttggacagatttggggttaagcctgatggc
tagctgtgtgaccttacacacctcgcttcatctgtttcctatgggaatttataatagta
<212> Type : DNA
<211> Length : 395
      SequenceName : 3028719F6
      SequenceDescription :

Sequence
--------
<213> OrganismName : HUMAN
<400> PreSequenceString :
gggggctgaggagtttggaaaatcttgctgtcagtagggagccattgagtgttgagttggggtgggggtgttatggttggcaca
tttgcatagttactgaggacaggttaggaggggcccctttctccctaatgccctacactgttcccaaccattatagtgaagctc
tttgaggtgattgagactgagaagacgctgtacctggtgatggagtacgcaagtgctggtgagccgcccaccctctccgccctg
cccctgtgccacctcccctgccgctgcacctgaccctgaccccgctcggcctctgccctgcaggagaagtgtttgactacctc
gtgtcgcatggccgcatgaaggagaaggaagctcgagccaagttccgacaggttggggcagggctgagggtggggctgactggg
tgcctgggtccctggagggcgtctg
<212> Type : DNA
<211> Length : 446
      SequenceName : 3081284F6
      SequenceDescription :

Sequence
--------
<213> OrganismName : HUMAN
<400> PreSequenceString :
aaaacaaagaaagaaaaaggatgattacttgaagtggtacaaatgacaaccaggtaccaagaaaacagtgagtttgggcaagtg
taatttttttagaactcacagtatatctagaatgggatgaaagggaagaagatataggatagtctcattcatatagaaagttatc
ctaaaatcatgtaacaaagaggttataaaaaccataaaatcatcacgaaaaatgacagccactagccctgaatgctgatgtaga
aaggacattgatgagaataaaccttctgt
<212> Type : DNA
<211> Length : 281
      SequenceName : 3100089H1
      SequenceDescription :

Sequence
--------
<213> OrganismName : HUMAN
<400> PreSequenceString :
cagggactgaccgtgagcctgggtgaaagtgagttccccgttggaggcaacagacgaggagaggatggaaggcctggcccccaa
gaatgagccctgaggttcagggagcggctggagtgagccggccccagatctccgtccagctgcgggtcccagaggcctgggtta
cactcgcagctcctgggggaggcccttgacgtgcctcagttcccaaacaggaaccctgggaaggaccagagaagtgcctattgc
gcagggagaggaa
<212> Type : DNA
<211> Length : 265
      SequenceName : 3171275H1
      SequenceDescription :

Sequence
--------
<213> OrganismName : HUMAN
<400> PreSequenceString :
ttgattggatctttgaaaagtagaagtttaatattagttgaaataattcattattgcataactttctattttgatataatttca
ggcttatgtnnnnnnnnnnnnnnnnnnnnnnnnnnnnnnnnnnnnnnnnnnnnnnnnnnnnnnnnnnnnnnnnnnngtggaata
attaatttttaattaactttgttaggccagtccctgagacgaataggaccttgtccctggatga
<212> Type : DNA
<211> Length : 232
      SequenceName : 3283686H1
      SequenceDescription :

Sequence
--------
<213> OrganismName : HUMAN
<400> PreSequenceString :
anaaaaattaggttggngtcctggaacatggacaatggctatttcttccggtaattgaagattattaagaacatgaacaactag
tgtttcatgagcaagttccttacctttactgttaaatagttctcgctcagcctaaatttttccaaatgtatgagatactctgaa
agcatatttagagtcagtatagatagttcctacctggttctgtaaatattttagggcctaagtgcgaataacttgcaagcttg
<212> Type : DNA
<211> Length : 251

SequenceName : 3290073H1
SequenceDescription :

Sequence
--------
<213> OrganismName : HUMAN
<400> PreSequenceString :
ggtgttcgtgtgaaagggtattgccctttacacgtgtgcttggttttgcctttcctatngtctaacacgctcaccgtgntttgc
atgctagtatgntatacangngntgntgnattatgntgatttgcatgctgtcttacatgcatgctgctgcattgtgttacacac
acgagtccatatgttacatgtgttacgtgcttatttttactaggtatttatgtttgcatattatatgtttctgtgtgcatcagc
atatcatggcacatgt
<212> Type : DNA
<211> Length : 268
SequenceName : 3335217F6
SequenceDescription :

Sequence
--------
<213> OrganismName : HUMAN
<400> PreSequenceString :
acggtactgttttctgtgggacatctgttggtttttgagaagcacctgctggcactgacggcagtcttggggaacacaggtgct
cctgccagagtcctcaccttctgctcacattgcagaaggccccctgctgaggccggctggccggaggaccaccccgcagaccac
gcgcccggggcctggcaccaagagggaggccccgatcagaaggggggagcggaaaccttattaagcttgcccatttcgccgtcgc
tctggtcatcatttaccgcaccctggggcagctcctgcccgagcgctacgaccccgaccgtcgcagcctccggttg
<212> Type : DNA
<211> Length : 328
SequenceName : 3461693F6
SequenceDescription :

Sequence
--------
<213> OrganismName : HUMAN;
CGGAAGTACCAGCTGCGGGAAGGAGCTGCCCACTGTGCGTCTTCTCAACTTGTTGGGACA
ACCTTCCTGCGGAAAGGAGAGACAACCTTCCTGCAGAAAGGAGCTACCTGCTTCAGGTCT
ACTGAGAGCTGTTCTGCCACTCAATAAAGTCTTCTCCATCTTGCTAGGNCTCCATTTGT

<3721604H1;DNA;HUMAN
<400> PreSequenceString :
acagagcatt aagtgcctct taaacttggt ctctctggtt ctgtgggtct ttttggaat 59

<212> Type : DNA
<211> Length : 59
SequenceName : 3674358H1
SequenceDescription :

Sequence
--------
<213> OrganismName : HUMAN
<400> PreSequenceString :
gcagcccttcggaaaagtggccagactgttagggtgcctgttcccatatctcctcaccaggcaggtcctccaggcctgggcctc
caggaatcncctgccagagcaattgagtctgtagctctgctactccctggtcagatcccccaggggcaaatgaaagcctctctg
ccactgtctctacagtggaatgcccttgctacccttggataacnaagg
<212> Type : DNA
<211> Length : 216
SequenceName : 3746214H1
SequenceDescription :

Sequence
--------
<213> OrganismName : HUMAN
<400> PreSequenceString :
gaaggagggggggaacgaaacaaaggaaaaaaatctaggtctgcacacaaagaactgctgggtgaacggctgtgggcatttcat
ggaaatattgcccgctttccccctttgaatgcttacctggcaggagtcgactttgccttccaggaagcctgcgcagatcatgcg

gtctgtgagggagaagttgtagagcacactacaggtttctggtctatgatgcccacggacgccttctgcaggagctcgggctt
ggtggctaccagggaaagagccagaaaggccacgtgcacag
<212> Type : DNA
<211> Length : 293
      SequenceName : 3820429H1
      SequenceDescription :

Sequence
--------
<213> OrganismName : HUMAN
<400> PreSequenceString :
aaaaaccccaaaaccaaaaccccacaaaatcagaggctcaagatgactgatgtgaagggagtggcgtttaagaggccatttat
tttgatgacgcagctgcccaggaacagagaacatgggagaaggcatagactgacaattaggaggaggagaacactttggaagga
gactcttattttggtggggcagctgctcaggaacaaaggttcctggtagggggcgcaacctgcgggatgggatggagggtatt
ctgaccaatgtccctggc
<212> Type : DNA
<211> Length : 270
      SequenceName : 3881595H1
      SequenceDescription :

Sequence
--------
<213> OrganismName : HUMAN
<400> PreSequenceString :
gttaaaatgaaatccagcgcctttattttggaaattccatcttgatttgatatttgggatatataaaaaatgtggttgtaatac
atctgatgaaatttaaacatcaaacatactgaagaccaaaatgaagttggaaaggcattagctaaaacaattggcatgtaaata
aggatcttagtagtgaatttttccttggctttcttcttgatagagttcatttagaaaactgagtgaactaagaaattgttttt
gtttgttg
<212> Type : DNA
<211> Length : 260
      SequenceName : 1869911H1
      SequenceDescription :

Sequence
--------
<213> OrganismName : HUMAN
<400> PreSequenceString :
gctggccagccacgagtgaccctgctgcccacgcccaacgtcagcgggctgagccaggagtttgaaagccactggccagagatc
gcagagaggtccccgtgtgtggctggcgtcatccctgtcatctactacagtgtcctgctgggcttggggctgcctgtcaacctc
ctgaccgcagtggccctggcgcgccttgccaccaggaccagga
<212> Type : DNA
<211> Length : 211
      SequenceName : 2021568H1
      SequenceDescription :

Sequence
--------
<213> OrganismName : HUMAN
<400> PreSequenceString :
caggaggaagagagaagccggtagtggctcagttctagtccaaaaggctcagaagtagggaagttgacagtgcagccttcagcc
tgtggccagaggcctaggagcccaaggcaggccaccagtgcaagtcactgagtccaaaggctgaagaacctggaatctgacgtc
caaaggcaggaggaaccacaggagaaagacggaaaccagaagactcagcaggccagcacatccctccttcttctgcctgttttg
ttctagccgcactggcagc
<212> Type : DNA
<211> Length : 271
      SequenceName : 205203T6
      SequenceDescription :

Sequence
--------
<213> OrganismName : HUMAN
<400> PreSequenceString :
caaaaaaacaaaaacaaaaacaaaacaaaaaaaaaacctcagatttcagggcctcaagcctacagttttaggtcaaggtggga

tnnnnnnnnnnnnnnnnnnnnnnnnnnnnnnnnnnnnnnnnnnnnnnnnnnnnnnnnnnnactcttggggacccc
tccctttacaaaatggatggacatttgagccaagccagactaattgtccttatctggtattgtgtcatactggaattaggaa
aagcagcccttggcttcaactggccatataccataaggacatgagacctaaagtgccatgtactcctgccatgcagagaagcca
gatcaaaagaatctaaca
<212> Type : DNA
<211> Length : 354
      SequenceName : 2225447F6
      SequenceDescription :

Sequence
--------
<213> OrganismName : HUMAN
<400> PreSequenceString :
aatttttgatagagacagagtctcactatgtagcacaggctggcctcaaactcctgtgctccagttattctcccacctcaaca
ttaagtacattttattaaactattcctaaattagagctccagttgggttggcagaagggtccaatattttgacagagaaatct
taaagttattttgatagatgtatgaggagaggctttaagagtgtctatacttctcatgaattggaaagaaatactttctctg
<212> Type : DNA
<211> Length : 250
      SequenceName : 224922R6
      SequenceDescription :

Sequence
--------
<213> OrganismName : HUMAN
<400> PreSequenceString :
gaaagagtagtctcatgttttcctcaagaaccaagatgaatagcaatanaattgcttccaaaatgggttccttctcccaatcag
attctgtagctcttcaccaaagggaacatgttgaactgcttagagccaggagattagccaagtcactggccattctcttagggg
tttttgctgtttgctgggctccatattctctgttcacaattgtcctttcattta
<212> Type : DNA
<211> Length : 223
      SequenceName : 2490994H1
      SequenceDescription :

Sequence
--------
<213> OrganismName : HUMAN
<400> PreSequenceString :
cctccctcccctccatccctccattcccctccatcccttcatcccctccatcccctccatccctctatgcccctccatcccctc
catccctctatgcccctccatcccctccatccctccatgcccctcaccaccacctgagggtctcccacccctctaccactctg
tgtctcctctcccaccctcttccctggagggcttacagccggctgtgcttccaggagccct
<212> Type : DNA
<211> Length : 229
      SequenceName : 2531082H1
      SequenceDescription :

Sequence
--------
<213> OrganismName : HUMAN
<400> PreSequenceString :
gtcagaaggaccatgaagagagagaagagggcagagccgtgcatggggctgctccccaggacctgagcaggaacctggagtttt
caggtgagaggctcacctgtgagcaggatacctgcctgctcgcctattggnctccannttgactagctacctagctaacatctt
tcctagtacctgt
<212> Type : DNA
<211> Length : 181
      SequenceName : 2673259F6
      SequenceDescription :

Sequence
--------
<213> OrganismName : HUMAN
<400> PreSequenceString :
tgacaacttgacttctctggggactctgaacttccctggtcgaacagtttttctaatggctattcaaggtaagatcagtgttt
ttctgtttcttaagaatggtacatttaaggtagattaatagatgtaaatcttcattgatttatatgtgttctctaaagattcat

gtgctttttatatgaataagtttaagtggccttttgaaagtaggaaaggtagacaacctaagtgacatctgtacgtaacca
<212> Type : DNA
<211> Length : 250
      SequenceName : 2716787H1
      SequenceDescription :

Sequence
--------
<213> OrganismName : HUMAN
<400> PreSequenceString :
aaaggttagtaggaaatgtgtttcccccatggggcattcctggagaaatctctagcaataaaggtacttgtttgactggacaa
gttgtaaaacagtttaataaggtttttttaggtgtcatagcattaggcaaagctaactgaattgactggattgtcatagtcaa
acatattgcagatccgcttccaatagaagacaatgaaatagtcactggaaagcctgtgctcctaattttagaacctcatgtatc
ttctgctcct
<212> Type : DNA
<211> Length : 262
      SequenceName : 2914934H1
      SequenceDescription :

Sequence
--------
<213> OrganismName : HUMAN
<400> PreSequenceString :
gtatatttatatattatactggaacagctcgtgcccttggtctcttgcctcagcacctgggtggcttgccgcccacaaactgc
tcactgttcaattaccattttcagaatgctttttatgtttgtttgatgaattcataaagtctctaatgtgaaataagccaagca
cagaaag
<212> Type : DNA
<211> Length : 175
      SequenceName : 2919406F6
      SequenceDescription :

Sequence
--------
<213> OrganismName : HUMAN
<400> PreSequenceString :
ttgcataatgaaaatccaaagtaaaactacaatgagaaactattttatacctattaaatattttaaaatttaatgctaacaaga
ttaggtaaagttaatattcacatgaaattagtagagctgcttttgaaagcagaatggtagttcatgtcaagagctatggtaaaa
attgacatttcattatcctaagggagtgattaagaaga
<212> Type : DNA
<211> Length : 206
      SequenceName : 2959801H1
      SequenceDescription :

Sequence
--------
<213> OrganismName : HUMAN
<400> PreSequenceString :
tttgaggcag gactggcact ggggtggggg catggtttgg ggttacctta aggaccctgg      60
atatttctca gataggac                                                    78
<212> Type : DNA
<211> Length : 78
      SequenceName : 3041874H1
      SequenceDescription :

Sequence
--------
<213> OrganismName : HUMAN
<400> PreSequenceString :
cgctgggagtcggctgcccgggctgcgcggggaacctggagggcaggagcaggcgggagttgttgcttcggggcgcagggtcca
gaccttgccgagaccggcgttgggagctagcagaaccgggaagaggctcgattgtgtccggtggggccgccgggagcctcggcg
acctttgtctcctgcctggctcctgcactggagtgtgacccggggcgctgcagc
<212> Type : DNA
<211> Length : 222

SequenceName : 3332309H1
SequenceDescription :

Sequence
--------
<213> OrganismName : HUMAN
<400> PreSequenceString :
agtgcaggagtgagtaggtcttttactgacactactctcagtgaggctcagcattcaatgtctatagatgcttaagcagtagta
agaaaggtataagaaagcagccagaaatcataatgagagctctttcataccatttatagcgtcctcatgtcactcctaatgtta
taatttctctggttatattagagaatcttaaagattgg
<212> Type : DNA
<211> Length : 206
SequenceName : 3918104H1
SequenceDescription :

Sequence
--------
<213> OrganismName : HUMAN
<400> PreSequenceString :
aatacaaagacaacatggggccagaactccagaaaggatgctgactttaagaggactcagccacccatctcagagagcacttca
gaggatccagtaagggtcagaaaagacagtgtaagctgatatttatctaatttaagagatgcaagctcaaatgccttcctgggg
catacaagaatatgtataaaagaaaaactgcaaatgtgatgataaaaggcaactgacttccacccctcagtttgag
<212> Type : DNA
<211> Length : 243
SequenceName : 4031124H1
SequenceDescription :

Sequence
--------
<213> OrganismName : HUMAN
<400> PreSequenceString :
tatgaaagacatctgttgtgttttgtatgtgcacatattgagagacatccataaactgaatagttgtaatattcagccattcta
aaatttattttaaccacgttactgaggtatgattgacatacaaaaagctgtacatatgtaatgtgtaaacttgatgtg
<212> Type : DNA
<211> Length : 163
SequenceName : 4056384H1
SequenceDescription :

Sequence
--------
<213> OrganismName : HUMAN
<400> PreSequenceString :
ggtattttaaaaaactgtgcacgttggggtactagaaacgtaatgggctttggagtacagcacacctgggttggaattctggtt
ctaccactttctgtgcagccttggcccaggtagttaatctttcagtgcaagcagtgttgtcattcataaacatggaggtgatgc
ggatcatgccagatatgctatggacattaactgatctgtgatattaaagcatagtcactggtacttggggttacctaa
<212> Type : DNA
<211> Length : 246
SequenceName : 4333034H1
SequenceDescription :

Sequence
--------
<213> OrganismName : HUMAN
<400> PreSequenceString :
tgggtgtgggtttagaggtccccaggcggcctcccccctgaacgtcccagcgcccccagagcaggtgtgcgagcactgatgggat
ttcctgcatttctgtgttaagggaaacagatggccaagcctcgggggcagtgattctgggactg
<212> Type : DNA
<211> Length : 149
SequenceName : 4516963H1
SequenceDescription :

Sequence
--------

<213> OrganismName : HUMAN
<400> PreSequenceString :
gctctgtgaattcccaaaacagcttgaaggggcaccgtgccctggttggggtggggtgaggggcccagtgactcttatggggcc
ctcaggccattcccttcatgggaaatggcagccaagagcatcaccaagaatgactggttcacgtccttgctcccagaactttcc
ggggctctctggctgaccacaggccaagttccagacccctctgggtggggatgctgagtcagggagtgaggggaccagatctgg
gttttggaagggatgggagggagggaggtggaaagcagggaggcctgggagagacgctgagcagtggctgagggtgtggtgcag
gacagcaccttttcccagaggattctttcggcggggttggttgcggggtggtgctgcccagtttctctcaaagtctgccatcg
gtcaccagccctgggagctggcaggggctggggagaaggaagagggaggatgttgcccagaatcgcccccacatggccaagcag
ttgcctgtcacctcctcttttctccctcttcagatcctggtgaagaccatgatacgtaaacgctcc
<212> Type : DNA
<211> Length : 570
SequenceName : 4539942F8
SequenceDescription :

Sequence
--------
<213> OrganismName : HUMAN
<400> PreSequenceString :
cagcaaccctggtgaagaaggttgaatttggaactgacacacttaacattacttggatgagctcattaagaacactcagactt
acaccttcaccatcagccaaagtgtgctggtcaccaacttgaaaccagcaaccatcaaggtctatgactactacctaccagatg
aacaggcaacaattcagtattctgatccctgtgaatgaggataggagctggaaactcagttagtcctctgtgacattactggag
ggtggaacattcttctgtcgctt
<212> Type : DNA
<211> Length : 275
SequenceName : 4558488F6
SequenceDescription :

Sequence
--------
<213> OrganismName : HUMAN
<400> PreSequenceString :
ccctgggctgctctgcacccggacacttgctctgtccccgccatgtacaacgggtcgtgctgccgcatcgagggggacaccatc
tcccaggtgatgccgccgctgctcattgtggcctttgtgctgggcgcactaggcaatggggtcgccctgtgtggtttctgcttc
cacatgacgtcctggctgcccagcactgtntaccttttcaatttggccgtggctgatttcctccttatgatctg
<212> Type : DNA
<211> Length : 242
SequenceName : 4609810F6
SequenceDescription :

Sequence
--------
<213> OrganismName : HUMAN
<400> PreSequenceString :
gggctacttcacctccctttctatgtctgcttcaggaaggaaagaaaggagaaataaggcttagggctagaatcaggaaataaa
ggctttcccagaaacctccacacttgtgcttaggttgtattagacagaactgtgtcacatagacactgctacttgtaagagaca
ctgggaaaccatgtctagcacttacagcctcgaatgttcctgctgtcctacattcaaagacattactctgtacatattaacctt
cactattcagatgaactacaactggaactggatctaggatatagg
<212> Type : DNA
<211> Length : 297
SequenceName : 4820983H1
SequenceDescription :

Sequence
--------
<213> OrganismName : HUMAN
<400> PreSequenceString :
gcgagtctccggtttccgcccagtctctggggacgcggtggcggggtctgaatcgcctggcttccagggagttttggcgggtc
cccgcacgggagcgtggcaggcgctggactcgacagcagatgtggaactggagagcttggcgcgccctcatactttgtcacaca
cctacgccaccagactggggtcgggcccctccgcgttctgctctggagggcctgggtctggcccagcatagcgcttttagaat
ctcctcagctgaa
<212> Type : DNA
<211> Length : 265
SequenceName : 4835576H1

SequenceDescription :

Sequence
--------
<213> OrganismName : HUMAN
<400> PreSequenceString :
ctgcctttattattttgtcgcgttattcaaaatcagataactgtatgcctattattattaagatagggtcttgctatgttgcc
taggctggccttgagcttgtggctcaagggatccctctcatcttagttttactaattactaagggcacagacccttgccact
<212> Type : DNA
<211> Length : 167
      SequenceName : 4902714H1
      SequenceDescription :

Sequence
--------
<213> OrganismName : HUMAN
<400> PreSequenceString :
cgcagcgcagactatagtttcttaaggaagattttcttaaatggggattcaccgacccaggcatctagtccattcttccagtgt
gggaagtatttattttccttgaaaggaagtctgtacataaatggattctgagaaagctgtgctagatctgccgctgctttttg
gtttgggtttttttgagcctgggtgacagagcgagactccatctc
<212> Type : DNA
<211> Length : 214
      SequenceName : 5020377F9
      SequenceDescription :

Sequence
--------
<213> OrganismName : HUMAN
<400> PreSequenceString :
ctggggcaggcaccaggctgtcttcagatacttcatacagaaatggtatctccctgacccttttctgcgatttgcctcatctgt
cttcatctcatcaagggtcaggacgtaggagccgacagacccagcacctttctgagtctgtcctgtccaagtgagggtgactgg
gggcttgttcttcttctcagagcctccctgtggngttctcttccctccttcagcctgtccatcaacacagcattgcgggatcct
t
<212> Type : DNA
<211> Length : 253
      SequenceName : 5104579H1
      SequenceDescription :

Sequence
--------
<213> OrganismName : HUMAN
<400> PreSequenceString :
taagaacgcggggagatggatttcatgccaatcgttccccagcgacccagactcagaccctggggcatcgggtgtgtgcatag
agggggcagtatatgggaagggctcaatcaggtggctccacggcccaggaaagagg
<212> Type : DNA
<211> Length : 140
      SequenceName : 5122374H1
      SequenceDescription :

Sequence
--------
<213> OrganismName : HUMAN
<400> PreSequenceString :
attcagcact ttattgtaaa ataggccttg tgttagaatt caagctgtct aaaccacctt      60

<212> Type : DNA
<211> Length : 60
      SequenceName : 5327443H1
      SequenceDescription :

Sequence
--------
<213> OrganismName : HUMAN

<400> PreSequenceString :
aagattttcaagacgaactgtgatttggggagggatggccatctcagtcaactttaactgataggaggtacaacaaatttatag
ttctgaactctgcacctggagtctgtctaccgaaatcaaaccacttgctgaccagcataaaaataaataggaaaatatttcagt
gttttccacacaac
<212> Type : DNA
<211> Length : 182
SequenceName : 5537789F6
SequenceDescription :

Sequence
--------
<213> OrganismName : HUMAN
<400> PreSequenceString :
gagctgcagggtctggcatcagctctgtgtcttttccatggccccaggctggctcaggtgtgagctgaagaggcaaaactgtct
gtttaggttgcattgggttcccctgaaa
<212> Type : DNA
<211> Length : 112
SequenceName : 5539620F6
SequenceDescription :

Sequence
--------
<213> OrganismName : HUMAN
<400> PreSequenceString :
tgcatgcatacgcaaggcagtttgatcccagtgcttttctttttagcaactatannnnnnnnnnnnnnnnnnnnnnnnnnnnnn
nnnnnnnnnnnnnnnnnnnnnnnnnnnnnnnnnntcagatcttagaatctttgcatttaaaaatgcaaactgattgagcatctg
a
<212> Type : DNA
<211> Length : 169
SequenceName : 5594552H1
SequenceDescription :

Sequence
--------
<213> OrganismName : HUMAN
<400> PreSequenceString :
ctctgagcccatggggaatcttcttatctcagcattgcagaattcagaatccacggtaacttttcatgtcattttgactgcttg
tcatgcatgcacttctttttagatttctctcatgaaaagtgttgacaaatattccttttggaaagagctcacttttccattac
actggttctaagtatcaggaatatatcagaggacatttttatccacttgtgggatgcatccacatggcta
<212> Type : DNA
<211> Length : 238
SequenceName : 3406131H1
SequenceDescription :

Sequence
--------
<213> OrganismName : HUMAN
<400> PreSequenceString :
cataacagcattgtggatgatgagaggatgaagtccactgagtacatcagcacaaaggaattcaccagcatcaggatggtcttg
gtggccattttcactagagaagtccttaatataaggctgctgctgtgaaagtgctgggagtgcctctgatgcctggacaaaaga
atgaccatgtatacacttgagagcagcgtgattcctatgaagaaggcatctctggataatgagagcataagaaatagcgtcctg
atgaggacattcattgggaaaaatgtgcaatatttgct
<212> Type : DNA
<211> Length : 290
SequenceName : 3568042H1
SequenceDescription :

Sequence
--------
<213> OrganismName : HUMAN
<400> PreSequenceString :
aaattggcttgatgtaattctgttccatagaaagaatttagataagactttttaaagctgagcccaactatgggtttgtaac
ctcaaatacatatgagttgggtaaatttctttcctcttgaggtccaaaataacttggggctc

<212> Type : DNA
<211> Length : 146
      SequenceName : 4288779H1
      SequenceDescription :

Sequence
--------
<213> OrganismName : HUMAN
<400> PreSequenceString :
gaaaatcaccttcccgaggagcaggcgtggtgcagacccacaggggtttgtggcgggagtagagggtctgtttggggcgagata
agagactgaacggcagagtagggagtgctggctaaggcagtgctgcagagcgaaactctagac
<212> Type : DNA
<211> Length : 147
      SequenceName : 4301823H1
      SequenceDescription :

Sequence
--------
<213> OrganismName : HUMAN
<400> PreSequenceString :
tttaataggtgggatgtggtgctagtccaacatgtgtgggaaatggaacaactttaaaatattctatttggggtttcagatatt
ggtatttacaagacatgtccatattctaatgtagatatcaaaacagtcgatacaggccaacatcaaaat
<212> Type : DNA
<211> Length : 153
      SequenceName : 4407508H1
      SequenceDescription :

Sequence
--------
<213> OrganismName : HUMAN
<400> PreSequenceString :
acattgacagctttgaagagtgcagcctccagtcagatggctctgaggtgctcctgagtcctgaagtcacctgtggtcctccag
acatgatcgtcaccactccctttgcattgaccatcccgcactgtgcagatgtcagttctgagcattggaatatccatttaaaga
agaggacacagcagggcaaatgggaggaagtgatgtcagtggaagatgaatctacatcctgttactgccttttggacccctttg
cgtgtcatgtgctcctggacagctttgggac
<212> Type : DNA
<211> Length : 283
      SequenceName : 4570377H1
      SequenceDescription :

Sequence
--------
<213> OrganismName : HUMAN
<400> PreSequenceString :
tgggagagagagtatctaggaggtacctgcgctttctccccaccacggtaagtaacgtgccatcattctcccatttcacagagg
gcagactgaggctctgaacgcggaagttcttgcaagatatcacgcagcagagcagggatgtgcaccagatctcctgactttaaa
aagcaccaaacatagtgattaagaacaaggact
<212> Type : DNA
<211> Length : 201
      SequenceName : 5801413H1
      SequenceDescription :

Sequence
--------
<213> OrganismName : HUMAN
<400> PreSequenceString :
aaatgaaaactgaaagaggctcaggtcaccgacttagatcccaccatcagtcagatgcgtatgtggtgccaacgctgctggatg
ctccaaccctcttgtcctccgagctttgggcttcttcggcccactgccctgtggctcccatcacctcctgctgtgccgtggagc
tgggcttccctcgcccggggccgtgtccagctatccagcccacactcagcgctgtatgcggatgctgctgccttcagtggtccc
aactgcctgccaaccgtcccaggtggagccttgctgctggcggctcttgtggttgggtgctctcgaaggttactgattcggaaa
g
<212> Type : DNA
<211> Length : 337

SequenceName : 5905620T9
SequenceDescription :

Sequence
--------
<213> OrganismName : HUMAN
<400> PreSequenceString :
gggtaacagagcaagactccgtctcaaaataaaataaaataaacattgtatatactttttaccagtaaatggtgaaaatatag
atgaattgggtggttttctaggaaaatgtaaattaccaaagtgacttaaggtgggacaggaaacctgaataaaatggaaaagta
gggaagaaactt
<212> Type : DNA
<211> Length : 180
SequenceName : 6421806H1
SequenceDescription :

Sequence
--------
<213> OrganismName : HUMAN
<400> PreSequenceString :
ggggaaacggaggcatgggcagaggaactgagctgtacctggaagtggaagacgcctgcgtagccatgctggaaatcctgtcca
ggagggaccacccggcgcaggagccggggatacagagtaagggaggcggcggcggcaaggaaccagcagttacctgggaggaga
ggccaggattaggtgaggatggaaggaggga
<212> Type : DNA
<211> Length : 199
SequenceName : 5058893F9
SequenceDescription :

Sequence
--------
<213> OrganismName : HUMAN
<400> PreSequenceString :
atcctcattg ctactgccct ctgcgctcct gcatctgcct gcccatattc ctcggacacc 60
acaccctgct 70
<212> Type : DNA
<211> Length : 70
SequenceName : 5189380H1
SequenceDescription :

Sequence
--------
<213> OrganismName : HUMAN
<400> PreSequenceString :
gccaccacgcccagcctcctatccctacttcttacaggttatcacataaggaatctgagccacttcttattttgaaattatttc
atctgtcttaatgtcagtttagtatctgtaacaataacttatttcattttctttccaatgaaatatatctttatctccataata
cttagtgttccataatgatatgtggaattgaagggctctctacaacacaatctcctcatgcccacacctaccttcaacatttat
cttaagctaagccactaagagctttctcaagttgtgcttcctaaagctgcagttctcaaaatccaccccacatacctatgaagc
ccatataggaagccccatttagaaataattttgtaatcgaataaatattagaaacacttcacacctcctcttgg
<212> Type : DNA
<211> Length : 412
SequenceName : 5375277T9
SequenceDescription :

Sequence
--------
<213> OrganismName : HUMAN
<400> PreSequenceString :
ctccccattgctcctagctcctgaggctcaggacccctggcttctgtcctccctgctcagggtcctgcagcgttgcctctgctc
agccatgctcctgctgctcgtcccagtgctcgaggtgatttttactctgggaggaaccagagcccagtcggtgacccagcttga
cagccacgtctctgtctctgaaggaaccccggtgctgctgaggtgcaactactcatcttcttattcaccatctctcttctggta
tgtgcaacaccccaacaaaggactccagcttctcctgaagtacacatcagcggccaccctggttaaaggcatcaacggttttga
ggctgaatttaagaagagtgaacctccttccacctgacgaaaccctcagcccat
<212> Type : DNA
<211> Length : 390

SequenceName : 5537736F6
SequenceDescription :

Sequence
--------
<213> OrganismName : HUMAN:
GTAAGCATTCGGAGGGCTCTCTGGCTTTCCGATCTTGGGTCGCTGTACTTGGCTCTGAAT
ACAGTCACCCCATCAGAGTAGCAAAACACTGTGGNTGTAGTGTGAGAATCCANTGTTAAT
GTTGCCGAANCCNNTGNATTTCCTTCCNTGANAACATAGCGCGAGAGAATTGGAATGNGT
TCCAAACNTGTGAC

<5541621H1;DNA;HUMAN
<400> PreSequenceString :
gaatttcttttttgttttggtgaacatatatgttcttatttctgatattatattgttcataggaatgtagtcatatttgcta
ctaaaaaacgttcatatggtttgataccacatttcagcatttcacaatgatctgcaacttccaaatgctctgcactagaacaag
tctgaaaatcactcaactggcctt
<212> Type : DNA
<211> Length : 192
SequenceName : 5538394H1
SequenceDescription :

Sequence
--------
<213> OrganismName : HUMAN
<400> PreSequenceString :
acaagattctgatataatgaacctaaatcaaaattcacaaatggtggctgaaatcctgtgagagtacaacataaatccatagtt
attgtgcataaacaattcaaaattagtttataaagggaacgtcaacaagtcctataaccaccaccttattactaat
<212> Type : DNA
<211> Length : 160
SequenceName : 5543360F8
SequenceDescription :

Sequence
--------
<213> OrganismName : HUMAN
<400> PreSequenceString :
ccctaatttccctaaccagtgctggcccaggtaacagaatattgggttgtgacatactatgttttgtctgacttgctggaataa
aattacttgagagcattgaaaccacacaatcagttttcacctaatatcagcagcgctcatttccccgtcttcctttggacccc
agagccacagagacaaccaacaacaagcagatggcccagtgccctgcctcagaagatttatgtattcctaaggaatagccagct
taatggagattcacagagagggtttctttctattcttattattcagataattca
<212> Type : DNA
<211> Length : 306
SequenceName : 5543749F6
SequenceDescription :

Sequence
--------
<213> OrganismName : HUMAN
<400> PreSequenceString :
cttgagaggaaatgcaaaagtttgaatccaggactacttcctaatcaatgctcaatgccagtcgatcggtccacccttggcaag
tgtaggtgtgcctttccaggtagctcattgaaaggggcacttctttgtagcaggcccgtggacttgattggcatgtgtgtgaca
ctttgtggcactcatgggtttgtgctagactttattaggttccatagtcctgtaggctctgaactctaaaagaatgtgtgagtt
acagaaaagattccacactggtcataaaagaaccaaatgctaaagatgcaaagatagtaaaataggtatatgagtccattatat
ataggaaggtgattccaagaatgaaactaccacat
<212> Type : DNA
<211> Length : 371
SequenceName : 5546249F6
SequenceDescription :

Sequence
--------
<213> OrganismName : HUMAN:
CAGGTGCTCAAAGGAAAATTTAAGACAGTCCATGGAGGAGAAAGGTATAGACAAGGTCAT

GCAGATAT

<5911413H1;DNA;HUMAN
<400> PreSequenceString :
ttattggtttaagttctaagcaattgctgctcttactgatggcatccgcagcctgtctggtgcagtcactgcagggatgccagc
ttcaacaggggaagtgcagcccggactgtgcactccatatagctagcaggggccaggaacaggagggagtcccaccttctactg
aattaaaggggcaggagccccatgcttcccaacacaaatgcagctgcccagatgtggctctggacccaggcatccctgtgctct
caggaagcccctccatgccctcacaggctccaa
<212> Type : DNA
<211> Length : 285
      SequenceName : 5695322H1
      SequenceDescription :

Sequence
--------
<213> OrganismName : HUMAN
<400> PreSequenceString :
ggacggctcctggtggctgtgctgctgctgtgagcgggcccctgctcctccatgcccccagctctccggctgggtgggcttgg
ccatggtcagcgtgaacgcgcccctcggggctccagtggagagttcttacggtaagtggggctgggggaagactggacagggcg
ggactgcggtcagctttgggaggccatgggacacctccccgtgtgtttcttacgggcccaaagctcctcctggagctcctctgg
aagggcaggaagccccacggaggcaatgtgaccgcttccctaagctctcaagccggggcatggcctgcttggtgcgagaagtgc
tgggaggggccgagaagccctggggcagaggcttggctcattttgtctgtccagaggccttcctgggaatgcatctcaggggtc
tttccaaaccccacagagggtggggcttggaagcaccgtcctgtttcgatgcggggcaaattgaggtccaccaggagaggctt
gctgggcctaggtcacgttgctggtgcattat
<212> Type : DNA
<211> Length : 536
      SequenceName : 7007735H1
      SequenceDescription :

Sequence
--------
<213> OrganismName : HUMAN
<400> PreSequenceString :
aggnggggcnancaannnnccagggagnagnnctgnnncntctctcaggagaggttcctgggaagaagcttgtcgctttggatca
gtgtaatttcttccnccgttcagggccactcatacagtaatcggaagtacgagtctgacgaagacagcctgggcagctctggac
gggtaatgcgccctgaggcacncttgtctctgcctcttcctgtctcctccaggctctgttgctgtttctcctgtctcatatctc
t
<212> Type : DNA
<211> Length : 253
      SequenceName : 930273R6
      SequenceDescription :

Sequence
--------
<213> OrganismName : HUMAN
<400> PreSequenceString :
agccagtggccagcatgagccggagggagggaagtctggaagacccccagactgattcctcagtctcacttcttccccacttgg
aggcccagatcccgtcagacacacagccttgcgcacctcctcaccaaatacgctgagcagctgctccaggaatatgtgagtggg
aatgggggtgggggtgccgggggcctggggaatgggagcagacatcacagaggtcctcgtatcacccattctctcaacctcatt
ttccaactgtggaaatcaggggctcctgtagtaggtgacagcaagttgcccctggtcacacagcaaattggcaggaggaccctg
tctgtgacccagatgccactctgtcctctccagcttagaggcaccctcctcagtgg
<212> Type : DNA
<211> Length : 392
      SequenceName : 6313632H1
      SequenceDescription :

Sequence
--------
<213> OrganismName : HUMAN
<400> PreSequenceString :
ctttgcaaacataaatctgttggtgctcaggctagctggtagagccccatcaaaaattggatgcctttgaaaagtttgcttaga
gcaggggttctaaatttggtgcgcacaaatctccatggataaaatnnnnnnnnnnnnnnnnnnnnnnnnnnnnnnnnnnnnnnn
nnnnnnnnnnnnnnnnnnnnnnnnnnnnnnnnnnnnnnnnnnnnnnnnnnnnnnnnnnnnnnnnnnnnnnnnnnnnnnnnnnnn

nnnnnnnnnnnnnnnnnnnnnnnnnnnnnnnnnnnnnnnnnnnnnnnnnnnnnnnnnnnnnnnnnnnnnn
nnnnnnnnnnnnnnnnnnnnnnnnnnnnnnnnnnnnnnnnnnnnnnnnnnntgttgatatttaataactgtcttttgat
atcattggtttgccttgtaatcttatgtatatagatttattaagttttccttttcttgcgtgtgagttgtccccattccaaag
tattccatctacttttctaatcgcgtcccctttgcaggcctctggggatggactgg
<212> Type : DNA
<211> Length : 562
      SequenceName : 6591535H1
      SequenceDescription :

Sequence
--------
<213> OrganismName : HUMAN
<400> PreSequenceString :
caagtatcactgtatttccctagaaaacctgaaatatcgatgaattttctgtggcactctaggggtcccttggtgcacactatg
ggaaccatgggattaggaccattaggatttgattatggcttcttcctgcctcagatctaatctttacctggcattttgcctta
aagatgaaagaagcatacattttgatgtatttnnnnnnnnnnnnnnnnnnnnnnnnnnnnnnnnnnnnnnnnnnnnn
nnnnnnnnnnnnnnnnnnnnnnnnnnnnnnnnnnnnnnnnnnnnnnnnnnnnnnnnnnnnnnnnnnnnnnnnnnnnn
nnnnnnnnnnnnnnnnnnnnnnnnnnnnnnnnnnnnnnnnnnnnnnnnnnnnnnnnnnnnnnnnnnnnnnnnnnnnn
nnnnnnnnnnnnnnnnnnnnnnnnnnnnnnnnnnnnnnnnnnnnnnnnnnnnnnnnnnnnnnnnnnnnnnnnnnnnn
nnnnnnnnnggcccttttcatttggggcttattctttggagagaacccggataaaagcctatcctttaat
<212> Type : DNA
<211> Length : 576
      SequenceName : 6755801J1
      SequenceDescription :

Sequence
--------
<213> OrganismName : HUMAN
<400> PreSequenceString :
tgaaaacaataacagcataactgagcaaatcactgatgtggtgaagcaaccagcctttatagctggtattggtggtgcctgctg
ggtaattctgatgggtttagcatatggttgtattggcgaagaaagaagaggaagggactcagtaattatgctgttacgtttca
aagaggagatggaggactaatgagcaatggaagccgtccaggtcttctcaatgctggtgatcccagctatccatggcttgctga
ttcttggccagccacgagcttgccagtaaataatagcaacagtggcccaaatgagattggaaattttggccgtggagatgtgct
gccaccagttccaggccaaggggataaaacagcaacgatgctctcagatggagccatttatagtagcattgacttcactaccaa
aaccagttacaacagttccagccaaataacacaggctaacccatatgccacgacacagatcttgcattccaacagcatacatga
attggctgtcgatct
<212> Type : DNA
<211> Length : 519
      SequenceName : 7157414H1
      SequenceDescription :

Sequence
--------
<213> OrganismName : HUMAN
<400> PreSequenceString :
gagagttataaaggtttacaagaatctcacctcatgatcaaactgggtaagatttgatagaattatctctaaggtttcattaa
aaaagttggggttgacattaatagtagactaacgcaagggtgaaatttggctttctcctcttgcacaggattttcatgtaacat
taaagacagattgtttggaagctaagtcctccctcttaatgagtgaaggtttttgccttgttttaaaatgttttggtcatgact
ttggctaaataaatagtttatagtaatccgtaattgtacttcataatatcaagtgctttgaaccgctaacatatttaacaggc
tcccccaaatc
<212> Type : DNA
<211> Length : 347
      SequenceName : AA663307
      SequenceDescription :

Sequence
--------
<213> OrganismName : HUMAN
<400> PreSequenceString :
ggatttccgggttgcagtgacctatggtttcaccactgctactgcgcctgggcaacaaatgagaccctgtctcaacaaacaaa
gcaaaacaaaacaagggcaactatgccatatgacatcatcaaatcacaggagtgataactcatcaaattcacaggtcctgttct
gtgctgcctaaccaatacttgttattggcagggttttaggggtctgtatactttttgtgttattatttaagaaatcattttcgg
ccaggcgcagtggctcacgcctgtaatcccagcactttgggaggccgaggtggacggatcacaaggtcaggagatcgagaccat

cctggctaacacggtgaaaccccgtctctactaaaatacaaaaattagctgggtgtggtggcgggcacctgtagtcccagctac
tcaggaggctgaggcaggagaatggcgtgaacccaggaggcggacttgnncagtgagcaagatcgcaccactgcactctagcgc
gggngacagagtgagantccgtctcaaaaaaataaaaagaagaaatgatttctcagccttganggtctaaaatatttaactttc
agcctgggccaacatggggagaactcatctctccccgcccccccccaaaaaaaaaggcttgggtgcggtggcccaccctg
ttaatcccagacacttggggaaggacgaggtggggcggatcacctgagtttcaggagtttgtagaccaagcctggccaaaatgg
gtgagaacccgncctctattaaaacataacaaaatttagcc
<212> Type : DNA
<211> Length : 797
      SequenceName : AA663482
      SequenceDescription :

Sequence
--------
<213> OrganismName : HUMAN
<400> PreSequenceString :
tcatcgtcagagtcttttattaagataaatccagtaaaatttgtgtattcacatataatcaaactgcccttctgaaataaac
atttgtatttattttaaaaatgggaatacaatttcatttttcccaagctaataataaagttatatttgggaaagataagtttaa
ctcttaacattacatgaaaatagttcaccttctttataaaagcaacctgcagaacaagaatgttggatgaaactctttttttgg
gatccctcactttatgaaattatcacatgtataaaagagtaaaattaaaattcaaagatttgacatataacttacacaacacga
gattcactcacaaagttgaaagcatatttttttctatgtattccactactataatcacaagatttatagataaatg
<212> Type : DNA
<211> Length : 412
      SequenceName : AA669885
      SequenceDescription :

Sequence
--------
<213> OrganismName : HUMAN
<400> PreSequenceString :
gtcgaccgggatcaggagattgagaccatctctgactaacacgatctgcccgccttggctcccaaagtgctaggattacaggca
caagctaccgcgcccggccctagcaaggccatttttacactttctgcagaaagggtacactccccagcagatttgccatgagag
tacaccgaacaaaggagacaggttcatttataacctgacgcatccaccctactgctgtgtccagtttccactggctggaatgga
acctcacatttgtacttgttttgattggcttagaactttttaaaaggcgtaaaggcagaggagaagaaaggaaggaggaagta
acttgtggaatgctgagaaaggcaaaaacatcttcaaatgtcgac
<212> Type : DNA
<211> Length : 381
      SequenceName : AA713864
      SequenceDescription :

Sequence
--------
<213> OrganismName : HUMAN
<400> PreSequenceString :
gaggacaaattattcaactgtttatcatacataagaccgcatgactataatacaaagggggttctttttttcatttaacgttac
aatatacacagcaagtccggactagatcttacaatctgaacacaggtatttaacctttttccatgctgtttatgtttacaatgca
cagaaaaaaaattgacctagtcagccacatggaattgaccaatagaatgaaacgcggtgttctgctgagtcatcttaaacaggt
tatcggcagaggtagattactgcttcttttctaggcttcttttgatcctggacaggtaaattacttatcttcattctc
<212> Type : DNA
<211> Length : 330
      SequenceName : AA729988
      SequenceDescription :

Sequence
--------
<213> OrganismName : HUMAN
<400> PreSequenceString :
gacgaagcaaggaaggtgctgtgtaatcattaaggagcggaggcttttggagctgctaaaatgccggattacctcggtgccgat
cagcggaagaccaaagaggatgagaaggacgacaagcccatccgagctctggatgaggggatattgccttgttgaaaacttat
ggtcagagcacttactctaggcagatcaagcaagttgaagatgacattcagcaacttctcaagaaaattaatgagctcactggt
attaaagaatctgacactggcctggccccaccagcactctgggatttggctgcagataagcagacactccagagtgaacagcct
ttacaggttgccaggtgtacaaagataatcaatgctgattcggaggacccaaaatacattatcaacgtaaagcagtttgccaag
tatgtggtggaccttagtgatcaggtggcacctactgacattgaagaagggatgagagtgggcgtggatagaaataaatatcaa
attcacattccattgcctgctaagattgacccaacagttaccatgatgcaggtggaagagaaaactgatgtcacatacagtgat

gttggtggctgtaaggaacagattgagtaactgcgagaagtagttgaaaccccattactcatccagagaggttttgtgaacttg
gcattggagctcccaagggcgtgctgctctttggtacagccggtacaggcaggaaactctgtggcgcgggcggttgctaatcgg
acgatggctgcttcattcgaagtatgggattctgagcttgtaccgaaatacgtccggtgagggggccgaattggttctgaatc
ctcttgaatggccaaaacaaaaaggcccgcctaatttccttggtcgaaattgtgc
<212> Type : DNA
<211> Length : 896
      SequenceName : AA736561
      SequenceDescription :

Sequence
--------
<213> OrganismName : HUMAN
<400> PreSequenceString :
tttttttttttttttttttttttgttgtagggatcctaatgtcttttatcatataaatattacacaaagaaaacttacattag
gtatcaaaaaactttacaatctgtacatttgtttttttcttgcatttctatattatacattttgagacagaaattcacaacggt
cagtaaaaaggtaagtcagcaaagaaaatgagaagtgaccctgctgttaatacaacaagatttgtccttgttttggcagataaa
tacctggccaaactaaagtcgtgtgagatgcagaaatttacactaggcttgataagccaaagcctttttttttttttccagat
ttctgtgatcccactagccccatgttgctcaacacaaatactattaacttgtatactatacctctttg
<212> Type : DNA
<211> Length : 404
      SequenceName : AA742351
      SequenceDescription :

Sequence
--------
<213> OrganismName : HUMAN
<400> PreSequenceString :
tttgtgaattgagaggtctgctactttattttgataatgcagggatattatttatctttgcagaatcaggtgactcccaacgtt
cccggaatcttctagtggtctgtgtcaggggtctgggctggctggggttcagtgatgtctactggaggcagcttccatgccttc
tggggtcctgagtctccatggcttgtggggtctgggtccccccctggattagtggatggccagagtggcatagacactgggctca
gctggagaggccccttcctgggatggaggaggctcagttgccttctgtctgagggtaaagctgtgcagccgggcgtaggtcaca
tcctgggggggcttcagatgcagcagcctcagtgtccatctgtctgtcctcttctgcctgtctgtcctttgtgtccaggaattcc
ccagacagtggggagggaggagaggccatttctctcctaggtct
<212> Type : DNA
<211> Length : 464
      SequenceName : AA745989
      SequenceDescription :

Sequence
--------
<213> OrganismName : HUMAN
<400> PreSequenceString :
ttttttttatattattgtaaatgaaactgttttctggatttctttttcagatacttcactattactatatagaaatgcagttg
attatttttaccttgcaagtttactgaatttatcagttctaataggttttttttggtagcatctttaggattttctatatataa
gatcatgtcgtctgcaaacagggaaatttgactttctcctttttaatttggatgccttttatttctttctcttgtctaattgct
ctggctaggactatgttgtccttcaacattacgttacatagaagtggtaaaaagtgggcatccctgtctttttcctgatcttag
agggaaagctttcagcttttccccattcagtatgacgttagctgtggatttgtcatatatggcctttttttgtgctgaggtata
ttccttttatatataattcattgagagtttttatcatgaaggaatgttgagttttgtcaaatgaatt
<212> Type : DNA
<211> Length : 487
      SequenceName : AA767859
      SequenceDescription :

Sequence
--------
<213> OrganismName : HUMAN
<400> PreSequenceString :
agatggagtcttgctctgttgcccaggctggagctcagtggcacaatcttagctcacttcaatcttcatcttttgggttcaagt
gattctcttgcctcagtctcccgagtagatgggattacagttgtgcgccgccatgcttggctaattttggtatttgtaatagag
acagggtttcgccatgttggccaggctgatcttgaactctcgacctcaggtgatccaccacctcggcttctcaaagtgctggga
ttacaggcgtgagcatgatgcctggctggaaagtcacttcttatctttgagcctaaagacaaatttcttgtgtgtaaaaccgg
atcataatatctgcatcctgggtggctagtgggaataaaatgagatcaggcaaggatctgcttccgggcaggtgctcaggaaat
gacagctgttatgattaatgagcatagcagaggacagagggtcacgt

<212> Type : DNA
<211> Length : 467
      SequenceName : AA861429
      SequenceDescription :

Sequence
--------
<213> OrganismName : HUMAN
<400> PreSequenceString :
taacaaaaacaactctgtattctcgtccccaccattaccatctctcctttgtgatgtcaaagagaccagaggaagtggacagac
tcggggaagaataggtaagtattagagacagaaaaagtatgaagtcccttctgtgaggactaacaatagtgatgccattttaca
accttgatgactgtgttctcaagaaacaaagcaaatgtcaaccccgtccatagcttcaacagagcataacagctctaaccgcaa
cagagcctaacggccctaaccacatctgtccccagttggaaagtgtccaaacagttccaactagccagcacttccccaacccct
ccccaacagaaactttattataaaagcctcagcttgtaagcgatcaggatctcagccagattcctaactggaactcttcgcagg
cttttcctgtaaataaacctgtttggccgtggaattgcctcctgttactcgctctctacgtttatatcttcctaacaccttccg
gctctaatg
<212> Type : DNA
<211> Length : 513
      SequenceName : AA873257
      SequenceDescription :

Sequence
--------
<213> OrganismName : HUMAN
<400> PreSequenceString :
tttttttggtattttcagttagattctttcttcttagaggtacagagaaagggaggaaaatagctacagacatggtgtaagta
ggtcacagcagtttcaatgcaccaaaaaatgtgacatctccatccagtgatatttgtgcatttctcttggtattgcaagttgg
agttcatctccttcttccagttttgcaatgccagctgaatagcaggaattattgggtagtgtttcaggcatattttgaatacat
cgaaacaaagtcaccagactcaattcatccccaaagacatggaccttcttcctctgaattagatgtcccatggcgtaggtctta
tcagtatataaaacctgaccatatataaaaaagtaaccagtt
<212> Type : DNA
<211> Length : 378
      SequenceName : AA906714
      SequenceDescription :

Sequence
--------
<213> OrganismName : HUMAN
<400> PreSequenceString :
aacattattaatcattagcttttaatattactctttgttgcattactaatataacctaggaataaccagtgggtatagggtcag
gtgctgaagggacattgtgagaagtgacctagaaggcaagaggtgagccttctgtcacgcctgcataagggccgcttgagggct
ccttggtcaagcggtaacccagtgtctgggaaggcaccggttacttagcagactgcgagagggagtctcctttccttggaggag
tcagggaaaactctgctccagcagcttcttgtgggaggctggatatttatcaggcctgcgcgcagtcaatccggaggcctaaaa
ccccttccctgtg
<212> Type : DNA
<211> Length : 349
      SequenceName : AA954331
      SequenceDescription :

Sequence
--------
<213> OrganismName : HUMAN
<400> PreSequenceString :
ggtgggtgagcagcatgggctttgaggagctgctggagcaggtgggcggctttgggcccttccaactgcggaatgtggcactgc
tggccctgccccgagtgctgctaccactgcacttcctcctgcccatcttcctggctgccgtgcctgcccaccgatgtgccctgc
cgggtgcccctgccaacttcagccatcaggatgtgtggctggaggcccatcttccccgggagcctgatggcacgctcagctcct
gcctccgctttgcctatccccaggctctccccaacaccacgttgggggaagaaaggcagagccgtggggagctggaggatgaac
ctgccacagtgccctgctctcagggctgggagtacgaccactcagaattctcctctaccattgcaactgagtcccaggtcggta
tttacataatccatctg
<212> Type : DNA
<211> Length : 437
      SequenceName : AI016020
      SequenceDescription :

Sequence
--------
<213> OrganismName : HUMAN
<400> PreSequenceString :
ttatttgggacaagaaactaaatgccttttcagtggttcatttgaatctaccatacttcaaaaattataaatctaccatcacca
aagaagtacagcgccatttgtattagttgcaaaattaggctatttacttttctgtatgtacttttgtgaggtttaaaacgttag
gcttattacttctccttggtatgagagaaaaagcacaggcactggaaagaaaaaggaataggtgtgaaatcttgattttgtgaa
ttaggatgaatgtgaccttatgtaaattacaaagttcctgaatctgtttgcgcagatgaaaatggaaatatactaactgcacct
accaaacacggctgactgaggattaaataaaacaatacatgtaaaggtagtgcctggtgcacagtagaaactccaggccgggcg
cgatggctcacggctgtgtgttatcttctacgccagtttttctgattctcagtttatagatgggaaaggagacgcttccagcag
cag
<212> Type : DNA
<211> Length : 507
      SequenceName : AI051962
      SequenceDescription :

Sequence
--------
<213> OrganismName : HUMAN
<400> PreSequenceString :
ttttttttctgagtgacaatccactttaataatccagcttcagctcagctgagaacttcccctctcaggtgcaaagggatggca
gagaagtctttccaagagggctcaatccactaagagattatggcttagagaagggaacagctcaaagaagcccttgaagagggt
gagggtctggaggactcctgtggtgcaggccatctcccggatagagtgcatggccagttggggggctgcctaaatccagcacccg
cagccccagccgagaagccaagataggtccaatggtggttccacaggggggtgtcattccggaccatgagatcctgcaggggggac
cttgactttgttggccacctctcggatcagggcctctgacaccgcgtttgaagcatagcgttgcttgctgttcaccttgatcac
ggngcccttgtggaataaaggccggtggttctcctcatgcttgtccaggtagttgggatgcacagcatgggccatgtctgcgct
gatcatgaaggacttgngtatggcttcctcgaaggctgtcgggtgctggcacgaggctgagatccgccgcagcaccagctctgt
cagcagtgactgtgctccctgtgcactctcagaccccacctcttcgttgtcatagagtgtgaccatgcgcacgtgaggctctgt
ggccaggga
<212> Type : DNA
<211> Length : 681
      SequenceName : AI097079
      SequenceDescription :

Sequence
--------
<213> OrganismName : HUMAN
<400> PreSequenceString :
gcttgtaattgctctatttattaatacatatatcacaaatgttcacaatatcaatgcattcttgttggcatgctagacagaggc
attacttttgaagatcttttaaaaatatttgacttgttccccttcacactcatttttaaattgtttggatgaggtcctcaat
gtctataatgtcaatgcaagaaaaaatacagagagaagtagtaaaataacacaatagcaattaattggggaaaaacaaaatatc
atggccttcacaagaagcaacaacacagctttgtaacagggttatacaggtttcaaccgagaggcaaagatgcactaaactgcc
tacacatacccaggtatgtaacaagcaaggaggccacctcaagttcagttttccttcttgcatgacacatgctttttttctcct
catggacaattattgaagtgtttcgttacatgtcttaggcagcctgaagaaggtaggaaagccttagtagagggaaaatcaaac
caaatgagattaagtcaaaagaacagataactatcgtctgcttatctacttaaaggtgtgtggcatttaccacagtgaggagct
aacaggagcct
<212> Type : DNA
<211> Length : 599
      SequenceName : AI147046
      SequenceDescription :

Sequence
--------
<213> OrganismName : HUMAN
<400> PreSequenceString :
gcggccgcccctgcatggggaaaggggccacaggaaggccctgcgagtgcagctggggccaacaggaacagcaggacaccctcc
agaggtggccccagcctcctctgcccagcccttcctgaactctccatcatccctgtctccagtcactcagtcagcatggctgtg
atggacactggacccacagggctgaaaagatccagagaacacacagaggaataggccaccttacacaccgatggggcccctcctc
tgggaagggagcactttgtgcacagaagtttgagggggaggagctgcgtgtggtggctcatgcctgtaaacccagcac
<212> Type : DNA
<211> Length : 330
      SequenceName : AI220935

SequenceDescription :

Sequence
--------
<213> OrganismName : HUMAN
<400> PreSequenceString :
tttttcacctttatcacaaagtttttattgaacaaaaataatatgacagtaaaaatgatacataaagtgaagatttgcagtca
tagaaaagatgtgtacaaagagaccaacattcaggttatatagttctataaacttttcccatagcagtttaacactgattaag
cagacataaaagggaaaagaaagtcattaatgttctttgaaacctattttctgagtaaaatacctctgctaacacacacatgca
cacacacacagaatgaggcagagctatatgactcagggatgaggtcaggacccgtgtggctcagaaaattctgcgttagctgcc
gcccacatgccttccgcttgtcatccggggtcacttcatactcggccaccccatccaggaaggcgacgcagcggctcagctccg
gcctcgtggcacagaactctcggaacagcaggcgcccaatggctgccgctcgcacaggctgtgatagtcacgctc
<212> Type : DNA
<211> Length : 496
      SequenceName : AI278029
      SequenceDescription :

Sequence
--------
<213> OrganismName : HUMAN
<400> PreSequenceString :
ttaagatttcacaaaaaaagtgataaattttctggggcctacagaagggtaaaggtagaagtaacacaagatgaatcaaggata
aaaggactccagtggtccaggaagtcaccgcgagggtgatgcagactctccaggtcatgatggccaaatatcggagggggtggc
agatggccacgtacagatcataggacatcaccaccaggaggagacattctgtgacagcaaaagtggaaaacagaaaggtctgca
tcatgcggcccgcaaaggagatgggcttggctggatgcaggaggttcaccagcatccggggcaccgtgttgcaggcgtaggcga
tgtcgacgaccgccaggtgtgagaggaagaagtacatgggggcgtgcagtctggagtccagtgagatgagccccagtatggtcc
cgttccccagcagggtgaagacgtagaacagggaga
<212> Type : DNA
<211> Length : 456
      SequenceName : AI339565
      SequenceDescription :

Sequence
--------
<213> OrganismName : HUMAN
<400> PreSequenceString :
tttcttttcttttcttttcggtctctccctttctttatgggcttttcctaaacactttcttcttttcttatgacctggatctca
gtttcactatgcacttccctaacctatttctcctaatttcagttaaggtaaaaagtccacaggtatcagaactggaacctgaag
ttaggttaaaatgcggtgacaaaagaagcagacttcattatgttcttaagagttcaggctcttaagatttattacccctttctt
ttgtcctctcctggagaatagaatgagccaagatcagtccttacttgagcaaatcactccagcatcctcagcatgatcacagtt
atgctttccccatccttgatgtttgcagttccagagagctgactcattttcggtggcatataaaatcatcaacccagattggtc
cagagccttctccaaaattagatgaaccagagaaactgacagcacttccacattcaagttgtctacaaatgacagatgcatgat
ctatgttgaagttatcatcacacactgttccccaccgtccttggaatttgatctctattcttccagaacacatattccctccac
gcgtcagcctcatttccaaattggatcc
<212> Type : DNA
<211> Length : 616
      SequenceName : AI341167
      SequenceDescription :

Sequence
--------
<213> OrganismName : HUMAN
<400> PreSequenceString :
atcagcaataacaaagtcttgaggaattttgtccttttatccatataatttagccttcatcgtttggcatacaggtagtgacc
tgcatatcctggcagtttttgaaatgatagtgactgttctaggcctccgctgatggcatagtcaaacaccagatttccttggag
atgattcggaaatgcctgctggaagattaatgaagactccattctgtgtttcataacagtagcgttgttttctccatcaaaaac
agagggtcacagaaatgtgctgaaaagatgtggtccagcaaggttctgtagtcccacctcagtgcctgtgggctgtgtttttca
ctgtaacaagcactttggtaaatcactccatggctntcctcatcctcctctttttttcttcttggtgggaagctggatngcgg
gcatgcatcaatctgtaatctcagctactcgggag
<212> Type : DNA
<211> Length : 455
      SequenceName : AI366098
      SequenceDescription :

Sequence
--------
<213> OrganismName : HUMAN
<400> PreSequenceString :
gagaggatttctttatttcgcgttcagacccaacacactcggacaccttggggccctgtgagggctaagcagggtggtggcgtc
agctcccggggaggccccactccctggggaggaaatacacggcaggaggccgcacccagcccccccacggagggacccgtgttg
ctctaacagggacactgaagttgcctctgccgccccgtgaggggcctgtggcggccccagacccagcccagccaggccagagga
gcctcccaggggccctcaggtggtgaggtgggggcttcccggccccggtgcccaccggcccttctagctgcctgtgctgggcca
ggtgtcctctgaggccggcaggagtctggcccccgctgcacctgcgcttggtggctggcaggggcggngtagggaggtcacccct
cgaggccggggtccccatgtgctaggggaagacctgccttcccagccagtgtccacagggcagcaattccacgctctgaggctc
agggaccaaggca
<212> Type : DNA
<211> Length : 517
      SequenceName : AI401546
      SequenceDescription :

Sequence
--------
<213> OrganismName : HUMAN
<400> PreSequenceString :
ctggaagtaatgtctgtttattgatagaaaacaggccacgtccagagaggcccagggccaaaacctccttctggaaggccgaag
tcccacagtgccggggagcgcaggagtcagctcggctccgtcttgccagggctgggggggaccctcatcctcacccgcttccgga
gctgcagtggatgtgggggcccctgcagcctccagaaccagttccagggccccctcgcccgccacacggtgcacctgctcgcct
ctggccgtcaggatctcttcgatatttctcttcccattcaggtccgagaaccagcccaggttgtcagcgatgatatggtggttc
tggcagccggggcaggtcacaatgaccacgccttggtgataggccagcttggagatgcgcttggaggacctagtcccgcagacc
ttgcaggtgtagacgagctggtagtgcgccg
<212> Type : DNA
<211> Length : 451
      SequenceName : AI435052
      SequenceDescription :

Sequence
--------
<213> OrganismName : HUMAN
<400> PreSequenceString :
tttgagatggggtctcgctctgtagcccaggcgcgacctcggctcactgcaagctctgcctcccgggctcatgccattctcctg
catcagcctcccgagtagctgggattacaggcgcccgccaccacgcctggctaattttttgtatttttagtagagatgggtttt
caccgtgttagccaggatggtctcaatctcctgacctcatgatccacccacctcggcctctcaaagtgctaggattaaaggcgt
gagccactgcgcccagccacaaaagatctgcttttaacaaaaatttgtaatgggttataaaaagtttatgtgaatcttacctta
tggtcaagcattaaaattgggtagatatgtctataaggctttcttaagaattgggtgtcacataaatagtacactaattccaag
gtaaaatttggcttatatggtataaaagtcatacaggaag
<212> Type : DNA
<211> Length : 460
      SequenceName : AI440504
      SequenceDescription :

Sequence
--------
<213> OrganismName : HUMAN
<400> PreSequenceString :
tcgcgtttctcattgtcttgagattctgggaggaagcctctggggcttcacagaggggctccctaggggtaagtgcaggaccct
ttgcagagctaccaggaaagagggctgatcacacctcaggcagccgggttacaatccgcataaaaatctgagtctggggagcgt
gcgacagaggcaggcagattgtttaaggcgttcgataaagtcggttgatgacagacacagatgtgtgttcccagccgcatttgt
gctctggtgtgtgacaggtctgtccttgcctgctttcagctcccagggcccctttgagtctgggcagcccagtcagtccccgtc
catttttggccttagcttttccttccctggctacatctgggcccagaacaagtctcc
<212> Type : DNA
<211> Length : 393
      SequenceName : AI458182
      SequenceDescription :

Sequence
--------

<213> OrganismName : HUMAN
<400> PreSequenceString :
aatttagaaacaaatatatattttgatagaaactgtaagtaaatattcagtactaccagaaatatactatcttcaactctcg
gctttttccaaaagatacagaatcatttttggaaaagatacaagatgtacagacaaatcacattcataccttacaatttaaa
ttcataatgaacaatgaatattttcatattttccagaagtttgaaatttcaaagttgttagcaaaattattcatttccataatt
tttacgagtaaaaatagaaagagctgatcatgtaccttaatattgtcactttatatattactcaaaagtacaaataaccctaaa
agtagaatcccaggcttatcatattataaaaatactgagacatttatcaaacataactaatataaatgaaactacatttttaaa
aatgtttcacaaacaccagtccatctttct
<212> Type : DNA
<211> Length : 450
      SequenceName : AI522316
      SequenceDescription :

Sequence
--------
<213> OrganismName : HUMAN
<400> PreSequenceString :
ttacattttctcagcaaggcaatttacttctatacaagagtgcgtctcatggatggtgcaatggcgagagcacaccggaacaa
gggaggggaatgggttcttatccctgatgcaggtacccctactgctgtgttgttcccctatgggctagggttggaccgcacaa
tctaagctaattctgactggctattttaaagagagcaggggtataagccagagtggtggggtgagaagtttagacaggaaagat
ggttacggaccaggtgactaaaggtgactcaggtcagagcaggtgacagaggctaggaggggattgtttactgaaactaggggc
aaggagacataaagaatgaggaaattaaaagttaaaatgaagaacaaagaacaggtgagctgaacatactgatacattggttct
ttggagaggatctcagaactcaatgtacttaacaaattacaggctaaaacctttgaagaagaatttatgatatcctacattatc
atggggtaaacaattcgtccctgacagaataaggctact
<212> Type : DNA
<211> Length : 543
      SequenceName : AI553823
      SequenceDescription :

Sequence
--------
<213> OrganismName : HUMAN
<400> PreSequenceString :
cggtggtgacgctcagggtgaccgcagtgaagaggtaaatgactcggagctgtcccccagggccctcccgaagcccgttttat
cccagtggattccgccgaccacgtatccaaagcctcctccgagacctgccaggagggcgtggatgttcaggcctcggtcctggt
ctgcggggctgcacacgtccatcatgtaggcgtggctggggttgtccgccgagtcggcgctaaagtccatcagcaccacaccgc
acacggtcagcagcaggccccacttgtggttcccggtcacgtcagccagggcgatgccaatgtcccggccattcagcaagagcg
agaggcccagcagtgcccctatagccaggacaagaatgaaagggcgtctccttccaaaccttgaggtacaccggtcactccaag
cacccaacagaggctgcagtaggaatccgaggatngggctgatgaaccacac
<212> Type : DNA
<211> Length : 472
      SequenceName : AI567491
      SequenceDescription :

Sequence
--------
<213> OrganismName : HUMAN
<400> PreSequenceString :
cttcttttactggcttccggccgtctcccaactcgaaccgctcccgcggcggccgaggcgggctgcgactccagtggctggctt
tcactttgtcgctgtagtcctctctcatcgttcggtgatgtgcagacacttccctgcgggcctcccgctccttgcgccgctcct
ccgcccgccgctcgcgctccttctgctcccgctgctccttctgctgctcccgcatcttgcgctcccgctcccgcttcctttcta
actgctccaagcggtccctttctctcctggaatgctcccttgccatctcccttctcttctgtctttcccattcccggcgagctt
tatcctgttcttctcgatgccgtttccgacgttcgtgctctctttctttttctttcactctagcatgcttccccccttctgctg
aatggctacgatgcctacgtttctcttttctcttttcatcttttctgtgatgagctttttctttccgagacatttgcctggggt
gtttgatggccaaagaatcatcttcttctcctctgtcttccatagagtcttctcttctatacggggagttccttattggatctc
ccat
<212> Type : DNA
<211> Length : 592
      SequenceName : AI580044
      SequenceDescription :

Sequence
--------

<213> OrganismName : HUMAN
<400> PreSequenceString :
acttgattataggcagccttgataacatctccagcagctgtggcctgggagatagtataaatcccaaagagtccagaatctgag
taactggcattaaatgcagaaacatcaaatggctgctgagttgccttggcaacagcctggtgcagatggctggtggtgttgctg
cccctcttgacatgtggcccagcaccgaggacatgctgaagaacactaaattcatttgcctctgcacttcccgcgacagcactt
tctgctacaaaagcagcatggacaagactgtctccattctgttctcggatttcacctccacggtagttggcctttgcaccagat
aaaccaagcccacccctcatgttgagaaactgttcagcaacttgctttagaacaggatgactcacaccaagtccaatcaaagcc
attcttgcacttgtgaaatggttctgaacgaagtaatgtaactcctctgatgtcacttttccaatcctatagtcaggacaatac
aagggattagccaaggcattccggtaagctgctgcatgcaaattttcaatgacatgagtctgcggattctgaaaggccacagct
ntgtcaatctttagctgaggctgaagtcagctacttcccacgacgaaattctggtgctgtg
<212> Type : DNA
<211> Length : 649
SequenceName : AI590075
SequenceDescription :

Sequence
--------
<213> OrganismName : HUMAN
<400> PreSequenceString :
ttttttttttttttttttttggagaaggaagggctttattcagctgggagcatcagcaggctactgccttaaaatccgagct
ccccgaatgcacaatttccgtcccttttaagggctcacaatactaaagatttcacatgaaagggtcatgattgatttgagcaag
caggcggtatgtgacaggggctgcatgcaccggtggtcagagagaaacagaacagggcagggagtttcacaatgttcttctata
caatgtctggaatctatgaataacatcagtttctaagttatgagttgattttaactactgggtttaggccaggcaggcccagg
ccggacgcgtgg
<212> Type : DNA
<211> Length : 348
SequenceName : AI625585
SequenceDescription :

Sequence
--------
<213> OrganismName : HUMAN
<400> PreSequenceString :
acatttgaaaatttaatggcaattacacacaaatttaatttaaaataatattaaggacttaatctgatttacagaaatgaaa
tgcaatgaagagattaagggctaacctccattactataaaattctctggctaccatctagcacaggagcaactggattgctttc
agcagtcttgaagcactaaaatatcttaaaattcatatggagaaatagtatgtatctctacattaaatttagatttgatattta
ttctctgacagatcagacccagaatattaattttatgtaattgaaccaaaaacatttagcaatccttcatttagtaatctttt
gtataaatataaaaatctctgataagtgaaatgtttttcaaattatacttctatttttcacagctttataggtaattataatt
ttcaaactttaaatatttcatgtgatttgatatatctgtacagaaagagttagataaaaacatttaataagattaaatctaatt
tgcaaaaattggtatctgacatttgttgtgtgctcttgcaaagagtgcataggacatttctgcagcaatca
<212> Type : DNA
<211> Length : 575
SequenceName : AI652076
SequenceDescription :

Sequence
--------
<213> OrganismName : HUMAN
<400> PreSequenceString :
gacaaagaaatttattacccctgaggcacaaaacaattaaacataattataattattcctaataatgtatactatgttacatta
gaattataggagtttcacataattttggaacatgtactgataacactttaatcaaaagcagattaatactttaataaaactatg
ttgtgctttaattccagtgtttatgaataaactgaataaaagtcctttaattttgggtaattttcacacatagaatttcttta
taggattaattttcacaaaccttccacaacttgctcaaaccttcagctttatcctatctaacataaaacaatcctttaaccct
ccaaactaggcaaaacttacattcccataccttcttacaatcttttaacaaaagcacattctactttncctgcgtaccttacat
gtaagactgcttttccagtagtctcaagaacatgttacatggctaactgttcacaacttttacttttgcgaaaaacctg
<212> Type : DNA
<211> Length : 499
SequenceName : AI671268
SequenceDescription :

Sequence
--------
<213> OrganismName : HUMAN

<400> PreSequenceString :
tttttaaaattaaactgtattgtttcctggaatgtaaatttagctttgcttaatagtaaaataagtgagccatcttgaacactt
taagtttatactatataatttcttaatgaacattggcaaatagagtagctaagagattgacaagcacattacgtttagataagc
atgtgatgcagaagttgattcaagcaagtgaatccctgacattttgaggatctcacattagacaccaacaaattaaagctccaa
aattatttatttttatgtgttcctcactttagaaacgtctactgcatattactggatatttgtatactaatacacttacctat
tttacattgtgttttaaatttagttaaaacttaaccgtagaagtctgttcaaacgcgaaaggtcttgctttgtttgttttaaat
acatttattttattagaaaggatgatatttcattgnggaatagtttctttcatttgggggctatgtgtttcaataacattcact
tggaatcagctganagctgtaatatgtctttgaaatgagccatgat
<212> Type : DNA
<211> Length : 550
      SequenceName : AI693066
      SequenceDescription :

Sequence
--------
<213> OrganismName : HUMAN
<400> PreSequenceString :
caaactgtaagtccggtctctatatatctttacttcaacctgattggtcctgggccaaggttccgggccaagctaagtcacact
ttctccaaaatagcccacaaactaagcacattccttccccttcccagtccataaaaaccccaaacccctgcctcataggggaa
ttccatttgggtcccctctctgctggcaaaagctttcttcttttgcttattaaaccttcactctaacctcacatttctgtct
gcactcattaatcttcttaaaaataaaacaaaaaactccaggtgttatctcaaacaacagaaaaactgttacatcttggtgcat
tactaaaattacatttctaaccaatgtcttttttttttgagacagtctc
<212> Type : DNA
<211> Length : 385
      SequenceName : AI698737
      SequenceDescription :

Sequence
--------
<213> OrganismName : HUMAN
<400> PreSequenceString :
atacggagtctggctttgccacccaggctggagtgcagtggcacgatctctgctgacggcaagctccgcctcctgggttcaggc
ccttctcctgcctcagcctcccgagtagctgggacaggcgcccactagcacgcccggctaatttttgtattttagtatagac
ggggtttcaccgtgttagccaggatggtcttgatctcctgacctcgtgatccgcccgccttagcctcccaaagtgctggatta
caggtgtgagccaccgcacctggccaaagttatttaatactatacaaattgctgtttaaattttcggatatctctttaaacttt
tatttttcaagttttcttggtgaaattagagaatatggtctctaaaattttggcattatggacaaagcgaccatttaagaactt
tcactttctttattggctttctgcatttccagag
<212> Type : DNA
<211> Length : 455
      SequenceName : AI709066
      SequenceDescription :

Sequence
--------
<213> OrganismName : HUMAN
<400> PreSequenceString :
ttactggtgaatcttttatttggacaagacaaaaacatctccacactgttccctttatacagaaaatggaacatctcaaatat
attagttttctctttttcaacagaattcatttcaatctggtcccaggaactgcttctcatttaggattcacgttttagtaac
acatatgcacccattacagccaaaagagcatacttgaattttggatagtcattcattattatggtgaccataccaacatatggt
aaaaaccctcttgctcttcccaccacgtccttcttttccagccagttctggccttctttgtacaagcctctatcatcaacttca
ttattatctcctttagtcagaaatttgatgtctccattatctttttcatgaactttgattactctgtgaactattggaatgtct
cgtccttcaactttaaaaacaactatttcaccagctctgattgggtcttcccggaaatttgtgaggaacaggaggtctcctctg
tgaaaggccggctccatactgccactcagcaccaccacgatggggctctcactgcctgtgagcacgatcaagcctttccatatc
atgagtgcagaagacacgatcatggcgaagtttaaaacctggtaatagagctggcgcttgttcatcttcttcangtccnctgaa
gatatcccagtccccgacgcggngagatgagcctccacggcgcccgcacgcac
<212> Type : DNA
<211> Length : 725
      SequenceName : AI740538
      SequenceDescription :

Sequence
--------
<213> OrganismName : HUMAN

<400> PreSequenceString :
ttttgattgatttaacataaatactatggtttatttctagatggcaaggaaaatcacgtgtaagaggtttccatttaaaagaat
tccactattcatcaaaattttcatctattctcaagactgttgatttcaaactaggtttagagtagcaaagcatatgaattttac
agtagaagacatgagaaggatataaaagtttgttttctgtattctttcaaaattcttatttgaaggccttctgggcatataag
ctattggaacttgtcatcacttaaactatttcctataaagatgaaaatcagctaataaaagtatttttagaaattaggctga
aaacctctaagtttggtaaaagacataaaactgcaaatttatgaattggagcaaattccaaagggaataaaacaaggaaactca
tccccaacataatgaaatggctaaaaactaaagataaagaaaaataccttgaaatctgacagaaaaatgacacactacatacag
ggaaacaacaatttgaatgactgcagatttctcatcaggaaat
<212> Type : DNA
<211> Length : 547
      SequenceName : AI743175
      SequenceDescription :

Sequence
--------
<213> OrganismName : HUMAN
<400> PreSequenceString :
tttttttgaatagtttaatagttattatttaagtaaggctttggcgtgcttttccacattttaggtttttttctctatgtgta
caccttgcaagatgtatcattaactcatgaaaccaccagctcctcaggcaacgaaaagggaggtagaaagaacgccagaaaggt
gggtctgtgtgttctggggtgcgtggggcccgcccccgaggaaggtgccaaccgtccctggagagcagaatcaccaggcctggg
aaagccatgcgggttcgagagcctgcgccatggccatgctgggcagggcccaaggggtgcccaggcgtctgcccactgagaaca
ctgttagtgcggagggatccacaggaggggagagcggcgccagggtcccggacacaggcaagggtctctagcccgccacccacc
cactgccctggggggtctctgtcaggcttctaggagacacagagactctgaggtcatgtctcgagggtggc
<212> Type : DNA
<211> Length : 490
      SequenceName : AI808824
      SequenceDescription :

Sequence
--------
<213> OrganismName : HUMAN
<400> PreSequenceString :
aaaattttaaatctttaatttccgttttcacgtattttcttcttgcttccaaaaggaaaggagtgcgtagctctgttgcctgta
catcgtccacagcccctgggtcggggcggggtcccctgggccgcccggggggtccacatgcagcccctgggtgggggccggcgc
ggggtgaggtccgggggccgccttattgctgaggtccggccggttggggccgccgctaggcgcgctggctgggcagctcctggg
agatgaagcgacgcaggcgctccaggtactggctgtagagctcgatgtcgttgtgcccggcgccctccacccacagcggctcca
ccgccttggggcagcgctcgtagagcgccagcccgtgcgagaagtcgatcacctcgtcctccgtgccgtggatgatgagcacgg
gagacgtgatcttggacaccttctcgatgttagggaaggcgtcgaagcagtaggtcttcttggtgtcggggaaggcgacgcgca
tgcccgaggtgagcggcgagtgcagcaccaccgcggcacactcgtagcgcgaggccaggtccacggtgggcaccgtgccgatgc
tctgcccgtacaggatggatgctgtccggctgatgccgtaccctgtgcgcanggcctgccaangcggcgtcatgtcngccatag
agttccctctcgaaggcctgcccgagctggcaccgtaagccggagtagtcgtaggaagagangtgcagtggaggcgggagccca
ngccaatgtagaagctgctcatcctgggcangttccacggccatgccgtgcgagaa
<212> Type : DNA
<211> Length : 812
      SequenceName : AI810687
      SequenceDescription :

Sequence
--------
<213> OrganismName : HUMAN
<400> PreSequenceString :
acggatattgtttcacgatgatagaagaggatgactctgggttgcctgtggtcacttctggttgcctaggactagaaggctcag
attttcagtgtcgggacactcccattcctcatcaaagaagatcaattgaatgctgcacagaaaggaacgaatgtaataaagacc
tacaccctacactgcctccattgaaaaacagagattctgttgatggacctatacaccacagggctttacttatatctgtgactg
tctgtagtttgctcttggtccttatcatattattttgttacttccggtataaaagacaagaaaccagacctcgatacagcattg
ggttagaacaggatgaaacttacattcctcctggagaatccctgagagacttaattgagcagtctcagagctcaggaagtggat
caggcctccctctgctggtccaaaggactatagctaagcagattcagatggtgaaacagattgcaaaaggtcgctatggtgaag
tttggatgggaaagtggcgtggcgaacaggtagctgtgaaagtgttcttcaccacagaggaagccagctggttcagagagacag
acatatatcagacagtgtcgatgaggcatgaagacattctgcgttcattgctgcagatatcaaaggtacagggttcctgg
<212> Type : DNA
<211> Length : 668
      SequenceName : AI821897
      SequenceDescription :

Sequence
--------
<213> OrganismName : HUMAN
<400> PreSequenceString :
gcacgagtctgatggtgtgtttgctggttttaacttaaaagaaaagatatttatttcttttgcatggcttccaaaggccacag
ttcaggctgcaataggatctgtggctttggacacagcaaggtcacatggagagaaacaattagaagactatggaatggatgtgt
tgacagtggcatttttgtccatcctcatcacagccccaattggaagtctgcttattggtttactgggccccaggcttctgcaga
aagttgaacatcaaaataaagatgaagaagttcaaggagagacttctgtgcaagtttagaggaagcgcggattctattactgga
aactttgggactgaaaggccaaagcttctgggcccaccatcaacgcagctccgctttcatttctttcacatacaactttccaca
taagatttcatgcggaaaaaaaaaaaaaaactcacaaaggttttatactgaaaaaaaaaaaaaaaaaaaaaactcgaggggggg
cccggtccca
<212> Type : DNA
<211> Length : 514
      SequenceName : AW023438
      SequenceDescription :

Sequence
--------
<213> OrganismName : HUMAN
<400> PreSequenceString :
gcgtcgacccaccccaaatgggtcctcactgccgcacactgtctaaaggaggggctcaaagtttacctaggcaagcacgcccta
gggcgtgtggaagctggtgagcaggtgagggaagttgtccactctatcccccaccctgaataccggagaagccccacccacctg
aaccacgaccatgacatcatgcttctggagctgcagtccccggtccagctcacaggctacatccaaaccctgcccctttcccac
aacaaccgcctaacccctggcaccacctgtcgggtgtctggctggggcaccaccaccagcccccaggtgaattaccccaaaact
ctacaatgtgccaacatccaacttcgctcagatgaggagtgtcgtcaagtctacccaggaaagatcactgacaacatgttgtgt
gccggcacaaaagaaggtggcaaagactcctgtgagggtgactctgggggccctctggtctgtaacagaacactgtatggcatc
gtctcctggggagacttcccatgtgggcaacctgaccggcctggtgtctacacccgtgtctcaagatacgtcctgtgg
<212> Type : DNA
<211> Length : 582
      SequenceName : AW243944
      SequenceDescription :

Sequence
--------
<213> OrganismName : HUMAN
<400> PreSequenceString :
ttttttttttttgagacaaagtcttgctctgtctcccaggctggagtgcagtggtgcaatcttggctcactgtaacctttgcc
tcctaggttcaagcaattctcctgcctcagtttcccaagtagctgggattacaggcatgcaccaccatgcccggctaatttttg
tattattattattattaattattacttttagtagagatggggtttcgctaagttggccaggctggtcttgaactcctaacctca
agtgatctgcctgcctcggcctcccaaagcgctggatttacaggtatgagccatcgcgctggacccaaagattggatcttctga
gaacatcagagaaagactgtccttgccacccacattgcagcaaaactttcgaaccttgaactctgggttcataatctcacaact
ga
<212> Type : DNA
<211> Length : 422
      SequenceName : AW439329
      SequenceDescription :

Sequence
--------
<213> OrganismName : HUMAN
<400> PreSequenceString :
acaattttaccagtgatcaaatgcagacaacttgttgctttatatatcttccattttgcactgattgagaaagccttatacta
cctagtattttggagttacatgcccaaacataaggcagttcaactcctttcaattcttagaggaagaaaccagtacattcttgt
cgtcccattcttttcacatactctgggtacaacttgtctttagatcaagggtaaatctgtgacactctctgattgatgtctatg
agttaaggcaaactgtaactggcttacagattcatctgtagaacaatctgtttcacaagcccctcacttttctctgaatgaaag
taggagttcatgagcccatctgctaaaggatgtaagggatttgaagttgtgtctcagttgattcatcagcatgtccctgaagtg
atctcccataagaaaatagaagacagggttgatgacactgttcagaaaggccaaaggccgtgtcacaatgtaaaaggagttgat
gacgacctgagtgcactgatactgctttcaactcaccaggcgtgaagcgatcctcacataccgcatgacgtgatatggtgttaa
tagcacagagataattaccagtgccatgatgaccaagattgagaggcgttt
<212> Type : DNA
<211> Length : 639
      SequenceName : AW612141

SequenceDescription :

Sequence
--------
<213> OrganismName : HUMAN
<400> PreSequenceString :
aaaagaaaaagccccctttcttccttttctgtttacttcaagggcataccttggaggtgctcagagaagcgtgaagtttgca
ctatggtggaggatggggaaagagttctaaagtgtctccagctgtgaacccaggaggtcaagtgggctattaaaatctaacgtt
gagtaaatgtgatagtgatgagaaaggaattttgtgtactgtaaccttgcagtagagatgcagctgtccttcgtgtgtggaaac
acacctctcctttacatagttgggaacctcattagaaatgacctcagctgccccatatctacgttcctttcagcagttgtccaa
gtaggagtgtatccagtgaagacatatcaaatcacaaagtcattgtcattagagtgtacttgattactgggcatccttgtaata
taatttcataccactgacacatgatacttgtaagagaacatctttcccagagtgcctcagaccttattgct
<212> Type : DNA
<211> Length : 491
      SequenceName : AW629690
      SequenceDescription :

Sequence
--------
<213> OrganismName : HUMAN
<400> PreSequenceString :
acgcgtccgcagcaagtaaaaataggagactttggtctggcctgcacagacatcctacagaagaacacagactggaccaacaga
aacgggaagagaacaccaacacatacgtccagagtgggtacttgtctgtacgcttcacccgaacagttggaaggatctgagtat
gatgccaagtcagatatgtacagcttgggtgtggtcctgctagagctctttcagccgtttggaacagaaatggagcgagcagaa
gttctaacaggtttaagaactggtcagttgccggaatccctccgtaaaaggtgtccagtgcaagccaagtatatccagcactta
acgagaaggaactcatcgcagagaccatctgccattcagctgctgcagagtgaacttttccaaaattctggaaatgttaacctc
accctacagatgaagataatagagcaagaaaaagaaattgcagaactaaagaagtagctaaacctcctttctcaagacaaaggg
gtgagg
<212> Type : DNA
<211> Length : 510
      SequenceName : AW630208
      SequenceDescription :

Sequence
--------
<213> OrganismName : HUMAN
<400> PreSequenceString :
gccaatgatgttgagctttattaatggcccctctccagaggctgctcagttgtccccagggaactcctcagagatcctctgcct
tcccacatatgagcccgaggacacctcgggagcagagaagtgaaagggtttccgggtcagacgctgcactccacgcctgcgtcc
tcctcgtggctgcagtcatgatggccccagctattcttggtgcagctccacagggtactctccgtgccccgacactgaacatta
tccagccagatctgcccagtgccagctcccactttgtacagggcccttcctttggagtaacccagcatgcggcagaagacaatg
gcatcagaattttgccactcgtcatcgcaaattgtcccccaggtaccactgtagtaaacttcagcccggcctcggttactactg
ccgacaatcctgacggacactgagttttcacctctttcaccttttctccctttactccttgctccccagaagatcctttcact
ccctggtctcccttctg
<212> Type : DNA
<211> Length : 521
      SequenceName : AW770551
      SequenceDescription :

Sequence
--------
<213> OrganismName : HUMAN
<400> PreSequenceString :
gtggtgtctaagagtaactttattgaaaatactaatcctccatgttacttctgactggccctgagtctgggaaggccgccaaag
tgtctaggtgatgtattactctttatggtagaacacctattcattataaacttcccccaatacaacccctgttgttgcagaaat
cttaggctgtgacaaccatagctgcctacacattccttgtatcttggggtaaaagcacacgtgctctggaaggaatgtgtaggt
ggctatgggtgcacaatttcaggggggttcgtgaactccagttaagacttgccctaattataccatgtaaataattcaataatgg
gcaattctgtagtagaaattttattcccacccataaaatatatcactaaatagctgaaaaatttacatattattttaaaacata
gacttaaaaaatcatattagcttctccttagcaaaatgcttttgttttatgtatttacaagaatatactgtacttcaggtacac
aattcactcaagccagcctgagaaggccttggatgcagatcaatgctccaataaagttcattatcagctcctcctgccttgtga
caggatgatttgattttacaaaagtccctttgaaaaacagagtaaacgcagacagctt
<212> Type : DNA
<211> Length : 646

SequenceName : AW772610
SequenceDescription :

Sequence
--------
<213> OrganismName : HUMAN
<400> PreSequenceString :
tgcttttacagnatataaaacacttaaaatacagttgcttatttcatttcctcagtaatcctagagagtatttcctgcatctta
ttgatgaggaattgaagctgggatagcttctctcctgactcttgaagtccgaaaacaagtggacggagctgtagcactaagcct
cagcctgctcgtcttcaacctgctcctggctgcagagtcttgattgactctgcctgtctccagtgtgctttcattcccttcagt
gggtaacttgtcagacctcgtcttgtttttgagactttggtttctgtcttgccttcttcctaactttgggtattcttagttcca
aatcctgaataactcctgtaaatggggcctcccagtagggggggttcctgtatttatattcacagatgtacgtattttaaatag
ttaacatctggaaatcagtattacatcttaaagttg
<212> Type : DNA
<211> Length : 456
SequenceName : H12102
SequenceDescription :

Sequence
--------
<213> OrganismName : HUMAN
<400> PreSequenceString :
tttttaaattnaaaggagtttaattgagcaataaacagttcaagaattgggcagccttcccagccagagtaggctcggacact
ccagcgcagtcacacggtggaaggtttgcggacagaaaatggaagtgaggtacagaaacagctgggcttggctacagcttggca
tttgccttatctgaacgtggtttgaacagttggctacatttgattggccaaaactcagtgattggcacaagtgtagtctgttta
cacctccacttgtcacgatatacagacaaacctttagggccaaacttaaatatataaggaggcagctttaggctaaactttatt
tcaatacctgtattccaacactttgggaggccgaggcgggaggatcacttgagcctaggagttagagnttcagcccaagcaaca
t
<212> Type : DNA
<211> Length : 421
SequenceName : H29257
SequenceDescription :

Sequence
--------
<213> OrganismName : HUMAN
<400> PreSequenceString :
gnaccaggnaactctactattagnagggacgactgccttcaagcatccctgcaactgccacagcaggtgggggcagggctcgtg
ggaccctctggacagccaccaagatatctgagcaaccaagagtcccagccccaccagtatctgcttctgtagcccacggcaccc
caaacttggagggacctncccactcccttnccccattcccaaagg
<212> Type : DNA
<211> Length : 213
SequenceName : H30357
SequenceDescription :

Sequence
--------
<213> OrganismName : HUMAN
<400> PreSequenceString :
gacagaatctcaccctgtcgcccaggctggagtgtaatggtgcgatctcggctcactgaaacctccgtctcctgggttcaaacg
gctctcctgcctcagcctcccaagcagccggaattacaggcgcccgcactacacccagctaattttgtgtttttagtagagac
gaggtttcaccatgttggccatgctggtctcgaactcctgacctcatgatccgcctgcctcagtctcccgaagtgctgggatta
caggcgtgacacncgtgcccggcctattctcccagtaattcttgatgtgtagggtgcatgggaagggcaggatgtttgaaatat
gtatatttgtgggaatgggttataattgaggctaattaaggtatggcctcatatac
<212> Type : DNA
<211> Length : 392
SequenceName : H72339
SequenceDescription :

Sequence
--------
<213> OrganismName : HUMAN
<400> PreSequenceString :

ccaggaagcccactgtaattcgaattccagccaaaccaggaaaatgtttacatgaggatccacaaagtccacctcctctccctg
ctgaaaaacctattggaaacactttcagtacagtatctggaaagctcagtaatgttgagagaactagaaacttggaatccaacc
acccaggtcaaacaggaggtttgtgcgagtaccccaaggttgccaccgagacctgtgaatggaaaaaccattccaactcaac
agcctccaaccaaggtgccccctgagagaccacctnccccaaagctttctgcaaccagaagatctaattaaggaaactgccttt
ttaat
<212> Type : DNA
<211> Length : 341
      SequenceName : H86407
      SequenceDescription :

Sequence
--------
<213> OrganismName : HUMAN
<400> PreSequenceString :
ttcccctgactcccatctcctagagggaaacnntcattaagagactggtgtatatctttatacattttactgtcttatttgtag
aaacaaatgtttctgtatgaaatcatttttatatcctttctaccatttcttttataactcagctctctctcttgttctctct
ctctgtatgtgtgtgtgtatatatgtgtatatatacatatatatatacatatactttaaacatctttttatggtgatagagg
tgcatcgtttttagcaatttcgtagttgtcccctgtatgaagcaggggggaaaggaagggctagtttcatgactgaagcagagct
gctgtttgggcaagaacatcatctttatttagctttcataaaccatcttggttgtggaaaagacagcacagccaggatgggctg
aaagtgcttggggatgtctaatctgcttagcataagnaccaaccaagattctggagaaagtcaggactcaagagtatgcaaatc
aggnagctt
<212> Type : DNA
<211> Length : 513
      SequenceName : N45149
      SequenceDescription :

Sequence
--------
<213> OrganismName : HUMAN
<400> PreSequenceString :
atctcttcaactggaatgacaacagattaaaaaaaatgtgcaaccataaggacaccgtaacctatgagtgacatgctaaaccag
aaacccaaaacaatgggagtgacatgctaaaccagaaacccaaaacaatgggactgatgtactaaaaccggaacccaaaacaat
gggagtgatgtactaaaaccagaacccaaaacaatggaagtgacgtgctaaaccagaaacccaaaacaatgggagtaacatgct
aaaaccagaacccaaaacaatgggagtgacgtgctaaaaccattacccaaaacaatgggagtgatgtgctaaaccggaaaccca
aaacaatnggtaacttaagagtgaggctaaggcccttacattttggtcacactctcaactaagtgagaacttgactgaaaagga
ggatt
<212> Type : DNA
<211> Length : 425
      SequenceName : N54592
      SequenceDescription :

Sequence
--------
<213> OrganismName : HUMAN
<400> PreSequenceString :
aaacatgtaatcttgctccntcaaaaacaatttactctcaaatggttcagaaaacaataacctgtatgtatgtgtgtgtacata
tatatgtgtgtatatatataggtatatataatgaaattacatatatataattagatatatattagatctatatatagagatatg
taaatgaaatttcattatgttaacaattggtggatatggaagagtatgagggctctttaaaccattcttgtaacttttctataa
gtttgagattatttcaaattacgaatttaaagcagttcagtggtaggaagtgattttcatcttcacttggtggtctgttatgtg
cctgcattgcagatatgattttgattcataagcatagctttgcttttctcagaaatcttttcctgctgagtcctcccttccctg
ccatccaagtacagaagagagcactc
<212> Type : DNA
<211> Length : 446
      SequenceName : N57580
      SequenceDescription :

Sequence
--------
<213> OrganismName : HUMAN
<400> PreSequenceString :
aaatttcggcangaggtngagaagagaatgcctgtgtccngagataattctgtaagnaaatgccagtccctaggnagagagaac
caaaatgtctaccngaccagatgtcaaggctacagtgggggacatttccagtgatggcaatttaaacgtggctcaagaggaatg
ctccaggggaagggatagtgggatgaattcttcccgctgttgtcaaacttaatgtatatgggnctcaaccacaggggatatccc

cagagctcctatgnaacactaggcaaattttgtgtttttgtttcagtccgccatggggctggccctccatctttgcagctctgt
aatttttccaatttacaccccccaaatgga
<212> Type : DNA
<211> Length : 367
      SequenceName : R09122
      SequenceDescription :

Sequence
--------
<213> OrganismName : HUMAN
<400> PreSequenceString :
tgagccaagccccacatgtctgtgaaatgtggtcttctagccatagaagtagcttgtgctcctagggtattccttgaggctctg
tatgaagaatgtccatttgtttgagtccctaacctacagaatattcctgaccatggaaatgtgatagataggagtaagcacatg
gaccaaaaggcctggaggctcatcatttaaggtacaagccaattatgtagtctttgaaagaatgctttgaaattcagcttgctt
gattcaattcccaatattttcatttgtattaaaagatttggcccttacaggttaaaaacaatgtcagtgtaaccaagtgacatt
tcaantttgggctncccaacatgtggatatgacatcacttcatgtggaaatnggcgangtcccca
<212> Type : DNA
<211> Length : 401
      SequenceName : R13802
      SequenceDescription :

Sequence
--------
<213> OrganismName : HUMAN
<400> PreSequenceString :
tnnnntnantagaattgcaaacatactttagcctaagaagcaggaagagaaaattatactattctttaaaaagttctttagtag
caattggactactctttccctattatctaaatacttcagtagtacttagtacttctctttctccagaatctaattcacaagtaa
ctatttgcattctatttccattcaaataaacacttttacctgatcatattctgaagcaccaggggtaaagaggccatcccaggg
nacagctcagctatcacacagcccagtgaccacatatcaatagcttcacaaaatgggtaacccaagantaatttcaggagctct
aggcaaaaacaaaaaatatccggattaaaanaaaaaacnttatttggggaaattaacttcatggncttaggtcccgttttg
gg
<212> Type : DNA
<211> Length : 422
      SequenceName : R88730
      SequenceDescription :

Sequence
--------
<213> OrganismName : HUMAN
<400> PreSequenceString :
aagagcctccaggacagtaattccctgtttaaagcttccctcttgatgagacattcttacagattgtggtccacagatgacctg
catcagaattgcttgtgatattgttaaaaatatagactctcagagaggattgtaggaagatggcagagtagcaagcaccaggga
acttttctccttacctagacaataattgcacgggcagaatctgtctgatacaactattttggagttcaggagtctattgaaggc
ttgcaaatttcagggacagcttgggacaataaagtacagttaatttcagtcagtttcaggccttaggcacagtaacaggcttct
ccattcttcccngccaaccagccctggacaggngctgtgcnatgttttccccgaaaaacttgcacacagctttaggggccgggt
t
<212> Type : DNA
<211> Length : 421
      SequenceName : R91509
      SequenceDescription :

Sequence
--------
<213> OrganismName : HUMAN
<400> PreSequenceString :
tgattacattccagccagggtgacagagtgagacccttctcagaaaaaagcaggggagatcattcccttaatctgtgagacaat
gacccccatcttccagtttatatccaagaacgaatcaatgtagatacaggaacaaggatcccgaattaggctacaggagacaca
gatgccagtattggntctgctctaattagctacatggctttaagcagtcatgttacctttctgtctctgtgtcttcaactgtaa
aacaaggtgggttcacctttactttcacaccattacacaaaaatttaacttcagtatggtattcagagggctttaaatgcaaga
gcttaaaacttaaccacttttaaaaaaaaaacaggggaaaaaaaaatcttttcacaatgttnggggttaggttaaaggntttct
ttagggattaccaacacccaaagggcaaattccat
<212> Type : DNA
<211> Length : 455

SequenceName : T94331
SequenceDescription :

Sequence
--------
<213> OrganismName : HUMAN
<400> PreSequenceString :
gcccaccttngcctnccaaagtnctggcattgcaggctgagcacggtgcccagcctgaacaccctttcctggtaaaacactcca
aaaccaggaaaagaaggaatgtacagcaacaaaataaaggccagtcatgcaaggcccatggctgaaagtctttcagtcatttta
ggtgaaagactgaantctttgcctccaagatcaggaataagagaaggatgcccgatctcactacttctattcaacacaggnttt
gaagtcaggctggtcacagtggctcacanctgtaatnccaggcnctttccggnagttcgagggcaggtgggatttacatggag
gctcttgagtttgcagnaccaccctgggccnacatgggccaanaaccccg
<212> Type : DNA
<211> Length : 386
SequenceName : T95173
SequenceDescription :

Sequence
--------
<213> OrganismName : HUMAN
<400> PreSequenceString :
aaagacacagttgtgcatgtctggcacaacctctcggcctgcaactgtgtttagtgataatgatgaactgtgtgtgtgtgactt
tgaaggattctgtatgtcaagtgaaaaaaaatgatgtctgacctccttacatatgtaaaaaatataccttcagagttcagcagt
aagctggaagaagtagatgtcaaagaagttttgaactttgataataatgcttcagttattcattcattgactcggtggtaaaat
agtcgaaatggttttgaatcagggttatgatagtgatgaagaaggtcaagataacaccacagaaaaggtgcttatagaagaaga
catggtgaaaatgtatggtgggcatattgaaggattacggcagcgtgcattaacaacagaattgaaaaaccatgtcagtttta
aaattgaagagagacttctgagctaaaaaccatgtt
<212> Type : DNA
<211> Length : 456
SequenceName : W01596
SequenceDescription :

Sequence
--------
<213> OrganismName : HUMAN
<400> PreSequenceString :
gagaaaaggaaaaatcaaatgagattcccactaaaaatatcngaaggtcagatgacaccatactatcctgtgggaatgggaaat
ggtacaccttgtagtttgaaacagaaccggcccagatcaagtactgtgatgtacatatgtcatcctgaatctaagcatgaaatt
ctttcagtagctgaagttacaacttgtgaatatgaagttgtcattttgacaccactcttgtgcagtcatcctaaatataggttc
agagcatctcctgtgaatgacatattttgtcaatcactgccaggatctccatttaagcccctcaccctgaggcagctggagcag
caggaagaaatactaagggtgccttttaggagaaataaagagggtgtcggttggtggaaatatgaattctgctatggcaaacat
gtacatcaataccatgaggacaaggatagtgggaaaacctctgtggttgtccgggacatgggaanccaagaagagcatattgaa
tgggctaagaagaatactgctagagcttatcatcttcaagacgatggtacccagacantcaggatggtgtncattttatggga
atggngatatttgtgatatantgacaacccagacaggtgactgtaaactaagtgcaagatccgt
<212> Type : DNA
<211> Length : 652
SequenceName : W07181
SequenceDescription :

Sequence
--------
<213> OrganismName : HUMAN
<400> PreSequenceString :
acattctgaggtcctgcanttcagtgttaacatgtgaattttggggatacacaactgaaccataacacaaactgtgaattctta
gcaccttaagttgtagaagagaacagagccactcctccagccattgctccaatggctctggttaagttgtagctcagtagagag
catgaatgctctcaaaaaagcactacagttgtcctcggtatccacaggggggttgcttcctggatcccctcagacaccaaaatgc
acagacattcaagtccctggtaaaaaatggtgtagcatttatgtataacatatgcacatcctccccgtgtactttacatcatct
ctacattacttgtaatacctaataaagtgtagatgctatnggaggatagttggatataccggnaattggtttttnaaaattta
aggngaatttttnaatggnttggggtttttttcccccgaaaaattttggggnccatggaattgtgga
<212> Type : DNA
<211> Length : 492
SequenceName : W38474
SequenceDescription :

Sequence
--------
<213> OrganismName : HUMAN
<400> PreSequenceString :
ctcactgaacaatgagacaatgttgaggactcaaatgaatcaaaccccgtgganatgacagaagtgaagaatctggtccctgtc
tttaaggagtttgcactccagtagaagacagaaggaacgtatgtttacaaaccacttcactggaagacgtcaaacaagctgaat
gaagggcgcttagaaaacgttaatagaagttctaagcgggagatgactccctactgggatgatgaaggatggcatcctagtga
agaagcagctcaaacattttgataaaatggcaacaaaatgcagacaccctgctccaggtattatttcaggtttagtacaagtct
gttaataccctatgtggtttcattaggataactttttacctatccttgaggtcatccatattcttacaggccttccagtcaata
atggaagagctcactctatacaaaaccaatatgcaagg
<212> Type : DNA
<211> Length : 458
      SequenceName : W77977
      SequenceDescription :

Sequence
--------
<213> OrganismName : HUMAN
<400> PreSequenceString :
aaggaatcgcctccggaatggtgagtcccaccaacaaacctgccagcagggcgagagtagggagaggtgtgagaattgtgggct
tcactggaaggtagagaccccttcctatgcaacttgtgtggctgggtcagcagctattcattgagtttgtctgtgtcactgaa
actgaccccagccaactgttctcagttcacagccctgttttcaaagaattacacatctctaaaggcaaacagggcacggacaag
gcaaactggagaggcaaactgtagcctgagatggcctgggcttgccatcacaggtattcaggtgctgagggcccttagaccaac
tagagcacctcactgcctaggaaatcaatgaaggggaaatgagttctagcggaccctgaaggatcagaattggataaagttctt
attggcagagaggcaccaggattgaagtgacag
<212> Type : DNA
<211> Length : 453
      SequenceName : 1329021F6
      SequenceDescription :

Sequence
--------
<213> OrganismName : HUMAN
<400> PreSequenceString :
gcacccacactggcgagcggccctacaaatgtccccgttgcggcaaggccttcgccgacagctcttacctgcttcgccaccagc
gcactcactctggccagaagccctacaagtgcccacattgtggcaaggccttcggcgacagctcctacctcctgcgacaccagc
gcacccacagccacgagcggccctacagctgcaccgagtgcggcaagtgctatagccagaactcgtccctgcgcaccatcagag
ggtgcaca
<212> Type : DNA
<211> Length : 260
      SequenceName : 1390130H1
      SequenceDescription :

Sequence
--------
<213> OrganismName : HUMAN
<400> PreSequenceString :
caaattcctgactataatgtagcataagaattggtttttatgccccagagagaactcatcagtgaaattaacaaaacttacat
ttaaaataattagtatgaatgttaatgatcccaagtaaagtttttaaacaaaataagcaaacatttgggcctcagaataagcaa
atttaatacatggacattcacaattagaatataagttcattctgagatatttatttactttgcagaaaattcccttccccatgg
aattgcttttgaaaatgtgatctcttcaagcatttgaagtataatttcattaaacacacataaccaacttatattgtgtaaatt
agggccttcctgactaggcacaaaactacctgcatttagtttaaatatgatctctttaaaaatttgtaaattaggcacctgccc
tcccccaaacacctttacttaccccagcttgtggaattgattccctctggctc
<212> Type : DNA
<211> Length : 473
      SequenceName : 1428534F6
      SequenceDescription :

Sequence
--------
<213> OrganismName : HUMAN
<400> PreSequenceString :

gttctttgcagggctattaatatctaaatgattattaatgcaaggatggtaaatatttgctcaataatatattaaattacaatg
agggatgaagttctctanagatcgtagaagtggcacttcttatgacctctggtttgcaacttttaag
<212> Type : DNA
<211> Length : 151
      SequenceName : 1594182F6
      SequenceDescription :

Sequence
--------
<213> OrganismName : HUMAN
<400> PreSequenceString :
ttcctttcagttgtgtgtcttatcttgattgggtcatgagaaagttncatcttagtgctactgtgtcttgaacgcctctgggt
gggagaacaggccttaggcttagagcctgcagcaggcagcagtatcttgctgggtcttaataacattgttttgcttttatcttt
atagttaccttgtgtttatgacaaaccttcaaaaagtttaaattttgaaacattgcataatataaaaatgtttttatttaaga
aggtgagtagatttgaagaaaatacacattgagtattatctaaaatgcttgggacaagaagtgtttcagattttggattttttt
tttttgatttgtaatatttgctgtggagcgttccctaatctgaaaatccagaatgcnccagtgaacatttactttgaccatgc
ctggctaggtttttttttttttggaattttnannagaaanggggt
<212> Type : DNA
<211> Length : 465
      SequenceName : 1671315F6
      SequenceDescription :

Sequence
--------
<213> OrganismName : HUMAN
<400> PreSequenceString :
aaccaactcagaccagaactgtttctcaacctgggacttcctaaactttccaaaaccttcctcttccagcaactgaacctcgc
cataaggcacttatccctggttcctagcaccccttatcccctcag
<212> Type : DNA
<211> Length : 129
      SequenceName : 1686702H1
      SequenceDescription :

Sequence
--------
<213> OrganismName : HUMAN
<400> PreSequenceString :
ccgggagcagcgccggcaagtggagtcgtcgtattccgggcggcccgcggcccacggggatgggacgtcccggggctgtgctga
ccccaaaacccttccacactttattagcctgtcgcccttctttcatgtttacagcaaatattttccgagtgcctaatgtgtccc
acttactgtgccacgtgcgagggtgcacgtgtc
<212> Type : DNA
<211> Length : 201
      SequenceName : 1731618H1
      SequenceDescription :

Sequence
--------
<213> OrganismName : HUMAN
<400> PreSequenceString :
ggatgaattcagaatctttttcattccaaaacttcctgcatataatggtagcaacccacaatgagacattcttttagtttctaa
aagcaggaaataagcattttcctgaaagtttcctcatctcttcatcatacac
<212> Type : DNA
<211> Length : 136
      SequenceName : 1879290H1
      SequenceDescription :

Sequence
--------
<213> OrganismName : HUMAN
<400> PreSequenceString :
ataagacactgtactttgacgtggagccgttcgtcttttacatcctgactgaggtggaccggcagggggcccacattgttggct
acttctccaaggagaaggagtcccc
<212> Type : DNA

<211> Length : 109
      SequenceName : 1961084H1
      SequenceDescription :

Sequence
--------
<213> OrganismName : HUMAN
<400> PreSequenceString :
gtcagatggaggaggctcagaaaagggaagggggtcatatagaggaggcgcaganaagggaaggggctcagatggaggaggtgc
agatnnngggaggnggtcanatggaggaggctcagagaagggaagggactcagatggnggagggggcgcagagaagagaagggg
ctcanatggaggaggaggcgcaganaagggaaggggctcaagatgggaagggggccctggaangtctcggctctncttctgtaa
aatcnggggagttttcagggtgaagggantncagtctgcangctgggatccccccctaatttncnanccggcttgctc
<212> Type : DNA
<211> Length : 329
      SequenceName : 2017923F6
      SequenceDescription :

Sequence
--------
<213> OrganismName : HUMAN
<400> PreSequenceString :
gtggggctggtgcccgtggtgggcggcgaagagagctggggggtcccctgctggccgcggctgtggcctatgggctgagcgcg
gggagttacgccccgctggttttcggtgtactccccgggctggtgggcgtcggaggtgtggtgcaggccacagggctggtgatg
atgctgatgagcctcggggggctcctgggccctcccctgtcaggcttcctaagggatgagacaggagacttcaccgcctc
<212> Type : DNA
<211> Length : 248
      SequenceName : 2194064H1
      SequenceDescription :

Sequence
--------
<213> OrganismName : HUMAN
<400> PreSequenceString :
ccagtggggagaaagtcggccagacatcactggaggaagaaagaggagggggccccctattaaccctgggcctcctggcttctc
tgtcgtcaccgcagagggagtgtggtgattgagaaacaagacagcttcaacaagaccggcagtggggtatgactcaccttccag
aagccaaaggcaaagaccagaccgctccccaatactctcctaaaataaacatgaagcact
<212> Type : DNA
<211> Length : 228
      SequenceName : 2397317F6
      SequenceDescription :

Sequence
--------
<213> OrganismName : HUMAN
<400> PreSequenceString :
tgcaaagttcagagatcaaaaatgtctcccaagttttccggcaacaaattgccgaggttgtatttgagtcagttacttaaggtg
ttttggtccccacagccatgccagtagcaacttgcttgtgagcaggcctcagtgcagtgggaatgactctgccatgcaccgtgt
ccccggccgggcctgtgttgtgcaatgctgtacatcacaacaggagggtaggggacaagagagcacaggtcctggcagctgcc
acagtctccaggggcttttgcgtttctctccagatttctaaggttaacatggggattagctgttttgcaatgaataaaggtaa
cattgcctggaatgttgcttaaagacactttttaaagctagttgattgttaagctgttgctacttaaattgaaaaactacttt
tggg
<212> Type : DNA
<211> Length : 424
      SequenceName : 2405358R6
      SequenceDescription :

Sequence
--------
<213> OrganismName : HUMAN
<400> PreSequenceString :
ggagcattccaggagagaaaggtgagtgggctgggtcagttgaggactttatgaaagggccgtggtagtgtgtcctgtcacagc
aatgaagaagaatttggcaatagaagtcacatttggtgcagaagtggtagacatggaaacagcacagtgaacagctggacgagg
ctgaccaagagaactgtcctgagggagtgagttttcactgc

<212> Type : DNA
<211> Length : 209
      SequenceName : 2459540H1
      SequenceDescription :

Sequence
--------
<213> OrganismName : HUMAN
<400> PreSequenceString :
cctacttgcgacaaggtggacttgggaggaaagccgtctgccaaagcctgaagcctccaagttctgtagttcaggaaccttgtc
tgttgtagcagggataaacccacaagaacatggatacagaacagttttggaatgaacattgccagtgctacaattgcactagt
ggggactgcttttctctcactaaatatagcagttaatatccag
<212> Type : DNA
<211> Length : 211
      SequenceName : 2470939H1
      SequenceDescription :

Sequence
--------
<213> OrganismName : HUMAN
<400> PreSequenceString :
gtcgggctttacggcgccggatggctctggacgtgaagtctcgggcaaagcgttatgagaactggattccttggggagggacag
tttgccaccgtttacaaggccagagataagaacaccaaccaaattgtcgccattaagaaaatcaaacttggacatagatcagaa
gctaaagatggtataaatagaaccgccttaagagagataaaattattacaggagctaagtcatccaaatataattggtctcctt
gatgcttttggacataaatctaatattagccttgtctttgattttatggaaactga
<212> Type : DNA
<211> Length : 308
      SequenceName : 2496221H1
      SequenceDescription :

Sequence
--------
<213> OrganismName : HUMAN
<400> PreSequenceString :
cgtcaagacgggacccagccagcatgcgtcgtgacagcttggggcattggaggtgggtgttagtggttagaagaaacttcccag
cagagcgggttgctggagccaagcgcgcatctgtcaagaatgctctctgggcgctgttcttgcaaggatcctctgttgaacctc
gagtgcagtagatgtggaaaggaagcgaaggtttgtgcatctaacagctaacgctgagggccctttggaatc
<212> Type : DNA
<211> Length : 240
      SequenceName : 2535659H1
      SequenceDescription :

Sequence
--------
<213> OrganismName : HUMAN
<400> PreSequenceString :
aaactttactgaaataaacaaacaagaaaactggatgaggtctccatcttgttttatgtcctcgggaatttgactttgtgagaa
catggcggtactttctcttggtctccggcttccaaggaagaagaattttaaagttcatgtcatagttaactctaaaaattacct
taagtagttaaaagcctttacaagctcaaaattaactactctagactccttccggaaagggcaatataaaccacccagtgctgt
aactcagtaactaagattttgtcctttcatagtgacagcccaggttcaattcctgacttaggaaataagtcctttcttgtttaa
tatctgcataaccattaccatttgttgattgtcttccccttctgt
<212> Type : DNA
<211> Length : 381
      SequenceName : 2668191F6
      SequenceDescription :

Sequence
--------
<213> OrganismName : HUMAN
<400> PreSequenceString :
tgctgtgtcactggccatggttactcatatttggcttagaataaatctcttcaaannnnnnnnnnnnnnnnnnnnnnnntaaag
gaaaaacaaatgcttttgcagaagccaagaagaaagtgaagggaaataaatgagagtggaaaaacgagagatcaattgtg
<212> Type : DNA

<211> Length : 164
      SequenceName : 2798009H1
      SequenceDescription :

Sequence
--------
<213> OrganismName : HUMAN
<400> PreSequenceString :
atcctgatacttcgctcctaaatcatctttttttccctaagtcttttgtatgcaccactaaaaaaaaaaaagccatttttcct
gcatgacccaacagaatgggctgtaattagcttgtaatatatgcaaatgtccatctggattctgacttcccaagctgcatggct
gccttcatttgcagggggaggtttgctgcagagggggcagcatcagcaggcgtgtggagtgagtgagccccgtcctccccct
cctcctgacattggctctcattgcctaaccccttgtgtcttggccccaggttgattttccgggggtaccgc
<212> Type : DNA
<211> Length : 324
      SequenceName : 2798810F6
      SequenceDescription :

Sequence
--------
<213> OrganismName : HUMAN
<400> PreSequenceString :
ggatggactcccagcaggtatgggacaggtgccaggtgtgtggtggggacaacagcacgtgcagcccacggaagggctctttca
cagctggcagagcgagaggtaggcggcctccctcggggcagaagctgggcttcccccagcctccaagatggccacagcccagag
cgttggtgcaggggctgctcaggtcacagggcctgcacactcactcagccctggatgcctcctgtggtgtcagcgtctccctct
tccacttcgccacccttctgtggcaggctcaggttttggcctt
<212> Type : DNA
<211> Length : 295
      SequenceName : 2858615H1
      SequenceDescription :

Sequence
--------
<213> OrganismName : HUMAN
<400> PreSequenceString :
tggagaacaggtgtgagaatggcatggcatatacagggagggaaggatgtattaaagaagannnnnnnnnnnnnnnnnnnnnn
nnnnnnnnnnnnnnnnnnnnnnnnnnnnnnnnnnnnnnnnnnnnnnnnnnnnnnnnnnnnnnnnnnnnnnnnnnnnnnnnnnn
nnnnnnnnnnnnnnnnncatgtacaacattgcccatgatgtggaagtcaaggggcaggtcagcgctgtaggccacataggctat
ggcaatggatgattagtaggtgcccactaccgggagatgggagctgcaggtggagaaggctttggagcgtccttcttgggagtt
aatgcgaacgaccgaggtcaagatgtgggcataggagag
<212> Type : DNA
<211> Length : 375
      SequenceName : 2894096F6
      SequenceDescription :

Sequence
--------
<213> OrganismName : HUMAN
<400> PreSequenceString :
gaggtttcacagcaaaggccccaccctcaagcctggaaacctgcagccctaaatgagaacaggcattcctgtttttgcggccaa
annnnnnnnnnnnnnnnnnnnnnnnnnnnnnnnnnnnnnnnnnnnnnnnnnnnnnnnnnnnnnnnnnagctccactggcagagcanc
aaaatggtncagcagngaaatagngaagaggngaagcntntnaacatgaagaa
<212> Type : DNA
<211> Length : 221
      SequenceName : 2937644F6
      SequenceDescription :

Sequence
--------
<213> OrganismName : HUMAN
<400> PreSequenceString :
gcccccaacaactgaatggaccccttgcggtcaaggggacaccagtggcaataagataccaaattatgaataggacctaaggt
catgccaggcaagggttaagtcacacaccctacatttaaactatattcctactgccacactgcttt
<212> Type : DNA

<211> Length : 150
      SequenceName : 2992331H1
      SequenceDescription :

Sequence
--------
<213> OrganismName : HUMAN
<400> PreSequenceString :
aagtccccca cccttggaca tttagatttt ttccatcttt ctgtatttgc agatatgctt      60
ctacgtacac ctttattc                                                    78
<212> Type : DNA
<211> Length : 78
      SequenceName : 3123232H1
      SequenceDescription :

Sequence
--------
<213> OrganismName : HUMAN
<400> PreSequenceString :
acacacatggagctgtcatcgcctgtggtaacaatgccttctacagtacttcccaaagggcctgctagttcacttaattctttt
atagagagaaggagtacactctaacactgatca
<212> Type : DNA
<211> Length : 117
      SequenceName : 3123948H1
      SequenceDescription :

Sequence
--------
<213> OrganismName : HUMAN
<400> PreSequenceString :
gcagtttgccctccgctcacgcagagcctctccgtggcttccgcaccttgagcattaggccagttctcctcttctctctaatcc
atccgtcacctctcctgtcatccgtttccatgccgtgaggtc
<212> Type : DNA
<211> Length : 126
      SequenceName : 3241088H1
      SequenceDescription :

Sequence
--------
<213> OrganismName : HUMAN
<400> PreSequenceString :
ctccagacagcatgaggctccctcgaaccgcccccatcaaatgctcatgtcaggaaatcatctgatatatgaattcacatcgag
ttggcaatgcctgataaggcttggggtgacctctccataggggatgaagtcaagatggcagtcagaaccacaagggaatggctc
agggataggtgcctggaaagggtcacattttcggaaatcctagtcttcagtgtcctggatctgtatgccttaccattttccatt
cccagccagaaatgctcaaactttgctgtgttcctgatcttgctcctgactcgtatgagtgtgtctgtgaaagccaagattaac
tcctttgattttgtagactgatgtgcnaatctggcaggtctgactttcttagctgtgaagatgcttgggcacaagtaccaagac
tgatctgcttnctgcgtgtgaca
<212> Type : DNA
<211> Length : 443
      SequenceName : 3253054R6
      SequenceDescription :

Sequence
--------
<213> OrganismName : HUMAN
<400> PreSequenceString :
caaccatcag gagtgcctca agggagaaat atcctctgag aatagccaca gacaaagtgg      60
ggaggtggga ctacaatccc cagccctgga a                                     91
<212> Type : DNA
<211> Length : 91
      SequenceName : 3330472H1
      SequenceDescription :

Sequence
--------
<213> OrganismName : HUMAN
<400> PreSequenceString :
aaactaaagagagaggatagataagacatgctgcagttcttgccctaactttcaatcccaacaactgaaatgtcttccagagaa
gtaactccccccggtagggatgtagcgcggtccctacgtgggtctaagggatctgacccacgacgcactgcactgggttcacga
agcgcctcctcgcaggcggttatctcggtatctctgagagcggcgggctctcgctcccgctccagggattcggggca
<212> Type : DNA
<211> Length : 245
      SequenceName : 3401107H1
      SequenceDescription :

Sequence
--------
<213> OrganismName : HUMAN
<400> PreSequenceString :
atgtgagctctcctccagcttcccagcagcaaccactgagcacttaccctgtgctgggtgcttgctaaacactttacatgcatg
atctcacagaattctaataataatccttcacttattatcaacttcccgagaaaggaaagtttaagcagctaacccttggtccta
cagctggatggcagctggtagagttgagacatctctcttgcgctagacagggaatgaatctgagtgaaggctaagaggatgtct
gcnnnnnnnnnnnnnnnnnnnngacataaactaatgcctacagctagctaattaaccaatcaccatcagttaaataacttttag
caagaaattaaccagtcca
<212> Type : DNA
<211> Length : 355
      SequenceName : 3434105F7
      SequenceDescription :

Sequence
--------
<213> OrganismName : HUMAN
<400> PreSequenceString :
gtggtgtggctgcagtggagagttcccaacaaggctacgcagaagacccccttgactgaagcaatggagggggtccagctgt
ctgctgccaggatcctcgggcagagctggtagaacgggtggcagccatcgatgtgactcacttggaggaggcagatggtggccc
agagcctactagaaacggtgtggacccccaccacgggccagagctgcctctgtgatccctggcagtacttcaagactgctccc
agcccggcctagcctctcagccaggaagctttccctacaggagcggccagcaggaagctatctggaggcgcaggctgggcctta
tgccacggggcctgccagccacatctcccccgggcctggcggagccncacatcgagtcccaccacgtggccatctcagatgca
gaggactgcgtg
<212> Type : DNA
<211> Length : 432
      SequenceName : 3495438F6
      SequenceDescription :

Sequence
--------
<213> OrganismName : HUMAN
<400> PreSequenceString :
aattgccagcctatcgaggctaganatatctcctgatccttgggaatgcctttactgcctgggttgaagcctctcatactagaa
tagaaaagattcaggtagttgtcaagatcattctgaaaganattatctctttgagcctccacagtcttcacaaagtaacaatgg
cctatatttatttcacaaactacccaaacngtggcaaaagccctagggatataatacttttcacactcctcctgaaanccaca
atcttccaggaaggtaganaggactaatcaagctattaaaaggacctcgagcaaatgatgccaggnagcctctttaccatggct
aaaacttctacccatggcacttcttcagatcagaacagcccttaaattacgctatggctagttctttgaagcctatactggaga
cctttctacatgcnaanctatattatacccagaggt
<212> Type : DNA
<211> Length : 456
      SequenceName : 3749346F6
      SequenceDescription :

Sequence
--------
<213> OrganismName : HUMAN
<400> PreSequenceString :
aagatacccagattgtcactgaaagagactcaccgtctatccttatctacctttttaagtccttttaggcagtattataaagca
ctaaatctctaattaaagnnnnnnnnnnnnnnnnnnnnnnnnnnnnnnnnnnnnnnnnnnnnnnncctcagattccaaatccaga
cactcaaatcccttatataaaattgtatagtatttacatacaatctatgaacatcctcccatagacnnnnnnnnnnnnnnnnnn

nnnnnnnnnnnnnnnnnnnnnnnnnnnnnnnnnnnnnnnnnnnnnnnnnnnnnnnnnnnnnnnnnnnn
nnnnnnnnnnnnnnnnnnnnnnnnnnnnnnnnnnnnnnnnnnnnnnnnnnnngattaggaaagacaaaag
tccgtgatatgagataatacctcaatttttaagtggttctgaacattcctgag
<212> Type : DNA
<211> Length : 474
      SequenceName : 3978404F6
      SequenceDescription :

Sequence
--------
<213> OrganismName : HUMAN
<400> PreSequenceString :
taccctgcttcacgtgcctaagtctggggtcttttctcataacccctcctggaatctgagggagaatccaggccagctccagta
aatcccacccatgtatcctttttatttccatctgaaaagagtttggaatgaaacaagcgttttcagaatgagcttcctcacagga
ctttgtgcttatcatgctgctgttttagcttctccagtcctttctcactctatccctcaccctttctgggtcactgacagtcca
gcttccccctgcaaagcatcctgggaaaacttgagttgtgttcccagagttgacaagtcctttctgatttcagatacgtcacac
tgtcaaatggaacctgcattttacccccttttcagctggcgttggacatctttgaggtgtcctgaagaggagggtggttatt
tggagactatcagtggaaatgaagagctcagctgatggctgtagatcctcacccacatggtccctcagccccgaaaggttttac
ttgagggctggttcctatttgataa
<212> Type : DNA
<211> Length : 529
      SequenceName : 3986782F7
      SequenceDescription :

Sequence
--------
<213> OrganismName : HUMAN
<400> PreSequenceString :
aggaattaaaagaaaatatgatttaaatgacttgacaaatagggaatctaaatagtgaaatacaaactataaaagaattaaat
ggaaattctggagttggaaggtacagttaccaaaattaagaattcactagatgggctcaatagcagattttaagatagtggaag
aaccagtgaacatgaaaatagaccaatagaaatgatccaatttgaaaaacagagagaaaaaaatgaagacaaatgaacagacct
tcagacacacataggatgatgttaaatgtcccacatacatgtaatgggagccccaaaaagggagagacaaaatattgatgaa
aaatattgaattgcagatttttttaaaaaagctcaaccaacactgactttataaacagaaaga
<212> Type : DNA
<211> Length : 402
      SequenceName : 4019605F6
      SequenceDescription :

Sequence
--------
<213> OrganismName : HUMAN
<400> PreSequenceString :
attaggtggcagagccagctccactctgcgtgtgcccccgactctattccgcttgtgagccccactcggaaagtaggcagcggt
ccggacttcccttagagtcttctaacttaaaaatcgggcggcggcacattcctgggaaagggaatgtgtcttccctgtgaatgt
gtctttcctgtgaatgttctctgcaaaagtcagatagcttaatcagac
<212> Type : DNA
<211> Length : 216
      SequenceName : 407169H1
      SequenceDescription :

Sequence
--------
<213> OrganismName : HUMAN
<400> PreSequenceString :
cctcgccagcacagggtgggatcagacggtctgatctgtgccagtcaggtggaagagtcctctcgtaggtttgcttggcatctt
tctgtttaaagtcacattgggtggttaaggagccaccaaacgggctgcccatctcctctgctggactggagccaaggcaagaga
cctggctgtgttgggcatgtcctttctgtagctaaagtccctggagaccccagtgacctgagagtcgggcagatcccaggaggt
cttctggggtggtctctgttcaaggg
<212> Type : DNA
<211> Length : 278
      SequenceName : 4097060H1
      SequenceDescription :

Sequence
--------
<213> OrganismName : HUMAN
<400> PreSequenceString :
ataaaaaatgggtctctgatgacgaatctggttatatggcaccagcagctgaggccaaaagcctggtagcctgccaccaagtgt
accccgaaagaggggtgtgtgggcccgagtcacctgggtggtcctttcaggatttgtggctctgaggtggcccctggtgctggc
atttggggaggtggaggggatagagggaagacattctgggcgaagagtggaagatccataggttcctcggtgaccctcatgggg
acagtacacccgggagattcatgttctccaggtacccggagctcacggactgaccctcttctcctgcctcctctggcctacgtg
ctccgggtagatccactttccttgaaggtgggggtggtggctgttgggcctggggaatcttttgctgtttacttggggaggctc
aggctctcacggttgacctctgcccagttagtttcatgtaagtagagcaacctagtttcacgaaggaagcaccaggc
<212> Type : DNA
<211> Length : 497
      SequenceName : 4130807F7
      SequenceDescription :

Sequence
--------
<213> OrganismName : HUMAN
<400> PreSequenceString :
aaaataggtattaataagaaaaagtgatgagtaaagtgccagtgaccagaaatgggctcctgtgtaggctgccagctggataat
aaagccctgaataaccaataagtgagcatgaaagtaaaattttgaatgttcattatgtgtgactagcttacaactggcctttc
aagacaatatgatagtttaaatctttatttttcttttatttacaaacttatttacttttcaggtgtatgaaatctgaagagaa
gtcccttggtgtgggattacatacattttgcacaagagtatttggtaaaaatagttatttttcccacatcctatcagaacaat
attattcttattatttt
<212> Type : DNA
<211> Length : 353
      SequenceName : 4177228F6
      SequenceDescription :

Sequence
--------
<213> OrganismName : HUMAN
<400> PreSequenceString :
cgtggacatcgtgggccccactgggggcatcatccactcctgccaccggactgaggagaacgtgaccggggagcaagggggcc
agatgtggtacaaccctatgctgcctatgctccttctggaaccccacagggcctcctcgtctatgccaaccggggcgcggaaga
agactttaaggagctacagactcagggcatcaaacttgaaggcaccattgccctgactcgatatgggggtgtagggcgtggggc
caaggctgtgaacgctgccaagcacggggtagctggggtgctggtgtacacagaccctgccgacatcaacgatgggctgagctc
acccgacgaaacctttcccaactcctggtacctgccccctcaggagtggagcgaggctcctactacgagtattttggggaccc
t
<212> Type : DNA
<211> Length : 421
      SequenceName : 4181072F6
      SequenceDescription :

Sequence
--------
<213> OrganismName : HUMAN
<400> PreSequenceString :
ggtgcacgcatctcatgtctgaatctcctctttctcccttctttctttcctccctcccaagcttacatacctttgttaccggct
ggtgttgtgaagtcnnnnnnnnnnnnnnnnnnnnnnnnnnnnnnnnnnnnnnnnnnnnnnnnnnnnntgtagcaatgcagcaagagact
tattaagcacagtaacactctcagagaggggagaacggctgacttctgcaaaatgggatcagcgtcagtttggtgaactttgg
gctttgggtctttttacatgtttt
<212> Type : DNA
<211> Length : 276
      SequenceName : 4228729H2
      SequenceDescription :

Sequence
--------
<213> OrganismName : HUMAN
<400> PreSequenceString :
gcgttctcctagaagacagaattcttacatgccccgtctccatctagcaggaaaagccatatcttgcaccagcagcatgaatgg
tccctccacactctgggc

<212> Type : DNA
<211> Length : 102
      SequenceName : 4294821H1
      SequenceDescription :

Sequence
--------
<213> OrganismName : HUMAN
<400> PreSequenceString :
attctgtggtatccatcttctgaagctgaacttggtcgctaagcaacaaagcagatgtgaaactgtgtgtcccttattaggtgt
ctcctattaggcagaaaggagttgtaaataagcaaaatagtggtaaaaatttttggtagcagtttagtagcacagcatcttcat
aatgctatatgtttttaaaatagtattttatccatagcacaaaggtgcctaagataagaatttctg
<212> Type : DNA
<211> Length : 236
      SequenceName : 5538589F6
      SequenceDescription :

Sequence
--------
<213> OrganismName : HUMAN
<400> PreSequenceString :
gaanaagtaacagacaccatcagcaggtgcctcactcctgatgtggaagctcgtccagatattgtagaagtcagttcgatgata
tcagatgtcatgatgaaatatttagacaacttatctacatcccagttgtccttggaaaagaagctagaacgggancgaagacgc
ncacaaaggtattttatggaagcc
<212> Type : DNA
<211> Length : 192
      SequenceName : 5546336H1
      SequenceDescription :

Sequence
--------
<213> OrganismName : HUMAN
<400> PreSequenceString :
gcccgtggggtgcaaaagatcctgcaggtgagtatattactatgtgggatcagtgtctcgaaagtcaaaaagagggcctgtggg
gactatatgaggactggatctttctttgtgatttgttttggagcaaggaaagttgaggctggcaattgctagtgagagctaatg
aggtctcttgagtttcc
<212> Type : DNA
<211> Length : 185
      SequenceName : 5560880H1
      SequenceDescription :

Sequence
--------
<213> OrganismName : HUMAN
<400> PreSequenceString :
gggtacctgcccatttctgggcttttacataaaaccctgtaagtgggttntggggacagctgtgtaacatgcacaaaaaataaa
tggaggaagaatgcattatctaaataggcactgacttgcgtgattgaagctgtaaccatcctttgcagcccagagttgctgtga
cctgtgtggagaggtgggagggccaggcagagagtgggtgtgaaagccacggaggatcccagggagtggattcctaagtaggag
gcgaggcaggtcatcagtgaggatcccagggagtggattcctaagtaggaggagaggcaggtcatcagtgaggatcccagggag
tggattcctaagtaggaggcgaggcaggtcatcagtgaggatcccagggagtggattcctaagtaggaggcgaggcaggtcatc
agtgaggatcccagggagtggctgcagtgactgtgcccagcaaacctccttaaggccctgccctggtgcctgcggctctaggag
attccctgggtagtcccagcacacgcaacctgttcag
<212> Type : DNA
<211> Length : 541
      SequenceName : 5771839H1
      SequenceDescription :

Sequence
--------
<213> OrganismName : HUMAN
<400> PreSequenceString :
ctgaataagcctctgatcccgcctgcatgggccagcccccctaagccatctgtgccctgcaggccacactgggatccaatgtccc
actctgaaccccagatatcaagctccacagcagaacagatttatagtaattctgaaacttttcaag

<212> Type : DNA
<211> Length : 150
      SequenceName : 5804485H1
      SequenceDescription :

Sequence
--------
<213> OrganismName : HUMAN
<400> PreSequenceString :
gcaggcccgtggctctcctgccctctgcatgcatcgcgagtactgccggaggaggcccctttggctttcagagcccagagcgcc
cgacctcctccaggagtctgcagatccacaggcctcagatgctgccacacccacccagcacacacccaggtctctgacacgcca
gccgcacacagcggtggggttcaggcgaaggtttcttccacagtgcagagtagaagagccgggaaggaccccatcaccatcaga
tgcatcagagcttcaaaggcccgcccatcaagacgctgccaaggagcatgaggccagccaggctggggaggacgttggcgagca
tccgcctgcccgtcagggagccacagctggatgatacatcgacagacagcagagggacggtgtagcacacaggcgagggcttgt
ccccctcagcctgggggagcgccgctcagtgcttggtggtcagcacgtgactgacactttgcc
<212> Type : DNA
<211> Length : 483
      SequenceName : 5825251H1
      SequenceDescription :

Sequence
--------
<213> OrganismName : HUMAN
<400> PreSequenceString :
gagccagggagagtgcttttgggctccagccccatggtaacgtctaggagtgtgttacaattaatgctcttttagtagttgcca
tctacagatggctaagtgtgtaagtcagagtgaagccttttacactctgccctcttgttacctgagtccttgtctggcatccag
gaaggataaggtcacacatggacttgagggtggtgaatgcagaggtttta
<212> Type : DNA
<211> Length : 218
      SequenceName : 5986963H1
      SequenceDescription :

Sequence
--------
<213> OrganismName : HUMAN
<400> PreSequenceString :
atttatttcagtcttctgtgtctggcttgtttggttttcagtgggtatgtttgattagagattgtttgtaatttacctgtttaa
tttctttttgttctcctgctatgtggctgcctcctttttggcactagatggtgctttaatccctggttcatcttggctctagt
aaacctttctcttgctctctcaatctctctctttctctctaatcttttatgttttctactaaattatgagcaccaggggccatg
ttattaggtattttttatgcttggtaaatatgtcttgataattacatgtgggtgtattgtataataagtatgatttaagtcac
caaggtaagggttcttaagctgggtcccataaactccttgacatgttgtgtaaagttgtatgttatatgcatgtatgcattttc
tggggtgtaggtctgtgactttcattactacctacttcatgttcatactgtggttaagaactatggtagcaagacaatatcaga
catattataaatact
<212> Type : DNA
<211> Length : 519
      SequenceName : 6530555H1
      SequenceDescription :

Sequence
--------
<213> OrganismName : HUMAN
<400> PreSequenceString :
acaaatctttctcttagaaaaatatgttcttaatattgcatgggatatacttatactaaaaaattatttgttgtttttctgaaa
ttgaaatctaactcggagtcctgtattttatctgagagccccagctggggacgggcgcggggccaagagagctgcgagggagga
ggggttgcaagcttctttcgggcggaggggtcacaaggcgaatggagaaggtgacaaggagcatggagaaaccaagaaaagggc
taacgcttggagagcaaggatggaaagacgctcaaagcggaggcggaacggtctgagtgattccaaaactagtgtgtccaagct
ggttagaactcaggcaggcgggtccagaagccgagggggcctgacagatacgcaggtgaagtccatctgtggtccgcgtgctggg
cgtgggcaccgggctagtccaagcattcctggtaaaggctcctccagagtggtaccacagcgctctattaa
<212> Type : DNA
<211> Length : 491
      SequenceName : 6712339H1
      SequenceDescription :

Sequence
--------
<213> OrganismName : HUMAN
<400> PreSequenceString :
ggaggcacaaaggtgaataaactcatcaaggagcttacagtttaatggggaagacagagacattatcaggtacttaaaaatat
gctgaagaccttagctaaagaaactttagaaatcaataatatggagttttccccaaaactaaaaatactctgcccccactgtgt
tcacaagatcagcatcaagcaaagattaatctcaagacttgttgtttttcttgttgggttttggacttacttgcaacccctttc
ttcttatttctcccttttggaatgggaacatctatcctatacatgtcccaccattgtattttggaagcacattaacttgtttga
tttcatgggttcacagctggagagcagtttgcctcaggatgaaacaaacctttgagtctcatctgtgtctggtttagatgagat
tctggtttttggacttttgagttggtgctggaagaagttaagact
<212> Type : DNA
<211> Length : 465
      SequenceName : AA774798
      SequenceDescription :

Sequence
--------
<213> OrganismName : HUMAN
<400> PreSequenceString :
ttttaaacatgaattttctaaatattatgccaagtaaaatgagctagacacaaagggacaaatatcagccaggcgctatggctc
aagcctgtaatctcagcacattgggagactgaggcaggtggattgcttggattgcttaagtccagaagtttgagactttacaag
aaatacatatattagccaggcatgcaccttgctaatagtcccagctacttgggaggctgagatggaaggatcaccttgagcctg
ggaggtcgaggatgcagtgagccgagattgcaccccctgcactccagcatcctgggaaacagagtaagactctgtcttaaacaaa
caaacaaccaaaaaaaaccccaaaaagctaagaaggctcggctgggcacggtggcacacgcctgtaagtcccaacacttcggga
aggccaaggtggatggatcacttgaggtcaggagtttgagaccagcttggccagctggtttgagaccagctcattttgtacttt
taggtagagat
<212> Type : DNA
<211> Length : 515
      SequenceName : AA007595
      SequenceDescription :

Sequence
--------
<213> OrganismName : HUMAN
<400> PreSequenceString :
ttgatggccaaacaggtttatttacaagaataagcctgtgaggggtcccagtcaggaatgtggctgagaccctgtcacttagaa
actgaggtttttataataaagtttctataggggagggtttgcgtaagtgctggcaggttggaactgttgggggctggagaagga
tgtggtaagggccatcaggctctgttgtggttagggttgttatgctctattaaaagtatgggcaaggttgacacctgctttgtt
tctgggaacaccgtcaccaaggtagaaaacagtcaccaatgtagagaaatggcatcgctcttgttagtccccacatttctttac
agcattgtgagaagggacgaatacaggagccatagatttcaagggtcctcccttacccctcctcagcccacgtctgcagggcct
ccgtgctgcaggcaaacataggcccctgaaggagatcacatcagacataggcagacaggcacatggtccaaataagtgcctgtc
caccagtggggacctgatgttccaggcccagtggctgca
<212> Type : DNA
<211> Length : 543
      SequenceName : AI939481
      SequenceDescription :

Sequence
--------
<213> OrganismName : HUMAN
<400> PreSequenceString :
aactttttaaaaagcaaatgtattcttcctccattatctaaaaaagattggcaaaaagttaaatctccttttacttcaaaagta
aagaaaatcatgcaatgagatgatcctgttgggccttcagcacagacccatcctctcttccccggctcccagcagtccagggca
ggtttctgtcctcagcaggctgagtttgttcagtccattcgttccttgctggatcagcacactctggatttctcagccctttc
agtctgagtgttcctccctcatggccttcacaagccagtcctgttctgtcccctgctcagagtcactggtgtcactcacctcca
tggacagcagcttcgagtacttgattttccgctctc
<212> Type : DNA
<211> Length : 372
      SequenceName : AI203269
      SequenceDescription :

Sequence
--------

<213> OrganismName : HUMAN
<400> PreSequenceString :
ttttttttttttttttttccggattgaggaactctctggaactgtctttcttcaagagagcatttcttataatacagatctgg
tagtggtgaattctctctgcttttatttgggaaaaactttatctcttcttcatatttgaataatagctttgcttgatacagtat
tcttgtatggccttcatggttttgattgagaagtctgtggccaggtaaattagagctcttttaagtgttatttccttcttttct
cttactgtttctaggatcctctctttgttcttgacctttgagagtttgattattatatgccttggggtaatcaaatctatttgg
tgttctctggcctttctgtacctggatatttgcaatttttgaat
<212> Type : DNA
<211> Length : 380
      SequenceName : AI015248
      SequenceDescription :

Sequence
--------
<213> OrganismName : HUMAN
<400> PreSequenceString :
ggggcatttccgggtccgggccgagcgggcgcacgcgcgggagcgggactcggcggcatggcgggctccggagccggtgtgcgt
tgctccctgctgcggctgcaggagaccttgtccgctgcggaccgctgcggtgctgccctggccggtcatcaactgatccgcggc
ctggggcaggaatgcgtcctgagcagcagccccgcggtgctggcattacagacatctttagtttttccagagatttcggtttg
cttgtatttgtccggaagtcactcaacagtattgaatttcgtgaatgtagagaagaaatcctaaagtttttatgtattttctta
gaaaaaatgggccagaagatcgcaccttactctgttgaaattaagaacacttgtaccagtgtttatacaaaagatagagctgct
aaatgtaaaattccagccctggaccttcttattaagttacttcagacttttagaagttctagactcatggatgaatttaaaatt
ggagaattatttagtaaattctatggagaacttgcattgaaaaaaaaaataccagatacagttttagaaaaagtatatgagctc
ctaggattattgggtgaagttcatcctagtgagatgataaataatgcagaaaacctgttccgcgcttttctgggtgaacttaag
acccagatgacatcagcagtaagagagcccaaactacctgttctggcaggatgtctgaaggggttgtcctcacttctgtgcaac
ttcactaagtccatggaagaagatccccagacttcaagggagattttaatttgtactaaaggcaattcgtcctcagattgat
ctgaagagatatgctgtgccctcagctggcttgcgcctatttgccctgcatgcatctcagtttagcacctgccttctggacaac
tacgtgtctctatttgaagtcttgttaaagtggtgtgcccacacaaatgtagaattgaaaaaagctgcactttcagccctggaa
tcctttctgaaacaggtttctaatatggtggcgaaaaatgcagaaatgcataaaaataaactgcagtactttatggagcagttt
tatggaatcatcagaaatgtggattcgaacaacaaggagttatctattgctatccgtggatatggactttttgcaggaccgtgc
aaggttataaacgcaaaagatgttgacttcatgtacgttgagctcattcagcgctgcaagcagatgttcctcacccagacagac
actggtgacgaccgtgtttatcagatgccaagcttcctccagtctgttgcaagcgtcttgctgtaccttgacacagttcctgag
gtgtatactccagttctggagcacctcgtggtgatgcagatagacagtttcccacagtacagtccaaaaatgcagctggtgtgt
tgcagagccatagtgaaggtgttcctagctttggcagcaaaagggccagttctcaggaattgcattagtactgtggtgcatcag
ggtttaatcagaatatgttctaaaccagtggtccttccaaagggccctgagtctgaatctgaagaccaccgtgcttcaggggaa
gtcagaactggcaaatggaaggtgcccacatacaaagactacgtggatctcttcagacatctcctgagctctgaccagatgatg
gattctattttagcagatgaagcatttttctctgtgaattcctccagtgaaagtctgaatcatttactttatgatgaatttgta
aaatccgttttgaagattgttgagaaattggatcttacacttgaaatacagactgttggggaacaagagaatggagatgaggcg
cctggtgtttggatgatcccaacttcagatccagcggctaacttgcatccagctaaacctaaagattttcggctttcattaac
ctggtggaattttgcagagagattctccctgagaaacaagcagaatttttgaaccatgggtgtactcattttcatatgaatta
attttgcaatctacaaggttgcccctcatcagtggtttctacaaattgctttctattacagtaagaaatgccaagaaaataaaa
tatttcgagggagttagtccaaagagtctgaaacactctcctgaagacccagaaaagtattcttgctttgctttatttgtgaaa
tttggcaaagaggtggcagttaaaatgaagcagtacaaagatgaacttttggcctcttgtttgacctttcttctgtccttgcca
cacaacatcattgaactcgatgttagagcctacgttcctgcactgcagatggctttcaaactgggcctgagctataccccttg
gcagaagtaggcctgaatgctctagaagaatggtcaatttatattgacagacatgtaatgcagccttattacaaagacattctc
ccctgcctggatggatacctgaagacttcagccttgtcagatgagaccaagaataactgggaagtgtcagctctttctcgggct
gcccagaaaggatttaataaagtggtgttaaagcatctgaagaagacaaagaacctttcatcaaacgaagcaatatccttagaa
gaaataagaattagagtagtacaaatgcttggatctctaggaggacaaataaacaaaaatcttctgacagtcacgtcctcagat
gagatgatgaagagctatgtggcctgggacagagagaagcggctgagctttgcagtgccctttagagagatgaaacctgtcatt
ttcctggatgtgttcctgcctcgagtcacagaattagcgctcacagccagtgacagacaaactaaagttgcagcctgtgaactt
ttacatagcatggttatgtttatgttgggcaaagccacgcagatgccagaaggggggacagggagccccacccatgtaccagctc
tataagcggacgtttcctgtgctgcttcgacttgcgtgtgatgttgatcaggtgacaaggcaactgtatgagccactagttatg
cagctgattcactggttcactaacaacaagaaatttgaaagtcaggatactgttgccttactagaagctatattggatggaatt
gtggaccctgttgacagtactttaagagattttgtggtcggtgtattcgagaattccttaaatggtccattaagcaaataaca
ccacagcagcaggagaagagtccagtaaacaccaaatcgcttttcaagcgactttatagccttgcgcttcaccccaatgctttc
aagaggctgggagcatcacttgcctttaataatatctacagggaattcagggaagaagagtctctggtggaacagtttgtgttt
gaagccttggtgatatacatggagagtctggccttagcacatgcagatgagaagtccttaggtacaattcaacagtgttgtgat
gccattgatcacctatgccgcatcattgaaaagaagcatgtttctttaaataaagcaaagaaacgacgtttgccgcgaggattt
ccaccttccgcatcattgtgtttattggatctggtcaagtggcttttagctcattgtgggaggccccagacagaatgtcgacac
aaatccattgaactcttttataaattcgttcctttattgccaggcaacagatcccctaatttgtggctgaaagatgttctcaag
gaagaaggtgtctcttttctcatcaacacctttgagggggtggctgtggccagccctcgggcatcctggcccagcccacccctc
ttgtaccttcgggggccattcagcctgcaggccacgctatgctggctggacctgctcctggccgcgttggagtgctacaacacg
ttcattggcgagagaactgtaggagcgctccaggtcctaggtactgaagcccagtcttcacttttgaaagcagtggctttcttc

ttagaaagcattgccatgcatgacattatagcagcagaaaagtgctttggcactggggcagcaggtaacagaacaagcccacaa
gagggagaaaggtacaactacagcaaatgcaccgttgtggtccggattatggagtttaccacgactctgctaaacacctccccg
gaaggatggaagctcctgaagaaggacttgtgtaatacacacctgatgagagtcctggtgcagacgctgtgtgagcccgcaagc
ataggtttcaacatcggagacgtccaggttatggctcatcttcctgatgtttgtgtgaatctgatgaaagctctaaagatgtcc
ccatacaaagatatcctagagacccatctgagagagaaaataacagcacagagcattgaggagctttgtgccgtcaacttgtat
ggccctgacgcgcaagtggacaggagcaggctggctgctgttgtgtctgcctgtaaacagcttcacagagctgggcttctgcat
aatatattaccgtctcagtccacagatttgcatcattctgttggcacagaacttctttccctggtttataaaggcattgcccct
ggagatgagagacagtgtctgccttctctagacctcagttgtaagcagctggccagcggacttctggagttagcctttgctttt
ggaggactgtgtgagcgccttgtgagtcttctcctgaacccagcggtgctgtccacggcgtccttgggcagctcacagggcagc
gtcatccacttctcccatggggagtatttctatagcttgttctcagaaacgatcaacacggaattattgaaaaatctggatctt
gctgtattggagctcatgcagtcttcagtggataataccaaaatggtgagtgccgttttgaacggcatgttagaccagagcttc
agggagcgagcaaaccagaaacaccaaggactgaaacttgcgactacaattctgcaacactggaagaagtgtgattcatggtgg
gccaaagattcccctctcgaaactaaaatggcagtgctggccttactggcaaaaattttacagattgattcatctgtatctttt
aatacaagtcatggttcattccctgaagtctttacaacatatattagtctacttgctgacacaaagctggatctacatttaaag
ggccaagctgtcactcttcttccattcttcaccagcctcactggaggcagtctggaggaacttagacgtgttctggagcagctc
atcgttgctcacttccccatgcagtccagggaatttcctccaggaactccgcggttcaataattatgtggactgcatgaaaaag
tttctagatgcattggaattatctcaaagccctatgttgttggaattgatgacagaagttctttgtcgggaacagcagcatgtc
atggaagaattatttcaatccagtttcaggaggattgccagaaggggttcatgtgtcacacaagtaggccttctggaaagcgtg
tatgaaatgttcaggaaggatgacccccgcctaagtttcacacgccagtcctttgtggaccgctccctcctcactctgctgtgg
cactgtagcctggatgctttgagagaattcttcagcacaattgtggtggatgccattgatgtgttgaagtccaggtttacaaag
ctaaatgaatctacctttgatactcaaatcaccaagaagatgggctactataagattctagacgtgatgtattctcgccttccc
aaagatgatgttcatgctaaggaatcaaaaattaatcaagttttccatggctcgtgtattacagaaggaaatgaacttacaaag
acattgattaaattgtgctacgatgcatttacagagaacatggcaggagagaatcagctgctggagaggagaagactttaccat
tgtgcagcatacaactgcgccatatctgtcatctgctgtgtcttcaatgagttaaaattttaccaaggttttctgtttagtgaa
aaaccagaaaagaacttgcttattttgaaaatctgatcgacctgaagcgccgctataattttcctgtagaagttgaggttcct
atggaaagaaagaaaaagtacattgaaattaggaaagaagccagagaagcagcaaatggggattcagatggtccttcctatatg
tcttccctgtcatatttggcagacagtaccctgagtgaggaaatgagtcaatttgatttctcaaccggagttcagagctattca
tacagctcccaagaccctagacctgccactggtcgttttcggagacgggagcagcgggacccacggtgcatgatgatgtgctg
gagctggagatggacgagctcaatcggcatgagtgcatggcgcccctgacggccctggtcaagcacatgcacagaagcctgggc
ccgcctcaaggagaagaggattcagtgccaagagatcttccttcttggatgaaattcctccatggcaaactgggaaatccaata
gtaccattaaatatccgtctcttcttagccaagcttgttattaatacagaagaggtctttcgcccttacgcgaagcactggctt
agccccttgctgcagctggctgcttctgaaaacaatggaggagaaggaattcactacatggtggttgagatagtggccactatt
ctttcatggacaggcttggccactccaacaggggtccctaaagatgaagtgttagcaaatcgattgcttaatttcctaatgaaa
catgtctttcatccaaaaagagctgtgtttagacacaaccttgaaattataaagacccttgtcgagtgctggaaggattgttta
tccatcccttataggttaatatttgaaaagttttccggtaaagatcctaattctaaagacaactcagtagggattcaattgcta
ggcatcgtgatggccaatgacctgcctccctatgacccacagtgtggcatccagagtagcgaatacttccaggctttggtgaat
aatatgtcctttgtaagatataaagaagtgtatgccgctgcagcagaagttctaggacttatacttcgatatgttatggagaga
aaaaacatactggaggagtctctgtgtgaactggttgcgaaacaattgaagcaacatcagaatactatggaggacaagtttatt
gtgtgcttgaacaaagtgaccaagagcttccctcctcttgcagacaggttcatgaatgctgtgttctttctgctgccaaaattt
catggagtgttgaaaacactctgtctggaggtggtactttgtcgtgtggagggaatgacagagctgtacttccagttaaagagc
aaggacttcgttcaagtcatgagacatagagatgatgaaagacaaaaagtatgtttggacataatttataagatgatgccaaag
ttaaaaccagtagaactccgagaacttctgaaccccgttgtggaattcgtttcccatccttctacaacatgtagggaacaaatg
tataatattctcatgtggattcatgataattacagagatccagaaagtgagacagataatgactcccaggaaatatttaagttg
gcaaaagatgtgctgattcaaggattgatcgatgagaaccctggacttcaattaattattcgaaatttctggagccatgaaact
aggttaccttcaaataccttggaccggttgctggcactaaattccttatattctcctaagatagaagtgcactttttaagttta
gcaacaaattttctgctcgaaatgaccagcatgagcccagattatccaaaccccatgttcgagcatcctctgtcagaatgcgaa
tttcaggaatataccattgattctgattggcgtttccgaagtactgttctcactccgatgtttgtggagacccaggcctcccag
ggcactctccagacccgtacccaggaagggtccctctcagctcgctggccagtggcagggcagataagggccacccagcagcag
catgacttcacactgacacagactgcagatggaagaagctcatttgattggctgaccgggagcagcactgacccgctggtcgac
cacaccagtccctcatctgactccttgctgtttgcccacaagaggagtgaaaggttacagagagcacccttgaagtcagtgggg
cctgattttgggaaaaaaaggctgggccttccaggggacgaggtggataacaaagtgaaaggtgcggccggccggacggaccta
ctacgactgcgcagacggtttatgagggaccaggagaagctcagtttgatgtatgccagaaaaggcgttgctgagcaaaaacga
gagaaggaaatcaagagtgagttaaaaatgaagcaggatgcccaggtcgttctgtacagaagctaccggcacggagaccttcct
gacattcagatcaagcacagcagcctcatcaccccgttacaggccgtggcccagagggacccaataattgcaaaacagctcttt
agcagcttgttttctggaattttgaaagagatggataaatttaagacactgtctgaaaaaaacaacatcactcaaaagttgctt
caagacttcaatcgttttcttaataccaccttctctttctttccaccctttgtctcttgtattcaggacattagctgtcagcac
gcagccctgctgagcctcgacccagcggctgttagcgctggttgcctggccagcctacagcagcccgtgggcatccgcctgcta
gaggaggctctgctccgcctgctgcctgctgagctgcctgccaagcgagtccgtgggaaggcccgcctccctcctgatgtcctc
agatgggtggagcttgctaagctgtatagatcaattggagaatacgacgtcctccgtgggattttaccagtgagataggaaca
aagcaaatcactcagagtgcattattagcagaagccagaagtgattattctgaagctgctaagcagtatgatgaggctctcaat
aaacaagactgggtagatggtgagcccacagaagccgagaaggattttgggaacttgcatcccttgactgttacaaccacctt
gctgagtggaaatcacttgaatactgttctacagccagtatagacagtgagaacccccccagacctaaataaaatctggagtgaa
ccatttatcaggaaacatatctaccttacatgatccgcagcaagctgaagctgctgctccagggagaggctgaccagtccctg

ctgacatttattgacaaagctatgcacggggagctccagaaggcgattctagagcttcattacagtcaagagctgagtctgctt
tacctcctgcaagatgatgttgacagagccaaatattacattcaaaatggcattcagagttttatgcagaattattctagtatt
gatgtcctcttacaccaaagtagactcaccaaattgcagtctgtacaggctttaacagaaattcaggagttcatcagctttata
agcaaacaaggcaatttatcatctcaagttccccttaagagacttctgaacacctggacaaacagatatccagatgctaaaatg
gacccaatgaacatctgggatgacatcatcacaaatcgatgtttctttctcagcaaaatagaggagaagcttacccctcttcca
gaagataatagtatgaatgtggatcaagatggagaccccagtgacaggatggaagtgcaagagcaggaagaagatatcagctcc
ctgatcaggagttgcaagttttccatgaaaatgaagatgatagacagtgcccggaagcagaacaatttctcacttgctatgaaa
ctactgaaggagctgcataaagagtcaaaaaccagagacgattggctggtgagctgggtgcagagctactgccgcctgagccac
tgccggagccggtcccagggctgctctgagcaggtgctcactgtgctgaaaacagtctctttgttggatgagaacaacgtgtca
agctacttaagcaaaaatattctggctttccgtgaccagaacattctcttgggtacaacttacaggatcatagcgaatgctctc
agcagtgagccagcctgccttgctgaaatcgaggaggacaaggctagaagaatcttagagctttctggatccagttcagaggat
tcagagaaggtgatcgcgggtctgtaccagagagcattccagcacctctctgaggctgtgcaggcggctgaggaggaggcccag
cctccctcctggagctgtgggcctgcagctggggtgattgatgcttacatgacgctggcagatttctgtgaccaacagctgcgc
aaggaggaagagaatgcatcagttattgattctgcagaactgcaggcgtatccagcacttgtggtggagaaaatgttgaaagct
ttaaaattaaattccaatgaagccagattgaagtttcctagattacttcagattatagaacggtatccagaggagactttgagc
ctcatgacaaaagagatctcttccgttccctgctggcagttcatcagctggatcagccacatggtggccttactggacaaagac
caagccgttgctgttcagcactctgtggaagaaatcactgataactacccgcaggctattgtttatcccttcatcataagcagc
gaaagctattccttcaaggatacttctactggtcataagaataaggagtttgtggcaaggattaaaagtaagttggatcaagga
ggagtgattcaagatttattaatgccttagatcagctctctaatcctgaactgctctttaaggattggagcaatgatgtaaga
gctgaactagcaaaaacccctgtaaataaaaaaaacattgaaaaaatgtatgaaagaatgtatgcagccttgggtgacccaaag
gctccaggcctgggggcctttagaaggaagtttattcagacttttggaaaagaatttgataaacattttgggaaaggaggttct
aaactactgagaatgaagctcagtgacttcaacgacattaccaacatgctacttttaaaaatgaacaaagactcaaagcccccct
gggaatctgaaagaatgttcaccctggatgagcgacttcaaagtggagttcctgagaaatgagctggagattcccggtcagtat
gacggtaggggaaagccattgccagagtaccacgtgcgaatcgccgggtttgatgagcgggtgacagtcatggcgtctctgcga
aggcccaagcgcatcatcatccgtggccatgacgagagggaacaccctttcctggtgaagggtggcgaggacctgcggcaggac
cagcgcgtggagcagctcttccaggtcatgaatgggatcctggcccaagactccgcctgcagccagagggccctgcagctgagg
acctatagcgttgtgcccatgacctccaggttaggattaattgagtggcttgaaaatactgttaccttgaaggaccttcttttg
aacaccatgtcccaagaggagaaggcggcttacctgagtgatcccagggcaccgccgtgtgaatataaagattggctgacaaaa
atgtcaggaaaacatgatgttggagcttacatgctaatgtataagggcgctaatcgtactgaaacagtcacgtcttttagaaaa
cgagaaagtaaagtgcctgctgatctcttaaagcgggccttcgtgaggatgagtacaagccctgaggctttcctggcgctccgc
tcccacttcgccagctctcacgctctgatatgcatcagccactggatcctcgggattggagacagacatctgaacaactttatg
gtggccatggagactggcggcgtgatcgggatcgactttgggcatgcgtttggatccgctacacagtttctgccagtccctgag
ttgatgccttttcggctaactcgccagtttatcaatctgatgttaccaatgaaagaaacgggccttatgtacagcatcatggta
cacgcactccgggccttccgctcagaccctggcctgctcaccaacaccatggatgtgtttgtcaaggagccctcctttgattgg
aaaaatttttgaacagaaaatgctgaaaaaaggagggtcatggattcaagaaataaatgttgctgaaaaaaattggtaccccga
cagaaaatatgttacgctaagagaaagttagcaggtgccaatccagcagtcattacttgtgatgagctactcctgggtcatgag
aaggcccctgccttcagagactatgtggctgtggcacgaggaagcaaagatcacaacattcgtgcccaagaaccagagagtggg
ctttcagaagagactcaagtgaagtgcctgatggaccaggcaacagaccccaacatccttggcagaacctgggaaggatgggag
ccctggatgtgaggtctgtgggagtctgcagatagaaagcattacattgtttaaagaatctactatactttggttggcagcatt
ccatgagctgatttcctgaaacactaaagagaaatgtcttttgtgctacagtttcgtagcatgagtttaaatcaagattatga
tgagtaaatgtgtatgggttaaatcaaagataaggttatagtaacatcaaagattaggtgaggtttatagaaagatagatatcc
aggcttaccaaagtattaagtcaagaatataatatgtgatcagctttcaaagcatttacaagtgctgcaagttagtgaaacagc
tgtctccgtaaatggaggaaatgtggggaagccttggaatgcccttctggttctggcacattggaaagcacactcagaaggctt
catcaccaagattttgggagagtaaagctaagtatagttgatgtaacattgtagaagcagcataggaacaataagaacaatagg
taaagctataattatggcttatatttagaaatgactgcatttgatattttaggatatttttctaggtttttcctttcattta
ttctcttctagttttgacattttatgatagatttgctctctagaaggaaacgtctttatttaggagggcaaaaattttggtcat
agcattcacttttgctattccaatctacaactggaagatacataaaagtgctttgcattgaatttgggataacttcaaaaatcc
catggttgttgttagggatagtactaagcatttcagttccaggagaataaaagaaattcctatttgaaatgaattcctcatttg
gaggaaaaaaagcatgcattctagcacaacaagatgaaattatggaatacaaaagtggctccttcccatgtgcagtccctgtcc
ccccccgccagtcctccacacccaaactgtttctgattggcttttagcttttgttgtttttttttttccttctaacacttgta
tttggaggctcttctgtgattttgagaagtatactcttgagtgtttaataaagtttttttccaaaagta
<212> Type : DNA
<211> Length : 13509
      SequenceName : U47077
      SequenceDescription :

Sequence
--------
<213> OrganismName : HUMAN
<400> PreSequenceString :
aatgctaatgataacccactaagctgatttgtgacccattaattgtcttgatccagtttgaaaaactctgaattataggctgt
gagcactcgctattgttagggttaggataatctaataagcatgaggtaatggctgatgcaaggaaaagactggttaatccagaa
tggctgataatttttcaaaagggaaacaaagctggcaataaagggaatggttagcaggaaagcaataattgaagacagcaac

agatgtaggtttgttgggagcgtgaggctccaggaataaataggtggagagggcaatgggcatggtggggctctcacccggatg
ggnatgatgtggctggggcaggggatgacaggaaggcccctgtccatgagtagactggccatgtgcttgacattgcgctggtgg
gctcgctcagggcttggacacctctcccttgagcagccgccagattctagagctccagaga
<212> Type : DNA
<211> Length : 481
      SequenceName : AA699919
      SequenceDescription :

Sequence
--------
<213> OrganismName : HUMAN
<400> PreSequenceString :
attattaaaaataataataaatatggaacacatcataaatttatgtgtcatccttgtgcagggaccatgaaaatcttctctgca
tcgttccaattttagtatatgtgctgctgaagcaagcactatactactaattatttttcctctgtttggatacatgggaagtg
ctgtggaaataaaggattccaggagtcagaaggggctatacatccaaaaccgttatcataatgagaagaattgacctaattcca
aagttcagtactgtaattttacaaccacctcagtctctacatgcacagaatgggtgcattaaagacaggtatcacagtgtcatc
tacccacctcctttaaagaatcactactataggctgggcgcagtggcttatacctgtaatctcagcactttgggagggcaaggt
gggcagatcacttgaagtcagaagttggagaccagcctagcc
<212> Type : DNA
<211> Length : 462
      SequenceName : AA864819
      SequenceDescription :

Sequence
--------
<213> OrganismName : HUMAN
<400> PreSequenceString :
agaaaaaactggctgcttctaagtgcgaagccggctgggcgggctggtgccagaaagggtgtgtcttcactgccctaagatggc
ctttaaggtatacttttgagggctttcttgggccactttccgtaaacacaggatgcaccgatggcaactggagttgtcatccga
tggtggggagaggcgtgcagatttttttgaagggacgagccctgaaggaggataggcagacccaccagggcctcagggtacaa
gaaacccgaaaaaggactggaggctgcaggcaatacggtggtaggaatctctgccggtttccagcgtcgcggaagcaccaaagg
gcggtccctgaaggaaacgaggggtcttggggctggagagcggcgcggagcgtggtgggaatatcgtcacaacgccgctgtcct
acatttctgtagcgaagacttgattacccctagctattacccgtgcaggagactgggccctggaccccaaatgagccaaagtct
agttgagaaaaatttacacagaataacctcggagttcatg
<212> Type : DNA
<211> Length : 544
      SequenceName : AI264247
      SequenceDescription :

Sequence
--------
<213> OrganismName : HUMAN
<400> PreSequenceString :
actttagacaaaaatatttatttttttttgacttgttattttgagagtataaaagtgatcatatttcctctcttaatgaga
gcttatatgtaattgcctgctgtcagtgaatcttgggtttaaggcccattagaaaaatagcaggctttagtagagtggtttttt
ccagtgagtacttttcagtcaacaggtttattgggaggtggctaaaatgcacatggaggtgagcccttccctgccagacaagca
aagaatcaagctgtatatttaaaacttggctgttaacccaaaggatttctacaggaagtttttttctcctttgatgtttgtaga
ggggtttttttcccctcccgacaaaatcaatacacagaaaacccaaagctcttaatttgcaaaactgaatggatttgtgaaaa
tagatacctttttggcagaaatgcaatttggcattaatccct
<212> Type : DNA
<211> Length : 462
      SequenceName : AA284067
      SequenceDescription :

Sequence
--------
<213> OrganismName : HUMAN
<400> PreSequenceString :
tttttttttttcaacctgacctgaggcatcaattcaggcacagtataactctgaccagtaagcggaagtaacccaaatgcttt
ccagggctgaggggacatcatgagagttgtggtcatgtacagaagcacagtcccaggggcacaggtggtacccctgtaaacaaa
gaatttgcaatattagggcacctcaagcaggacatccattaggctgtacaggtgaacagacctcctaccaggggaactcctgcc
caagtgtttccagtcccatctgaaaagttcccagagtccttggcttccaagggactgcctgaaggcagtgagtcccaaacagtt
ttgcttgttcaggatacaagttgatccatctcatgagcatggatgacatttgtgggagatcttcatggaaagcatagaagctag

acccactctgttgtcagtctgaactcaaagcactcacagcacatggaaaggcagcactgggaattttccttcagcaagaggagg
cttccagggatcaagcctccccaggtgccaggctttaattatttctccaatattacaaaaacagtcgtgccccgttcagcaat
cataacattgcatttttcaaccatctcttactctcagctagggcctttctctggaagggttgtatgtaagaggcacaaaagca
ttcttgtagataaactttttttttttttttggagaaagggtcggcctgttggcccagccagagtggcgtggcaaatttg
ggctactggcaaccccgcccctggg
<212> Type : DNA
<211> Length : 782
      SequenceName : AA714835
      SequenceDescription :

Sequence
--------
<213> OrganismName : HUMAN
<400> PreSequenceString :
ggggtgagagggaagtagaaagggcaggaagcaggatcaccaaggggcaacaaggaaatttttgggaggatggctttcttcac
tatcttgattgtgataatgatttcagctgtatactaaggctcagatattatcaaattgtaatctctaggcagtttattatatgt
caatacctcaataaatctgcctaaaatgttcaaaaattactgtaattacacaaaatacattttataaaaggaagtcatgagtg
gagcatggttgagaaccccagcatctggtagattcagaagtggggaaaagcatctctcacttccgctttttcaggccggcgtgc
tgtgcaatttatccagtggc
<212> Type : DNA
<211> Length : 356
      SequenceName : AA961061
      SequenceDescription :

Sequence
--------
<213> OrganismName : HUMAN
<400> PreSequenceString :
gtaccggttcggaattcccgggtcgacccacgcgtccggaaggctacagagagagccaggttttggtgccatgcacacagggaa
acttagagttcagagaggggtgtgatttgcctgacctcacacagcaagttagagacccagctccacgactcattgtcttgctg
cccagagctgctggctcccctgtttactctgagctgatcgatcaccttagcacacagctggctaggagagaaccatgcagtcac
ttcggccacacctgcccgttgacccttgctacctcggcaggctttgatcccttctgacctggaggccagaggctaggctgaggt
cactcagcagacatcaaggacctgggcagatgggccggctgggatggtggcgagctgtacagataaaaagggacatgaaaatga
aaagcccgagcctgagttttcatcacggttccactcctgagtggtcttgggtgaatcacttcatctgccaaggcctggatttcc
tcatctgcaaactcagaaaactaaggctttggccctcgtcatcctgcccacccagcggggcttcccaacccaccacacagccat
ggacgggaaggtggcagtgcaagagcgtgggcctcctgcggtctcctgggtccccgaggagggagagaagttggaccaggaaga
cgaggaccaggtgaaggatcggggccaatggaccaacaagatggagtttgtgctgtcagtggccggggagatcattggctggg
caatgtctggaggtttccctatctctgctacaaaaacggaggtggagccttcttcatcccctacttcatcttcttctttgtctg
cggcatcccggtgttcttcctggaggtggcgttgggccaatacaccagccaagggagtgtcacagcctggaggaagatctgccc
cctcttccagggcattggtctggcatctgtggtcatcgagtcatatttgaatgtctactacatcatcatccttgcctgggctct
cttctacctgttcagctccttcacttctgagctgccctggacgacctgcaacaactttggaacacagagcattgcacggactt
tctgaaccactcaggagccggcacagtgaccccatttgagaattttacctcacctgtcatggaattctgggagagacgagttct
gggcatcacctcgggcatccatgacctgggctccctgcgctgggagctggccctgtgcctcctgctcgcctgggtcatctgcta
tttctgcatctggaaggggggtcaagtccacaggcaaggtggtttatttcacagccacgtttccgtacctgatgcttgtcatttt
gctgatcagaggtgtcacccttcccggagcctaccagggcatcatctactacttgaagccagatttgttccgcctcaaggaccc
tcaggtgtggatggatgcgggcacccagatcttcttctcctttgccatctgccaggggtgcctgacagccctgggcagctacaa
caagtatcacaacaactgctacaaggactgcatcgccctctgcttcctgaacagtgccaccagctttgtggctgggtttgttgt
cttctccatcctgggcttcatgtcccaagagcaaggggtgcccatttctgaagtggccgagtcaggtcctggctggccttcat
cgccttccccaaggctgtgactatgatgcccttatcccagctgtggtcctgcctgttctttatcatgctcatattcctagggct
ggacagccagtttgtctgtgtggagtgcctggtgacagcctccatagacatgttccccaggcagctccggaagagcgggcggcg
cgagctcctcatcctcaccatcgccgtcatgtgctacctgatagggcttttcctggtcaccgagggcgggatgtacatcttcca
gctgtttgactactatgcttccagtggcatatgcctgctgttcctgtcattgtttgaagtggtctgcataagctgggtgtatgg
ggcggaccgtttctatgacaacattgaggacatgattggctaccggccatggcccctggtgaagatctcctggctcttcctgac
ccctggactttgcctggccactttcctcttctccttgagcaagtacacccccctcaagtacaacaacgtctatgtgtacccgcc
ctggggatactccattggctggttcctggctctgtcctccatggtctgtgtcccactcttcgtcgtcatcaccctcctgaagac
tcggggtcctttcaggaagcgtctgcgtcacgtcatcacccctgactccagtctgccacagcccaagcaacatccctgcttgga
tggcagtgctggccggaactttgggccctccccaacaagggaaggactgatagccggggagaaggagacccatttgtagggtgt
ggccagagcgaggcggctcctaagccgggaacctaggtcagggccaccctccattctcagcggacagcctctgcctctgtctcc
tgccacaatcctgctgggaactctggagagccacaggcacccccagctggaggccagactcctctcttgtgctagctggagcag
ctccttcccctttgctgataacaccaccactgggacgtgccatgttgggacgccactccctgtggaaggcaccatcgtttttat
aaagggggtctttttggaggccgccatctgattgcaacacctcgagttatgaggattccactgtggggatgcctcttgttaga
gcgtactgcatttgtacacggggagaggagctataattggaacgcacactgccgtccaatgtggagagcctgatgggacaatac
cctgttggaagtgacaactgaacacactgtgttggatcggaggttccgttaggggatccttccttaggcttaacgacagaggca

agcctttgcatgccgtcagtctggagtttcctccgagtctctcatggcatctccagctcctgccctagttccgcactgttcttg
cagtgtttcatcaactcctggagcattggaatggaaggggcttgggagatgattcctagacttcacaaacactcggcatgcctc
cctgcactgtccgttcctctgcccaaggccgatattgctaactgatcacagattctttcccacctcacaatcctccgaatgtgc
tccaggcgacaccatttgccatcctgcttctaacgcaaacccctgacttcatggatgaggaacctggagaccaaagagacaaag
ggactttttcaagttcacatggggacccccttcttgggggccagagatatgactaaaaccttatctccttgtgctcaggccagt
gtcttcccattaacccccctgccttagttaacaagtgtgtatggattgcca
<212> Type : DNA
<211> Length : 3410
      SequenceName : U27699
      SequenceDescription :

Sequence
--------
<213> OrganismName : HUMAN
<400> PreSequenceString :
tttttttttttttttttttgaactcgagattattttattatagtcaatgtttttcacacacacacacatatatatacacacac
acacacatctaaaatgagtcagattctctgattactcttatgttcattatgtaaactccagtttagaatatttcatctatttaa
tctgcaatttctagtatattggcataggtttgccgcctgttccattgttagttttggaaaacgtgtgtacgatctgtagggctg
tctgctggctcactcccatatggaaatgcatgcctgctctttctttctctttttcattgtagttagaattcatgagtgtgattc
atgttttcaagaatgagcttcactggctttgttgaattttcaagctttggtttttcctcatgaacaactcctcttattattgtt
attattccctttcttacgccttcatttggaataa
<212> Type : DNA
<211> Length : 454
      SequenceName : AA737674
      SequenceDescription :

Sequence
--------
<213> OrganismName : HUMAN
<400> PreSequenceString :
tttgtattcaattttattggatataaattccacatcagtaaacctgattctcttaaaaatctacacagaaaaaaaaaaagagag
agaggttgagttgggtagttgttggtatttttgtttttgtttctttgcttattcagttgaggtttattgtaaaacttgacacca
gaaaaaggaagaagtggcgtttttgtgctgtgagcgtgtgacgtgtgtcctttccagagaaaggacagtcatggtgcttttta
tcctctctgcccaaagaaggaaagctcttaacagccagcaggaggctttgtagggaccagcgttatcancgccagtcgcgctga
cca
<212> Type : DNA
<211> Length : 339
      SequenceName : AI148008
      SequenceDescription :

Sequence
--------
<213> OrganismName : HUMAN
<400> PreSequenceString :
tttggattatatcataagtgtaataggtgacagcctgctgaaacattttaggggcattccttgtacaaaacgtacacataaatt
aaaaaatcaatttagctttctacaacggtggatgccaacccaaacacatttccagggagaatatttcagttagatgtagatctt
cttcatcttgcaaattgacttctgaggtacatgacagtgaatgtcattccagccccattcgtctgaagaacggaaatttattat
cgcacaacgctcatcaaggttattgggttcacctgagtgccagaatgtgacaagttcattgtatggtgtctggtcaacccattg
ccaatgtcgccgacccccctggatctgacagccccagaaaataagaagaatttcttttcagattctgaatgatgaaatcctgttc
ttccctgggggtgatcaccaccagatcagctcacatcacagaagaggtgctttgactcttagtccaagatgcatcccagtagaa
atatagtagcaactaggactgaatgaagtccaggggttgggcagcagctccaatcttctatgtacttatcttacaggacgcagg
tcggcttgagtgagaccgttgaactccgtaacttggacgacctctggacgttttgcatacataaaattgtgaggcactcggtcc
tgaggctctcttcaggcaacaatgtgtgggcgcacacacttggacattctctatcaggtgggtgcgaagcgactggtaaaactt
tctccaaagatggtgacttgcggagttgtggttgtatgctaaacaggccaagcctcgagagcccttcgcgtctaggcctgaggc
angtaggggcatcgcacaccaatgtcaggtcagcacgagagtagcaacttggaggagaagttgatacgtgggagcacgtgactg
ccagcgcggtagttacgagcatatagaatgggtacgctcgaaggttacgacggtttcgtcgcgcaggcgtgtgggtgtctgcta
gagtc
<212> Type : DNA
<211> Length : 1013
      SequenceName : AA868502
      SequenceDescription :

Sequence

--------
<213> OrganismName : HUMAN
<400> PreSequenceString :
gagtgagccagatgaagaggagctttattgagtgacagaacagctctgaggaaacccacagggggcagctcctttctgcagcc
agggtgtcccaacgtctgttcagctcctagcagaaagggttaggcaagtcgtcccaacaagtgttcagctatcagtagagaggg
tagctcctctctgcagctggtcgtcccatcatctccctgtcatctttccatcatctctgcagctttcagcaaagagggggccct
agagtgggttgctcctgtctgcaggtggtcatcccaacatttgcccagctctggctgagcctggggcttttatgggcctcaaag
gggaggaagtgcatgctgtgccaactggtccaagggtggccatgggcaggcccgaaaaaggcaccataagttcccactccagtc
cacaggactggcagcctggcccccagccttcaagcccttcttggcctgaaggtggggcctcaccaa
<212> Type : DNA
<211> Length : 486
      SequenceName : AI339252
      SequenceDescription :

Sequence
--------
<213> OrganismName : HUMAN
<400> PreSequenceString :
ctttttcgctctatgccctcctggccccttctccactttccatggtccctccaactcaaagaactcaattatctgcctgcaga
tttccccttcccctcctcgcagcctgccctaaacacaccctcctcccaagcccatgcagaggcacagaaggaaggccaagccag
ggctcggaagggctgctgccaggcctcctccagcctcaactatgtgttccccgggcttggctgcctcggccgactctcttaggc
ccaacgatggaaatggggacgtttccagggcaatgacagttaacagctaagaggagtgacaggaaggaagatgtattcttcaat
ccttcagtcccaactgccccacagggaggtgcttcagaaggcccaggcctcagagcctcccatctccctgaggtgccaggtgga
cctcccgtaatgcagttcttagaatcag
<212> Type : DNA
<211> Length : 448
      SequenceName : BE222911
      SequenceDescription :

Sequence
--------
<213> OrganismName : HUMAN
<400> PreSequenceString :
atctttttgtcaatttattattattatgggctcatgaattcctattccattcaatgtataatctgttactatcatctattttga
tggtctaatagtcccagattaccttggaaaccattatgcaatgatttcttagatatgacaccaaaagcacatgcaacaaaagga
aaaatagataaattgcatatcatcaaaattaaaaactttggggcttcaaagaacaccaccaagaaagtgaaaggcaacccacag
aatgggaaaaaatatctgccatatatctgctgctggtctagtatccagaatatataaagaacacttccaactcagtaataaaaa
gaaaactcaattttaaactgggcaaaagtttgggaaaatagctattttcccaaagatatacaatggccaataagcacatgaaag
taaaatcaacattcataaccatccaggaaatgccaatcnaaaccacaaaatggataccagttcacacccactaggagaactttt
gtccaggaa
<212> Type : DNA
<211> Length : 513
      SequenceName : N36113
      SequenceDescription :

Sequence
--------
<213> OrganismName : HUMAN
<400> PreSequenceString :
tggcaatgaaagaaggcaagtttattagagcaacagtgtatggcaaagtggctgctccccagagcagggttctttcataggcag
agctgcccagagcacagcagcagcagcagcagcagtccttatggattgctggctagctatatttatacacagtcttcgttatat
gctatttaaggagtaggttattcagaattttctagaaaaggggtgggggagttcctggaaccatataaggaaacttctgggttg
ttgccatggcatttgtaaactgtcatggcactggtgggactgtctttatgcttattagcagtgagggcaagagggtcactttg
gttgctatccgctggtttcggctggtgtctttgctgcatcctgctttgattagtagggtcatgatcggtgctcggaatataagt
<212> Type : DNA
<211> Length : 420
      SequenceName : AA814523
      SequenceDescription :

Sequence
--------
<213> OrganismName : HUMAN
<400> PreSequenceString :

tttttttttttttttttttttttttttttttttttttttttttttttttggggaggggcccacccaatgtttattgaacag
cgaaaacaaaaaaacagcccctggagccctatatggggaaaggaccccttctggacaaaaaaatattcggggcccatggggt
ttgagtctgttaaaaggaccctaaggcccatgggtggggatctttgcgtcataaacacgggggagcccatgggggaaccccc
cttgtctgtaggtttccaggctggcccaaaggggagggcaaaaagttggggtcccaaggatctccccacaacttcctccag
ggcaggatttccacccaggcccaggctgcccgttcctttccttttccgccagtttgggctgggggtgccacgggtcacacccaa
aggagggggggtaggaggccaaggccccancccccccatgtttcttccccgccccgaaagaggaa
<212> Type : DNA
<211> Length : 484
      SequenceName : AI378131
      SequenceDescription :

Sequence
--------
<213> OrganismName : HUMAN
<400> PreSequenceString :
cagctccgaggggagtcggaccggagcaaacagccacccccggcttcgtcccccacgaagaggaagggcaggagccgtgccctc
gaggccgtgcccgccccgcccgccagcggcccccgggctcccgccaaggagtccccacccaagagggtgcccgatcccagccca
gtcaccaagggcactgcggccgagagcggggaggaggcggcgcgggccatcccccgcgagctcccggtcaagagcagctcgctg
ctgccggagatcaagcccgagcacaagaggggcccgctccccaaccacttcaacggccgggcagagggaggtcgaagcagagag
ctgggcagagcggccggagcgcctggagcttctgacgccgacggcttgaagcccaggaaccatttcggcgtgggcaggtcgaca
gtgaccactaaagtgaccctccctgccaagcccaaacatgtggaactaaatcttaaaacccctaagaatcttgacagtttggga
aatgagcacaatccatttagccagccagttcacaaaggcaacactgccaccaaaatctccttatttgaaaacaaacggacaaac
agtagcccaagacacactgacattcgaggcccaaggaatactcctgcctctagtaaaacgtttgttgggagggcaaagctgaat
ttagccaaaaaagccaaagaaatggagcaacctgaaaagaaagtaatgccaaacagtccccagaatggtgtgctggttaaggaa
actgctatagaaaccaaagttaccgtctcggaagaagagattctgccagcaaccagaggaatgaatggagactcttctgagaat
caagctcttggtcctcagcctaaccaagatgataaagcagatgtacaaacagatgctggctgcctttcagaaccagtggcttct
gctctgattcctgtcaaggatcataagctcttagagaaggaggactcagaggctgcagacagcaaaagccttgtacttgaaaat
gtaaccgatacagcacaagacatccccaccactgtggataccaaagatttacctccaacggccatgccaaagccacagcataca
ttttctgactcacagtcccctgctgagtcatctcctgggccttctctttcactgtctgcacccgctcctggggatgttcccaaa
gacacatgtgttcaatcacccataagcagtttcccatgcactgatctaaaagtgtcagaaaaccataaaggatgtgttttgcct
gtgtctcgtcagaacaatgagaaaatgccacttttagaacttggaggagaaacaacccctcctttgtccacagagcgtagtcca
gaagctgtgggaagtgagtgtccatccagagtcctcgtccaggtcaggtccttcgtgctccccgtggagagcacccaggatgtg
agctcccaggtcatcccagagagctctgaagttagagaagtgcagttgccaacttgtcacagtaatgaacctgaagtggtttcc
gttgcaagttgtgctcccccacaagaggaagtactgggcaatgaacactctcattgcacagcagagctcgcggcaaaatctggc
ccacaagtcataccgccagcatcagagaaaactctgcctattcaggctcaaagtcagggcagcagaacaccccctgatggctgaa
tccagtcccaccaactctcccagcagcggaaatcacttagccactcctcaaaggccagatcagactgttacaaatggccaggat
agccctgccagccttttgaacatttctgctggtagtgatgatagtgtatttgattcttcttctgatatggaaaaattcactgaa
attataaaacagatggatagcgcagtttgtatgcccatgaaaagaaagaaggccaggatgccaaactctcctgctcctcacttt
gccatgcctcctattcacgaagaccatttagaaaaggtgtttgatcccaaagtgtttacctttggtttggggaagaagaaggaa
agtcagccagaaatgtcaccggctttacatttgatgcagaaccttgacacaaaatccaaactgagacccaaacgtgcatctgct
gaacagagcgtcctcttcaagtccctgcacaccaacactaatgggaacagtgagcctctggtgatgccggaaatcaatgacaaa
gagaacagggacgtcacaaatggtggcattaagagatcgagactagaaaaaagtgcacttttctcaagcttgttatcttcttta
ccacaagacaaaatcttttctccttctgtgacatcagtcaacactatgaccacggctttcagtacttctcagaacggttcccta
tctcagtcttcagtgtcacagcccacgactgagggtgccccgccctgtggtttgaacaaagaacagtcaaatcttctgcccgac
aactccttaaaggtcttcaatttcaactcgtcaagtacatcacactccagtttgaaaagtccaagccacatggaaaaataccg
caaaaagagaaaaccaaagaagatctggattcacgaagcaacctacacttgccagaaactaaattttctgaattgtcaaaactg
aagaatgatgatatggaaaaggctaatcatattgaaagtgttattaaatcaaacttgccaaactgtgcaaacagtgacaccgac
ttcatgggtcttttcaaatcaagccggtatgacccaagcatttctttttctggaatgtcattatcagacacaatgacacttaga
ggaagtgtccaaaataaactcaatccccgacctggaaaggtagtgatatatagtgaacccgacgtctctgagaagtgcattgaa
gttttcagtgacattcaggattgcagttcttggagcctctctccagtgatactcataaaagttgttagaggatgttggatttg
tatgagcaaccaaattttgaagggcactccatccccttagaagaaggagaattggaactctctggtctctggggtatagaagac
attttggaaaggcacgaagaagcagagtctgataagccagtggtgattggttccatcagacatgtggttcaggattacagagtt
agtcacattgacttatttactgaaccagaagggttaggaatcctaagttcctactttgatgatactgaagaaatgcagggattt
ggtgtaatgcagaagacttgttccatgaaagtacattggggcacgtggctgatttatgaagaacctggatttcagggtgttcct
ttcatcctggaacctggtgaataccctgacttgtccttctgggatacagaagcagcgtacattggatccatgcggcctctgaaa
atgggtggccgtaaagttgaattccctacagatccaaaggtagttgtttatgaaaagcctttctttgaaggaaaatgtgtggaa
ctagaaacaggaatgtgtagttttgtcatggagggaggtgaaacagaagaggcgactggagacgatcatttgccgtttacgtca
gtggggtctatgaaagttctaagaggcatttgggttgcatatgagaagcctggatttaccggtcatcagtatttgctagaagaa
ggagaatacagggactggaaagcctggggaggttacaatggagagcttcagtctttacgacctatattaggtgatttttcaaat
gctcacatgataatgtacagtgaaaaaaactttggatccaaaggttccagtattgatgtattgggaattgttgctaatttaaag
gagactggatatggagtgaagacacagtctattaatgtactgagtggagtatgggtagcctatgaaaatcctgacttcacagga
gaacagtatatactggataaaggattttataccagttttgaggactggggaggcaaaaattataagatctcttctgttcaacct
atatgtttggattctttcactggcccaaggagacgaaatcagattcacttgttttcagaaccacagtttcaaggtcacagtcaa

agttttgaagaaacaacaagtcaaattgatgattcattttctaccaagtcttgcagagtttcaggaggcagctgggttgtatat
gatggagaaaatttcactggtaatcaatacgtgttggaagaaggccattatccttgtctgtctgcaatgggatgcccgcctgga
gcaactttcaagtctcttcgttttatagatgttgaattttctgaaccaacaattattctctttgaaagagaagacttcaaagga
aaaaagattgaacttaatgcagaaactgtcaatctccgatccctgggattcaacacacaaatacgctctgttcaggttattggt
ggcatatgggttacttatgaatatggcagttacagagggcgacagttcctattgtcacctgcagaagtacctaattggtatgaa
ttcagtggctgtcgccaaataggttctctacgaccttttgttcagaagcgaatttatttcagacttcgaaacaaagcaacaggg
ttattcatgtcaaccaatggaaacttagaggatctgaagcttctgaggatacaggtcatggaggatgtcggggccgatgatcag
atttggatctatcaagaaggatgtatcaaatgcaggatagcagaagactgctgcctgacgattgtgggcagcctggtaacatct
ggctccaagctaggcctggccctggaccagaatgctgacagccagttctggagcttgaagtccgatggcaggatttacagcaag
ttgaagccaaatttagttttagacattaaaggggcacacagtatgatcaaaatcacattatcctcaacactgtcagcaaagag
aagtttacacaagtgtgggaagccatggtcctatatacctgaacaaagaaggaagaagaatcttctggaggtccttccagccac
cttatttcttaaaaggacaatgctgatggaagaccagactggaaagtggatcgactcctccttcattgattctaaattcaacc
ttaaatcatgctgccatgactcagagaacttactcatcgtttcaaaagactatcatagctttaaaccaataatttgtcctcctt
tcatttcttgcctttcattttggtagctgcttaaacaggttgcctaattagcagcttttgggtgattttgtaaaatgttatat
caagatttcaagactgtgtacattttaaattatttccaaagatagtgacaggagagaactggaacaaatttaccaactttgtgg
acctacaaagcccttacactttaaagggtaagacaaaggcttaagtttgaaaggtagagaactgtttagcatctgagaagaaat
actttattaggcctgtaattttggttcttggccttaaacactttctggaacctttaaatatgctgcatagcacaatgggaaagc
cttaggtattcacacatttaaggaactctaaacaaaatactattttcctttagttcatattaaaaattaatacattttaaaaat
ttaatgtcaaagtctggtaacatttgttagtaggatttgagttattatttttgagacaggatctcaggctggagtgcagtggc
acaatcacggctcactgcagcctctacctccccaggctcaggtgatcctcccacctcagcctcccaagtagctgggactatagg
cacacatcaccaagcccagccaaatttgttttttttttgtagagatggggtttcatcacgttgcccaggctgatctcgaacct
ctgggctcaagcaattcactcgcctcggcctcccaaaatgctgggattacaggcctgagccactgcgcccagccaggatttgaa
ttattttaactcatccatgggctgccctagaatgtcacaaatgagggttgtttaatgcctttcttatagctgctactggaacac
tattatgacctaatttatgagccatccttactcatctacaagtgctgaagcaatgttacatactttttgctaaactcagattt
tttagcctaatttcttgtcctcctatccacctgcatccacacatggcctgcatggggctgccttccctgcagtgttctgcagcc
atgcttcagggtatagctgttggtggacagcctcaggtcttggggcactatagccactaaacgaggtgtgaaaggctcaagag
gatgaccagcaattaattatccccagaaagtgaaggaaaagagacctttagggatgttgctggtcaagtcttgatttgaccgga
gtcaaatcaatcttcaagcaatcttggaatcctcaactgcagtaagcatttcaaaatgcaaacaaactgcttaacaactgacaa
gacaccagcccatatgctgctcttccaacagtgggttctagctttgaacaaaagtgctaaacatttccttgaatatattcttcc
tctttttgtcctcatcactcaatactggtgctcttgtcacaggtagaacagcttgtttcttttccatctattcaagtgtgtttc
taattctaaaatgctgatcttctctggagtctatggtaggcaattatggtcactggaatagtttgtcttgttttaaaatattat
tggtgcatgtacaacagcatccaacatatctgtcttgttcctagatatatagctctgatttaggccttttgtgcataccatta
caatatggtggggtaagacattctacagtagcctgtgctgaactgatctcttaaataaacttgcttctggttaactaaaaaaa
aaaaaaaaaaaa
<212> Type : DNA
<211> Length : 6817
      SequenceName : U83115
      SequenceDescription :

Sequence
--------
<213> OrganismName : HUMAN
<400> PreSequenceString :
gagaagacgacagaagggagttccaaataggcagtttgtgactgatgatgagcatgttgatgataaagtctcctttatataaca
gccggcagacgacttggtcacgcccacacattttaaattcacataaaggtaaatatatggtcaggcgtggtggcccacacctgt
agtctcagcaccttgggaggcgaaggcgggtggatcatttgaggtcaggagttcaagaccagtctggccaacatggtgaaactc
tatctctaccaaaatacaaaaaaaaaaaagtcgtatcga
<212> Type : DNA
<211> Length : 291
      SequenceName : AI308320
      SequenceDescription :

Sequence
--------
<213> OrganismName : HUMAN
<400> PreSequenceString :
agttcaaatatataccttttatttgaacaagagaatggaggactaggataaaacctacctgggtgaggcagccaaatccatggtg
ccttggtgggggcagacagttcctggggggataacactgaatcccacaaaggcagaaacaatccacacacaggccactgggatca
gcacctgaggggcgagactgagaaaagaactcctcccccaaaaccttctgagccagccttgcaggggtagacagaagccaaaag
gtaggggatggagtgagcactccaggccagcccttctgacctctactgccctgcccagacccacacataccgaaaggagccgga
agacagggagaggagtgtgagttgatgacgttgaagctcgcctccaggggcacacatgaaactggcaggagcccaggtgctgag
aacagaccaccatgagcaggataaccctag
<212> Type : DNA

<211> Length : 450
      SequenceName : AW167520
      SequenceDescription :

Sequence
--------
<213> OrganismName : HUMAN
<400> PreSequenceString :
ttttaaaatgtttattttatttcacctaatatgtacacatagttatataattttgcttagtgaaaagttcatgttttaggtt
ccaagaacttggccaaacttgaacgctggagagaagcgaaattcactccctagctctggaaaggggaatttggtagaagacgga
tctcatccttccaatttcttcccctcaggtagtttctgtaaataattttatgaaaagtatagcaggattctctgttccagtgg
ttatagacaatgggattgacaatcaaatttgcatgtgacaagagaatggtcatcttccttgcccacttgggcttatctttagcc
tgagctagctgaaaaagagtgacacagtccatggtgcgcactggtagcgagcacagagcaaacat
<212> Type : DNA
<211> Length : 401
      SequenceName : AI243166
      SequenceDescription :

Sequence
--------
<213> OrganismName : HUMAN
<400> PreSequenceString :
tgataacccaaggtattcacagcaagatacagtgagtcttaaagttaagcaccgtgcaattagctttgcttccttgggttttg
aaacatgcatctgtataaacctgcctgtgcagacatcccgagccccaagctgggtctgccaaaatccagtgaatcggctctaaa
atgtagatggcacctagcagtgaccaagactcagcctcaggcggcctgcaaacctgtgaggcccagtggagcagccgaacagaa
atatgtggaaaagtttctacgtgttcatggaatttcgttgcaggaaaccaccagagcagagacgggcatggcatacaggaatct
tggaaaatcaggactcagagtttcttgcttgggtcttggaacatgggtgacatttggaggtcaaatttcagatgaggttgctga
acggctgatgaccatcgcctatgaaagtggtgttaacctctttgatactgccgaagtctatgctgctggaaaggctgaagtgat
tctggggagcatcatcaagaagaaaggctggaggaggtccagtctggtcataacaaccaaactctactggggtggaaaagctga
aacagaaagagggctgtcaagaaagcatattattgaaggattgaagggctccctccagaggctgcagctcgagtatgtggatgt
ggtctttgcaaatcgaccggacagtaacactcccatggaagaaattgtccgagccatgacacatgtgataaaccaaggcatggc
gatgtactggggcacctcgagatggagtgctatggagatcatggaagcctattctgtagcaagacagttcaatatgatcccacc
ggtctgtgaacaagctgagtaccatcttttccagagagagaaagtggaggtccagctgccagagctctaccacaaaataggtgt
tggcgcaatgacatggtctccacttgcctgtggaatcatctcaggaaaatacggaaacggggtgcctgaaagttccagggcttc
actgaagtgctaccagtggttgaaagaaagaattgtaagtgaagaagggagaaaacagcaaaacaagctaaaagacctttcccc
aattgcggagcgtctgggatgcacactacctcagctagctgttgcgtggtgcctgagaaatgaaggtgtgagttctgtgctcct
gggatcatccactcctgaacaactcattgaaaaccttggtgccattcaggttctcccaaagatgacatcacatgtggtaaatga
gattgataacatactgcgcaacaagccctacagcaagaaggactatagatcataaggcaatgcatgaaccacagaagctgcatg
gttaaaatagcggcctgtgcccagtacagaaaggtgttactaaccagtcttttgaatcacttagcagcttgctcgtcaacctct
agtgtccctccctggattctttgaggtgtctgctgtcgctaccactgtgcacatctgaaaactcacaaccaagaaaatccattc
tattttcttatcttggactggagtcacctattcttgcattgctgtatacacctcatgcttatgcaatgggaagaatatgggggc
cagggggtgtggtactaccttcaggcatttggtaactcaaagaaggctgtacagatatatttttttcaaaaagaacaaaatccac
agatgcaatgtgagttgcgtaagaaacagagtagatagactaaattcagtgaaggaaaggaattgagagattttttcttagtaaa
tagattattgttaagtaaatagttattaaaaatatatctcactgcaaaaaaaaaaaaagcagtatcttcactcaaaagtcttgc
ttggaagaataagcagaaagaattttatatattttttttctattttcacattcatactaacaagttttgttccatttgttattc
aataaaacaaaaatttctaggtatttgctttattacctttcaaatatttactgttgcttggccccaagaatggccttgtacaac
ttatccagaatgtctattaggattctaatgttatgtccacttacaagtagagacagtaaaaggatgaatacccaatctttagtg
acaatgcagctgatttatgaaagagagggctacactgctatggaaacttagcttcaaagaaaatgcaatgtatctgcaattagg
tgttcatttttttactacattttattaaaacctgctttatactttcaactgcttgtaggcacaacttctgcaagtttaaatattt
gagctttacaaataaacatacacatgctcagtttttttaagtaaacctgtaaaatacccaggaaggcaaatgttcattgtttaa
ttagcactgggattttataatataatgtttggtatttttgaggcattgttaacatgaaagtcaaccactggctttgtgaaaaat
gctatgtcactattcagaatatgctgggtaaattaacttgcctagtgaaaagcaaaatgttaaagaaagaacttctggttctat
aatcatattatatgcactaaactatatgcatgaaagttctttgcatggattaatggggcttacccttgttgcactcgaaatctg
aggtgtatctagccctgccactat[?]ggctacttaccctcattaatatcccacttgagaaaaattg[?]gagactatactgtgtcaa
tatctgtaaaaagagagaaaacatgtttttttttttgaaggggggtggtgtgggagtggccctttaactcctatttggctatc
tgaggatgtacaaaattctcatttaatttctggtcagcaagttccccacacagaaatcactctgaggtttacagaagaactgt
aatattattttaaaatgcgatttctgtcattagttctagatatgtacttcatggttaaattctaaatctgaaaatgctagtgg
gagatatcaagaaattttctttttgattactagtacctgtattctaacagagagtttgaatttttttgcccgtgttatcagaatg
atggaaattgatcattttcagttgttcattgtgtattcaatccagctgaactgctgtatgtatagaggagcttgaggtgctgtc
taatgggaaatgtgatttgattgatttatttgcttagagtaataaaagcattttgtgcattcaatctt
<212> Type : DNA
<211> Length : 3176
      SequenceName : L39833

SequenceDescription :

Sequence
--------
<213> OrganismName : HUMAN
<400> PreSequenceString :
atggctctgcagatccctggaggcttttgggcagcagctgtgaccgtgatgctggtgatgctgagcaccccagtggctgaggcc
agagactttcccaaggatttcttggtccagtttaagggcatgtgctacttcaccaacgggacagagcgcgtgcgcggtgtggcc
agatacatctataaccgcgaggagtacgggcgcttcgacagcgacgttggggagttccaggcggtgaccgagctggggcggagc
atcgaggactggaacaactataaggacttcttggagcaggagcgggccgcggtggacaaggtgtgcagacacaactacgaggcg
gagctgcgcacgaccttgcagcggcaagtggagcccacagtgaccatctccccatccaggacagaggccctcaaccaccacaac
ctgctggtctgctcagtgacagatttctatccagcccagatcaaagtccggtggtttcggaatgaccaggaggagacagccggt
gttgtgtccacctccctcattaggaatggtgactggaccttccagattctggtgatgctggaaataactccccagcgtggagac
atctacacctgccaagtggagcaccccagcctccagagccccatcaccgtggagtggcgggctcagtctgaatctgcccagagc
aagatgctgagtggcattggaggcttcgtgctggggctgatcttcctcgggctgggccttatcatccgtcacaggggtcagaaa
ggacctcgagggcctccaccagcaggtcaccttctgtgatccccatcccaaggcactggtggtgactctgcttcctgcactgac
ccagagcctctgcctgtgcactgcaagctgtgtctactcaggccccaaggggactctctgtttccattctcccccacagacct
gtcaagagaagcatgacaaacaaaatcatttaccgactttagtgctttttccat
<212> Type : DNA
<211> Length : 979
      SequenceName : X87344
      SequenceDescription :

Sequence
--------
<213> OrganismName : HUMAN
<400> PreSequenceString :
caatagttcctttccttatttattagattccagatacatgggaccaggatggaagcatcctagggcatttgatttacatgggg
tttctgggagaacatatacagaggcagaaagacatacaagtaggtgatgctgcaatggggaactgccatttaaaaaatatata
catcttaagaagccattccagccatattatcattattagaaaataaaacaatctttccacatagtgctttctttggtgctgtt
ccgtgagcatggactctggacccttcagtcttcttgtcacaagctggtcaccctcccctgccacttccatctgcacagagggag
ggcagagtctatggggaaaggaggcccccccccaccgacacaggtgggtngcaggttccagctcctctaaggcgctgccatnggc
cttcccgactctggctggagtcttgagagcctcttact
<212> Type : DNA
<211> Length : 458
      SequenceName : AA016239
      SequenceDescription :

Sequence
--------
<213> OrganismName : HUMAN
<400> PreSequenceString :
tttttgtcttgcctgttattttattatttgacattctttaattttgaaaagacacactggacttgattaagatctggctcttc
attgtctccagtcaaatgtcctgtggcgactctctgtggtactgacccttcacatcttggtttgatatggcttactttgtgcaa
agagagggtacagtggagggagtgtatggcggtggactcccaggtgcaggctgcataaactttggtcaagtctgaagcttctac
atcttcattctgtggctctcttaaggccatcaggcaagcttctgtgcagattgccttgatgtcagcaccaaagaggtcatcttt
agtcattatcaagtcgttcaggtttacatcatcggccagtgtcatcatgcttgtatgcatctgaaagatgctcttcttagtatg
ttcattacggaggggaatgccgatcttcctgtcagtgcaggctcgcctgctaagggctgnatacaaaattctgtttggttgccg
catgataactttacatgtnctctagatcaaatccatcaactgttcgcagttc
<212> Type : DNA
<211> Length : 556
      SequenceName : AI024468
      SequenceDescription :

Sequence
--------
<213> OrganismName : HUMAN
<400> PreSequenceString :
tttttttttccaaattattaaatttttttaattaaaaaatttttaaaattatatttgcatatatgaaggtaagtaagacactg
gacaaattatagagtctcaggcttatgtggaattatgactgctctcaacaattactgaaatcccctttcaaagaaaatcttcac
ataaacctatgtttgactatgtaatagaatgaagacagccacagatctggcattctcaaaacatcacacaagtactgcatgtaa
tgctgcaaaataactgttttcctctaaaatttcactaaacaacctcaggcagtacaataaaacaaaacattaagacctcccagt
ggtcatcctaagaattagacagatttctttgatttttcttctctgcttcttccttttccttttctatttcagttacatataat

tcaacttcttttgcactaaacatcttcaaaggttgatttcttcttattatagcaagttcaatgtttttccaccagactggaca
acttctagcaaagcttttattgctaacttgatagcttcactgtcacttgctatggcatcttctgtgtaattcttttctagaaat
tctcgaacagttttagcacttcggcctattgcatttgccttccaagcatgataagtaccaaaaggatctgtctgatacaatctt
ggagatncattattattcaaaccctccaattaaggcggaatacc
<212> Type : DNA
<211> Length : 716
      SequenceName : AI190605
      SequenceDescription :

Sequence
--------
<213> OrganismName : HUMAN
<400> PreSequenceString :
tatttgcattatttttactgttgtcttgttctttttatgttttgcatcctgaatatatttgctccacagttggttgacccta
cgtaattccatggatgcaggacccacaaatgtggatggccgaccgcacttagagaatgctcacctgtctgcagccaagggctgg
aatcacacatctggctgactcactgaaccacaggtgtagctctcagagcctccgggttggaccgaaagaacacagcgtggagta
ggctccacggactacctcatggaccactcctcagggaaagcctctggaaaatatcgcgcccccaagaagggaaaaggaggag
tgcaggagggtggcgcacccacaaaggacccaacgccgggaagaggcagaggatgctgggaagagagaagcctccttgccctga
cacagatatttcttcctct
<212> Type : DNA
<211> Length : 439
      SequenceName : AI656222
      SequenceDescription :

Sequence
--------
<213> OrganismName : HUMAN
<400> PreSequenceString :
taaaatttatactatcaataattgagtttatttcaattaataatgttaacaatgacacatttaacattatgttagagctcaatt
tgtatatattaacatgtatttttaaataatttacgtatttatcgtcgaatcatgtatttttttaaaggggggatgctcaactct
ctcaggcaaaaatgagcacctttccgaacaccgtggggagaggcagggatgcaggcagacatatcctaaactcaccctcccggc
agcatcaaagtagccttcggcagggatttgttataatcagataaggattcgggaaggccctttgagccatgacgc
<212> Type : DNA
<211> Length : 327
      SequenceName : AI695385
      SequenceDescription :

Sequence
--------
<213> OrganismName : HUMAN
<400> PreSequenceString :
ggcaatagaacctagaagtcagggaagctcatcccccaaggctcaggaaagctcccaggnaantggaatcttatctgcatgtcc
ctacttgcactcagcagagggccgcagaaggatcctgttcagggctttaaggaactaaagcattggaaggcatcccatttctac
gggagattggaatagagccctggctgttctggccagtccctgtctcagatgttgcacttccagcacattctacagttattctag
agagctacaagcaagaaagaggggagtctgtgttggtacaaggccacctggggagctgtccctcagagctatcccagtactcag
gagaagttacaaatgtggataagtccagagtttgaatcaagatagacctccaagataatgtctttttgtgaggatttgaagnta
gtcttgaaancaaattagttctttttaaaa
<212> Type : DNA
<211> Length : 450
      SequenceName : T75463
      SequenceDescription :

## Claims

1. A method of determining whether a patient will respond to treatment with an FTI by analyzing the expression of a gene that is differentially modulated in the presence of an FTI.

2. The method of claim 1 wherein the differential modulation is at least 1.5 fold.

3. The method of claim 1 wherein the differential modulation is at least 1.7 fold.

4. The method of claim 1 wherein the analysis is of the expression of more than one gene.

5. The method of claim 1 wherein the gene correlates with one or more nucleic acid sequences identified in Tables 1-3

6. The method of claim 1 used to monitor the therapy of a patient.

7. The method of claim 5 wherein the FTI is (B)-6-[amino(4-chlorophenyl)(1-methyl-1H-imidazol-5-yl)methyl]-4-(3-chlorophenyl)-1-methyl-2(1H)-quinolinone).

8. The method of claim 1 wherein the analysis is of the expression of a group of genes correlating with nucleic acid sequences identified in Tables 1-3 and wherein the FTI is (B)-6-[amino(4-chlorophenyl)(1-methyl-1H-imidazol-5-yl)methyl]-4-(3-chlorophenyl)-1-methyl-2(1H)-quinolinone).

9. A method of treating a patient comprising:
a) analyzing the gene expression profile of said patient to determine whether the patient will respond to treatment with an FTI, and
b) treating the patient with the FTI if the analysis indicates that the patient will respond.

10. The method of claim 9 wherein the analysis is of the expression of more than one gene.

11. The method of claim 9 wherein the FTI is selected from the group consisting of quinolines or quinoline derivatives.

12. The method of claim 11 wherein the FTI is selected from the group consisting of
7-(3-chlorophenyl)-9-[(4-chlorophenyl)-1H-imidazol-1-ylmethyl]2,3-dihydro-1H,5H-benzo[ij]quinolizin-5-one,
7-(3-chlorophenyl)-9-[(4-chlorophenyl)-1H-imidazol-1-ylmethyl]-1,2-dihydro- 4H-pyrrolo[3,2,1-ij]quinoline-4-one,
8-[amino(4-chlorophenyl)(1-methyl-1H-imidazol-5-yl),methyl]-6-(3-chlorophen yl)-1,2-dihydro-4H-pyrrolo[3,2,1-ij]quinolin-4-one,
8-[amino(4-chlorophenyl)(1-niethyl-1H-imidazol-5-yl)methyl]-6-(3-chioropheny 1)-2,3-dihydro-1H,5H-benzo[ij]quinolizin-5-one, and
(B)-6-[amino(4-chlorophenyl)(1-methyl-1H-imidazol-5-yl)methyl]4-(3-chlorophenyl)-1-methyl-2(1H)-quinolinone).

13. The method of claim 12 wherein the FTI is (B)-5-[amino(4-chlorophenyl)(1-methyl-1H-imidazol-5-yl)methyl]-4-(3-chlorophenyl)-1-methyl-2(1H)-quinolinone)

14. The method of claim 10 wherein the genes correlate with one or more nucleic acid sequences identified in Tables 1-3.

15. The method of claim 9 wherein the treatment comprises the administration of an FTI and another therapeutic composition.

16. The method of claim 15 wherein said another therapeutic composition modulates MAPK/ERK signaling pathways, TGFβ, WNT or apoptotic pathways.

17. The method of claim 16 wherein said another composition is selected from the group consisting of tyrosine kinase inhibitors, MEK kinase inhibitors, PI3 kinase inhibitors, MAP kinase inhibitors, apoptosis modulators, and combinations thereof.

18. Articles for assessing the efficacy of treatment of a patient with an FTI comprising a medium with which patient gene expression profiles indicative of FTI response are determined.

19. The articles of claim 18 wherein the gene expression profiles are obtained from a group of genes correlating to more than one nucleic acid sequences identified in Tables 1-3.

20. The articles of claim 19 wherein the nucleic acid sequences are found in Table 3.

21. The articles of claim 18 comprising representations of gene expression profiles fixed to a medium.

22. The articles of claim 18 wherein the medium is computer readable.

23. Kits comprising articles for obtaining gene expression profiles for determining response to FTI treatment.

24. The kits of claim 23 further comprising instructions.
